# EUROPEAN PATENT APPLICATION

(11) **EP 2 253 715 A1**
(43) Date of publication of application: **24.11.2010**
(21) Application number: 09006558.2
(22) Date of filing: 14.05.2009
(51) Int. Cl.: C12Q 1/68

(54) **New targets for cancer therapy and/or diagnosis**

(71) Applicant: RWTH Aachen, 52056 Aachen (DE)
(72) Inventor: Dahl, Edgar, Priv.-Doz. Dr. rer. nat., 4851 Gemmenich (BE); Wiesmann, Frank, 52062 Aachen (DE)
(74) Representative: Kröncke, Rolf

(57) **Abstract**

The present invention relates to new targets for cancer therapy and / or diagnosis. In particular, the present invention provides new prophylactic and therapeutic targets and diagnostic markers for cancer, in particular solid tumours. In a first aspect, the present invention relates to methods of screening, design, engineering or otherwise producing a prophylactic or therapeutic agent for use in treatment of cancer. In a further aspect, the present invention relates to diagnostic methods as well as methods for stratification of cancer-therapy.

## Description

### Field of the invention

The present invention relates to new targets for cancer therapy and/or diagnosis. In particular, the present invention provides new prophylactic and therapeutic targets, and markers for cancer, in particular solid tumours, e.g. for the diagnosis of cancer. In a first aspect, the present invention relates to methods of screening, design, engineering or otherwise producing a prophylactic or therapeutic agent for use in treatment of cancer. In a further aspect, the present invention relates to diagnostic methods as well as methods for stratification of cancer-therapy.

### Background of the invention

Cancer is one of the most prominent causes of human death. For example, breast cancer is one of the most common causes of cancer in women. The likelihood of developing invasive breast cancer during a woman's lifetime is approximately 1 in 7. In this aspect it is expected that the global market for breast cancer is about 11 to 12 Billion US Dollar by 2008. There is an intense search for new compounds useful for the treatment and diagnosis of cancer, e. g. solid tumours, in particular breast cancer.

Considerable commercial and academic resources are directed to identification of candidate therapeutic agents for the treatment of various types of cancer. For example in case of breast cancer, herceptin was developed representing a humanised antibody approved for the treatment of HER2-positive metastasic breast cancer. Other newly designed therapeutic agents include humanised anti-CD20-antibodies, like Rituximab. A further example, Tykerb is a dual kinase inhibitor which inhibits both ErbB-2 and EGFR kinases and may be more effective than e.g. the compound herceptin.

Typically, the various types of cancers, for example breast cancer, are multifactorial diseases with no standardised medication available for patients.

In spite of major advance in early detection and adjacent therapy, diagnosis and treatment as well as stratification of treatment regimen remains a major clinical and social problem.

It is known that molecules of the group of "secreted frizzled related proteins" (SFRPs) are antagonists of the Wnt signalling pathway. The Wnt signalling pathway plays a central role in the development of solid tumours since this signalling pathway activates well known oncogenes like cyclin D1 and c-myc and, thus, promotes development and growth of tumours. Wnt targets can be activated via canonical (β-catenin-dependent) and non-canonical (e.g. EGFR-mediated) signalling (Schlange et al. 2007; Breast Cancer Res. 9:R63). Reactivation of the Wnt signalling cascade in tumours has recently been associated with tumour progression and poor prognosis (Janssen et al. 2006; Gastroenterology. 131:1096-109). SFRP-molecules are able to bind to Wnt-molecules and, thus, to inhibit binding of the Wnt ligand molecules to their intrinsic receptors of the frizzled-family. As a consequence, the activation of the Wnt signalling pathway is reduced or suppressed, thus, explaining the tumour-suppressive properties of SFRP molecules, especially SFRP1. The interplay between the Wnt signalling cascade and SFRP1 is a negative modulator of said signalling cascade or pathway as known in the art. Benefical effects of modulation of SFRP1 have been speculated in the art. However, how SFRP1 specifically impacts and alters the cell response to this event has not been described so far.

In recent years, the tumour-suppressive role of the *SFRP1* gene has been analysed in greater depth. For example it is known that SFRP1 expression is lost during the development of many human solid tumours due to hypermethylation of the *SFRP1* gene promoter. This has been shown e.g. for colon carcinoma (Caldwell et al. 2004; Cancer Res. 64:883-8), lung carcinoma (Fukui et al. 2005; Oncogene 24:6323-7), prostate carcinoma (Lodygin et al. 2005; Cancer Res. 65:4218-27), breast cancer (Veeck et al. 2006; Oncogene 25:3479-88), stomach cancer (Nojima et al. 2007; Oncogene 26:4699-713) and bladder cancer (Urakami et al. 2006; Clin. Cancer Res. 12:2109-16). In these tumour entities, loss of SFRP1 expression due to hypermethylation has often been associated with more agressive types of cancer and reduced overall patient survival. For example, breast cancer patients afflicted with a hypermethylated SFRP1 promoter (i.e. SFRP1-negative tumours) present clearly reduced overall survival in Kaplan-Meier analysis (Veeck et al. 2006; supra). Recently it has been established by further analysis in cell culture that *SFRP1* represents a tumour-suppressor gene, e. g. Schlange et al. (2007). supra; Suzuki et al. (2008). Br J Cancer, 98, 1147-1156.

Thus, there are many lines of evidence nowadays that SFRP1 represents an important novel tumor suppressor gene in the human genome that gets inactivated during human cancer development primarily by epigenetic silencing.

SFRP1 expression could in principle be re-established in the tumours by gene therapy or by epigenetic drugs that could demethylate the *SFRP1* promoter. However, gene therapy approaches have not been very successfully in the last decade and the development of epigenetic drugs that could target specific regions of the human genome is still in its infancy.

Further, involvement of SFRP1 is described for bone formation. For example, Zhou et al., (J. Biol. Chem., 2008, 283(4), 1936-45) report that SFRP1 has a role in osteoblasts formation. Gaur et al., (J. Cell Physiol., 2006, 208(1), 87-96) describe that SFRP1 regulates Wnt signalling for BMP2 induced chondrocyte differentiation.

Thus, the problem underlying the present invention is to provide further targets useful for the development and the design of new prophylactic and therapeutic agents for treatment of e.g. cancers, in particular, solid tumours or agents influencing bone formation, metabolic bone disorders and osteoporosis. Namely, the problem underlying the present invention is to provide targets and their use in methods for generating new therapeutic drugs which modulate expression of proteins and nucleic acid molecules encoding said proteins whereby said expression of nucleic acid molecules encoding these proteins is under regulation of the SFRP1 molecule.

The biological importance of SFRP1 in tumour development can be addressed with respect to drug development, if it is known which genes are regulated by the SFRP1 signalling cascade. Since SFRP1 is a tumor suppressor, it can be expected that SFRP1 suppresses activation of putative oncogenes in those healthy tissues where SFRP1 is abundantly expressed. Thus the present invention has identified "druggable molecules" (i.e. typical drug target molecules that can be targeted by small molecule inhibitors or activators, biological drugs or similar art in the field) that get activated in e.g. breast cell lines when SFRP1 expression is lost.

### Summary of the present invention

The present invention uses two newly developed model systems to determine nucleic acids and proteins encoding said nucleic acids which expression is modulated depending on SFRP1 expression. Namely, in a first model the influence of an apparent loss of SFRP1 through a siRNA-mediated knockdown of mRNA expression in high-expressing benign breast cells and, in a second mode, a forced overexpression of SFRP1 in malignant breast cancer cells without endogenous expression of SFRP1 has been studied.

In the first model, a transformation from normal to more malignant breast cells has been simulated by significantly reducing SFRP1 mRNA expression. While in the second model, the transformation from the cancerous cell to "normalized cells" has been simulated.

Based on the two models mentioned above and described in further detail in the experimental part section, the present inventors found new genes and gene products which expression is altered due to changes in the SFRP1 expression, a known tumour suppressor-gene, as shown in table 2. Based on the first model using a benign cell line wherein the SFRP1 expression was reduced by using siRNA, genes have been identified which expression is increased at least twofold, preferably at least threefold, like at least fourfold compared to the normal cell wherein SFRP1 expression was not manipulated, table 2 upper part.

In addition, based on the second model described in detail below, genes have been identified which expression is downregulated in cells wherein SFRP1 expression were increased, namely in tumour cells normally having strongly suppressed or no SFRP1 expression. Said genes identified according to the present invention are downregulated in cells expressing the SFRP1 gene in an amount of at least two times, preferably three times, like at least four times compared to the non-manipulated cell line having diminished or no expression of the *SFRP1* gene, table 2 lower part.

The genes and gene products identified using the model cells and systems are potential new drug targets for prophylactic or therapeutic agent for use in particular in prophylaxis or treatment of cancers whereby said genes and gene products belong to new classes of SFRP1 expression linked molecules, kinases and transmembrane receptors. Namely, agents or drugs allowing suppressing the expression or allowing suppressing the function of the genes and gene products, proteins, as identified herein, will allow to diminish the development of tumour cells and, in addition, allow to convert tumour cells into cells undergoing e. g. cell death. In another aspect, said agent or drugs are useful for bone formation, metabolic bone disorders, and osteoporosis The identified compounds or agents may represent lead structures for further development of pharmaceuticals. Thus, methods are provided for the screening, developing and engineering lead structures and pharmaceuticals useful for treating cancer or as a prophylactic or therapeutic agent for bone formation, metabolic bone disorders, and osteoporosis.

Moreover, the present invention provides marker genes and gene products useful in various applications. For example, the marker molecules according to the present invention e.g. shown in table 2, allows for the diagnosis of cancer. Further, the marker molecules shown in table 2 and according to Seq. ID Nos. 1 to 130 may be used for the risk assessment of candidate molecules, e.g. drug candidate molecules. Namley, the marker molecules according to the present invention may be used to determine whether said candidate molecules may have adverse effects on cells and the organism, like inducing or promoting the risk of an individual to obtain cancer. Namely, the candidate molecules may be screened on their ability to modulate, i.e. to increase or decrease, the expression of a gene or gene product according to any one of Seq. ID. Nos. 1 to 130. In case the genes or gene products according to the present invention are modulated, for the candidate molecules, there is a risk of interfering with cell development and eventually, may result in the induction of cancerous diseases. For example, the candidate agents are potential drugs and the above method allow to determine the risk of adverse effects of said potential drugs on an organism.

The genes and gene products identified herein are under the control of the tumour suppressor gene *SFRP1* since manipulating the expression of SFRP1, i. e. increasing or decreasing the expression levels of SFRP1, results in an increase or decrease, respectively, of said gene or gene products.

Hence, the genes identified herein are downstream target genes of the SFRP1 controlled signalling cascade.

### Brief description of the drawings

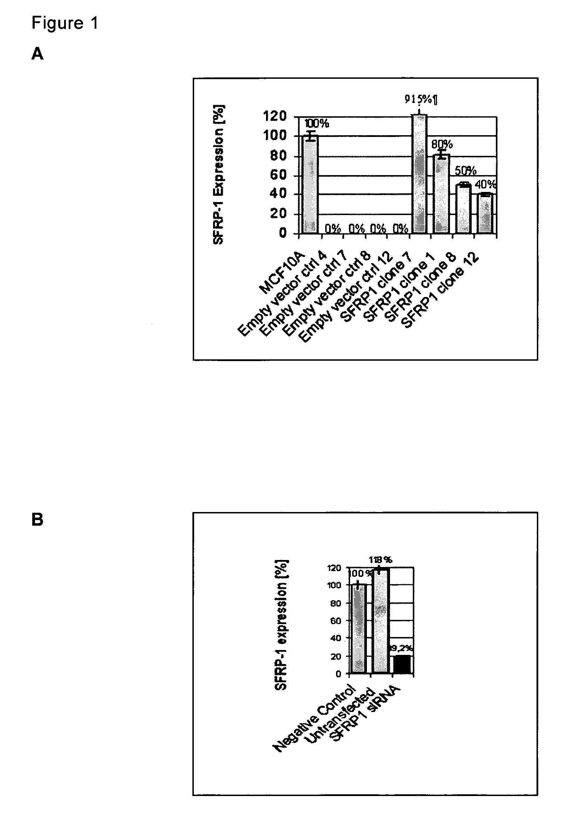

Fig.1 A-C: Expression of SFRP1 mRNA in cell lines (A+B) and primary breast cancer tissues (C) used for validation experiments.
(A) SFRP1 was re-expressed in SFRP1 deficient SKBR3 breast cancer cells (clone 7, 1, 8, 12) to levels (40-915%) corresponding to that found in a benign cell line (MCF10A) (set equal to 100%). (*=out of scale) (B) In another model, SFRP1 was downregulated in vitro by siRNA in MCF10A cell lines to simulate absence of SFRP1 expression. The knockdown of SFRP1 mRNA was >80%.
(C) Expression of SFRP1 target genes was analysed by realtime PCR in a collection of breast cancer samples with clearly varying levels of SFRP1 expression. Expression in normal breast tissue was set equal to 100%. SFRP1 "high expressers" had 20 to 320% SFRP1 expression compared to normal, while SFRP1 "low expressers" had 0-19% SFRP1 expression compared to normal.

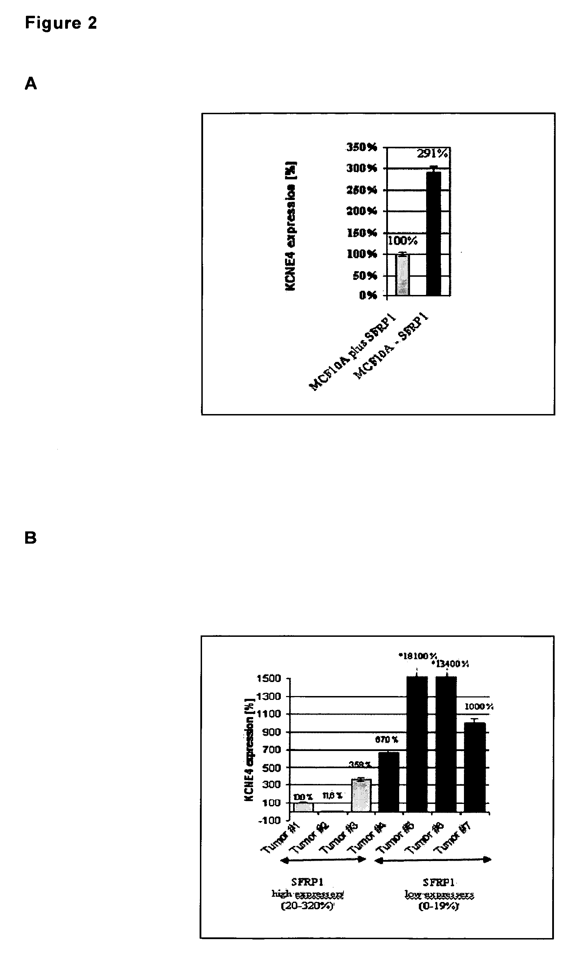

Fig.2: KCNE4 is a SFRP1 target gene *in vitro* and a likely SFRP1 target gene in human breast cancer tissue. (A): Induction of expression of KCNE4 mRNA in MCF10A cells after siRNA induced loss of SFRP1 expression. (B) In breast cancer tissues KCNE4 mRNA is more abundantly expressed in those tumours that show low SFRP1 expression (black bars) compared to those tumours that exhibit abundant SFRP1 expression (grey bars). (*=out of scale)

### Detailed description of the present invention

In a first aspect, the present invention provides a method of screening, designing, engineering or otherwise producing a prophylactic or therapeutic agent for use in the prophylaxis or treatment of cancer, in particular, solid tumours, including the step of determining whether a candidate agent can selectively modulate the expression or function of SFRP1 modulated proteins or nucleic acids encoding the proteins of any one of Seq. ID Nos. 1 to 130 in normal cells or cancer cells to thereby modify one or more cancer-related properties of the normal cell or cancer cell.

That is, the prophylactic or therapeutic agent for use in treatment or prophylaxis of cancer is a compound which modulates the expression of either the nucleic acid or the protein encoded by said nucleic acid or modulates the function of the protein identified in table 2. By modulating the expression or function, the candidate agent allows to prevent or treat cancer, in particular solid tumours, in a subject.

Thus, the prophylactic or therapeutic agent identified according to a method of the present invention allows treating or preventing the development of cancer. Preferably, said cancer is a solid tumour cancer, e. g., colon cancer, lung cancer, prostate cancer, breast cancer, stomach cancer, bladder cancer, renal cell cancer, ovarian cancer, liver cancer and pancreatic cancer.

In a further aspect, the present invention relates to a method of screening, designing, engineering or otherwise producing a prophylactic or therapeutic agent for use in bone formation, metabolic bone disorders, and osteoporosis, including the step of determining whether a candidate agent can selectively modulate the expression or function of at least one of SFRP1 modulated proteins of Seq. ID Nos. 1 to 130 or nucleic acid encoding the proteins in osteoblasts, osteocytes or chondrocytes.

The present inventors found new classes of molecules being SFRP1 expression depending regulated in tissue, kinases and transmembrane receptors. That is, depending on the SFRP1 expression in a cell, the expression of genes and gene products of kinases and transmembrane receptors are regulated, and, thus, may eventually influence the development of cancer or bone formation.

The present inventors identified kinases of Seq. ID. Nos. 1 to 22 and 65 to 72 as identified in table 2 which expression are SFRP1 depending and, in addition, which expression is different in normal cells and tumour cells depending on the level of SFRP1 expression. These kinases represent a drug target according to the present invention allowing the detection and development of lead structures for pharmaceuticals or the therapeutic or prophylactic agent itself.

In another aspect, the drug target for the method according to the present invention is a nucleic acid or protein representing a transmembrane receptor of Seq. ID Nos 23 to 64 and 73 to 130 as identified in table 2.

The cancer-relating properties of a cell which may be modified with the prophylactic or therapeutic agent according to the present invention is preferably selected from the group of molecules related to the six acquired capabilities of cancer cells (Hanahan and Weinberg; Cell 100:57-70), i.e. self-sufficiency in growth signals, evading apoptosis, insensitivity to anti-growth signals, sustained angiogenesis, tissue invasion and metastasis and limitless replicative potential.

By "cancer-related properties" is generally meant any physiological and/or pathological manifestation of a cell which results from cancer of the cell. Within the scope is promotion of transcription, proliferation of the cell, death of the cell (such as apoptosis and necrosis) and invasiveness (whereby invasiveness is inclusive of metastasis, migration and loss of adhesion) (Weinberg, supra).

As used herein, the term "protein" is meant an amino acid-polymer or polypeptide. The amino acids may be natural or non-natural amino acids, D- or L-amino acids as are well understood in the art.

The term "protein" includes and encompasses a "polypeptide", which is typically used to describe a protein having more than fifty (50) amino acids and an "oligopeptide", which is typically used to describe a protein having more than 1 but no more than fifty (50) amino acids.

In the context of the present invention, the term "modulation", "modulator" or "modulating" includes within its scope any interaction which interferes with, inhibits, blocks or hinders, activates or augments either the expression or function of the proteins as defined herein according to Seq. ID Nos. 1 to 130 or functional fragments thereof.

By "selective" or "selectively" is meant a candidate agent that primary affects expression or function of the protein or nucleic acid of Seq. ID Nos. 1 to 130.

The prophylactic or therapeutic agent according to the present invention is preferably selected from the group consisting of an isolated nucleic acid, a protein including polypeptides or oligopeptides, or a small molecule.

That is, in a preferred aspect, the prophylactic or therapeutic agent is a protein or polypeptide interacting with the protein as defined in Seq. ID Nos. 1 to 130, thus, modulating, preferably, decreasing the function of said protein.

For example, the protein is a ligand of the transmembrane-receptor protein or, alternatively, the protein is an antibody, in particular, a monoclonal antibody. Depending on the subject, the antibody is genetically adapted to the organism to be applied to. That is, for the human being, the monoclonal antibody, typically derived from mice, is humanised according to methods known in the art. Preferably, the monoclonal antibody is a fully humanized antibody for application in human subjects.

Alternatively, the prophylactic or therapeutic agent is an isolated nucleic acid, like DNA or RNA or modified DNA or RNA molecules with modifications known in the art. Nucleic acid molecule is typically categorised in interacting with the nucleic acid strand and encoding the proteins according to the present invention. For example, the nucleic acid molecule is a DNA oligonucleotide or a modified version (e.g. morpholinos), silencer RNA, an interfering RNA, an antisense RNA, an artifical micro RNA, ribozyme, etc.

In this connection, the term "isolated nucleic acid" or "isolated nucleic acid molecule" refers to a nucleic acid molecule DNA or RNA that has been removed from its native environment. For example, recombinant nucleic acid molecules contained in a vector are considered isolated for the purpose of the present invention.

Further, the term "gene" is used herein to describe a discrete nucleic acid or locus, unit or region within a gene known that may comprise one or more introns, exons, splice sites, open reading frames and 5' and/or 3' non-coding regulatory sequences such as a promoter and/or polyadenylation sequence. The skilled person is well aware of suitable nucleic acid molecules allowing modulation of the transcription or translation of genes or gene products of Seq. ID Nos. 1 to 130.

In a further aspect, the prophylactic or therapeutic agent is a small molecule. In this context, the term "small molecule" particularly refers to small organic molecules. Typically, said small molecules are part of screening libraries comprising chemical, typically organical, synthetic compounds.

Persons skilled in the art will be aware that anti-cancer therapeutic agents of the invention or agents or compounds useful as a prophylactic or therapeutic agent in bone formation, metabolic bone disorders, and osteoporosis,may be identified by any number of methods. Accordingly, designing, screening, engineering and/or producing methods involves determination of whether a candidate agent can directly modulate expression or function of a nucleic acid or protein as defined herein. In preferred embodiments, methods of the invention involve determining whether the candidate agent can alter expression or function of the nucleic acid or protein, respectively.

In one embodiment, anti-cancer therapeutic agents may be identified by way of screening libraries of molecules such as synthetic chemical libraries, including combinatorial libraries, by methods known in the art.

It is also contemplated that libraries of naturally-occurring molecules may be screened by methodology such as reviewed in Kolb, 1998, Prog. Drug. Res. 51 185. More rational approaches to designing anti-cancer agents may employ X-ray crystallography, NMR spectroscopy, computer assisted screening of structural databases, computer-assisted modelling, or more traditional biophysical techniques which detect molecular binding interactions, as are well known in the art.

A review of structural bioinformatics approaches to drug discovery is provided in Fauman et al, 2003, Meth. Biochem. Anal. 44: 477.

Computer-assisted structural database searching and bioinformatics approaches are becoming increasingly utilized as a procedure for identifying and/or engineering agonists and antagonist molecules. Examples of database searching methods may be found in United States Patent No. 5,752,019 and International Publication WO 97/41526 (directed to identifying EPO mimetics) and United States Patents 7,158,891 and 5,680,331 which are directed to more general computational approaches to protein modelling and structural mimicry of protein activity.

Generally, other applicable methods include any of a variety of biophysical techniques which identify molecular interactions. Methods applicable to potentially useful techniques such as competitive radioligand binding assays, electrophysiology, analytical ultracentrifugation, microcalorimetry, surface plasmon resonance and optical biosensor-based methods are provided in Chapter 20 of CURRENT PROTOCOLS IN PROTEIN SCIENCE Eds. Coligan et al., (John Wiley & Sons, 1997) which is incorporated herein by reference.

A person skilled in the art will appreciate that modulating agents may be in the form of a binding partner and as such, identified by interaction assays such as yeast two-hybrid approaches and the like, but without limitation thereto. Two-hybrid screening methods are provided in Chapter 20 of CURRENT PROTOKOLS IN PROTEIN SCIENCE Eds. Coligan et al., (John Wiley & Sons, 1997) which is incorporated herein by reference.

In a further aspect, the present invention relates to a method for diagnosing cancer, in particular solid tumours, especially breast cancer, or for determining bone formation ability, bone repair ability, bone healing ability or bone formation ability, in a subject, preferably human, comprising steps of determining the level of a protein or a nucleic acid molecule encoding said protein of Seq. ID Nos. 1 to 130 in a test sample; comparing the level of expression of the protein or nucleic acid molecule of interest in the test sample with the level of expression within a reference sample hereby an alteration, e.g. an increase or decrease, of at least twofold of the expression level in the test sample compared to the reference sample, is e.g. indicative for cancer. Preferably, the increase is at least threefold, like at least fourfold or at least fivefold.

In a further aspect, the present invention relates to a method for determining whether a subject is predisposed to cancer or suffering from cancer, in particular, solid tumours, like breast cancer. Said method includes the step of detecting the level of protein or nucleic acid encoding the same of Seq. ID Nos. 1 to 130 in a tissue to thereby determine whether said subject is predisposed to cancer or suffering from cancer.

The terms "predisposed" or "predisposition" are used in the context of a probability that an individual may display clinical symptoms of cancer or that any existing, manifested clinical symptoms of cancer are the result of an underlying biochemical cause.

Moreover, the present invention relates to a method for a risk assessment of a candidate agent, like a drug candidate, to have adverse effects on an organism, like an individual to be treated with said candidate agent. Namely, in case the candidate agent modulates the expression of a gene or gene product according to any one of the sequence of Seq. ID. Nos. 1 to 130 there is a risk for influencing the development of cancer or influencing bone development in the future.

It will be readily appreciated by a person of skill in the art that a number of methods may be utilised to measure the expression levels of the protein of interest, in particular of the transmembrane receptors, in a test sample. By way of example only, fluorescence activated cell sorting (FACS) analysis using labelled antibodies is readily amenable to quantitative measurement of cell surface expression of proteins. For example, immunofluorescence and other fluorescence microscopy methods can also be used to stain tissue to detect levels of the protein as well as other conventional immunohistochemistry techniques.

Alternatively, relative protein expression levels may be determined by other protein-based methods which include immunoassays, for example ELISA and immunoblotting to detect relative expression levels of one as more of said proteins.

The invention further contemplates use of microarray technology to determine the expression pattern profile of cells in order to analyse whether nucleic acid or protein expression is up-regulated in patients with cancer.

Proteomic pattern analysis provides an alternative diagnostic method which is particularly useful for global expression pattern analysis of proteins. Methods of cancer diagnosis using proteomic patterns are provided in Conrads et al Expert Rev Mol Diagn. 2003 Jul;3(4):411-20 and is incorporated herein by reference.

In particular embodiments, a plurality of said proteins may be used in a protein library displayed in a number of ways, e.g., in phage display or cell display systems or by two-dimensional gel electrophoresis, or more specifically, differential two-dimensional gel electrophoresis (2D-DIGE). These particular embodiments may generally be referred to as "proteomic" or "protein profiling" methods, such as described in Chapters 3.9.1 and 22 of CURRENT PROTOCOLS IN PROTEIN SCIENCE Eds. Coligan et al., John Wiley & Sons NY USA (1996-2002).

In embodiments relating to protein arrays, preferably a cancer-associated protein of the invention is located at an identifiable address on the array. Preferably, the protein array comprises a substrate to which is immobilized, impregnated, bound or otherwise coupled cancer-associated protein, or a fragment thereof.

The substrate may be a chemically-derivatized aluminium chip, a synthetic membrane such as PVDF or nitrocellulose, a glass slide or microtiter plates. Detection of substrate-bound proteins may be performed using mass spectrometry, ELISA, immunohistochemistry, fluorescence microscopy or by colorimetric detection.

A person of skill in the art will contemplate that the diagnostic methods of the invention may involve measuring expression levels of a nucleic acid encoding the protein as defined herein for individual.

It is also contemplated that relative levels of nucleic acids may be measured and/or compared in the diagnostic methods of the present invention. By way of example, mRNA levels may be measured. Measurement of relative levels of a nucleic acid level compared to an expressed level of a reference nucleic acid may be conveniently performed using a nucleic acid array.

Nucleic acid array technology has become well known in the art and examples of methods applicable to array technology are provided in Chapter 22 of CURRENT PROTOCOLS IN MOLECULAR BIOLOGY Eds. Ausubel et al. (John Wiley & Sons NY USA 1995-2001).

An array con be generated by various methods, e.g., by photolithographic methods (see, e.g., U.S. Patent Nos. 5,143,854), mechanical methods (e.g., directed-flow methods as described in U.S. Patent No. 5,384,261), pin-based methods (e.g., as described in U.S. Patent No. 5,288,514), and bead-based techniques (e.g., as described in PCT US/93/04145).

Reference is also made to Affymetrix nucleic acid array systems such as described in United States Patent 5,858,659 and United States Patent 6,300,063 which provide specific teaching in relation to nucleic acid array-based detection of disease-related polymorphisms.

In another particular form of this embodiment, quantitative or semi-quantitative PCR using primers corresponding to nucleic acids as defined herein may be used to quantify relative expression levels of said nucleic acid, to thereby determine whether an individual is predisposed to or suffering from cancer.

PCR amplification is not linear and, hence, end point analysis does not always allow for the accurate determination of nucleic acid expression levels. Real-time PCR analysis provides a high throughput means of measuring gene expression levels. It uses specific primers, and fluorescence detection to measure the amount of product after each cycle. Hydridization probes utilise either quencher dyes or fluorescence directly to generate a signal. This method may be used to validate and quantify nucleic acid expression differences in cells or tissues obtained from cancer sufferers compared to cells or tissues obtained from non-sufferers.

In a particular preferred embodiment, the method for diagnosis or cancer or determining whether a subject is predisposed to cancer or suffers from cancer is based on quantitative or semi-quantitative PCR, e.g. realtime PCR analysis.

An elevated level of the protein or nucleic acid molecules in test samples or other types of probes, e. g. tissue, compared to reference samples obtained from a healthy control subject will be indicative for cancer or predisposition of cancer.

Finally, the present invention refers to a drug target for the prophylaxis or treatment of cancer, in particular, solid tumours, or for the prophylaxis or treatment of bone formation ability, bone repair ability, bone healing ability or metabolic bone disorders, and osteoporosis, the drug target is selective from the group of Seq. ID Nos 1 to 130.

The drug targets represent druggable molecules, that is, molecules which allow the development of lead structures or drugs interacting therewith in order to inhibit function or expression thereof. In particular, these drugs targets are drug targets for drugs of the group of small molecules.

For example, the prophylactic or therapeutic agent of the drug target kinase is a kinase inhibitor. For the transmembrane receptor molecules representing suitable drug targets for cancer, in particular solid tumours, the prophylactic or therapeutic agent is a ligand of that receptor which inhibits signalling through the receptor or which act as an antagonist of the receptor. Alternatively, said agent may represent a ligand of the receptor allowing inhibiting interaction of the receptor with its natural ligand. Of course, it is possible that the inhibitor is a small molecule.

The invention will be described in more detail with the following example. However, it is clear that the example represents non-limiting examples of the invention.

### Examples

The impact of SFRP1 loss in human cell lines was analysed in two different cell models. The human SKBR3 breast cell line, that does not endogenously express SFRP1, was stably transfected with a SFRP1 containing vector and served as the SFRP1-plus-model. Transfection experiments were done with Fugene 6 trasnfections reagents (Roche, Switzerland) by using the pEF6N5-His vector (Invitrogen, USA) alone (empty vector control in Figure 1A) or with the integrated SFRP1 coding region (SFRP1 clones in Figure 1A). This approach allowed elevation of SFRP1-mRNA levels in this cancer cell line clones to expression levels equivalent or larger to that of benign breast cells (MCF10A) as determined by real-time PCR by using GAPDH as internal loading control (Figure 1A). On the other hand, the benign cell line MCF10A was transiently transfected by lipofection with SFRP1 specific silencer RNA (siRNA) which induced an artificial reduction of SFRP1 mRNA expression in these cells (SFRP1-minus-model) (Figure 1B). A permanent knockdown of SFRP1 was maintained by multiple transfections with siRNA after every 48 hours. Namely, 5x10⁵ cells were seeded in each well of a 6-well plate to cell confluence rates of approximately 50% and cultured for 144 hours. Cells were transfected with two SFRP1-specific siRNAs (50 nmol) after every 48 hours. The SFRP1-specific knockdown was confirmed by gene-specific real-time PCR and western blot analysis. Cells treated with non-complementary siRNA and untransfected cells served as negative controls. Expression levels in SFRP1-siRNA transfected cells were compared to these controls. Alexa-Fluor 488 labeled siRNA was used for the confirmation of transfection efficiency in one additional well. A sufficient knockdown was achieved when the expression level reached less than 30% of the initial expression level. Knockdown experiments were repeated four-times in total. Sequences used for SFRP1 silencing are provided in Table 1.

**Table 1: Sequences used for in-vitro silencing of SFRP-1 in MCF10A cells.**

| | | |
|---|---|---|
| Seq. ID.No. 131 | SFRP1 siRNA-1 sense | CGC UUA UGU UAA UAG UAA UdtUdt |
| Seq. ID.No. 132 | SFRP1 siRNA-1 antisense | AUU ACU AUU AAC AUA AGC GdAdT |
| Seq. ID.No. 133 | SFRP1 siRNA-1 TARGET | ATC GCT TAT GTT AAT AGT AAT |
| Seq. ID.No. 134 | SFRP1 siRNA-2 sense | GGG CCA UUU AGA UUA GGA AdTdT |
| Seq. ID.No. 135 | SFRP1 siRNA-2 antisense | UUC CUA AUC UAA AUG GCC CdTdT |
| Seq. ID.No. 136 | SFRP1 siRNA-2 TARGET | AAG GGC CAT TTA GAT TAG GAA |

After a validation step, in which the modified expression of SFRP1 was confirmed by real-time PCR analysis and western blot analysis with a SFRP1-specific antibody, the resulting chances in global RNA expression in both models were analysed using Affymetrix expression arrays (U133 PLUS 2.0 array). Results of Affymetrix expression analysis of SFRP1-plus- and SFRP1-minus-transfectants were clustered with DChip_2007 and grouped according to their pathway affiliation (KEGG Pathway http://www.genome.jp/kegg and www.qenecards.org). Genes that are activated after loss of SFRP1 expression were collected if the fold change upregulation in the absence of SFRP1 was at least three-fold.

Table 2 provides a list of druggable molecules of the classes kinase and transmembrane receptors which are regulated by SFRP1. It is differentiated between drug targets identified with the first model (MCF10A cells) and drug targets identified with the second model (SKBR3 cells).

In table 2, the HGNC name (humane genome nomenclature commite; now www.gennames.org), a description of the gene, of the protein class as well as the fold change compared to the non-manipulated cell line are provided.

As demonstrated, the drug targets identified are increased at least threefold when converting the benign MCF10A cell line to a more malignant cell line by diminishing SFRP1 expression. Further, genes are identified having an at least threefold decrease in expression level in SKBR3 breast cancer cells undergoing forced expression of the SFRP1 gene.

### KCNE4 gene as an SFRP1-regulated (suppressed) target gene activated in the benign MCF10A cell model after reduction of SFRP1 expression

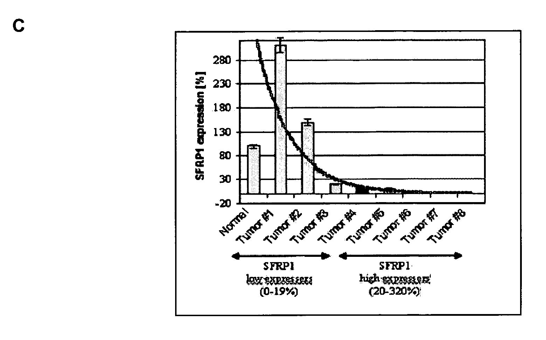

Kinases and transmembrane receptors repressed in their expression by SFRP1 action in vitro (i.e. in the MCF-10A / SKBR3 cell models) may constitute interesting novel drug targets, if their expression is also repressed by SFRP1 in human tumour tissue. Therefore, seven human breast cancer tissues were analyzed for SFRP1 expression by realtime PCR analysis. Tumours were subsequently grouped into "low expressers" (exhibiting 0-19% SFRP1 expression compared to the level found in normal breast tissue) and "high expressers" (exhibiting roughly the same amount of SFRP1 as found in normal breast tissue), see figure 1C. Then expression of SFRP1-regulated candidate genes was analyzed in this tumour cohort as follows: Real-time PCR was performed on an iQ™ 5 real-time PCR Detection System using the iQ™ SYBR® Green Supermix (Bio-Rad Laboratories, Munich, Germany). All reactions consisted of 10 µM forward primer, 10 µM reverse primer, 10 µl iQ™ SYBR® Green Supermix and 2 µl of cDNA as PCR template in a final reaction volume of 20 µl. Gene expression was quantified with the comparative CT method, normalizing CT values to the housekeeping gene Glyeraldehyde-3-phosphate dehydrogenase (GAPDH) and calculating relative expression values. The cycling conditions were set up to an initial denaturation at 95°C for 15 min, followed by 40 cycles with denaturation at 95°C for 20 s, annealing at 60°C for 20 s and elongation at 72°C for 30 s. Subsequent melting curve analyses as well as gel electrophoretical analyses of the obtained products were carried out to verify the specificity of the PCR products. All reactions were performed in triplicate to ensure accuracy of the results.
Figure 2A demonstrates that KCNE4 expression is different in SFRP1 expression regulated MCF10A cells (Fig 2A), i.e. MCF10A cells with RNAi-mediated loss of SFRP1 show a strong upregulation of KCNE4 expression.
Figure 2B demonstrates that this strong upregulation of KCNE4 can also be detected in human breast cancer specimens showing loss of SFRP1 expression (black bars).

**Table 2**

| **MCF10A-Screen** | | | | | | |
|---|---|---|---|---|---|---|
| **No.** | **SeqID** | **Fold change** | **HGNC Name** | **Gene description** | **Probe set** | **Protein class** |
| 1 | 1, 2 | 3,47 | MAP2K7 | mitogen-activated protein kinase kinase 7 | 216206_x_at | kinase |
| 2 | 3 to 8 | 9,15 | PRB1 | proline-rich protein BstNI subfamily 1 | 207752_x_at | p. kinase |
| 3 | 9 to 14 | 3,96 | RBBP6 | Retinoblastoma binding protein 6 | 227635_at | p. kinase |
| 4 | 15 to 18 | 3,19 | RIPK5 | receptor interacting protein kinase 5 | 211514_at | kinase |
| 5 | 19 to 22 | 3,47 | TSSK6 | testis-specific serine kinase 6 | 224409_s_at | kinase |
| 6 | 23 to 26 | 10,23 | ASGR2 | asialoglycoprotein receptor 2 (Ashwell receptor) | 206130_s_at | receptor |
| 7 | 31, 32 | 3,15 | C12orf59 | chromosome 12 open reading frame 59 | 236646_at | p. receptor |
| 8 | 33 to 35 | 3,54 | CD58 | CD58 molecule (lymphocyte function-associated antigen 3) | 222061 at | p. receptor |
| 9 | 36,37 | 4,84 | CLEC1A | C-type lectin domain family 1, member A | 219761_at | p. receptor |
| 10 | 38, 39 | 3,47 | FFAR2 | free fatty acid receptor 2 (GPR43) | 221345_at | receptor |
| 11 | 40, 41 | 3,11 | GLDN | gliomedin | 230360_at | p. receptor |
| 12 | 42,43 | 5,33 | GRM3 | glutamate receptor, metabotropic 3 | 205814_at | receptor |
| 13 | 44,45 | 3,53 | ITGA9 | integrin, alpha 9 | 1555335_at | p. receptor |
| 14 | 46,47 | 6 | KCNE4 | potassium voltage-gated channel, Isk-related family, member 4 | 1552507_at | receptor |
| 15 | 48,49 | 3,66 | KCNT2 | potassium channel, subfamily T, member 2 | 234103_at | p. receptor |
| 16 | 50, 51 | 3,52 | LINGO1 | leucine rich repeat and Ig domain containing 1 | 227933_at | p. receptor |
| 17 | 52, 53 | 3,57 | LTB4R2 | leukotriene B4 receptor 2 | 220130_x_at | receptor |
| 18 | 54 to 59 | 3,03 | NRXN3 | neurexin 3 | 215020_at | p. receptor |
| 19 | 60,61 | 3,41 | PIGT | phosphatidylinositol glycan anchor biosynthesis, class T | 1568986_x_at | p. receptor |
| 20 | 62, 63 | 6,11 | SLC22A8 | solute carrier family 22 (organic anion transporter), member 8 | 231352_at | p. receptor |
| 21 | 64 | 3,77 | TAAR3 | trace amine associated receptor 3 (GPCR) | 221393_at | receptor |

| **SKBR3-Screen** | | | | | | |
|---|---|---|---|---|---|---|
| **No.** | **SeqID** | **Fold change** | **HGNC Name** | **Gene description** | **Probe set** | **Protein class** |
| 1 | 65 to 68 | -3,51 | GPRK7 | G protein-coupled receptor kinase 7 | 1552929_at | kinase |
| 2 | 69, 70 | -3,05 | STK22D | serine/threonine kinase 22D (spermiogenesis associated) | 211694 at | kinase |
| 3 | 71, 72 | -3,58 | TBK1 | TANK-binding kinase 1 | 1554391_at | kinase |
| 4 | 73, 74 | -8,37 | GPR31 | G protein-coupled receptor 31; (PUMA-G) | 208556_at | receptor |
| 5 | 75, 76 | -3,04 | GPR44 | G protein-coupled receptor 44 | 216464_x_at | receptor |
| 6 | 77 to 82 | -4,25 | GRIA2 | glutamate receptor, ionotropic, AMPA 2 | 205358_at | receptor |
| 7 | 83, 84 | | IL13RA2 | interleukin 13 receptor, alpha 2 | 206172_at | receptor |
| 8 | 85, 86 | -2,9 | IL18RAP | interleukin 18 receptor accessory protein | 207072_at | p. receptor |
| 9 | 87, 88 | -6,92 | KCNK3 | Potassium channel, subfamily K, member 3 | 228127_at | receptor |
| 10 | 89, 90 | -4,2 | KIAA0746 | KIAA0746 protein | 212314_at | p. receptor |
| 11 | 91, 92 | -6,74 | KIR3DL3 | killer cell immunoglobulin-like receptor, three domains | 216676_x_at | receptor |
| 12 | 93, 94 | -4,05 | LILRA1 | leukocyte immunoglobulin-like receptor, subfamily, member 1 | 210660_at | receptor |
| 13 | 95 to 106 | -2,92 | NRP2 | Neuropilin 2 | 228103_s_at | p. receptor |
| 14 | 107, 108 | -3,79 | OR4D1 | olfactory receptor, family 4, subfamily D, member 1 | 1567068_at | receptor |
| 15 | 109, 110 | -3,14 | PAG1 | phosphoprotein ass. with glycosphingolipid-microdomains | 225626 at | p. receptor |
| 16 | 111 to 114 | -4,84 | PCDHGB6 | protocadherin gamma subfamily B, 6 | 221682_s_at | p. receptor |
| 17 | 115 to 118 | -4,47 | PKD1L2 | polycystic kidney disease 1-like 2 | 1561849_at | p. receptor |
| 18 | 119, 120 | -4,57 | SCUBE2 | signal peptide, CUB domain, EGF-like 2 | 219197_s_at | p. receptor |
| 19 | 121 to 126 | -2,98 | SIRPB1 | signal-regulatory protein beta 1 | 206934_at | p. receptor |
| 20 | 127, 128 | -7,18 | SLITRK6 | SLIT and NTRK-like family, member 6 | 232481_s_at | p. receptor |
| 21 | 129, 130 | -3,34 | SPAG4 | sperm associated antigen 4 | 219888_at | p. receptor |

| | | | | | | |
|---|---|---|---|---|---|---|
| p. kinase= http://www.itb.cnr.it/kinweb/blast.php p.receptor = http://www.cbs.dtu.dk/services/TMHMM/ | | | | | | |

## Claims

1. A method of screening, designing, engineering or otherwise producing a prophylactic or therapeutic agent for use in prophylaxis or treatment of cancer, including the step of determining whether a candidate agent can selectively modulate the expression or function of at least one of SFRP1 modulated proteins or nucleic acid encoding the proteins of Seq. ID Nos. 1 to 130 in a normal or cancer cell to thereby modify one or more cancer-related properties of said normal or cancer cell.

2. A method of screening, designing, engineering or otherwise producing a prophylactic or therapeutic agent for use in bone formation, metabolic bone disorders, and osteoporosis, including the step of determining whether a candidate agent can selectively modulate the expression or function of at least one of SFRP1 modulated proteins or nucleic acid encoding the proteins of Seq. ID Nos. 1 to 130 in osteoblasts, osteocytes or chondrocytes.

3. The method of claim 1 wherein the cancer is a cancer selected from the group of colon cancer, lung cancer, prostate cancer, breast cancer, stomach cancer, bladder cancer, renal cell cancer, ovarian cancer, liver cancer and pancreatic cancer.

4. The method according to any one of the preceding claims wherein the protein is a kinase of Seq. ID Nos. 1 to 22 and 65 to 72 or wherein the protein is a transmembrane receptor of Seq. ID Nos. 23 to 64 and 73 to 130.

5. The method according to any one of the preceeding claims for producing a therapeutic agent for use in treatment of cancer, in particular breast cancer, or a prophylactic or therapeutic agent for bone formation, metabolic bone disorders, and osteoporosis whereby said candidate agent selectively downregulates a protein or nucleic acids encoding said proteins of Seq. ID Nos. 1 to 64.

6. A method according to any one of claims 1 to 4 for producing a therapeutic or prophylactic agent for use in treatment of cancer, in particular breast cancer, or a prophylactic or therapeutic agent for bone formation, metabolic bone disorders, and osteoporosis whereby a candidate agent can selectively inhibit upregulation of the expression of a protein or a nucleic acid encoding to the same of Seq. ID Nos. 65 to 130.

7. The method according to any one of the preceding claims wherein the candidate agent is selected from the group consisting of an isolated nucleic acid, a protein or a small molecule or wherein the prophylactic or therapeutic agent is an antibody, in particular, a monoclonal antibody or wherein the isolated nucleic acid is a siRNA-molecule.

8. A method for diagnosing cancer, in particular breast cancer, in a subject comprising the steps of determining the level of a protein or a nucleic acid molecule encoding said protein of Seq. ID Nos. 1 to 130 in a test sample; comparing that level of expression with a reference sample whereby an increase of at least twofold of the expression level in the test sample compared to the reference sample is indicative for cancer.

9. A method for determining bone formation ability, bone repair ability, or bone healing ability of a candicate compound comprising the steps of determining the level of a protein or a nucleic acid molecule encoding said protein of Seq. ID Nos. 1 to 130 in a test sample incubated with said candidate compound; comparing said level of expression with a reference sample not incubated with said candidate compound, whereby an alteration of at least twofold of the expression level in the test sample compared to the reference sample is indicative for alteration of bone formation ability, bone repair ability, or bone healing ability.

10. The method according to claim 8 or 9 wherein the present level is determined on nucleic acid level, preferably by nucleic acid amplification methods, in particular PCR.

11. The use of a nucleic acid molecule or a protein encoded by said nucleic acid molecule of Seq. ID Nos. 1 to 130 for diagnosis of cancer, in particular, breast cancer.

12. A method of stratifying a subject for cancer therapy regimen or. a method for stratifying a subject for bone formation regimen comprising the step of determining the expression level of a protein or nucleic acid encoding the same of Seq. ID Nos. 1 to 130.

13. A method for determining whether a subject is predisposed to cancer, in particular breast cancer, or suffering from cancer, in particular breast cancer, said method including the step of detecting the level of a protein or nucleic acid encoding the same of Seq. ID Nos. 1 to 130 in a sample to thereby determine whether said subject is predisposed to cancer or suffering from cancer, in particular breast cancer, preferably, wherein the level is an elevated level compared to a reference sample of a healthy control subject.

14. A drug target for the prophylaxis or treatment of cancer or for the prophylaxis or treatment of bone repair ability, bone healing ability or bone formation ability, in particular, solid tumours, selected from the group of Seg. ID Nos. 1 to 130.

15. Use of a peptide or a nucleic acid encoding the peptide of Seq. ID Nos. 1 to 130 as a screening tool for an agent for preventing or treating cancer or for an agent for use in bone formation, metabolic bone disorders, and osteoporosis.


SEQUENCE LISTING

<110>  RWTH Aachen

<120>  New targets for cancer therapy an/or diagnosis

<130>  4589-001

<160>  136

<170>  PatentIn version 3.3

<210>  1
<211>  3386
<212>  DNA
<213>  human

<220>
<221>  CDS
<222>  (66)..(1322)

<400>  1
   gcggtgtttg tctgccggac tgacgggcgg ccgggcggtg cgcggcggcg gtggcggcgg      60
   ggaag atg gcg gcg tcc tcc ctg gaa cag aag ctg tcc cgc ctg gaa gca      110
         Met Ala Ala Ser Ser Leu Glu Gln Lys Leu Ser Arg Leu Glu Ala
         1               5                   10                  15
   aag ctg aag cag gag aac cgg gag gcc cgg cgg agg atc gac ctc aac      158
   Lys Leu Lys Gln Glu Asn Arg Glu Ala Arg Arg Arg Ile Asp Leu Asn
                   20                  25                  30
   ctg gat atc agc ccc cag cgg ccc agg ccc acc ctg cag ctc ccg ctg      206
   Leu Asp Ile Ser Pro Gln Arg Pro Arg Pro Thr Leu Gln Leu Pro Leu
               35                  40                  45
   gcc aac gat ggg ggc agc cgc tcg cca tcc tca gag agc tcc ccg cag      254
   Ala Asn Asp Gly Gly Ser Arg Ser Pro Ser Ser Glu Ser Ser Pro Gln
           50                  55                  60
   cac ccc acg ccc ccc gcc cgg ccc cgc cac atg ctg ggg ctc ccg tca      302
   His Pro Thr Pro Pro Ala Arg Pro Arg His Met Leu Gly Leu Pro Ser
       65                  70                  75
   acc ctg ttc aca ccc cgc agc atg gag agc att gag att gac cag aag      350
   Thr Leu Phe Thr Pro Arg Ser Met Glu Ser Ile Glu Ile Asp Gln Lys
   80                  85                  90                  95
   ctg cag gag atc atg aag cag acg ggc tac ctg acc atc ggg ggc cag      398
   Leu Gln Glu Ile Met Lys Gln Thr Gly Tyr Leu Thr Ile Gly Gly Gln
                   100                 105                 110
   cgc tac cag gca gaa atc aac gac ctg gag aac ttg ggc gag atg ggc      446
   Arg Tyr Gln Ala Glu Ile Asn Asp Leu Glu Asn Leu Gly Glu Met Gly
               115                 120                 125
   agc ggc acc tgc ggc cag gtg tgg aag atg cgc ttc cgg aag acc ggc      494
   Ser Gly Thr Cys Gly Gln Val Trp Lys Met Arg Phe Arg Lys Thr Gly
           130                 135                 140
   cac gtc att gcc gtt aag caa atg cgg cgc tcc ggg aac aag gag gag      542
   His Val Ile Ala Val Lys Gln Met Arg Arg Ser Gly Asn Lys Glu Glu
       145                 150                 155
   aac aag cgc atc ctc atg gac ctg gat gtg gtg ctg aag agc cac gac      590
   Asn Lys Arg Ile Leu Met Asp Leu Asp Val Val Leu Lys Ser His Asp
   160                 165                 170                 175
   tgc ccc tac atc gtg cag tgc ttt ggg acg ttc atc acc aac acg gac 638
   Cys Pro Tyr Ile Val Gln Cys Phe Gly Thr Phe Ile Thr Asn Thr Asp
   180 185 190
   gtc ttc atc gcc atg gag ctc atg ggc acc tgc gct gag aag ctc aag 686
   Val Phe Ile Ala Met Glu Leu Met Gly Thr Cys Ala Glu Lys Leu Lys
   195 200 205
   aag cgg atg cag ggc ccc atc ccc gag cgc att ctg ggc aag atg aca 734
   Lys Arg Met Gln Gly Pro Ile Pro Glu Arg Ile Leu Gly Lys Met Thr
   210 215 220
   gtg gcg att gtg aag gcg ctg tac tac ctg aag gag aag cac ggt gtc 782
   Val Ala Ile Val Lys Ala Leu Tyr Tyr Leu Lys Glu Lys His Gly Val
   225 230 235
   atc cac cgc gac gtc aag ccc tcc aac atc ctg ctg gac gag cgg ggc 830
   Ile His Arg Asp Val Lys Pro Ser Asn Ile Leu Leu Asp Glu Arg Gly
   240 245 250 255
   cag atc aag ctc tgc gac ttc ggc atc agc ggc cgc ctg gtg gac tcc 878
   Gln Ile Lys Leu Cys Asp Phe Gly Ile Ser Gly Arg Leu Val Asp Ser
   260 265 270
   aaa gcc aag acg cgg agc gcc ggc tgt gcc gcc tac atg gca ccc gag 926
   Lys Ala Lys Thr Arg Ser Ala Gly Cys Ala Ala Tyr Met Ala Pro Glu
   275 280 285
   cgc att gac ccc cca gac ccc acc aag ccg gac tat gac atc cgg gcc 974
   Arg Ile Asp Pro Pro Asp Pro Thr Lys Pro Asp Tyr Asp Ile Arg Ala
   290 295 300
   gac gta tgg agc ctg ggc atc tcg ttg gtg gag ctg gca aca gga cag 1022
   Asp Val Trp Ser Leu Gly Ile Ser Leu Val Glu Leu Ala Thr Gly Gln
   305 310 315
   ttt ccc tac aag aac tgc aag acg gac ttt gag gtc ctc acc aaa gtc 1070
   Phe Pro Tyr Lys Asn Cys Lys Thr Asp Phe Glu Val Leu Thr Lys Val
   320 325 330 335
   cta cag gaa gag ccc ccg ctt ctg ccc gga cac atg ggc ttc tcg ggg 1118
   Leu Gln Glu Glu Pro Pro Leu Leu Pro Gly His Met Gly Phe Ser Gly
   340 345 350
   gac ttc cag tcc ttc gtc aaa gac tgc ctt act aaa gat cac agg aag 1166
   Asp Phe Gln Ser Phe Val Lys Asp Cys Leu Thr Lys Asp His Arg Lys
   355 360 365
   aga cca aag tat aat aag cta ctt gaa cac agc ttc atc aag cgc tac 1214
   Arg Pro Lys Tyr Asn Lys Leu Leu Glu His Ser Phe Ile Lys Arg Tyr
   370 375 380
   gag acg ctg gag gtg gac gtg gcg tcc tgg ttc aag gat gtc atg gcg 1262
   Glu Thr Leu Glu Val Asp Val Ala Ser Trp Phe Lys Asp Val Met Ala
   385 390 395
   aag act gag tca ccg cgg act agc ggc gtc ctg agc cag ccc cac ctg 1310
   Lys Thr Glu Ser Pro Arg Thr Ser Gly Val Leu Ser Gln Pro His Leu
   400 405 410 415
   ccc ttc ttc agg tagctgcttg gcggcggcca gccccacagg gggccagggg 1362
   Pro Phe Phe Arg
   catggccaca ggcccccctc cccacttggc cacccagctg cctgccaggg gagacctggg 1422
   acctggacgg ccacctagga ctgaggacag agagtggggg gtgcccaccc accccccccg 1482
   ccccgggcct accaagcccc cgcccttccc accccggggt cagccggccg tgtgcgtccc 1542
   ccgacagaca ctgtgaacgg aagacagcag gccgcgatca gagtcgctgt tcattcagcc 1602
   gcagcctctg ggccggggcg gcccccaggg gccaggagag agccctggag tcccgcagcc 1662
   accatgcacg ctcccagcgt gctgtgtcct tcgccactcc cacgcgcccg ttcctcttcc 1722
   gtcgccctct gtcccctgct ctacctctct gtccttgtct ggctctcccg tcaccctccc 1782
   tgcctctgtc tctcttctgg cctgagcctg ggcccagcca cctcctgacg ggtcccctgg 1842
   gtctgcatag gtctcccatg gcgcaatgag tcagtggccc ccagccaggc agtgtgggca 1902
   ttgccactgc ggctggacgg ggctgcgcgc tcgcgctctc tctctctctc tctctctctc 1962
   tttgatctca gggggtcctt tttggagttt attgtatttt attgtacttg gtggggtgtt 2022
   tggggtgggg gcggaggaga gcttgttctc gtggggttgt cggtaccttc agaaactttt 2082
   accaaagtca cgattagctg cttgtggtgg ggccccaacc gccctcgggc actggggagc 2142
   tgggctgggg ctgctgctct ggggtctccg ggggccacag cttggggtga gttgaagacc 2202
   tcaggggatg tggaggggtc tgcggggccc tggccgcaca ggatggcctt cagggaaggt 2262
   ggtcttgggg catggtgcag agcaggtgac cggagggaat cggtgacgga gcggggccaa 2322
   gggaggggtc cggagggagt cagggatgga gggcagaggg agtggatgtg ggggtttgag 2382
   gacgtgtgac aagctccagc aggggtgggg gccgggctga gggtgggggt gcgaggtggt 2442
   cactcccatc gtgcccctgg ccgtccctcc actcacccac acctggccca gtccacgttg 2502
   aggtccagga ctgggaagga ccgggtgagt gcaccgggga cccaggccag gtgccccccg 2562
   gagcctgctg gggtggccag agcaggaggg ggtgtgtttc ctttttgtgg gtgttgcatg 2622
   caaatcaagt ggacaagaaa aaataacaaa acaaaaaaca agaaaaaaaa aacacaaaac 2682
   cccgtaaaat cacaaagaaa atccaacacc aaaggcgcag aagccggctg gccgtggtgg 2742
   gggcagcgta ggcgtagcat ccctctcctc tcacttagcc tgttgactct tgttattatc 2802
   atgatattca caaaacgccg catgtttaaa aagtcataga tgtcatcttc tctctgcccc 2862
   cagggaggaa agccaccttc tcttgcccct tggccccttt gtcaggggcc aggggtctgc 2922
   cgggtggggg tgccaacagg cctggccctt tcctcccctg catccagcca tgggggcctc 2982
   tgcgattgcc ggaaggttgc atggctggtc ccagggccag cacaggcccg aggccgggct 3042
   gcctggtttt atttttattt aactttattt tctgttttat gagtgtgtgt ccgcccaccc 3102
   ccaccccctt cagtgttaag tggggagccc tgggggagtc tctcctgcct cccagcctct 3162
   cccaagacct cccccctcgt caccagccat ccctctggac caggcagagg gcggaccggg 3222
   tgggcagggg cctgagggtg gctcgggcca gcccaccagc caatggaccc ctcctcaggc 3282
   cgccagtgtc gccctgcccc tttttaaaac aaaatgccct cgtttgtaaa cccttagacg 3342
   cttgagaata aaccccttcc ttttcttcca aaaaaaaaaa aaaa 3386

<210> 2
<211> 419

<212>  PRT
<213>  human

<400>  2

Met Ala Ala Ser Ser Leu Glu Gln Lys Leu Ser Arg Leu Glu Ala Lys
1               5                   10                  15

Leu Lys Gln Glu Asn Arg Glu Ala Arg Arg Arg Ile Asp Leu Asn Leu
            20                  25                  30

Asp Ile Ser Pro Gln Arg Pro Arg Pro Thr Leu Gln Leu Pro Leu Ala
        35                  40                  45

Asn Asp Gly Gly Ser Arg Ser Pro Ser Ser Glu Ser Ser Pro Gln His
    50                  55                  60

Pro Thr Pro Pro Ala Arg Pro Arg His Met Leu Gly Leu Pro Ser Thr
65                  70                  75                  80

Leu Phe Thr Pro Arg Ser Met Glu Ser Ile Glu Ile Asp Gln Lys Leu
                85                  90                  95

Gln Glu Ile Met Lys Gln Thr Gly Tyr Leu Thr Ile Gly Gly Gln Arg
            100                 105                 110

Tyr Gln Ala Glu Ile Asn Asp Leu Glu Asn Leu Gly Glu Met Gly Ser
        115                 120                 125

Gly Thr Cys Gly Gln Val Trp Lys Met Arg Phe Arg Lys Thr Gly His
    130                 135                 140

Val Ile Ala Val Lys Gln Met Arg Arg Ser Gly Asn Lys Glu Glu Asn
145                 150                 155                 160

Lys Arg Ile Leu Met Asp Leu Asp Val Val Leu Lys Ser His Asp Cys
                165                 170                 175

Pro Tyr Ile Val Gln Cys Phe Gly Thr Phe Ile Thr Asn Thr Asp Val
            180                 185                 190

Phe Ile Ala Met Glu Leu Met Gly Thr Cys Ala Glu Lys Leu Lys Lys
        195                 200                 205

Arg Met Gln Gly Pro Ile Pro Glu Arg Ile Leu Gly Lys Met Thr Val
    210                 215                 220

Ala Ile Val Lys Ala Leu Tyr Tyr Leu Lys Glu Lys His Gly Val Ile
225                 230                 235                 240

His Arg Asp Val Lys Pro Ser Asn Ile Leu Leu Asp Glu Arg Gly Gln
                245                 250                 255

Ile Lys Leu Cys Asp Phe Gly Ile Ser Gly Arg Leu Val Asp Ser Lys
            260                 265                 270

Ala Lys Thr Arg Ser Ala Gly Cys Ala Ala Tyr Met Ala Pro Glu Arg
        275                 280                 285

Ile Asp Pro Pro Asp Pro Thr Lys Pro Asp Tyr Asp Ile Arg Ala Asp
    290                 295                 300

Val Trp Ser Leu Gly Ile Ser Leu Val Glu Leu Ala Thr Gly Gln Phe
305                 310                 315                 320

Pro Tyr Lys Asn Cys Lys Thr Asp Phe Glu Val Leu Thr Lys Val Leu
                325                 330                 335

Gln Glu Glu Pro Pro Leu Leu Pro Gly His Met Gly Phe Ser Gly Asp
            340                 345                 350

Phe Gln Ser Phe Val Lys Asp Cys Leu Thr Lys Asp His Arg Lys Arg
        355                 360                 365

Pro Lys Tyr Asn Lys Leu Leu Glu His Ser Phe Ile Lys Arg Tyr Glu
    370                 375                 380

Thr Leu Glu Val Asp Val Ala Ser Trp Phe Lys Asp Val Met Ala Lys
385                 390                 395                 400

Thr Glu Ser Pro Arg Thr Ser Gly Val Leu Ser Gln Pro His Leu Pro
                405                 410                 415

Phe Phe Arg

<210>  3
<211>  1173
<212>  DNA
<213>  human

<220>
<221>  CDS
<222>  (38)..(1033)

<400>  3
gcacagagtt gggagtgact ccagagcctc ctgcaag atg ctg ttg att ctg ctg       55
                                         Met Leu Leu Ile Leu Leu
                                         1               5

tca gtg gcc ttg ctg gcc ctg agc tca gct cag aac tta aat gaa gat       103
Ser Val Ala Leu Leu Ala Leu Ser Ser Ala Gln Asn Leu Asn Glu Asp
            10                  15                  20

gtc agc cag gaa gaa tct ccc tcc cta ata gca gga aat cca caa gga       151
Val Ser Gln Glu Glu Ser Pro Ser Leu Ile Ala Gly Asn Pro Gln Gly
        25                  30                  35

cca tcc cca caa gga ggc aac aag ccc cag ggc ccc cca cct cct cca 199
Pro Ser Pro Gln Gly Gly Asn Lys Pro Gln Gly Pro Pro Pro Pro Pro
    40                  45                  50

gga aag cca caa gga cca ccc cca caa gga ggc aac aaa cct caa ggt 247
Gly Lys Pro Gln Gly Pro Pro Pro Gln Gly Gly Asn Lys Pro Gln Gly
55                  60                  65                  70

ccc cca cct cca gga aag cca caa gga cca ccc cca caa ggg gac aag 295
Pro Pro Pro Pro Gly Lys Pro Gln Gly Pro Pro Pro Gln Gly Asp Lys
                75                  80                  85

tcc cga agt ccc cga tct cct cca gga aaa cca caa gga cca ccc cca 343
Ser Arg Ser Pro Arg Ser Pro Pro Gly Lys Pro Gln Gly Pro Pro Pro
            90                  95                  100

caa gga ggt aac cag ccc caa ggt ccc cca cct cct cca gga aag cca 391
Gln Gly Gly Asn Gln Pro Gln Gly Pro Pro Pro Pro Pro Gly Lys Pro
        105                 110                 115

caa gga cca ccc cca caa gga ggc aac aga cct caa ggt ccc cca cct 439
Gln Gly Pro Pro Pro Gln Gly Gly Asn Arg Pro Gln Gly Pro Pro Pro
    120                 125                 130

cca gga aag cca caa gga cca ccc cca caa gga gac aag tcc cga agt 487
Pro Gly Lys Pro Gln Gly Pro Pro Pro Gln Gly Asp Lys Ser Arg Ser
135                 140                 145                 150

ccc cga tct cct cca gga aag cca caa gga cca ccc cca caa gga ggt 535
Pro Arg Ser Pro Pro Gly Lys Pro Gln Gly Pro Pro Pro Gln Gly Gly
                155                 160                 165

aac caa ccc caa ggt ccc cca cct cct cca gga aag cca caa gga cca 583
Asn Gln Pro Gln Gly Pro Pro Pro Pro Pro Gly Lys Pro Gln Gly Pro
            170                 175                 180

ccc cca caa gga ggc aag aaa cct cag ggt ccc cca cct cca gga aag 631
Pro Pro Gln Gly Gly Lys Lys Pro Gln Gly Pro Pro Pro Pro Gly Lys
        185                 190                 195

cca caa gga cca ccc cca caa gga gac aag tcc cga agt tcc caa tct 679
Pro Gln Gly Pro Pro Pro Gln Gly Asp Lys Ser Arg Ser Ser Gln Ser
    200                 205                 210

cct cca gga aag cca caa gga cca ccc cca caa gga ggc aac cag ccc 727
Pro Pro Gly Lys Pro Gln Gly Pro Pro Pro Gln Gly Gly Asn Gln Pro
215                 220                 225                 230

caa ggt ccc cca cct cct cca gga aag cca caa gga cca ccc cca caa 775
Gln Gly Pro Pro Pro Pro Pro Gly Lys Pro Gln Gly Pro Pro Pro Gln
                235                 240                 245

gga ggc aac aaa cct caa ggt ccc cca cct cca gga aag cca caa gga 823
Gly Gly Asn Lys Pro Gln Gly Pro Pro Pro Pro Gly Lys Pro Gln Gly
            250                 255                 260

cca ccc gca caa gga ggc agc aag tcc caa agt gcc cga tct cct cca 871
Pro Pro Ala Gln Gly Gly Ser Lys Ser Gln Ser Ala Arg Ser Pro Pro
        265                 270                 275

gga aag cca caa gga cca ccc caa caa gaa ggc aac aat cct caa ggt 919
Gly Lys Pro Gln Gly Pro Pro Gln Gln Glu Gly Asn Asn Pro Gln Gly
    280                 285                 290

ccc cca cct cca gca gga ggc aat ccc cag cag cct cag gca cct cct 967
Pro Pro Pro Pro Ala Gly Gly Asn Pro Gln Gln Pro Gln Ala Pro Pro
295                 300                 305                 310

gct gga cag ccc cag gga cca cca cgc cct cct caa ggg ggc aga cct      1015
Ala Gly Gln Pro Gln Gly Pro Pro Arg Pro Pro Gln Gly Gly Arg Pro
                315                 320                 325

tcc aga cct ccc cag tga cagcctcccc agtcatctag gattcaatga            1063
Ser Arg Pro Pro Gln
            330

caggaagtga ataagaagat gagagtgatt caaatgattc aaattccatg acattggaaa   1123

aaggtcatca tagctctaac ttcaatatac caataaaata atcagcttgc              1173


<210>  4
<211>  331
<212>  PRT
<213>  human

<400>  4

Met Leu Leu Ile Leu Leu Ser Val Ala Leu Leu Ala Leu Ser Ser Ala
1               5                   10                  15

Gln Asn Leu Asn Glu Asp Val Ser Gln Glu Glu Ser Pro Ser Leu Ile
            20                  25                  30

Ala Gly Asn Pro Gln Gly Pro Ser Pro Gln Gly Gly Asn Lys Pro Gln
        35                  40                  45

Gly Pro Pro Pro Pro Pro Gly Lys Pro Gln Gly Pro Pro Pro Gln Gly
    50                  55                  60

Gly Asn Lys Pro Gln Gly Pro Pro Pro Pro Gly Lys Pro Gln Gly Pro
65                  70                  75                  80

Pro Pro Gln Gly Asp Lys Ser Arg Ser Pro Arg Ser Pro Pro Gly Lys
                85                  90                  95

Pro Gln Gly Pro Pro Pro Gln Gly Gly Asn Gln Pro Gln Gly Pro Pro
            100                 105                 110

Pro Pro Pro Gly Lys Pro Gln Gly Pro Pro Pro Gln Gly Gly Asn Arg
        115                 120                 125

Pro Gln Gly Pro Pro Pro Pro Gly Lys Pro Gln Gly Pro Pro Pro Gln
    130                 135                 140

Gly Asp Lys Ser Arg Ser Pro Arg Ser Pro Pro Gly Lys Pro Gln Gly
145                 150                 155                 160

Pro Pro Pro Gln Gly Gly Asn Gln Pro Gln Gly Pro Pro Pro Pro Pro
                165                 170                 175

Gly Lys Pro Gln Gly Pro Pro Pro Gln Gly Gly Lys Lys Pro Gln Gly
            180                 185                 190

Pro Pro Pro Pro Gly Lys Pro Gln Gly Pro Pro Pro Gln Gly Asp Lys
        195                 200                 205

Ser Arg Ser Ser Gln Ser Pro Pro Gly Lys Pro Gln Gly Pro Pro Pro
    210                 215                 220

Gln Gly Gly Asn Gln Pro Gln Gly Pro Pro Pro Pro Pro Gly Lys Pro
225                 230                 235                 240

Gln Gly Pro Pro Pro Gln Gly Gly Asn Lys Pro Gln Gly Pro Pro Pro
                245                 250                 255

Pro Gly Lys Pro Gln Gly Pro Pro Ala Gln Gly Gly Ser Lys Ser Gln
            260                 265                 270

Ser Ala Arg Ser Pro Pro Gly Lys Pro Gln Gly Pro Pro Gln Gln Glu
        275                 280                 285

Gly Asn Asn Pro Gln Gly Pro Pro Pro Pro Ala Gly Gly Asn Pro Gln
    290                 295                 300

Gln Pro Gln Ala Pro Pro Ala Gly Gln Pro Gln Gly Pro Pro Arg Pro
305                 310                 315                 320

Pro Gln Gly Gly Arg Pro Ser Arg Pro Pro Gln
                325                 330

<210>  5
<211>  774
<212>  DNA
<213>  human

<220>
<221>  CDS
<222>  (38)..(634)

<400>  5
gcacagagtt gggagtgact ccagagcctc ctgcaag atg ctg ttg att ctg ctg       55
                                         Met Leu Leu Ile Leu Leu
                                         1               5

tca gtg gcc ttg ctg gcc ctg agc tca gct cag aac tta aat gaa gat       103
Ser Val Ala Leu Leu Ala Leu Ser Ser Ala Gln Asn Leu Asn Glu Asp
            10                  15                  20

gtc agc cag gaa gaa tct ccc tcc cta ata gca gga aat cca caa gga       151
Val Ser Gln Glu Glu Ser Pro Ser Leu Ile Ala Gly Asn Pro Gln Gly
        25                  30                  35

cca tcc cca caa gga ggc aac aag ccc cag ggc ccc cca cct cct cca       199
Pro Ser Pro Gln Gly Gly Asn Lys Pro Gln Gly Pro Pro Pro Pro Pro
    40                  45                  50

gga aag cca caa gga cca ccc cca caa gga ggc aac aaa cct caa ggt       247
Gly Lys Pro Gln Gly Pro Pro Pro Gln Gly Gly Asn Lys Pro Gln Gly
55                  60                  65                  70

ccc cca cct cca gga aag cca caa gga cca ccc cca caa ggg gac aag       295

Pro Pro Pro Pro Gly Lys Pro Gln Gly Pro Pro Pro Gln Gly Asp Lys
                75                  80                  85

tcc cga agt ccc cga tct cct cca gga aaa cca caa gga cca ccc cca      343
Ser Arg Ser Pro Arg Ser Pro Pro Gly Lys Pro Gln Gly Pro Pro Pro
            90                  95                  100

caa gga gga aag cca caa gga cca ccc cca caa gga ggc aac aaa cct      391
Gln Gly Gly Lys Pro Gln Gly Pro Pro Pro Gln Gly Gly Asn Lys Pro
        105                 110                 115

caa ggt ccc cca cct cca gga aag cca caa gga cca ccc gca caa gga      439
Gln Gly Pro Pro Pro Pro Gly Lys Pro Gln Gly Pro Pro Ala Gln Gly
    120                 125                 130

ggc agc aag tcc caa agt gcc cga tct cct cca gga aag cca caa gga      487
Gly Ser Lys Ser Gln Ser Ala Arg Ser Pro Pro Gly Lys Pro Gln Gly
135                 140                 145                 150

cca ccc caa caa gaa ggc aac aat cct caa ggt ccc cca cct cca gca      535
Pro Pro Gln Gln Glu Gly Asn Asn Pro Gln Gly Pro Pro Pro Pro Ala
                155                 160                 165

gga ggc aat ccc cag cag cct cag gca cct cct gct gga cag ccc cag      583
Gly Gly Asn Pro Gln Gln Pro Gln Ala Pro Pro Ala Gly Gln Pro Gln
            170                 175                 180

gga cca cca cgc cct cct caa ggg ggc aga cct tcc aga cct ccc cag      631
Gly Pro Pro Arg Pro Pro Gln Gly Gly Arg Pro Ser Arg Pro Pro Gln
        185                 190                 195

tga cagcctcccc agtcatctag gattcaatga caggaagtga ataagaagat      684

gagagtgatt caaatgattc aaattccatg acattggaaa aaggtcatca tagctctaac      744

ttcaatatac caataaaata atcagcttgc      774

<210>  6
<211>  198
<212>  PRT
<213>  human

<400>  6

Met Leu Leu Ile Leu Leu Ser Val Ala Leu Leu Ala Leu Ser Ser Ala
1               5                   10                  15

Gln Asn Leu Asn Glu Asp Val Ser Gln Glu Glu Ser Pro Ser Leu Ile
            20                  25                  30

Ala Gly Asn Pro Gln Gly Pro Ser Pro Gln Gly Gly Asn Lys Pro Gln
        35                  40                  45

Gly Pro Pro Pro Pro Pro Gly Lys Pro Gln Gly Pro Pro Pro Gln Gly
    50                  55                  60

Gly Asn Lys Pro Gln Gly Pro Pro Pro Pro Gly Lys Pro Gln Gly Pro
65                  70                  75                  80

Pro Pro Gln Gly Asp Lys Ser Arg Ser Pro Arg Ser Pro Pro Gly Lys
                85                  90                  95

Pro Gln Gly Pro Pro Pro Gln Gly Gly Lys Pro Gln Gly Pro Pro Pro
            100                 105                 110

Gln Gly Gly Asn Lys Pro Gln Gly Pro Pro Pro Pro Gly Lys Pro Gln
        115                 120                 125

Gly Pro Pro Ala Gln Gly Gly Ser Lys Ser Gln Ser Ala Arg Ser Pro
    130                 135                 140

Pro Gly Lys Pro Gln Gly Pro Pro Gln Gln Glu Gly Asn Asn Pro Gln
145                 150                 155                 160

Gly Pro Pro Pro Pro Ala Gly Gly Asn Pro Gln Gln Pro Gln Ala Pro
                165                 170                 175

Pro Ala Gly Gln Pro Gln Gly Pro Pro Arg Pro Pro Gln Gly Gly Arg
            180                 185                 190

Pro Ser Arg Pro Pro Gln
        195

<210> 7
<211> 714
<212> DNA
<213> human

<220>
<221> CDS
<222> (38)..(574)

<400> 7
   gcacagagtt gggagtgact ccagagcctc ctgcaag atg ctg ttg att ctg ctg 55
   Met Leu Leu Ile Leu Leu
   1 5
   tca gtg gcc ttg ctg gcc ctg agc tca gct cag aac tta aat gaa gat 103
   Ser Val Ala Leu Leu Ala Leu Ser Ser Ala Gln Asn Leu Asn Glu Asp
   10 15 20
   gtc agc cag gaa gaa tct ccc tcc cta ata gca gga aat cca caa gga 151
   Val Ser Gln Glu Glu Ser Pro Ser Leu Ile Ala Gly Asn Pro Gln Gly
   25 30 35
   cca tcc cca caa gga ggc aac aag ccc cag ggc ccc cca cct cct cca 199
   Pro Ser Pro Gln Gly Gly Asn Lys Pro Gln Gly Pro Pro Pro Pro Pro
   40 45 50
   gga aag cca caa gga cca ccc cca caa gga ggc aac aaa cct caa ggt 247
   Gly Lys Pro Gln Gly Pro Pro Pro Gln Gly Gly Asn Lys Pro Gln Gly
   55 60 65 70
   ccc cca cct cca gga aag cca caa gga cca ccc cca caa ggg gac aag 295
   Pro Pro Pro Pro Gly Lys Pro Gln Gly Pro Pro Pro Gln Gly Asp Lys
   75 80 85
   tcc cga agt ccc cga tct cct cca gga aaa cca caa gga cca ccc cca 343
   Ser Arg Ser Pro Arg Ser Pro Pro Gly Lys Pro Gln Gly Pro Pro Pro
   90 95 100
   caa gga gga aag cca caa gga cca ccc gca caa gga ggc agc aag tcc     391
   Gln Gly Gly Lys Pro Gln Gly Pro Pro Ala Gln Gly Gly Ser Lys Ser
           105                 110                 115
   caa agt gcc cga tct cct cca gga aag cca caa gga cca ccc caa caa     439
   Gln Ser Ala Arg Ser Pro Pro Gly Lys Pro Gln Gly Pro Pro Gln Gln
       120                 125                 130
   gaa ggc aac aat cct caa ggt ccc cca cct cca gca gga ggc aat ccc     487
   Glu Gly Asn Asn Pro Gln Gly Pro Pro Pro Pro Ala Gly Gly Asn Pro
   135                 140                 145                 150
   cag cag cct cag gca cct cct gct gga cag ccc cag gga cca cca cgc 535
   Gln Gln Pro Gln Ala Pro Pro Ala Gly Gln Pro Gln Gly Pro Pro Arg
   155 160 165
   cct cct caa ggg ggc aga cct tcc aga cct ccc cag tga cagcctcccc     584
   Pro Pro Gln Gly Gly Arg Pro Ser Arg Pro Pro Gln
               170                 175
   agtcatctag gattcaatga caggaagtga ataagaagat gagagtgatt caaatgattc     644
   aaattccatg acattggaaa aaggtcatca tagctctaac ttcaatatac caataaaata     704
   atcagcttgc                                                            714

<210>  8
<211>  178
<212>  PRT
<213>  human

<400>  8

Met Leu Leu Ile Leu Leu Ser Val Ala Leu Leu Ala Leu Ser Ser Ala
1               5                   10                  15

Gln Asn Leu Asn Glu Asp Val Ser Gln Glu Glu Ser Pro Ser Leu Ile
            20                  25                  30

Ala Gly Asn Pro Gln Gly Pro Ser Pro Gln Gly Gly Asn Lys Pro Gln
        35                  40                  45

Gly Pro Pro Pro Pro Pro Gly Lys Pro Gln Gly Pro Pro Pro Gln Gly
    50                  55                  60

Gly Asn Lys Pro Gln Gly Pro Pro Pro Pro Gly Lys Pro Gln Gly Pro
65                  70                  75                  80

Pro Pro Gln Gly Asp Lys Ser Arg Ser Pro Arg Ser Pro Pro Gly Lys
                85                  90                  95

Pro Gln Gly Pro Pro Pro Gln Gly Gly Lys Pro Gln Gly Pro Pro Ala
            100                 105                 110

Gln Gly Gly Ser Lys Ser Gln Ser Ala Arg Ser Pro Pro Gly Lys Pro
        115                 120                 125

Gln Gly Pro Pro Gln Gln Glu Gly Asn Asn Pro Gln Gly Pro Pro Pro
    130                 135                 140

Pro Ala Gly Gly Asn Pro Gln Gln Pro Gln Ala Pro Pro Ala Gly Gln
145                 150                 155                 160

Pro Gln Gly Pro Pro Arg Pro Pro Gln Gly Gly Arg Pro Ser Arg Pro
                165                 170                 175

Pro Gln

<210>  9
<211>  6853
<212>  DNA
<213>  human

<220>
<221>  CDS
<222>  (1041)..(6419)

<400>  9
gccaggccgc gtccgccata gtacctggct tggaggtgtc gccgccgctc ggtgagagcc      60

cccgagcggc agggggccaa cacaaaaagg gagccggaga agccctagcc gctgcccagc     120

agcttgcggg cgtgttctcg cggttccggg cctcaaggcg acggaaacga aaggcgagcg     180

aagcgcggag gatccggcga gaagaagcgt cagggagcct cggcggtgtc cccggggtcc     240

gccgaagcca cccggccgcc ggctggggcc cggggtggtg aggaagtgct ccgaggcctc     300

gccgaggcct agcgccggct ttgtgtccga ggcggcggcg gcggcggggg gaggcggagc     360

cgggggcggc ctgcgggaag gcctctcctc cgccgaccgc gcgttttcgg cctaggccgt     420

ggggccgctc gtggcctccg gggagcaggc gccaggggtt tgtgtgcggt gggggcctgg     480

gcctgggcct ggggaagctg acgccggtcg tccggaagcc aggaggaggc gtgaggccgc     540

tcgtggactc cgggcctagg ccctctcccc tcaaccttct cccggggcct gggtcacccc     600

aatccacgga gagagagacc cgccgggagg tgcggccgcg ctatggaccc ctgaccccgt     660

ggggtcgctc ggactcttaa cgtgtggact gaccgctact gactgcaccg ccaatccccc     720

cgtctctgcc ggccccttag catgagcgag ggggacccag ccgggtgaca ttgtgcccgt     780

tggcggattc tcgatttccc ctcttccccg tcctcgtcct cctcctcccc catgaagtga     840

ttctgagtat cggggggtct ctggattatt gttctgacga acccctgctt gtggttgggg     900

ggtatttaat ctgaggcctt agggtccttc ggtgtctttg agtgttttgt gtgtacatat     960

tttgctctta aagtttataa atatacgtat attgagagtg tccacgtctc ctcgctgaac    1020

cttaggaatc ccttggcacc atg tcc tgt gtg cat tat aaa ttt tcc tct aaa    1073
                      Met Ser Cys Val His Tyr Lys Phe Ser Ser Lys
                      1               5                   10

ctc aac tat gat acc gtc acc ttt gat ggg ctc cac atc tcc ctc tgc    1121
Leu Asn Tyr Asp Thr Val Thr Phe Asp Gly Leu His Ile Ser Leu Cys
            15                  20                  25

gac tta aag aag cag att atg ggg aga gag aag ctg aaa gct gcc gac    1169

Asp Leu Lys Lys Gln Ile Met Gly Arg Glu Lys Leu Lys Ala Ala Asp
        30                  35                  40

tgc gac ctg cag atc acc aat gcg cag acg aaa gaa gaa tat act gat      1217
Cys Asp Leu Gln Ile Thr Asn Ala Gln Thr Lys Glu Glu Tyr Thr Asp
    45                  50                  55

gat aat gct ctg att cct aag aat tct tct gta att gtt aga aga att      1265
Asp Asn Ala Leu Ile Pro Lys Asn Ser Ser Val Ile Val Arg Arg Ile
60                  65                  70                  75

cct att gga ggt gtt aaa tct aca agc aag aca tat gtt ata agt cga      1313
Pro Ile Gly Gly Val Lys Ser Thr Ser Lys Thr Tyr Val Ile Ser Arg
                80                  85                  90

act gaa cca gcg atg gca act aca aaa gca att gat gac tct tcc gcg      1361
Thr Glu Pro Ala Met Ala Thr Thr Lys Ala Ile Asp Asp Ser Ser Ala
            95                  100                 105

tct att tct ctg gcc cag ctt aca aag act gcc aat ctg gct gaa gcc      1409
Ser Ile Ser Leu Ala Gln Leu Thr Lys Thr Ala Asn Leu Ala Glu Ala
        110                 115                 120

aat gct tct gaa gaa gat aaa att aaa gca atg atg tcg caa tct ggc      1457
Asn Ala Ser Glu Glu Asp Lys Ile Lys Ala Met Met Ser Gln Ser Gly
    125                 130                 135

cat gaa tac gac cca atc aat tac atg aag aaa cct cta ggt cca cca      1505
His Glu Tyr Asp Pro Ile Asn Tyr Met Lys Lys Pro Leu Gly Pro Pro
140                 145                 150                 155

cct cca tct tac acg tgt ttc cgt tgt ggt aaa cct gga cat tat att      1553
Pro Pro Ser Tyr Thr Cys Phe Arg Cys Gly Lys Pro Gly His Tyr Ile
                160                 165                 170

aag aat tgc cca aca aat ggg gat aaa aac ttt gaa tct ggt cct agg      1601
Lys Asn Cys Pro Thr Asn Gly Asp Lys Asn Phe Glu Ser Gly Pro Arg
            175                 180                 185

att aaa aag agc act gga att ccc aga agt ttc atg atg gaa gtg aaa      1649
Ile Lys Lys Ser Thr Gly Ile Pro Arg Ser Phe Met Met Glu Val Lys
        190                 195                 200

gat cct aat atg aaa ggt gca atg ctt acc aac act gga aaa tat gca      1697
Asp Pro Asn Met Lys Gly Ala Met Leu Thr Asn Thr Gly Lys Tyr Ala
    205                 210                 215

ata cca act ata gat gca gaa gca tat gca att ggg aag aaa gag aaa      1745
Ile Pro Thr Ile Asp Ala Glu Ala Tyr Ala Ile Gly Lys Lys Glu Lys
220                 225                 230                 235

cct ccc ttc tta cca gag gag cca tct tct tcc tca gaa gaa gat gat      1793
Pro Pro Phe Leu Pro Glu Glu Pro Ser Ser Ser Ser Glu Glu Asp Asp
                240                 245                 250

cct atc cca gat gaa ttg ttg tgt ctc atc tgc aag gat att atg act      1841
Pro Ile Pro Asp Glu Leu Leu Cys Leu Ile Cys Lys Asp Ile Met Thr
            255                 260                 265

gat gct gtt gtg att ccc tgc tgt gga aac agt tac tgt gat gaa tgt      1889
Asp Ala Val Val Ile Pro Cys Cys Gly Asn Ser Tyr Cys Asp Glu Cys
        270                 275                 280

ata aga aca gca ctc ctg gaa tca gat gag cac aca tgt ccg acg tgt      1937
Ile Arg Thr Ala Leu Leu Glu Ser Asp Glu His Thr Cys Pro Thr Cys
    285                 290                 295

cat caa aat gat gtt tct cct gat gct tta att gcc aat aaa ttt tta      1985

His Gln Asn Asp Val Ser Pro Asp Ala Leu Ile Ala Asn Lys Phe Leu
300                 305                 310                 315

cga cag gct gta aat aac ttc aaa aat gaa act ggc tat aca aaa aga      2033
Arg Gln Ala Val Asn Asn Phe Lys Asn Glu Thr Gly Tyr Thr Lys Arg
                320                 325                 330

cta cga aaa cag tta cct cct cca cca ccc cca ata cca cct ccg aga      2081
Leu Arg Lys Gln Leu Pro Pro Pro Pro Pro Pro Ile Pro Pro Pro Arg
            335                 340                 345

cca ctg att cag agg aac cta caa cct ctg atg aga tct ccg ata tca      2129
Pro Leu Ile Gln Arg Asn Leu Gln Pro Leu Met Arg Ser Pro Ile Ser
        350                 355                 360

aga caa caa gat cct ctt atg att cca gtg aca tct tca tca act cac      2177
Arg Gln Gln Asp Pro Leu Met Ile Pro Val Thr Ser Ser Ser Thr His
    365                 370                 375

cca gct ccg tct ata tct tca tta act tct aat cag tct tcc ttg gcc      2225
Pro Ala Pro Ser Ile Ser Ser Leu Thr Ser Asn Gln Ser Ser Leu Ala
380                 385                 390                 395

cct cct gtg tct gga aat ccg tct tct gct cca gct cct gta cct gat      2273
Pro Pro Val Ser Gly Asn Pro Ser Ser Ala Pro Ala Pro Val Pro Asp
                400                 405                 410

ata act gca aca gta tcc ata tca gtt cat tca gaa aaa tca gat gga      2321
Ile Thr Ala Thr Val Ser Ile Ser Val His Ser Glu Lys Ser Asp Gly
            415                 420                 425

cct ttt cgg gat tct gat aat aaa ata ttg cca gct gca gct ctt gca      2369
Pro Phe Arg Asp Ser Asp Asn Lys Ile Leu Pro Ala Ala Ala Leu Ala
        430                 435                 440

tca gag cac tca aag gga acc tcc tca att gca att acc gct ctt atg      2417
Ser Glu His Ser Lys Gly Thr Ser Ser Ile Ala Ile Thr Ala Leu Met
    445                 450                 455

gaa gag aag ggt tac cag gtg cct gtt ctt gga acc cca tct ttg ctt      2465
Glu Glu Lys Gly Tyr Gln Val Pro Val Leu Gly Thr Pro Ser Leu Leu
460                 465                 470                 475

gga cag tca tta ttg cat gga cag ttg atc ccc aca act ggt cca gta      2513
Gly Gln Ser Leu Leu His Gly Gln Leu Ile Pro Thr Thr Gly Pro Val
                480                 485                 490

aga ata aat act gct cgt cca ggt ggt ggt cga cca ggc tgg gaa cat      2561
Arg Ile Asn Thr Ala Arg Pro Gly Gly Gly Arg Pro Gly Trp Glu His
            495                 500                 505

tcc aac aaa ctt ggc tat ctg gtt tct cca cca caa caa att aga aga      2609
Ser Asn Lys Leu Gly Tyr Leu Val Ser Pro Pro Gln Gln Ile Arg Arg
        510                 515                 520

ggg gag agg agc tgc tac aga agt ata aac cgt ggg cga cac cac agc      2657
Gly Glu Arg Ser Cys Tyr Arg Ser Ile Asn Arg Gly Arg His His Ser
    525                 530                 535

gaa aga tca cag agg act caa ggc ccg tca cta cca gca act cca gtc      2705
Glu Arg Ser Gln Arg Thr Gln Gly Pro Ser Leu Pro Ala Thr Pro Val
540                 545                 550                 555

ttt gta cct gtt cca cca cct cct ttg tat ccg cct cct ccc cat aca      2753
Phe Val Pro Val Pro Pro Pro Pro Leu Tyr Pro Pro Pro Pro His Thr
                560                 565                 570

ctt cct ctc cct ccg ggt gtt cct cct cca cag ttt tct cct cag ttt      2801

Leu Pro Leu Pro Pro Gly Val Pro Pro Pro Gln Phe Ser Pro Gln Phe
            575                 580                 585

cct cct ggc cag cca cca ccc gct ggg tat agt gtc cct cct cca ggg 2849
Pro Pro Gly Gln Pro Pro Pro Ala Gly Tyr Ser Val Pro Pro Pro Gly
        590                 595                 600

ttt cct cca gct cct gcc aat tta tca aca cct tgg gta tca tca gga 2897
Phe Pro Pro Ala Pro Ala Asn Leu Ser Thr Pro Trp Val Ser Ser Gly
    605                 610                 615

gtg cag aca gct cat tca aat acc atc cca aca aca caa gca cca cct 2945
Val Gln Thr Ala His Ser Asn Thr Ile Pro Thr Thr Gln Ala Pro Pro
620                 625                 630                 635

ttg tcc agg gaa gaa ttc tat aga gag cag cga cga cta aaa gaa gag 2993
Leu Ser Arg Glu Glu Phe Tyr Arg Glu Gln Arg Arg Leu Lys Glu Glu
                640                 645                 650

gaa aag aaa aag tcc aag cta gat gag ttt aca aat gat ttt gct aag 3041
Glu Lys Lys Lys Ser Lys Leu Asp Glu Phe Thr Asn Asp Phe Ala Lys
            655                 660                 665

gaa ttg atg gaa tac aaa aag att caa aag gag cgt agg cgc tca ttt 3089
Glu Leu Met Glu Tyr Lys Lys Ile Gln Lys Glu Arg Arg Arg Ser Phe
        670                 675                 680

tcc agg tct aaa tct ccc tat agt ggt tct tcg tat tca aga agt tca 3137
Ser Arg Ser Lys Ser Pro Tyr Ser Gly Ser Ser Tyr Ser Arg Ser Ser
    685                 690                 695

tat act tat tct aaa tca aga tct ggt tca aca cgt tca cgc tct tat 3185
Tyr Thr Tyr Ser Lys Ser Arg Ser Gly Ser Thr Arg Ser Arg Ser Tyr
700                 705                 710                 715

tct cga tca ttc agc cgc tca cat tct cgt tcc tat tca cgg tca cct 3233
Ser Arg Ser Phe Ser Arg Ser His Ser Arg Ser Tyr Ser Arg Ser Pro
                720                 725                 730

cca tac ccc aga aga ggc aga ggc aag agc cgc aat tac cgt tca cgg 3281
Pro Tyr Pro Arg Arg Gly Arg Gly Lys Ser Arg Asn Tyr Arg Ser Arg
            735                 740                 745

tct aga tct cat gga tat cat cga tct agg tca agg tca ccc cct tac 3329
Ser Arg Ser His Gly Tyr His Arg Ser Arg Ser Arg Ser Pro Pro Tyr
        750                 755                 760

aga cgc tat cat tca cga tca aga tct cct caa gcg ttt agg gga cag 3377
Arg Arg Tyr His Ser Arg Ser Arg Ser Pro Gln Ala Phe Arg Gly Gln
    765                 770                 775

tct cct aat aaa cgt aat gta cct caa ggg gaa aca gaa cgt gaa tat 3425
Ser Pro Asn Lys Arg Asn Val Pro Gln Gly Glu Thr Glu Arg Glu Tyr
780                 785                 790                 795

ttt aat aga tac aga gaa gtt cca cca cca tat gac atg aaa gca tat 3473
Phe Asn Arg Tyr Arg Glu Val Pro Pro Pro Tyr Asp Met Lys Ala Tyr
                800                 805                 810

tat ggg aga agt gtt gac ttt aga gac cca ttt gaa aaa gaa cgc tac 3521
Tyr Gly Arg Ser Val Asp Phe Arg Asp Pro Phe Glu Lys Glu Arg Tyr
            815                 820                 825

cga gaa tgg gag aga aaa tat aga gag tgg tat gaa aaa tat tat aaa 3569
Arg Glu Trp Glu Arg Lys Tyr Arg Glu Trp Tyr Glu Lys Tyr Tyr Lys
        830                 835                 840

ggt tat gct gct gga gca cag cct aga ccc tca gca aat aga gag aac 3617

Gly Tyr Ala Ala Gly Ala Gln Pro Arg Pro Ser Ala Asn Arg Glu Asn
    845                 850                 855

ttt tct cca gag aga ttt ttg cca ctt aac atc agg aat tct ccc ttc 3665
Phe Ser Pro Glu Arg Phe Leu Pro Leu Asn Ile Arg Asn Ser Pro Phe
860                 865                 870                 875

aca aga ggc cgc aga gaa gac tat gtt ggt ggg caa agt cat aga agt 3713
Thr Arg Gly Arg Arg Glu Asp Tyr Val Gly Gly Gln Ser His Arg Ser
                880                 885                 890

cga aac ata ggt agc aac tat cca gaa aag ctt tca gca aga gat ggt 3761
Arg Asn Ile Gly Ser Asn Tyr Pro Glu Lys Leu Ser Ala Arg Asp Gly
            895                 900                 905

cac aat cag aag gat aat aca aag tca aaa gag aag gag agt gaa aac 3809
His Asn Gln Lys Asp Asn Thr Lys Ser Lys Glu Lys Glu Ser Glu Asn
        910                 915                 920

gct cca gga gat ggt aaa gga aat aag cat aag aaa cac aga aaa aga 3857
Ala Pro Gly Asp Gly Lys Gly Asn Lys His Lys Lys His Arg Lys Arg
    925                 930                 935

aga aaa ggg gag gaa agt gag ggt ttt ctg aac cca gag tta tta gag 3905
Arg Lys Gly Glu Glu Ser Glu Gly Phe Leu Asn Pro Glu Leu Leu Glu
940                 945                 950                 955

act tct agg aaa tca aga gaa cct aca ggt gtt gaa gaa aat aaa aca 3953
Thr Ser Arg Lys Ser Arg Glu Pro Thr Gly Val Glu Glu Asn Lys Thr
                960                 965                 970

gac tca ttg ttt gtt ctc cca agt aga gat gat gcc aca cct gtt aga 4001
Asp Ser Leu Phe Val Leu Pro Ser Arg Asp Asp Ala Thr Pro Val Arg
            975                 980                 985

gat gaa cca atg gat gca gaa tca atc act ttt aaa tca gtg tct gaa 4049
Asp Glu Pro Met Asp Ala Glu Ser Ile Thr Phe Lys Ser Val Ser Glu
        990                 995                 1000

aaa gac aag aga gaa agg gat aaa cca aaa gca aag ggt gat aaa 4094
Lys Asp Lys Arg Glu Arg Asp Lys Pro Lys Ala Lys Gly Asp Lys
    1005                1010                1015

acc aaa cgg aag aat gat gga tct gct gtg tcc aaa aaa gaa aat 4139
Thr Lys Arg Lys Asn Asp Gly Ser Ala Val Ser Lys Lys Glu Asn
    1020                1025                1030

att gta aaa cct gct aaa gga ccc caa gaa aaa gta gat gga gaa 4184
Ile Val Lys Pro Ala Lys Gly Pro Gln Glu Lys Val Asp Gly Glu
    1035                1040                1045

cgt gag aga tct cct cga tct gaa cct cca att aaa aaa gcc aaa 4229
Arg Glu Arg Ser Pro Arg Ser Glu Pro Pro Ile Lys Lys Ala Lys
    1050                1055                1060

gag gag act ccg aag act gac aat act aaa tca tca tct tcc tct 4274
Glu Glu Thr Pro Lys Thr Asp Asn Thr Lys Ser Ser Ser Ser Ser
    1065                1070                1075

cag aag gat gaa aaa atc act gga acc ccc aga aaa gct cac tct 4319
Gln Lys Asp Glu Lys Ile Thr Gly Thr Pro Arg Lys Ala His Ser
    1080                1085                1090

aaa tca gca aaa gaa cac caa gaa aca aaa cca gtc aaa gag gaa 4364
Lys Ser Ala Lys Glu His Gln Glu Thr Lys Pro Val Lys Glu Glu
    1095                1100                1105

aaa gtg aag aag gac tat tcc aaa gat gtc aaa tca gaa aag cta 4409

Lys Val Lys Lys Asp Tyr Ser Lys Asp Val Lys Ser Glu Lys Leu
    1110                1115                1120

aca act aag gaa gaa aag gcc aag aag cct aat gag aaa aac aaa      4454
Thr Thr Lys Glu Glu Lys Ala Lys Lys Pro Asn Glu Lys Asn Lys
    1125                1130                1135

cca ctt gat aat aag gga gaa aaa aga aaa aga aaa act gaa gaa      4499
Pro Leu Asp Asn Lys Gly Glu Lys Arg Lys Arg Lys Thr Glu Glu
    1140                1145                1150

aaa ggc gta gat aaa gat ttt gag tct tct tca atg aaa atc tcg      4544
Lys Gly Val Asp Lys Asp Phe Glu Ser Ser Ser Met Lys Ile Ser
    1155                1160                1165

aaa cta gaa gtg act gaa ata gtg aaa cca tca cca aag cgc aaa      4589
Lys Leu Glu Val Thr Glu Ile Val Lys Pro Ser Pro Lys Arg Lys
    1170                1175                1180

atg gaa cct gat act gaa aaa atg gat agg acc cct gaa aag gac      4634
Met Glu Pro Asp Thr Glu Lys Met Asp Arg Thr Pro Glu Lys Asp
    1185                1190                1195

aaa att tct tta agt gcg cca gcc aaa aaa atc aaa ctc aac aga      4679
Lys Ile Ser Leu Ser Ala Pro Ala Lys Lys Ile Lys Leu Asn Arg
    1200                1205                1210

gaa act ggg aag aaa att gga agt aca gaa aat ata tca aac aca      4724
Glu Thr Gly Lys Lys Ile Gly Ser Thr Glu Asn Ile Ser Asn Thr
    1215                1220                1225

aaa gaa ccc tct gaa aaa ttg gag tca aca tct agc aaa gtt aaa      4769
Lys Glu Pro Ser Glu Lys Leu Glu Ser Thr Ser Ser Lys Val Lys
    1230                1235                1240

caa gaa aaa gtc aaa gga aag gtc aga cga aaa gtg act gga act      4814
Gln Glu Lys Val Lys Gly Lys Val Arg Arg Lys Val Thr Gly Thr
    1245                1250                1255

gaa gga tcc agc tca act ctg gtg gat tac acc agt acg agc tca      4859
Glu Gly Ser Ser Ser Thr Leu Val Asp Tyr Thr Ser Thr Ser Ser
    1260                1265                1270

act gga ggc agt cct gtg cgg aaa tct gaa gaa aaa aca gat aca      4904
Thr Gly Gly Ser Pro Val Arg Lys Ser Glu Glu Lys Thr Asp Thr
    1275                1280                1285

aag cga act gtg att aaa acg atg gaa gaa tat aat aat gac aat      4949
Lys Arg Thr Val Ile Lys Thr Met Glu Glu Tyr Asn Asn Asp Asn
    1290                1295                1300

acc gcg cca gct gaa gat gtt atc att atg att cag gtt cct caa      4994
Thr Ala Pro Ala Glu Asp Val Ile Ile Met Ile Gln Val Pro Gln
    1305                1310                1315

tcc aaa tgg gat aaa gat gac ttt gaa tct gaa gaa gaa gat gtt      5039
Ser Lys Trp Asp Lys Asp Asp Phe Glu Ser Glu Glu Glu Asp Val
    1320                1325                1330

aaa tcc aca cag cct ata tca agt gta gga aaa cct gct agt gtt      5084
Lys Ser Thr Gln Pro Ile Ser Ser Val Gly Lys Pro Ala Ser Val
    1335                1340                1345

ata aaa aat gtt agt aca aag cca tca aat ata gtc aag tat cct      5129
Ile Lys Asn Val Ser Thr Lys Pro Ser Asn Ile Val Lys Tyr Pro
    1350                1355                1360

gag aaa gaa agt gag cca tcc gag aaa att cag aaa ttc acc aag      5174

Glu Lys Glu Ser Glu Pro Ser Glu Lys Ile Gln Lys Phe Thr Lys
    1365                1370                1375

gac gtg agc cat gaa atc ata caa cat gag gtt aaa agt tca aaa      5219
Asp Val Ser His Glu Ile Ile Gln His Glu Val Lys Ser Ser Lys
    1380                1385                1390

aac tct gca tct agt gaa aaa ggg aaa acc aaa gat cga gat tat      5264
Asn Ser Ala Ser Ser Glu Lys Gly Lys Thr Lys Asp Arg Asp Tyr
    1395                1400                1405

tca gtg ttg gaa aag gag aac cct gaa aag agg aag aac agc act      5309
Ser Val Leu Glu Lys Glu Asn Pro Glu Lys Arg Lys Asn Ser Thr
    1410                1415                1420

cag cca gag aaa gag agt aat ttg gac cgt ctg aat gaa caa gga      5354
Gln Pro Glu Lys Glu Ser Asn Leu Asp Arg Leu Asn Glu Gln Gly
    1425                1430                1435

aat ttt aaa agt ctg tct caa tct tcc aaa gag gct aga acg tca      5399
Asn Phe Lys Ser Leu Ser Gln Ser Ser Lys Glu Ala Arg Thr Ser
    1440                1445                1450

gat aaa cat gat tcc act cgt gct tcc tca aat aaa gac ttc act      5444
Asp Lys His Asp Ser Thr Arg Ala Ser Ser Asn Lys Asp Phe Thr
    1455                1460                1465

ccc aat aga gac aaa aaa act gac tat gac acc aga gag tat tca      5489
Pro Asn Arg Asp Lys Lys Thr Asp Tyr Asp Thr Arg Glu Tyr Ser
    1470                1475                1480

agt tcc aaa cgt aga gat gaa aag aat gaa tta aca aga cga aaa      5534
Ser Ser Lys Arg Arg Asp Glu Lys Asn Glu Leu Thr Arg Arg Lys
    1485                1490                1495

gac tct cct tct cgg aat aaa gat tct gca tct gga cag aaa aat      5579
Asp Ser Pro Ser Arg Asn Lys Asp Ser Ala Ser Gly Gln Lys Asn
    1500                1505                1510

aaa cca agg gaa gag aga gat ttg cct aaa aaa gga aca gga gat      5624
Lys Pro Arg Glu Glu Arg Asp Leu Pro Lys Lys Gly Thr Gly Asp
    1515                1520                1525

tcc aaa aaa agt aat tct agt ccc tca aga gac aga aaa cct cat      5669
Ser Lys Lys Ser Asn Ser Ser Pro Ser Arg Asp Arg Lys Pro His
    1530                1535                1540

gat cac aaa gcc act tat gat act aaa cgg cca aat gaa gag aca      5714
Asp His Lys Ala Thr Tyr Asp Thr Lys Arg Pro Asn Glu Glu Thr
    1545                1550                1555

aaa tct gta gat aaa aat cct tgt aag gat cgt gag aag cat gta      5759
Lys Ser Val Asp Lys Asn Pro Cys Lys Asp Arg Glu Lys His Val
    1560                1565                1570

tta gaa gca agg aac aat aaa gag tca agt ggc aat aaa cta ctt      5804
Leu Glu Ala Arg Asn Asn Lys Glu Ser Ser Gly Asn Lys Leu Leu
    1575                1580                1585

tat ata ctt aac cca cca gag aca cag gtt gaa aaa gag caa att      5849
Tyr Ile Leu Asn Pro Pro Glu Thr Gln Val Glu Lys Glu Gln Ile
    1590                1595                1600

act ggg caa att gac aag agt act gtc aag cct aaa ccc cag tta      5894
Thr Gly Gln Ile Asp Lys Ser Thr Val Lys Pro Lys Pro Gln Leu
    1605                1610                1615

agt cat tcc tct aga ctt tcc tct gac tta act aga gaa act gat      5939

Ser His Ser Ser Arg Leu Ser Ser Asp Leu Thr Arg Glu Thr Asp
    1620                1625                1630

gaa gct gct ttt gaa cca gac tat aat gaa agt gac agt gaa agt 5984
Glu Ala Ala Phe Glu Pro Asp Tyr Asn Glu Ser Asp Ser Glu Ser
    1635                1640                1645

aat gtt tct gta aaa gaa gag gaa tct tca gga aac att tct aag 6029
Asn Val Ser Val Lys Glu Glu Glu Ser Ser Gly Asn Ile Ser Lys
    1650                1655                1660

gac ctg aaa gat aaa ata gtg gag aaa gca aaa gag agc ctg gac 6074
Asp Leu Lys Asp Lys Ile Val Glu Lys Ala Lys Glu Ser Leu Asp
    1665                1670                1675

aca gca gca gtt gtc cag gtg ggc ata agc agg aat cag agc cac 6119
Thr Ala Ala Val Val Gln Val Gly Ile Ser Arg Asn Gln Ser His
    1680                1685                1690

agc agc ccc agc gtc agc ccc agc aga agc cac agt cct tct gga 6164
Ser Ser Pro Ser Val Ser Pro Ser Arg Ser His Ser Pro Ser Gly
    1695                1700                1705

agc cag acc cga agc cac agt agc agt gcc agc tca gca gaa agt 6209
Ser Gln Thr Arg Ser His Ser Ser Ser Ala Ser Ser Ala Glu Ser
    1710                1715                1720

cag gac agc aag aag aag aag aaa aag aag gaa aag aaa aaa cac 6254
Gln Asp Ser Lys Lys Lys Lys Lys Lys Lys Glu Lys Lys Lys His
    1725                1730                1735

aag aaa cat aaa aag cat aag aag cat aag aaa cat gca ggc act 6299
Lys Lys His Lys Lys His Lys Lys His Lys Lys His Ala Gly Thr
    1740                1745                1750

gaa gtg gaa ttg gaa aaa agc caa aaa cac aaa cac aag aaa aag 6344
Glu Val Glu Leu Glu Lys Ser Gln Lys His Lys His Lys Lys Lys
    1755                1760                1765

aag tca aag aag aac aaa gat aaa gag aag gag aag gag aaa gat 6389
Lys Ser Lys Lys Asn Lys Asp Lys Glu Lys Glu Lys Glu Lys Asp
    1770                1775                1780

gac caa aaa gtg aaa tct gtc act gtg taa aaagacagat tttttaaatt 6439
Asp Gln Lys Val Lys Ser Val Thr Val
    1785                1790

gacttaatta ctaagtcatc tgtattaaat tttgttataa tgtaaagaga ttcaagcctt 6499

gtaaataatg acatggaaga ccctgtgctg cacttaaaat attgctgctt gattatttga 6559

tttttacatc agagctttat aacacgaact tttgtacaga attgtgagtt gtgaccatgt 6619

aacatgagag gttttgctag ggcctattat ttttaaccac cattaattag ttggggtgga 6679

gtttactgta atgtgaaatt ttcacatttg aattttttaa ttgcctggca aaagctgata 6739

taagttctaa aatatcagca gaatgatttg ctgaattcat tacaaccctg ttatgtcact 6799

ttttgattac aataaaagtt ttcagtaaac ttttcaaaaa aaaaaaaaaa aaaa 6853

<210> 10
<211> 1792
<212> PRT
<213> human

<400> 10

Met Ser Cys Val His Tyr Lys Phe Ser Ser Lys Leu Asn Tyr Asp Thr
1               5                   10                  15

Val Thr Phe Asp Gly Leu His Ile Ser Leu Cys Asp Leu Lys Lys Gln
            20                  25                  30

Ile Met Gly Arg Glu Lys Leu Lys Ala Ala Asp Cys Asp Leu Gln Ile
        35                  40                  45

Thr Asn Ala Gln Thr Lys Glu Glu Tyr Thr Asp Asp Asn Ala Leu Ile
    50                  55                  60

Pro Lys Asn Ser Ser Val Ile Val Arg Arg Ile Pro Ile Gly Gly Val
65                  70                  75                  80

Lys Ser Thr Ser Lys Thr Tyr Val Ile Ser Arg Thr Glu Pro Ala Met
                85                  90                  95

Ala Thr Thr Lys Ala Ile Asp Asp Ser Ser Ala Ser Ile Ser Leu Ala
            100                 105                 110

Gln Leu Thr Lys Thr Ala Asn Leu Ala Glu Ala Asn Ala Ser Glu Glu
        115                 120                 125

Asp Lys Ile Lys Ala Met Met Ser Gln Ser Gly His Glu Tyr Asp Pro
    130                 135                 140

Ile Asn Tyr Met Lys Lys Pro Leu Gly Pro Pro Pro Pro Ser Tyr Thr
145                 150                 155                 160

Cys Phe Arg Cys Gly Lys Pro Gly His Tyr Ile Lys Asn Cys Pro Thr
                165                 170                 175

Asn Gly Asp Lys Asn Phe Glu Ser Gly Pro Arg Ile Lys Lys Ser Thr
            180                 185                 190

Gly Ile Pro Arg Ser Phe Met Met Glu Val Lys Asp Pro Asn Met Lys
        195                 200                 205

Gly Ala Met Leu Thr Asn Thr Gly Lys Tyr Ala Ile Pro Thr Ile Asp
    210                 215                 220

Ala Glu Ala Tyr Ala Ile Gly Lys Lys Glu Lys Pro Pro Phe Leu Pro
225                 230                 235                 240

Glu Glu Pro Ser Ser Ser Ser Glu Glu Asp Asp Pro Ile Pro Asp Glu
                245                 250                 255

Leu Leu Cys Leu Ile Cys Lys Asp Ile Met Thr Asp Ala Val Val Ile
            260                 265                 270

Pro Cys Cys Gly Asn Ser Tyr Cys Asp Glu Cys Ile Arg Thr Ala Leu
        275                 280                 285

Leu Glu Ser Asp Glu His Thr Cys Pro Thr Cys His Gln Asn Asp Val
    290                 295                 300

Ser Pro Asp Ala Leu Ile Ala Asn Lys Phe Leu Arg Gln Ala Val Asn
305                 310                 315                 320

Asn Phe Lys Asn Glu Thr Gly Tyr Thr Lys Arg Leu Arg Lys Gln Leu
                325                 330                 335

Pro Pro Pro Pro Pro Pro Ile Pro Pro Pro Arg Pro Leu Ile Gln Arg
            340                 345                 350

Asn Leu Gln Pro Leu Met Arg Ser Pro Ile Ser Arg Gln Gln Asp Pro
        355                 360                 365

Leu Met Ile Pro Val Thr Ser Ser Ser Thr His Pro Ala Pro Ser Ile
    370                 375                 380

Ser Ser Leu Thr Ser Asn Gln Ser Ser Leu Ala Pro Pro Val Ser Gly
385                 390                 395                 400

Asn Pro Ser Ser Ala Pro Ala Pro Val Pro Asp Ile Thr Ala Thr Val
                405                 410                 415

Ser Ile Ser Val His Ser Glu Lys Ser Asp Gly Pro Phe Arg Asp Ser
            420                 425                 430

Asp Asn Lys Ile Leu Pro Ala Ala Ala Leu Ala Ser Glu His Ser Lys
        435                 440                 445

Gly Thr Ser Ser Ile Ala Ile Thr Ala Leu Met Glu Glu Lys Gly Tyr
    450                 455                 460

Gln Val Pro Val Leu Gly Thr Pro Ser Leu Leu Gly Gln Ser Leu Leu
465                 470                 475                 480

His Gly Gln Leu Ile Pro Thr Thr Gly Pro Val Arg Ile Asn Thr Ala
                485                 490                 495

Arg Pro Gly Gly Gly Arg Pro Gly Trp Glu His Ser Asn Lys Leu Gly
            500                 505                 510

Tyr Leu Val Ser Pro Pro Gln Gln Ile Arg Arg Gly Glu Arg Ser Cys
        515                 520                 525

Tyr Arg Ser Ile Asn Arg Gly Arg His His Ser Glu Arg Ser Gln Arg
    530                 535                 540

Thr Gln Gly Pro Ser Leu Pro Ala Thr Pro Val Phe Val Pro Val Pro
545                 550                 555                 560

Pro Pro Pro Leu Tyr Pro Pro Pro Pro His Thr Leu Pro Leu Pro Pro
                565                 570                 575

Gly Val Pro Pro Pro Gln Phe Ser Pro Gln Phe Pro Pro Gly Gln Pro
            580                 585                 590

Pro Pro Ala Gly Tyr Ser Val Pro Pro Pro Gly Phe Pro Pro Ala Pro
        595                 600                 605

Ala Asn Leu Ser Thr Pro Trp Val Ser Ser Gly Val Gln Thr Ala His
    610                 615                 620

Ser Asn Thr Ile Pro Thr Thr Gln Ala Pro Pro Leu Ser Arg Glu Glu
625                 630                 635                 640

Phe Tyr Arg Glu Gln Arg Arg Leu Lys Glu Glu Glu Lys Lys Lys Ser
                645                 650                 655

Lys Leu Asp Glu Phe Thr Asn Asp Phe Ala Lys Glu Leu Met Glu Tyr
            660                 665                 670

Lys Lys Ile Gln Lys Glu Arg Arg Arg Ser Phe Ser Arg Ser Lys Ser
        675                 680                 685

Pro Tyr Ser Gly Ser Ser Tyr Ser Arg Ser Ser Tyr Thr Tyr Ser Lys
    690                 695                 700

Ser Arg Ser Gly Ser Thr Arg Ser Arg Ser Tyr Ser Arg Ser Phe Ser
705                 710                 715                 720

Arg Ser His Ser Arg Ser Tyr Ser Arg Ser Pro Pro Tyr Pro Arg Arg
                725                 730                 735

Gly Arg Gly Lys Ser Arg Asn Tyr Arg Ser Arg Ser Arg Ser His Gly
            740                 745                 750

Tyr His Arg Ser Arg Ser Arg Ser Pro Pro Tyr Arg Arg Tyr His Ser
        755                 760                 765

Arg Ser Arg Ser Pro Gln Ala Phe Arg Gly Gln Ser Pro Asn Lys Arg
    770                 775                 780

Asn Val Pro Gln Gly Glu Thr Glu Arg Glu Tyr Phe Asn Arg Tyr Arg
785                 790                 795                 800

Glu Val Pro Pro Pro Tyr Asp Met Lys Ala Tyr Tyr Gly Arg Ser Val
                805                 810                 815

Asp Phe Arg Asp Pro Phe Glu Lys Glu Arg Tyr Arg Glu Trp Glu Arg
            820                 825                 830

Lys Tyr Arg Glu Trp Tyr Glu Lys Tyr Tyr Lys Gly Tyr Ala Ala Gly
        835                 840                 845

Ala Gln Pro Arg Pro Ser Ala Asn Arg Glu Asn Phe Ser Pro Glu Arg
    850                 855                 860

Phe Leu Pro Leu Asn Ile Arg Asn Ser Pro Phe Thr Arg Gly Arg Arg
865                 870                 875                 880

Glu Asp Tyr Val Gly Gly Gln Ser His Arg Ser Arg Asn Ile Gly Ser
                885                 890                 895

Asn Tyr Pro Glu Lys Leu Ser Ala Arg Asp Gly His Asn Gln Lys Asp
            900                 905                 910

Asn Thr Lys Ser Lys Glu Lys Glu Ser Glu Asn Ala Pro Gly Asp Gly
        915                 920                 925

Lys Gly Asn Lys His Lys Lys His Arg Lys Arg Arg Lys Gly Glu Glu
    930                 935                 940

Ser Glu Gly Phe Leu Asn Pro Glu Leu Leu Glu Thr Ser Arg Lys Ser
945                 950                 955                 960

Arg Glu Pro Thr Gly Val Glu Glu Asn Lys Thr Asp Ser Leu Phe Val
                965                 970                 975

Leu Pro Ser Arg Asp Asp Ala Thr Pro Val Arg Asp Glu Pro Met Asp
            980                 985                 990

Ala Glu Ser Ile Thr Phe Lys Ser  Val Ser Glu Lys Asp  Lys Arg Glu
        995                 1000                1005

Arg Asp  Lys Pro Lys Ala Lys  Gly Asp Lys Thr Lys  Arg Lys Asn
    1010                1015                1020

Asp Gly  Ser Ala Val Ser Lys  Lys Glu Asn Ile Val  Lys Pro Ala
    1025                1030                1035

Lys Gly  Pro Gln Glu Lys Val  Asp Gly Glu Arg Glu  Arg Ser Pro
    1040                1045                1050

Arg Ser  Glu Pro Pro Ile Lys  Lys Ala Lys Glu Glu  Thr Pro Lys
    1055                1060                1065

Thr Asp  Asn Thr Lys Ser Ser  Ser Ser Ser Gln Lys  Asp Glu Lys
    1070                1075                1080

Ile Thr Gly Thr Pro Arg Lys Ala His Ser Lys Ser Ala Lys Glu
    1085                1090                1095

His Gln Glu Thr Lys Pro Val Lys Glu Glu Lys Val Lys Lys Asp
    1100                1105                1110

Tyr Ser Lys Asp Val Lys Ser Glu Lys Leu Thr Thr Lys Glu Glu
    1115                1120                1125

Lys Ala Lys Lys Pro Asn Glu Lys Asn Lys Pro Leu Asp Asn Lys
    1130                1135                1140

Gly Glu Lys Arg Lys Arg Lys Thr Glu Glu Lys Gly Val Asp Lys
    1145                1150                1155

Asp Phe Glu Ser Ser Ser Met Lys Ile Ser Lys Leu Glu Val Thr
    1160                1165                1170

Glu Ile Val Lys Pro Ser Pro Lys Arg Lys Met Glu Pro Asp Thr
    1175                1180                1185

Glu Lys Met Asp Arg Thr Pro Glu Lys Asp Lys Ile Ser Leu Ser
    1190                1195                1200

Ala Pro Ala Lys Lys Ile Lys Leu Asn Arg Glu Thr Gly Lys Lys
    1205                1210                1215

Ile Gly Ser Thr Glu Asn Ile Ser Asn Thr Lys Glu Pro Ser Glu
    1220                1225                1230

Lys Leu Glu Ser Thr Ser Ser Lys Val Lys Gln Glu Lys Val Lys
    1235                1240                1245

Gly Lys Val Arg Arg Lys Val Thr Gly Thr Glu Gly Ser Ser Ser
    1250                1255                1260

Thr Leu Val Asp Tyr Thr Ser Thr Ser Ser Thr Gly Gly Ser Pro
    1265                1270                1275

Val Arg Lys Ser Glu Glu Lys Thr Asp Thr Lys Arg Thr Val Ile
    1280                1285                1290

Lys Thr Met Glu Glu Tyr Asn Asn Asp Asn Thr Ala Pro Ala Glu
    1295                1300                1305

Asp Val Ile Ile Met Ile Gln Val Pro Gln Ser Lys Trp Asp Lys
    1310                1315                1320

Asp Asp Phe Glu Ser Glu Glu Glu Asp Val Lys Ser Thr Gln Pro
    1325                1330                1335

Ile Ser Ser Val Gly Lys Pro Ala Ser Val Ile Lys Asn Val Ser
    1340                1345                1350

Thr Lys Pro Ser Asn Ile Val Lys Tyr Pro Glu Lys Glu Ser Glu
    1355                1360                1365

Pro Ser Glu Lys Ile Gln Lys Phe Thr Lys Asp Val Ser His Glu
    1370                1375                1380

Ile Ile Gln His Glu Val Lys Ser Ser Lys Asn Ser Ala Ser Ser
    1385                1390                1395

Glu Lys Gly Lys Thr Lys Asp Arg Asp Tyr Ser Val Leu Glu Lys
    1400                1405                1410

Glu Asn Pro Glu Lys Arg Lys Asn Ser Thr Gln Pro Glu Lys Glu
    1415                1420                1425

Ser Asn Leu Asp Arg Leu Asn Glu Gln Gly Asn Phe Lys Ser Leu
    1430                1435                1440

Ser Gln Ser Ser Lys Glu Ala Arg Thr Ser Asp Lys His Asp Ser
    1445                1450                1455

Thr Arg Ala Ser Ser Asn Lys Asp Phe Thr Pro Asn Arg Asp Lys
    1460                1465                1470

Lys Thr Asp Tyr Asp Thr Arg Glu Tyr Ser Ser Ser Lys Arg Arg
    1475                1480                1485

Asp Glu Lys Asn Glu Leu Thr Arg Arg Lys Asp Ser Pro Ser Arg
    1490                1495                1500

Asn Lys Asp Ser Ala Ser Gly Gln Lys Asn Lys Pro Arg Glu Glu
    1505                1510                1515

Arg Asp Leu Pro Lys Lys Gly Thr Gly Asp Ser Lys Lys Ser Asn
    1520                1525                1530

Ser Ser Pro Ser Arg Asp Arg Lys Pro His Asp His Lys Ala Thr
    1535                1540                1545

Tyr Asp Thr Lys Arg Pro Asn Glu Glu Thr Lys Ser Val Asp Lys
    1550                1555                1560

Asn Pro Cys Lys Asp Arg Glu Lys His Val Leu Glu Ala Arg Asn
    1565                1570                1575

Asn Lys Glu Ser Ser Gly Asn Lys Leu Leu Tyr Ile Leu Asn Pro
    1580                1585                1590

Pro Glu Thr Gln Val Glu Lys Glu Gln Ile Thr Gly Gln Ile Asp
    1595                1600                1605

Lys Ser Thr Val Lys Pro Lys Pro Gln Leu Ser His Ser Ser Arg
    1610                1615                1620

Leu Ser Ser Asp Leu Thr Arg Glu Thr Asp Glu Ala Ala Phe Glu
    1625                1630                1635

Pro Asp Tyr Asn Glu Ser Asp Ser Glu Ser Asn Val Ser Val Lys
    1640                1645                1650

Glu Glu Glu Ser Ser Gly Asn Ile Ser Lys Asp Leu Lys Asp Lys
    1655                1660                1665

Ile Val Glu Lys Ala Lys Glu Ser Leu Asp Thr Ala Ala Val Val
    1670                1675                1680

Gln Val Gly Ile Ser Arg Asn Gln Ser His Ser Ser Pro Ser Val
    1685                1690                1695

Ser Pro Ser Arg Ser His Ser Pro Ser Gly Ser Gln Thr Arg Ser
    1700                1705                1710

His Ser Ser Ser Ala Ser Ser Ala Glu Ser Gln Asp Ser Lys Lys
    1715                1720                1725

Lys Lys Lys Lys Lys Glu Lys Lys Lys His Lys Lys His Lys Lys
    1730                1735                1740

His Lys Lys His Lys Lys His Ala Gly Thr Glu Val Glu Leu Glu
    1745                1750                1755

Lys Ser Gln Lys His Lys His Lys Lys Lys Lys Ser Lys Lys Asn
    1760                1765                1770

Lys Asp Lys Glu Lys Glu Lys Glu Lys Asp Asp Gln Lys Val Lys
    1775                1780                1785

Ser Val Thr Val
    1790

<210> 11
<211> 6751
<212> DNA
<213> human

<220>
<221> CDS
<222> (1041)..(6317)

<400> 11

gccaggccgc gtccgccata gtacctggct tggaggtgtc gccgccgctc ggtgagagcc 60

cccgagcggc agggggccaa cacaaaaagg gagccggaga agccctagcc gctgcccagc 120

agcttgcggg cgtgttctcg cggttccggg cctcaaggcg acggaaacga aaggcgagcg 180

aagcgcggag gatccggcga gaagaagcgt cagggagcct cggcggtgtc cccggggtcc 240

gccgaagcca cccggccgcc ggctggggcc cggggtggtg aggaagtgct ccgaggcctc 300

gccgaggcct agcgccggct ttgtgtccga ggcggcggcg gcggcggggg gaggcggagc 360

cgggggcggc ctgcgggaag gcctctcctc cgccgaccgc gcgttttcgg cctaggccgt 420

ggggccgctc gtggcctccg gggagcaggc gccaggggtt tgtgtgcggt gggggcctgg 480

gcctgggcct ggggaagctg acgccggtcg tccggaagcc aggaggaggc gtgaggccgc 540

tcgtggactc cgggcctagg ccctctcccc tcaaccttct cccggggcct gggtcacccc 600

aatccacgga gagagagacc cgccgggagg tgcggccgcg ctatggaccc ctgaccccgt 660

ggggtcgctc ggactcttaa cgtgtggact gaccgctact gactgcaccg ccaatccccc 720

cgtctctgcc ggccccttag catgagcgag ggggacccag ccgggtgaca ttgtgcccgt 780

tggcggattc tcgatttccc ctcttccccg tcctcgtcct cctcctcccc catgaagtga 840

ttctgagtat cggggggtct ctggattatt gttctgacga acccctgctt gtggttgggg 900

ggtatttaat ctgaggcctt agggtccttc ggtgtctttg agtgttttgt gtgtacatat 960

tttgctctta aagtttataa atatacgtat attgagagtg tccacgtctc ctcgctgaac 1020

cttaggaatc ccttggcacc atg tcc tgt gtg cat tat aaa ttt tcc tct aaa 1073
Met Ser Cys Val His Tyr Lys Phe Ser Ser Lys
1               5                   10

ctc aac tat gat acc gtc acc ttt gat ggg ctc cac atc tcc ctc tgc 1121
Leu Asn Tyr Asp Thr Val Thr Phe Asp Gly Leu His Ile Ser Leu Cys
            15                  20                  25

gac tta aag aag cag att atg ggg aga gag aag ctg aaa gct gcc gac 1169
Asp Leu Lys Lys Gln Ile Met Gly Arg Glu Lys Leu Lys Ala Ala Asp
        30                  35                  40

tgc gac ctg cag atc acc aat gcg cag acg aaa gaa gaa tat act gat 1217
Cys Asp Leu Gln Ile Thr Asn Ala Gln Thr Lys Glu Glu Tyr Thr Asp
    45                  50                  55

gat aat gct ctg att cct aag aat tct tct gta att gtt aga aga att 1265
Asp Asn Ala Leu Ile Pro Lys Asn Ser Ser Val Ile Val Arg Arg Ile
60                  65                  70                  75

cct att gga ggt gtt aaa tct aca agc aag aca tat gtt ata agt cga 1313
Pro Ile Gly Gly Val Lys Ser Thr Ser Lys Thr Tyr Val Ile Ser Arg
                80                  85                  90

act gaa cca gcg atg gca act aca aaa gca att gat gac tct tcc gcg 1361
Thr Glu Pro Ala Met Ala Thr Thr Lys Ala Ile Asp Asp Ser Ser Ala
            95                  100                 105

tct att tct ctg gcc cag ctt aca aag act gcc aat ctg gct gaa gcc 1409
Ser Ile Ser Leu Ala Gln Leu Thr Lys Thr Ala Asn Leu Ala Glu Ala
        110                 115                 120

aat gct tct gaa gaa gat aaa att aaa gca atg atg tcg caa tct ggc 1457
Asn Ala Ser Glu Glu Asp Lys Ile Lys Ala Met Met Ser Gln Ser Gly

125                 130                 135

cat gaa tac gac cca atc aat tac atg aag aaa cct cta ggt cca cca     1505
His Glu Tyr Asp Pro Ile Asn Tyr Met Lys Lys Pro Leu Gly Pro Pro
140                 145                 150                 155

cct cca tct tac acg tgt ttc cgt tgt ggt aaa cct gga cat tat att     1553
Pro Pro Ser Tyr Thr Cys Phe Arg Cys Gly Lys Pro Gly His Tyr Ile
                160                 165                 170

aag aat tgc cca aca aat ggg gat aaa aac ttt gaa tct ggt cct agg     1601
Lys Asn Cys Pro Thr Asn Gly Asp Lys Asn Phe Glu Ser Gly Pro Arg
            175                 180                 185

att aaa aag agc act gga att ccc aga agt ttc atg atg gaa gtg aaa     1649
Ile Lys Lys Ser Thr Gly Ile Pro Arg Ser Phe Met Met Glu Val Lys
        190                 195                 200

gat cct aat atg aaa ggt gca atg ctt acc aac act gga aaa tat gca     1697
Asp Pro Asn Met Lys Gly Ala Met Leu Thr Asn Thr Gly Lys Tyr Ala
    205                 210                 215

ata cca act ata gat gca gaa gca tat gca att ggg aag aaa gag aaa     1745
Ile Pro Thr Ile Asp Ala Glu Ala Tyr Ala Ile Gly Lys Lys Glu Lys
220                 225                 230                 235

cct ccc ttc tta cca gag gag cca tct tct tcc tca gaa gaa gat gat     1793
Pro Pro Phe Leu Pro Glu Glu Pro Ser Ser Ser Ser Glu Glu Asp Asp
                240                 245                 250

cct atc cca gat gaa ttg ttg tgt ctc atc tgc aag gat att atg act     1841
Pro Ile Pro Asp Glu Leu Leu Cys Leu Ile Cys Lys Asp Ile Met Thr
            255                 260                 265

gat gct gtt gtg att ccc tgc tgt gga aac agt tac tgt gat gaa tgt     1889
Asp Ala Val Val Ile Pro Cys Cys Gly Asn Ser Tyr Cys Asp Glu Cys
        270                 275                 280

ata aga aca gca ctc ctg gaa tca gat gag cac aca tgt ccg acg tgt     1937
Ile Arg Thr Ala Leu Leu Glu Ser Asp Glu His Thr Cys Pro Thr Cys
    285                 290                 295

cat caa aat gat gtt tct cct gat gct tta att gcc aat aaa ttt tta     1985
His Gln Asn Asp Val Ser Pro Asp Ala Leu Ile Ala Asn Lys Phe Leu
300                 305                 310                 315

cga cag gct gta aat aac ttc aaa aat gaa act ggc tat aca aaa aga     2033
Arg Gln Ala Val Asn Asn Phe Lys Asn Glu Thr Gly Tyr Thr Lys Arg
                320                 325                 330

cta cga aaa cag tta cct cct cca cca ccc cca ata cca cct ccg aga     2081
Leu Arg Lys Gln Leu Pro Pro Pro Pro Pro Pro Ile Pro Pro Pro Arg
            335                 340                 345

cca ctg att cag agg aac cta caa cct ctg atg aga tct ccg ata tca     2129
Pro Leu Ile Gln Arg Asn Leu Gln Pro Leu Met Arg Ser Pro Ile Ser
        350                 355                 360

aga caa caa gat cct ctt atg att cca gtg aca tct tca tca act cac     2177
Arg Gln Gln Asp Pro Leu Met Ile Pro Val Thr Ser Ser Ser Thr His
    365                 370                 375

cca gct ccg tct ata tct tca tta act tct aat cag tct tcc ttg gcc     2225
Pro Ala Pro Ser Ile Ser Ser Leu Thr Ser Asn Gln Ser Ser Leu Ala
380                 385                 390                 395

cct cct gtg tct gga aat ccg tct tct gct cca gct cct gta cct gat     2273
Pro Pro Val Ser Gly Asn Pro Ser Ser Ala Pro Ala Pro Val Pro Asp

                400                 405                 410

ata act gca aca gta tcc ata tca gtt cat tca gaa aaa tca gat gga 2321
Ile Thr Ala Thr Val Ser Ile Ser Val His Ser Glu Lys Ser Asp Gly
            415                 420                 425

cct ttt cgg gat tct gat aat aaa ata ttg cca gct gca gct ctt gca 2369
Pro Phe Arg Asp Ser Asp Asn Lys Ile Leu Pro Ala Ala Ala Leu Ala
        430                 435                 440

tca gag cac tca aag gga acc tcc tca att gca att acc gct ctt atg 2417
Ser Glu His Ser Lys Gly Thr Ser Ser Ile Ala Ile Thr Ala Leu Met
    445                 450                 455

gaa gag aag ggt tac cag gtg cct gtt ctt gga acc cca tct ttg ctt 2465
Glu Glu Lys Gly Tyr Gln Val Pro Val Leu Gly Thr Pro Ser Leu Leu
460                 465                 470                 475

gga cag tca tta ttg cat gga cag ttg atc ccc aca act ggt cca gta 2513
Gly Gln Ser Leu Leu His Gly Gln Leu Ile Pro Thr Thr Gly Pro Val
                480                 485                 490

aga ata aat act gct cgt cca ggt ggt ggt cga cca ggc tgg gaa cat 2561
Arg Ile Asn Thr Ala Arg Pro Gly Gly Gly Arg Pro Gly Trp Glu His
            495                 500                 505

tcc aac aaa ctt ggc tat ctg gtt tct cca cca caa caa att aga aga 2609
Ser Asn Lys Leu Gly Tyr Leu Val Ser Pro Pro Gln Gln Ile Arg Arg
        510                 515                 520

ggg gag agg agc tgc tac aga agt ata aac cgt ggg cga cac cac agc 2657
Gly Glu Arg Ser Cys Tyr Arg Ser Ile Asn Arg Gly Arg His His Ser
    525                 530                 535

gaa aga tca cag agg act caa ggc ccg tca cta cca gca act cca gtc 2705
Glu Arg Ser Gln Arg Thr Gln Gly Pro Ser Leu Pro Ala Thr Pro Val
540                 545                 550                 555

ttt gta cct gtt cca cca cct cct ttg tat ccg cct cct ccc cat aca 2753
Phe Val Pro Val Pro Pro Pro Pro Leu Tyr Pro Pro Pro Pro His Thr
                560                 565                 570

ctt cct ctc cct ccg ggt gtt cct cct cca cag ttt tct cct cag ttt 2801
Leu Pro Leu Pro Pro Gly Val Pro Pro Pro Gln Phe Ser Pro Gln Phe
            575                 580                 585

cct cct ggc cag cca cca ccc gct ggg tat agt gtc cct cct cca ggg 2849
Pro Pro Gly Gln Pro Pro Pro Ala Gly Tyr Ser Val Pro Pro Pro Gly
        590                 595                 600

ttt cct cca gct cct gcc aat tta tca aca cct tgg gta tca tca gga 2897
Phe Pro Pro Ala Pro Ala Asn Leu Ser Thr Pro Trp Val Ser Ser Gly
    605                 610                 615

gtg cag aca gct cat tca aat acc atc cca aca aca caa gca cca cct 2945
Val Gln Thr Ala His Ser Asn Thr Ile Pro Thr Thr Gln Ala Pro Pro
620                 625                 630                 635

ttg tcc agg gaa gaa ttc tat aga gag cag cga cga cta aaa gaa gag 2993
Leu Ser Arg Glu Glu Phe Tyr Arg Glu Gln Arg Arg Leu Lys Glu Glu
                640                 645                 650

tct aaa tct ccc tat agt ggt tct tcg tat tca aga agt tca tat act 3041
Ser Lys Ser Pro Tyr Ser Gly Ser Ser Tyr Ser Arg Ser Ser Tyr Thr
            655                 660                 665

tat tct aaa tca aga tct ggt tca aca cgt tca cgc tct tat tct cga 3089
Tyr Ser Lys Ser Arg Ser Gly Ser Thr Arg Ser Arg Ser Tyr Ser Arg

        670                 675                 680

tca ttc agc cgc tca cat tct cgt tcc tat tca cgg tca cct cca tac 3137
Ser Phe Ser Arg Ser His Ser Arg Ser Tyr Ser Arg Ser Pro Pro Tyr
    685                 690                 695

ccc aga aga ggc aga ggc aag agc cgc aat tac cgt tca cgg tct aga 3185
Pro Arg Arg Gly Arg Gly Lys Ser Arg Asn Tyr Arg Ser Arg Ser Arg
700                 705                 710                 715

tct cat gga tat cat cga tct agg tca agg tca ccc cct tac aga cgc 3233
Ser His Gly Tyr His Arg Ser Arg Ser Arg Ser Pro Pro Tyr Arg Arg
                720                 725                 730

tat cat tca cga tca aga tct cct caa gcg ttt agg gga cag tct cct 3281
Tyr His Ser Arg Ser Arg Ser Pro Gln Ala Phe Arg Gly Gln Ser Pro
            735                 740                 745

aat aaa cgt aat gta cct caa ggg gaa aca gaa cgt gaa tat ttt aat 3329
Asn Lys Arg Asn Val Pro Gln Gly Glu Thr Glu Arg Glu Tyr Phe Asn
        750                 755                 760

aga tac aga gaa gtt cca cca cca tat gac atg aaa gca tat tat ggg 3377
Arg Tyr Arg Glu Val Pro Pro Pro Tyr Asp Met Lys Ala Tyr Tyr Gly
    765                 770                 775

aga agt gtt gac ttt aga gac cca ttt gaa aaa gaa cgc tac cga gaa 3425
Arg Ser Val Asp Phe Arg Asp Pro Phe Glu Lys Glu Arg Tyr Arg Glu
780                 785                 790                 795

tgg gag aga aaa tat aga gag tgg tat gaa aaa tat tat aaa ggt tat 3473
Trp Glu Arg Lys Tyr Arg Glu Trp Tyr Glu Lys Tyr Tyr Lys Gly Tyr
                800                 805                 810

gct gct gga gca cag cct aga ccc tca gca aat aga gag aac ttt tct 3521
Ala Ala Gly Ala Gln Pro Arg Pro Ser Ala Asn Arg Glu Asn Phe Ser
            815                 820                 825

cca gag aga ttt ttg cca ctt aac atc agg aat tct ccc ttc aca aga 3569
Pro Glu Arg Phe Leu Pro Leu Asn Ile Arg Asn Ser Pro Phe Thr Arg
        830                 835                 840

ggc cgc aga gaa gac tat gtt ggt ggg caa agt cat aga agt cga aac 3617
Gly Arg Arg Glu Asp Tyr Val Gly Gly Gln Ser His Arg Ser Arg Asn
    845                 850                 855

ata ggt agc aac tat cca gaa aag ctt tca gca aga gat ggt cac aat 3665
Ile Gly Ser Asn Tyr Pro Glu Lys Leu Ser Ala Arg Asp Gly His Asn
860                 865                 870                 875

cag aag gat aat aca aag tca aaa gag aag gag agt gaa aac gct cca 3713
Gln Lys Asp Asn Thr Lys Ser Lys Glu Lys Glu Ser Glu Asn Ala Pro
                880                 885                 890

gga gat ggt aaa gga aat aag cat aag aaa cac aga aaa aga aga aaa 3761
Gly Asp Gly Lys Gly Asn Lys His Lys Lys His Arg Lys Arg Arg Lys
            895                 900                 905

ggg gag gaa agt gag ggt ttt ctg aac cca gag tta tta gag act tct 3809
Gly Glu Glu Ser Glu Gly Phe Leu Asn Pro Glu Leu Leu Glu Thr Ser
        910                 915                 920

agg aaa tca aga gaa cct aca ggt gtt gaa gaa aat aaa aca gac tca 3857
Arg Lys Ser Arg Glu Pro Thr Gly Val Glu Glu Asn Lys Thr Asp Ser
    925                 930                 935

ttg ttt gtt ctc cca agt aga gat gat gcc aca cct gtt aga gat gaa 3905
Leu Phe Val Leu Pro Ser Arg Asp Asp Ala Thr Pro Val Arg Asp Glu

940                 945                 950                 955

cca atg gat gca gaa tca atc act ttt aaa tca gtg tct gaa aaa gac     3953
Pro Met Asp Ala Glu Ser Ile Thr Phe Lys Ser Val Ser Glu Lys Asp
            960                 965                 970

aag aga gaa agg gat aaa cca aaa gca aag ggt gat aaa acc aaa cgg     4001
Lys Arg Glu Arg Asp Lys Pro Lys Ala Lys Gly Asp Lys Thr Lys Arg
        975                 980                 985

aag aat gat gga tct gct gtg tcc aaa aaa gaa aat att  gta aaa cct     4049
Lys Asn Asp Gly Ser Ala Val Ser Lys Lys Glu Asn Ile  Val Lys Pro
    990                 995                 1000

gct aaa  gga ccc caa gaa aaa  gta gat gga gaa cgt  gag aga tct     4094
Ala Lys  Gly Pro Gln Glu Lys  Val Asp Gly Glu Arg  Glu Arg Ser
    1005                 1010                 1015

cct cga  tct gaa cct cca att  aaa aaa gcc aaa gag  gag act ccg     4139
Pro Arg  Ser Glu Pro Pro Ile  Lys Lys Ala Lys Glu  Glu Thr Pro
    1020                 1025                 1030

aag act  gac aat act aaa tca  tca tct tcc tct cag  aag gat gaa     4184
Lys Thr  Asp Asn Thr Lys Ser  Ser Ser Ser Ser Gln  Lys Asp Glu
    1035                 1040                 1045

aaa atc  act gga acc ccc aga  aaa gct cac tct aaa  tca gca aaa     4229
Lys Ile  Thr Gly Thr Pro Arg  Lys Ala His Ser Lys  Ser Ala Lys
    1050                 1055                 1060

gaa cac  caa gaa aca aaa cca  gtc aaa gag gaa aaa  gtg aag aag     4274
Glu His  Gln Glu Thr Lys Pro  Val Lys Glu Glu Lys  Val Lys Lys
    1065                 1070                 1075

gac tat  tcc aaa gat gtc aaa  tca gaa aag cta aca  act aag gaa     4319
Asp Tyr  Ser Lys Asp Val Lys  Ser Glu Lys Leu Thr  Thr Lys Glu
    1080                 1085                 1090

gaa aag  gcc aag aag cct aat  gag aaa aac aaa cca  ctt gat aat     4364
Glu Lys  Ala Lys Lys Pro Asn  Glu Lys Asn Lys Pro  Leu Asp Asn
    1095                 1100                 1105

aag gga  gaa aaa aga aaa aga  aaa act gaa gaa aaa  ggc gta gat     4409
Lys Gly  Glu Lys Arg Lys Arg  Lys Thr Glu Glu Lys  Gly Val Asp
    1110                 1115                 1120

aaa gat  ttt gag tct tct tca  atg aaa atc tcg aaa  cta gaa gtg     4454
Lys Asp  Phe Glu Ser Ser Ser  Met Lys Ile Ser Lys  Leu Glu Val
    1125                 1130                 1135

act gaa  ata gtg aaa cca tca  cca aag cgc aaa atg  gaa cct gat     4499
Thr Glu  Ile Val Lys Pro Ser  Pro Lys Arg Lys Met  Glu Pro Asp
    1140                 1145                 1150

act gaa  aaa atg gat agg acc  cct gaa aag gac aaa  att tct tta     4544
Thr Glu  Lys Met Asp Arg Thr  Pro Glu Lys Asp Lys  Ile Ser Leu
    1155                 1160                 1165

agt gcg  cca gcc aaa aaa atc  aaa ctc aac aga gaa  act ggg aag     4589
Ser Ala  Pro Ala Lys Lys Ile  Lys Leu Asn Arg Glu  Thr Gly Lys
    1170                 1175                 1180

aaa att  gga agt aca gaa aat  ata tca aac aca aaa  gaa ccc tct     4634
Lys Ile  Gly Ser Thr Glu Asn  Ile Ser Asn Thr Lys  Glu Pro Ser
    1185                 1190                 1195

gaa aaa  ttg gag tca aca tct  agc aaa gtt aaa caa  gaa aaa gtc     4679
Glu Lys  Leu Glu Ser Thr Ser  Ser Lys Val Lys Gln  Glu Lys Val

1200                1205                1210

aaa gga aag gtc aga cga aaa gtg act gga act gaa gga tcc agc      4724
Lys Gly Lys Val Arg Arg Lys Val Thr Gly Thr Glu Gly Ser Ser
    1215                1220                1225

tca act ctg gtg gat tac acc agt acg agc tca act gga ggc agt      4769
Ser Thr Leu Val Asp Tyr Thr Ser Thr Ser Ser Thr Gly Gly Ser
    1230                1235                1240

cct gtg cgg aaa tct gaa gaa aaa aca gat aca aag cga act gtg      4814
Pro Val Arg Lys Ser Glu Glu Lys Thr Asp Thr Lys Arg Thr Val
    1245                1250                1255

att aaa acg atg gaa gaa tat aat aat gac aat acc gcg cca gct      4859
Ile Lys Thr Met Glu Glu Tyr Asn Asn Asp Asn Thr Ala Pro Ala
    1260                1265                1270

gaa gat gtt atc att atg att cag gtt cct caa tcc aaa tgg gat      4904
Glu Asp Val Ile Ile Met Ile Gln Val Pro Gln Ser Lys Trp Asp
    1275                1280                1285

aaa gat gac ttt gaa tct gaa gaa gaa gat gtt aaa tcc aca cag      4949
Lys Asp Asp Phe Glu Ser Glu Glu Glu Asp Val Lys Ser Thr Gln
    1290                1295                1300

cct ata tca agt gta gga aaa cct gct agt gtt ata aaa aat gtt      4994
Pro Ile Ser Ser Val Gly Lys Pro Ala Ser Val Ile Lys Asn Val
    1305                1310                1315

agt aca aag cca tca aat ata gtc aag tat cct gag aaa gaa agt      5039
Ser Thr Lys Pro Ser Asn Ile Val Lys Tyr Pro Glu Lys Glu Ser
    1320                1325                1330

gag cca tcc gag aaa att cag aaa ttc acc aag gac gtg agc cat      5084
Glu Pro Ser Glu Lys Ile Gln Lys Phe Thr Lys Asp Val Ser His
    1335                1340                1345

gaa atc ata caa cat gag gtt aaa agt tca aaa aac tct gca tct      5129
Glu Ile Ile Gln His Glu Val Lys Ser Ser Lys Asn Ser Ala Ser
    1350                1355                1360

agt gaa aaa ggg aaa acc aaa gat cga gat tat tca gtg ttg gaa      5174
Ser Glu Lys Gly Lys Thr Lys Asp Arg Asp Tyr Ser Val Leu Glu
    1365                1370                1375

aag gag aac cct gaa aag agg aag aac agc act cag cca gag aaa      5219
Lys Glu Asn Pro Glu Lys Arg Lys Asn Ser Thr Gln Pro Glu Lys
    1380                1385                1390

gag agt aat ttg gac cgt ctg aat gaa caa gga aat ttt aaa agt      5264
Glu Ser Asn Leu Asp Arg Leu Asn Glu Gln Gly Asn Phe Lys Ser
    1395                1400                1405

ctg tct caa tct tcc aaa gag gct aga acg tca gat aaa cat gat      5309
Leu Ser Gln Ser Ser Lys Glu Ala Arg Thr Ser Asp Lys His Asp
    1410                1415                1420

tcc act cgt gct tcc tca aat aaa gac ttc act ccc aat aga gac      5354
Ser Thr Arg Ala Ser Ser Asn Lys Asp Phe Thr Pro Asn Arg Asp
    1425                1430                1435

aaa aaa act gac tat gac acc aga gag tat tca agt tcc aaa cgt      5399
Lys Lys Thr Asp Tyr Asp Thr Arg Glu Tyr Ser Ser Ser Lys Arg
    1440                1445                1450

aga gat gaa aag aat gaa tta aca aga cga aaa gac tct cct tct      5444
Arg Asp Glu Lys Asn Glu Leu Thr Arg Arg Lys Asp Ser Pro Ser

1455                1460                1465

cgg aat aaa gat tct gca tct gga cag aaa aat aaa cca agg gaa      5489
Arg Asn Lys Asp Ser Ala Ser Gly Gln Lys Asn Lys Pro Arg Glu
    1470                1475                1480

gag aga gat ttg cct aaa aaa gga aca gga gat tcc aaa aaa agt      5534
Glu Arg Asp Leu Pro Lys Lys Gly Thr Gly Asp Ser Lys Lys Ser
    1485                1490                1495

aat tct agt ccc tca aga gac aga aaa cct cat gat cac aaa gcc      5579
Asn Ser Ser Pro Ser Arg Asp Arg Lys Pro His Asp His Lys Ala
    1500                1505                1510

act tat gat act aaa cgg cca aat gaa gag aca aaa tct gta gat      5624
Thr Tyr Asp Thr Lys Arg Pro Asn Glu Glu Thr Lys Ser Val Asp
    1515                1520                1525

aaa aat cct tgt aag gat cgt gag aag cat gta tta gaa gca agg      5669
Lys Asn Pro Cys Lys Asp Arg Glu Lys His Val Leu Glu Ala Arg
    1530                1535                1540

aac aat aaa gag tca agt ggc aat aaa cta ctt tat ata ctt aac      5714
Asn Asn Lys Glu Ser Ser Gly Asn Lys Leu Leu Tyr Ile Leu Asn
    1545                1550                1555

cca cca gag aca cag gtt gaa aaa gag caa att act ggg caa att      5759
Pro Pro Glu Thr Gln Val Glu Lys Glu Gln Ile Thr Gly Gln Ile
    1560                1565                1570

gac aag agt act gtc aag cct aaa ccc cag tta agt cat tcc tct      5804
Asp Lys Ser Thr Val Lys Pro Lys Pro Gln Leu Ser His Ser Ser
    1575                1580                1585

aga ctt tcc tct gac tta act aga gaa act gat gaa gct gct ttt      5849
Arg Leu Ser Ser Asp Leu Thr Arg Glu Thr Asp Glu Ala Ala Phe
    1590                1595                1600

gaa cca gac tat aat gaa agt gac agt gaa agt aat gtt tct gta      5894
Glu Pro Asp Tyr Asn Glu Ser Asp Ser Glu Ser Asn Val Ser Val
    1605                1610                1615

aaa gaa gag gaa tct tca gga aac att tct aag gac ctg aaa gat      5939
Lys Glu Glu Glu Ser Ser Gly Asn Ile Ser Lys Asp Leu Lys Asp
    1620                1625                1630

aaa ata gtg gag aaa gca aaa gag agc ctg gac aca gca gca gtt      5984
Lys Ile Val Glu Lys Ala Lys Glu Ser Leu Asp Thr Ala Ala Val
    1635                1640                1645

gtc cag gtg ggc ata agc agg aat cag agc cac agc agc ccc agc      6029
Val Gln Val Gly Ile Ser Arg Asn Gln Ser His Ser Ser Pro Ser
    1650                1655                1660

gtc agc ccc agc aga agc cac agt cct tct gga agc cag acc cga      6074
Val Ser Pro Ser Arg Ser His Ser Pro Ser Gly Ser Gln Thr Arg
    1665                1670                1675

agc cac agt agc agt gcc agc tca gca gaa agt cag gac agc aag      6119
Ser His Ser Ser Ser Ala Ser Ser Ala Glu Ser Gln Asp Ser Lys
    1680                1685                1690

aag aag aag aaa aag aag gaa aag aaa aaa cac aag aaa cat aaa      6164
Lys Lys Lys Lys Lys Lys Glu Lys Lys Lys His Lys Lys His Lys
    1695                1700                1705

aag cat aag aag cat aag aaa cat gca ggc act gaa gtg gaa ttg      6209
Lys His Lys Lys His Lys Lys His Ala Gly Thr Glu Val Glu Leu

1710                    1715                    1720

gaa aaa  agc caa aaa cac aaa  cac aag aaa aag aag  tca aag aag       6254
Glu Lys  Ser Gln Lys His Lys  His Lys Lys Lys Lys  Ser Lys Lys
    1725                    1730                    1735

aac aaa  gat aaa gag aag gag  aag gag aaa gat gac  caa aaa gtg       6299
Asn Lys  Asp Lys Glu Lys Glu  Lys Glu Lys Asp Asp  Gln Lys Val
    1740                    1745                    1750

aaa tct  gtc act gtg taa aaagacagat tttttaaatt gacttaatta            6347
Lys Ser  Val Thr Val
    1755

ctaagtcatc tgtattaaat tttgttataa tgtaaagaga ttcaagcctt gtaaataatg    6407

acatggaaga ccctgtgctg cacttaaaat attgctgctt gattatttga tttttacatc    6467

agagctttat aacacgaact tttgtacaga attgtgagtt gtgaccatgt aacatgagag    6527

gttttgctag ggcctattat ttttaaccac cattaattag ttggggtgga gtttactgta    6587

atgtgaaatt ttcacatttg aattttttaa ttgcctggca aaagctgata taagttctaa    6647

aatatcagca gaatgatttg ctgaattcat tacaaccctg ttatgtcact ttttgattac     6707

aataaaagtt ttcagtaaac ttttcaaaaa aaaaaaaaaa aaaa                     6751

<210>  12
<211>  1758
<212>  PRT
<213>  human

<400>  12

Met Ser Cys Val His Tyr Lys Phe Ser Ser Lys Leu Asn Tyr Asp Thr
1               5                   10                  15

Val Thr Phe Asp Gly Leu His Ile Ser Leu Cys Asp Leu Lys Lys Gln
            20                  25                  30

Ile Met Gly Arg Glu Lys Leu Lys Ala Ala Asp Cys Asp Leu Gln Ile
        35                  40                  45

Thr Asn Ala Gln Thr Lys Glu Glu Tyr Thr Asp Asp Asn Ala Leu Ile
    50                  55                  60

Pro Lys Asn Ser Ser Val Ile Val Arg Arg Ile Pro Ile Gly Gly Val
65                  70                  75                  80

Lys Ser Thr Ser Lys Thr Tyr Val Ile Ser Arg Thr Glu Pro Ala Met
                85                  90                  95

Ala Thr Thr Lys Ala Ile Asp Asp Ser Ser Ala Ser Ile Ser Leu Ala
            100                 105                 110

Gln Leu Thr Lys Thr Ala Asn Leu Ala Glu Ala Asn Ala Ser Glu Glu
        115                 120                 125

Asp Lys Ile Lys Ala Met Met Ser Gln Ser Gly His Glu Tyr Asp Pro
    130                 135                 140

Ile Asn Tyr Met Lys Lys Pro Leu Gly Pro Pro Pro Pro Ser Tyr Thr
145                 150                 155                 160

Cys Phe Arg Cys Gly Lys Pro Gly His Tyr Ile Lys Asn Cys Pro Thr
                165                 170                 175

Asn Gly Asp Lys Asn Phe Glu Ser Gly Pro Arg Ile Lys Lys Ser Thr
            180                 185                 190

Gly Ile Pro Arg Ser Phe Met Met Glu Val Lys Asp Pro Asn Met Lys
        195                 200                 205

Gly Ala Met Leu Thr Asn Thr Gly Lys Tyr Ala Ile Pro Thr Ile Asp
    210                 215                 220

Ala Glu Ala Tyr Ala Ile Gly Lys Lys Glu Lys Pro Pro Phe Leu Pro
225                 230                 235                 240

Glu Glu Pro Ser Ser Ser Ser Glu Glu Asp Asp Pro Ile Pro Asp Glu
                245                 250                 255

Leu Leu Cys Leu Ile Cys Lys Asp Ile Met Thr Asp Ala Val Val Ile
            260                 265                 270

Pro Cys Cys Gly Asn Ser Tyr Cys Asp Glu Cys Ile Arg Thr Ala Leu
        275                 280                 285

Leu Glu Ser Asp Glu His Thr Cys Pro Thr Cys His Gln Asn Asp Val
    290                 295                 300

Ser Pro Asp Ala Leu Ile Ala Asn Lys Phe Leu Arg Gln Ala Val Asn
305                 310                 315                 320

Asn Phe Lys Asn Glu Thr Gly Tyr Thr Lys Arg Leu Arg Lys Gln Leu
                325                 330                 335

Pro Pro Pro Pro Pro Pro Ile Pro Pro Pro Arg Pro Leu Ile Gln Arg
            340                 345                 350

Asn Leu Gln Pro Leu Met Arg Ser Pro Ile Ser Arg Gln Gln Asp Pro
        355                 360                 365

Leu Met Ile Pro Val Thr Ser Ser Ser Thr His Pro Ala Pro Ser Ile
    370                 375                 380

Ser Ser Leu Thr Ser Asn Gln Ser Ser Leu Ala Pro Pro Val Ser Gly
385                 390                 395                 400

Asn Pro Ser Ser Ala Pro Ala Pro Val Pro Asp Ile Thr Ala Thr Val
                405                 410                 415

Ser Ile Ser Val His Ser Glu Lys Ser Asp Gly Pro Phe Arg Asp Ser
            420                 425                 430

Asp Asn Lys Ile Leu Pro Ala Ala Ala Leu Ala Ser Glu His Ser Lys
        435                 440                 445

Gly Thr Ser Ser Ile Ala Ile Thr Ala Leu Met Glu Glu Lys Gly Tyr
    450                 455                 460

Gln Val Pro Val Leu Gly Thr Pro Ser Leu Leu Gly Gln Ser Leu Leu
465                 470                 475                 480

His Gly Gln Leu Ile Pro Thr Thr Gly Pro Val Arg Ile Asn Thr Ala
                485                 490                 495

Arg Pro Gly Gly Gly Arg Pro Gly Trp Glu His Ser Asn Lys Leu Gly
            500                 505                 510

Tyr Leu Val Ser Pro Pro Gln Gln Ile Arg Arg Gly Glu Arg Ser Cys
        515                 520                 525

Tyr Arg Ser Ile Asn Arg Gly Arg His His Ser Glu Arg Ser Gln Arg
    530                 535                 540

Thr Gln Gly Pro Ser Leu Pro Ala Thr Pro Val Phe Val Pro Val Pro
545                 550                 555                 560

Pro Pro Pro Leu Tyr Pro Pro Pro Pro His Thr Leu Pro Leu Pro Pro
                565                 570                 575

Gly Val Pro Pro Pro Gln Phe Ser Pro Gln Phe Pro Pro Gly Gln Pro
            580                 585                 590

Pro Pro Ala Gly Tyr Ser Val Pro Pro Pro Gly Phe Pro Pro Ala Pro
        595                 600                 605

Ala Asn Leu Ser Thr Pro Trp Val Ser Ser Gly Val Gln Thr Ala His
    610                 615                 620

Ser Asn Thr Ile Pro Thr Thr Gln Ala Pro Pro Leu Ser Arg Glu Glu
625                 630                 635                 640

Phe Tyr Arg Glu Gln Arg Arg Leu Lys Glu Glu Ser Lys Ser Pro Tyr
                645                 650                 655

Ser Gly Ser Ser Tyr Ser Arg Ser Ser Tyr Thr Tyr Ser Lys Ser Arg
            660                 665                 670

Ser Gly Ser Thr Arg Ser Arg Ser Tyr Ser Arg Ser Phe Ser Arg Ser
        675                 680                 685

His Ser Arg Ser Tyr Ser Arg Ser Pro Pro Tyr Pro Arg Arg Gly Arg
    690                 695                 700

Gly Lys Ser Arg Asn Tyr Arg Ser Arg Ser Arg Ser His Gly Tyr His
705                 710                 715                 720

Arg Ser Arg Ser Arg Ser Pro Pro Tyr Arg Arg Tyr His Ser Arg Ser
                725                 730                 735

Arg Ser Pro Gln Ala Phe Arg Gly Gln Ser Pro Asn Lys Arg Asn Val
            740                 745                 750

Pro Gln Gly Glu Thr Glu Arg Glu Tyr Phe Asn Arg Tyr Arg Glu Val
        755                 760                 765

Pro Pro Pro Tyr Asp Met Lys Ala Tyr Tyr Gly Arg Ser Val Asp Phe
    770                 775                 780

Arg Asp Pro Phe Glu Lys Glu Arg Tyr Arg Glu Trp Glu Arg Lys Tyr
785                 790                 795                 800

Arg Glu Trp Tyr Glu Lys Tyr Tyr Lys Gly Tyr Ala Ala Gly Ala Gln
                805                 810                 815

Pro Arg Pro Ser Ala Asn Arg Glu Asn Phe Ser Pro Glu Arg Phe Leu
            820                 825                 830

Pro Leu Asn Ile Arg Asn Ser Pro Phe Thr Arg Gly Arg Arg Glu Asp
        835                 840                 845

Tyr Val Gly Gly Gln Ser His Arg Ser Arg Asn Ile Gly Ser Asn Tyr
    850                 855                 860

Pro Glu Lys Leu Ser Ala Arg Asp Gly His Asn Gln Lys Asp Asn Thr
865                 870                 875                 880

Lys Ser Lys Glu Lys Glu Ser Glu Asn Ala Pro Gly Asp Gly Lys Gly
                885                 890                 895

Asn Lys His Lys Lys His Arg Lys Arg Arg Lys Gly Glu Glu Ser Glu
            900                 905                 910

Gly Phe Leu Asn Pro Glu Leu Leu Glu Thr Ser Arg Lys Ser Arg Glu
        915                 920                 925

Pro Thr Gly Val Glu Glu Asn Lys Thr Asp Ser Leu Phe Val Leu Pro
    930                 935                 940

Ser Arg Asp Asp Ala Thr Pro Val Arg Asp Glu Pro Met Asp Ala Glu
945                 950                 955                 960

Ser Ile Thr Phe Lys Ser Val Ser Glu Lys Asp Lys Arg Glu Arg Asp
                965                 970                 975

Lys Pro Lys Ala Lys Gly Asp Lys Thr Lys Arg Lys Asn Asp Gly Ser
            980                 985                 990

Ala Val Ser Lys Lys Glu Asn Ile  Val Lys Pro Ala Lys  Gly Pro Gln
        995                 1000                 1005

Glu Lys  Val Asp Gly Glu Arg  Glu Arg Ser Pro Arg  Ser Glu Pro
    1010                 1015                 1020

Pro Ile  Lys Lys Ala Lys Glu  Glu Thr Pro Lys Thr  Asp Asn Thr
    1025                 1030                 1035

Lys Ser  Ser Ser Ser Ser Gln  Lys Asp Glu Lys Ile  Thr Gly Thr
    1040                 1045                 1050

Pro Arg  Lys Ala His Ser Lys  Ser Ala Lys Glu His  Gln Glu Thr
    1055                 1060                 1065

Lys Pro  Val Lys Glu Glu Lys  Val Lys Lys Asp Tyr  Ser Lys Asp
    1070                 1075                 1080

Val Lys  Ser Glu Lys Leu Thr  Thr Lys Glu Glu Lys  Ala Lys Lys
    1085                 1090                 1095

Pro Asn  Glu Lys Asn Lys Pro  Leu Asp Asn Lys Gly  Glu Lys Arg
    1100                 1105                 1110

Lys Arg  Lys Thr Glu Glu Lys  Gly Val Asp Lys Asp  Phe Glu Ser
    1115                 1120                 1125

Ser Ser  Met Lys Ile Ser Lys  Leu Glu Val Thr Glu  Ile Val Lys
    1130                 1135                 1140

Pro Ser  Pro Lys Arg Lys Met  Glu Pro Asp Thr Glu  Lys Met Asp
    1145                 1150                 1155

Arg Thr  Pro Glu Lys Asp Lys  Ile Ser Leu Ser Ala  Pro Ala Lys
    1160                 1165                 1170

Lys Ile  Lys Leu Asn Arg Glu  Thr Gly Lys Lys Ile  Gly Ser Thr
    1175                 1180                 1185

Glu Asn  Ile Ser Asn Thr Lys  Glu Pro Ser Glu Lys  Leu Glu Ser
    1190                 1195                 1200

Thr Ser Ser Lys Val Lys Gln Glu Lys Val Lys Gly Lys Val Arg
    1205                1210                1215

Arg Lys Val Thr Gly Thr Glu Gly Ser Ser Ser Thr Leu Val Asp
    1220                1225                1230

Tyr Thr Ser Thr Ser Ser Thr Gly Gly Ser Pro Val Arg Lys Ser
    1235                1240                1245

Glu Glu Lys Thr Asp Thr Lys Arg Thr Val Ile Lys Thr Met Glu
    1250                1255                1260

Glu Tyr Asn Asn Asp Asn Thr Ala Pro Ala Glu Asp Val Ile Ile
    1265                1270                1275

Met Ile Gln Val Pro Gln Ser Lys Trp Asp Lys Asp Asp Phe Glu
    1280                1285                1290

Ser Glu Glu Glu Asp Val Lys Ser Thr Gln Pro Ile Ser Ser Val
    1295                1300                1305

Gly Lys Pro Ala Ser Val Ile Lys Asn Val Ser Thr Lys Pro Ser
    1310                1315                1320

Asn Ile Val Lys Tyr Pro Glu Lys Glu Ser Glu Pro Ser Glu Lys
    1325                1330                1335

Ile Gln Lys Phe Thr Lys Asp Val Ser His Glu Ile Ile Gln His
    1340                1345                1350

Glu Val Lys Ser Ser Lys Asn Ser Ala Ser Ser Glu Lys Gly Lys
    1355                1360                1365

Thr Lys Asp Arg Asp Tyr Ser Val Leu Glu Lys Glu Asn Pro Glu
    1370                1375                1380

Lys Arg Lys Asn Ser Thr Gln Pro Glu Lys Glu Ser Asn Leu Asp
    1385                1390                1395

Arg Leu Asn Glu Gln Gly Asn Phe Lys Ser Leu Ser Gln Ser Ser
    1400                1405                1410

Lys Glu Ala Arg Thr Ser Asp Lys His Asp Ser Thr Arg Ala Ser
    1415                1420                1425

Ser Asn Lys Asp Phe Thr Pro Asn Arg Asp Lys Lys Thr Asp Tyr
    1430                1435                1440

Asp Thr Arg Glu Tyr Ser Ser Ser Lys Arg Arg Asp Glu Lys Asn
    1445                1450                1455

Glu Leu Thr Arg Arg Lys Asp Ser Pro Ser Arg Asn Lys Asp Ser
    1460                1465                1470

Ala Ser Gly Gln Lys Asn Lys Pro Arg Glu Glu Arg Asp Leu Pro
    1475                1480                1485

Lys Lys Gly Thr Gly Asp Ser Lys Lys Ser Asn Ser Ser Pro Ser
    1490                1495                1500

Arg Asp Arg Lys Pro His Asp His Lys Ala Thr Tyr Asp Thr Lys
    1505                1510                1515

Arg Pro Asn Glu Glu Thr Lys Ser Val Asp Lys Asn Pro Cys Lys
    1520                1525                1530

Asp Arg Glu Lys His Val Leu Glu Ala Arg Asn Asn Lys Glu Ser
    1535                1540                1545

Ser Gly Asn Lys Leu Leu Tyr Ile Leu Asn Pro Pro Glu Thr Gln
    1550                1555                1560

Val Glu Lys Glu Gln Ile Thr Gly Gln Ile Asp Lys Ser Thr Val
    1565                1570                1575

Lys Pro Lys Pro Gln Leu Ser His Ser Ser Arg Leu Ser Ser Asp
    1580                1585                1590

Leu Thr Arg Glu Thr Asp Glu Ala Ala Phe Glu Pro Asp Tyr Asn
    1595                1600                1605

Glu Ser Asp Ser Glu Ser Asn Val Ser Val Lys Glu Glu Glu Ser
    1610                1615                1620

Ser Gly Asn Ile Ser Lys Asp Leu Lys Asp Lys Ile Val Glu Lys
    1625                1630                1635

Ala Lys Glu Ser Leu Asp Thr Ala Ala Val Val Gln Val Gly Ile
    1640                1645                1650

Ser Arg Asn Gln Ser His Ser Ser Pro Ser Val Ser Pro Ser Arg
    1655                1660                1665

Ser His Ser Pro Ser Gly Ser Gln Thr Arg Ser His Ser Ser Ser
    1670                1675                1680

Ala Ser Ser Ala Glu Ser Gln Asp Ser Lys Lys Lys Lys Lys Lys
    1685                1690                1695

Lys Glu Lys Lys Lys His Lys Lys His Lys Lys His Lys Lys His
    1700                1705                1710

Lys Lys His Ala Gly Thr Glu Val Glu Leu Glu Lys Ser Gln Lys
    1715                1720                1725

His Lys His Lys Lys Lys Lys Ser Lys Lys Asn Lys Asp Lys Glu
    1730                1735                1740

Lys Glu Lys Glu Lys Asp Asp Gln Lys Val Lys Ser Val Thr Val
    1745                1750                1755

<210> 13
<211> 1780
<212> DNA
<213> human

<220>
<221> CDS
<222> (1041)..(1397)

<400> 13
gccaggccgc gtccgccata gtacctggct tggaggtgtc gccgccgctc ggtgagagcc 60

cccgagcggc agggggccaa cacaaaaagg gagccggaga agccctagcc gctgcccagc 120

agcttgcggg cgtgttctcg cggttccggg cctcaaggcg acggaaacga aaggcgagcg 180

aagcgcggag gatccggcga gaagaagcgt cagggagcct cggcggtgtc cccggggtcc 240

gccgaagcca cccggccgcc ggctggggcc cggggtggtg aggaagtgct ccgaggcctc 300

gccgaggcct agcgccggct ttgtgtccga ggcggcggcg gcggcggggg gaggcggagc 360

cgggggcggc ctgcgggaag gcctctcctc cgccgaccgc gcgttttcgg cctaggccgt 420

ggggccgctc gtggcctccg gggagcaggc gccaggggtt tgtgtgcggt gggggcctgg 480

gcctgggcct ggggaagctg acgccggtcg tccggaagcc aggaggaggc gtgaggccgc 540

tcgtggactc cgggcctagg ccctctcccc tcaaccttct cccggggcct gggtcacccc 600

aatccacgga gagagagacc cgccgggagg tgcggccgcg ctatggaccc ctgaccccgt 660

ggggtcgctc ggactcttaa cgtgtggact gaccgctact gactgcaccg ccaatccccc 720

cgtctctgcc ggccccttag catgagcgag ggggacccag ccgggtgaca ttgtgcccgt 780

tggcggattc tcgatttccc ctcttccccg tcctcgtcct cctcctcccc catgaagtga 840

ttctgagtat cggggggtct ctggattatt gttctgacga acccctgctt gtggttgggg 900

ggtatttaat ctgaggcctt agggtccttc ggtgtctttg agtgttttgt gtgtacatat 960

tttgctctta aagtttataa atatacgtat attgagagtg tccacgtctc ctcgctgaac 1020

cttaggaatc ccttggcacc atg tcc tgt gtg cat tat aaa ttt tcc tct aaa 1073
Met Ser Cys Val His Tyr Lys Phe Ser Ser Lys
1               5                   10

ctc aac tat gat acc gtc acc ttt gat ggg ctc cac atc tcc ctc tgc 1121
Leu Asn Tyr Asp Thr Val Thr Phe Asp Gly Leu His Ile Ser Leu Cys
            15                  20                  25

gac tta aag aag cag att atg ggg aga gag aag ctg aaa gct gcc gac 1169
Asp Leu Lys Lys Gln Ile Met Gly Arg Glu Lys Leu Lys Ala Ala Asp
        30                  35                  40

tgc gac ctg cag atc acc aat gcg cag acg aaa gaa gaa tat act gat 1217
Cys Asp Leu Gln Ile Thr Asn Ala Gln Thr Lys Glu Glu Tyr Thr Asp
    45                  50                  55

gat aat gct ctg att cct aag aat tct tct gta att gtt aga aga att 1265
Asp Asn Ala Leu Ile Pro Lys Asn Ser Ser Val Ile Val Arg Arg Ile
60                  65                  70                  75

cct att gga ggt gtt aaa tct aca agc aag aca tat gtt ata agt cga 1313
Pro Ile Gly Gly Val Lys Ser Thr Ser Lys Thr Tyr Val Ile Ser Arg
                80                  85                  90

act gaa cca gcg atg gca act aca aaa gca gta tgt aaa aac aca atc 1361
Thr Glu Pro Ala Met Ala Thr Thr Lys Ala Val Cys Lys Asn Thr Ile
            95                  100                 105

tca cac ttt ttc tac aca ttg ctt tta cct tta taa tgtagcagtg 1407
Ser His Phe Phe Tyr Thr Leu Leu Leu Pro Leu
        110                 115

aagtaaatca ttttagaact taatatccaa ctgatcatag tacatattgt aaataaaatg 1467

tattttgatg acagctcagt tgaatatgga taatatgtgg catcacttgc acacttattt 1527

tgtagaaatg ggtaatttgt gcccgtaaca ctgtttcata ttaaatatga tagcattatc 1587

cctgtatgac actgtgttgt acagttaatg tatgatcctt tttagatcgt ttaggtttta 1647

cactaaggaa catgatgaca tgttctacat ttgtctgtct atagttagta ttttgtatgt 1707

atgtacaggc tgttgtgtgc tttttgtttc ttgcaataaa aaatgtttgg agtgtaaaaa 1767

aaaaaaaaaa aaa 1780

<210> 14
<211> 118
<212> PRT
<213> human

<400> 14

Met Ser Cys Val His Tyr Lys Phe Ser Ser Lys Leu Asn Tyr Asp Thr
1               5                   10                  15

Val Thr Phe Asp Gly Leu His Ile Ser Leu Cys Asp Leu Lys Lys Gln
            20                  25                  30

Ile Met Gly Arg Glu Lys Leu Lys Ala Ala Asp Cys Asp Leu Gln Ile
        35                  40                  45

Thr Asn Ala Gln Thr Lys Glu Glu Tyr Thr Asp Asp Asn Ala Leu Ile
    50                  55                  60

Pro Lys Asn Ser Ser Val Ile Val Arg Arg Ile Pro Ile Gly Gly Val
65                  70                  75                  80

Lys Ser Thr Ser Lys Thr Tyr Val Ile Ser Arg Thr Glu Pro Ala Met
                85                  90                  95

Ala Thr Thr Lys Ala Val Cys Lys Asn Thr Ile Ser His Phe Phe Tyr

100                 105                 110

Thr Leu Leu Leu Pro Leu
        115

<210>  15
<211>  7926
<212>  DNA
<213>  human

<220>
<221>  CDS
<222>  (65)..(2854)

<400>  15
ggggaggagg gaggccgggc ctgcggccgg ggaccgagcc gcaaagacag agcgggcaga      60

ggcg atg gag ggc gac ggg gtg cca tgg ggc agc gag ccc gtc tcg ggt      109
     Met Glu Gly Asp Gly Val Pro Trp Gly Ser Glu Pro Val Ser Gly
     1               5                   10                  15

ccc ggc ccc ggc ggc ggc gga atg atc cgc gag ctg tgc cgg ggc ttc      157
Pro Gly Pro Gly Gly Gly Gly Met Ile Arg Glu Leu Cys Arg Gly Phe
                20                  25                  30

ggc cgc tac cgc cgc tac ctg gga cgg ctg cga cag aac ctg cgc gag      205
Gly Arg Tyr Arg Arg Tyr Leu Gly Arg Leu Arg Gln Asn Leu Arg Glu
            35                  40                  45

acc cag aag ttc ttc cgc gac atc aag tgc tcc cac aac cac act tgt      253
Thr Gln Lys Phe Phe Arg Asp Ile Lys Cys Ser His Asn His Thr Cys
        50                  55                  60

ctc tcc tcc ctc acg ggc ggc ggc ggg gcc gag cgc ggc cct gca ggc      301
Leu Ser Ser Leu Thr Gly Gly Gly Gly Ala Glu Arg Gly Pro Ala Gly
    65                  70                  75

gat gtc gcc gaa acc ggg ctg cag gcg ggc caa ctg agc tgc att tcc      349
Asp Val Ala Glu Thr Gly Leu Gln Ala Gly Gln Leu Ser Cys Ile Ser
80                  85                  90                  95

ttc cca cct aag gaa gag aag tac ctc cag cag att gtg gac tgc ctc      397
Phe Pro Pro Lys Glu Glu Lys Tyr Leu Gln Gln Ile Val Asp Cys Leu
                100                 105                 110

cct tgc ata ctg atc ctc ggc cag gat tgt aac gtc aag tgc cag ctg      445
Pro Cys Ile Leu Ile Leu Gly Gln Asp Cys Asn Val Lys Cys Gln Leu
            115                 120                 125

ttg aat ctg ctg ttg ggg gtg cag gtg ctt ccc acc acc aag ctg ggc      493
Leu Asn Leu Leu Leu Gly Val Gln Val Leu Pro Thr Thr Lys Leu Gly
        130                 135                 140

agt gag gag agc tgt aag ctt cgg cgc ctc cgc ttc acc tat ggg act      541
Ser Glu Glu Ser Cys Lys Leu Arg Arg Leu Arg Phe Thr Tyr Gly Thr
    145                 150                 155

cag act cgg gtc agc ctg gcg ctc cct gga cag tat gaa cta gtg cac      589
Gln Thr Arg Val Ser Leu Ala Leu Pro Gly Gln Tyr Glu Leu Val His
160                 165                 170                 175

acg ctg gtt gct cat cag ggc aac tgg gag acc atc cct gag gag gat      637
Thr Leu Val Ala His Gln Gly Asn Trp Glu Thr Ile Pro Glu Glu Asp
                180                 185                 190

ctg gag gtc caa gag aac aat gag gat gct gct cat gtt tta gcg gaa 685
Leu Glu Val Gln Glu Asn Asn Glu Asp Ala Ala His Val Leu Ala Glu
            195                 200                 205

ctg gag gta acg atg cac cat gct ctc tta cag gaa gtg gac gtt gtg 733
Leu Glu Val Thr Met His His Ala Leu Leu Gln Glu Val Asp Val Val
        210                 215                 220

gta gca cca tgc caa ggc ctc cgg ccc aca gtg gat gtt ctg ggt gac 781
Val Ala Pro Cys Gln Gly Leu Arg Pro Thr Val Asp Val Leu Gly Asp
    225                 230                 235

ttg gtg aat gat ttc ttg cct gtg ata acc tat gca ctc cac aaa gat 829
Leu Val Asn Asp Phe Leu Pro Val Ile Thr Tyr Ala Leu His Lys Asp
240                 245                 250                 255

gaa ctc tct gag agg gat gag caa gag ctt cag gaa atc cga aag tat 877
Glu Leu Ser Glu Arg Asp Glu Gln Glu Leu Gln Glu Ile Arg Lys Tyr
                260                 265                 270

ttc tcc ttt cct gta ttc ttt ttc aaa gtg ccg aaa ctg ggc tcg gag 925
Phe Ser Phe Pro Val Phe Phe Phe Lys Val Pro Lys Leu Gly Ser Glu
            275                 280                 285

ata ata gac tcc tca acc agg aga atg gag agc gaa aga tca ccg ctt 973
Ile Ile Asp Ser Ser Thr Arg Arg Met Glu Ser Glu Arg Ser Pro Leu
        290                 295                 300

tat cgc cag cta att gac ctg ggc tat ctg agc agc agt cac tgg aac 1021
Tyr Arg Gln Leu Ile Asp Leu Gly Tyr Leu Ser Ser Ser His Trp Asn
    305                 310                 315

tgt ggg gct cct ggc cag gat act aaa gct cag agc atg ttg gtg gaa 1069
Cys Gly Ala Pro Gly Gln Asp Thr Lys Ala Gln Ser Met Leu Val Glu
320                 325                 330                 335

cag agt gaa aag ctg aga cac ttg agc aca ttt tct cac cag gtg tta 1117
Gln Ser Glu Lys Leu Arg His Leu Ser Thr Phe Ser His Gln Val Leu
                340                 345                 350

cag act cgc ctg gtg gat gca gcc aag gcc ctg aac ctg gtg cac tgc 1165
Gln Thr Arg Leu Val Asp Ala Ala Lys Ala Leu Asn Leu Val His Cys
            355                 360                 365

cac tgc ctt gac atc ttt att aac cag gca ttt gac atg cag cgg gac 1213
His Cys Leu Asp Ile Phe Ile Asn Gln Ala Phe Asp Met Gln Arg Asp
        370                 375                 380

ctg cag atc act ccc aaa cgt ctg gaa tat act cga aaa aag gag aat 1261
Leu Gln Ile Thr Pro Lys Arg Leu Glu Tyr Thr Arg Lys Lys Glu Asn
    385                 390                 395

gag ttg tat gaa tca ttg atg aat att gcc aac cga aag cag gag gaa 1309
Glu Leu Tyr Glu Ser Leu Met Asn Ile Ala Asn Arg Lys Gln Glu Glu
400                 405                 410                 415

atg aag gat atg att gtt gag aca ctt aat acc atg aag gag gaa ctt 1357
Met Lys Asp Met Ile Val Glu Thr Leu Asn Thr Met Lys Glu Glu Leu
                420                 425                 430

ctg gat gat gct act aac atg gag ttt aaa gac gtc att gtc cct gag 1405
Leu Asp Asp Ala Thr Asn Met Glu Phe Lys Asp Val Ile Val Pro Glu
            435                 440                 445

aat gga gaa cca gta ggc acc aga gag atc aaa tgc tgc atc cga cag 1453
Asn Gly Glu Pro Val Gly Thr Arg Glu Ile Lys Cys Cys Ile Arg Gln
        450                 455                 460

atc cag gaa ctc atc atc tcc cga ctt aat cag gca gtg gct aat aag     1501
Ile Gln Glu Leu Ile Ile Ser Arg Leu Asn Gln Ala Val Ala Asn Lys
    465                 470                 475

ctg atc agc tca gtg gat tac ctg agg gaa agc ttc gtc gga acc ctg     1549
Leu Ile Ser Ser Val Asp Tyr Leu Arg Glu Ser Phe Val Gly Thr Leu
480                 485                 490                 495

gaa cga tgt ctg cag agc ctg gag aag tct cag gat gtc tca gtt cac     1597
Glu Arg Cys Leu Gln Ser Leu Glu Lys Ser Gln Asp Val Ser Val His
                500                 505                 510

atc acc agt aat tat ctc aaa cag atc tta aat gct gcc tat cat gtt     1645
Ile Thr Ser Asn Tyr Leu Lys Gln Ile Leu Asn Ala Ala Tyr His Val
            515                 520                 525

gaa gtc acg ttt cac tca ggg tcg tca gtt aca agg atg cta tgg gag     1693
Glu Val Thr Phe His Ser Gly Ser Ser Val Thr Arg Met Leu Trp Glu
        530                 535                 540

caa atc aaa cag atc atc cag cgc atc aca tgg gtg agc cca cct gcc     1741
Gln Ile Lys Gln Ile Ile Gln Arg Ile Thr Trp Val Ser Pro Pro Ala
    545                 550                 555

atc act ctg gaa tgg aag agg aag gtg gcc cag gaa gcc att gag agc     1789
Ile Thr Leu Glu Trp Lys Arg Lys Val Ala Gln Glu Ala Ile Glu Ser
560                 565                 570                 575

ctc agc gcc tcc aaa ttg gct aag agc att tgc agc caa ttc cgg act     1837
Leu Ser Ala Ser Lys Leu Ala Lys Ser Ile Cys Ser Gln Phe Arg Thr
                580                 585                 590

cgg ctc aat agt tcc cac gag gct ttt gca gcc tcc ttg cgg cag ctg     1885
Arg Leu Asn Ser Ser His Glu Ala Phe Ala Ala Ser Leu Arg Gln Leu
            595                 600                 605

gaa gct ggc cac tca ggc cgg tta gag aaa acg gaa gat cta tgg ctg     1933
Glu Ala Gly His Ser Gly Arg Leu Glu Lys Thr Glu Asp Leu Trp Leu
        610                 615                 620

agg gtt cgg aaa gat cat gct ccc cgc ctg gcc cgc ctt tct ctg gaa     1981
Arg Val Arg Lys Asp His Ala Pro Arg Leu Ala Arg Leu Ser Leu Glu
    625                 630                 635

agc tgt tct tta cag gat gtc ttg ctt cat cgt aaa cct aaa ctg gga     2029
Ser Cys Ser Leu Gln Asp Val Leu Leu His Arg Lys Pro Lys Leu Gly
640                 645                 650                 655

cag gaa ctg ggc cgg ggc cag tat ggt gtg gta tac ctg tgt gac aac     2077
Gln Glu Leu Gly Arg Gly Gln Tyr Gly Val Val Tyr Leu Cys Asp Asn
                660                 665                 670

tgg gga gga cac ttc cct tgt gcc ctc aaa tca gtt gtc cct cca gat     2125
Trp Gly Gly His Phe Pro Cys Ala Leu Lys Ser Val Val Pro Pro Asp
            675                 680                 685

gag aag cac tgg aat gat ctg gct ttg gaa ttt cac tat atg agg tct     2173
Glu Lys His Trp Asn Asp Leu Ala Leu Glu Phe His Tyr Met Arg Ser
        690                 695                 700

ctg ccg aag cat gag cga ttg gtg gat ctc cat ggt tca gtc att gac     2221
Leu Pro Lys His Glu Arg Leu Val Asp Leu His Gly Ser Val Ile Asp
    705                 710                 715

tac aac tat ggt ggt ggc tcc agc att gct gtg ctc ctc att atg gag     2269
Tyr Asn Tyr Gly Gly Gly Ser Ser Ile Ala Val Leu Leu Ile Met Glu
720                 725                 730                 735

cgg cta cac cgg gat ctc tac aca ggg ctg aag gct ggg ctg acc ctg     2317
Arg Leu His Arg Asp Leu Tyr Thr Gly Leu Lys Ala Gly Leu Thr Leu
                740                 745                 750

gag aca cgt ttg cag ata gca cta gat gtg gtg gag gga atc cgc ttc     2365
Glu Thr Arg Leu Gln Ile Ala Leu Asp Val Val Glu Gly Ile Arg Phe
            755                 760                 765

ctg cac agc cag gga ctt gtc cat cgt gat atc aaa ctg aaa aat gtg     2413
Leu His Ser Gln Gly Leu Val His Arg Asp Ile Lys Leu Lys Asn Val
        770                 775                 780

ctg ctg gat aag cag aac cgt gcc aag atc act gac tta gga ttc tgc     2461
Leu Leu Asp Lys Gln Asn Arg Ala Lys Ile Thr Asp Leu Gly Phe Cys
    785                 790                 795

aag cca gag gcc atg atg tca ggc agc att gtg ggg aca cca atc cat     2509
Lys Pro Glu Ala Met Met Ser Gly Ser Ile Val Gly Thr Pro Ile His
800                 805                 810                 815

atg gcc cct gaa ctt ttc aca ggg aag tac gat aat tcc gtg gat gtc     2557
Met Ala Pro Glu Leu Phe Thr Gly Lys Tyr Asp Asn Ser Val Asp Val
                820                 825                 830

tac gct ttt gga att ctt ttc tgg tat atc tgc tca ggc tct gtc aag     2605
Tyr Ala Phe Gly Ile Leu Phe Trp Tyr Ile Cys Ser Gly Ser Val Lys
            835                 840                 845

ctc cct gag gca ttt gag agg tgt gct agc aaa gac cat ctc tgg aac     2653
Leu Pro Glu Ala Phe Glu Arg Cys Ala Ser Lys Asp His Leu Trp Asn
        850                 855                 860

aat gtg cgg agg ggg gct cgc cca gaa cgt ctt cct gtg ttt gat gag     2701
Asn Val Arg Arg Gly Ala Arg Pro Glu Arg Leu Pro Val Phe Asp Glu
    865                 870                 875

gag tgc tgg cag ttg atg gaa gcc tgt tgg gat ggc gac ccc ttg aag     2749
Glu Cys Trp Gln Leu Met Glu Ala Cys Trp Asp Gly Asp Pro Leu Lys
880                 885                 890                 895

agg cct ctc ttg ggc att gtc cag ccc atg ctc cag ggc atc atg aat     2797
Arg Pro Leu Leu Gly Ile Val Gln Pro Met Leu Gln Gly Ile Met Asn
                900                 905                 910

cgg ctc tgc aag tcc aat tct gag cag cca aac aga gga cta gat gat     2845
Arg Leu Cys Lys Ser Asn Ser Glu Gln Pro Asn Arg Gly Leu Asp Asp
            915                 920                 925

tct act tga aagcaaagac ctttctcttt cactctctag ttatttcctt             2894
Ser Thr

cccccctcacc ttttggccat ggggagaatt tgacatttat tcactatagg acacactccc     2954

aagggaactg gtgcttgctg ggaaacttgg aaccttccca ggcagggatg actcctggac     3014

agtgaagagt tgaatgactg agcatattca gcagctcact gaagcgccaa gctatccctt     3074

tagcaaaaaa gtgtctcaga tgtgtaaaag ctgaggaatg tggtgttctg gcttcacaaa     3134

tgaaaaggag gcagatgtta ccattgtctt ttcactgtat atacttctaa gacagcaagc     3194

gggacactgc agtggcaata gtgttaaaaa atctcattct catgattttt ggctctagct     3254

aggaatagtt tagtcaggac tcagaaaatt tgagcttagt tctgggatta tgaaaacatg     3314

gggacaaaaa caataacttg tggatgtctg gttcctgctg tctgcagcca ggtatctcag     3374

gttgcaatgg tcagaagtcc cagtgaggga gctagaaaca gagctatctg ttcctcatag 3434
tgccagtgtg tttacattta taggaccaca tatccctggt ttctggggga gggtttcact 3494
gttacatcac caagaggccc tgaaaagagg aaaaaaaaaa aaaaagaggc caggcatggt 3554
ggctcacgcc tgtaatctca gcactttggg aggctgaggc gggcggatca caaggtccag 3614
agttcaagag cagcctggcc aacatggtga aaccccgtct ctactaaaga tacaaaaaat 3674
tagccgggcg tggtggtgca tgcctgtaat ccaagatact caggaggctg aggcaggaga 3734
atcgcttgaa cctgggaggc ggaggttgca gtgagccgag atcatgccat tgcactccag 3794
cctgggcgac agagtgagac tctgtctcaa aaaaaaaaaa aaaatagaat gtgtatgcat 3854
atatttgtgt gtatatgtat atatgtgtgt gtgtctatat ctctatctat atagacatac 3914
acacaggagt tattggattt agagttactg ctaaccccag aacagaatct gttcttttac 3974
ctttgagtct tttcttttgt ttacctcttt tcatctcaaa gtgggaaagc tccagataac 4034
atggtactta ggagtgagtg tctaagcaaa accaacccaa tgcgaagtat ttgggtgaga 4094
actgagaggt gggagatgat tggaggccat ggcctatgta caaacatact ggaagaaaac 4154
cagcttgttt ttaaaactta acacaatcag aaatttttct ttttgtgttt gaatctaaca 4214
cactgacgtt tggaaaactt gatcaatgag cttttaacca cgatttctcc cgcaatttgt 4274
tccttctttt taccttttta aaaatctgtt agaagaaaaa aagcagcaga cgaaagagaa 4334
aaaccctcct ctggtagttc tgtgtgtgcc cctcacccct gtgtgctagt gtctgggaag 4394
tttccctgaa aggtgggagg cgggtgggtg gtcttttgga gggttcctca tgaagtgtgc 4454
ttgaggaaag aaattctgat tctaggcaga ggaaggggaa caatttaaat attggtgcat 4514
ctcagatgca cttgacttca agccttcctc aaccaaactg gtccattaga ttccgaaaaa 4574
caggtagacg aggaagtaga cagtggaagt gagtagtctc cctccatctg cctaatcccc 4634
aataactgtg gtctggcccc tgtgttcagc ccgagagttt gtaaaacagg tccccaggta 4694
ggtgtataag atgagttgca aattagccgg gcatgctggc acgcaccagt aatcccagct 4754
acttgggagg ctgaggcagg agaatcactt gaacccggga ggcagaggtc atggtgagcc 4814
gagatcacgc cactgcactc cagcctgggc aacagagcga gactctatct caaaaaaaaa 4874
caaaaaaaaa aacaaaaaac aaagatgagt cgcacatctg ctgctgggat tttctagggt 4934
tgtctatgac agaatgacca tcttttaaat attaatggag tataaatgga gtctataatg 4994
ggtctttcat ataactttta actctttggc tatcctgggc tctgatcatg atatgtttga 5054
gcatagtagc tccctaaact cttggagcct ttcagaaatt ccatgtagtg ctaataacct 5114
ttttacttgc aaatgataaa gtaactgact cagtgtcttt gggctctgca ggctttagcc 5174
ttgttttggt aggatgttca gtatattctc agaattttta ccacctattt agctatactt 5234
caagtactct attctctgca ctaacttact gtccttctgg gaactatgat ggggcttcaa 5294
gggagtcagg tgtcaagaaa ggaggggtaa gtactctcta cttgctggaa aatcattgta 5354
agtctgttgt gtatgaaatt ttaggaaaag tcttatatag ttccctttcc agactgtttt 5414

cttttgactc taactataat cataagtctg tgcttccatt atgaaggtca cattcctagc 5474
attcttggga agtattagct catactctca ttcacatatg ggaggttaga tttttttcct 5534
ctacccagag tcccttttta gtttacccca cggcttagtt tgctgtcaga taccagttct 5594
ctagcagatt ccccaacagt tttttcccca gtggggaggg tggaagggag ggactgatat 5654
aagtaaggag atgagtagtt gtgttacagt tcattttctt taaaaaaaag tattttgaga 5714
aaaggtgcaa aatattcttg aaagtctcta ggccagccaa agtccacata tggatatttg 5774
gggatgttat ttaaatactc agcaggctga gtgcaggtat acgtgtgtat agagtgtgta 5834
tactttgctt ttgttttata acattgtcag ctaattttgg ctttgttctt tggcatccat 5894
tcttattata agtgatggaa tgccatgaaa tgtactgtat tgtatattac ttgggaggac 5954
actaggtttt caaagaagtc tggcagcact tgggttatag agggctcagc atgaccaaag 6014
tcctaaagat tttacctccc agctttggtt gtatgtgttc ccaagtggtg aggtgagatt 6074
gtggctggag gaagaaaaag gatggcatga aagaatgacc cttattgtgg tctgtccttc 6134
taatcaacat aattagggca gcttacccca acatgaaaat gatgacctgg ccatttttaa 6194
tggccagagc ttgaagtctg gtctctcatt ttgtttcaga aatgagtgag aggccttcct 6254
gcagagcagt caggagaaat ggtggagcct ggggccctgg aatggctgac cctgtgccta 6314
gagagctgat ggcaagggct gtctggcatg gctcatttgg gtggggtttc cccaggcata 6374
ccttagttcc ctgatcctct gcaaagagta gctcattgac tgagcaagcg cttcaggagg 6434
actgtgctgg ttgttcccag accaaagaga gtcacttttg actccatcaa cttcaagggg 6494
acagagccta ggcagcagat tctcctaatt tttaacacta aaattcaagg aagcagtgga 6554
ggaaagaatg cattactacc acagatagaa ataggggact catagaactc tcgactagta 6614
cctgtcagct ctcaaatgca ttcttgtgtc tttgcttgag ctgccaaaga agggtcacaa 6674
agtgttcttc ctcatggaag aagtgcctcg ggttgcaggg ctgcaaagt gacaactgca 6734
ctgaatgaca ctttattttt tggctttggc ttattattta gcactgggag gcccagggga 6794
attggtgatt acagtagcac tttgggaagt taaagccccc tttgggcttg ctttggagag 6854
atgatggcat gcttggtatt ccatttggat aatcaggaag cctccctagt ccatccatat 6914
tgtgtcagag tgggattaac aaccctggaa gagtccactg ccaaaagagt agttgctttc 6974
ctggaatttc caaagctttt ggattttctg gatcaagtaa cccacttttt aaaacctgag 7034
gtcaggagtt cgagaccagc ctggtaacat ggtgaaaccc cgtctctatt aaaaatagaa 7094
aaaattagcc aggcatggtg gcgggcacct atagtcccag ctactcagga ggctgaggta 7154
ggagaattgc ttgaacccag gagacagagg ctgcattgag ccaagatcac accactgcac 7214
tccagcctgg gcagcagagc aagactccat ctcaaaaaaa aaaaaaagaa aaagaaaaag 7274
aaggttaatc cttcagttat ggaggtggga tgaatagagc ttgtttgatg ttaaagtggg 7334
taaggaggga gtggccttga gacacttgta ttccaaactc tcctggaggt ttccagtagc 7394
actactgttc ctaaaagggt ttcattttta acttcatctg ttttgttaac atccagtcca 7454

attgaggtga tctcagaggt gcatcaggac atctagcact ggggaggcca ccttgcccag 7514

atagttgaaa agaaaattgg tctgggcagc ctgttgtctt ttgtcttcat gtaatgtttt 7574

ttctttgttt taaaggacta atgtttatta cagtgttaaa taaaagtgta agatactaag 7634

tgtgtagaat aaaagtgcaa taacaaaaga caatgacttt ggcacacact tcagtcttta 7694

tcctctctcc tttcttgtgc tacctggctc tttccataat attgttacag caggaccgtc 7754

ttaattgtgt gcattttgaa gagatgcgac tctgggttaa tcttcattag tgtaatattg 7814

aagggttggg tttggtttta tagagtattc tgtatacttg ttgggataca caaataccag 7874

atgtgctgta taataaagat cacattaacg ttttaaaaaa aaaaaaaaaa aa 7926

<210> 16
<211> 929
<212> PRT
<213> human

<400> 16

Met Glu Gly Asp Gly Val Pro Trp Gly Ser Glu Pro Val Ser Gly Pro
1               5                   10                  15

Gly Pro Gly Gly Gly Gly Met Ile Arg Glu Leu Cys Arg Gly Phe Gly
            20                  25                  30

Arg Tyr Arg Arg Tyr Leu Gly Arg Leu Arg Gln Asn Leu Arg Glu Thr
        35                  40                  45

Gln Lys Phe Phe Arg Asp Ile Lys Cys Ser His Asn His Thr Cys Leu
    50                  55                  60

Ser Ser Leu Thr Gly Gly Gly Gly Ala Glu Arg Gly Pro Ala Gly Asp
65                  70                  75                  80

Val Ala Glu Thr Gly Leu Gln Ala Gly Gln Leu Ser Cys Ile Ser Phe
                85                  90                  95

Pro Pro Lys Glu Glu Lys Tyr Leu Gln Gln Ile Val Asp Cys Leu Pro
            100                 105                 110

Cys Ile Leu Ile Leu Gly Gln Asp Cys Asn Val Lys Cys Gln Leu Leu
        115                 120                 125

Asn Leu Leu Leu Gly Val Gln Val Leu Pro Thr Thr Lys Leu Gly Ser
    130                 135                 140

Glu Glu Ser Cys Lys Leu Arg Arg Leu Arg Phe Thr Tyr Gly Thr Gln
145                 150                 155                 160

Thr Arg Val Ser Leu Ala Leu Pro Gly Gln Tyr Glu Leu Val His Thr
                165                 170                 175

Leu Val Ala His Gln Gly Asn Trp Glu Thr Ile Pro Glu Glu Asp Leu
            180                 185                 190

Glu Val Gln Glu Asn Asn Glu Asp Ala Ala His Val Leu Ala Glu Leu
        195                 200                 205

Glu Val Thr Met His His Ala Leu Leu Gln Glu Val Asp Val Val Val
    210                 215                 220

Ala Pro Cys Gln Gly Leu Arg Pro Thr Val Asp Val Leu Gly Asp Leu
225                 230                 235                 240

Val Asn Asp Phe Leu Pro Val Ile Thr Tyr Ala Leu His Lys Asp Glu
                245                 250                 255

Leu Ser Glu Arg Asp Glu Gln Glu Leu Gln Glu Ile Arg Lys Tyr Phe
            260                 265                 270

Ser Phe Pro Val Phe Phe Phe Lys Val Pro Lys Leu Gly Ser Glu Ile
        275                 280                 285

Ile Asp Ser Ser Thr Arg Arg Met Glu Ser Glu Arg Ser Pro Leu Tyr
    290                 295                 300

Arg Gln Leu Ile Asp Leu Gly Tyr Leu Ser Ser Ser His Trp Asn Cys
305                 310                 315                 320

Gly Ala Pro Gly Gln Asp Thr Lys Ala Gln Ser Met Leu Val Glu Gln
                325                 330                 335

Ser Glu Lys Leu Arg His Leu Ser Thr Phe Ser His Gln Val Leu Gln
            340                 345                 350

Thr Arg Leu Val Asp Ala Ala Lys Ala Leu Asn Leu Val His Cys His
        355                 360                 365

Cys Leu Asp Ile Phe Ile Asn Gln Ala Phe Asp Met Gln Arg Asp Leu
    370                 375                 380

Gln Ile Thr Pro Lys Arg Leu Glu Tyr Thr Arg Lys Lys Glu Asn Glu
385                 390                 395                 400

Leu Tyr Glu Ser Leu Met Asn Ile Ala Asn Arg Lys Gln Glu Glu Met
                405                 410                 415

Lys Asp Met Ile Val Glu Thr Leu Asn Thr Met Lys Glu Glu Leu Leu
            420                 425                 430

Asp Asp Ala Thr Asn Met Glu Phe Lys Asp Val Ile Val Pro Glu Asn
        435                 440                 445

Gly Glu Pro Val Gly Thr Arg Glu Ile Lys Cys Cys Ile Arg Gln Ile
    450                 455                 460

Gln Glu Leu Ile Ile Ser Arg Leu Asn Gln Ala Val Ala Asn Lys Leu
465                 470                 475                 480

Ile Ser Ser Val Asp Tyr Leu Arg Glu Ser Phe Val Gly Thr Leu Glu
                485                 490                 495

Arg Cys Leu Gln Ser Leu Glu Lys Ser Gln Asp Val Ser Val His Ile
            500                 505                 510

Thr Ser Asn Tyr Leu Lys Gln Ile Leu Asn Ala Ala Tyr His Val Glu
        515                 520                 525

Val Thr Phe His Ser Gly Ser Ser Val Thr Arg Met Leu Trp Glu Gln
    530                 535                 540

Ile Lys Gln Ile Ile Gln Arg Ile Thr Trp Val Ser Pro Pro Ala Ile
545                 550                 555                 560

Thr Leu Glu Trp Lys Arg Lys Val Ala Gln Glu Ala Ile Glu Ser Leu
                565                 570                 575

Ser Ala Ser Lys Leu Ala Lys Ser Ile Cys Ser Gln Phe Arg Thr Arg
            580                 585                 590

Leu Asn Ser Ser His Glu Ala Phe Ala Ala Ser Leu Arg Gln Leu Glu
        595                 600                 605

Ala Gly His Ser Gly Arg Leu Glu Lys Thr Glu Asp Leu Trp Leu Arg
    610                 615                 620

Val Arg Lys Asp His Ala Pro Arg Leu Ala Arg Leu Ser Leu Glu Ser
625                 630                 635                 640

Cys Ser Leu Gln Asp Val Leu Leu His Arg Lys Pro Lys Leu Gly Gln
                645                 650                 655

Glu Leu Gly Arg Gly Gln Tyr Gly Val Val Tyr Leu Cys Asp Asn Trp
            660                 665                 670

Gly Gly His Phe Pro Cys Ala Leu Lys Ser Val Val Pro Pro Asp Glu
        675                 680                 685

Lys His Trp Asn Asp Leu Ala Leu Glu Phe His Tyr Met Arg Ser Leu
    690                 695                 700

Pro Lys His Glu Arg Leu Val Asp Leu His Gly Ser Val Ile Asp Tyr
705                 710                 715                 720

Asn Tyr Gly Gly Gly Ser Ser Ile Ala Val Leu Leu Ile Met Glu Arg
                725                 730                 735

Leu His Arg Asp Leu Tyr Thr Gly Leu Lys Ala Gly Leu Thr Leu Glu
            740                 745                 750

Thr Arg Leu Gln Ile Ala Leu Asp Val Val Glu Gly Ile Arg Phe Leu
        755                 760                 765

His Ser Gln Gly Leu Val His Arg Asp Ile Lys Leu Lys Asn Val Leu
    770                 775                 780

Leu Asp Lys Gln Asn Arg Ala Lys Ile Thr Asp Leu Gly Phe Cys Lys
785                 790                 795                 800

Pro Glu Ala Met Met Ser Gly Ser Ile Val Gly Thr Pro Ile His Met
                805                 810                 815

Ala Pro Glu Leu Phe Thr Gly Lys Tyr Asp Asn Ser Val Asp Val Tyr
            820                 825                 830

Ala Phe Gly Ile Leu Phe Trp Tyr Ile Cys Ser Gly Ser Val Lys Leu
        835                 840                 845

Pro Glu Ala Phe Glu Arg Cys Ala Ser Lys Asp His Leu Trp Asn Asn
    850                 855                 860

Val Arg Arg Gly Ala Arg Pro Glu Arg Leu Pro Val Phe Asp Glu Glu
865                 870                 875                 880

Cys Trp Gln Leu Met Glu Ala Cys Trp Asp Gly Asp Pro Leu Lys Arg
                885                 890                 895

Pro Leu Leu Gly Ile Val Gln Pro Met Leu Gln Gly Ile Met Asn Arg
            900                 905                 910

Leu Cys Lys Ser Asn Ser Glu Gln Pro Asn Arg Gly Leu Asp Asp Ser
        915                 920                 925

Thr

<210>  17
<211>  7791
<212>  DNA
<213>  human

<220>
<221>  CDS
<222>  (65)..(2719)

<400>  17
   ggggaggagg gaggccgggc ctgcggccgg ggaccgagcc gcaaagacag agcgggcaga      60
   ggcg atg gag ggc gac ggg gtg cca tgg ggc agc gag ccc gtc tcg ggt 109
   Met Glu Gly Asp Gly Val Pro Trp Gly Ser Glu Pro Val Ser Gly
   1 5 10 15
   ccc ggc ccc ggc ggc ggc gga atg atc cgc gag ctg tgc cgg ggc ttc 157
   Pro Gly Pro Gly Gly Gly Gly Met Ile Arg Glu Leu Cys Arg Gly Phe
   20 25 30
   ggc cgc tac cgc cgc tac ctg gga cgg ctg cga cag aac ctg cgc gag 205
   Gly Arg Tyr Arg Arg Tyr Leu Gly Arg Leu Arg Gln Asn Leu Arg Glu
   35 40 45
   acc cag aag ttc ttc cgc gac atc aag tgc tcc cac aac cac act tgt 253
   Thr Gln Lys Phe Phe Arg Asp Ile Lys Cys Ser His Asn His Thr Cys
   50 55 60
   ctc tcc tcc ctc acg ggc ggc ggc ggg gcc gag cgc ggc cct gca ggc 301
   Leu Ser Ser Leu Thr Gly Gly Gly Gly Ala Glu Arg Gly Pro Ala Gly
   65 70 75
   gat gtc gcc gaa acc ggg ctg cag gcg ggc caa ctg agc tgc att tcc 349
   Asp Val Ala Glu Thr Gly Leu Gln Ala Gly Gln Leu Ser Cys Ile Ser
   80 85 90 95
   ttc cca cct aag gaa gag aag tac ctc cag cag att gtg gac tgc ctc 397
   Phe Pro Pro Lys Glu Glu Lys Tyr Leu Gln Gln Ile Val Asp Cys Leu
   100 105 110
   cct tgc ata ctg atc ctc ggc cag gat tgt aac gtc aag tgc cag ctg 445
   Pro Cys Ile Leu Ile Leu Gly Gln Asp Cys Asn Val Lys Cys Gln Leu
   115 120 125
   ttg aat ctg ctg ttg ggg gtg cag gtg ctt ccc acc acc aag ctg ggc 493
   Leu Asn Leu Leu Leu Gly Val Gln Val Leu Pro Thr Thr Lys Leu Gly
   130 135 140
   agt gag gag agc tgt aag ctt cgg cgc ctc cgc ttc acc tat ggg act 541
   Ser Glu Glu Ser Cys Lys Leu Arg Arg Leu Arg Phe Thr Tyr Gly Thr
   145 150 155
   cag act cgg gtc agc ctg gcg ctc cct gga cag tat gaa cta gtg cac 589
   Gln Thr Arg Val Ser Leu Ala Leu Pro Gly Gln Tyr Glu Leu Val His
   160 165 170 175
   acg ctg gtt gct cat cag ggc aac tgg gag acc atc cct gag gag gat 637
   Thr Leu Val Ala His Gln Gly Asn Trp Glu Thr Ile Pro Glu Glu Asp
   180 185 190
   ctg gag gtc caa gag aac aat gag gat gct gct cat gtt tta gcg gaa 685
   Leu Glu Val Gln Glu Asn Asn Glu Asp Ala Ala His Val Leu Ala Glu
   195 200 205
   ctg gag gta acg atg cac cat gct ctc tta cag gaa gtg gac gtt gtg 733
   Leu Glu Val Thr Met His His Ala Leu Leu Gln Glu Val Asp Val Val
   210 215 220
   gta gca cca tgc caa ggc ctc cgg ccc aca gtg gat gtt ctg ggt gac 781
   Val Ala Pro Cys Gln Gly Leu Arg Pro Thr Val Asp Val Leu Gly Asp
   225 230 235
   ttg gtg aat gat ttc ttg cct gtg ata acc tat gca ctc cac aaa gat 829
   Leu Val Asn Asp Phe Leu Pro Val Ile Thr Tyr Ala Leu His Lys Asp
   240 245 250 255
   gaa ctc tct gag agg gat gag caa gag ctt cag gaa atc cga aag tat 877
   Glu Leu Ser Glu Arg Asp Glu Gln Glu Leu Gln Glu Ile Arg Lys Tyr
   260 265 270
   ttc tcc ttt cct gta ttc ttt ttc aaa gtg ccg aaa ctg ggc tcg gag 925
   Phe Ser Phe Pro Val Phe Phe Phe Lys Val Pro Lys Leu Gly Ser Glu
   275 280 285
   ata ata gac tcc tca acc agg aga atg gag agc gaa aga tca ccg ctt 973
   Ile Ile Asp Ser Ser Thr Arg Arg Met Glu Ser Glu Arg Ser Pro Leu
   290 295 300
   tat cgc cag cta att gac ctg ggc tat ctg agc agc agt cac tgg aac 1021
   Tyr Arg Gln Leu Ile Asp Leu Gly Tyr Leu Ser Ser Ser His Trp Asn
   305 310 315
   tgt ggg gct cct ggc cag gat act aaa gct cag agc atg ttg gtg gaa 1069
   Cys Gly Ala Pro Gly Gln Asp Thr Lys Ala Gln Ser Met Leu Val Glu
   320 325 330 335
   cag agt gaa aag ctg aga cac ttg agc aca ttt tct cac cag gtg tta 1117
   Gln Ser Glu Lys Leu Arg His Leu Ser Thr Phe Ser His Gln Val Leu
   340 345 350
   cag act cgc ctg gtg gat gca gcc aag gcc ctg aac ctg gtg cac tgc 1165
   Gln Thr Arg Leu Val Asp Ala Ala Lys Ala Leu Asn Leu Val His Cys
   355 360 365
   cac tgc ctt gac atc ttt att aac cag gca ttt gac atg cag cgg gac 1213
   His Cys Leu Asp Ile Phe Ile Asn Gln Ala Phe Asp Met Gln Arg Asp
   370 375 380
   ctg cag atc act ccc aaa cgt ctg gaa tat act cga aaa aag gag aat 1261
   Leu Gln Ile Thr Pro Lys Arg Leu Glu Tyr Thr Arg Lys Lys Glu Asn
   385 390 395
   gag ttg tat gaa tca ttg atg aat att gcc aac cga aag cag gag gaa 1309
   Glu Leu Tyr Glu Ser Leu Met Asn Ile Ala Asn Arg Lys Gln Glu Glu
   400 405 410 415
   atg aag gat atg att gtt gag aca ctt aat acc atg aag gag gaa ctt 1357
   Met Lys Asp Met Ile Val Glu Thr Leu Asn Thr Met Lys Glu Glu Leu
   420 425 430
   ctg gat gat gct act aac atg gag ttt aaa gac gtc att gtc cct gag 1405
   Leu Asp Asp Ala Thr Asn Met Glu Phe Lys Asp Val Ile Val Pro Glu
   435 440 445
   aat gga gaa cca gta ggc acc aga gag atc aaa tgc tgc atc cga cag 1453
   Asn Gly Glu Pro Val Gly Thr Arg Glu Ile Lys Cys Cys Ile Arg Gln
   450 455 460
   atc cag gaa ctc atc atc tcc cga ctt aat cag gca gtg gct aat aag 1501
   Ile Gln Glu Leu Ile Ile Ser Arg Leu Asn Gln Ala Val Ala Asn Lys
   465 470 475
   ctg atc agc tca gtg gat tac ctg agg gaa agc ttc gtc gga acc ctg 1549
   Leu Ile Ser Ser Val Asp Tyr Leu Arg Glu Ser Phe Val Gly Thr Leu
   480 485 490 495
   gaa cga tgt ctg cag agc ctg gag aag tct cag gat gtc tca gtt cac 1597
   Glu Arg Cys Leu Gln Ser Leu Glu Lys Ser Gln Asp Val Ser Val His
   500 505 510
   atc acc agt aat tat ctc aaa cag atc tta aat gct gcc tat cat gtt 1645
   Ile Thr Ser Asn Tyr Leu Lys Gln Ile Leu Asn Ala Ala Tyr His Val
   515 520 525
   gaa gtc acg ttt cac tca ggg tcg tca gtt aca agg atg cta tgg gag 1693
   Glu Val Thr Phe His Ser Gly Ser Ser Val Thr Arg Met Leu Trp Glu
   530 535 540
   caa atc aaa cag atc atc cag cgc atc aca tgg gtg agc cca cct gcc     1741
   Gln Ile Lys Gln Ile Ile Gln Arg Ile Thr Trp Val Ser Pro Pro Ala
       545                 550                 555
   atc act ctg gaa tgg aag agg aag gtg gcc cag gaa gcc att gag agc     1789
   Ile Thr Leu Glu Trp Lys Arg Lys Val Ala Gln Glu Ala Ile Glu Ser
   560                 565                 570                 575
   ctc agc gcc tcc aaa ttg gct aag agc att tgc agc caa ttc cgg act     1837
   Leu Ser Ala Ser Lys Leu Ala Lys Ser Ile Cys Ser Gln Phe Arg Thr
                   580                 585                 590
   cgg ctc aat agt tcc cac gag gct ttt gca gcc tcc ttg cgg cag ctg     1885
   Arg Leu Asn Ser Ser His Glu Ala Phe Ala Ala Ser Leu Arg Gln Leu
               595                 600                 605
   gaa gct ggc cac tca ggc cgg tta gag aaa acg gaa gat cta tgg ctg     1933
   Glu Ala Gly His Ser Gly Arg Leu Glu Lys Thr Glu Asp Leu Trp Leu
           610                 615                 620
   agg gtt cgg aaa gat cat gct ccc cgc ctg gcc cgc ctt tct ctg gaa     1981
   Arg Val Arg Lys Asp His Ala Pro Arg Leu Ala Arg Leu Ser Leu Glu
       625                 630                 635
   agc tgt tct tta cag gat gtc ttg ctt cat cgt aaa cct aaa ctg gga     2029
   Ser Cys Ser Leu Gln Asp Val Leu Leu His Arg Lys Pro Lys Leu Gly
   640                 645                 650                 655
   cag gaa ctg ggc cgg ggc cag tat ggt gtg gta tac ctg tgt gac aac     2077
   Gln Glu Leu Gly Arg Gly Gln Tyr Gly Val Val Tyr Leu Cys Asp Asn
                   660                 665                 670
   tgg gga gga cac ttc cct tgt gcc ctc aaa tca gtt gtc cct cca gat     2125
   Trp Gly Gly His Phe Pro Cys Ala Leu Lys Ser Val Val Pro Pro Asp
               675                 680                 685
   gag aag cac tgg aat gat ctg gct ttg gaa ttt cac tat atg agg tct     2173
   Glu Lys His Trp Asn Asp Leu Ala Leu Glu Phe His Tyr Met Arg Ser
           690                 695                 700
   ctg ccg aag cat gag cga ttg gtg gat ctc cat ggt tca gtc att gac     2221
   Leu Pro Lys His Glu Arg Leu Val Asp Leu His Gly Ser Val Ile Asp
       705                 710                 715
   tac aac tat ggt ggt ggc tcc agc att gct gtg ctc ctc att atg gag     2269
   Tyr Asn Tyr Gly Gly Gly Ser Ser Ile Ala Val Leu Leu Ile Met Glu
   720                 725                 730                 735
   cgg cta cac cgg gat ctc tac aca ggg ctg aag gct ggg ctg acc ctg     2317
   Arg Leu His Arg Asp Leu Tyr Thr Gly Leu Lys Ala Gly Leu Thr Leu
                   740                 745                 750
   gag aca cgt ttg cag ata gca cta gat gtg gtg gag gga atc cgc ttc     2365
   Glu Thr Arg Leu Gln Ile Ala Leu Asp Val Val Glu Gly Ile Arg Phe
               755                 760                 765
   ctg cac agc cag gga ctt gtc cat cgt gat atc aaa ctg aaa aat gtg     2413
   Leu His Ser Gln Gly Leu Val His Arg Asp Ile Lys Leu Lys Asn Val
           770                 775                 780
   ctg ctg gat aag cag aac cgt gcc aag atc act gac tta gga ttc tgc     2461
   Leu Leu Asp Lys Gln Asn Arg Ala Lys Ile Thr Asp Leu Gly Phe Cys
       785                 790                 795
   aag cca gag gcc atg atg tca ggc agc att gtg ggg aca cca atc cat     2509
   Lys Pro Glu Ala Met Met Ser Gly Ser Ile Val Gly Thr Pro Ile His
   800                 805                 810                 815
   atg gcc cct gaa ctt ttc aca ggg gct cgc cca gaa cgt ctt cct gtg     2557
   Met Ala Pro Glu Leu Phe Thr Gly Ala Arg Pro Glu Arg Leu Pro Val
                   820                 825                 830
   ttt gat gag gag tgc tgg cag ttg atg gaa gcc tgt tgg gat ggc gac     2605
   Phe Asp Glu Glu Cys Trp Gln Leu Met Glu Ala Cys Trp Asp Gly Asp
                   835                 840                 845
   ccc ttg aag agg cct ctc ttg ggc att gtc cag ccc atg ctc cag ggc     2653
   Pro Leu Lys Arg Pro Leu Leu Gly Ile Val Gln Pro Met Leu Gln Gly
           850                 855                 860
   atc atg aat cgg ctc tgc aag tcc aat tct gag cag cca aac aga gga     2701
   Ile Met Asn Arg Leu Cys Lys Ser Asn Ser Glu Gln Pro Asn Arg Gly
       865                 870                 875
   cta gat gat tct act tga aagcaaagac ctttctcttt cactctctag            2749
   Leu Asp Asp Ser Thr
   880
   ttatttcctt ccccctcacc ttttggccat ggggagaatt tgacatttat tcactatagg     2809
   acacactccc aagggaactg gtgcttgctg ggaaacttgg aaccttccca ggcagggatg     2869
   actcctggac agtgaagagt tgaatgactg agcatattca gcagctcact gaagcgccaa     2929
   gctatccctt tagcaaaaaa gtgtctcaga tgtgtaaaag ctgaggaatg tggtgttctg     2989
   gcttcacaaa tgaaaaggag gcagatgtta ccattgtctt ttcactgtat atacttctaa     3049
   gacagcaagc gggacactgc agtggcaata gtgttaaaaa atctcattct catgattttt     3109
   ggctctagct aggaatagtt tagtcaggac tcagaaaatt tgagcttagt tctgggatta     3169
   tgaaaacatg gggacaaaaa caataacttg tggatgtctg gttcctgctg tctgcagcca     3229
   ggtatctcag gttgcaatgg tcagaagtcc cagtgaggga gctagaaaca gagctatctg     3289
   ttcctcatag tgccagtgtg tttacattta taggaccaca tatccctggt ttctggggga     3349
   gggtttcact gttacatcac caagaggccc tgaaaagagg aaaaaaaaaa aaaaagaggc     3409
   caggcatggt ggctcacgcc tgtaatctca gcactttggg aggctgaggc gggcggatca     3469
   caaggtccag agttcaagag cagcctggcc aacatggtga aaccccgtct ctactaaaga     3529
   tacaaaaaat tagccgggcg tggtggtgca tgcctgtaat ccaagatact caggaggctg     3589
   aggcaggaga atcgcttgaa cctgggaggc ggaggttgca gtgagccgag atcatgccat     3649
   tgcactccag cctgggcgac agagtgagac tctgtctcaa aaaaaaaaaa aaaatagaat     3709
   gtgtatgcat atatttgtgt gtatatgtat atatgtgtgt gtgtctatat ctctatctat     3769
   atagacatac acacaggagt tattggattt agagttactg ctaaccccag aacagaatct     3829
   gttcttttac ctttgagtct tttcttttgt ttacctcttt tcatctcaaa gtgggaaagc     3889
   tccagataac atggtactta ggagtgagtg tctaagcaaa accaacccaa tgcgaagtat     3949
   ttgggtgaga actgagaggt gggagatgat tggaggccat ggcctatgta caaacatact     4009
   ggaagaaaac cagcttgttt ttaaaactta acacaatcag aaatttttct ttttgtgttt     4069
   gaatctaaca cactgacgtt tggaaaactt gatcaatgag cttttaacca cgatttctcc     4129
   cgcaatttgt tccttctttt taccttttta aaaatctgtt agaagaaaaa aagcagcaga     4189
   cgaaagagaa aaaccctcct ctggtagttc tgtgtgtgcc cctcacccct gtgtgctagt 4249
   gtctgggaag tttccctgaa aggtgggagg cgggtgggtg gtcttttgga gggttcctca 4309
   tgaagtgtgc ttgaggaaag aaattctgat tctaggcaga ggaaggggaa caatttaaat 4369
   attggtgcat ctcagatgca cttgacttca agccttcctc aaccaaactg gtccattaga 4429
   ttccgaaaaa caggtagacg aggaagtaga cagtggaagt gagtagtctc cctccatctg 4489
   cctaatcccc aataactgtg gtctggcccc tgtgttcagc ccgagagttt gtaaaacagg 4549
   tccccaggta ggtgtataag atgagttgca aattagccgg gcatgctggc acgcaccagt 4609
   aatcccagct acttgggagg ctgaggcagg agaatcactt gaacccggga ggcagaggtc 4669
   atggtgagcc gagatcacgc cactgcactc cagcctgggc aacagagcga gactctatct 4729
   caaaaaaaaa caaaaaaaaa aacaaaaaac aaagatgagt cgcacatctg ctgctgggat 4789
   tttctagggt tgtctatgac agaatgacca tcttttaaat attaatggag tataaatgga 4849
   gtctataatg ggtctttcat ataactttta actctttggc tatcctgggc tctgatcatg 4909
   atatgtttga gcatagtagc tccctaaact cttggagcct ttcagaaatt ccatgtagtg 4969
   ctaataacct ttttacttgc aaatgataaa gtaactgact cagtgtcttt gggctctgca 5029
   ggctttagcc ttgttttggt aggatgttca gtatattctc agaattttta ccacctattt 5089
   agctatactt caagtactct attctctgca ctaacttact gtccttctgg gaactatgat 5149
   ggggcttcaa gggagtcagg tgtcaagaaa ggaggggtaa gtactctcta cttgctggaa 5209
   aatcattgta agtctgttgt gtatgaaatt ttaggaaaag tcttatatag ttccctttcc 5269
   agactgtttt cttttgactc taactataat cataagtctg tgcttccatt atgaaggtca 5329
   cattcctagc attcttggga agtattagct catactctca ttcacatatg ggaggttaga 5389
   tttttttcct ctacccagag tcccttttta gtttacccca cggcttagtt tgctgtcaga 5449
   taccagttct ctagcagatt ccccaacagt tttttcccca gtggggaggg tggaagggag 5509
   ggactgatat aagtaaggag atgagtagtt gtgttacagt tcattttctt taaaaaaaag 5569
   tattttgaga aaaggtgcaa aatattcttg aaagtctcta ggccagccaa agtccacata 5629
   tggatatttg gggatgttat ttaaatactc agcaggctga gtgcaggtat acgtgtgtat 5689
   agagtgtgta tactttgctt ttgttttata acattgtcag ctaattttgg ctttgttctt 5749
   tggcatccat tcttattata agtgatggaa tgccatgaaa tgtactgtat tgtatattac 5809
   ttgggaggac actaggtttt caaagaagtc tggcagcact tgggttatag agggctcagc 5869
   atgaccaaag tcctaaagat tttacctccc agctttggtt gtatgtgttc ccaagtggtg 5929
   aggtgagatt gtggctggag gaagaaaaag gatggcatga aagaatgacc cttattgtgg 5989
   tctgtccttc taatcaacat aattagggca gcttacccca acatgaaaat gatgacctgg 6049
   ccatttttaa tggccagagc ttgaagtctg gtctctcatt ttgtttcaga aatgagtgag 6109
   aggccttcct gcagagcagt caggagaaat ggtggagcct ggggccctgg aatggctgac 6169
   cctgtgccta gagagctgat ggcaagggct gtctggcatg gctcatttgg gtggggtttc 6229
   cccaggcata ccttagttcc ctgatcctct gcaaagagta gctcattgac tgagcaagcg 6289
   cttcaggagg actgtgctgg ttgttcccag accaaagaga gtcacttttg actccatcaa 6349
   cttcaagggg acagagccta ggcagcagat tctcctaatt tttaacacta aaattcaagg 6409
   aagcagtgga ggaaagaatg cattactacc acagatagaa ataggggact catagaactc 6469
   tcgactagta cctgtcagct ctcaaatgca ttcttgtgtc tttgcttgag ctgccaaaga 6529
   agggtcacaa agtgttcttc ctcatggaag aagtgcctcg gggttgcagg gctgcaaagt 6589
   gacaactgca ctgaatgaca ctttattttt tggctttggc ttattattta gcactgggag 6649
   gcccagggga attggtgatt acagtagcac tttgggaagt taaagccccc tttgggcttg 6709
   ctttggagag atgatggcat gcttggtatt ccatttggat aatcaggaag cctccctagt 6769
   ccatccatat tgtgtcagag tgggattaac aaccctggaa gagtccactg ccaaaagagt 6829
   agttgctttc ctggaatttc caaagctttt ggattttctg gatcaagtaa cccacttttt 6889
   aaaacctgag gtcaggagtt cgagaccagc ctggtaacat ggtgaaaccc cgtctctatt 6949
   aaaaatagaa aaaattagcc aggcatggtg gcgggcacct atagtcccag ctactcagga 7009
   ggctgaggta ggagaattgc ttgaacccag gagacagagg ctgcattgag ccaagatcac 7069
   accactgcac tccagcctgg gcagcagagc aagactccat ctcaaaaaaa aaaaaaagaa 7129
   aaagaaaaag aaggttaatc cttcagttat ggaggtggga tgaatagagc ttgtttgatg 7189
   ttaaagtggg taaggaggga gtggccttga gacacttgta ttccaaactc tcctggaggt 7249
   ttccagtagc actactgttc ctaaaagggt ttcattttta acttcatctg ttttgttaac 7309
   atccagtcca attgaggtga tctcagaggt gcatcaggac atctagcact ggggaggcca 7369
   ccttgcccag atagttgaaa agaaaattgg tctgggcagc ctgttgtctt ttgtcttcat 7429
   gtaatgtttt ttctttgttt taaaggacta atgtttatta cagtgttaaa taaaagtgta 7489
   agatactaag tgtgtagaat aaaagtgcaa taacaaaaga caatgacttt ggcacacact 7549
   tcagtcttta tcctctctcc tttcttgtgc tacctggctc tttccataat attgttacag 7609
   caggaccgtc ttaattgtgt gcattttgaa gagatgcgac tctgggttaa tcttcattag 7669
   tgtaatattg aagggttggg tttggtttta tagagtattc tgtatacttg ttgggataca 7729
   caaataccag atgtgctgta taataaagat cacattaacg ttttaaaaaa aaaaaaaaaa 7789
   aa 7791

<210> 18
<211> 884
<212> PRT
<213> human

<400> 18

Met Glu Gly Asp Gly Val Pro Trp Gly Ser Glu Pro Val Ser Gly Pro
1               5                   10                  15

Gly Pro Gly Gly Gly Gly Met Ile Arg Glu Leu Cys Arg Gly Phe Gly

            20                  25                  30

Arg Tyr Arg Arg Tyr Leu Gly Arg Leu Arg Gln Asn Leu Arg Glu Thr
        35                  40                  45

Gln Lys Phe Phe Arg Asp Ile Lys Cys Ser His Asn His Thr Cys Leu
    50                  55                  60

Ser Ser Leu Thr Gly Gly Gly Gly Ala Glu Arg Gly Pro Ala Gly Asp
65                  70                  75                  80

Val Ala Glu Thr Gly Leu Gln Ala Gly Gln Leu Ser Cys Ile Ser Phe
                85                  90                  95

Pro Pro Lys Glu Glu Lys Tyr Leu Gln Gln Ile Val Asp Cys Leu Pro
            100                 105                 110

Cys Ile Leu Ile Leu Gly Gln Asp Cys Asn Val Lys Cys Gln Leu Leu
        115                 120                 125

Asn Leu Leu Leu Gly Val Gln Val Leu Pro Thr Thr Lys Leu Gly Ser
    130                 135                 140

Glu Glu Ser Cys Lys Leu Arg Arg Leu Arg Phe Thr Tyr Gly Thr Gln
145                 150                 155                 160

Thr Arg Val Ser Leu Ala Leu Pro Gly Gln Tyr Glu Leu Val His Thr
                165                 170                 175

Leu Val Ala His Gln Gly Asn Trp Glu Thr Ile Pro Glu Glu Asp Leu
            180                 185                 190

Glu Val Gln Glu Asn Asn Glu Asp Ala Ala His Val Leu Ala Glu Leu
        195                 200                 205

Glu Val Thr Met His His Ala Leu Leu Gln Glu Val Asp Val Val Val
    210                 215                 220

Ala Pro Cys Gln Gly Leu Arg Pro Thr Val Asp Val Leu Gly Asp Leu
225                 230                 235                 240

Val Asn Asp Phe Leu Pro Val Ile Thr Tyr Ala Leu His Lys Asp Glu
                245                 250                 255

Leu Ser Glu Arg Asp Glu Gln Glu Leu Gln Glu Ile Arg Lys Tyr Phe
            260                 265                 270

Ser Phe Pro Val Phe Phe Phe Lys Val Pro Lys Leu Gly Ser Glu Ile
        275                 280                 285

Ile Asp Ser Ser Thr Arg Arg Met Glu Ser Glu Arg Ser Pro Leu Tyr

290                 295                 300

Arg Gln Leu Ile Asp Leu Gly Tyr Leu Ser Ser Ser His Trp Asn Cys
305                 310                 315                 320

Gly Ala Pro Gly Gln Asp Thr Lys Ala Gln Ser Met Leu Val Glu Gln
                325                 330                 335

Ser Glu Lys Leu Arg His Leu Ser Thr Phe Ser His Gln Val Leu Gln
            340                 345                 350

Thr Arg Leu Val Asp Ala Ala Lys Ala Leu Asn Leu Val His Cys His
        355                 360                 365

Cys Leu Asp Ile Phe Ile Asn Gln Ala Phe Asp Met Gln Arg Asp Leu
    370                 375                 380

Gln Ile Thr Pro Lys Arg Leu Glu Tyr Thr Arg Lys Lys Glu Asn Glu
385                 390                 395                 400

Leu Tyr Glu Ser Leu Met Asn Ile Ala Asn Arg Lys Gln Glu Glu Met
                405                 410                 415

Lys Asp Met Ile Val Glu Thr Leu Asn Thr Met Lys Glu Glu Leu Leu
            420                 425                 430

Asp Asp Ala Thr Asn Met Glu Phe Lys Asp Val Ile Val Pro Glu Asn
        435                 440                 445

Gly Glu Pro Val Gly Thr Arg Glu Ile Lys Cys Cys Ile Arg Gln Ile
    450                 455                 460

Gln Glu Leu Ile Ile Ser Arg Leu Asn Gln Ala Val Ala Asn Lys Leu
465                 470                 475                 480

Ile Ser Ser Val Asp Tyr Leu Arg Glu Ser Phe Val Gly Thr Leu Glu
                485                 490                 495

Arg Cys Leu Gln Ser Leu Glu Lys Ser Gln Asp Val Ser Val His Ile
            500                 505                 510

Thr Ser Asn Tyr Leu Lys Gln Ile Leu Asn Ala Ala Tyr His Val Glu
        515                 520                 525

Val Thr Phe His Ser Gly Ser Ser Val Thr Arg Met Leu Trp Glu Gln
    530                 535                 540

Ile Lys Gln Ile Ile Gln Arg Ile Thr Trp Val Ser Pro Pro Ala Ile
545                 550                 555                 560

Thr Leu Glu Trp Lys Arg Lys Val Ala Gln Glu Ala Ile Glu Ser Leu

                565                 570                 575

Ser Ala Ser Lys Leu Ala Lys Ser Ile Cys Ser Gln Phe Arg Thr Arg
            580                 585                 590

Leu Asn Ser Ser His Glu Ala Phe Ala Ala Ser Leu Arg Gln Leu Glu
        595                 600                 605

Ala Gly His Ser Gly Arg Leu Glu Lys Thr Glu Asp Leu Trp Leu Arg
    610                 615                 620

Val Arg Lys Asp His Ala Pro Arg Leu Ala Arg Leu Ser Leu Glu Ser
625                 630                 635                 640

Cys Ser Leu Gln Asp Val Leu Leu His Arg Lys Pro Lys Leu Gly Gln
                645                 650                 655

Glu Leu Gly Arg Gly Gln Tyr Gly Val Val Tyr Leu Cys Asp Asn Trp
            660                 665                 670

Gly Gly His Phe Pro Cys Ala Leu Lys Ser Val Val Pro Pro Asp Glu
        675                 680                 685

Lys His Trp Asn Asp Leu Ala Leu Glu Phe His Tyr Met Arg Ser Leu
    690                 695                 700

Pro Lys His Glu Arg Leu Val Asp Leu His Gly Ser Val Ile Asp Tyr
705                 710                 715                 720

Asn Tyr Gly Gly Gly Ser Ser Ile Ala Val Leu Leu Ile Met Glu Arg
                725                 730                 735

Leu His Arg Asp Leu Tyr Thr Gly Leu Lys Ala Gly Leu Thr Leu Glu
            740                 745                 750

Thr Arg Leu Gln Ile Ala Leu Asp Val Val Glu Gly Ile Arg Phe Leu
        755                 760                 765

His Ser Gln Gly Leu Val His Arg Asp Ile Lys Leu Lys Asn Val Leu
    770                 775                 780

Leu Asp Lys Gln Asn Arg Ala Lys Ile Thr Asp Leu Gly Phe Cys Lys
785                 790                 795                 800

Pro Glu Ala Met Met Ser Gly Ser Ile Val Gly Thr Pro Ile His Met
                805                 810                 815

Ala Pro Glu Leu Phe Thr Gly Ala Arg Pro Glu Arg Leu Pro Val Phe
            820                 825                 830

Asp Glu Glu Cys Trp Gln Leu Met Glu Ala Cys Trp Asp Gly Asp Pro

835                 840                 845

Leu Lys Arg Pro Leu Leu Gly Ile Val Gln Pro Met Leu Gln Gly Ile
    850                 855                 860

Met Asn Arg Leu Cys Lys Ser Asn Ser Glu Gln Pro Asn Arg Gly Leu
865                 870                 875                 880

Asp Asp Ser Thr

<210>  19
<211>  2012
<212>  DNA
<213>  human

<220>
<221>  CDS
<222>  (131)..(1234)

<400>  19
agggggcagg aacagcaggc ctggccagcc caaaggactc tctatccagg atgtaaatga      60

gcacactgct ggcccatgcg cctcggggct gtagagggca gcctcagagg cactgggcat     120

tcctggcacc atg gat gac gct gct gtc ctc aag cga cga ggc tac ctc        169
           Met Asp Asp Ala Ala Val Leu Lys Arg Arg Gly Tyr Leu
           1               5                   10

ctg ggg ata aat tta gga gag ggc tcc tat gca aaa gta aaa tct gct       217
Leu Gly Ile Asn Leu Gly Glu Gly Ser Tyr Ala Lys Val Lys Ser Ala
    15                  20                  25

tac tct gag cgc ctg aag ttc aat gtg gcg atc aag atc atc gac cgc       265
Tyr Ser Glu Arg Leu Lys Phe Asn Val Ala Ile Lys Ile Ile Asp Arg
30                  35                  40                  45

aag aag gcc ccc gca gac ttc ttg gag aaa ttc ctt ccc cgg gaa att       313
Lys Lys Ala Pro Ala Asp Phe Leu Glu Lys Phe Leu Pro Arg Glu Ile
                50                  55                  60

gag att ctg gcc atg tta aac cac tgc tcc atc att aag acc tac gag       361
Glu Ile Leu Ala Met Leu Asn His Cys Ser Ile Ile Lys Thr Tyr Glu
            65                  70                  75

atc ttt gag aca tca cat ggc aag gtc tac atc gtc atg gag ctc gcg       409
Ile Phe Glu Thr Ser His Gly Lys Val Tyr Ile Val Met Glu Leu Ala
        80                  85                  90

gtc cag ggc gac ctc ctc gag tta atc aaa acc cgg gga gcc ctg cat       457
Val Gln Gly Asp Leu Leu Glu Leu Ile Lys Thr Arg Gly Ala Leu His
    95                  100                 105

gag gac gaa gct cgc aag aag ttc cac cag ctt tcc ttg gcc atc aag       505
Glu Asp Glu Ala Arg Lys Lys Phe His Gln Leu Ser Leu Ala Ile Lys
110                 115                 120                 125

tac tgc cac gac ctg gac gtc gtc cac cgg gac ctc aag tgt gac aac       553
Tyr Cys His Asp Leu Asp Val Val His Arg Asp Leu Lys Cys Asp Asn
                130                 135                 140

ctt ctc ctt gac aag gac ttc aac atc aag ctg tcc gac ttc agc ttc       601
Leu Leu Leu Asp Lys Asp Phe Asn Ile Lys Leu Ser Asp Phe Ser Phe

145                 150                 155

tcc aag cgc tgc ctg cgg gat gac agt ggt cga atg gca tta agc aag      649
Ser Lys Arg Cys Leu Arg Asp Asp Ser Gly Arg Met Ala Leu Ser Lys
    160                 165                 170

acc ttc tgt ggg tca cca gcg tat gcg gcc cca gag gtg ctg cag ggc      697
Thr Phe Cys Gly Ser Pro Ala Tyr Ala Ala Pro Glu Val Leu Gln Gly
    175                 180                 185

att ccc tac cag ccc aag gtg tac gac atc tgg agc cta ggc gtg atc      745
Ile Pro Tyr Gln Pro Lys Val Tyr Asp Ile Trp Ser Leu Gly Val Ile
190                 195                 200                 205

ctc tac atc atg gtc tgc ggc tcc atg ccc tac gac gac tcc aac atc      793
Leu Tyr Ile Met Val Cys Gly Ser Met Pro Tyr Asp Asp Ser Asn Ile
                210                 215                 220

aag aag atg ctg cgt atc cag aag gag cac cgc gtc aac ttc cca cgc      841
Lys Lys Met Leu Arg Ile Gln Lys Glu His Arg Val Asn Phe Pro Arg
            225                 230                 235

tcc aag cac ctg aca ggc gag tgc aag gac ctc atc tac cac atg ctg      889
Ser Lys His Leu Thr Gly Glu Cys Lys Asp Leu Ile Tyr His Met Leu
        240                 245                 250

cag ccc gac gtc aac cgg cgg ctc cac atc gac gag atc ctc agc cac      937
Gln Pro Asp Val Asn Arg Arg Leu His Ile Asp Glu Ile Leu Ser His
    255                 260                 265

tgc tgg atg cag ccc aag gca cgg gga tct ccc tct gtg gcc atc aac      985
Cys Trp Met Gln Pro Lys Ala Arg Gly Ser Pro Ser Val Ala Ile Asn
270                 275                 280                 285

aag gag ggg gag agt tcc cgg gga act gaa ccc ttg tgg acc ccc gaa      1033
Lys Glu Gly Glu Ser Ser Arg Gly Thr Glu Pro Leu Trp Thr Pro Glu
                290                 295                 300

cct ggc tct gac aag aag tct gcc acc aag ctg gag cct gag gga gag      1081
Pro Gly Ser Asp Lys Lys Ser Ala Thr Lys Leu Glu Pro Glu Gly Glu
            305                 310                 315

gca cag ccc cag gca cag cct gag aca aaa ccc gag ggg aca gca atg      1129
Ala Gln Pro Gln Ala Gln Pro Glu Thr Lys Pro Glu Gly Thr Ala Met
        320                 325                 330

caa atg tcc agg cag tcg gag atc ctg ggt ttc ccc agc aag ccg tcg      1177
Gln Met Ser Arg Gln Ser Glu Ile Leu Gly Phe Pro Ser Lys Pro Ser
    335                 340                 345

act atg gag aca gag gaa ggg ccc ccc caa cag cct cca gag acg cgg      1225
Thr Met Glu Thr Glu Glu Gly Pro Pro Gln Gln Pro Pro Glu Thr Arg
350                 355                 360                 365

gca cag tga gcttcttgcg gcccagggaa tgagatggag ctcacggctt      1274
Ala Gln

aaagcccaag ctctgaagaa gtcaagggtg gagccagaga aggaaggcag tcccagatga      1334

gcctctattt tcatcagctt cttctctctc cccttgaact tggtaaccca catggttctc      1394

ccgtggcccc taggtggatg aggccaaagt caaatccaag gctgagacag tcgtgcgact      1454

cctactcccc cagagcgtga cccggagcag gtgctggaca cagagcctgt ctcagcagag      1514

ggtccccact ggccgcaacg gctcagtgac agcaagagca ggaagagcag caggaaggca      1574

ccgctgtcca ccttgggcac catttatcct cctttcatcg tccccggggc agttgcgtga 1634

ccctgctggg aggccagacc aggccagact gagggtcagg ggaccaggct gggttggggg 1694

ttgggtgggg ctggacgggg acgaggcgcg gagaggaggc gggaaagagc ttcggctggg 1754

ccacgggctc gcgggcactc tcggcggcag cctcgggggc tccgactcca gctctgggca 1814

agatccataa acttttaaag aagcctcaaa acatactttt aaagcattaa gctttaaagt 1874

gctagaaatc ttgtgcccca tatgtgcttc tttcagtatt atcctagatc ctcaggagta 1934

gcatggcaac tggagaaaat gggaggaaag acaagaagaa atagaagctg atttaacacc 1994

aaaaaaaaaa aaaaaaaa 2012

<210> 20
<211> 367
<212> PRT
<213> human

<400> 20

Met Asp Asp Ala Ala Val Leu Lys Arg Arg Gly Tyr Leu Leu Gly Ile
1               5                   10                  15

Asn Leu Gly Glu Gly Ser Tyr Ala Lys Val Lys Ser Ala Tyr Ser Glu
            20                  25                  30

Arg Leu Lys Phe Asn Val Ala Ile Lys Ile Ile Asp Arg Lys Lys Ala
        35                  40                  45

Pro Ala Asp Phe Leu Glu Lys Phe Leu Pro Arg Glu Ile Glu Ile Leu
    50                  55                  60

Ala Met Leu Asn His Cys Ser Ile Ile Lys Thr Tyr Glu Ile Phe Glu
65                  70                  75                  80

Thr Ser His Gly Lys Val Tyr Ile Val Met Glu Leu Ala Val Gln Gly
                85                  90                  95

Asp Leu Leu Glu Leu Ile Lys Thr Arg Gly Ala Leu His Glu Asp Glu
            100                 105                 110

Ala Arg Lys Lys Phe His Gln Leu Ser Leu Ala Ile Lys Tyr Cys His
        115                 120                 125

Asp Leu Asp Val Val His Arg Asp Leu Lys Cys Asp Asn Leu Leu Leu
    130                 135                 140

Asp Lys Asp Phe Asn Ile Lys Leu Ser Asp Phe Ser Phe Ser Lys Arg
145                 150                 155                 160

Cys Leu Arg Asp Asp Ser Gly Arg Met Ala Leu Ser Lys Thr Phe Cys
                165                 170                 175

Gly Ser Pro Ala Tyr Ala Ala Pro Glu Val Leu Gln Gly Ile Pro Tyr
          180                 185                 190

Gln Pro Lys Val Tyr Asp Ile Trp Ser Leu Gly Val Ile Leu Tyr Ile
        195                 200                 205

Met Val Cys Gly Ser Met Pro Tyr Asp Asp Ser Asn Ile Lys Lys Met
    210                 215                 220

Leu Arg Ile Gln Lys Glu His Arg Val Asn Phe Pro Arg Ser Lys His
225                 230                 235                 240

Leu Thr Gly Glu Cys Lys Asp Leu Ile Tyr His Met Leu Gln Pro Asp
                245                 250                 255

Val Asn Arg Arg Leu His Ile Asp Glu Ile Leu Ser His Cys Trp Met
            260                 265                 270

Gln Pro Lys Ala Arg Gly Ser Pro Ser Val Ala Ile Asn Lys Glu Gly
        275                 280                 285

Glu Ser Ser Arg Gly Thr Glu Pro Leu Trp Thr Pro Glu Pro Gly Ser
    290                 295                 300

Asp Lys Lys Ser Ala Thr Lys Leu Glu Pro Glu Gly Glu Ala Gln Pro
305                 310                 315                 320

Gln Ala Gln Pro Glu Thr Lys Pro Glu Gly Thr Ala Met Gln Met Ser
                325                 330                 335

Arg Gln Ser Glu Ile Leu Gly Phe Pro Ser Lys Pro Ser Thr Met Glu
            340                 345                 350

Thr Glu Glu Gly Pro Pro Gln Gln Pro Pro Glu Thr Arg Ala Gln
        355                 360                 365

<210>  21
<211>  1442
<212>  DNA
<213>  human

<220>
<221>  CDS
<222>  (234)..(1055)

<400>  21
ccggggcggg gtcccggacc cccaccctaa ggcggagtga cgcccgcagt cacttcacaa      60

ggcaaaaatt gttacggggc aataaaaggc acaacagcgg ccaatgtctg gcagtgggca     120

catgggggtg cgggggtgta ggtgccaagc gccatggctt agacccgaga ttggagtccg     180

gccgcccccc gacagcagcc gcctcctgcc ccccgtgcgc cctaggcgcc acc atg        236
                                                           Met
                                                           1

tcg gga gac aaa ctt ctg agc gaa ctc ggt tat aag ctg ggc cgc aca 284
Ser Gly Asp Lys Leu Leu Ser Glu Leu Gly Tyr Lys Leu Gly Arg Thr
            5                   10                  15

att gga gag ggc agc tac tcc aag gtg aag gtg gcc aca tcc aag aag 332
Ile Gly Glu Gly Ser Tyr Ser Lys Val Lys Val Ala Thr Ser Lys Lys
        20                  25                  30

tac aag ggt acc gtg gcc atc aag gtg gtg gac cgg cgg cga gcg ccc 380
Tyr Lys Gly Thr Val Ala Ile Lys Val Val Asp Arg Arg Arg Ala Pro
    35                  40                  45

ccg gac ttc gtc aac aag ttc ctg ccg cga gag ctg tcc atc ctg cgg 428
Pro Asp Phe Val Asn Lys Phe Leu Pro Arg Glu Leu Ser Ile Leu Arg
50                  55                  60                  65

ggc gtg cga cac ccg cac atc gtg cac gtc ttc gag ttc atc gag gtg 476
Gly Val Arg His Pro His Ile Val His Val Phe Glu Phe Ile Glu Val
                70                  75                  80

tgc aac ggg aaa ctg tac atc gtg atg gaa gcg gcc gcc acc gac ctg 524
Cys Asn Gly Lys Leu Tyr Ile Val Met Glu Ala Ala Ala Thr Asp Leu
            85                  90                  95

ctg caa gcc gtg cag cgc aac ggg cgc atc ccc gga gtt cag gcg cgc 572
Leu Gln Ala Val Gln Arg Asn Gly Arg Ile Pro Gly Val Gln Ala Arg
        100                 105                 110

gac ctc ttt gcg cag atc gcc ggc gcc gtg cgc tac ctg cac gat cat 620
Asp Leu Phe Ala Gln Ile Ala Gly Ala Val Arg Tyr Leu His Asp His
    115                 120                 125

cac ctg gtg cac cgc gac ctc aag tgc gaa aac gtg ctg ctg agc ccg 668
His Leu Val His Arg Asp Leu Lys Cys Glu Asn Val Leu Leu Ser Pro
130                 135                 140                 145

gac gag cgc cgc gtc aag ctc acc gac ttc ggc ttc ggc cgc cag gcc 716
Asp Glu Arg Arg Val Lys Leu Thr Asp Phe Gly Phe Gly Arg Gln Ala
                150                 155                 160

cat ggc tac cca gac ctg agc acc acc tac tgc ggc tca gcc gcc tac 764
His Gly Tyr Pro Asp Leu Ser Thr Thr Tyr Cys Gly Ser Ala Ala Tyr
            165                 170                 175

gcg tca ccc gag gtg ctc ctg ggc atc ccc tac gac ccc aag aag tac 812
Ala Ser Pro Glu Val Leu Leu Gly Ile Pro Tyr Asp Pro Lys Lys Tyr
        180                 185                 190

gat gtg tgg agc atg ggc gtc gtg ctc tac gtc atg gtc acc ggg tgc 860
Asp Val Trp Ser Met Gly Val Val Leu Tyr Val Met Val Thr Gly Cys
    195                 200                 205

atg ccc ttc gac gac tcg gac atc gcc ggc ctg ccc cgg cgc cag aaa 908
Met Pro Phe Asp Asp Ser Asp Ile Ala Gly Leu Pro Arg Arg Gln Lys
210                 215                 220                 225

cgc ggc gtg ctc tat ccc gaa ggc ctc gag ctg tcc gag cgc tgc aag 956
Arg Gly Val Leu Tyr Pro Glu Gly Leu Glu Leu Ser Glu Arg Cys Lys
                230                 235                 240

gcc ctg atc gcc gag ctg ctg cag ttc agc ccg tcc gcc agg ccc tcc 1004
Ala Leu Ile Ala Glu Leu Leu Gln Phe Ser Pro Ser Ala Arg Pro Ser
            245                 250                 255

gcg ggc cag gta gcg cgc aac tgc tgg ctg cgc gcc ggg gac tcc ggc 1052
Ala Gly Gln Val Ala Arg Asn Cys Trp Leu Arg Ala Gly Asp Ser Gly
        260                 265                 270

tag aagccgggtg gttccagcca ttcctgcagc caagggcact gggccagggc 1105

ggcgcacgcg caagaggcgc gcttcgaggg aatatgcgaa gctgccgcgt gctgctgcac 1165

atgcgctttt tcccttccgc ttccctccct ttcttcccac gggggagtcc gcagttgccc 1225

ttgttcggaa tccacgttcc ccgcgatccc gggagctgga ggcgcatgcg catccgcgat 1285

tccctgcgac caggccccga gagggcgaga ccagagggga cggaagcatt gcgcctgcgc 1345

ggaactctca gcctctgcgc ggagggcgtc ccttcccaac cagccgtggg tgccaggttc 1405

ccggttggaa cctgcaataa actcgctgtt cctcgca 1442

<210> 22
<211> 273
<212> PRT
<213> human

<400> 22

Met Ser Gly Asp Lys Leu Leu Ser Glu Leu Gly Tyr Lys Leu Gly Arg
1               5                   10                  15

Thr Ile Gly Glu Gly Ser Tyr Ser Lys Val Lys Val Ala Thr Ser Lys
            20                  25                  30

Lys Tyr Lys Gly Thr Val Ala Ile Lys Val Val Asp Arg Arg Arg Ala
        35                  40                  45

Pro Pro Asp Phe Val Asn Lys Phe Leu Pro Arg Glu Leu Ser Ile Leu
    50                  55                  60

Arg Gly Val Arg His Pro His Ile Val His Val Phe Glu Phe Ile Glu
65                  70                  75                  80

Val Cys Asn Gly Lys Leu Tyr Ile Val Met Glu Ala Ala Ala Thr Asp
                85                  90                  95

Leu Leu Gln Ala Val Gln Arg Asn Gly Arg Ile Pro Gly Val Gln Ala
            100                 105                 110

Arg Asp Leu Phe Ala Gln Ile Ala Gly Ala Val Arg Tyr Leu His Asp
        115                 120                 125

His His Leu Val His Arg Asp Leu Lys Cys Glu Asn Val Leu Leu Ser
    130                 135                 140

Pro Asp Glu Arg Arg Val Lys Leu Thr Asp Phe Gly Phe Gly Arg Gln
145                 150                 155                 160

Ala His Gly Tyr Pro Asp Leu Ser Thr Thr Tyr Cys Gly Ser Ala Ala
                165                 170                 175

Tyr Ala Ser Pro Glu Val Leu Leu Gly Ile Pro Tyr Asp Pro Lys Lys

180                 185                 190

Tyr Asp Val Trp Ser Met Gly Val Val Leu Tyr Val Met Val Thr Gly
        195                 200                 205

Cys Met Pro Phe Asp Asp Ser Asp Ile Ala Gly Leu Pro Arg Arg Gln
    210                 215                 220

Lys Arg Gly Val Leu Tyr Pro Glu Gly Leu Glu Leu Ser Glu Arg Cys
225                 230                 235                 240

Lys Ala Leu Ile Ala Glu Leu Leu Gln Phe Ser Pro Ser Ala Arg Pro
                245                 250                 255

Ser Ala Gly Gln Val Ala Arg Asn Cys Trp Leu Arg Ala Gly Asp Ser
            260                 265                 270

Gly

<210>  23
<211>  1454
<212>  DNA
<213>  human

<220>
<221>  CDS
<222>  (266)..(1201)

<400>  23
ccaggccagg aggtgccagg ttagagcttg gacactcaga ccctccagtg caggccttgc      60

ccggaccagc ccctcctcca ctctcctctc ccctgtgagc tccacctgcc ccagttctcc     120

tggctttaac ccctccttgg ccaaggccag ggttgcctgc gggagccagg tgtccgctct     180

ccacaccttt cacagcccca gccctcagag caacctcagc ccagcccagc ccagctccag     240

ctccagctcc agcccgggcc ccatc atg gcc aag gac ttt caa gat atc cag       292
                            Met Ala Lys Asp Phe Gln Asp Ile Gln
                            1               5

cag ctg agc tcg gag gaa aat gac cat cct ttc cat caa ggt gag ggg       340
Gln Leu Ser Ser Glu Glu Asn Asp His Pro Phe His Gln Gly Glu Gly
10                  15                  20                  25

cca ggc act cgc agg ctg aat ccc agg aga gga aat cca ttt ttg aaa       388
Pro Gly Thr Arg Arg Leu Asn Pro Arg Arg Gly Asn Pro Phe Leu Lys
                30                  35                  40

ggg cca cct cct gcc cag ccc ctg gca cag cgt ctc tgc tcc atg gtc       436
Gly Pro Pro Pro Ala Gln Pro Leu Ala Gln Arg Leu Cys Ser Met Val
            45                  50                  55

tgc ttc agt ctg ctt gcc ctg agc ttc aac atc ctg ctg ctg gtg gtc       484
Cys Phe Ser Leu Leu Ala Leu Ser Phe Asn Ile Leu Leu Leu Val Val
        60                  65                  70

atc tgt gtg act ggg tcc caa agt gag ggt cac aga ggt gca cag ctg       532
Ile Cys Val Thr Gly Ser Gln Ser Glu Gly His Arg Gly Ala Gln Leu

75                  80                  85

caa gcc gag ctg cgg agc ctg aag gaa gct ttc agc aac ttc tcc tcg      580
Gln Ala Glu Leu Arg Ser Leu Lys Glu Ala Phe Ser Asn Phe Ser Ser
90                  95                  100                 105

agc acc ctg acg gag gtc cag gca atc agc acc cac gga ggc agc gtg      628
Ser Thr Leu Thr Glu Val Gln Ala Ile Ser Thr His Gly Gly Ser Val
                110                 115                 120

ggt gac aag atc aca tcc cta gga gcc aag ctg gag aaa cag cag cag      676
Gly Asp Lys Ile Thr Ser Leu Gly Ala Lys Leu Glu Lys Gln Gln Gln
            125                 130                 135

gac ctg aaa gca gat cac gat gcc ctg ctc ttc cat ctg aag cac ttc      724
Asp Leu Lys Ala Asp His Asp Ala Leu Leu Phe His Leu Lys His Phe
        140                 145                 150

ccc gtg gac ctg cgc ttc gtg gcc tgc cag atg gag ctc ctc cac agc      772
Pro Val Asp Leu Arg Phe Val Ala Cys Gln Met Glu Leu Leu His Ser
    155                 160                 165

aac ggc tcc caa agg acc tgc tgc ccc gtc aac tgg gtg gag cac caa      820
Asn Gly Ser Gln Arg Thr Cys Cys Pro Val Asn Trp Val Glu His Gln
170                 175                 180                 185

ggc agc tgc tac tgg ttc tct cac tcc ggg aag gcc tgg gct gag gcg      868
Gly Ser Cys Tyr Trp Phe Ser His Ser Gly Lys Ala Trp Ala Glu Ala
                190                 195                 200

gag aag tac tgc cag ctg gag aac gca cac ctg gtg gtc atc aac tcc      916
Glu Lys Tyr Cys Gln Leu Glu Asn Ala His Leu Val Val Ile Asn Ser
            205                 210                 215

tgg gag gag cag aaa ttc att gta caa cac acg aac ccc ttc aat acc      964
Trp Glu Glu Gln Lys Phe Ile Val Gln His Thr Asn Pro Phe Asn Thr
        220                 225                 230

tgg ata ggt ctc acg gac agt gat ggc tct tgg aaa tgg gtg gat ggc      1012
Trp Ile Gly Leu Thr Asp Ser Asp Gly Ser Trp Lys Trp Val Asp Gly
    235                 240                 245

aca gac tat agg cac aac tac aag aac tgg gct gtc act cag cca gat      1060
Thr Asp Tyr Arg His Asn Tyr Lys Asn Trp Ala Val Thr Gln Pro Asp
250                 255                 260                 265

aat tgg cac ggg cac gag ctg ggt gga agt gaa gac tgt gtt gaa gtc      1108
Asn Trp His Gly His Glu Leu Gly Gly Ser Glu Asp Cys Val Glu Val
                270                 275                 280

cag ccg gat ggc cgc tgg aac gat gac ttc tgc ctg cag gtg tac cgc      1156
Gln Pro Asp Gly Arg Trp Asn Asp Asp Phe Cys Leu Gln Val Tyr Arg
            285                 290                 295

tgg gtg tgt gag aaa agg cgg aat gcc acc ggc gag gtg gcc tga          1201
Trp Val Cys Glu Lys Arg Arg Asn Ala Thr Gly Glu Val Ala
        300                 305                 310

ccccagcaca cctctggcta acccataccc cacacctgcc cagctctggc ttctctgttg    1261

aggattttga ggaaaggaag aaacactgag acaggggtat ggggaagagc tgagcaaaga    1321

gagaaaggag gtagtttaag agtccctgac cctggaggac tgagatccca cctccttctg    1381

taattcattg taattattat aatcgtcagc ctcttcaatg gcgtaggaaa gaagaaacaa    1441

atgcttgaat ctc                                                       1454

<210>  24
<211>  311
<212>  PRT
<213>  human

<400>  24

Met Ala Lys Asp Phe Gln Asp Ile Gln Gln Leu Ser Ser Glu Glu Asn
1               5                   10                  15

Asp His Pro Phe His Gln Gly Glu Gly Pro Gly Thr Arg Arg Leu Asn
            20                  25                  30

Pro Arg Arg Gly Asn Pro Phe Leu Lys Gly Pro Pro Pro Ala Gln Pro
        35                  40                  45

Leu Ala Gln Arg Leu Cys Ser Met Val Cys Phe Ser Leu Leu Ala Leu
    50                  55                  60

Ser Phe Asn Ile Leu Leu Leu Val Val Ile Cys Val Thr Gly Ser Gln
65                  70                  75                  80

Ser Glu Gly His Arg Gly Ala Gln Leu Gln Ala Glu Leu Arg Ser Leu
                85                  90                  95

Lys Glu Ala Phe Ser Asn Phe Ser Ser Ser Thr Leu Thr Glu Val Gln
            100                 105                 110

Ala Ile Ser Thr His Gly Gly Ser Val Gly Asp Lys Ile Thr Ser Leu
        115                 120                 125

Gly Ala Lys Leu Glu Lys Gln Gln Gln Asp Leu Lys Ala Asp His Asp
    130                 135                 140

Ala Leu Leu Phe His Leu Lys His Phe Pro Val Asp Leu Arg Phe Val
145                 150                 155                 160

Ala Cys Gln Met Glu Leu Leu His Ser Asn Gly Ser Gln Arg Thr Cys
                165                 170                 175

Cys Pro Val Asn Trp Val Glu His Gln Gly Ser Cys Tyr Trp Phe Ser
            180                 185                 190

His Ser Gly Lys Ala Trp Ala Glu Ala Glu Lys Tyr Cys Gln Leu Glu
        195                 200                 205

Asn Ala His Leu Val Val Ile Asn Ser Trp Glu Glu Gln Lys Phe Ile
    210                 215                 220

Val Gln His Thr Asn Pro Phe Asn Thr Trp Ile Gly Leu Thr Asp Ser
225                 230                 235                 240

Asp Gly Ser Trp Lys Trp Val Asp Gly Thr Asp Tyr Arg His Asn Tyr
                245                 250                 255

Lys Asn Trp Ala Val Thr Gln Pro Asp Asn Trp His Gly His Glu Leu
            260                 265                 270

Gly Gly Ser Glu Asp Cys Val Glu Val Gln Pro Asp Gly Arg Trp Asn
        275                 280                 285

Asp Asp Phe Cys Leu Gln Val Tyr Arg Trp Val Cys Glu Lys Arg Arg
    290                 295                 300

Asn Ala Thr Gly Glu Val Ala
305                 310

<210> 25
<211> 1382
<212> DNA
<213> human

<220>
<221> CDS
<222> (266)..(1129)

<400> 25
ccaggccagg aggtgccagg ttagagcttg gacactcaga ccctccagtg caggccttgc 60

ccggaccagc ccctcctcca ctctcctctc ccctgtgagc tccacctgcc ccagttctcc 120

tggctttaac cctccttggg ccaaggccag ggttgcctgc gggagccagg tgtccgctct 180

ccacaccttt cacagcccca gccctcagag caacctcagc ccagcccagc ccagctccag 240

ctccagctcc agcccgggcc ccatc atg gcc aag gac ttt caa gat atc cag 292
Met Ala Lys Asp Phe Gln Asp Ile Gln
1               5

cag ctg agc tcg gag gaa aat gac cat cct ttc cat caa ggg cca cct 340
Gln Leu Ser Ser Glu Glu Asn Asp His Pro Phe His Gln Gly Pro Pro
10                  15                  20                  25

cct gcc cag ccc ctg gca cag cgt ctc tgc tcc atg gtc tgc ttc agt 388
Pro Ala Gln Pro Leu Ala Gln Arg Leu Cys Ser Met Val Cys Phe Ser
                30                  35                  40

ctg ctt gcc ctg agc ttc aac atc ctg ctg ctg gtg gtc atc tgt gtg 436
Leu Leu Ala Leu Ser Phe Asn Ile Leu Leu Leu Val Val Ile Cys Val
            45                  50                  55

act ggg tcc caa agt gca cag ctg caa gcc gag ctg cgg agc ctg aag 484
Thr Gly Ser Gln Ser Ala Gln Leu Gln Ala Glu Leu Arg Ser Leu Lys
        60                  65                  70

gaa gct ttc agc aac ttc tcc tcg agc acc ctg acg gag gtc cag gca 532
Glu Ala Phe Ser Asn Phe Ser Ser Ser Thr Leu Thr Glu Val Gln Ala
    75                  80                  85

atc agc acc cac gga ggc agc gtg ggt gac aag atc aca tcc cta gga 580
Ile Ser Thr His Gly Gly Ser Val Gly Asp Lys Ile Thr Ser Leu Gly
90                  95                  100                 105

gcc aag ctg gag aaa cag cag cag gac ctg aaa gca gat cac gat gcc 628

Ala Lys Leu Glu Lys Gln Gln Gln Asp Leu Lys Ala Asp His Asp Ala
                110                 115                 120

ctg ctc ttc cat ctg aag cac ttc ccc gtg gac ctg cgc ttc gtg gcc      676
Leu Leu Phe His Leu Lys His Phe Pro Val Asp Leu Arg Phe Val Ala
            125                 130                 135

tgc cag atg gag ctc ctc cac agc aac ggc tcc caa agg acc tgc tgc      724
Cys Gln Met Glu Leu Leu His Ser Asn Gly Ser Gln Arg Thr Cys Cys
        140                 145                 150

ccc gtc aac tgg gtg gag cac caa ggc agc tgc tac tgg ttc tct cac      772
Pro Val Asn Trp Val Glu His Gln Gly Ser Cys Tyr Trp Phe Ser His
    155                 160                 165

tcc ggg aag gcc tgg gct gag gcg gag aag tac tgc cag ctg gag aac      820
Ser Gly Lys Ala Trp Ala Glu Ala Glu Lys Tyr Cys Gln Leu Glu Asn
170                 175                 180                 185

gca cac ctg gtg gtc atc aac tcc tgg gag gag cag aaa ttc att gta      868
Ala His Leu Val Val Ile Asn Ser Trp Glu Glu Gln Lys Phe Ile Val
                190                 195                 200

caa cac acg aac ccc ttc aat acc tgg ata ggt ctc acg gac agt gat      916
Gln His Thr Asn Pro Phe Asn Thr Trp Ile Gly Leu Thr Asp Ser Asp
            205                 210                 215

ggc tct tgg aaa tgg gtg gat ggc aca gac tat agg cac aac tac aag      964
Gly Ser Trp Lys Trp Val Asp Gly Thr Asp Tyr Arg His Asn Tyr Lys
        220                 225                 230

aac tgg gct gtc act cag cca gat aat tgg cac ggg cac gag ctg ggt      1012
Asn Trp Ala Val Thr Gln Pro Asp Asn Trp His Gly His Glu Leu Gly
    235                 240                 245

gga agt gaa gac tgt gtt gaa gtc cag ccg gat ggc cgc tgg aac gat      1060
Gly Ser Glu Asp Cys Val Glu Val Gln Pro Asp Gly Arg Trp Asn Asp
250                 255                 260                 265

gac ttc tgc ctg cag gtg tac cgc tgg gtg tgt gag aaa agg cgg aat      1108
Asp Phe Cys Leu Gln Val Tyr Arg Trp Val Cys Glu Lys Arg Arg Asn
                270                 275                 280

gcc acc ggc gag gtg gcc tga ccccagcaca cctctggcta acccataccc      1159
Ala Thr Gly Glu Val Ala
            285

cacacctgcc cagctctggc ttctctgttg aggattttga ggaaaggaag aaacactgag      1219

acaggggtat ggggaagagc tgagcaaaga gagaaaggag gtagtttaag agtccctgac      1279

cctggaggac tgagatccca cctccttctg taattcattg taattattat aatcgtcagc      1339

ctcttcaatg gcgtaggaaa gaagaaacaa atgcttgaat ctc      1382

<210>  26
<211>  287
<212>  PRT
<213>  human

<400>  26

Met Ala Lys Asp Phe Gln Asp Ile Gln Gln Leu Ser Ser Glu Glu Asn
1               5                   10                  15

Asp His Pro Phe His Gln Gly Pro Pro Pro Ala Gln Pro Leu Ala Gln

20                  25                  30

Arg Leu Cys Ser Met Val Cys Phe Ser Leu Leu Ala Leu Ser Phe Asn
        35                  40                  45

Ile Leu Leu Leu Val Val Ile Cys Val Thr Gly Ser Gln Ser Ala Gln
    50                  55                  60

Leu Gln Ala Glu Leu Arg Ser Leu Lys Glu Ala Phe Ser Asn Phe Ser
65                  70                  75                  80

Ser Ser Thr Leu Thr Glu Val Gln Ala Ile Ser Thr His Gly Gly Ser
                85                  90                  95

Val Gly Asp Lys Ile Thr Ser Leu Gly Ala Lys Leu Glu Lys Gln Gln
            100                 105                 110

Gln Asp Leu Lys Ala Asp His Asp Ala Leu Leu Phe His Leu Lys His
        115                 120                 125

Phe Pro Val Asp Leu Arg Phe Val Ala Cys Gln Met Glu Leu Leu His
    130                 135                 140

Ser Asn Gly Ser Gln Arg Thr Cys Cys Pro Val Asn Trp Val Glu His
145                 150                 155                 160

Gln Gly Ser Cys Tyr Trp Phe Ser His Ser Gly Lys Ala Trp Ala Glu
                165                 170                 175

Ala Glu Lys Tyr Cys Gln Leu Glu Asn Ala His Leu Val Val Ile Asn
            180                 185                 190

Ser Trp Glu Glu Gln Lys Phe Ile Val Gln His Thr Asn Pro Phe Asn
        195                 200                 205

Thr Trp Ile Gly Leu Thr Asp Ser Asp Gly Ser Trp Lys Trp Val Asp
    210                 215                 220

Gly Thr Asp Tyr Arg His Asn Tyr Lys Asn Trp Ala Val Thr Gln Pro
225                 230                 235                 240

Asp Asn Trp His Gly His Glu Leu Gly Gly Ser Glu Asp Cys Val Glu
                245                 250                 255

Val Gln Pro Asp Gly Arg Trp Asn Asp Asp Phe Cys Leu Gln Val Tyr
            260                 265                 270

Arg Trp Val Cys Glu Lys Arg Arg Asn Ala Thr Gly Glu Val Ala
        275                 280                 285

<210>  27

<211>  1202
<212>  DNA
<213>  human

<220>
<221>  CDS
<222>  (71)..(949)

<400>  27
ccccagccct cagagcaacc tcagcccagc ccagcccagc tccagctcca gctccagccc      60

gggccccatc atg gcc aag gac ttt caa gat atc cag cag ctg agc tcg      109
           Met Ala Lys Asp Phe Gln Asp Ile Gln Gln Leu Ser Ser
           1               5                   10

gag gaa aat gac cat cct ttc cat caa ggg cca cct cct gcc cag ccc      157
Glu Glu Asn Asp His Pro Phe His Gln Gly Pro Pro Pro Ala Gln Pro
    15                  20                  25

ctg gca cag cgt ctc tgc tcc atg gtc tgc ttc agt ctg ctt gcc ctg      205
Leu Ala Gln Arg Leu Cys Ser Met Val Cys Phe Ser Leu Leu Ala Leu
30                  35                  40                  45

agc ttc aac atc ctg ctg ctg gtg gtc atc tgt gtg act ggg tcc caa      253
Ser Phe Asn Ile Leu Leu Leu Val Val Ile Cys Val Thr Gly Ser Gln
                50                  55                  60

agt gag ggt cac aga ggt gca cag ctg caa gcc gag ctg cgg agc ctg      301
Ser Glu Gly His Arg Gly Ala Gln Leu Gln Ala Glu Leu Arg Ser Leu
            65                  70                  75

aag gaa gct ttc agc aac ttc tcc tcg agc acc ctg acg gag gtc cag      349
Lys Glu Ala Phe Ser Asn Phe Ser Ser Ser Thr Leu Thr Glu Val Gln
        80                  85                  90

gca atc agc acc cac gga ggc agc gtg ggt gac aag atc aca tcc cta      397
Ala Ile Ser Thr His Gly Gly Ser Val Gly Asp Lys Ile Thr Ser Leu
    95                  100                 105

gga gcc aag ctg gag aaa cag cag cag gac ctg aaa gca gat cac gat      445
Gly Ala Lys Leu Glu Lys Gln Gln Gln Asp Leu Lys Ala Asp His Asp
110                 115                 120                 125

gcc ctg ctc ttc cat ctg aag cac ttc ccc gtg gac ctg cgc ttc gtg      493
Ala Leu Leu Phe His Leu Lys His Phe Pro Val Asp Leu Arg Phe Val
                130                 135                 140

gcc tgc cag atg gag ctc ctc cac agc aac ggc tcc caa agg acc tgc      541
Ala Cys Gln Met Glu Leu Leu His Ser Asn Gly Ser Gln Arg Thr Cys
            145                 150                 155

tgc ccc gtc aac tgg gtg gag cac caa ggc agc tgc tac tgg ttc tct      589
Cys Pro Val Asn Trp Val Glu His Gln Gly Ser Cys Tyr Trp Phe Ser
        160                 165                 170

cac tcc ggg aag gcc tgg gct gag gcg gag aag tac tgc cag ctg gag      637
His Ser Gly Lys Ala Trp Ala Glu Ala Glu Lys Tyr Cys Gln Leu Glu
    175                 180                 185

aac gca cac ctg gtg gtc atc aac tcc tgg gag gag cag aaa ttc att      685
Asn Ala His Leu Val Val Ile Asn Ser Trp Glu Glu Gln Lys Phe Ile
190                 195                 200                 205

gta caa cac acg aac ccc ttc aat acc tgg ata ggt ctc acg gac agt      733
Val Gln His Thr Asn Pro Phe Asn Thr Trp Ile Gly Leu Thr Asp Ser
                210                 215                 220

gat ggc tct tgg aaa tgg gtg gat ggc aca gac tat agg cac aac tac     781
Asp Gly Ser Trp Lys Trp Val Asp Gly Thr Asp Tyr Arg His Asn Tyr
            225                 230                 235

aag aac tgg gct gtc act cag cca gat aat tgg cac ggg cac gag ctg     829
Lys Asn Trp Ala Val Thr Gln Pro Asp Asn Trp His Gly His Glu Leu
        240                 245                 250

ggt gga agt gaa gac tgt gtt gaa gtc cag ccg gat ggc cgc tgg aac     877
Gly Gly Ser Glu Asp Cys Val Glu Val Gln Pro Asp Gly Arg Trp Asn
    255                 260                 265

gat gac ttc tgc ctg cag gtg tac cgc tgg gtg tgt gag aaa agg cgg     925
Asp Asp Phe Cys Leu Gln Val Tyr Arg Trp Val Cys Glu Lys Arg Arg
270                 275                 280                 285

aat gcc acc ggc gag gtg gcc tga ccccagcaca cctctggcta acccataccc     979
Asn Ala Thr Gly Glu Val Ala
                290

cacacctgcc cagctctggc ttctctgttg aggattttga ggaaaggaag aaacactgag     1039

acaggggtat ggggaagagc tgagcaaaga gagaaaggag gtagtttaag agtccctgac     1099

cctggaggac tgagatccca cctccttctg taattcattg taattattat aatcgtcagc     1159

ctcttcaatg gcgtaggaaa gaagaaacaa atgcttgaat ctc     1202

<210>  28
<211>  292
<212>  PRT
<213>  human

<400>  28

Met Ala Lys Asp Phe Gln Asp Ile Gln Gln Leu Ser Ser Glu Glu Asn
1               5                   10                  15

Asp His Pro Phe His Gln Gly Pro Pro Pro Ala Gln Pro Leu Ala Gln
            20                  25                  30

Arg Leu Cys Ser Met Val Cys Phe Ser Leu Leu Ala Leu Ser Phe Asn
        35                  40                  45

Ile Leu Leu Leu Val Val Ile Cys Val Thr Gly Ser Gln Ser Glu Gly
    50                  55                  60

His Arg Gly Ala Gln Leu Gln Ala Glu Leu Arg Ser Leu Lys Glu Ala
65                  70                  75                  80

Phe Ser Asn Phe Ser Ser Ser Thr Leu Thr Glu Val Gln Ala Ile Ser
                85                  90                  95

Thr His Gly Gly Ser Val Gly Asp Lys Ile Thr Ser Leu Gly Ala Lys
            100                 105                 110

Leu Glu Lys Gln Gln Gln Asp Leu Lys Ala Asp His Asp Ala Leu Leu
        115                 120                 125

Phe His Leu Lys His Phe Pro Val Asp Leu Arg Phe Val Ala Cys Gln
    130                 135                 140

Met Glu Leu Leu His Ser Asn Gly Ser Gln Arg Thr Cys Cys Pro Val
145                 150                 155                 160

Asn Trp Val Glu His Gln Gly Ser Cys Tyr Trp Phe Ser His Ser Gly
                165                 170                 175

Lys Ala Trp Ala Glu Ala Glu Lys Tyr Cys Gln Leu Glu Asn Ala His
            180                 185                 190

Leu Val Val Ile Asn Ser Trp Glu Glu Gln Lys Phe Ile Val Gln His
        195                 200                 205

Thr Asn Pro Phe Asn Thr Trp Ile Gly Leu Thr Asp Ser Asp Gly Ser
    210                 215                 220

Trp Lys Trp Val Asp Gly Thr Asp Tyr Arg His Asn Tyr Lys Asn Trp
225                 230                 235                 240

Ala Val Thr Gln Pro Asp Asn Trp His Gly His Glu Leu Gly Gly Ser
                245                 250                 255

Glu Asp Cys Val Glu Val Gln Pro Asp Gly Arg Trp Asn Asp Asp Phe
            260                 265                 270

Cys Leu Gln Val Tyr Arg Trp Val Cys Glu Lys Arg Arg Asn Ala Thr
        275                 280                 285

Gly Glu Val Ala
    290

<210>   29
<211>   1542
<212>   DNA
<213>   human

<220>
<221>   CDS
<222>   (354)..(1289)

<400>   29
ccaggccagg aggtgccagg ttagagcttg gacactcaga ccctccagtg caggccttgc      60

ccggaccagc ccctcctcca ctctcctctc ccctgtgagc tccacctgcc ccagttctcc     120

tggctttaac ccctccttgg ccaaggccag ggttgcctgc gggagccagg tgtccgctct     180

ccacaccttt cacagatcct acccaggcca ccaccccgaa cccctccgac cctgcgttcc     240

atcccaccat caacctccac gtgaatctgc acctctgccc cagccccagc cctcagagca     300

acctcagccc agcccagccc agctccagct ccagctccag cccgggcccc atc atg        356
                                                           Met
                                                           1

gcc aag gac ttt caa gat atc cag cag ctg agc tcg gag gaa aat gac 404
Ala Lys Asp Phe Gln Asp Ile Gln Gln Leu Ser Ser Glu Glu Asn Asp
            5                   10                  15

cat cct ttc cat caa ggt gag ggg cca ggc act cgc agg ctg aat ccc 452
His Pro Phe His Gln Gly Glu Gly Pro Gly Thr Arg Arg Leu Asn Pro
        20                  25                  30

agg aga gga aat cca ttt ttg aaa ggg cca cct cct gcc cag ccc ctg 500
Arg Arg Gly Asn Pro Phe Leu Lys Gly Pro Pro Pro Ala Gln Pro Leu
    35                  40                  45

gca cag cgt ctc tgc tcc atg gtc tgc ttc agt ctg ctt gcc ctg agc 548
Ala Gln Arg Leu Cys Ser Met Val Cys Phe Ser Leu Leu Ala Leu Ser
50                  55                  60                  65

ttc aac atc ctg ctg ctg gtg gtc atc tgt gtg act ggg tcc caa agt 596
Phe Asn Ile Leu Leu Leu Val Val Ile Cys Val Thr Gly Ser Gln Ser
                70                  75                  80

gag ggt cac aga ggt gca cag ctg caa gcc gag ctg cgg agc ctg aag 644
Glu Gly His Arg Gly Ala Gln Leu Gln Ala Glu Leu Arg Ser Leu Lys
            85                  90                  95

gaa gct ttc agc aac ttc tcc tcg agc acc ctg acg gag gtc cag gca 692
Glu Ala Phe Ser Asn Phe Ser Ser Ser Thr Leu Thr Glu Val Gln Ala
        100                 105                 110

atc agc acc cac gga ggc agc gtg ggt gac aag atc aca tcc cta gga 740
Ile Ser Thr His Gly Gly Ser Val Gly Asp Lys Ile Thr Ser Leu Gly
    115                 120                 125

gcc aag ctg gag aaa cag cag cag gac ctg aaa gca gat cac gat gcc 788
Ala Lys Leu Glu Lys Gln Gln Gln Asp Leu Lys Ala Asp His Asp Ala
130                 135                 140                 145

ctg ctc ttc cat ctg aag cac ttc ccc gtg gac ctg cgc ttc gtg gcc 836
Leu Leu Phe His Leu Lys His Phe Pro Val Asp Leu Arg Phe Val Ala
                150                 155                 160

tgc cag atg gag ctc ctc cac agc aac ggc tcc caa agg acc tgc tgc 884
Cys Gln Met Glu Leu Leu His Ser Asn Gly Ser Gln Arg Thr Cys Cys
            165                 170                 175

ccc gtc aac tgg gtg gag cac caa ggc agc tgc tac tgg ttc tct cac 932
Pro Val Asn Trp Val Glu His Gln Gly Ser Cys Tyr Trp Phe Ser His
        180                 185                 190

tcc ggg aag gcc tgg gct gag gcg gag aag tac tgc cag ctg gag aac 980
Ser Gly Lys Ala Trp Ala Glu Ala Glu Lys Tyr Cys Gln Leu Glu Asn
    195                 200                 205

gca cac ctg gtg gtc atc aac tcc tgg gag gag cag aaa ttc att gta 1028
Ala His Leu Val Val Ile Asn Ser Trp Glu Glu Gln Lys Phe Ile Val
210                 215                 220                 225

caa cac acg aac ccc ttc aat acc tgg ata ggt ctc acg gac agt gat 1076
Gln His Thr Asn Pro Phe Asn Thr Trp Ile Gly Leu Thr Asp Ser Asp
                230                 235                 240

ggc tct tgg aaa tgg gtg gat ggc aca gac tat agg cac aac tac aag 1124
Gly Ser Trp Lys Trp Val Asp Gly Thr Asp Tyr Arg His Asn Tyr Lys
            245                 250                 255

aac tgg gct gtc act cag cca gat aat tgg cac ggg cac gag ctg ggt 1172
Asn Trp Ala Val Thr Gln Pro Asp Asn Trp His Gly His Glu Leu Gly
        260                 265                 270

gga agt gaa gac tgt gtt gaa gtc cag ccg gat ggc cgc tgg aac gat 1220
Gly Ser Glu Asp Cys Val Glu Val Gln Pro Asp Gly Arg Trp Asn Asp
    275                 280                 285

gac ttc tgc ctg cag gtg tac cgc tgg gtg tgt gag aaa agg cgg aat 1268
Asp Phe Cys Leu Gln Val Tyr Arg Trp Val Cys Glu Lys Arg Arg Asn
290                 295                 300                 305

gcc acc ggc gag gtg gcc tga ccccagcaca cctctggcta acccataccc 1319
Ala Thr Gly Glu Val Ala
                310

cacacctgcc cagctctggc ttctctgttg aggattttga ggaaaggaag aaacactgag 1379

acaggggtat ggggaagagc tgagcaaaga gagaaaggag gtagtttaag agtccctgac 1439

cctggaggac tgagatccca cctccttctg taattcattg taattattat aatcgtcagc 1499

ctcttcaatg gcgtaggaaa gaagaaacaa atgcttgaat ctc 1542

<210> 30
<211> 311
<212> PRT
<213> human

<400> 30

Met Ala Lys Asp Phe Gln Asp Ile Gln Gln Leu Ser Ser Glu Glu Asn
1               5                   10                  15

Asp His Pro Phe His Gln Gly Glu Gly Pro Gly Thr Arg Arg Leu Asn
            20                  25                  30

Pro Arg Arg Gly Asn Pro Phe Leu Lys Gly Pro Pro Pro Ala Gln Pro
        35                  40                  45

Leu Ala Gln Arg Leu Cys Ser Met Val Cys Phe Ser Leu Leu Ala Leu
    50                  55                  60

Ser Phe Asn Ile Leu Leu Leu Val Val Ile Cys Val Thr Gly Ser Gln
65                  70                  75                  80

Ser Glu Gly His Arg Gly Ala Gln Leu Gln Ala Glu Leu Arg Ser Leu
                85                  90                  95

Lys Glu Ala Phe Ser Asn Phe Ser Ser Ser Thr Leu Thr Glu Val Gln
            100                 105                 110

Ala Ile Ser Thr His Gly Gly Ser Val Gly Asp Lys Ile Thr Ser Leu
        115                 120                 125

Gly Ala Lys Leu Glu Lys Gln Gln Gln Asp Leu Lys Ala Asp His Asp
    130                 135                 140

Ala Leu Leu Phe His Leu Lys His Phe Pro Val Asp Leu Arg Phe Val
145                 150                 155                 160

Ala Cys Gln Met Glu Leu Leu His Ser Asn Gly Ser Gln Arg Thr Cys
                165                 170                 175

Cys Pro Val Asn Trp Val Glu His Gln Gly Ser Cys Tyr Trp Phe Ser
            180                 185                 190

His Ser Gly Lys Ala Trp Ala Glu Ala Glu Lys Tyr Cys Gln Leu Glu
        195                 200                 205

Asn Ala His Leu Val Val Ile Asn Ser Trp Glu Glu Gln Lys Phe Ile
    210                 215                 220

Val Gln His Thr Asn Pro Phe Asn Thr Trp Ile Gly Leu Thr Asp Ser
225                 230                 235                 240

Asp Gly Ser Trp Lys Trp Val Asp Gly Thr Asp Tyr Arg His Asn Tyr
                245                 250                 255

Lys Asn Trp Ala Val Thr Gln Pro Asp Asn Trp His Gly His Glu Leu
            260                 265                 270

Gly Gly Ser Glu Asp Cys Val Glu Val Gln Pro Asp Gly Arg Trp Asn
        275                 280                 285

Asp Asp Phe Cys Leu Gln Val Tyr Arg Trp Val Cys Glu Lys Arg Arg
    290                 295                 300

Asn Ala Thr Gly Glu Val Ala
305                 310

<210>  31
<211>  2800
<212>  DNA
<213>  human

<220>
<221>  CDS
<222>  (634)..(1125)

<400>  31
attatcaaca tagtgaaggg gagggtttca aagagcaaga aggggttgtt agtcacacgg      60

agagagaaga aaggagatta gcagggtgac tttcaccctg caacagtcac gctggagtac     120

tctggtgaag gatcaagaat attaccagtt ttctgatgaa gaaaggaata tatttggtca     180

ccagcaatct gcctttgacc atcctcaaag ccaagttttt catcgctgtc tagaaaaata     240

cttcattcag aggacacata tgtcctcttt gaatgtctcc tttttgggag cagtaggaga     300

aagaaacttc aagatgtaga aagaattaat agtgtaacgg aaagaaagaa ggcaaaagga     360

agtaaatgtg gaggccatag aaatagtaat aagaataaag aagaaaaaca ggaaagaaga     420

gggaaaagcc atagaaaata acagccagag cgagtaggag gagacagaga gaacgagaga     480

gagaaaagct gataaattcc tgagaaaagt ttctcttctt aagaattcta ggtcaagaag 540

taaaatatgg cacagagcat tgaaaggagg caacggatgc ccagtgcaag attctgaaga 600

agcaggaatt cagcccgatg ggagtccgag ttc atg tcg tgg cgg cct cag ccc 654
Met Ser Trp Arg Pro Gln Pro
1               5

tgc tgt att tca tcc tgt tgc ctg acc aca gac tgg gta cat ctc tgg 702
Cys Cys Ile Ser Ser Cys Cys Leu Thr Thr Asp Trp Val His Leu Trp
        10                  15                  20

tat ata tgg ttg cta gtg gta att ggc gcg ctg ctt ctc ctg tgt ggc 750
Tyr Ile Trp Leu Leu Val Val Ile Gly Ala Leu Leu Leu Leu Cys Gly
    25                  30                  35

ctg acg tcc ctg tgc ttc cgc tgc tgc tgt ctg agc cgc cag caa aat 798
Leu Thr Ser Leu Cys Phe Arg Cys Cys Cys Leu Ser Arg Gln Gln Asn
40                  45                  50                  55

ggg gaa gat ggg ggc cca cca ccc tgt gaa gtg acc gtc att gct ttc 846
Gly Glu Asp Gly Gly Pro Pro Pro Cys Glu Val Thr Val Ile Ala Phe
                60                  65                  70

gat cac gac agc act ctc cag agc act atc aca tct ctg cag tcg gtg 894
Asp His Asp Ser Thr Leu Gln Ser Thr Ile Thr Ser Leu Gln Ser Val
            75                  80                  85

ttt ggc cct gca gct cgg agg atc ctg gct gtg gct cac tcc cac agc 942
Phe Gly Pro Ala Ala Arg Arg Ile Leu Ala Val Ala His Ser His Ser
        90                  95                  100

tcc ctg ggc cag ctg ccc tcc tct ttg gac acc ctc cca ggg tat gaa 990
Ser Leu Gly Gln Leu Pro Ser Ser Leu Asp Thr Leu Pro Gly Tyr Glu
    105                 110                 115

gaa gct ctt cac atg agt cgc ttc aca gta gcc atg tgc ggg cag aaa 1038
Glu Ala Leu His Met Ser Arg Phe Thr Val Ala Met Cys Gly Gln Lys
120                 125                 130                 135

gca cct gat cta ccc cca gta cct gaa gaa aag cag ctg cct cca aca 1086
Ala Pro Asp Leu Pro Pro Val Pro Glu Glu Lys Gln Leu Pro Pro Thr
                140                 145                 150

gag aag gag tcg act cga ata gtt gac tct tgg aac tga tgagagctgt 1135
Glu Lys Glu Ser Thr Arg Ile Val Asp Ser Trp Asn
            155                 160

cattttataa ataggagtgg agtgatgtcc agagtctgtg ggaaaatgga acacatactt 1195

ttctaaccct cagaagtttt aagatggcat ctaacaccat cattctatgg gaaagatggt 1255

tcttactctt cgttcacagg cctttatatc ttccgataca gaatgctcta attgggaact 1315

ctaattttgt atccaatggc caaaatctgc aagtaatctc tagccacact gattactact 1375

aaaccaggaa agcatcaagg tgtcttgaat tcctttaact attgagtgca tatagaattc 1435

ctgtacccac atgatactgc aagttgtgtc tctctctgtc agcgaatcca ctgcggttaa 1495

ctggaaaaga aagacaacag tgtcagcaca gccatcgaca ttaatgcact gaatgcatgc 1555

atctttcctc ctgagacagc aatcgatttt acaccgaatg acaatgatca tcttagacag 1615

cacaacatac ccactcggat atctaaaagc tagggatggc attgctgata tgggcaaaga 1675

gaacacagta tagtatttaa gtgccaaata tcagtctttc tttctctctg gtcctacccc 1735

tcagcagtat gaaaaactcc atactgtgca gtcacagttg gattaattct tcagttcctc 1795

cgcactgcaa acacatatat gtgcgcacat gcatgtatac ctgcaccctg ttttaactct 1855

aaaggaatag tgttgcttta cttctttcct gttttgcctg gaccacttaa agccacaaca 1915

cctctatagt gacacacgct agtctctagt ggtggccctc actgccacct agaggagcca 1975

tggtggaaaa cacactctct cctttgagcc tatctgcaca tctctcgagt tcttggagca 2035

aaaactaaat gctgaactaa gcctggttga gatgcttccc atggaccatg ccgcagcaca 2095

gtgctaatct atccacaaaa cataccacct cccaaagtat tattattgga aaatcgagga 2155

agtgacgcac atttagggaa aaactactca ccttagaaaa gtcactgaaa tccttttttt 2215

tttttttgag atggagtttt gctcttgtag cccaggctgg gatgcaatgg catggtctca 2275

gctcactgta acctccacct cccggattca agcaattctt ctgcctcagc ttcccgacta 2335

gctgggatta cagctgcctg ccaccgtgcc cagctaattt ttgtattttt agtggagagg 2395

gggtttcacc atgttggcca gtctggtcta gaactcctga cgtcaggtga tccgcccacc 2455

ttggcctccc aaagtgctgg aattagaggc ctgaccccct gctcctggcc tgaaatcttt 2515

aaagccgttt tttccctaaa aaatgggaaa taataacacc tcagaaggtt tttgtgaaga 2575

tcaaagaagc taaatatatg tggcatgatt tgtaaagtgt tatgcatatg tatgttattc 2635

ttcctactgt cttctaacct tcccttgcct gctatgactt atctgagagc catgttccca 2695

tttatctttt tgccaactat gttactgttg tcacacctga aatggctttg tttttatcaa 2755

taaatacttg ttgattgcgg taaacagcaa cagaaaaaaa aaaaa 2800

<210> 32
<211> 163
<212> PRT
<213> human

<400> 32

Met Ser Trp Arg Pro Gln Pro Cys Cys Ile Ser Ser Cys Cys Leu Thr
1               5                   10                  15

Thr Asp Trp Val His Leu Trp Tyr Ile Trp Leu Leu Val Val Ile Gly
            20                  25                  30

Ala Leu Leu Leu Leu Cys Gly Leu Thr Ser Leu Cys Phe Arg Cys Cys
        35                  40                  45

Cys Leu Ser Arg Gln Gln Asn Gly Glu Asp Gly Gly Pro Pro Pro Cys
    50                  55                  60

Glu Val Thr Val Ile Ala Phe Asp His Asp Ser Thr Leu Gln Ser Thr
65                  70                  75                  80

Ile Thr Ser Leu Gln Ser Val Phe Gly Pro Ala Ala Arg Arg Ile Leu
                85                  90                  95

Ala Val Ala His Ser His Ser Ser Leu Gly Gln Leu Pro Ser Ser Leu
            100                 105                 110

Asp Thr Leu Pro Gly Tyr Glu Glu Ala Leu His Met Ser Arg Phe Thr
        115                 120                 125

Val Ala Met Cys Gly Gln Lys Ala Pro Asp Leu Pro Pro Val Pro Glu
    130                 135                 140

Glu Lys Gln Leu Pro Pro Thr Glu Lys Glu Ser Thr Arg Ile Val Asp
145                 150                 155                 160

Ser Trp Asn

<210>  33
<211>  1395
<212>  DNA
<213>  human

<220>
<221>  CDS
<222>  (122)..(868)

<400>  33
gggccgccgg ctgccagccc agggcggggc ggagccctac ttctggccga ccgcgtaggc      60

ggtgcttgaa cttagggctg cttgtggctg ggcactcgcg cagaggccgg cccgacgagc     120

c atg gtt gct ggg agc gac gcg ggg cgg gcc ctg ggg gtc ctc agc gtg     169
  Met Val Ala Gly Ser Asp Ala Gly Arg Ala Leu Gly Val Leu Ser Val
  1               5                   10                  15

gtc tgc ctg ctg cac tgc ttt ggt ttc atc agc tgt ttt tcc caa caa       217
Val Cys Leu Leu His Cys Phe Gly Phe Ile Ser Cys Phe Ser Gln Gln
            20                  25                  30

ata tat ggt gtt gtg tat ggg aat gta act ttc cat gta cca agc aat       265
Ile Tyr Gly Val Val Tyr Gly Asn Val Thr Phe His Val Pro Ser Asn
        35                  40                  45

gtg cct tta aaa gag gtc cta tgg aaa aaa caa aag gat aaa gtt gca       313
Val Pro Leu Lys Glu Val Leu Trp Lys Lys Gln Lys Asp Lys Val Ala
    50                  55                  60

gaa ctg gaa aat tct gaa ttc aga gct ttc tca tct ttt aaa aat agg       361
Glu Leu Glu Asn Ser Glu Phe Arg Ala Phe Ser Ser Phe Lys Asn Arg
65                  70                  75                  80

gtt tat tta gac act gtg tca ggt agc ctc act atc tac aac tta aca       409
Val Tyr Leu Asp Thr Val Ser Gly Ser Leu Thr Ile Tyr Asn Leu Thr
                85                  90                  95

tca tca gat gaa gat gag tat gaa atg gaa tcg cca aat att act gat       457
Ser Ser Asp Glu Asp Glu Tyr Glu Met Glu Ser Pro Asn Ile Thr Asp
            100                 105                 110

acc atg aag ttc ttt ctt tat gtg ctt gag tct ctt cca tct ccc aca       505
Thr Met Lys Phe Phe Leu Tyr Val Leu Glu Ser Leu Pro Ser Pro Thr
        115                 120                 125

cta act tgt gca ttg act aat gga agc att gaa gtc caa tgc atg ata       553

Leu Thr Cys Ala Leu Thr Asn Gly Ser Ile Glu Val Gln Cys Met Ile
    130                 135                 140

cca gag cat tac aac agc cat cga gga ctt ata atg tac tca tgg gat      601
Pro Glu His Tyr Asn Ser His Arg Gly Leu Ile Met Tyr Ser Trp Asp
145                 150                 155                 160

tgt cct atg gag caa tgt aaa cgt aac tca acc agt ata tat ttt aag      649
Cys Pro Met Glu Gln Cys Lys Arg Asn Ser Thr Ser Ile Tyr Phe Lys
                165                 170                 175

atg gaa aat gat ctt cca caa aaa ata cag tgt act ctt agc aat cca      697
Met Glu Asn Asp Leu Pro Gln Lys Ile Gln Cys Thr Leu Ser Asn Pro
            180                 185                 190

tta ttt aat aca aca tca tca atc att ttg aca acc tgt atc cca agc      745
Leu Phe Asn Thr Thr Ser Ser Ile Ile Leu Thr Thr Cys Ile Pro Ser
        195                 200                 205

agc ggt cat tca aga cac aga tat gca ctt ata ccc ata cca tta gca      793
Ser Gly His Ser Arg His Arg Tyr Ala Leu Ile Pro Ile Pro Leu Ala
    210                 215                 220

gta att aca aca tgt att gtg ctg tat atg aat ggt att ctg aaa tgt      841
Val Ile Thr Thr Cys Ile Val Leu Tyr Met Asn Gly Ile Leu Lys Cys
225                 230                 235                 240

gac aga aaa cca gac aga acc aag taa gtacactgca ggctgtgcac            888
Asp Arg Lys Pro Asp Arg Thr Lys
                245

tctgtgccag aacaacagca gccctgctgt agatgcccat tacttgggaa gccagattgt    948

actcatctgt tggttgcctt gtacagtaat aagaagtaac atcagcagat atactaacca   1008

tagtgtatta agtaccaact gtgtgccatg cactgcacta gccactctct gcatatcgtt   1068

tcctggaacc ctctcaatga gccttgcagg aggagtatca taccccttgc ttagagacag   1128

cgcatatgag attcatagaa gtcaagtgag atatcaaagt cacgttgcta gtaagtgatg   1188

ggctgggatt taaatctagg tctttggact ccaagagtta tactgttaac cactacaata   1248

cactccctcc acctcccatc ccagaaaaag ttggatcagg gatagggttc acttcaggag   1308

ccagggtcag agctgggcct gtagtcacat ataaaagata ggacacaaac acaattagca   1368

gcagtgttgg tttaataaac cgccgta                                        1395

<210>  34
<211>  248
<212>  PRT
<213>  human

<400>  34

Met Val Ala Gly Ser Asp Ala Gly Arg Ala Leu Gly Val Leu Ser Val
1               5                   10                  15

Val Cys Leu Leu His Cys Phe Gly Phe Ile Ser Cys Phe Ser Gln Gln
            20                  25                  30

Ile Tyr Gly Val Val Tyr Gly Asn Val Thr Phe His Val Pro Ser Asn
        35                  40                  45

Val Pro Leu Lys Glu Val Leu Trp Lys Lys Gln Lys Asp Lys Val Ala
    50                  55                  60

Glu Leu Glu Asn Ser Glu Phe Arg Ala Phe Ser Ser Phe Lys Asn Arg
65                  70                  75                  80

Val Tyr Leu Asp Thr Val Ser Gly Ser Leu Thr Ile Tyr Asn Leu Thr
                85                  90                  95

Ser Ser Asp Glu Asp Glu Tyr Glu Met Glu Ser Pro Asn Ile Thr Asp
            100                 105                 110

Thr Met Lys Phe Phe Leu Tyr Val Leu Glu Ser Leu Pro Ser Pro Thr
        115                 120                 125

Leu Thr Cys Ala Leu Thr Asn Gly Ser Ile Glu Val Gln Cys Met Ile
    130                 135                 140

Pro Glu His Tyr Asn Ser His Arg Gly Leu Ile Met Tyr Ser Trp Asp
145                 150                 155                 160

Cys Pro Met Glu Gln Cys Lys Arg Asn Ser Thr Ser Ile Tyr Phe Lys
                165                 170                 175

Met Glu Asn Asp Leu Pro Gln Lys Ile Gln Cys Thr Leu Ser Asn Pro
            180                 185                 190

Leu Phe Asn Thr Thr Ser Ser Ile Ile Leu Thr Thr Cys Ile Pro Ser
        195                 200                 205

Ser Gly His Ser Arg His Arg Tyr Ala Leu Ile Pro Ile Pro Leu Ala
    210                 215                 220

Val Ile Thr Thr Cys Ile Val Leu Tyr Met Asn Gly Ile Leu Lys Cys
225                 230                 235                 240

Asp Arg Lys Pro Asp Arg Thr Lys
                245

<210>  35
<211>  250
<212>  PRT
<213>  human

<400>  35

Met Val Ala Gly Ser Asp Ala Gly Arg Ala Leu Gly Val Leu Ser Val
1               5                   10                  15

Val Cys Leu Leu His Cys Phe Gly Phe Ile Ser Cys Phe Ser Gln Gln
            20                  25                  30

Ile Tyr Gly Val Val Tyr Gly Asn Val Thr Phe His Val Pro Ser Asn
        35                  40                  45

Val Pro Leu Lys Glu Val Leu Trp Lys Lys Gln Lys Asp Lys Val Ala
    50                  55                  60

Glu Leu Glu Asn Ser Glu Phe Arg Ala Phe Ser Ser Phe Lys Asn Arg
65                  70                  75                  80

Val Tyr Leu Asp Thr Val Ser Gly Ser Leu Thr Ile Tyr Asn Leu Thr
                85                  90                  95

Ser Ser Asp Glu Asp Glu Tyr Glu Met Glu Ser Pro Asn Ile Thr Asp
            100                 105                 110

Thr Met Lys Phe Phe Leu Tyr Val Leu Glu Ser Leu Pro Ser Pro Thr
        115                 120                 125

Leu Thr Cys Ala Leu Thr Asn Gly Ser Ile Glu Val Gln Cys Met Ile
    130                 135                 140

Pro Glu His Tyr Asn Ser His Arg Gly Leu Ile Met Tyr Ser Trp Asp
145                 150                 155                 160

Cys Pro Met Glu Gln Cys Lys Arg Asn Ser Thr Ser Ile Tyr Phe Lys
                165                 170                 175

Met Glu Asn Asp Leu Pro Gln Lys Ile Gln Cys Thr Leu Ser Asn Pro
            180                 185                 190

Leu Phe Asn Thr Thr Ser Ser Ile Ile Leu Thr Thr Cys Ile Pro Ser
        195                 200                 205

Ser Gly His Ser Arg His Arg Tyr Ala Leu Ile Pro Ile Pro Leu Ala
    210                 215                 220

Val Ile Thr Thr Cys Ile Val Leu Tyr Met Asn Gly Ile Leu Lys Cys
225                 230                 235                 240

Asp Arg Lys Pro Asp Arg Thr Asn Ser Asn
                245                 250

<210>  36
<211>  1791
<212>  DNA
<213>  human

<220>
<221>  CDS
<222>  (85)..(927)

<400>  36
ccttgcttcc tgaactagct cacagtagcc cggcggccca gggcaatccg accacatttc      60

actctcaccg ctgtaggaat ccag atg cag gcc aag tac agc agc acg agg 111
Met Gln Ala Lys Tyr Ser Ser Thr Arg
1               5

gac atg ctg gat gat gat ggg gac acc acc atg agc ctg cat tct caa 159
Asp Met Leu Asp Asp Asp Gly Asp Thr Thr Met Ser Leu His Ser Gln
10                  15                  20                  25

ggc tct gcc aca act cgg cat cca gag ccc cgg cgc aca gag cac agg 207
Gly Ser Ala Thr Thr Arg His Pro Glu Pro Arg Arg Thr Glu His Arg
                30                  35                  40

gct ccc tct tca acg tgg cga cca gtg gcc ctg acc ctg ctg act ttg 255
Ala Pro Ser Ser Thr Trp Arg Pro Val Ala Leu Thr Leu Leu Thr Leu
            45                  50                  55

tgc ttg gtg ctg ctg ata ggg ctg gca gcc ctg ggg ctt ttg ttt ttt 303
Cys Leu Val Leu Leu Ile Gly Leu Ala Ala Leu Gly Leu Leu Phe Phe
        60                  65                  70

cag tac tac cag ctc tcc aat act ggt caa gac acc att tct caa atg 351
Gln Tyr Tyr Gln Leu Ser Asn Thr Gly Gln Asp Thr Ile Ser Gln Met
    75                  80                  85

gaa gaa aga tta gga aat acg tcc caa gag ttg caa tct ctt caa gtc 399
Glu Glu Arg Leu Gly Asn Thr Ser Gln Glu Leu Gln Ser Leu Gln Val
90                  95                  100                 105

cag aat ata aag ctt gca gga agt ctg cag cat gtg gct gaa aaa ctc 447
Gln Asn Ile Lys Leu Ala Gly Ser Leu Gln His Val Ala Glu Lys Leu
                110                 115                 120

tgt cgt gag ctg tat aac aaa gct gga gca cac agg tgc agc cct tgt 495
Cys Arg Glu Leu Tyr Asn Lys Ala Gly Ala His Arg Cys Ser Pro Cys
            125                 130                 135

aca gaa caa tgg aaa tgg cat gga gac aat tgc tac cag ttc tat aaa 543
Thr Glu Gln Trp Lys Trp His Gly Asp Asn Cys Tyr Gln Phe Tyr Lys
        140                 145                 150

gac agc aaa agt tgg gag gac tgt aaa tat ttc tgc ctt agt gaa aac 591
Asp Ser Lys Ser Trp Glu Asp Cys Lys Tyr Phe Cys Leu Ser Glu Asn
    155                 160                 165

tct acc atg ctg aag ata aac aaa caa gaa gac ctg gaa ttt gcc gcg 639
Ser Thr Met Leu Lys Ile Asn Lys Gln Glu Asp Leu Glu Phe Ala Ala
170                 175                 180                 185

tct cag agc tac tct gag ttt ttc tac tct tat tgg aca ggg ctt ttg 687
Ser Gln Ser Tyr Ser Glu Phe Phe Tyr Ser Tyr Trp Thr Gly Leu Leu
                190                 195                 200

cgc cct gac agt ggc aag gcc tgg ctg tgg atg gat gga acc cct ttc 735
Arg Pro Asp Ser Gly Lys Ala Trp Leu Trp Met Asp Gly Thr Pro Phe
            205                 210                 215

act tct gaa ctg ttc cat att ata ata gat gtc acc agc cca aga agc 783
Thr Ser Glu Leu Phe His Ile Ile Ile Asp Val Thr Ser Pro Arg Ser
        220                 225                 230

aga gac tgt gtg gcc atc ctt aat ggg atg atc ttc tca aag gac tgc 831
Arg Asp Cys Val Ala Ile Leu Asn Gly Met Ile Phe Ser Lys Asp Cys
    235                 240                 245

aaa gaa ttg aag cgt tgt gtc tgt gag aga agg gca gga atg gtg aag 879
Lys Glu Leu Lys Arg Cys Val Cys Glu Arg Arg Ala Gly Met Val Lys
250                 255                 260                 265

cca gag agc ctc cat gtc ccc cct gaa aca tta ggc gaa ggt gac tga 927
Pro Glu Ser Leu His Val Pro Pro Glu Thr Leu Gly Glu Gly Asp
                270                 275                 280

ttcgccctct gcaactacaa atagcagagt gagccaggcg gtgccaaagc aagggctagt 987

tgagacattg ggaaatggaa cataatcagg aaagactatc tctctgacta gtacaaaatg 1047

ggttctcgtg tttcctgttc aggatcacca gcatttctga gcttgggttt atgcacgtat 1107

ttaacagtca caagaagtct tatttacatg ccaccaacca acctcagaaa cccataatgt 1167

catctgcctt cttggcttag agataacttt tagctctctt tcttctcaat gtctaatatc 1227

acctccctgt tttcatgtct tccttacact tggtggaata agaaactttt tgaagtagag 1287

gaaatacatt gaggtaacat ccttttctct gacagtcaag tagtccatca gaaattggca 1347

gtcacttccc agattgtacc agcaaataca caaggaattc tttttgtttg tttcagttca 1407

tactagtccc ttcccaatcc atcagtaaag accccatctg ccttgtccat gccgtttccc 1467

aacagggatg tcacttgata tgagaatctc aaatctcaat gccttataag cattccttcc 1527

tgtgtccatt aagactctga taattgtctc ccctccatag gaatttctcc caggaaagaa 1587

atatatcccc atctccgttt catatcagaa ctaccgtccc cgatattccc ttcagagaga 1647

ttaaagacca gaaaaaagtg agcctcttca tctgcacctg taatagtttc agttcctatt 1707

ttcttccatt gacccatatt tatacctttc aggtactgaa gatttaataa taataaatgt 1767

aaatactgtg aaaaaaaaaa aaaa 1791

<210> 37
<211> 280
<212> PRT
<213> human

<400> 37

Met Gln Ala Lys Tyr Ser Ser Thr Arg Asp Met Leu Asp Asp Asp Gly
1               5                   10                  15

Asp Thr Thr Met Ser Leu His Ser Gln Gly Ser Ala Thr Thr Arg His
            20                  25                  30

Pro Glu Pro Arg Arg Thr Glu His Arg Ala Pro Ser Ser Thr Trp Arg
        35                  40                  45

Pro Val Ala Leu Thr Leu Leu Thr Leu Cys Leu Val Leu Leu Ile Gly
    50                  55                  60

Leu Ala Ala Leu Gly Leu Leu Phe Phe Gln Tyr Tyr Gln Leu Ser Asn
65                  70                  75                  80

Thr Gly Gln Asp Thr Ile Ser Gln Met Glu Glu Arg Leu Gly Asn Thr
                85                  90                  95

Ser Gln Glu Leu Gln Ser Leu Gln Val Gln Asn Ile Lys Leu Ala Gly

            100                 105                 110

Ser Leu Gln His Val Ala Glu Lys Leu Cys Arg Glu Leu Tyr Asn Lys
        115                 120                 125

Ala Gly Ala His Arg Cys Ser Pro Cys Thr Glu Gln Trp Lys Trp His
    130                 135                 140

Gly Asp Asn Cys Tyr Gln Phe Tyr Lys Asp Ser Lys Ser Trp Glu Asp
145                 150                 155                 160

Cys Lys Tyr Phe Cys Leu Ser Glu Asn Ser Thr Met Leu Lys Ile Asn
                165                 170                 175

Lys Gln Glu Asp Leu Glu Phe Ala Ala Ser Gln Ser Tyr Ser Glu Phe
            180                 185                 190

Phe Tyr Ser Tyr Trp Thr Gly Leu Leu Arg Pro Asp Ser Gly Lys Ala
        195                 200                 205

Trp Leu Trp Met Asp Gly Thr Pro Phe Thr Ser Glu Leu Phe His Ile
    210                 215                 220

Ile Ile Asp Val Thr Ser Pro Arg Ser Arg Asp Cys Val Ala Ile Leu
225                 230                 235                 240

Asn Gly Met Ile Phe Ser Lys Asp Cys Lys Glu Leu Lys Arg Cys Val
                245                 250                 255

Cys Glu Arg Arg Ala Gly Met Val Lys Pro Glu Ser Leu His Val Pro
            260                 265                 270

Pro Glu Thr Leu Gly Glu Gly Asp
        275                 280

<210> 38
<211> 2069
<212> DNA
<213> human

<220>
<221> CDS
<222> (1)..(993)

<400> 38
atg ctg ccg gac tgg aag agc tcc ttg atc ctc atg gct tac atc atc 48
Met Leu Pro Asp Trp Lys Ser Ser Leu Ile Leu Met Ala Tyr Ile Ile
1               5                   10                  15

atc ttc ctc act ggc ctc cct gcc aac ctc ctg gcc ctg cgg gcc ttt 96
Ile Phe Leu Thr Gly Leu Pro Ala Asn Leu Leu Ala Leu Arg Ala Phe
            20                  25                  30

gtg ggg cgg atc cgc cag ccc cag cct gca cct gtg cac atc ctc ctg 144
Val Gly Arg Ile Arg Gln Pro Gln Pro Ala Pro Val His Ile Leu Leu

        35                  40                  45

ctg agc ctg acg ctg gcc gac ctc ctc ctg ctg ctg ctg ctg ccc ttc 192
Leu Ser Leu Thr Leu Ala Asp Leu Leu Leu Leu Leu Leu Leu Pro Phe
    50                  55                  60

aag atc atc gag gct gcg tcg aac ttc cgc tgg tac ctg ccc aag gtc 240
Lys Ile Ile Glu Ala Ala Ser Asn Phe Arg Trp Tyr Leu Pro Lys Val
65                  70                  75                  80

gtc tgc gcc ctc acg agt ttt ggc ttc tac agc agc atc tac tgc agc 288
Val Cys Ala Leu Thr Ser Phe Gly Phe Tyr Ser Ser Ile Tyr Cys Ser
                85                  90                  95

acg tgg ctc ctg gcg ggc atc agc atc gag cgc tac ctg gga gtg gct 336
Thr Trp Leu Leu Ala Gly Ile Ser Ile Glu Arg Tyr Leu Gly Val Ala
            100                 105                 110

ttc ccc gtg cag tac aag ctc tcc cgc cgg cct ctg tat gga gtg att 384
Phe Pro Val Gln Tyr Lys Leu Ser Arg Arg Pro Leu Tyr Gly Val Ile
        115                 120                 125

gca gct ctg gtg gcc tgg gtt atg tcc ttt ggt cac tgc acc atc gtg 432
Ala Ala Leu Val Ala Trp Val Met Ser Phe Gly His Cys Thr Ile Val
    130                 135                 140

atc atc gtt caa tac ttg aac acg act gag cag gtc aga agt ggc aat 480
Ile Ile Val Gln Tyr Leu Asn Thr Thr Glu Gln Val Arg Ser Gly Asn
145                 150                 155                 160

gaa att acc tgc tac gag aac ttc acc gat aac cag ttg gac gtg gtg 528
Glu Ile Thr Cys Tyr Glu Asn Phe Thr Asp Asn Gln Leu Asp Val Val
                165                 170                 175

ctg ccc gtg cgg ctg gag ctg tgc ctg gtg ctc ttc ttc atc ccc atg 576
Leu Pro Val Arg Leu Glu Leu Cys Leu Val Leu Phe Phe Ile Pro Met
            180                 185                 190

gca gtc acc atc ttc tgc tac tgg cgt ttt gtg tgg atc atg ctc tcc 624
Ala Val Thr Ile Phe Cys Tyr Trp Arg Phe Val Trp Ile Met Leu Ser
        195                 200                 205

cag ccc ctt gtg ggg gcc cag agg cgg cgc cga gcc gtg ggg ctg gct 672
Gln Pro Leu Val Gly Ala Gln Arg Arg Arg Arg Ala Val Gly Leu Ala
    210                 215                 220

gtg gtg acg ctg ctc aat ttc ctg gtg tgc ttc gga cct tac aac gtg 720
Val Val Thr Leu Leu Asn Phe Leu Val Cys Phe Gly Pro Tyr Asn Val
225                 230                 235                 240

tcc cac ctg gtg ggg tat cac cag aga aaa agc ccc tgg tgg cgg tca 768
Ser His Leu Val Gly Tyr His Gln Arg Lys Ser Pro Trp Trp Arg Ser
                245                 250                 255

ata gcc gtg gtg ttc agt tca ctc aac gcc agt ctg gac ccc ctg ctc 816
Ile Ala Val Val Phe Ser Ser Leu Asn Ala Ser Leu Asp Pro Leu Leu
            260                 265                 270

ttc tat ttc tct tct tca gtg gtg cgc agg gca ttt ggg aga ggg ctg 864
Phe Tyr Phe Ser Ser Ser Val Val Arg Arg Ala Phe Gly Arg Gly Leu
        275                 280                 285

cag gtg ctg cgg aat cag ggc tcc tcc ctg ttg gga cgc aga ggc aaa 912
Gln Val Leu Arg Asn Gln Gly Ser Ser Leu Leu Gly Arg Arg Gly Lys
    290                 295                 300

gac aca gca gag ggg aca aat gag gac agg ggt gtg ggt caa gga gaa 960
Asp Thr Ala Glu Gly Thr Asn Glu Asp Arg Gly Val Gly Gln Gly Glu

305                 310                 315                 320

ggg atg cca agt tcg gac ttc act aca gag tag cagtttccct ggaccttcag    1013
Gly Met Pro Ser Ser Asp Phe Thr Thr Glu
                325                 330

aggtcgcctg ggttacacag gagctgggaa gcctgggaga ggcggagcag gaaggctccc    1073

atccagattc agaaatcctt agacccagcc caggactgcg actttgaaaa aaatgccttt    1133

caccagcttg gtatcccttc ctgactgaat tgtcctactc aaaggagcat aagtcagaga    1193

tgcacgaaga agtagttagg tatagaagca cctgccgggt gtggtggctc atgcctataa    1253

tcccagaact ttgggaggct gaggcaggtg gatcacttga ggtcgggaga ttgagaacat    1313

cctggtcaac atgggaaaac cccgtctcta ctaaaaatac aaaaaaatta gctgggcatg    1373

gtggcacatg cctataatcc cagctactct ggaggctgag gcaggagaat ccttgaaccc    1433

gggagttgga ggttgcagtg agctgagatc acgccactgc actccagcct agcgacagag    1493

caagactcca tttaaaaaaa aaaaaaaaaa aaaaaagaag caccttcagg ctggagaagc    1553

agcgtagcta acacaagtcc agtccttgtg atgtggctgg tagttgggga tggccaggct    1613

gaagcagaga gtcctagaga aatctcgata caagcttcaa agcaacacct agacactgct    1673

ctagcggttg atcctggaga taaaccaaca agagagagat ggaagagaaa tactaaatga    1733

ggtcaaagaa gactcagaaa ggttctgagc ctggagatga gcagggaggc ctcagggctt    1793

agacctttaa tgataggggt ttccctgcat tggtttgacc tgttgccttt ttgatgtgct    1853

ctgtttgttt tcatgtgttg tcttgtctcc cctgctaaac tgggagctgc caggggtctg    1913

ggtcttatct ccttcctcca tggtacccca cacaggccag gatgtggttt ggtacccagc    1973

aatcagagat tggcactccc tcatacaggg gaaagcaacc tggtctagca aattgaaaat    2033

aaagatgata aaactctgaa aaaaaaaaaa aaaaaa                              2069

<210>  39
<211>  330
<212>  PRT
<213>  human

<400>  39
   Met Leu Pro Asp Trp Lys Ser Ser Leu Ile Leu Met Ala Tyr Ile Ile
   1               5                   10                  15
   Ile Phe Leu Thr Gly Leu Pro Ala Asn Leu Leu Ala Leu Arg Ala Phe
               20                  25                  30
   Val Gly Arg Ile Arg Gln Pro Gln Pro Ala Pro Val His Ile Leu Leu
           35                  40                  45
   Leu Ser Leu Thr Leu Ala Asp Leu Leu Leu Leu Leu Leu Leu Pro Phe
       50                  55                  60
   Lys Ile Ile Glu Ala Ala Ser Asn Phe Arg Trp Tyr Leu Pro Lys Val
   65                  70                  75                  80
   Val Cys Ala Leu Thr Ser Phe Gly Phe Tyr Ser Ser Ile Tyr Cys Ser
                   85                  90                  95
   Thr Trp Leu Leu Ala Gly Ile Ser Ile Glu Arg Tyr Leu Gly Val Ala
               100                 105                 110
   Phe Pro Val Gln Tyr Lys Leu Ser Arg Arg Pro Leu Tyr Gly Val Ile
           115                 120                 125
   Ala Ala Leu Val Ala Trp Val Met Ser Phe Gly His Cys Thr Ile Val
       130                 135                 140
   Ile Ile Val Gln Tyr Leu Asn Thr Thr Glu Gln Val Arg Ser Gly Asn
   145                 150                 155                 160
   Glu Ile Thr Cys Tyr Glu Asn Phe Thr Asp Asn Gln Leu Asp Val Val
                   165                 170                 175
   Leu Pro Val Arg Leu Glu Leu Cys Leu Val Leu Phe Phe Ile Pro Met
               180                 185                 190
   Ala Val Thr Ile Phe Cys Tyr Trp Arg Phe Val Trp Ile Met Leu Ser
           195                 200                 205
   Gln Pro Leu Val Gly Ala Gln Arg Arg Arg Arg Ala Val Gly Leu Ala
       210                 215                 220
   Val Val Thr Leu Leu Asn Phe Leu Val Cys Phe Gly Pro Tyr Asn Val
   225                 230                 235                 240
   Ser His Leu Val Gly Tyr His Gln Arg Lys Ser Pro Trp Trp Arg Ser
                   245                 250                 255
   Ile Ala Val Val Phe Ser Ser Leu Asn Ala Ser Leu Asp Pro Leu Leu
               260                 265                 270
   Phe Tyr Phe Ser Ser Ser Val Val Arg Arg Ala Phe Gly Arg Gly Leu
           275                 280                 285
   Gln Val Leu Arg Asn Gln Gly Ser Ser Leu Leu Gly Arg Arg Gly Lys
       290                 295                 300
   Asp Thr Ala Glu Gly Thr Asn Glu Asp Arg Gly Val Gly Gln Gly Glu
   305                 310                 315                 320
   Gly Met Pro Ser Ser Asp Phe Thr Thr Glu
                   325                 330

<210>  40
<211>  5084

<212>  DNA
<213>  human

<220>
<221>  CDS
<222>  (170)..(1825)

<400>  40
ctctgggctg ctcgggcgcc accactactg tccccatccc gccccgccag ctgggccaaa      60

gggtgacttg actcacgacg ctgccaccag cccacggctt gcccgaggcg tataaaggct     120

gccagggccg caggctgcca agccctgccc tgcccaaggc gcatagagc atg gcc cga     178
                                                      
                                                      1

ggc gct gag gga ggc cgt ggg gac gcg ggt tgg ggc ctg cgt ggc gcc       226

    5                   10                  15

ctg gcg gcc gtg gcg ctg ctc tcg gcg ctc aac gct gcg ggc acg gtg       274

20                  25                  30                  35

ttc gcg ctg tgc cag tgg cgc ggg ctg agc tcg gcg ctg cgg gct ttg       322

                40                  45                  50

gag gcg cag cgg ggc cgg gag cag cgc gag gac agt gcc ctg cgc tcc       370

            55                  60                  65

ttc ctg gcc gag ttg agc cgc gcg ccg cgc ggg gcg tcc gca cca ccc       418

        70                  75                  80

caa gac ccg gcc agc tca gct cgc aac aag cgc agc cac agc ggc gag       466

    85                  90                  95

ccc gcg ccg cat atc cgc gcc gag agc cat gac atg ctg atg atg atg       514

100                 105                 110                 115

acc tac tcc atg gtg ccg atc cga gtg atg gtg gac ctg tgc aac agc       562

                120                 125                 130

acc aag ggc atc tgc ctc aca gga cct tct gga cca cca gga cct ccg       610

            135                 140                 145

gga gcc ggc ggg ttg cca gga cac aac gga ttg gat gga cag cct ggt       658

        150                 155                 160

cct cag ggc cca aaa gga gaa aaa gga gca aat gga aaa aga gga aaa       706

    165                 170                 175

atg ggg ata cct gga gct gca gga aat cca ggg gaa agg gga gaa aag       754

180                 185                 190                 195

gga gac cat ggt gaa ctg ggc ctg cag gga aat gag ggc cca cca ggg       802

                200                 205                 210

cag aag gga gaa aag ggt gac aaa gga gat gtg tcc aac gac gtg ctc 850

            215                 220                 225

ctg gca ggt gcc aaa ggt gac caa ggc cca ccc ggt cca cct ggg ccc 898

        230                 235                 240

cca ggc cct cca ggt cct cca ggg ccc cct gga agc aga aga gcc aaa 946

    245                 250                 255

ggc cct cgg cag cca agc atg ttc aac ggc cag tgc cca ggt gag act 994

260                 265                 270                 275

tgt gcc ata cca aat gat gat acc ttg gtt gga aaa gct gat gag aaa 1042

                280                 285                 290

gcc agt gaa cac cat tcc cca caa gca gaa tcc atg atc act tcc att 1090

            295                 300                 305

gga aac cca gtg caa gta ctg aaa gtg aca gag aca ttt ggg act tgg 1138

        310                 315                 320

ata aga gag tct gct aac aag agt gat gac cgg att tgg gtg aca gag 1186

    325                 330                 335

cat ttt tca ggc atc atg gtt aag gaa ttc aag gat cag ccc tca ctt 1234

340                 345                 350                 355

ctg aat ggc agt tac acg ttc atc cac ctt cca tac tat ttc cat ggc 1282

                360                 365                 370

tgt ggg cac gtt gtt tac aac aac tct ctc tac tac cac aaa ggg ggt 1330

            375                 380                 385

tct aat acc cta gtg aga ttt gaa ttt ggc cag gaa aca tcc caa act 1378

        390                 395                 400

ctg aag ctt gaa aat gcc ttg tat ttt gat cga aaa tac ctt ttt gca 1426

    405                 410                 415

aat tcc aaa act tac ttc aat cta gct gta gat gaa aag ggc ctt tgg 1474

420                 425                 430                 435

att atc tat gcg tca agt gtg gac ggc tcg agc att ctt gta gca caa 1522

                440                 445                 450

ctg gat gag agg aca ttc tca gtg gtg caa cac gtc aat acc acg tac 1570

            455                 460                 465

cct aaa tcc aag gct ggc aac gcc ttc att gcc cga gga atc ctc tat 1618

        470                 475                 480

gtc aca gac acc aaa gat atg agg gtc aca ttt gcc ttt gat ttg tta     1666

    485                 490                 495

gga ggg aaa cag atc aat gca aac ttt gat tta aga act tcc cag tct     1714

500                 505                 510                 515

gtt ctt gcc atg tta gca tac aac atg aga gat cag cat tta tat tca     1762

                520                 525                 530

tgg gaa gat ggc cat tta atg ctt tat cct gtg cag ttt ttg tca act     1810

            535                 540                 545

acc tta aat cag tga tgtgctgcat tcggctccct tcagcaaatt tcaggggttt     1865

        550

tctgggacca gttctccccc aacaggaaac ttgttttttt aacgtcagcc agatatttag     1925

aaaataacct caaaagtgtt tatatggtca gtgagccccg cttagtgaaa tagcaacaga     1985

ttggaagttg aaatggctga gatttggtga tctccccaca gctggctctg caagttacct     2045

ctttctcctt gggccttagt ttccccattg gtaatctgaa ttggctaaga tgattgggga     2105

gattttctgt acctgtaggt aatttggtga ttcttggtgg ctgctcttct cacaactttt     2165

atgtatctgc ttctgtcgtt tagctttttt agccacatgc tgaccaaatt tacctttgag     2225

ttgataagtc cagtggcttg agtagtgaat ccctcagtgc tgacttatat cttgttcttt     2285

gaaaaaatgc attgactctt taagacatct aaagtatcac attatccata atttattgct     2345

tttctttgca tctgcacctg ccaccacaga ataaccatta ccctcagctg ctgattgggc     2405

agctctgaga ttagcaaaag ccagggacag ctacatgttc aggttttttt tttttttttt     2465

ttttcaataa gctatttttt ttcttttctt attttaaata gagagagagt cttgctatgt     2525

ttcccaggct ggtcttgaac tcctggggct caagtgatcc tcctgccttg gcctcccaaa     2585

atgctggatt acaggcatgt gtgcctggcc caggtttctt aataaaacag aatcatgatc     2645

ttccaggttc cccccagttt ctgatcatgt tgatttgtag ctgtggatca tgaacactga     2705

atcctcagat cactctgact tcttatgctt ctcctgtgga tccactatca aagtactaaa     2765

tgctgtgtaa gtagacgtta atctggctgg aaccatggga agcactttgc agtgttcaga     2825

agagaggctc catttgtggc tattatgtag aactgggcca gagccagtcc attgcctgtt     2885

tttttaaata aggttttact gagcacagcc acactcattt gtttatgcag tacggcctga     2945

cattgctttt gctctgcaac agcagagtcg agtcattgca acaaagagca tatggcccca     3005

cagtgcctaa aatattgacc agctacccct ttatggaaaa agattgctga ctcctgataa     3065

agaatataaa gtgagcctga ttcttgaaaa aatcagaacc agagcctgtt ttgttttgtt     3125

ctaaactaag aagccgcata ggatgtgact tgcgttttga gtagagggga aggctgataa     3185

cggcgtaaga tgaagtggcc ctccacaaag gctggttagg ggacagttct ttctctaaca     3245

tagttttaaa ggatgtgatc tggtcccctt ggatgccagg agagaatcca gttgaacttg     3305

ctcctaaatg ctcttaaata tgcatatttt ctgccaactc acttctttaa acatctttca 3365
gcccagcgct gcggccccgg gaagggccac tgcgaataga gaggaagctg gaaaagttcc 3425
tggggctctg cagccaggaa ggggaaccag ggcaaatctt atgtaaagat ttttcagcaa 3485
cttgtcccaa tttgtgtgta ttctgaaact ttctctttgg gaccaaattc attctcaatg 3545
gccctgagtt caatatatta ttaacagcag tattttaaaa cttagggttg aactgggcat 3605
ggtggcacat aactgcaatc ccagctactt tggaggcagg gatgggagga tcacttgagg 3665
ccaggatctc aggaccagcc tagagagatc ccatctctaa aaaataaaat ataagaaaat 3725
aaaacttagg ggatatacag atttaaatat tcaaatctcc ctgctcccct gaaagtcccc 3785
aggcagctgt caatgacttg tttgttgtgt tctcaatatg atggctattt gaaacttcac 3845
ctacttttca ttagattggt tgtaccatgt caccttagct tttaaaaata ctcttttcag 3905
attcacgttc tctaacaaag agtctcatgt tcaagatcaa tatgtctaat aagtgctggt 3965
gtccttttaa agtatttaaa tatatatgtt gctgttgctg aatacaggag accaggttag 4025
gaatatagtt tcataataat agtacataca atactaattg tatataaggt agcaaccaaa 4085
agaggttgtt aattagcaca tattcctttt agaaaaatgt ttcagaaacc tcagtcttga 4145
tatctgagct atctgggctc ccttacttgt gagtaaggga tcatgctcac cactggagaa 4205
gcttacaccg ggactttttt tcttttttct tttttttttg ctatgacaga gtaatgctaa 4265
cgtaaggaca actgagtttg atcagtgttt aatcgcagtg ggtaatctta tctgattgtc 4325
tttaaaagtg aaaaggatta agattttatt ctttcttgta aacattactt gattttttaa 4385
agaagttttg ggctcactgc taaaatagag tatacaactg aatgttttta agtcaagata 4445
ctgttttagg agtttaccct ctcatttata accaaagttg ctctaaaaca ctttccaaat 4505
atctgcactt ctgatgtcag aatcaaacca gataattctc taattcttct ttaatctaaa 4565
gtagatagct tcccactgga aagtaaacaa aaccatccct cccaacctca aagctaggcc 4625
acactctatt tcaaggcatt ttctttcagc tgataaggtg tcctcctgaa gccaagtagg 4685
tggttctggt ctccaagtat cgttaagcac aggtgctatg acagaaaaag ttctggggtg 4745
gaagttttaa gatgaggagt tctgatctta ggcatcttaa cagtcacaag gtgaaaagtc 4805
aaatgaaaca gtacaattct tgatgagtga ggtgtcatct tccaaccaca cagaggacgt 4865
tttggctatg atcatctgat ggcaagtgaa ggagaaatga gtgatagggc tttgcgtttt 4925
catccagatg ctgtggccct gtgtttcaca gcattaagag ccataatttc caacctgcac 4985
agatcctgaa caacaaatga ataacgatga atgtcttttt ggttgtaatt taacaagtca 5045
aataaaataa tcattgctga gcacaatcac atgttgaaa 5084

<210> 41
<211> 551
<212> PRT
<213> human

<400> 41

<210>  42
<211>  4260
<212>  DNA
<213>  human

<220>
<221>  CDS
<222>  (1100)..(3739)

<400>  42
gtgcagttga gtcgcgagta cggctgagct gcgtaccggc ctccctggct ctcacactcc      60

ctctctgctc ccgctctcct aatctcctct ggcatgcggt cagccccctg cccagggacc     120

acaggagagt tcttgtaagg actgttagtc cctgcttacc tgaaagccaa gcgctctagc     180

agagctttaa agttggagcc gccaccctcc ctaccgcccc atgccccttc accccactcc     240

gaaattcacc gacctttgca tgcactgcct aaggatttca gagtgaggca aagcagtcgg     300

caaatctacc ctggcttttc gtataaaaat cctctcgtct aggtaccctg gctcactgaa     360

gactctgcag atataccctt ataagaggga gggtggggga gggaaaagaa cgagagaggg     420

aggaaagaat gaaaaggaga ggatgccagg aggtccgtgc ttctgccaag agtcccaatt     480

agatgcgacg gcttcagcct ggtcaaggtg aaggaaagtt gcttccgcgc ctaggaagtg     540

ggtttgcctg ataagagaag gaggagggga ctcggctggg aagagctccc ctcccctccg     600

cggaagacca ctgggtcccc tctttcccca acctcctccc tctcttctac tccacccctc     660

cgttttccca ctccccactg actcggatgc ctggatgttc tgccaccggg cagtggtcca     720

gcgtgcagcc gggagggggc aggggcaggg ggcactgtga caggaagctg cgcgcacaag     780

ttggccattt cgagggcaaa ataagttctc ccttggattt ggaaaggaca aagccagtaa     840

gctacctctt ttgtgtcgga tgaggaggac caaccatgag ccagagcccg ggtgcaggct     900

caccgccgcc gctgccaccg cggtcagctc cagttcctgc caggagttgt cggtgcgagg     960

aattttgtga caggctctgt tagtctgttc ctcccttatt tgaaggacag gccaaagatc    1020

cagtttggaa atgagagagg actagcatga cacattggct ccaccattga tatctcccag    1080

aggtacagaa acaggattc atg aag atg ttg aca aga ctg caa gtt ctt acc     1132
                     Met Lys Met Leu Thr Arg Leu Gln Val Leu Thr
                     1               5                   10

tta gct ttg ttt tca aag gga ttt tta ctc tct tta ggg gac cat aac      1180
Leu Ala Leu Phe Ser Lys Gly Phe Leu Leu Ser Leu Gly Asp His Asn
            15                  20                  25

ttt cta agg aga gag att aaa ata gaa ggt gac ctt gtt tta ggg ggc      1228
Phe Leu Arg Arg Glu Ile Lys Ile Glu Gly Asp Leu Val Leu Gly Gly
        30                  35                  40

ctg ttt cct att aac gaa aaa ggc act gga act gaa gaa tgt ggg cga      1276
Leu Phe Pro Ile Asn Glu Lys Gly Thr Gly Thr Glu Glu Cys Gly Arg
    45                  50                  55

atc aat gaa gac cga ggg att caa cgc ctg gaa gcc atg ttg ttt gct 1324
Ile Asn Glu Asp Arg Gly Ile Gln Arg Leu Glu Ala Met Leu Phe Ala
60                  65                  70                  75

att gat gaa atc aac aaa gat gat tac ttg cta cca gga gtg aag ttg 1372
Ile Asp Glu Ile Asn Lys Asp Asp Tyr Leu Leu Pro Gly Val Lys Leu
                80                  85                  90

ggt gtt cac att ttg gat aca tgt tca agg gat acc tat gca ttg gag 1420
Gly Val His Ile Leu Asp Thr Cys Ser Arg Asp Thr Tyr Ala Leu Glu
            95                  100                 105

caa tca ctg gag ttt gtc agg gca tct ttg aca aaa gtg gat gaa gct 1468
Gln Ser Leu Glu Phe Val Arg Ala Ser Leu Thr Lys Val Asp Glu Ala
        110                 115                 120

gag tat atg tgt cct gat gga tcc tat gcc att caa gaa aac atc cca 1516
Glu Tyr Met Cys Pro Asp Gly Ser Tyr Ala Ile Gln Glu Asn Ile Pro
    125                 130                 135

ctt ctc att gca ggg gtc att ggt ggc tct tat agc agt gtt tcc ata 1564
Leu Leu Ile Ala Gly Val Ile Gly Gly Ser Tyr Ser Ser Val Ser Ile
140                 145                 150                 155

cag gtg gca aac ctg ctg cgg ctc ttc cag atc cct cag atc agc tac 1612
Gln Val Ala Asn Leu Leu Arg Leu Phe Gln Ile Pro Gln Ile Ser Tyr
                160                 165                 170

gca tcc acc agc gcc aaa ctc agt gat aag tcg cgc tat gat tac ttt 1660
Ala Ser Thr Ser Ala Lys Leu Ser Asp Lys Ser Arg Tyr Asp Tyr Phe
            175                 180                 185

gcc agg acc gtg ccc ccc gac ttc tac cag gcc aaa gcc atg gct gag 1708
Ala Arg Thr Val Pro Pro Asp Phe Tyr Gln Ala Lys Ala Met Ala Glu
        190                 195                 200

atc ttg cgc ttc ttc aac tgg acc tac gtg tcc aca gta gcc tcc gag 1756
Ile Leu Arg Phe Phe Asn Trp Thr Tyr Val Ser Thr Val Ala Ser Glu
    205                 210                 215

ggt gat tac ggg gag aca ggg atc gag gcc ttc gag cag gaa gcc cgc 1804
Gly Asp Tyr Gly Glu Thr Gly Ile Glu Ala Phe Glu Gln Glu Ala Arg
220                 225                 230                 235

ctg cgc aac atc tgc atc gct acg gcg gag aag gtg ggc cgc tcc aac 1852
Leu Arg Asn Ile Cys Ile Ala Thr Ala Glu Lys Val Gly Arg Ser Asn
                240                 245                 250

atc cgc aag tcc tac gac agc gtg atc cga gaa ctg ttg cag aag ccc 1900
Ile Arg Lys Ser Tyr Asp Ser Val Ile Arg Glu Leu Leu Gln Lys Pro
            255                 260                 265

aac gcg cgc gtc gtg gtc ctc ttc atg cgc agc gac gac tcg cgg gag 1948
Asn Ala Arg Val Val Val Leu Phe Met Arg Ser Asp Asp Ser Arg Glu
        270                 275                 280

ctc att gca gcc gcc agc cgc gcc aat gcc tcc ttc acc tgg gtg gcc 1996
Leu Ile Ala Ala Ala Ser Arg Ala Asn Ala Ser Phe Thr Trp Val Ala
    285                 290                 295

agc gac ggc tgg ggc gcg cag gag agc atc atc aag ggc agc gag cat 2044
Ser Asp Gly Trp Gly Ala Gln Glu Ser Ile Ile Lys Gly Ser Glu His
300                 305                 310                 315

gtg gcc tac ggc gcc atc acc ctg gag ctg gcc tcc cag cct gtc cgc 2092
Val Ala Tyr Gly Ala Ile Thr Leu Glu Leu Ala Ser Gln Pro Val Arg
                320                 325                 330

cag ttc gac cgc tac ttc cag agc ctc aac ccc tac aac aac cac cgc 2140
Gln Phe Asp Arg Tyr Phe Gln Ser Leu Asn Pro Tyr Asn Asn His Arg
            335                 340                 345

aac ccc tgg ttc cgg gac ttc tgg gag caa aag ttt cag tgc agc ctc 2188
Asn Pro Trp Phe Arg Asp Phe Trp Glu Gln Lys Phe Gln Cys Ser Leu
        350                 355                 360

cag aac aaa cgc aac cac agg cgc gtc tgc gac aag cac ctg gcc atc 2236
Gln Asn Lys Arg Asn His Arg Arg Val Cys Asp Lys His Leu Ala Ile
    365                 370                 375

gac agc agc aac tac gag caa gag tcc aag atc atg ttt gtg gtg aac 2284
Asp Ser Ser Asn Tyr Glu Gln Glu Ser Lys Ile Met Phe Val Val Asn
380                 385                 390                 395

gcg gtg tat gcc atg gcc cac gct ttg cac aaa atg cag cgc acc ctc 2332
Ala Val Tyr Ala Met Ala His Ala Leu His Lys Met Gln Arg Thr Leu
                400                 405                 410

tgt ccc aac act acc aag ctt tgt gat gct atg aag atc ctg gat ggg 2380
Cys Pro Asn Thr Thr Lys Leu Cys Asp Ala Met Lys Ile Leu Asp Gly
            415                 420                 425

aag aag ttg tac aag gat tac ttg ctg aaa atc aac ttc acg gct cca 2428
Lys Lys Leu Tyr Lys Asp Tyr Leu Leu Lys Ile Asn Phe Thr Ala Pro
        430                 435                 440

ttc aac cca aat aaa gat gca gat agc ata gtc aag ttt gac act ttt 2476
Phe Asn Pro Asn Lys Asp Ala Asp Ser Ile Val Lys Phe Asp Thr Phe
    445                 450                 455

gga gat gga atg ggg cga tac aac gtg ttc aat ttc caa aat gta ggt 2524
Gly Asp Gly Met Gly Arg Tyr Asn Val Phe Asn Phe Gln Asn Val Gly
460                 465                 470                 475

gga aag tat tcc tac ttg aaa gtt ggt cac tgg gca gaa acc tta tcg 2572
Gly Lys Tyr Ser Tyr Leu Lys Val Gly His Trp Ala Glu Thr Leu Ser
                480                 485                 490

cta gat gtc aac tct atc cac tgg tcc cgg aac tca gtc ccc act tcc 2620
Leu Asp Val Asn Ser Ile His Trp Ser Arg Asn Ser Val Pro Thr Ser
            495                 500                 505

cag tgc agc gac ccc tgt gcc ccc aat gaa atg aag aat atg caa cca 2668
Gln Cys Ser Asp Pro Cys Ala Pro Asn Glu Met Lys Asn Met Gln Pro
        510                 515                 520

ggg gat gtc tgc tgc tgg att tgc atc ccc tgt gaa ccc tac gaa tac 2716
Gly Asp Val Cys Cys Trp Ile Cys Ile Pro Cys Glu Pro Tyr Glu Tyr
    525                 530                 535

ctg gct gat gag ttt acc tgt atg gat tgt ggg tct gga cag tgg ccc 2764
Leu Ala Asp Glu Phe Thr Cys Met Asp Cys Gly Ser Gly Gln Trp Pro
540                 545                 550                 555

act gca gac cta act gga tgc tat gac ctt cct gag gac tac atc agg 2812
Thr Ala Asp Leu Thr Gly Cys Tyr Asp Leu Pro Glu Asp Tyr Ile Arg
                560                 565                 570

tgg gaa gac gcc tgg gcc att ggc cca gtc acc att gcc tgt ctg ggt 2860
Trp Glu Asp Ala Trp Ala Ile Gly Pro Val Thr Ile Ala Cys Leu Gly
            575                 580                 585

ttt atg tgt aca tgc atg gtt gta act gtt ttt atc aag cac aac aac 2908
Phe Met Cys Thr Cys Met Val Val Thr Val Phe Ile Lys His Asn Asn
        590                 595                 600

aca ccc ttg gtc aaa gca tcg ggc cga gaa ctc tgc tac atc tta ttg 2956
Thr Pro Leu Val Lys Ala Ser Gly Arg Glu Leu Cys Tyr Ile Leu Leu
    605                 610                 615

ttt ggg gtt ggc ctg tca tac tgc atg aca ttc ttc ttc att gcc aag 3004
Phe Gly Val Gly Leu Ser Tyr Cys Met Thr Phe Phe Phe Ile Ala Lys
620                 625                 630                 635

cca tca cca gtc atc tgt gca ttg cgc cga ctc ggg ctg ggg agt tcc 3052
Pro Ser Pro Val Ile Cys Ala Leu Arg Arg Leu Gly Leu Gly Ser Ser
                640                 645                 650

ttc gct atc tgt tac tca gcc ctg ctg acc aag aca aac tgc att gcc 3100
Phe Ala Ile Cys Tyr Ser Ala Leu Leu Thr Lys Thr Asn Cys Ile Ala
            655                 660                 665

cgc atc ttc gat ggg gtc aag aat ggc gct cag agg cca aaa ttc atc 3148
Arg Ile Phe Asp Gly Val Lys Asn Gly Ala Gln Arg Pro Lys Phe Ile
        670                 675                 680

agc ccc agt tct cag gtt ttc atc tgc ctg ggt ctg atc ctg gtg caa 3196
Ser Pro Ser Ser Gln Val Phe Ile Cys Leu Gly Leu Ile Leu Val Gln
    685                 690                 695

att gtg atg gtg tct gtg tgg ctc atc ctg gag gcc cca ggc acc agg 3244
Ile Val Met Val Ser Val Trp Leu Ile Leu Glu Ala Pro Gly Thr Arg
700                 705                 710                 715

agg tat acc ctt gca gag aag cgg gaa aca gtc atc cta aaa tgc aat 3292
Arg Tyr Thr Leu Ala Glu Lys Arg Glu Thr Val Ile Leu Lys Cys Asn
                720                 725                 730

gtc aaa gat tcc agc atg ttg atc tct ctt acc tac gat gtg atc ctg 3340
Val Lys Asp Ser Ser Met Leu Ile Ser Leu Thr Tyr Asp Val Ile Leu
            735                 740                 745

gtg atc tta tgc act gtg tac gcc ttc aaa acg cgg aag tgc cca gaa 3388
Val Ile Leu Cys Thr Val Tyr Ala Phe Lys Thr Arg Lys Cys Pro Glu
        750                 755                 760

aat ttc aac gaa gct aag ttc ata ggt ttt acc atg tac acc acg tgc 3436
Asn Phe Asn Glu Ala Lys Phe Ile Gly Phe Thr Met Tyr Thr Thr Cys
    765                 770                 775

atc atc tgg ttg gcc ttc ctc cct ata ttt tat gtg aca tca agt gac 3484
Ile Ile Trp Leu Ala Phe Leu Pro Ile Phe Tyr Val Thr Ser Ser Asp
780                 785                 790                 795

tac aga gtg cag acg aca acc atg tgc atc tct gtc agc ctg agt ggc 3532
Tyr Arg Val Gln Thr Thr Thr Met Cys Ile Ser Val Ser Leu Ser Gly
                800                 805                 810

ttt gtg gtc ttg ggc tgt ttg ttt gca ccc aag gtt cac atc atc ctg 3580
Phe Val Val Leu Gly Cys Leu Phe Ala Pro Lys Val His Ile Ile Leu
            815                 820                 825

ttt caa ccc cag aag aat gtt gtc aca cac aga ctg cac ctc aac agg 3628
Phe Gln Pro Gln Lys Asn Val Val Thr His Arg Leu His Leu Asn Arg
        830                 835                 840

ttc agt gtc agt gga act ggg acc aca tac tct cag tcc tct gca agc 3676
Phe Ser Val Ser Gly Thr Gly Thr Thr Tyr Ser Gln Ser Ser Ala Ser
    845                 850                 855

acg tat gtg cca acg gtg tgc aat ggg cgg gaa gtc ctc gac tcc acc 3724
Thr Tyr Val Pro Thr Val Cys Asn Gly Arg Glu Val Leu Asp Ser Thr
860                 865                 870                 875

acc tca tct ctg tga ttgtgaattg cagttcagtt cttgtgtttt tagactgtta 3779
Thr Ser Ser Leu

gacaaaagtg ctcacgtgca gctccagaat atggaaacag agcaaaagaa caaccctagt 3839

accttttttt agaaacagta cgataaatta tttttgagga ctgtatatag tgatgtgcta 3899

gaactttcta ggctgagtct agtgccccta ttattaacaa ttcccccaga acatggaaat 3959

aaccattgtt tacagagctg agcattggtg acagggtctg acatggtcag tctactaaaa 4019

aacaaaaaaa aaaaacaaaa aaaaaaaaac aaaagaaaaa aataaaaata cggtggcaat 4079

attatgtaac cttttttcct atgaagtttt ttgtaggtcc ttgttgtaac taatttagga 4139

tgagtttcta tgttgtatat taaagttaca ttatgtgtaa cagattgatt ttctcagcac 4199

aaaataaaaa gcatctgtat taatgtaaag atactgagaa taaaaccttc aaggttttcc 4259

a 4260

<210> 43
<211> 879
<212> PRT
<213> human

<400> 43

Met Lys Met Leu Thr Arg Leu Gln Val Leu Thr Leu Ala Leu Phe Ser
1               5                   10                  15

Lys Gly Phe Leu Leu Ser Leu Gly Asp His Asn Phe Leu Arg Arg Glu
            20                  25                  30

Ile Lys Ile Glu Gly Asp Leu Val Leu Gly Gly Leu Phe Pro Ile Asn
        35                  40                  45

Glu Lys Gly Thr Gly Thr Glu Glu Cys Gly Arg Ile Asn Glu Asp Arg
    50                  55                  60

Gly Ile Gln Arg Leu Glu Ala Met Leu Phe Ala Ile Asp Glu Ile Asn
65                  70                  75                  80

Lys Asp Asp Tyr Leu Leu Pro Gly Val Lys Leu Gly Val His Ile Leu
                85                  90                  95

Asp Thr Cys Ser Arg Asp Thr Tyr Ala Leu Glu Gln Ser Leu Glu Phe
            100                 105                 110

Val Arg Ala Ser Leu Thr Lys Val Asp Glu Ala Glu Tyr Met Cys Pro
        115                 120                 125

Asp Gly Ser Tyr Ala Ile Gln Glu Asn Ile Pro Leu Leu Ile Ala Gly
    130                 135                 140

Val Ile Gly Gly Ser Tyr Ser Ser Val Ser Ile Gln Val Ala Asn Leu
145                 150                 155                 160

Leu Arg Leu Phe Gln Ile Pro Gln Ile Ser Tyr Ala Ser Thr Ser Ala
                165                 170                 175

Lys Leu Ser Asp Lys Ser Arg Tyr Asp Tyr Phe Ala Arg Thr Val Pro
            180                 185                 190

Pro Asp Phe Tyr Gln Ala Lys Ala Met Ala Glu Ile Leu Arg Phe Phe
        195                 200                 205

Asn Trp Thr Tyr Val Ser Thr Val Ala Ser Glu Gly Asp Tyr Gly Glu
    210                 215                 220

Thr Gly Ile Glu Ala Phe Glu Gln Glu Ala Arg Leu Arg Asn Ile Cys
225                 230                 235                 240

Ile Ala Thr Ala Glu Lys Val Gly Arg Ser Asn Ile Arg Lys Ser Tyr
                245                 250                 255

Asp Ser Val Ile Arg Glu Leu Leu Gln Lys Pro Asn Ala Arg Val Val
            260                 265                 270

Val Leu Phe Met Arg Ser Asp Asp Ser Arg Glu Leu Ile Ala Ala Ala
        275                 280                 285

Ser Arg Ala Asn Ala Ser Phe Thr Trp Val Ala Ser Asp Gly Trp Gly
    290                 295                 300

Ala Gln Glu Ser Ile Ile Lys Gly Ser Glu His Val Ala Tyr Gly Ala
305                 310                 315                 320

Ile Thr Leu Glu Leu Ala Ser Gln Pro Val Arg Gln Phe Asp Arg Tyr
                325                 330                 335

Phe Gln Ser Leu Asn Pro Tyr Asn Asn His Arg Asn Pro Trp Phe Arg
            340                 345                 350

Asp Phe Trp Glu Gln Lys Phe Gln Cys Ser Leu Gln Asn Lys Arg Asn
        355                 360                 365

His Arg Arg Val Cys Asp Lys His Leu Ala Ile Asp Ser Ser Asn Tyr
    370                 375                 380

Glu Gln Glu Ser Lys Ile Met Phe Val Val Asn Ala Val Tyr Ala Met
385                 390                 395                 400

Ala His Ala Leu His Lys Met Gln Arg Thr Leu Cys Pro Asn Thr Thr
                405                 410                 415

Lys Leu Cys Asp Ala Met Lys Ile Leu Asp Gly Lys Lys Leu Tyr Lys
            420                 425                 430

Asp Tyr Leu Leu Lys Ile Asn Phe Thr Ala Pro Phe Asn Pro Asn Lys
        435                 440                 445

Asp Ala Asp Ser Ile Val Lys Phe Asp Thr Phe Gly Asp Gly Met Gly
    450                 455                 460

Arg Tyr Asn Val Phe Asn Phe Gln Asn Val Gly Gly Lys Tyr Ser Tyr
465                 470                 475                 480

Leu Lys Val Gly His Trp Ala Glu Thr Leu Ser Leu Asp Val Asn Ser
                485                 490                 495

Ile His Trp Ser Arg Asn Ser Val Pro Thr Ser Gln Cys Ser Asp Pro
            500                 505                 510

Cys Ala Pro Asn Glu Met Lys Asn Met Gln Pro Gly Asp Val Cys Cys
        515                 520                 525

Trp Ile Cys Ile Pro Cys Glu Pro Tyr Glu Tyr Leu Ala Asp Glu Phe
    530                 535                 540

Thr Cys Met Asp Cys Gly Ser Gly Gln Trp Pro Thr Ala Asp Leu Thr
545                 550                 555                 560

Gly Cys Tyr Asp Leu Pro Glu Asp Tyr Ile Arg Trp Glu Asp Ala Trp
                565                 570                 575

Ala Ile Gly Pro Val Thr Ile Ala Cys Leu Gly Phe Met Cys Thr Cys
            580                 585                 590

Met Val Val Thr Val Phe Ile Lys His Asn Asn Thr Pro Leu Val Lys
        595                 600                 605

Ala Ser Gly Arg Glu Leu Cys Tyr Ile Leu Leu Phe Gly Val Gly Leu
    610                 615                 620

Ser Tyr Cys Met Thr Phe Phe Phe Ile Ala Lys Pro Ser Pro Val Ile
625                 630                 635                 640

Cys Ala Leu Arg Arg Leu Gly Leu Gly Ser Ser Phe Ala Ile Cys Tyr
                645                 650                 655

Ser Ala Leu Leu Thr Lys Thr Asn Cys Ile Ala Arg Ile Phe Asp Gly
            660                 665                 670

Val Lys Asn Gly Ala Gln Arg Pro Lys Phe Ile Ser Pro Ser Ser Gln
        675                 680                 685

Val Phe Ile Cys Leu Gly Leu Ile Leu Val Gln Ile Val Met Val Ser
    690                 695                 700

Val Trp Leu Ile Leu Glu Ala Pro Gly Thr Arg Arg Tyr Thr Leu Ala
705                 710                 715                 720

Glu Lys Arg Glu Thr Val Ile Leu Lys Cys Asn Val Lys Asp Ser Ser
                725                 730                 735

Met Leu Ile Ser Leu Thr Tyr Asp Val Ile Leu Val Ile Leu Cys Thr
            740                 745                 750

Val Tyr Ala Phe Lys Thr Arg Lys Cys Pro Glu Asn Phe Asn Glu Ala
        755                 760                 765

Lys Phe Ile Gly Phe Thr Met Tyr Thr Thr Cys Ile Ile Trp Leu Ala
    770                 775                 780

Phe Leu Pro Ile Phe Tyr Val Thr Ser Ser Asp Tyr Arg Val Gln Thr
785                 790                 795                 800

Thr Thr Met Cys Ile Ser Val Ser Leu Ser Gly Phe Val Val Leu Gly
                805                 810                 815

Cys Leu Phe Ala Pro Lys Val His Ile Ile Leu Phe Gln Pro Gln Lys
            820                 825                 830

Asn Val Val Thr His Arg Leu His Leu Asn Arg Phe Ser Val Ser Gly
        835                 840                 845

Thr Gly Thr Thr Tyr Ser Gln Ser Ser Ala Ser Thr Tyr Val Pro Thr
    850                 855                 860

Val Cys Asn Gly Arg Glu Val Leu Asp Ser Thr Thr Ser Ser Leu
865                 870                 875

<210>  44
<211>  3962
<212>  DNA
<213>  human

<220>
<221>  CDS
<222>  (54)..(3161)

<400>  44
cgcgctcggc gccctgctcg ccgggcagag gggaaggcgg cggccggctg ggg atg      56
                                                           Met
                                                           1

ggc ggc ccg gct gcg ccg agg ggc gcc ggg agg ctc cgc gcg ctg ctg      104
Gly Gly Pro Ala Ala Pro Arg Gly Ala Gly Arg Leu Arg Ala Leu Leu
            5                   10                  15

ctg gcg ctg gtg gtc gcg ggg atc ccc gcg ggc gcc tac aac ctc gac      152
Leu Ala Leu Val Val Ala Gly Ile Pro Ala Gly Ala Tyr Asn Leu Asp
        20                  25                  30

ccg cag cgc ccc gtg cac ttc cag ggc ccc gct gac tcg ttc ttc ggc 200
Pro Gln Arg Pro Val His Phe Gln Gly Pro Ala Asp Ser Phe Phe Gly
    35                  40                  45

tac gca gtt ctg gag cat ttc cac gac aac acg cgc tgg gtc ctt gtg 248
Tyr Ala Val Leu Glu His Phe His Asp Asn Thr Arg Trp Val Leu Val
50                  55                  60                  65

ggc gca cca aag gca gat tcc aaa tac agc cct tca gtg aag tct cct 296
Gly Ala Pro Lys Ala Asp Ser Lys Tyr Ser Pro Ser Val Lys Ser Pro
                70                  75                  80

ggg gct gtg ttt aag tgc cgt gtt cac acc aac cct gac cgg aga tgc 344
Gly Ala Val Phe Lys Cys Arg Val His Thr Asn Pro Asp Arg Arg Cys
            85                  90                  95

acc gaa ctg gac atg gct cga ggg aag aat cgg ggc acg tcc tgc gga 392
Thr Glu Leu Asp Met Ala Arg Gly Lys Asn Arg Gly Thr Ser Cys Gly
        100                 105                 110

aag acc tgc cgg gaa gac cgc gat gat gag tgg atg ggg gtg agc ctg 440
Lys Thr Cys Arg Glu Asp Arg Asp Asp Glu Trp Met Gly Val Ser Leu
    115                 120                 125

gcc cga cag ccc aag gct gat ggc cgt gtg ttg gcc tgt gct cat cgc 488
Ala Arg Gln Pro Lys Ala Asp Gly Arg Val Leu Ala Cys Ala His Arg
130                 135                 140                 145

tgg aag aac atc tac tat gaa gcc gac cac atc cta ccc cat ggc ttc 536
Trp Lys Asn Ile Tyr Tyr Glu Ala Asp His Ile Leu Pro His Gly Phe
                150                 155                 160

tgc tac atc atc ccc tcc aac ctc cag gcc aaa ggc agg acg ctg atc 584
Cys Tyr Ile Ile Pro Ser Asn Leu Gln Ala Lys Gly Arg Thr Leu Ile
            165                 170                 175

cct tgc tat gaa gag tat aag aag aag tac gga gag gaa cac ggc tcc 632
Pro Cys Tyr Glu Glu Tyr Lys Lys Lys Tyr Gly Glu Glu His Gly Ser
        180                 185                 190

tgc cag gct ggg ata gcg ggc ttc ttc acc gag gag ctg gtg gtg atg 680
Cys Gln Ala Gly Ile Ala Gly Phe Phe Thr Glu Glu Leu Val Val Met
    195                 200                 205

ggt gct cca ggg tca ttt tat tgg gct gga acc atc aaa gtg ctg aac 728
Gly Ala Pro Gly Ser Phe Tyr Trp Ala Gly Thr Ile Lys Val Leu Asn
210                 215                 220                 225

ctt acg gac aac acc tat tta aaa ctg aac gac gaa gtg atc atg aac 776
Leu Thr Asp Asn Thr Tyr Leu Lys Leu Asn Asp Glu Val Ile Met Asn
                230                 235                 240

agg cgg tac acc tac ctg ggc tac gca gtg acc gct ggc cac ttc tct 824
Arg Arg Tyr Thr Tyr Leu Gly Tyr Ala Val Thr Ala Gly His Phe Ser
            245                 250                 255

cac ccg tcc acc att gat gtg gta gga ggt gcc cca cag gac aaa ggc 872
His Pro Ser Thr Ile Asp Val Val Gly Gly Ala Pro Gln Asp Lys Gly
        260                 265                 270

atc ggc aag gtt tat att ttc aga gct gac cga aga tca ggc acc tta 920
Ile Gly Lys Val Tyr Ile Phe Arg Ala Asp Arg Arg Ser Gly Thr Leu
    275                 280                 285

att aag atc ttt caa gca tca ggt aaa aag atg ggc tct tac ttc ggc 968
Ile Lys Ile Phe Gln Ala Ser Gly Lys Lys Met Gly Ser Tyr Phe Gly
290                 295                 300                 305

tcc tcc ttg tgc gca gtt gac ctg aat ggg gac ggc ctc tct gac ctg 1016
Ser Ser Leu Cys Ala Val Asp Leu Asn Gly Asp Gly Leu Ser Asp Leu
                310                 315                 320

ctg gtg ggg gcc ccc atg ttt tct gag atc agg gat gag gga cag gtc 1064
Leu Val Gly Ala Pro Met Phe Ser Glu Ile Arg Asp Glu Gly Gln Val
            325                 330                 335

act gtc tac atc aac aga gga aat gga gcc ctc gag gag cag ctg gct 1112
Thr Val Tyr Ile Asn Arg Gly Asn Gly Ala Leu Glu Glu Gln Leu Ala
        340                 345                 350

ctg act ggg gat ggt gcc tac aat gcg cac ttt gga gag agc att gcc 1160
Leu Thr Gly Asp Gly Ala Tyr Asn Ala His Phe Gly Glu Ser Ile Ala
    355                 360                 365

agc ctg gac gat ctg gac aat gat ggg ttc cca gat gtg gcc att ggt 1208
Ser Leu Asp Asp Leu Asp Asn Asp Gly Phe Pro Asp Val Ala Ile Gly
370                 375                 380                 385

gca ccc aag gag gat gac ttc gca ggg gcg gtc tat atc tat cat ggt 1256
Ala Pro Lys Glu Asp Asp Phe Ala Gly Ala Val Tyr Ile Tyr His Gly
                390                 395                 400

gat gcc ggt ggg ata gtc cct cag tac tca atg aaa ctg tct ggg cag 1304
Asp Ala Gly Gly Ile Val Pro Gln Tyr Ser Met Lys Leu Ser Gly Gln
            405                 410                 415

aag ata aat cca gtg ctc cgg atg ttt ggt cag tcc ata tcg gga ggc 1352
Lys Ile Asn Pro Val Leu Arg Met Phe Gly Gln Ser Ile Ser Gly Gly
        420                 425                 430

att gat atg gat gga aat ggc tat cct gat gtc act gtt gga gcc ttc 1400
Ile Asp Met Asp Gly Asn Gly Tyr Pro Asp Val Thr Val Gly Ala Phe
    435                 440                 445

atg tcc gac agc gtg gtt ctt ctc aga gca agg cct gtc att acg gtg 1448
Met Ser Asp Ser Val Val Leu Leu Arg Ala Arg Pro Val Ile Thr Val
450                 455                 460                 465

gat gtc tcc atc ttc ctc ccg ggc tcc atc aac atc aca gcg cct cag 1496
Asp Val Ser Ile Phe Leu Pro Gly Ser Ile Asn Ile Thr Ala Pro Gln
                470                 475                 480

tgt cac gac gga cag cag cct gtg aac tgc ctg aac gtc acc acc tgc 1544
Cys His Asp Gly Gln Gln Pro Val Asn Cys Leu Asn Val Thr Thr Cys
            485                 490                 495

ttc agc ttc cat ggc aaa cac gtt cca gga gag att ggc ctg aat tat 1592
Phe Ser Phe His Gly Lys His Val Pro Gly Glu Ile Gly Leu Asn Tyr
        500                 505                 510

gtt ctg atg gct gac gtg gcc aaa aag gag aag ggc cag atg ccc agg 1640
Val Leu Met Ala Asp Val Ala Lys Lys Glu Lys Gly Gln Met Pro Arg
    515                 520                 525

gtc tac ttt gtg ctg ctg gga gag acc atg ggt cag gtc aca gag aag 1688
Val Tyr Phe Val Leu Leu Gly Glu Thr Met Gly Gln Val Thr Glu Lys
530                 535                 540                 545

ctg cag ctg act tac atg gag gag acg tgt cgt cac tat gtg gcc cat 1736
Leu Gln Leu Thr Tyr Met Glu Glu Thr Cys Arg His Tyr Val Ala His
                550                 555                 560

gtg aag cgg agg gtg cag gac gtc atc agc ccg atc gtg ttt gaa gca 1784
Val Lys Arg Arg Val Gln Asp Val Ile Ser Pro Ile Val Phe Glu Ala
            565                 570                 575

gcc tac agc ctc agt gag cat gtg act gga gag gag gag agg gaa ctg     1832
Ala Tyr Ser Leu Ser Glu His Val Thr Gly Glu Glu Glu Arg Glu Leu
        580                 585                 590

ccg cct ctg aca cca gtt ctc cgc tgg aaa aag gga caa aag att gcc     1880
Pro Pro Leu Thr Pro Val Leu Arg Trp Lys Lys Gly Gln Lys Ile Ala
    595                 600                 605

caa aag aat cag act gtt ttt gaa agg aat tgc cgt tca gag gac tgt     1928
Gln Lys Asn Gln Thr Val Phe Glu Arg Asn Cys Arg Ser Glu Asp Cys
610                 615                 620                 625

gcc gca gac ctg cag ctt cag ggt aaa ctg ctg ctc tcc agt atg gat     1976
Ala Ala Asp Leu Gln Leu Gln Gly Lys Leu Leu Leu Ser Ser Met Asp
                630                 635                 640

gag aaa acc ctg tat cta gct ttg ggg gct gtg aag aac atc tcc cta     2024
Glu Lys Thr Leu Tyr Leu Ala Leu Gly Ala Val Lys Asn Ile Ser Leu
            645                 650                 655

aac atc tct atc tcc aac ctc gga gat gat gcc tat gat gcc aac gtg     2072
Asn Ile Ser Ile Ser Asn Leu Gly Asp Asp Ala Tyr Asp Ala Asn Val
        660                 665                 670

tcc ttc aat gtt tcc cgg gag ctc ttc ttc atc aac atg tgg cag aag     2120
Ser Phe Asn Val Ser Arg Glu Leu Phe Phe Ile Asn Met Trp Gln Lys
    675                 680                 685

gag gag atg ggc atc tcc tgt gag ctg ctg gaa tcg gac ttc ctc aaa     2168
Glu Glu Met Gly Ile Ser Cys Glu Leu Leu Glu Ser Asp Phe Leu Lys
690                 695                 700                 705

tgc agc gtg gga ttt cct ttc atg agg tca aag tca aag tat gaa ttc     2216
Cys Ser Val Gly Phe Pro Phe Met Arg Ser Lys Ser Lys Tyr Glu Phe
                710                 715                 720

agc gtg atc ttt gat aca agc cac ctg tct ggg gaa gag gaa gtt ctc     2264
Ser Val Ile Phe Asp Thr Ser His Leu Ser Gly Glu Glu Glu Val Leu
            725                 730                 735

agc ttc att gtt act gct cag agt ggc aac acg gag cgc tct gaa tcc     2312
Ser Phe Ile Val Thr Ala Gln Ser Gly Asn Thr Glu Arg Ser Glu Ser
        740                 745                 750

ctg cat gac aac acc ctc gtg ctg atg gtg cca ctg atg cac gag gtg     2360
Leu His Asp Asn Thr Leu Val Leu Met Val Pro Leu Met His Glu Val
    755                 760                 765

gac acg tcc atc acc gga atc atg tct cca acc tcc ttt gta tat ggc     2408
Asp Thr Ser Ile Thr Gly Ile Met Ser Pro Thr Ser Phe Val Tyr Gly
770                 775                 780                 785

gag tcc gtg gac gca gcc aac ttc att cag ctg gat gac ctg gag tgt     2456
Glu Ser Val Asp Ala Ala Asn Phe Ile Gln Leu Asp Asp Leu Glu Cys
                790                 795                 800

cac ttt cag ccc atc aat atc acc ctt cag gtc tac aac act ggc cca     2504
His Phe Gln Pro Ile Asn Ile Thr Leu Gln Val Tyr Asn Thr Gly Pro
            805                 810                 815

agc acc ctt cca ggg tca tct gtc agc atc tct ttc cct aat cga ctc     2552
Ser Thr Leu Pro Gly Ser Ser Val Ser Ile Ser Phe Pro Asn Arg Leu
        820                 825                 830

tca tct ggt ggt gca gag atg ttt cat gtc cag gaa atg gtg gtg ggc     2600
Ser Ser Gly Gly Ala Glu Met Phe His Val Gln Glu Met Val Val Gly
    835                 840                 845

caa gag aag gga aac tgc tct ttc cag aaa aac cca act ccc tgc atc 2648
Gln Glu Lys Gly Asn Cys Ser Phe Gln Lys Asn Pro Thr Pro Cys Ile
850                 855                 860                 865

atc cct caa gaa caa gaa aat atc ttc cac aca ata ttt gct ttt ttc 2696
Ile Pro Gln Glu Gln Glu Asn Ile Phe His Thr Ile Phe Ala Phe Phe
                870                 875                 880

aca aag tct gga aga aaa gtc ttg gac tgt gaa aaa cca gga att tct 2744
Thr Lys Ser Gly Arg Lys Val Leu Asp Cys Glu Lys Pro Gly Ile Ser
            885                 890                 895

tgc cta aca gca cac tgt aac ttt agt gct ctt gct aaa gaa gaa agt 2792
Cys Leu Thr Ala His Cys Asn Phe Ser Ala Leu Ala Lys Glu Glu Ser
        900                 905                 910

cgt act ata gac att tac atg ctg ctg aac aca gaa ata ctg aaa aag 2840
Arg Thr Ile Asp Ile Tyr Met Leu Leu Asn Thr Glu Ile Leu Lys Lys
    915                 920                 925

gac agt tcg tct gtc atc cag ttc atg tcc cgc gcc aag gtg aag gtg 2888
Asp Ser Ser Ser Val Ile Gln Phe Met Ser Arg Ala Lys Val Lys Val
930                 935                 940                 945

gat cct gcc cta agg gtg gtg gaa ata gct cat ggg aac cca gaa gag 2936
Asp Pro Ala Leu Arg Val Val Glu Ile Ala His Gly Asn Pro Glu Glu
                950                 955                 960

gtg acg gtg gtc ttc gag gcc ctg cac aat ctg gag ccc cgt ggc tac 2984
Val Thr Val Val Phe Glu Ala Leu His Asn Leu Glu Pro Arg Gly Tyr
            965                 970                 975

gtc gtg ggg tgg atc atc gcc atc agt ttg ttg gtg gga atc ctc atc 3032
Val Val Gly Trp Ile Ile Ala Ile Ser Leu Leu Val Gly Ile Leu Ile
        980                 985                 990

ttc ctg ctg ctg gcc gtg ctg ctc tgg aag atg ggc ttc ttt cgc cga 3080
Phe Leu Leu Leu Ala Val Leu Leu Trp Lys Met Gly Phe Phe Arg Arg
    995                 1000                1005

agg tac aaa gaa att atc gaa gct gag aag aac cgg aaa gag aat 3125
Arg Tyr Lys Glu Ile Ile Glu Ala Glu Lys Asn Arg Lys Glu Asn
1010                1015                1020

gaa gac agt tgg gac tgg gtc cag aaa aac cag tga gctgccacac 3171
Glu Asp Ser Trp Asp Trp Val Gln Lys Asn Gln
1025                1030                1035

cagtcacatg acctgatcac tagcctgtca tccttggtct ttgtatcttc catatttgga 3231

agaaaaaaat cttctccaga tttttcggag gccccactga tgctgttctc ttcttcattc 3291

tatcaagccc aggtgccagc ctgaggcagc cacttcggcc aggtcacacg accggggcca 3351

gcaccacttc ctttaaagat gaactctgaa ctttggagag tgagctacag agccgagcaa 3411

tatttatgga tgcaacacgc atggtcaacc ctcaggggaa aactgttacc taaagtattt 3471

ttataaatat aagcctttta tactgattat gtcttttata tttgtatcga tgttttatta 3531

tttctattaa atagttatat aattcactca agcactgata tctggcctaa aatcttggaa 3591

gtacatgtcc atgaatacaa attttaaagg atgaaaatct taccgtactt tggaacttgc 3651

tgtttaaaaa gacagatgaa ataagttgaa gaaacctcat gtaatgaatc caccaggctg 3711

gcagtggtgc atataaactg tgggtgtggc aagaccccga agacatttca catctttatc 3771

gcctcgatca agtgtggagt cacatgctaa tgtgtgctaa agaactgtaa gtgtttttc 3831
atatgtactt ttcattggaa gattcccaac aagaatttgg atggaaaacc tgatccctag 3891
caagaagtct gctctgtatc acctttatat agcagacatg tcaccctgct tctacacaga 3951
tgatggatga a 3962

<210> 45
<211> 1035
<212> PRT
<213> human

<400> 45

Met Gly Gly Pro Ala Ala Pro Arg Gly Ala Gly Arg Leu Arg Ala Leu
1               5                   10                  15

Leu Leu Ala Leu Val Val Ala Gly Ile Pro Ala Gly Ala Tyr Asn Leu
            20                  25                  30

Asp Pro Gln Arg Pro Val His Phe Gln Gly Pro Ala Asp Ser Phe Phe
        35                  40                  45

Gly Tyr Ala Val Leu Glu His Phe His Asp Asn Thr Arg Trp Val Leu
    50                  55                  60

Val Gly Ala Pro Lys Ala Asp Ser Lys Tyr Ser Pro Ser Val Lys Ser
65                  70                  75                  80

Pro Gly Ala Val Phe Lys Cys Arg Val His Thr Asn Pro Asp Arg Arg
                85                  90                  95

Cys Thr Glu Leu Asp Met Ala Arg Gly Lys Asn Arg Gly Thr Ser Cys
            100                 105                 110

Gly Lys Thr Cys Arg Glu Asp Arg Asp Asp Glu Trp Met Gly Val Ser
        115                 120                 125

Leu Ala Arg Gln Pro Lys Ala Asp Gly Arg Val Leu Ala Cys Ala His
    130                 135                 140

Arg Trp Lys Asn Ile Tyr Tyr Glu Ala Asp His Ile Leu Pro His Gly
145                 150                 155                 160

Phe Cys Tyr Ile Ile Pro Ser Asn Leu Gln Ala Lys Gly Arg Thr Leu
                165                 170                 175

Ile Pro Cys Tyr Glu Glu Tyr Lys Lys Lys Tyr Gly Glu Glu His Gly
            180                 185                 190

Ser Cys Gln Ala Gly Ile Ala Gly Phe Phe Thr Glu Glu Leu Val Val
        195                 200                 205

Met Gly Ala Pro Gly Ser Phe Tyr Trp Ala Gly Thr Ile Lys Val Leu
    210                 215                 220

Asn Leu Thr Asp Asn Thr Tyr Leu Lys Leu Asn Asp Glu Val Ile Met
225                 230                 235                 240

Asn Arg Arg Tyr Thr Tyr Leu Gly Tyr Ala Val Thr Ala Gly His Phe
                245                 250                 255

Ser His Pro Ser Thr Ile Asp Val Val Gly Gly Ala Pro Gln Asp Lys
            260                 265                 270

Gly Ile Gly Lys Val Tyr Ile Phe Arg Ala Asp Arg Arg Ser Gly Thr
        275                 280                 285

Leu Ile Lys Ile Phe Gln Ala Ser Gly Lys Lys Met Gly Ser Tyr Phe
    290                 295                 300

Gly Ser Ser Leu Cys Ala Val Asp Leu Asn Gly Asp Gly Leu Ser Asp
305                 310                 315                 320

Leu Leu Val Gly Ala Pro Met Phe Ser Glu Ile Arg Asp Glu Gly Gln
                325                 330                 335

Val Thr Val Tyr Ile Asn Arg Gly Asn Gly Ala Leu Glu Glu Gln Leu
            340                 345                 350

Ala Leu Thr Gly Asp Gly Ala Tyr Asn Ala His Phe Gly Glu Ser Ile
        355                 360                 365

Ala Ser Leu Asp Asp Leu Asp Asn Asp Gly Phe Pro Asp Val Ala Ile
    370                 375                 380

Gly Ala Pro Lys Glu Asp Asp Phe Ala Gly Ala Val Tyr Ile Tyr His
385                 390                 395                 400

Gly Asp Ala Gly Gly Ile Val Pro Gln Tyr Ser Met Lys Leu Ser Gly
                405                 410                 415

Gln Lys Ile Asn Pro Val Leu Arg Met Phe Gly Gln Ser Ile Ser Gly
            420                 425                 430

Gly Ile Asp Met Asp Gly Asn Gly Tyr Pro Asp Val Thr Val Gly Ala
        435                 440                 445

Phe Met Ser Asp Ser Val Val Leu Leu Arg Ala Arg Pro Val Ile Thr
    450                 455                 460

Val Asp Val Ser Ile Phe Leu Pro Gly Ser Ile Asn Ile Thr Ala Pro
465                 470                 475                 480

Gln Cys His Asp Gly Gln Gln Pro Val Asn Cys Leu Asn Val Thr Thr
                485                 490                 495

Cys Phe Ser Phe His Gly Lys His Val Pro Gly Glu Ile Gly Leu Asn
            500                 505                 510

Tyr Val Leu Met Ala Asp Val Ala Lys Lys Glu Lys Gly Gln Met Pro
        515                 520                 525

Arg Val Tyr Phe Val Leu Leu Gly Glu Thr Met Gly Gln Val Thr Glu
    530                 535                 540

Lys Leu Gln Leu Thr Tyr Met Glu Glu Thr Cys Arg His Tyr Val Ala
545                 550                 555                 560

His Val Lys Arg Arg Val Gln Asp Val Ile Ser Pro Ile Val Phe Glu
                565                 570                 575

Ala Ala Tyr Ser Leu Ser Glu His Val Thr Gly Glu Glu Glu Arg Glu
            580                 585                 590

Leu Pro Pro Leu Thr Pro Val Leu Arg Trp Lys Lys Gly Gln Lys Ile
        595                 600                 605

Ala Gln Lys Asn Gln Thr Val Phe Glu Arg Asn Cys Arg Ser Glu Asp
    610                 615                 620

Cys Ala Ala Asp Leu Gln Leu Gln Gly Lys Leu Leu Leu Ser Ser Met
625                 630                 635                 640

Asp Glu Lys Thr Leu Tyr Leu Ala Leu Gly Ala Val Lys Asn Ile Ser
                645                 650                 655

Leu Asn Ile Ser Ile Ser Asn Leu Gly Asp Asp Ala Tyr Asp Ala Asn
            660                 665                 670

Val Ser Phe Asn Val Ser Arg Glu Leu Phe Phe Ile Asn Met Trp Gln
        675                 680                 685

Lys Glu Glu Met Gly Ile Ser Cys Glu Leu Leu Glu Ser Asp Phe Leu
    690                 695                 700

Lys Cys Ser Val Gly Phe Pro Phe Met Arg Ser Lys Ser Lys Tyr Glu
705                 710                 715                 720

Phe Ser Val Ile Phe Asp Thr Ser His Leu Ser Gly Glu Glu Glu Val
                725                 730                 735

Leu Ser Phe Ile Val Thr Ala Gln Ser Gly Asn Thr Glu Arg Ser Glu
            740                 745                 750

Ser Leu His Asp Asn Thr Leu Val Leu Met Val Pro Leu Met His Glu
        755                 760                 765

Val Asp Thr Ser Ile Thr Gly Ile Met Ser Pro Thr Ser Phe Val Tyr
    770                 775                 780

Gly Glu Ser Val Asp Ala Ala Asn Phe Ile Gln Leu Asp Asp Leu Glu
785                 790                 795                 800

Cys His Phe Gln Pro Ile Asn Ile Thr Leu Gln Val Tyr Asn Thr Gly
                805                 810                 815

Pro Ser Thr Leu Pro Gly Ser Ser Val Ser Ile Ser Phe Pro Asn Arg
            820                 825                 830

Leu Ser Ser Gly Gly Ala Glu Met Phe His Val Gln Glu Met Val Val
        835                 840                 845

Gly Gln Glu Lys Gly Asn Cys Ser Phe Gln Lys Asn Pro Thr Pro Cys
    850                 855                 860

Ile Ile Pro Gln Glu Gln Glu Asn Ile Phe His Thr Ile Phe Ala Phe
865                 870                 875                 880

Phe Thr Lys Ser Gly Arg Lys Val Leu Asp Cys Glu Lys Pro Gly Ile
                885                 890                 895

Ser Cys Leu Thr Ala His Cys Asn Phe Ser Ala Leu Ala Lys Glu Glu
            900                 905                 910

Ser Arg Thr Ile Asp Ile Tyr Met Leu Leu Asn Thr Glu Ile Leu Lys
        915                 920                 925

Lys Asp Ser Ser Ser Val Ile Gln Phe Met Ser Arg Ala Lys Val Lys
    930                 935                 940

Val Asp Pro Ala Leu Arg Val Val Glu Ile Ala His Gly Asn Pro Glu
945                 950                 955                 960

Glu Val Thr Val Val Phe Glu Ala Leu His Asn Leu Glu Pro Arg Gly
                965                 970                 975

Tyr Val Val Gly Trp Ile Ile Ala Ile Ser Leu Leu Val Gly Ile Leu
            980                 985                 990

Ile Phe Leu Leu Leu Ala Val Leu  Leu Trp Lys Met Gly  Phe Phe Arg
        995                 1000                 1005

Arg Arg  Tyr Lys Glu Ile Ile  Glu Ala Glu Lys Asn  Arg Lys Glu
    1010                 1015                 1020

Asn Glu Asp Ser Trp Asp Trp Val Gln Lys Asn Gln
    1025                1030                1035

<210> 46
<211> 2976
<212> DNA
<213> human

<220>
<221> CDS
<222> (155)..(667)

<400> 46
gatgcacttc ttgactatat atcccaactg cagcagcgga gttgtcagag cgcagagccg 60

gacagagcag aagaaccctc ttggactgga cgatttggga attcaaaact tgggacaaac 120

tgtcagcctt gcccctgctg tggaggcagc ctca atg ctg aaa atg gag cct ctg 175
Met Leu Lys Met Glu Pro Leu
1               5

aac agc acg cac ccc ggc acc gcc gcc tcc agc agc ccc ctg gag tcc 223
Asn Ser Thr His Pro Gly Thr Ala Ala Ser Ser Ser Pro Leu Glu Ser
        10                  15                  20

cgt gcg gcc ggc ggc ggc agc ggc aat ggc aac gag tac ttc tac att 271
Arg Ala Ala Gly Gly Gly Ser Gly Asn Gly Asn Glu Tyr Phe Tyr Ile
    25                  30                  35

ctg gtt gtc atg tcc ttc tac ggc att ttc ttg atc gga atc atg ctg 319
Leu Val Val Met Ser Phe Tyr Gly Ile Phe Leu Ile Gly Ile Met Leu
40                  45                  50                  55

ggc tac atg aaa tcc aag agg cgg gag aag aag tcc agc ctc ctg ctg 367
Gly Tyr Met Lys Ser Lys Arg Arg Glu Lys Lys Ser Ser Leu Leu Leu
                60                  65                  70

ctg tac aaa gac gag gag cgg ctc tgg ggg gag gcc atg aag ccg ctg 415
Leu Tyr Lys Asp Glu Glu Arg Leu Trp Gly Glu Ala Met Lys Pro Leu
            75                  80                  85

cct gtg gtg tcg ggc ctg agg tcg gtg cag gtg ccc ctg atg ctg aac 463
Pro Val Val Ser Gly Leu Arg Ser Val Gln Val Pro Leu Met Leu Asn
        90                  95                  100

atg ctg cag gag agc gtg gcg ccc gcg ctg tcc tgc acc ctc tgt tcc 511
Met Leu Gln Glu Ser Val Ala Pro Ala Leu Ser Cys Thr Leu Cys Ser
    105                 110                 115

atg gaa ggg gac agc gtg agc tcc gag tcc tcc tcc ccg gac gtg cac 559
Met Glu Gly Asp Ser Val Ser Ser Glu Ser Ser Ser Pro Asp Val His
120                 125                 130                 135

ctc acc att cag gag gag ggg gca gac gat gag ctg gag gag acc tcg 607
Leu Thr Ile Gln Glu Glu Gly Ala Asp Asp Glu Leu Glu Glu Thr Ser
                140                 145                 150

gag acg ccc ctc aac gag agc agc gaa ggg tcc tcg gag aac atc cat 655
Glu Thr Pro Leu Asn Glu Ser Ser Glu Gly Ser Ser Glu Asn Ile His
            155                 160                 165

cag aat tcc tag cacccccggg acccctgccg gtggctccat cagccagcaa 707
Gln Asn Ser
        170

ccttagagag aggaaagaca gttttcaagt gtctggtttc actttcacag tgcggctgcc 767
actttgaaga gacccttggt aaacccctga ttcggggtgg ggtgggggac taggctcagc 827
cggaaccagc acctccaagg agtccgggag gtgcctgtgg tttgcaccca ccactgaaaa 887
agccgcggag atgcgcagcg cgtacactga ctttggggcc tgggtgttgg ggttctgatc 947
agaatttggc gggatgatat gtttgccatt ttctcactgg atgccctggg tagctcctgc 1007
agggtctgcc tgttcccagg gctgccgaat gcttaggaca cgctgagaga ctagttgtga 1067
tttgctattt tgcctagagc tttgtccttc tagatctgat tggctgtaag tatctctact 1127
gtgtacctgt ggcattcctt cacagtgggt tacaagcttc ttttggatta gagggggatt 1187
tttgatggga gaaagctgga gatctgaacc cagcccattt gcacactata agaaaaaaaa 1247
gtaactttta aacctgttaa cattggccgg ggttataaga gatgatcttc tattttgacc 1307
ttttgtctaa cttatgacct tgaactctga cctgtgacca tgcagcatca catgatggca 1367
tgacgttctt tggatcagaa gagcttcccc agaatctaac ctgcactccc gatggtggtt 1427
caggagactc ttcctgatct ttctagaagg ggtaaagtgg ggttgaacaa ggccaagccg 1487
ttagctctgc tgctgctcag tttcccagcc tagttttctg agctgggaga ggacataatg 1547
tggtatttcg tcacgtagct gagacctaga agggcatact caggcggaga ttgcacaaag 1607
ctgggctcca agttctgtgc ttcctgatcc cgagccctga cactcatttg ctgtgtggtc 1667
ccgggcatgt catcaagaag ctcgctgtct gcatgaggct cccgacgatc tccatcccca 1727
gggggttggg aggatgtctt tagattttag gactctgtga taaatattcc acagcctggt 1787
gtgaggaagc ttggaccaaa tgcttcccct ttggccggtt agtctgaaca aggatctgct 1847
gatttaaagg agcagttgga gattcagaac acatataact ggggaattca gggcaagcta 1907
ccaacccagc cctgctgtag ttttcccagg actgaaggaa agaggcagac accacattct 1967
cctctgcaaa gttctctgga gaatgtgtga tgttaacact cccagctaag ggaaagcaaa 2027
gtctaaagga aaacacacag gaatgcttcc aatgtctcca agtattcctg tggtcaacag 2087
tttcgcagat ccgttaggcc cttaaagaca aggaggaagt aaaaggtcaa atttaaatct 2147
atttaaaaaa acttttaaaa aaattattta tttattatta tttttatttt ttaagacaga 2207
atctcgctct gttgcccagg ctggagtgca gtggtgcaat cttggctcac tgcaacctcc 2267
acctcctggg tttcagcgat tctcctgcct cagcctcctg agtagctggg attacaggcg 2327
cctgccacca cgcccagcta attttttgat ttttagtaga gatggggttt cgccattttg 2387
gccaggctgg tctcaaactc ctggccttaa atgatcctcc cacctcggcc tcccaaagtg 2447
ctgggattac aggcatgagc cactgcaccc ggccactttt ttttttttta aagaaaaatg 2507
ctctgcatgg attggagaca cagcaataac tactgttgcc atggaagggt taacagtgta 2567
ggagctggtt tatcagtccg ctttgacata cagctaaagg aaatttatgt ttgggggaaa 2627
aaggccctct gttcacttta aaattcagtg tggacttatg ccaaaggggg ctgtttaagt 2687
tgaaagaagc caagttaagt ttggcctctt gcctggaatc acttgaattc tgaaatttca 2747

ctgcgacgga catgtgcctt gtcacatttt ccattgctta atcctgaagt ttggtgcaag 2807

tctctctgca cctattaaaa agtgatgtat atacttcctt cttattctgt tgagttgtat 2867

agaattgtct tttgtattta acactttgta attttcacaa tattttttaa tttaaataaa 2927

taaacacatt ttttccctcc tgggaagtca tgaaaaaaaa aaaaaaaaa 2976

<210> 47
<211> 170
<212> PRT
<213> human

<400> 47

Met Leu Lys Met Glu Pro Leu Asn Ser Thr His Pro Gly Thr Ala Ala
1               5                   10                  15

Ser Ser Ser Pro Leu Glu Ser Arg Ala Ala Gly Gly Gly Ser Gly Asn
            20                  25                  30

Gly Asn Glu Tyr Phe Tyr Ile Leu Val Val Met Ser Phe Tyr Gly Ile
        35                  40                  45

Phe Leu Ile Gly Ile Met Leu Gly Tyr Met Lys Ser Lys Arg Arg Glu
    50                  55                  60

Lys Lys Ser Ser Leu Leu Leu Leu Tyr Lys Asp Glu Glu Arg Leu Trp
65                  70                  75                  80

Gly Glu Ala Met Lys Pro Leu Pro Val Val Ser Gly Leu Arg Ser Val
                85                  90                  95

Gln Val Pro Leu Met Leu Asn Met Leu Gln Glu Ser Val Ala Pro Ala
            100                 105                 110

Leu Ser Cys Thr Leu Cys Ser Met Glu Gly Asp Ser Val Ser Ser Glu
        115                 120                 125

Ser Ser Ser Pro Asp Val His Leu Thr Ile Gln Glu Glu Gly Ala Asp
    130                 135                 140

Asp Glu Leu Glu Glu Thr Ser Glu Thr Pro Leu Asn Glu Ser Ser Glu
145                 150                 155                 160

Gly Ser Ser Glu Asn Ile His Gln Asn Ser
                165                 170

<210> 48
<211> 5909
<212> DNA
<213> human

<220>
<221> CDS

<222>  (61)..(3468)

<400>  48
   ctttctttct ccctcctctc ctccatttgt tgtttgatgt ttcccactct ttgaggaagg      60
   atg gtt gat ttg gag agc gaa gtg ccc cct ctg cct ccc agg tac agg      108
   ttt cga gat ttg ctg cta ggg gac caa gga tgg caa aac gac gac agg      156
   gta caa gtt gaa ttc tat atg aat gaa aat aca ttt aaa gaa aga cta      204
   aaa tta ttt ttc ata aaa aac cag aga tca agt cta agg ata cgc ctg      252
   ttc aat ttt tct ctc aaa tta cta agc tgc tta tta tac ata atc cga      300
   gta cta cta gaa aac cct tca caa gga aat gaa tgg tct cat atc ttt      348
   tgg gtg aac aga agt cta cct ttg tgg ggc tta cag gtt tca gtg gca      396
   ttg ata agt ctg ttt gaa aca ata tta ctt ggt tat ctt agt tat aag      444
   gga aac atc tgg gaa cag att tta cga ata ccc ttc atc ttg gaa ata      492
   att aat gca gtt ccc ttc att atc tca ata ttc tgg cct tcc tta agg      540
   aat cta ttt gtc cca gtc ttt ctg aac tgt tgg ctt gcc aaa cat gcc      588
   ttg gaa aat atg att aat gat cta cac aga gcc att cag cgt aca cag      636
   tct gca atg ttt aat caa gtt ttg att tta ata tct aca tta cta tgc      684
   ctt atc ttc acc tgc att tgt ggg atc caa cat ctg gaa cga ata gga      732
   aag aag ctg aat ctc ttt gac tcc ctt tat ttc tgc att gtg acg ttt      780
   tct act gtg ggc ttc ggg gat gtc act cct gaa aca tgg tcc tcc aag      828
   ctt ttt gta gtt gct atg att tgt gtt gct ctt gtg gtt cta ccc ata 876
   cag ttt gaa cag ctg gct tat ttg tgg atg gag aga caa aag tca gga 924
   gga aac tat agt cga cat aga gct caa act gaa aag cat gtc gtc ctg 972
   tgt gtc agc tca ctg aag att gat tta ctt atg gat ttt tta aat gaa 1020
   ttc tat gct cat cct agg ctc cag gat tat tat gtg gtg att ttg tgt 1068
   cct act gaa atg gat gta cag gtt cga agg gta ctg cag att cca atg 1116
   tgg tcc caa cga gtt atc tac ctt caa ggt tca gcc ctt aaa gat caa 1164
   gac cta ttg aga gca aag atg gat gac gct gag gcc tgt ttt att ctc 1212
   agt agc cgt tgt gaa gtg gat agg aca tca tct gat cac caa aca att 1260
   ttg aga gca tgg gct gtg aaa gat ttt gct cca aat tgt cct ttg tat 1308
   gtc cag ata tta aag cct gaa aat aaa ttt cac atc aaa ttt gct gat 1356
   cat gtt gtt tgt gaa gaa gag ttt aaa tac gcc atg tta gct tta aac 1404
   tgt ata tgc cca gca aca tct aca ctt att aca cta ctg gtt cat acc 1452
   tct aga ggg caa gaa ggc cag caa tcg cca gaa caa tgg cag aag atg 1500
   tac ggt aga tgc tcc ggg aat gaa gtc tac cac att gtt ttg gaa gaa 1548
   agt aca ttt ttt gct gaa tat gaa gga aag agt ttt aca tat gcc tct 1596
   ttc cat gca cac aaa aag ttt ggc gtc tgc ttg att ggt gtt agg agg 1644
   gag gat aat aaa aac att ttg ctg aat cca ggt cct cga tac att atg 1692
   aat tct aca gac ata tgc ttt tat att aat att acc aaa gaa gag aat 1740
   tca gca ttt aaa aac caa gac cag cag aga aaa agc aat gtg tcc agg 1788
   tcg ttt tat cat gga cct tcc aga tta cct gta cat agc ata att gcc 1836
   agc atg ggt act gtg gct ata gac ttg caa gat aca agc tgt aga tca 1884
   gca agt ggc cct acc ctg tct ctt cct aca gag gga agc aaa gaa ata 1932
   aga aga cct agc att gct cct gtt tta gag gtt gca gat aca tca tcg 1980
   att caa aca tgt gat ctt cta agt gac caa tca gaa gat gaa act aca 2028
   cca gat gaa gaa atg tct tca aac tta gag tat gct aaa ggt tac cca 2076
   cct tat tct cca tat ata gga agt tca ccc act ttt tgt cat ctc ctt 2124
   cat gaa aaa gta cca ttt tgc tgc tta aga tta gac aag agt tgc caa 2172
   cat aac tac tat gag gat gca aaa gcc tat gga ttc aaa aat aaa cta 2220
   att ata gtt gca gct gaa aca gct gga aat gga tta tat aac ttt att 2268
   gtt cct ctc agg gca tat tat aga cca aag aaa gaa ctt aat ccc ata 2316
   gta ctg cta ttg gat aac ccg cca gat atg cat ttt ctg gat gca atc 2364
   tgt tgg ttt cca atg gtt tac tac atg gtg ggc tct att gac aac cta 2412
   gat gac tta ctc agg tgt gga gtg act ttt gct gct aat atg gtg gtt 2460
   gtg gat aaa gag agc acc atg agt gcc gag gaa gac tac atg gca gat     2508
   gcc aaa acc att gtg aac gtg cag aca ctc ttc agg ttg ttt tcc agt     2556
   ctc agt att atc aca gag cta act cac ccc gcc aac atg aga ttc atg     2604
   caa ttc aga gcc aaa gac tgt tac tct ctt gct ctt tca aaa ctg gaa     2652
   aag aaa gaa cgg gag aga ggc tct aac ttg gcc ttt atg ttt cga ctg     2700
   cct ttt gct gct ggg agg gtg ttt agc atc agt atg ttg gac act ctg     2748
   ctg tat cag tca ttt gtg aag gat tat atg att tct atc acg aga ctt     2796
   ctg ttg gga ctg gac act aca cca gga tct ggg ttt ctt tgt tct atg     2844
   aaa atc act gca gat gac tta tgg atc aga act tat gcc aga ctt tat     2892
   cag aag ttg tgt tct tct act gga gat gtt ccc att gga atc tac agg     2940
   act gag tct cag aaa ctt act aca tct gag tct caa ata tct atc agt     2988
   gta gaa gag tgg gaa gac acc aaa gac tcc aaa gaa caa ggg cac cac     3036
   cgc agc aac cac cgc aac tca aca  tcc agt gac cag tcg  gac cat ccc     3084
   ttg ctg  cgg aga aaa agc atg  cag tgg gcc cga aga  ctg agc aga     3129
   aaa ggc  cca aaa cac tct ggt  aaa aca gct gaa aaa  ata acc cag     3174
   cag cga  ctg aac ctc tac agg  agg tca gaa aga caa  gag ctt gct     3219
   gaa ctt  gtg aaa aat aga atg  aaa cac ttg ggt ctt  tct aca gtg     3264
   gga tat gat gaa atg aat gat cat caa agt acc ctc tcc tac atc 3309
   ctg att aac cca tct cca gat acc aga ata gag ctg aat gat gtt 3354
   gta tac tta att cga cca gat cca ctg gcc tac ctt cca aac agt 3399
   gag ccc agt cga aga aac agc atc tgc aat gtc act ggt caa gat 3444
   tct cgg gag gaa act caa ctt tga taaaaataaa atgagaaact 3488
   tttttcctac aaagaccttg cttgaaacca caaaagtttt gctggcacga aagaaactag 3548
   atggaaatat atgtaattct ctcatattta aaaacgtaat ctcttctctt agaagtatag 3608
   atcattttga aacttaatgt actacttact ggtactctcc ctattaatat ttgaaggacc 3668
   tcaatggaat aaatttgaaa agctaaatta aaatacaaaa atttaaatct gacatttaat 3728
   tgttttataa taatccaaac tctatgaaag caattttaaa aattattaag gttttatgaa 3788
   gttgacaaaa tctaactata tttggtgcat cacaatggac acagaatgct gctgctcctc 3848
   ttaaaaatta aatgtgtcat attatattct ttaaacttac tgttttacaa aattgagctc 3908
   atcgtaaatg tctagtcttc tcacatagag attaaccaac aaacttgtgt ggctgacttt 3968
   tgtgtaagaa tcatagtttg ctttagaata caaatcttta agtcatttta actttttttt 4028
   ctgccttacg atataaaaat atttatctta gaatttgaga tgttcatagc atgttttatt 4088
   acattgaaga aactaaaaca taaatgaaaa gaaacactag gttcctgcac tttttggtaa 4148
   ctttatgtct agcaaatatt ttatgccaag aaaagcatac tataaagcaa atatctatta 4208
   ttctcctaaa cgaatgccta gcatagagaa aatacttaat acacatttgt tgacttaaat 4268
   ttaattcaag gattgaaaaa ttaactggat atcttgaaat atacagtaat gattgtcctt 4328
   agactcttga actttaccat ctttcctatt catatatcta tatagtaaat ttcactagaa 4388
   aaattctttt aaaattgaca gaagataatt tatacctttt atggactctg aagacacttc 4448
   aaaacattaa aagtccttat gtctttggta atgaaacaat aacactcaat gaaggatgta 4508
   ttaaaatttt tgacttaatt ttgaaatcgt atatatgagc tatactttaa cattatgaga 4568
   gaaaagcata aaacaaaaat aggtagttct tggcttttaa cattaatgca aatcatgcag 4628
   aatttgagtt ataaatttaa atataaattg accattgatg atatccagtt tttcattttt 4688
   tacctgtatt gcattttccc cctagagaaa tagatcaaaa gagcacaaga gtatgcgtac 4748
   atagtttacc aggtagtaga agtgtgttaa aatgttcctg taaaagaaac attggtaaat 4808
   ttaaatacat actgttctaa ttttgtattt tttaattttt gaatttgaca ttgagtttaa 4868
   ttcagcaaca acaaaaaata catataaaac tagaaaggga ctttttttcc tttcttttca 4928
   taatgaagca gatcaactta aaggataata aaatttttaa agaaaagata ttctaatgta 4988
   ctctcaataa ttctacagaa ataaaactgt aaagtgcaat gtgaaatcaa agattatagt 5048
   cattgtataa tttggcttgg aggctatgaa atgtcttttt ttctttttgg tattttacat 5108
   tattcacatt ttagaataac aagaacacca agaattaccc ctaaaacaga gaccctgtat 5168
   ttaatctact ttgatcagag aagtagaatt tataacaggt tagctaaaat tgggagcatg 5228
   ccttaaaact taaagattgt atgcatatat gtgtatatgt tataaacgtg aaatatattt 5288
   gacacacaca ttcacatata aattgttaaa aactgaaggc agaatggaac taatatatgt 5348
   aacagagaaa aacaataaat tttatgaact tgttttatat ttgcatatca agagtcaagt 5408
   atgatgtttc tttaagttga ctttttttac ttcattattt ttaggaataa atgtaagatt 5468
   ttacaaatct tttatttccc cacaagatct gaagtttggt atttttgcat tatgacagtt 5528
   gttgagacta ggattttaag ctaggatatg attatatttc ctatataact aaaaatttgg 5588
   tttcataaat tttaaaataa ttatttttga ctatgaacat tagtccaaat ttaatatttg 5648
   acacagttca taccagcttg ctacaataat gataatttat tagtctttct gttatttaaa 5708
   gaataaaaac atgcttataa aagactttta atgaaatgtt gcctttttaa aataattata 5768
   cttgcacatg aaaataaaat ataaagtcaa taatagtcct tgtagcccaa tgggaattga 5828
   ttctgtttat tgtctgtacc attttgctac cagttacatt gaactgcttt aaaataaata 5888
   ataaaattat ttctaatgat g 5909

<210> 49
<211> 1135
<212> PRT
<213> human

<400> 49

<210> 50
<211> 2932
<212> DNA
<213> human

<220>
<221> CDS
<222> (53)..(1915)

<400>  50
ggagagacat gcgattggtg accgagccga gcggaccgaa ggcgcgcccg ag atg cag      58
                                                          Met Gln
                                                          1

gtg agc aag agg atg ctg gcg ggg ggc gtg agg agc atg ccc agc ccc      106
Val Ser Lys Arg Met Leu Ala Gly Gly Val Arg Ser Met Pro Ser Pro
        5                   10                  15

ctc ctg gcc tgc tgg cag ccc atc ctc ctg ctg gtg ctg ggc tca gtg      154
Leu Leu Ala Cys Trp Gln Pro Ile Leu Leu Leu Val Leu Gly Ser Val
    20                  25                  30

ctg tca ggc tcg gcc acg ggc tgc ccg ccc cgc tgc gag tgc tcc gcc      202
Leu Ser Gly Ser Ala Thr Gly Cys Pro Pro Arg Cys Glu Cys Ser Ala
35                  40                  45                  50

cag gac cgc gct gtg ctg tgc cac cgc aag cgc ttt gtg gca gtc ccc      250
Gln Asp Arg Ala Val Leu Cys His Arg Lys Arg Phe Val Ala Val Pro
                55                  60                  65

gag ggc atc ccc acc gag acg cgc ctg ctg gac cta ggc aag aac cgc      298
Glu Gly Ile Pro Thr Glu Thr Arg Leu Leu Asp Leu Gly Lys Asn Arg
            70                  75                  80

atc aaa acg ctc aac cag gac gag ttc gcc agc ttc ccg cac ctg gag      346
Ile Lys Thr Leu Asn Gln Asp Glu Phe Ala Ser Phe Pro His Leu Glu
        85                  90                  95

gag ctg gag ctc aac gag aac atc gtg agc gcc gtg gag ccc ggc gcc      394
Glu Leu Glu Leu Asn Glu Asn Ile Val Ser Ala Val Glu Pro Gly Ala
    100                 105                 110

ttc aac aac ctc ttc aac ctc cgg acg ctg ggt ctc cgc agc aac cgc      442
Phe Asn Asn Leu Phe Asn Leu Arg Thr Leu Gly Leu Arg Ser Asn Arg
115                 120                 125                 130

ctg aag ctc atc ccg cta ggc gtc ttc act ggc ctc agc aac ctg acc      490
Leu Lys Leu Ile Pro Leu Gly Val Phe Thr Gly Leu Ser Asn Leu Thr
                135                 140                 145

aag ctg gac atc agc gag aac aag atc gtt atc cta ctg gac tac atg      538
Lys Leu Asp Ile Ser Glu Asn Lys Ile Val Ile Leu Leu Asp Tyr Met
            150                 155                 160

ttt cag gac ctg tac aac ctc aag tca ctg gag gtt ggc gac aat gac      586
Phe Gln Asp Leu Tyr Asn Leu Lys Ser Leu Glu Val Gly Asp Asn Asp
        165                 170                 175

ctc gtc tac atc tct cac cgc gcc ttc agc ggc ctc aac agc ctg gag      634
Leu Val Tyr Ile Ser His Arg Ala Phe Ser Gly Leu Asn Ser Leu Glu
    180                 185                 190

cag ctg acg ctg gag aaa tgc aac ctg acc tcc atc ccc acc gag gcg      682
Gln Leu Thr Leu Glu Lys Cys Asn Leu Thr Ser Ile Pro Thr Glu Ala
195                 200                 205                 210

ctg tcc cac ctg cac ggc ctc atc gtc ctg agg ctc cgg cac ctc aac      730
Leu Ser His Leu His Gly Leu Ile Val Leu Arg Leu Arg His Leu Asn
                215                 220                 225

atc aat gcc atc cgg gac tac tcc ttc aag agg ctg tac cga ctc aag      778
Ile Asn Ala Ile Arg Asp Tyr Ser Phe Lys Arg Leu Tyr Arg Leu Lys
            230                 235                 240

gtc ttg gag atc tcc cac tgg ccc tac ttg gac acc atg aca ccc aac      826
Val Leu Glu Ile Ser His Trp Pro Tyr Leu Asp Thr Met Thr Pro Asn

        245                 250                 255

tgc ctc tac ggc ctc aac ctg acg tcc ctg tcc atc aca cac tgc aat 874
Cys Leu Tyr Gly Leu Asn Leu Thr Ser Leu Ser Ile Thr His Cys Asn
    260                 265                 270

ctg acc gct gtg ccc tac ctg gcc gtc cgc cac cta gtc tat ctc cgc 922
Leu Thr Ala Val Pro Tyr Leu Ala Val Arg His Leu Val Tyr Leu Arg
275                 280                 285                 290

ttc ctc aac ctc tcc tac aac ccc atc agc acc att gag ggc tcc atg 970
Phe Leu Asn Leu Ser Tyr Asn Pro Ile Ser Thr Ile Glu Gly Ser Met
                295                 300                 305

ttg cat gag ctg ctc cgg ctg cag gag atc cag ctg gtg ggc ggg cag 1018
Leu His Glu Leu Leu Arg Leu Gln Glu Ile Gln Leu Val Gly Gly Gln
            310                 315                 320

ctg gcc gtg gtg gag ccc tat gcc ttc cgc ggc ctc aac tac ctg cgc 1066
Leu Ala Val Val Glu Pro Tyr Ala Phe Arg Gly Leu Asn Tyr Leu Arg
        325                 330                 335

gtg ctc aat gtc tct ggc aac cag ctg acc aca ctg gag gaa tca gtc 1114
Val Leu Asn Val Ser Gly Asn Gln Leu Thr Thr Leu Glu Glu Ser Val
    340                 345                 350

ttc cac tcg gtg ggc aac ctg gag aca ctc atc ctg gac tcc aac ccg 1162
Phe His Ser Val Gly Asn Leu Glu Thr Leu Ile Leu Asp Ser Asn Pro
355                 360                 365                 370

ctg gcc tgc gac tgt cgg ctc ctg tgg gtg ttc cgg cgc cgc tgg cgg 1210
Leu Ala Cys Asp Cys Arg Leu Leu Trp Val Phe Arg Arg Arg Trp Arg
                375                 380                 385

ctc aac ttc aac cgg cag cag ccc acg tgc gcc acg ccc gag ttt gtc 1258
Leu Asn Phe Asn Arg Gln Gln Pro Thr Cys Ala Thr Pro Glu Phe Val
            390                 395                 400

cag ggc aag gag ttc aag gac ttc cct gat gtg cta ctg ccc aac tac 1306
Gln Gly Lys Glu Phe Lys Asp Phe Pro Asp Val Leu Leu Pro Asn Tyr
        405                 410                 415

ttc acc tgc cgc cgc gcc cgc atc cgg gac cgc aag gcc cag cag gtg 1354
Phe Thr Cys Arg Arg Ala Arg Ile Arg Asp Arg Lys Ala Gln Gln Val
    420                 425                 430

ttt gtg gac gag ggc cac acg gtg cag ttt gtg tgc cgg gcc gat ggc 1402
Phe Val Asp Glu Gly His Thr Val Gln Phe Val Cys Arg Ala Asp Gly
435                 440                 445                 450

gac ccg ccg ccc gcc atc ctc tgg ctc tca ccc cga aag cac ctg gtc 1450
Asp Pro Pro Pro Ala Ile Leu Trp Leu Ser Pro Arg Lys His Leu Val
                455                 460                 465

tca gcc aag agc aat ggg cgg ctc aca gtc ttc cct gat ggc acg ctg 1498
Ser Ala Lys Ser Asn Gly Arg Leu Thr Val Phe Pro Asp Gly Thr Leu
            470                 475                 480

gag gtg cgc tac gcc cag gta cag gac aac ggc acg tac ctg tgc atc 1546
Glu Val Arg Tyr Ala Gln Val Gln Asp Asn Gly Thr Tyr Leu Cys Ile
        485                 490                 495

gcg gcc aac gcg ggc ggc aac gac tcc atg ccc gcc cac ctg cat gtg 1594
Ala Ala Asn Ala Gly Gly Asn Asp Ser Met Pro Ala His Leu His Val
    500                 505                 510

cgc agc tac tcg ccc gac tgg ccc cat cag ccc aac aag acc ttc gct 1642
Arg Ser Tyr Ser Pro Asp Trp Pro His Gln Pro Asn Lys Thr Phe Ala

515                 520                 525                 530

ttc atc tcc aac cag ccg ggc gag gga gag gcc aac agc acc cgc gcc      1690
Phe Ile Ser Asn Gln Pro Gly Glu Gly Glu Ala Asn Ser Thr Arg Ala
                535                 540                 545

act gtg cct ttc ccc ttc gac atc aag acc ctc atc atc gcc acc acc      1738
Thr Val Pro Phe Pro Phe Asp Ile Lys Thr Leu Ile Ile Ala Thr Thr
            550                 555                 560

atg ggc ttc atc tct ttc ctg ggc gtc gtc ctc ttc tgc ctg gtg ctg      1786
Met Gly Phe Ile Ser Phe Leu Gly Val Val Leu Phe Cys Leu Val Leu
        565                 570                 575

ctg ttt ctc tgg agc cgg ggc aag ggc aac aca aag cac aac atc gag      1834
Leu Phe Leu Trp Ser Arg Gly Lys Gly Asn Thr Lys His Asn Ile Glu
    580                 585                 590

atc gag tat gtg ccc cga aag tcg gac gca ggc atc agc tcc gcc gac      1882
Ile Glu Tyr Val Pro Arg Lys Ser Asp Ala Gly Ile Ser Ser Ala Asp
595                 600                 605                 610

gcg ccc cgc aag ttc aac atg aag atg ata tga ggccggggcg gggggcaggg    1935
Ala Pro Arg Lys Phe Asn Met Lys Met Ile
                615                 620

acccccgggc ggccgggcag gggaaggggc ctggccgcca cctgctcact ctccagtcct      1995

tcccacctcc tccctaccct tctacacacg ttctctttct ccctcccgcc tccgtcccct      2055

gctgcccccc gccagccctc accacctgcc ctccttctac caggacctca gaagcccaga      2115

cctggggacc ccacctacac aggggcattg acagactgga gttgaaagcc gacgaaccga      2175

cacgcggcag agtcaataat tcaataaaaa agttacgaac tttctctgta acttgggttt      2235

caataattat ggatttttat gaaaacttga aataataaaa agagaaaaaa actatttcct      2295

atagctagtc ggaatgcaaa cttttgacgt cctgattgct ccagggccct cttccaactc      2355

agtttcttgt ttttctcttc ctcctcctcc tcttcttcct cctttctctt ctcttcccca      2415

gtggggaggg atcactcagg aaaacaggaa aggaggttcc agccccaccc acctgcccac      2475

cccgccccag gcaccatcag gagcaggcta gggggcaggc ctgggcccag ctccgggctg      2535

gctttttgca gggcgcaggt ggaggggaca ggtctgccga tgggggtggg agcctgtctg      2595

ctgggctgcc aggcggcacc actgcaaggg gtgggagcct ggcttgggtg tggctgagac      2655

tctggacaga ggctggggtc ctcctggggg acagcacagt cagtggagag agccaggggc      2715

tggaggtggg gcccacccca gcctctggtc ccagctctgc tgctcacttg ctgtgtggcc      2775

tcaagcaggt cactggcctc tctgggcctc agtctccaca tctgtacaaa tgggaacatt      2835

accccctgcc ctgcctacct cacagggctg ttgtgaggaa ttgatgagat gatgtatgtg      2895

aaacactttg taacctgtaa agcgctgtgc acacgtg                             2932

<210>  51
<211>  620
<212>  PRT
<213>  human

<400>  51

Met Gln Val Ser Lys Arg Met Leu Ala Gly Gly Val Arg Ser Met Pro
1               5                   10                  15

Ser Pro Leu Leu Ala Cys Trp Gln Pro Ile Leu Leu Leu Val Leu Gly
            20                  25                  30

Ser Val Leu Ser Gly Ser Ala Thr Gly Cys Pro Pro Arg Cys Glu Cys
        35                  40                  45

Ser Ala Gln Asp Arg Ala Val Leu Cys His Arg Lys Arg Phe Val Ala
    50                  55                  60

Val Pro Glu Gly Ile Pro Thr Glu Thr Arg Leu Leu Asp Leu Gly Lys
65                  70                  75                  80

Asn Arg Ile Lys Thr Leu Asn Gln Asp Glu Phe Ala Ser Phe Pro His
                85                  90                  95

Leu Glu Glu Leu Glu Leu Asn Glu Asn Ile Val Ser Ala Val Glu Pro
            100                 105                 110

Gly Ala Phe Asn Asn Leu Phe Asn Leu Arg Thr Leu Gly Leu Arg Ser
        115                 120                 125

Asn Arg Leu Lys Leu Ile Pro Leu Gly Val Phe Thr Gly Leu Ser Asn
    130                 135                 140

Leu Thr Lys Leu Asp Ile Ser Glu Asn Lys Ile Val Ile Leu Leu Asp
145                 150                 155                 160

Tyr Met Phe Gln Asp Leu Tyr Asn Leu Lys Ser Leu Glu Val Gly Asp
                165                 170                 175

Asn Asp Leu Val Tyr Ile Ser His Arg Ala Phe Ser Gly Leu Asn Ser
            180                 185                 190

Leu Glu Gln Leu Thr Leu Glu Lys Cys Asn Leu Thr Ser Ile Pro Thr
        195                 200                 205

Glu Ala Leu Ser His Leu His Gly Leu Ile Val Leu Arg Leu Arg His
    210                 215                 220

Leu Asn Ile Asn Ala Ile Arg Asp Tyr Ser Phe Lys Arg Leu Tyr Arg
225                 230                 235                 240

Leu Lys Val Leu Glu Ile Ser His Trp Pro Tyr Leu Asp Thr Met Thr
                245                 250                 255

Pro Asn Cys Leu Tyr Gly Leu Asn Leu Thr Ser Leu Ser Ile Thr His
            260                 265                 270

Cys Asn Leu Thr Ala Val Pro Tyr Leu Ala Val Arg His Leu Val Tyr
        275                 280                 285

Leu Arg Phe Leu Asn Leu Ser Tyr Asn Pro Ile Ser Thr Ile Glu Gly
    290                 295                 300

Ser Met Leu His Glu Leu Leu Arg Leu Gln Glu Ile Gln Leu Val Gly
305                 310                 315                 320

Gly Gln Leu Ala Val Val Glu Pro Tyr Ala Phe Arg Gly Leu Asn Tyr
                325                 330                 335

Leu Arg Val Leu Asn Val Ser Gly Asn Gln Leu Thr Thr Leu Glu Glu
            340                 345                 350

Ser Val Phe His Ser Val Gly Asn Leu Glu Thr Leu Ile Leu Asp Ser
        355                 360                 365

Asn Pro Leu Ala Cys Asp Cys Arg Leu Leu Trp Val Phe Arg Arg Arg
    370                 375                 380

Trp Arg Leu Asn Phe Asn Arg Gln Gln Pro Thr Cys Ala Thr Pro Glu
385                 390                 395                 400

Phe Val Gln Gly Lys Glu Phe Lys Asp Phe Pro Asp Val Leu Leu Pro
                405                 410                 415

Asn Tyr Phe Thr Cys Arg Arg Ala Arg Ile Arg Asp Arg Lys Ala Gln
            420                 425                 430

Gln Val Phe Val Asp Glu Gly His Thr Val Gln Phe Val Cys Arg Ala
        435                 440                 445

Asp Gly Asp Pro Pro Pro Ala Ile Leu Trp Leu Ser Pro Arg Lys His
    450                 455                 460

Leu Val Ser Ala Lys Ser Asn Gly Arg Leu Thr Val Phe Pro Asp Gly
465                 470                 475                 480

Thr Leu Glu Val Arg Tyr Ala Gln Val Gln Asp Asn Gly Thr Tyr Leu
                485                 490                 495

Cys Ile Ala Ala Asn Ala Gly Gly Asn Asp Ser Met Pro Ala His Leu
            500                 505                 510

His Val Arg Ser Tyr Ser Pro Asp Trp Pro His Gln Pro Asn Lys Thr
        515                 520                 525

Phe Ala Phe Ile Ser Asn Gln Pro Gly Glu Gly Glu Ala Asn Ser Thr
    530                 535                 540

Arg Ala Thr Val Pro Phe Pro Phe Asp Ile Lys Thr Leu Ile Ile Ala
545                 550                 555                 560

Thr Thr Met Gly Phe Ile Ser Phe Leu Gly Val Val Leu Phe Cys Leu
                565                 570                 575

Val Leu Leu Phe Leu Trp Ser Arg Gly Lys Gly Asn Thr Lys His Asn
            580                 585                 590

Ile Glu Ile Glu Tyr Val Pro Arg Lys Ser Asp Ala Gly Ile Ser Ser
        595                 600                 605

Ala Asp Ala Pro Arg Lys Phe Asn Met Lys Met Ile
    610                 615                 620

<210>  52
<211>  2798
<212>  DNA
<213>  human

<220>
<221>  CDS
<222>  (1618)..(2787)

<400>  52
aaactggccc tggccctgaa ccaaatacct tgaaccctcg taaactccat accctgaccc      60
ccttgttttg gatataccca ggtagaacaa ctctctctca ctgtctgttg tgaggatacg     120
ctgtagccca ctcattaagt acattctcct aataaatgct ttggactgat caccctgcca     180
gtcttttgtc ttgggcaatc tatacttttc tcagaggttc ccaaggccta ctgaagggac     240
ttaacatact cttaatggct ttcctctctc ttgttttacc ttatgccctc acttcctgag     300
ttaacctccc aaatacagga tcacctgtac ccaagccctt agctcaagaa tacaggatca     360
cctgtaccca agcccttagc tcaagctctg ctttggaaga acccaaacta agacagtgct     420
cctggtgccc tccccaagca acctcaagtt ctggctgtta cttgagcaga ggcctttctt     480
ttcccttccc ccagctctat ccatctgcca ggcccccctc aaatctcttc atttccaagt     540
tttgcttgac ttttccaaga ggagagggct gcttcttagt atgtccctac tcatcctttc     600
ctttcttgtc ttgtatcctg gtgcagcctg gtaatggggc ctcttcatgg ttgtgtgtca     660
tgactcccta accattatgc ctccatgcat cccctgttcc tcctggaacc tagcaccatg     720
ccttacatgg aaaagctgtc attgacagcc cggtgagagc cctgagggtg gagtgactgg     780
ggcagggcct gaggcaagag gtgggaggag gtaggaggcc aggggctcag ccggaccagg     840
agactggaaa caggcaagga taaggcaggt gggggactga gttgtttggg tcacctctgc     900
aggccagaga gaccaggcaa catacacact gcagaaggtg ggctgggagg attggggcca     960
gagctggggg agggatgaga acagaagcag gaccaggatt cagcagagtc ctcctatttc    1020
cttccaccac cagggaatct tactgcccca cttcagcttg tgctgtttcc tggcaaggca    1080
ggctctcaca tgcctggacg cctgggtgcg ttggtgatgg gaaggagcag ggtgagggag    1140

gggccccagg agaggcccag gatgagcctc atcttgtccc tccccattct tgtcttaccc 1200

tctgcaaatg tgataggcac aggacaggag taggcacctc gcctactgct gcttaacctt 1260

tcagcttctc caggccccca atcctgcttg ctcccagctt ggtaagtaga tctgtgcacg 1320

tccctttaca ccccaccatc cagttttgcc cagatgtgct agaatggggc tggacaaaga 1380

aggaggggcc agactagagg agtggtggta gagatagtga cagcctgggg tgaggacttt 1440

atgcctgttt accactgagc tctgggaagg aggccaggag tggggcaggt caactgactg 1500

ggagcagggg atctgggttc caagaaggag ttgtgtttga ggtggggtct gggtcctcgt 1560

ggaagtcagg actcccaggc agaaaagagg caggctgcag ggaagtaagg aggaggc 1617

atg gca cct tct cat cgg gca tca cag gtg ggg ttt tgc ccc acc cct 1665
Met Ala Pro Ser His Arg Ala Ser Gln Val Gly Phe Cys Pro Thr Pro
1               5                   10                  15

gaa cgc cct ctg tgg cgc ctt cca ccc acc tgt agg ccc aga agg atg 1713
Glu Arg Pro Leu Trp Arg Leu Pro Pro Thr Cys Arg Pro Arg Arg Met
            20                  25                  30

tcg gtc tgc tac cgt ccc cca ggg aac gag aca ctg ctg agc tgg aag 1761
Ser Val Cys Tyr Arg Pro Pro Gly Asn Glu Thr Leu Leu Ser Trp Lys
        35                  40                  45

act tcg cgg gcc aca ggc aca gcc ttc ctg ctg ctg gcg gcg ctg ctg 1809
Thr Ser Arg Ala Thr Gly Thr Ala Phe Leu Leu Leu Ala Ala Leu Leu
    50                  55                  60

ggg ctg cct ggc aac ggc ttc gtg gtg tgg agc ttg gcg ggc tgg cgg 1857
Gly Leu Pro Gly Asn Gly Phe Val Val Trp Ser Leu Ala Gly Trp Arg
65                  70                  75                  80

cct gca cgg ggg cga ccg ctg gcg gcc acg ctt gtg ctg cac ctg gcg 1905
Pro Ala Arg Gly Arg Pro Leu Ala Ala Thr Leu Val Leu His Leu Ala
                85                  90                  95

ctg gcc gac ggc gcg gtg ctg ctg ctc acg ccg ctc ttt gtg gcc ttc 1953
Leu Ala Asp Gly Ala Val Leu Leu Leu Thr Pro Leu Phe Val Ala Phe
            100                 105                 110

ctg acc cgg cag gcc tgg ccg ctg ggc cag gcg ggc tgc aag gcg gtg 2001
Leu Thr Arg Gln Ala Trp Pro Leu Gly Gln Ala Gly Cys Lys Ala Val
        115                 120                 125

tac tac gtg tgc gcg ctc agc atg tac gcc agc gtg ctg ctc acc ggc 2049
Tyr Tyr Val Cys Ala Leu Ser Met Tyr Ala Ser Val Leu Leu Thr Gly
    130                 135                 140

ctg ctc agc ctg cag cgc tgc ctc gca gtc acc cgc ccc ttc ctg gcg 2097
Leu Leu Ser Leu Gln Arg Cys Leu Ala Val Thr Arg Pro Phe Leu Ala
145                 150                 155                 160

cct cgg ctg cgc agc ccg gcc ctg gcc cgc cgc ctg ctg ctg gcg gtc 2145
Pro Arg Leu Arg Ser Pro Ala Leu Ala Arg Arg Leu Leu Leu Ala Val
                165                 170                 175

tgg ctg gcc gcc ctg ttg ctc gcc gtc ccg gcc gcc gtc tac cgc cac 2193
Trp Leu Ala Ala Leu Leu Leu Ala Val Pro Ala Ala Val Tyr Arg His
            180                 185                 190

ctg tgg agg gac cgc gta tgc cag ctg tgc cac ccg tcg ccg gtc cac 2241
Leu Trp Arg Asp Arg Val Cys Gln Leu Cys His Pro Ser Pro Val His
        195                 200                 205

gcc gcc gcc cac ctg agc ctg gag act ctg acc gct ttc gtg ctt cct 2289
Ala Ala Ala His Leu Ser Leu Glu Thr Leu Thr Ala Phe Val Leu Pro
    210                 215                 220

ttc ggg ctg atg ctc ggc tgc tac agc gtg acg ctg gca cgg ctg cgg 2337
Phe Gly Leu Met Leu Gly Cys Tyr Ser Val Thr Leu Ala Arg Leu Arg
225                 230                 235                 240

ggc gcc cgc tgg ggc tcc ggg cgg cac ggg gcg cgg gtg ggc cgg ctg 2385
Gly Ala Arg Trp Gly Ser Gly Arg His Gly Ala Arg Val Gly Arg Leu
                245                 250                 255

gtg agc gcc atc gtg ctt gcc ttc ggc ttg ctc tgg gcc ccc tac cac 2433
Val Ser Ala Ile Val Leu Ala Phe Gly Leu Leu Trp Ala Pro Tyr His
            260                 265                 270

gca gtc aac ctt ctg cag gcg gtc gca gcg ctg gct cca ccg gaa ggg 2481
Ala Val Asn Leu Leu Gln Ala Val Ala Ala Leu Ala Pro Pro Glu Gly
        275                 280                 285

gcc ttg gcg aag ctg ggc gga gcc ggc cag gcg gcg cga gcg gga act 2529
Ala Leu Ala Lys Leu Gly Gly Ala Gly Gln Ala Ala Arg Ala Gly Thr
    290                 295                 300

acg gcc ttg gcc ttc ttc agt tct agc gtc aac ccg gtg ctc tac gtc 2577
Thr Ala Leu Ala Phe Phe Ser Ser Ser Val Asn Pro Val Leu Tyr Val
305                 310                 315                 320

ttc acc gct gga gat ctg ctg ccc cgg gca ggt ccc cgt ttc ctc acg 2625
Phe Thr Ala Gly Asp Leu Leu Pro Arg Ala Gly Pro Arg Phe Leu Thr
                325                 330                 335

cgg ctc ttc gaa ggc tct ggg gag gcc cga ggg ggc ggc cgc tct agg 2673
Arg Leu Phe Glu Gly Ser Gly Glu Ala Arg Gly Gly Gly Arg Ser Arg
            340                 345                 350

gaa ggg acc atg gag ctc cga act acc cct cag ctg aaa gtg gtg ggg 2721
Glu Gly Thr Met Glu Leu Arg Thr Thr Pro Gln Leu Lys Val Val Gly
        355                 360                 365

cag ggc cgc ggc aat gga gac ccg ggg ggt ggg atg gag aag gac ggt 2769
Gln Gly Arg Gly Asn Gly Asp Pro Gly Gly Gly Met Glu Lys Asp Gly
    370                 375                 380

ccg gaa tgg gac ctt tga cagcagaccc t 2798
Pro Glu Trp Asp Leu
385

<210> 53
<211> 389
<212> PRT
<213> human

<400> 53

Met Ala Pro Ser His Arg Ala Ser Gln Val Gly Phe Cys Pro Thr Pro
1               5                   10                  15

Glu Arg Pro Leu Trp Arg Leu Pro Pro Thr Cys Arg Pro Arg Arg Met
            20                  25                  30

Ser Val Cys Tyr Arg Pro Pro Gly Asn Glu Thr Leu Leu Ser Trp Lys
        35                  40                  45

Thr Ser Arg Ala Thr Gly Thr Ala Phe Leu Leu Leu Ala Ala Leu Leu
    50                  55                  60

Gly Leu Pro Gly Asn Gly Phe Val Val Trp Ser Leu Ala Gly Trp Arg
65                  70                  75                  80

Pro Ala Arg Gly Arg Pro Leu Ala Ala Thr Leu Val Leu His Leu Ala
                85                  90                  95

Leu Ala Asp Gly Ala Val Leu Leu Leu Thr Pro Leu Phe Val Ala Phe
            100                 105                 110

Leu Thr Arg Gln Ala Trp Pro Leu Gly Gln Ala Gly Cys Lys Ala Val
        115                 120                 125

Tyr Tyr Val Cys Ala Leu Ser Met Tyr Ala Ser Val Leu Leu Thr Gly
    130                 135                 140

Leu Leu Ser Leu Gln Arg Cys Leu Ala Val Thr Arg Pro Phe Leu Ala
145                 150                 155                 160

Pro Arg Leu Arg Ser Pro Ala Leu Ala Arg Arg Leu Leu Leu Ala Val
                165                 170                 175

Trp Leu Ala Ala Leu Leu Leu Ala Val Pro Ala Ala Val Tyr Arg His
            180                 185                 190

Leu Trp Arg Asp Arg Val Cys Gln Leu Cys His Pro Ser Pro Val His
        195                 200                 205

Ala Ala Ala His Leu Ser Leu Glu Thr Leu Thr Ala Phe Val Leu Pro
    210                 215                 220

Phe Gly Leu Met Leu Gly Cys Tyr Ser Val Thr Leu Ala Arg Leu Arg
225                 230                 235                 240

Gly Ala Arg Trp Gly Ser Gly Arg His Gly Ala Arg Val Gly Arg Leu
                245                 250                 255

Val Ser Ala Ile Val Leu Ala Phe Gly Leu Leu Trp Ala Pro Tyr His
            260                 265                 270

Ala Val Asn Leu Leu Gln Ala Val Ala Ala Leu Ala Pro Pro Glu Gly
        275                 280                 285

Ala Leu Ala Lys Leu Gly Gly Ala Gly Gln Ala Ala Arg Ala Gly Thr
    290                 295                 300

Thr Ala Leu Ala Phe Phe Ser Ser Ser Val Asn Pro Val Leu Tyr Val
305                 310                 315                 320

Phe Thr Ala Gly Asp Leu Leu Pro Arg Ala Gly Pro Arg Phe Leu Thr
                325                 330                 335

Arg Leu Phe Glu Gly Ser Gly Glu Ala Arg Gly Gly Gly Arg Ser Arg
            340                 345                 350

Glu Gly Thr Met Glu Leu Arg Thr Thr Pro Gln Leu Lys Val Val Gly
        355                 360                 365

Gln Gly Arg Gly Asn Gly Asp Pro Gly Gly Gly Met Glu Lys Asp Gly
    370                 375                 380

Pro Glu Trp Asp Leu
385

<210>  54
<211>  6163
<212>  DNA
<213>  human

<220>
<221>  CDS
<222>  (492)..(3677)

<400>  54
   caggactgga gggcttgagg aatgtggtgg tccctctctt cgttgtttct gccccctgag      60
   gttgtgcttt ctcagggata tgcactctgc acttccattc ctgcagtgaa attaactcga     120
   gcttggcagc tcgagaggag aatgtggcca ctttccgagg ctcagagtat ctgtgctacg     180
   acctgtctca gaacccgatc cagagcagca gtgatgaaat caccctctcc tttaagacct     240
   ggcagcgtaa cggcctcatc ctgcacacgg gcaagtcggc tgactatgtc aacctggctc     300
   tgaaggatgg tgcggtctcc ttggtcatta acctggggtc cggggccttt gaggccattg     360
   tggagccagt gaatggaaaa ttcaacgaca acgcctggca tgatgtcaaa gtgacacgca     420
   acctccggca ggtgacaatc tctgtggatg gcattcttac cacgacgggc tacactcaag     480
   aggactatac c atg ctg ggc tcg gac gac ttc ttc tat gta gga gga agc      530
                Met Leu Gly Ser Asp Asp Phe Phe Tyr Val Gly Gly Ser
                1               5                   10
   cca agt acc gct gac ttg cct ggc tcc cct gtc agc aac aac ttc atg      578
   Pro Ser Thr Ala Asp Leu Pro Gly Ser Pro Val Ser Asn Asn Phe Met
       15                  20                  25
   ggc tgc ctt aaa gag gtt gtt tat aag aat aat gac atc cgt ctg gag      626
   Gly Cys Leu Lys Glu Val Val Tyr Lys Asn Asn Asp Ile Arg Leu Glu
   30                  35                  40                  45
   ctg tct cgc ctg gcc cgg att gcg gac acc aag atg aaa atc tat ggc      674
   Leu Ser Arg Leu Ala Arg Ile Ala Asp Thr Lys Met Lys Ile Tyr Gly
                   50                  55                  60
   gaa gtt gtg ttt aag tgt gag aat gtg gcc aca ctg gac ccc atc aac      722
   Glu Val Val Phe Lys Cys Glu Asn Val Ala Thr Leu Asp Pro Ile Asn
               65                  70                  75
   ttt gag acc cca gag gct tac atc agc ttg ccc aag tgg aac act aaa 770
   Phe Glu Thr Pro Glu Ala Tyr Ile Ser Leu Pro Lys Trp Asn Thr Lys
   80 85 90
   cgt atg ggc tcc atc tcc ttt gac ttc cgc acc aca gag ccc aat ggc 818
   Arg Met Gly Ser Ile Ser Phe Asp Phe Arg Thr Thr Glu Pro Asn Gly
   95 100 105
   ctg atc ctc ttc act cat gga aag ccc caa gag agg aag gat gct cgg 866
   Leu Ile Leu Phe Thr His Gly Lys Pro Gln Glu Arg Lys Asp Ala Arg
   110 115 120 125
   agc cag aag aat aca aaa gta gac ttc ttt gcc gtg gaa ctc ctc gat 914
   Ser Gln Lys Asn Thr Lys Val Asp Phe Phe Ala Val Glu Leu Leu Asp
   130 135 140
   ggc aac ctg tac ttg ctg ctt gac atg ggc tct ggc acc atc aaa gtg 962
   Gly Asn Leu Tyr Leu Leu Leu Asp Met Gly Ser Gly Thr Ile Lys Val
   145 150 155
   aaa gcc act cag aag aaa gcc aat gat ggg gaa tgg tac cat gtg gac 1010
   Lys Ala Thr Gln Lys Lys Ala Asn Asp Gly Glu Trp Tyr His Val Asp
   160 165 170
   att cag cga gat ggc aga tca ggt act ata tca gtg aac agc agg cgc 1058
   Ile Gln Arg Asp Gly Arg Ser Gly Thr Ile Ser Val Asn Ser Arg Arg
   175 180 185
   acg cca ttc acc gcc agt ggg gag agc gag atc ctg gac ctg gaa gga 1106
   Thr Pro Phe Thr Ala Ser Gly Glu Ser Glu Ile Leu Asp Leu Glu Gly
   190 195 200 205
   gac atg tac ctg gga ggg ctg ccg gag aac cgt gct ggc ctt att ctc 1154
   Asp Met Tyr Leu Gly Gly Leu Pro Glu Asn Arg Ala Gly Leu Ile Leu
   210 215 220
   ccc acc gag ctg tgg act gcc atg ctc aac tat ggc tac gtg ggc tgc 1202
   Pro Thr Glu Leu Trp Thr Ala Met Leu Asn Tyr Gly Tyr Val Gly Cys
   225 230 235
   atc cgc gac cta ttc att gat ggg cgc agc aag aac att cga cag ctg 1250
   Ile Arg Asp Leu Phe Ile Asp Gly Arg Ser Lys Asn Ile Arg Gln Leu
   240 245 250
   gca gag atg cag aat gct gcg ggt gtc aag tcc tcc tgt tca cgg atg 1298
   Ala Glu Met Gln Asn Ala Ala Gly Val Lys Ser Ser Cys Ser Arg Met
   255 260 265
   agt gcc aag cag tgt gac agc tac ccc tgc aag aat aat gct gtg tgc 1346
   Ser Ala Lys Gln Cys Asp Ser Tyr Pro Cys Lys Asn Asn Ala Val Cys
   270 275 280 285
   aag gac ggc tgg aac cgc ttc atc tgc gac tgc acc ggc acc gga tac 1394
   Lys Asp Gly Trp Asn Arg Phe Ile Cys Asp Cys Thr Gly Thr Gly Tyr
   290 295 300
   tgg gga aga acc tgc gaa agg gag gca tcc atc ctg agc tat gat ggt 1442
   Trp Gly Arg Thr Cys Glu Arg Glu Ala Ser Ile Leu Ser Tyr Asp Gly
   305 310 315
   agc atg tac atg aag atc atc atg ccc atg gtc atg cat act gag gca 1490
   Ser Met Tyr Met Lys Ile Ile Met Pro Met Val Met His Thr Glu Ala
   320 325 330
   gag gat gtg tcc ttc cgc ttc atg tcc cag cga gct tat ggg ctg ctg 1538
   Glu Asp Val Ser Phe Arg Phe Met Ser Gln Arg Ala Tyr Gly Leu Leu
   335 340 345
   gtg gct acg acc tcc agg gac tct gcc gac acc ctg cgt ctg gag ctg 1586
   Val Ala Thr Thr Ser Arg Asp Ser Ala Asp Thr Leu Arg Leu Glu Leu
   350 355 360 365
   gat ggg ggg cgt gtc aag ctc atg gtt aac tta gac tgt atc agg ata 1634
   Asp Gly Gly Arg Val Lys Leu Met Val Asn Leu Asp Cys Ile Arg Ile
   370 375 380
   aac tgt aac tcc agc aaa gga cca gag acc ttg tat gca ggg cag aag 1682
   Asn Cys Asn Ser Ser Lys Gly Pro Glu Thr Leu Tyr Ala Gly Gln Lys
   385 390 395
   ctc aat gac aac gag tgg cac acc gtt cgg gtg gtg cgg aga gga aaa 1730
   Leu Asn Asp Asn Glu Trp His Thr Val Arg Val Val Arg Arg Gly Lys
   400 405 410
   agc ctt aag tta acc gtg gat gat gat gtg gct gag ggt aca atg gtg 1778
   Ser Leu Lys Leu Thr Val Asp Asp Asp Val Ala Glu Gly Thr Met Val
   415 420 425
   gga gac cat acc cgt ttg gag ttc cac aac att gaa acg gga atc atg 1826
   Gly Asp His Thr Arg Leu Glu Phe His Asn Ile Glu Thr Gly Ile Met
   430 435 440 445
   act gag aaa cgc tac atc tcc gtt gtc ccc tcc agc ttt att ggc cat 1874
   Thr Glu Lys Arg Tyr Ile Ser Val Val Pro Ser Ser Phe Ile Gly His
   450 455 460
   ctg cag agc ctc atg ttt aat ggc ctt ctc tac att gac ttg tgc aaa 1922
   Leu Gln Ser Leu Met Phe Asn Gly Leu Leu Tyr Ile Asp Leu Cys Lys
   465 470 475
   aat ggt gac att gat tat tgt gag ctg aag gct cgt ttt gga ctg agg 1970
   Asn Gly Asp Ile Asp Tyr Cys Glu Leu Lys Ala Arg Phe Gly Leu Arg
   480 485 490
   aac atc atc gct gac cct gtc acc ttt aag acc aag agc agc tac ctg 2018
   Asn Ile Ile Ala Asp Pro Val Thr Phe Lys Thr Lys Ser Ser Tyr Leu
   495 500 505
   agc ctt gcc act ctt cag gct tac acc tcc atg cac ctc ttc ttc cag 2066
   Ser Leu Ala Thr Leu Gln Ala Tyr Thr Ser Met His Leu Phe Phe Gln
   510 515 520 525
   ttc aag acc acc tca cca gat ggc ttc att ctc ttc aat agt ggt gat 2114
   Phe Lys Thr Thr Ser Pro Asp Gly Phe Ile Leu Phe Asn Ser Gly Asp
   530 535 540
   ggc aat gac ttc att gca gtc gag ctt gtc aag ggg tat ata cac tac 2162
   Gly Asn Asp Phe Ile Ala Val Glu Leu Val Lys Gly Tyr Ile His Tyr
   545 550 555
   gtt ttt gac ctc gga aac ggt ccc aat gtg atc aaa ggc aac agt gac 2210
   Val Phe Asp Leu Gly Asn Gly Pro Asn Val Ile Lys Gly Asn Ser Asp
   560 565 570
   cgc ccc ctg aat gac aac cag tgg cac aat gtc gtc atc act cgg gac 2258
   Arg Pro Leu Asn Asp Asn Gln Trp His Asn Val Val Ile Thr Arg Asp
   575 580 585
   aat agt aac act cat agc ctg aaa gtg gac acc aaa gtg gtc act cag 2306
   Asn Ser Asn Thr His Ser Leu Lys Val Asp Thr Lys Val Val Thr Gln
   590 595 600 605
   gtt atc aat ggt gcc aaa aat ctg gat ttg aaa ggt gat ctc tat atg 2354
   Val Ile Asn Gly Ala Lys Asn Leu Asp Leu Lys Gly Asp Leu Tyr Met
   610 615 620
   gct ggt ctg gcc caa ggc atg tac agc aac ctc cca aag ctc gtg gcc     2402
   Ala Gly Leu Ala Gln Gly Met Tyr Ser Asn Leu Pro Lys Leu Val Ala
               625                 630                 635
   tct cga gat ggc ttt cag ggc tgt cta gca tca gtg gac ttg aat gga     2450
   Ser Arg Asp Gly Phe Gln Gly Cys Leu Ala Ser Val Asp Leu Asn Gly
           640                 645                 650
   cgc ctg cca gac ctc atc aat gat gct ctt cat cgg agc gga cag atc     2498
   Arg Leu Pro Asp Leu Ile Asn Asp Ala Leu His Arg Ser Gly Gln Ile
       655                 660                 665
   gag cgt ggc tgt gaa gga ccc agt acc acc tgc cag gaa gat tca tgt     2546
   Glu Arg Gly Cys Glu Gly Pro Ser Thr Thr Cys Gln Glu Asp Ser Cys
   670                 675                 680                 685
   gcc aac cag ggg gtc tgc atg caa caa tgg gag ggc ttc acc tgt gat     2594
   Ala Asn Gln Gly Val Cys Met Gln Gln Trp Glu Gly Phe Thr Cys Asp
                   690                 695                 700
   tgt tct atg acc tct tat tct gga aac cag tgc aat gat cct ggc gct     2642
   Cys Ser Met Thr Ser Tyr Ser Gly Asn Gln Cys Asn Asp Pro Gly Ala
               705                 710                 715
   acg tac atc ttt ggg aaa agt ggt ggg ctt atc ctc tac acc tgg cca     2690
   Thr Tyr Ile Phe Gly Lys Ser Gly Gly Leu Ile Leu Tyr Thr Trp Pro
           720                 725                 730
   gcc aat gac agg ccc agc acg cgg tct gac cgc ctt gcc gtg ggc ttc     2738
   Ala Asn Asp Arg Pro Ser Thr Arg Ser Asp Arg Leu Ala Val Gly Phe
       735                 740                 745
   agc acc act gtg aag gat ggc atc ttg gtc cgc atc gac agt gct cca     2786
   Ser Thr Thr Val Lys Asp Gly Ile Leu Val Arg Ile Asp Ser Ala Pro
   750                 755                 760                 765
   gga ctt ggt gac ttc ctc cag ctt cac ata gaa cag ggg aaa att gga     2834
   Gly Leu Gly Asp Phe Leu Gln Leu His Ile Glu Gln Gly Lys Ile Gly
                   770                 775                 780
   gtt gtc ttc aac att ggc aca gtt gac atc tcc atc aaa gag gag aga     2882
   Val Val Phe Asn Ile Gly Thr Val Asp Ile Ser Ile Lys Glu Glu Arg
               785                 790                 795
   acc cct gta aat gac ggc aaa tac cat gtg gta cgc ttc acc agg aac     2930
   Thr Pro Val Asn Asp Gly Lys Tyr His Val Val Arg Phe Thr Arg Asn
           800                 805                 810
   ggc ggc aac gcc acc ctg cag gtg gac aac tgg cca gtg aat gaa cat     2978
   Gly Gly Asn Ala Thr Leu Gln Val Asp Asn Trp Pro Val Asn Glu His
       815                 820                 825
   tat cct aca ggc cgg cag tta acc atc ttc aac act cag gcg caa ata     3026
   Tyr Pro Thr Gly Arg Gln Leu Thr Ile Phe Asn Thr Gln Ala Gln Ile
   830                 835                 840                 845
   gcc att ggt gga aag gac aaa gga cgc ctc ttc caa ggc caa ctc tct     3074
   Ala Ile Gly Gly Lys Asp Lys Gly Arg Leu Phe Gln Gly Gln Leu Ser
                   850                 855                 860
   ggg ctc tat tat gat ggt ttg aaa gta ctg aac atg gcg gct gag aac     3122
   Gly Leu Tyr Tyr Asp Gly Leu Lys Val Leu Asn Met Ala Ala Glu Asn
               865                 870                 875
   aac ccc aat att aaa atc aat gga agt gtt cgg ctg gtt gga gaa gtc     3170
   Asn Pro Asn Ile Lys Ile Asn Gly Ser Val Arg Leu Val Gly Glu Val
           880                 885                 890
   cca tca att ttg gga aca aca cag acg acc tcc atg cca cca gaa atg 3218
   Pro Ser Ile Leu Gly Thr Thr Gln Thr Thr Ser Met Pro Pro Glu Met
   895 900 905
   tct act act gtc atg gaa acc act act aca atg gcg act acc aca acc 3266
   Ser Thr Thr Val Met Glu Thr Thr Thr Thr Met Ala Thr Thr Thr Thr
   910 915 920 925
   cgt aag aat cgc tct aca gcc agc att cag cca aca tca gat gat ctt 3314
   Arg Lys Asn Arg Ser Thr Ala Ser Ile Gln Pro Thr Ser Asp Asp Leu
   930 935 940
   gtt tca tct gct gaa tgt tca agt gat gat gaa gac ttt gtt gaa tgt 3362
   Val Ser Ser Ala Glu Cys Ser Ser Asp Asp Glu Asp Phe Val Glu Cys
   945 950 955
   gag ccg agt aca gca aac ccc acg gag ccg gga atc aga cgg gtt ccg 3410
   Glu Pro Ser Thr Ala Asn Pro Thr Glu Pro Gly Ile Arg Arg Val Pro
   960 965 970
   ggg gcc tca gag gtg atc cgg gag tcg agc agc aca aca ggg atg gtc 3458
   Gly Ala Ser Glu Val Ile Arg Glu Ser Ser Ser Thr Thr Gly Met Val
   975 980 985
   gtc ggc att gtg gct gct gcc gcc ctc tgc atc ttg atc ctc ctg tac 3506
   Val Gly Ile Val Ala Ala Ala Ala Leu Cys Ile Leu Ile Leu Leu Tyr
   990 995 1000 1005
   gcc atg tac aag tac agg aac agg gac gag ggg tcc tat caa gtg 3551
   Ala Met Tyr Lys Tyr Arg Asn Arg Asp Glu Gly Ser Tyr Gln Val
   1010 1015 1020
   gac gag acg cgg aac tac atc agc aac tcc gcc cag agc aac ggc 3596
   Asp Glu Thr Arg Asn Tyr Ile Ser Asn Ser Ala Gln Ser Asn Gly
   1025 1030 1035
   acg ctc atg aag gag aag cag cag agc tcg aag agc ggc cac aag 3641
   Thr Leu Met Lys Glu Lys Gln Gln Ser Ser Lys Ser Gly His Lys
   1040 1045 1050
   aaa cag aaa aac aag gac agg gag tat tac gtg taa acatgcgaac 3687
   Lys Gln Lys Asn Lys Asp Arg Glu Tyr Tyr Val
   1055 1060
   actgctcaca cgcgagtttt cacagttatt tctatccacg cctatgaatc tttggacggt 3747
   gagatctcac agatgtcaga actgctggaa ctatgaaatg gggtatataa ccacgactct 3807
   ggtggggaaa accgtttttt aaaggacaca cacacacaca gcgatgcatc tctctctaaa 3867
   gctcagccac ggctgcggca aggtcccagc ggtcgctggg agacagaagg ttttgtgccc 3927
   tgctgtatca taaagcacac acttagcgct ctggagccgg acggtggctc caccacttcc 3987
   gcaggcctgg aaacttcctt ctccggagga ccttttacta aaaggtagaa gacttcatgg 4047
   cttacttgtt ccataactcc aagtgagtct gtaatgtttg tgaagcttga ctgtaaccat 4107
   gttttttctg tttaattatg taaaaaacaa aactacaaca acaaaaaaag aaaaaagtta 4167
   aaaaagaaaa aaacaccaaa aaacaaaaac aaacaaaaaa aaaaacccac aacccttatc 4227
   tggttctgac cagtgtgcgt gtaactttat gatctgaggg gaaaaatggc ttttgggttt 4287
   ttgtttattt ttttgataat gactggacat cagaagagga aaaaaactca aaacaaaagc 4347
   gagagagact attgccatat gaactcaaaa gctatcatgg tgttcactct acatatcagg 4407
   ttatggtgtc tctagaatct gttgtttgtt tcctataaga tgctttgctg aacacatagc 4467
   aaaattcatg tgacggatga taaattgatt cgaaaagctg gtcccccagg atctaatttc 4527
   agaatttacc acccaaaccc ggaacagatg ggtttagggc tggtgttatc agagctattg 4587
   gctttacgta acaatattgt tcctgtccat tcacccagcc aaattgtgtt aaagaggaaa 4647
   gccccacaaa ctaaaacagc ctttcctagg gaagaggaag gggaggtggg ctggatctgt 4707
   gactgattga aatgcatgca aataaaaaag acaatattaa agtctgttat caaaccagac 4767
   agtaggggag ttcaactcgt gatggaacca caaaaggtca cacaagccaa ccatgtcatg 4827
   ccagagtaca aaacacatag ttctttcccc gccccgaatg tgacaatggt tttcatagtg 4887
   gtttaatttt gtagcctgac atttatggat aactctgtcc ttccatttgc tcacttctct 4947
   cttcacccat cttttttaaa aacaaataaa tgaataaagc tgctgtgaca cacacacaaa 5007
   aggaatttaa tagtataata tatatataaa taaatatata tacagatata tttatcatgg 5067
   tatgtttgat gggatgactg acacaggaaa tctgttaaag tcttaaaatg gaatgagaat 5127
   gttgttttaa aagaaaatag caaaacaaca aaaaagcaaa ccttaaaatg tgaagaaagt 5187
   gtgaatttta gttttgtcac agttaactgt gtcaaagaga attaaaaaaa aaaacttcag 5247
   attttgttta catattttac tacatttttg ctggtataat tccttagcca cctatgtaca 5307
   tactgcttta agaaatgttt ttttcctgtt tatttctgtt tggtttatat tctggttgtc 5367
   tttttctttt tgtaaagagg aaacaatgta cagaaaaaca ataaactggt tgtatggcca 5427
   tagctatccg aaaagcaaga gacaaagcaa gacaaatatt cacacaaaaa tgaagtgtgt 5487
   cctctggagg gtcagatata caatttcttt tgtacagatg aaaatcaatc agctgcttag 5547
   atttagaaat ctactcttgc tggtctttgt aagttgcatg aatatttgac tttgaaaaaa 5607
   tatcttaacg acatggggca aaaagtgcaa tctaaatggt agcctttact aatgtgtgtg 5667
   gaaagaggtg ttcctcatta tctaatattt caatgtgtta agagtttaat ttttttgtta 5727
   tcattaaaaa agacaggatt ataaagagat atcaaagcac gattttagat aacctaaacg 5787
   gcccagccta tacgaagttg attatatctc gatgtctgta aaagattgct gttcttggag 5847
   tcttgaggtc ttgtgaattg atttcctgct ttctttcatt tttttcaatt taagtaataa 5907
   tacatttgtt atattccttt cagtgtaagt ttctatttgg acaattttat gggaacatgt 5967
   gcattctcta tgtgagcttc tatcatattc ctgttttatt agcagaacct aaaggaattt 6027
   atttaatgat gttgtgacat tactgctttt tcttttttct tttcttagtt catatttgca 6087
   ttttcgttca aggatatgct tagcaataaa atgttcttcc caaaaaaaaa aaaaaaaaaa 6147
   aaaaaaaaaa aaaaaa 6163

<210> 55
<211> 1061
<212> PRT
<213> human

<400> 55

Met Leu Gly Ser Asp Asp Phe Phe Tyr Val Gly Gly Ser Pro Ser Thr
1               5                   10                  15

Ala Asp Leu Pro Gly Ser Pro Val Ser Asn Asn Phe Met Gly Cys Leu
            20                  25                  30

Lys Glu Val Val Tyr Lys Asn Asn Asp Ile Arg Leu Glu Leu Ser Arg
        35                  40                  45

Leu Ala Arg Ile Ala Asp Thr Lys Met Lys Ile Tyr Gly Glu Val Val
    50                  55                  60

Phe Lys Cys Glu Asn Val Ala Thr Leu Asp Pro Ile Asn Phe Glu Thr
65                  70                  75                  80

Pro Glu Ala Tyr Ile Ser Leu Pro Lys Trp Asn Thr Lys Arg Met Gly
                85                  90                  95

Ser Ile Ser Phe Asp Phe Arg Thr Thr Glu Pro Asn Gly Leu Ile Leu
            100                 105                 110

Phe Thr His Gly Lys Pro Gln Glu Arg Lys Asp Ala Arg Ser Gln Lys
        115                 120                 125

Asn Thr Lys Val Asp Phe Phe Ala Val Glu Leu Leu Asp Gly Asn Leu
    130                 135                 140

Tyr Leu Leu Leu Asp Met Gly Ser Gly Thr Ile Lys Val Lys Ala Thr
145                 150                 155                 160

Gln Lys Lys Ala Asn Asp Gly Glu Trp Tyr His Val Asp Ile Gln Arg
                165                 170                 175

Asp Gly Arg Ser Gly Thr Ile Ser Val Asn Ser Arg Arg Thr Pro Phe
            180                 185                 190

Thr Ala Ser Gly Glu Ser Glu Ile Leu Asp Leu Glu Gly Asp Met Tyr
        195                 200                 205

Leu Gly Gly Leu Pro Glu Asn Arg Ala Gly Leu Ile Leu Pro Thr Glu
    210                 215                 220

Leu Trp Thr Ala Met Leu Asn Tyr Gly Tyr Val Gly Cys Ile Arg Asp
225                 230                 235                 240

Leu Phe Ile Asp Gly Arg Ser Lys Asn Ile Arg Gln Leu Ala Glu Met
                245                 250                 255

Gln Asn Ala Ala Gly Val Lys Ser Ser Cys Ser Arg Met Ser Ala Lys
            260                 265                 270

Gln Cys Asp Ser Tyr Pro Cys Lys Asn Asn Ala Val Cys Lys Asp Gly
        275                 280                 285

Trp Asn Arg Phe Ile Cys Asp Cys Thr Gly Thr Gly Tyr Trp Gly Arg
    290                 295                 300

Thr Cys Glu Arg Glu Ala Ser Ile Leu Ser Tyr Asp Gly Ser Met Tyr
305                 310                 315                 320

Met Lys Ile Ile Met Pro Met Val Met His Thr Glu Ala Glu Asp Val
                325                 330                 335

Ser Phe Arg Phe Met Ser Gln Arg Ala Tyr Gly Leu Leu Val Ala Thr
            340                 345                 350

Thr Ser Arg Asp Ser Ala Asp Thr Leu Arg Leu Glu Leu Asp Gly Gly
        355                 360                 365

Arg Val Lys Leu Met Val Asn Leu Asp Cys Ile Arg Ile Asn Cys Asn
    370                 375                 380

Ser Ser Lys Gly Pro Glu Thr Leu Tyr Ala Gly Gln Lys Leu Asn Asp
385                 390                 395                 400

Asn Glu Trp His Thr Val Arg Val Val Arg Arg Gly Lys Ser Leu Lys
                405                 410                 415

Leu Thr Val Asp Asp Asp Val Ala Glu Gly Thr Met Val Gly Asp His
            420                 425                 430

Thr Arg Leu Glu Phe His Asn Ile Glu Thr Gly Ile Met Thr Glu Lys
        435                 440                 445

Arg Tyr Ile Ser Val Val Pro Ser Ser Phe Ile Gly His Leu Gln Ser
    450                 455                 460

Leu Met Phe Asn Gly Leu Leu Tyr Ile Asp Leu Cys Lys Asn Gly Asp
465                 470                 475                 480

Ile Asp Tyr Cys Glu Leu Lys Ala Arg Phe Gly Leu Arg Asn Ile Ile
                485                 490                 495

Ala Asp Pro Val Thr Phe Lys Thr Lys Ser Ser Tyr Leu Ser Leu Ala
            500                 505                 510

Thr Leu Gln Ala Tyr Thr Ser Met His Leu Phe Phe Gln Phe Lys Thr
        515                 520                 525

Thr Ser Pro Asp Gly Phe Ile Leu Phe Asn Ser Gly Asp Gly Asn Asp
    530                 535                 540

Phe Ile Ala Val Glu Leu Val Lys Gly Tyr Ile His Tyr Val Phe Asp
545                 550                 555                 560

Leu Gly Asn Gly Pro Asn Val Ile Lys Gly Asn Ser Asp Arg Pro Leu
                565                 570                 575

Asn Asp Asn Gln Trp His Asn Val Val Ile Thr Arg Asp Asn Ser Asn
            580                 585                 590

Thr His Ser Leu Lys Val Asp Thr Lys Val Val Thr Gln Val Ile Asn
        595                 600                 605

Gly Ala Lys Asn Leu Asp Leu Lys Gly Asp Leu Tyr Met Ala Gly Leu
    610                 615                 620

Ala Gln Gly Met Tyr Ser Asn Leu Pro Lys Leu Val Ala Ser Arg Asp
625                 630                 635                 640

Gly Phe Gln Gly Cys Leu Ala Ser Val Asp Leu Asn Gly Arg Leu Pro
                645                 650                 655

Asp Leu Ile Asn Asp Ala Leu His Arg Ser Gly Gln Ile Glu Arg Gly
            660                 665                 670

Cys Glu Gly Pro Ser Thr Thr Cys Gln Glu Asp Ser Cys Ala Asn Gln
        675                 680                 685

Gly Val Cys Met Gln Gln Trp Glu Gly Phe Thr Cys Asp Cys Ser Met
    690                 695                 700

Thr Ser Tyr Ser Gly Asn Gln Cys Asn Asp Pro Gly Ala Thr Tyr Ile
705                 710                 715                 720

Phe Gly Lys Ser Gly Gly Leu Ile Leu Tyr Thr Trp Pro Ala Asn Asp
                725                 730                 735

Arg Pro Ser Thr Arg Ser Asp Arg Leu Ala Val Gly Phe Ser Thr Thr
            740                 745                 750

Val Lys Asp Gly Ile Leu Val Arg Ile Asp Ser Ala Pro Gly Leu Gly
        755                 760                 765

Asp Phe Leu Gln Leu His Ile Glu Gln Gly Lys Ile Gly Val Val Phe
    770                 775                 780

Asn Ile Gly Thr Val Asp Ile Ser Ile Lys Glu Glu Arg Thr Pro Val
785                 790                 795                 800

Asn Asp Gly Lys Tyr His Val Val Arg Phe Thr Arg Asn Gly Gly Asn
                805                 810                 815

Ala Thr Leu Gln Val Asp Asn Trp Pro Val Asn Glu His Tyr Pro Thr
            820                 825                 830

Gly Arg Gln Leu Thr Ile Phe Asn Thr Gln Ala Gln Ile Ala Ile Gly
        835                 840                 845

Gly Lys Asp Lys Gly Arg Leu Phe Gln Gly Gln Leu Ser Gly Leu Tyr
    850                 855                 860

Tyr Asp Gly Leu Lys Val Leu Asn Met Ala Ala Glu Asn Asn Pro Asn
865                 870                 875                 880

Ile Lys Ile Asn Gly Ser Val Arg Leu Val Gly Glu Val Pro Ser Ile
                885                 890                 895

Leu Gly Thr Thr Gln Thr Thr Ser Met Pro Pro Glu Met Ser Thr Thr
            900                 905                 910

Val Met Glu Thr Thr Thr Thr Met Ala Thr Thr Thr Thr Arg Lys Asn
        915                 920                 925

Arg Ser Thr Ala Ser Ile Gln Pro Thr Ser Asp Asp Leu Val Ser Ser
    930                 935                 940

Ala Glu Cys Ser Ser Asp Asp Glu Asp Phe Val Glu Cys Glu Pro Ser
945                 950                 955                 960

Thr Ala Asn Pro Thr Glu Pro Gly Ile Arg Arg Val Pro Gly Ala Ser
                965                 970                 975

Glu Val Ile Arg Glu Ser Ser Ser Thr Thr Gly Met Val Val Gly Ile
            980                 985                 990

Val Ala Ala Ala Ala Leu Cys Ile  Leu Ile Leu Leu Tyr  Ala Met Tyr
        995                 1000                 1005

Lys Tyr  Arg Asn Arg Asp Glu  Gly Ser Tyr Gln Val  Asp Glu Thr
    1010                 1015                 1020

Arg Asn  Tyr Ile Ser Asn Ser  Ala Gln Ser Asn Gly  Thr Leu Met
    1025                 1030                 1035

Lys Glu  Lys Gln Gln Ser Ser  Lys Ser Gly His Lys  Lys Gln Lys
    1040                 1045                 1050

Asn Lys  Asp Arg Glu Tyr Tyr  Val
    1055                 1060

<210>  56
<211>  4766
<212>  DNA
<213>  human

<220>
<221>  CDS
<222>  (982)..(2280)

<400>  56
gatttgctct cgggagtgcg ctatttgcat atgacttgcc catttgtgaa tttggctccc      60

caactcctcg cttcacttca cctgtgcctc cctggtccgc gcacttcgca ggcgcccgcc     120

cgcctctcgg ccacctctgc agcctgccag gcacctcctc ttgcgctctc gctgatttcg     180

cccacccacc tccctccacc ccgtgccacg ttggtttggg tggctgctcc gcaccgggtt     240

cctcctcctc tcctctttcc ctccagtcct tcttattctc ttcctctggc tgcggtggct     300

gctgctgctg ctggttttca tccagttggg aaacccagga agccggcggc tgctccgtgg     360

cgctagtcca gcacgcccag ggttaaaagc ctcgcgccct tccgcggaga ctcccaggaa     420

cacccgaaga actcttccac ctgcagcccc cttttgcctg gcagttctgc attgcatctc     480

atggaagtgg aaacaggaaa ataaaaatgt tcaaactcct tggatgttgg gataaactca     540

cctgaaccca cttgggttcg ggctgcctcc ttctcttcct tcattgccac ctttcctctg     600

tgtggctccc gggagtgtgc ggttaagtca tcagactcga agtgcctaga gatccggagg     660

aagccgcgcc ggtcttcccc tgacatgcgt ggcatgccgg ggctccgtag gaggtttgct     720

atacctggga ggaccctggc attctaaatt tcagctccgg gaaagagaag ggcttttttg     780

ccttttatct tttttttttc tttctttaag tagtaatttt ttaactgatt cattgtttgg     840

aaagcgcata ttgcttccct cttccccgaa ttctggcaac tcttcctcct gctatgatgg     900

gcccttgggc atcatgaact tcattactcc tcactggctg gaattcaaac tgcccatctg     960

tagtggtccc gtgcgttgac c atg cac ctg aga atc cac gcg aga cgg agc      1011
                        Met His Leu Arg Ile His Ala Arg Arg Ser
                        1               5                   10

cct cct cgc cgg ccg gcc tgg acg ctt ggg atc tgg ttc ctg ttc tgg      1059
Pro Pro Arg Arg Pro Ala Trp Thr Leu Gly Ile Trp Phe Leu Phe Trp
                15                  20                  25

gga tgt atc gtc agc tct gta tgg agt tct tct aat gta gct tcc tcc      1107
Gly Cys Ile Val Ser Ser Val Trp Ser Ser Ser Asn Val Ala Ser Ser
            30                  35                  40

tcc tcc acc tct tcc tcg ccg ggg tct cac tct cag cac gag cac cat      1155
Ser Ser Thr Ser Ser Ser Pro Gly Ser His Ser Gln His Glu His His
        45                  50                  55

ttc cat ggc agc aag cat cac tca gtg cct att tct atc tat cgt tcc      1203
Phe His Gly Ser Lys His His Ser Val Pro Ile Ser Ile Tyr Arg Ser
    60                  65                  70

cct gtt tcc ctt cga gga gga cac gct ggc gct acg tac atc ttt ggg      1251
Pro Val Ser Leu Arg Gly Gly His Ala Gly Ala Thr Tyr Ile Phe Gly
75                  80                  85                  90

aaa agt ggt ggg ctt atc ctc tac acc tgg cca gcc aat gac agg ccc      1299
Lys Ser Gly Gly Leu Ile Leu Tyr Thr Trp Pro Ala Asn Asp Arg Pro
                95                  100                 105

agc acg cgg tct gac cgc ctt gcc gtg ggc ttc agc acc act gtg aag      1347

Ser Thr Arg Ser Asp Arg Leu Ala Val Gly Phe Ser Thr Thr Val Lys
            110                 115                 120

gat ggc atc ttg gtc cgc atc gac agt gct cca gga ctt ggt gac ttc 1395
Asp Gly Ile Leu Val Arg Ile Asp Ser Ala Pro Gly Leu Gly Asp Phe
        125                 130                 135

ctc cag ctt cac ata gaa cag ggg aaa att gga gtt gtc ttc aac att 1443
Leu Gln Leu His Ile Glu Gln Gly Lys Ile Gly Val Val Phe Asn Ile
    140                 145                 150

ggc aca gtt gac atc tcc atc aaa gag gag aga acc cct gta aat gac 1491
Gly Thr Val Asp Ile Ser Ile Lys Glu Glu Arg Thr Pro Val Asn Asp
155                 160                 165                 170

ggc aaa tac cat gtg gta cgc ttc acc agg aac ggc ggc aac gcc acc 1539
Gly Lys Tyr His Val Val Arg Phe Thr Arg Asn Gly Gly Asn Ala Thr
                175                 180                 185

ctg cag gtg gac aac tgg cca gtg aat gaa cat tat cct aca ggc cgg 1587
Leu Gln Val Asp Asn Trp Pro Val Asn Glu His Tyr Pro Thr Gly Arg
            190                 195                 200

cag tta acc atc ttc aac act cag gcg caa ata gcc att ggt gga aag 1635
Gln Leu Thr Ile Phe Asn Thr Gln Ala Gln Ile Ala Ile Gly Gly Lys
        205                 210                 215

gac aaa gga cgc ctc ttc caa ggc caa ctc tct ggg ctc tat tat gat 1683
Asp Lys Gly Arg Leu Phe Gln Gly Gln Leu Ser Gly Leu Tyr Tyr Asp
    220                 225                 230

ggt ttg aaa gta ctg aac atg gcg gct gag aac aac ccc aat att aaa 1731
Gly Leu Lys Val Leu Asn Met Ala Ala Glu Asn Asn Pro Asn Ile Lys
235                 240                 245                 250

atc aat gga agt gtt cgg ctg gtt gga gaa gtc cca tca att ttg gga 1779
Ile Asn Gly Ser Val Arg Leu Val Gly Glu Val Pro Ser Ile Leu Gly
                255                 260                 265

aca aca cag acg acc tcc atg cca cca gaa atg tct act act gtc atg 1827
Thr Thr Gln Thr Thr Ser Met Pro Pro Glu Met Ser Thr Thr Val Met
            270                 275                 280

gaa acc act act aca atg gcg act acc aca acc cgt aag aat cgc tct 1875
Glu Thr Thr Thr Thr Met Ala Thr Thr Thr Thr Arg Lys Asn Arg Ser
        285                 290                 295

aca gcc agc att cag cca aca tca gat gat ctt gtt tca tct gct gaa 1923
Thr Ala Ser Ile Gln Pro Thr Ser Asp Asp Leu Val Ser Ser Ala Glu
    300                 305                 310

tgt tca agt gat gat gaa gac ttt gtt gaa tgt gag ccg agt aca ggt 1971
Cys Ser Ser Asp Asp Glu Asp Phe Val Glu Cys Glu Pro Ser Thr Gly
315                 320                 325                 330

agg tca gca aac ccc acg gag ccg gga atc aga cgg gtt ccg ggg gcc 2019
Arg Ser Ala Asn Pro Thr Glu Pro Gly Ile Arg Arg Val Pro Gly Ala
                335                 340                 345

tca gag gtg atc cgg gag tcg agc agc aca aca ggg atg gtc gtc ggc 2067
Ser Glu Val Ile Arg Glu Ser Ser Ser Thr Thr Gly Met Val Val Gly
            350                 355                 360

att gtg gct gct gcc gcc ctc tgc atc ttg atc ctc ctg tac gcc atg 2115
Ile Val Ala Ala Ala Ala Leu Cys Ile Leu Ile Leu Leu Tyr Ala Met
        365                 370                 375

tac aag tac agg aac agg gac gag ggg tcc tat caa gtg gac gag acg 2163

Tyr Lys Tyr Arg Asn Arg Asp Glu Gly Ser Tyr Gln Val Asp Glu Thr
    380                 385                 390

cgg aac tac atc agc aac tcc gcc cag agc aac ggc acg ctc atg aag     2211
Arg Asn Tyr Ile Ser Asn Ser Ala Gln Ser Asn Gly Thr Leu Met Lys
395                 400                 405                 410

gag aag cag cag agc tcg aag agc ggc cac aag aaa cag aaa aac aag     2259
Glu Lys Gln Gln Ser Ser Lys Ser Gly His Lys Lys Gln Lys Asn Lys
                415                 420                 425

gac agg gag tat tac gtg taa acatgcgaac actgctcaca cgcgagtttt        2310
Asp Arg Glu Tyr Tyr Val
            430

cacagttatt tctatccacg cctatgaatc tttggacggt gagatctcac agatgtcaga   2370

actgctggaa ctatgaaatg gggtatataa ccacgactct ggtggggaaa accgtttttt   2430

aaaggacaca cacacacaca gcgatgcatc tctctctaaa gctcagccac ggctgcggca   2490

aggtcccagc ggtcgctggg agacagaagg ttttgtgccc tgctgtatca taaagcacac   2550

acttagcgct ctggagccgg acggtggctc caccacttcc gcaggcctgg aaacttcctt   2610

ctccggagga ccttttacta aaaggtagaa gacttcatgg cttacttgtt ccataactcc   2670

aagtgagtct gtaatgtttg tgaagcttga ctgtaaccat gttttttctg tttaattatg    2730

taaaaaacaa aactacaaca acaaaaaaag aaaaaagtta aaaaagaaaa aaacaccaaa   2790

aaacaaaaac aaacaaaaaa aaaaacccac aacccttatc tggttctgac cagtgtgcgt   2850

gtaactttat gatctgaggg gaaaaatggc ttttgggttt ttgtttattt ttttgataat   2910

gactggacat cagaagagga aaaaaactca aaacaaaagc gagagagact attgccatat   2970

gaactcaaaa gctatcatgg tgttcactct acatatcagg ttatggtgtc tctagaatct   3030

gttgtttgtt tcctataaga tgctttgctg aacacatagc aaaattcatg tgacggatga   3090

taaattgatt cgaaaagctg gtcccccagg atctaatttc agaatttacc acccaaaccc   3150

ggaacagatg ggtttagggc tggtgttatc agagctattg gctttacgta acaatattgt   3210

tcctgtccat tcacccagcc aaattgtgtt aaagaggaaa gccccacaaa ctaaaacagc   3270

ctttcctagg gaagaggaag gggaggtggg ctggatctgt gactgattga aatgcatgca   3330

aataaaaaag acaatattaa agtctgttat caaaccagac agtaggggag ttcaactcgt   3390

gatggaacca caaaaggtca cacaagccaa ccatgtcatg ccagagtaca aaacacatag   3450

ttctttcccc gccccgaatg tgacaatggt tttcatagtg gtttaatttt gtagcctgac   3510

atttatggat aactctgtcc ttccatttgc tcacttctct cttcacccat cttttttaaa    3570

aacaaataaa tgaataaagc tgctgtgaca cacacacaaa aggaatttaa tagtataata   3630

tatatataaa taaatatata tacagatata tttatcatgg tatgtttgat gggatgactg   3690

acacaggaaa tctgttaaag tcttaaaatg gaatgagaat gttgttttaa aagaaaatag   3750

caaaacaaca aaaaagcaaa ccttaaaatg tgaagaaagt gtgaatttta gttttgtcac   3810

agttaactgt gtcaaagaga attaaaaaaa aaaacttcag attttgttta catattttac   3870

tacatttttg ctggtataat tccttagcca cctatgtaca tactgcttta agaaatgttt   3930

ttttcctgtt tatttctgtt tggtttatat tctggttgtc tttttctttt tgtaaagagg 3990
aaacaatgta cagaaaaaca ataaactggt tgtatggcca tagctatccg aaaagcaaga 4050
gacaaagcaa gacaaatatt cacacaaaaa tgaagtgtgt cctctggagg gtcagatata 4110
caatttcttt tgtacagatg aaaatcaatc agctgcttag atttagaaat ctactcttgc 4170
tggtctttgt aagttgcatg aatatttgac tttgaaaaaa tatcttaacg acatggggca 4230
aaaagtgcaa tctaaatggt agcctttact aatgtgtgtg gaaagaggtg ttcctcatta 4290
tctaatattt caatgtgtta agagtttaat ttttttgtta tcattaaaaa agacaggatt 4350
ataaagagat atcaaagcac gattttagat aacctaaacg gcccagccta tacgaagttg 4410
attatatctc gatgtctgta aaagattgct gttcttggag tcttgaggtc ttgtgaattg 4470
atttcctgct ttctttcatt tttttcaatt taagtaataa tacatttgtt atattccttt 4530
cagtgtaagt ttctatttgg acaattttat gggaacatgt gcattctcta tgtgagcttc 4590
tatcatattc ctgttttatt agcagaacct aaaggaattt atttaatgat gttgtgacat 4650
tactgctttt tcttttttct tttcttagtt catatttgca ttttcgttca aggatatgct 4710
tagcaataaa atgttcttcc caaaaaaaaa aaaaaaaaaa aaaaaaaaaa aaaaaa 4766

<210> 57
<211> 432
<212> PRT
<213> human

<400> 57

Met His Leu Arg Ile His Ala Arg Arg Ser Pro Pro Arg Arg Pro Ala
1               5                   10                  15

Trp Thr Leu Gly Ile Trp Phe Leu Phe Trp Gly Cys Ile Val Ser Ser
            20                  25                  30

Val Trp Ser Ser Ser Asn Val Ala Ser Ser Ser Ser Thr Ser Ser Ser
        35                  40                  45

Pro Gly Ser His Ser Gln His Glu His His Phe His Gly Ser Lys His
    50                  55                  60

His Ser Val Pro Ile Ser Ile Tyr Arg Ser Pro Val Ser Leu Arg Gly
65                  70                  75                  80

Gly His Ala Gly Ala Thr Tyr Ile Phe Gly Lys Ser Gly Gly Leu Ile
                85                  90                  95

Leu Tyr Thr Trp Pro Ala Asn Asp Arg Pro Ser Thr Arg Ser Asp Arg
            100                 105                 110

Leu Ala Val Gly Phe Ser Thr Thr Val Lys Asp Gly Ile Leu Val Arg
        115                 120                 125

Ile Asp Ser Ala Pro Gly Leu Gly Asp Phe Leu Gln Leu His Ile Glu
    130                 135                 140

Gln Gly Lys Ile Gly Val Val Phe Asn Ile Gly Thr Val Asp Ile Ser
145                 150                 155                 160

Ile Lys Glu Glu Arg Thr Pro Val Asn Asp Gly Lys Tyr His Val Val
                165                 170                 175

Arg Phe Thr Arg Asn Gly Gly Asn Ala Thr Leu Gln Val Asp Asn Trp
            180                 185                 190

Pro Val Asn Glu His Tyr Pro Thr Gly Arg Gln Leu Thr Ile Phe Asn
        195                 200                 205

Thr Gln Ala Gln Ile Ala Ile Gly Gly Lys Asp Lys Gly Arg Leu Phe
    210                 215                 220

Gln Gly Gln Leu Ser Gly Leu Tyr Tyr Asp Gly Leu Lys Val Leu Asn
225                 230                 235                 240

Met Ala Ala Glu Asn Asn Pro Asn Ile Lys Ile Asn Gly Ser Val Arg
                245                 250                 255

Leu Val Gly Glu Val Pro Ser Ile Leu Gly Thr Thr Gln Thr Thr Ser
            260                 265                 270

Met Pro Pro Glu Met Ser Thr Thr Val Met Glu Thr Thr Thr Thr Met
        275                 280                 285

Ala Thr Thr Thr Thr Arg Lys Asn Arg Ser Thr Ala Ser Ile Gln Pro
    290                 295                 300

Thr Ser Asp Asp Leu Val Ser Ser Ala Glu Cys Ser Ser Asp Asp Glu
305                 310                 315                 320

Asp Phe Val Glu Cys Glu Pro Ser Thr Gly Arg Ser Ala Asn Pro Thr
                325                 330                 335

Glu Pro Gly Ile Arg Arg Val Pro Gly Ala Ser Glu Val Ile Arg Glu
            340                 345                 350

Ser Ser Ser Thr Thr Gly Met Val Val Gly Ile Val Ala Ala Ala Ala
        355                 360                 365

Leu Cys Ile Leu Ile Leu Leu Tyr Ala Met Tyr Lys Tyr Arg Asn Arg
    370                 375                 380

Asp Glu Gly Ser Tyr Gln Val Asp Glu Thr Arg Asn Tyr Ile Ser Asn
385                 390                 395                 400

Ser Ala Gln Ser Asn Gly Thr Leu Met Lys Glu Lys Gln Gln Ser Ser
                405                 410                 415

Lys Ser Gly His Lys Lys Gln Lys Asn Lys Asp Arg Glu Tyr Tyr Val
            420                 425                 430

<210> 58
<211> 4847
<212> DNA
<213> human

<220>
<221> CDS
<222> (982)..(2361)

<400> 58
gatttgctct cgggagtgcg ctatttgcat atgacttgcc catttgtgaa tttggctccc 60

caactcctcg cttcacttca cctgtgcctc cctggtccgc gcacttcgca ggcgcccgcc 120

cgcctctcgg ccacctctgc agcctgccag gcacctcctc ttgcgctctc gctgatttcg 180

cccacccacc tccctccacc ccgtgccacg ttggtttggg tggctgctcc gcaccgggtt 240

cctcctcctc tcctctttcc ctccagtcct tcttattctc ttcctctggc tgcggtggct 300

gctgctgctg ctggttttca tccagttggg aaacccagga agccggcggc tgctccgtgg 360

cgctagtcca gcacgcccag ggttaaaagc ctcgcgccct tccgcggaga ctcccaggaa 420

cacccgaaga actcttccac ctgcagcccc cttttgcctg gcagttctgc attgcatctc 480

atggaagtgg aaacaggaaa ataaaaatgt tcaaactcct tggatgttgg gataaactca 540

cctgaaccca cttgggttcg ggctgcctcc ttctcttcct tcattgccac ctttcctctg 600

tgtggctccc gggagtgtgc ggttaagtca tcagactcga agtgcctaga gatccggagg 660

aagccgcgcc ggtcttcccc tgacatgcgt ggcatgccgg ggctccgtag gaggtttgct 720

atacctggga ggaccctggc attctaaatt tcagctccgg gaaagagaag ggcttttttg 780

ccttttatct tttttttttc tttctttaag tagtaatttt ttaactgatt cattgtttgg 840

aaagcgcata ttgcttccct cttccccgaa ttctggcaac tcttcctcct gctatgatgg 900

gcccttgggc atcatgaact tcattactcc tcactggctg gaattcaaac tgcccatctg 960

tagtggtccc gtgcgttgac c atg cac ctg aga atc cac gcg aga cgg agc 1011
Met His Leu Arg Ile His Ala Arg Arg Ser
1               5                   10

cct cct cgc cgg ccg gcc tgg acg ctt ggg atc tgg ttc ctg ttc tgg 1059
Pro Pro Arg Arg Pro Ala Trp Thr Leu Gly Ile Trp Phe Leu Phe Trp
                15                  20                  25

gga tgt atc gtc agc tct gta tgg agt tct tct aat gta gct tcc tcc 1107
Gly Cys Ile Val Ser Ser Val Trp Ser Ser Ser Asn Val Ala Ser Ser
            30                  35                  40

tcc tcc acc tct tcc tcg ccg ggg tct cac tct cag cac gag cac cat 1155
Ser Ser Thr Ser Ser Ser Pro Gly Ser His Ser Gln His Glu His His
        45                  50                  55

ttc cat ggc agc aag cat cac tca gtg cct att tct atc tat cgt tcc 1203
Phe His Gly Ser Lys His His Ser Val Pro Ile Ser Ile Tyr Arg Ser
    60                  65                  70

cct gtt tcc ctt cga gga gga cac gct ggc gct acg tac atc ttt ggg 1251
Pro Val Ser Leu Arg Gly Gly His Ala Gly Ala Thr Tyr Ile Phe Gly
75                  80                  85                  90

aaa agt ggt ggg ctt atc ctc tac acc tgg cca gcc aat gac agg ccc 1299
Lys Ser Gly Gly Leu Ile Leu Tyr Thr Trp Pro Ala Asn Asp Arg Pro
                95                  100                 105

agc acg cgg tct gac cgc ctt gcc gtg ggc ttc agc acc act gtg aag 1347
Ser Thr Arg Ser Asp Arg Leu Ala Val Gly Phe Ser Thr Thr Val Lys
            110                 115                 120

gat ggc atc ttg gtc cgc atc gac agt gct cca gga ctt ggt gac ttc 1395
Asp Gly Ile Leu Val Arg Ile Asp Ser Ala Pro Gly Leu Gly Asp Phe
        125                 130                 135

ctc cag ctt cac ata gaa cag ggg aaa att gga gtt gtc ttc aac att 1443
Leu Gln Leu His Ile Glu Gln Gly Lys Ile Gly Val Val Phe Asn Ile
    140                 145                 150

ggc aca gtt gac atc tcc atc aaa gag gag aga acc cct gta aat gac 1491
Gly Thr Val Asp Ile Ser Ile Lys Glu Glu Arg Thr Pro Val Asn Asp
155                 160                 165                 170

ggc aaa tac cat gtg gta cgc ttc acc agg aac ggc ggc aac gcc acc 1539
Gly Lys Tyr His Val Val Arg Phe Thr Arg Asn Gly Gly Asn Ala Thr
                175                 180                 185

ctg cag gtg gac aac tgg cca gtg aat gaa cat tat cct aca ggc aac 1587
Leu Gln Val Asp Asn Trp Pro Val Asn Glu His Tyr Pro Thr Gly Asn
            190                 195                 200

act gat aat gaa cgc ttc caa atg gta aaa cag aaa atc ccc ttc aaa 1635
Thr Asp Asn Glu Arg Phe Gln Met Val Lys Gln Lys Ile Pro Phe Lys
        205                 210                 215

tat aat cgg cct gta gag gag tgg ctg cag gaa aaa ggc cgg cag tta 1683
Tyr Asn Arg Pro Val Glu Glu Trp Leu Gln Glu Lys Gly Arg Gln Leu
    220                 225                 230

acc atc ttc aac act cag gcg caa ata gcc att ggt gga aag gac aaa 1731
Thr Ile Phe Asn Thr Gln Ala Gln Ile Ala Ile Gly Gly Lys Asp Lys
235                 240                 245                 250

gga cgc ctc ttc caa ggc caa ctc tct ggg ctc tat tat gat ggt ttg 1779
Gly Arg Leu Phe Gln Gly Gln Leu Ser Gly Leu Tyr Tyr Asp Gly Leu
                255                 260                 265

aaa gta ctg aac atg gcg gct gag aac aac ccc aat att aaa atc aat 1827
Lys Val Leu Asn Met Ala Ala Glu Asn Asn Pro Asn Ile Lys Ile Asn
            270                 275                 280

gga agt gtt cgg ctg gtt gga gaa gtc cca tca att ttg gga aca aca 1875
Gly Ser Val Arg Leu Val Gly Glu Val Pro Ser Ile Leu Gly Thr Thr
        285                 290                 295

cag acg acc tcc atg cca cca gaa atg tct act act gtc atg gaa acc 1923
Gln Thr Thr Ser Met Pro Pro Glu Met Ser Thr Thr Val Met Glu Thr
    300                 305                 310

act act aca atg gcg act acc aca acc cgt aag aat cgc tct aca gcc 1971
Thr Thr Thr Met Ala Thr Thr Thr Thr Arg Lys Asn Arg Ser Thr Ala
315                 320                 325                 330

agc att cag cca aca tca gat gat ctt gtt tca tct gct gaa tgt tca     2019
Ser Ile Gln Pro Thr Ser Asp Asp Leu Val Ser Ser Ala Glu Cys Ser
                335                 340                 345

agt gat gat gaa gac ttt gtt gaa tgt gag ccg agt aca gca aac ccc     2067
Ser Asp Asp Glu Asp Phe Val Glu Cys Glu Pro Ser Thr Ala Asn Pro
            350                 355                 360

acg gag ccg gga atc aga cgg gtt ccg ggg gcc tca gag gtg atc cgg     2115
Thr Glu Pro Gly Ile Arg Arg Val Pro Gly Ala Ser Glu Val Ile Arg
        365                 370                 375

gag tcg agc agc aca aca ggg atg gtc gtc ggc att gtg gct gct gcc     2163
Glu Ser Ser Ser Thr Thr Gly Met Val Val Gly Ile Val Ala Ala Ala
    380                 385                 390

gcc ctc tgc atc ttg atc ctc ctg tac gcc atg tac aag tac agg aac     2211
Ala Leu Cys Ile Leu Ile Leu Leu Tyr Ala Met Tyr Lys Tyr Arg Asn
395                 400                 405                 410

agg gac gag ggg tcc tat caa gtg gac gag acg cgg aac tac atc agc     2259
Arg Asp Glu Gly Ser Tyr Gln Val Asp Glu Thr Arg Asn Tyr Ile Ser
                415                 420                 425

aac tcc gcc cag agc aac ggc acg ctc atg aag gag aag cag cag agc     2307
Asn Ser Ala Gln Ser Asn Gly Thr Leu Met Lys Glu Lys Gln Gln Ser
            430                 435                 440

tcg aag agc ggc cac aag aaa cag aaa aac aag gac agg gag tat tac     2355
Ser Lys Ser Gly His Lys Lys Gln Lys Asn Lys Asp Arg Glu Tyr Tyr
        445                 450                 455

gtg taa acatgcgaac actgctcaca cgcgagtttt cacagttatt tctatccacg     2411
Val

cctatgaatc tttggacggt gagatctcac agatgtcaga actgctggaa ctatgaaatg     2471

gggtatataa ccacgactct ggtggggaaa accgtttttt aaaggacaca cacacacaca     2531

gcgatgcatc tctctctaaa gctcagccac ggctgcggca aggtcccagc ggtcgctggg     2591

agacagaagg ttttgtgccc tgctgtatca taaagcacac acttagcgct ctggagccgg     2651

acggtggctc caccacttcc gcaggcctgg aaacttcctt ctccggagga ccttttacta     2711

aaaggtagaa gacttcatgg cttacttgtt ccataactcc aagtgagtct gtaatgtttg     2771

tgaagcttga ctgtaaccat gttttttctg tttaattatg taaaaaacaa aactacaaca     2831

acaaaaaaag aaaaaagtta aaaaagaaaa aaacaccaaa aaacaaaaac aaacaaaaaa     2891

aaaaacccac aacccttatc tggttctgac cagtgtgcgt gtaactttat gatctgaggg     2951

gaaaaatggc ttttgggttt ttgtttattt ttttgataat gactggacat cagaagagga     3011

aaaaaactca aaacaaaagc gagagagact attgccatat gaactcaaaa gctatcatgg     3071

tgttcactct acatatcagg ttatggtgtc tctagaatct gttgtttgtt tcctataaga     3131

tgctttgctg aacacatagc aaaattcatg tgacggatga taaattgatt cgaaaagctg     3191

gtcccccagg atctaatttc agaatttacc acccaaaccc ggaacagatg ggtttagggc     3251

tggtgttatc agagctattg gctttacgta acaatattgt tcctgtccat tcacccagcc     3311

aaattgtgtt aaagaggaaa gccccacaaa ctaaaacagc ctttcctagg gaagaggaag     3371

gggaggtggg ctggatctgt gactgattga aatgcatgca aataaaaaag acaatattaa 3431
agtctgttat caaaccagac agtaggggag ttcaactcgt gatggaacca caaaaggtca 3491
cacaagccaa ccatgtcatg ccagagtaca aaacacatag ttctttcccc gccccgaatg 3551
tgacaatggt tttcatagtg gtttaatttt gtagcctgac atttatggat aactctgtcc 3611
ttccatttgc tcacttctct cttcacccat cttttttaaa aacaaataaa tgaataaagc 3671
tgctgtgaca cacacacaaa aggaatttaa tagtataata tatatataaa taaatatata 3731
tacagatata tttatcatgg tatgtttgat gggatgactg acacaggaaa tctgttaaag 3791
tcttaaaatg gaatgagaat gttgttttaa aagaaaatag caaaacaaca aaaaagcaaa 3851
ccttaaaatg tgaagaaagt gtgaatttta gttttgtcac agttaactgt gtcaaagaga 3911
attaaaaaaa aaaacttcag attttgttta catattttac tacatttttg ctggtataat 3971
tccttagcca cctatgtaca tactgcttta agaaatgttt ttttcctgtt tatttctgtt 4031
tggtttatat tctggttgtc tttttctttt tgtaaagagg aaacaatgta cagaaaaaca 4091
ataaactggt tgtatggcca tagctatccg aaaagcaaga gacaaagcaa gacaaatatt 4151
cacacaaaaa tgaagtgtgt cctctggagg gtcagatata caatttcttt tgtacagatg 4211
aaaatcaatc agctgcttag atttagaaat ctactcttgc tggtctttgt aagttgcatg 4271
aatatttgac tttgaaaaaa tatcttaacg acatggggca aaaagtgcaa tctaaatggt 4331
agcctttact aatgtgtgtg gaaagaggtg ttcctcatta tctaatattt caatgtgtta 4391
agagtttaat ttttttgtta tcattaaaaa agacaggatt ataaagagat atcaaagcac 4451
gattttagat aacctaaacg gcccagccta tacgaagttg attatatctc gatgtctgta 4511
aaagattgct gttcttggag tcttgaggtc ttgtgaattg atttcctgct ttctttcatt 4571
tttttcaatt taagtaataa tacatttgtt atattccttt cagtgtaagt ttctatttgg 4631
acaattttat gggaacatgt gcattctcta tgtgagcttc tatcatattc ctgttttatt 4691
agcagaacct aaaggaattt atttaatgat gttgtgacat tactgctttt tcttttttct 4751
tttcttagtt catatttgca ttttcgttca aggatatgct tagcaataaa atgttcttcc 4811
caaaaaaaaa aaaaaaaaaa aaaaaaaaaa aaaaaa 4847

<210> 59
<211> 459
<212> PRT
<213> human

<400> 59

Met His Leu Arg Ile His Ala Arg Arg Ser Pro Pro Arg Arg Pro Ala
1               5                   10                  15

Trp Thr Leu Gly Ile Trp Phe Leu Phe Trp Gly Cys Ile Val Ser Ser
            20                  25                  30

Val Trp Ser Ser Ser Asn Val Ala Ser Ser Ser Ser Thr Ser Ser Ser
        35                  40                  45

Pro Gly Ser His Ser Gln His Glu His His Phe His Gly Ser Lys His
    50                  55                  60

His Ser Val Pro Ile Ser Ile Tyr Arg Ser Pro Val Ser Leu Arg Gly
65                  70                  75                  80

Gly His Ala Gly Ala Thr Tyr Ile Phe Gly Lys Ser Gly Gly Leu Ile
                85                  90                  95

Leu Tyr Thr Trp Pro Ala Asn Asp Arg Pro Ser Thr Arg Ser Asp Arg
            100                 105                 110

Leu Ala Val Gly Phe Ser Thr Thr Val Lys Asp Gly Ile Leu Val Arg
        115                 120                 125

Ile Asp Ser Ala Pro Gly Leu Gly Asp Phe Leu Gln Leu His Ile Glu
    130                 135                 140

Gln Gly Lys Ile Gly Val Val Phe Asn Ile Gly Thr Val Asp Ile Ser
145                 150                 155                 160

Ile Lys Glu Glu Arg Thr Pro Val Asn Asp Gly Lys Tyr His Val Val
                165                 170                 175

Arg Phe Thr Arg Asn Gly Gly Asn Ala Thr Leu Gln Val Asp Asn Trp
            180                 185                 190

Pro Val Asn Glu His Tyr Pro Thr Gly Asn Thr Asp Asn Glu Arg Phe
        195                 200                 205

Gln Met Val Lys Gln Lys Ile Pro Phe Lys Tyr Asn Arg Pro Val Glu
    210                 215                 220

Glu Trp Leu Gln Glu Lys Gly Arg Gln Leu Thr Ile Phe Asn Thr Gln
225                 230                 235                 240

Ala Gln Ile Ala Ile Gly Gly Lys Asp Lys Gly Arg Leu Phe Gln Gly
                245                 250                 255

Gln Leu Ser Gly Leu Tyr Tyr Asp Gly Leu Lys Val Leu Asn Met Ala
            260                 265                 270

Ala Glu Asn Asn Pro Asn Ile Lys Ile Asn Gly Ser Val Arg Leu Val
        275                 280                 285

Gly Glu Val Pro Ser Ile Leu Gly Thr Thr Gln Thr Thr Ser Met Pro
    290                 295                 300

Pro Glu Met Ser Thr Thr Val Met Glu Thr Thr Thr Thr Met Ala Thr
305                 310                 315                 320

Thr Thr Thr Arg Lys Asn Arg Ser Thr Ala Ser Ile Gln Pro Thr Ser
                325                 330                 335

Asp Asp Leu Val Ser Ser Ala Glu Cys Ser Ser Asp Asp Glu Asp Phe
            340                 345                 350

Val Glu Cys Glu Pro Ser Thr Ala Asn Pro Thr Glu Pro Gly Ile Arg
        355                 360                 365

Arg Val Pro Gly Ala Ser Glu Val Ile Arg Glu Ser Ser Ser Thr Thr
    370                 375                 380

Gly Met Val Val Gly Ile Val Ala Ala Ala Ala Leu Cys Ile Leu Ile
385                 390                 395                 400

Leu Leu Tyr Ala Met Tyr Lys Tyr Arg Asn Arg Asp Glu Gly Ser Tyr
                405                 410                 415

Gln Val Asp Glu Thr Arg Asn Tyr Ile Ser Asn Ser Ala Gln Ser Asn
            420                 425                 430

Gly Thr Leu Met Lys Glu Lys Gln Gln Ser Ser Lys Ser Gly His Lys
        435                 440                 445

Lys Gln Lys Asn Lys Asp Arg Glu Tyr Tyr Val
    450                 455

<210>  60
<211>  2228
<212>  DNA
<213>  human

<220>
<221>  CDS
<222>  (74)..(1810)

<400>  60
acacgccttt cttattggat accggctttt catggggccg agcgccgctg ggtaggcgga      60
agtagccgca ggc atg gcg gcg gct atg ccg ctt gct ctg ctc gtc ctg        109
ttg ctc ctg ggg ccc ggc ggc tgg tgc ctt gca gaa ccc cca cgc gac        157
agc ctg cgg gag gaa ctt gtc atc acc ccg ctg cct tcc ggg gac gta        205
gcc gcc aca ttc cag ttc cgc acg cgc tgg gat tcg gag ctt cag cgg        253
gaa gga gtg tcc cat tac agg ctc ttt ccc aaa gcc ctg ggg cag ctg        301
atc tcc aag tat tct cta cgg gag ctg cac ctg tca ttc aca caa ggc 349
ttt tgg agg acc cga tac tgg ggg cca ccc ttc ctg cag gcc cca tca 397
ggt gca gag ctg tgg gtc tgg ttc caa gac act gtc act gat gtg gat 445
aaa tct tgg aag gag ctc agt aat gtc ctc tca ggg atc ttc tgc gcc 493
tct ctc aac ttc atc gac tcc acc aac aca gtc act ccc act gcc tcc 541
ttc aaa ccc ctg ggt ctg gcc aat gac act gac cac tac ttt ctg cgc 589
tat gct gtg ctg ccg cgg gag gtg gtc tgc acc gaa aac ctc acc ccc 637
tgg aag aag ctc ttg ccc tgt agt tcc aag gca ggc ctc tct gtg ctg 685
ctg aag gca gat cgc ttg ttc cac acc agc tac cac tcc cag gca gtg 733
cat atc cgc cct gtt tgc aga aat gca cgc tgt act agc atc tcc tgg 781
gag ctg agg cag acc ctg tca gtt gta ttt gat gcc ttc atc acg ggg 829
cag gga aag aaa gac tgg tcc ctc ttc cgg atg ttc tcc cga acc ctc 877
acg gag ccc tgc ccc ctg gct tca gag agc cga gtc tat gtg gac atc 925
acc acc tac aac cag gac aac gag aca tta gag gtg cac cca ccc ccg 973
acc act aca tat cag gac gtc atc cta ggc act cgg aag acc tat gcc 1021
atc tat gac ttg ctt gac acc gcc atg atc aac aac tct cga aac ctc 1069
aac atc cag ctc aag tgg aag aga ccc cca gag aat gag gcc ccc cca 1117
gtg ccc ttc ctg cat gcc cag cgg tac gtg agt ggc tat ggg ctg cag      1165
aag ggg gag ctg agc aca ctg ctg tac aac acc cac cca tac cgg gcc      1213
ttc ccg gtg ctg ctg ctg gac acc gta ccc tgg tat ctg cgg ctg tat      1261
gtg cac acc ctc acc atc acc tcc aag ggc aag gag aac aaa cca agt      1309
tac atc cac tac cag cct gcc cag gac cgg ctg caa ccc cac ctc ctg      1357
gag atg ctg att cag ctg ccg gcc aac tca gtc acc aag gtt tcc atc      1405
cag ttt gag cgg gcg ctg ctg aag tgg acc gag tac acg cca gat cct      1453
aac cat ggc ttc tat gtc agc cca tct gtc ctc agc gcc ctt gtg ccc      1501
agc atg gta gca gcc aag cca gtg gac tgg gaa gag agt ccc ctc ttc      1549
aac agc ctg ttc cca gtc tct gat ggc tct aac tac ttt gtg cgg ctc      1597
tac acg gag ccg ctg ctg gtg aac ctg ccg aca ccg gac ttc agc atg      1645
ccc tac aac gtg atc tgc ctc acg tgc act gtg gtg gcc gtg tgc tat      1693
ggc tcc ttc tac aat ctc ctc acc cga acc ttc cac atc gag gag ccc      1741
cgc aca ggt ggc ctg gcc aag cgg ctg gcc aac ctt atc cgg cgc gcc      1789
cga ggt gtc ccc cca ctc tga ttcttgccct ttccagcagc tgcagctgcc      1840
gtttctctct ggggagggga gcccaagggc tgtttctgcc acttgctctc ctcagagttg      1900
gcttttgaac caaagtgccc tggaccaggt cagggcctac agctgtgttg tccagtacag      1960
gagccacgag ccaaatgtgg catttgaatt tgaattaact tagaaattca tttcctcacc      2020
tgtagtggcc acctctatat tgaggtgctc aataagcaaa agtggtcggt ggctgctgta 2080
ttggacagca cagaaaaaga tttccatcac cacagaaagg tcggctggca gcactggcca 2140
aggtgatggg gtgtgctaca cagtgtatgt cactgtgtag tggatggagt ttactgtttg 2200
tggaataaaa acggctgttt ccgtggtt 2228

<210> 61
<211> 578
<212> PRT
<213> human

<400> 61

<210>  62
<211>  2207
<212>  DNA
<213>  human

<220>
<221>  CDS
<222>  (137)..(1765)

<400>  62
   agcttgttag agctgagctg ccctactaca gcagctgccg gcccctagga cagagcaggg      60
   acctcaacta cactgatcac cagccccatc ggatccagac ccggccacca gctctggctc     120
   gtcttgcccc agtgcc atg acc ttc tcg gag atc ctg gac cgt gtg gga agc     172
                     Met Thr Phe Ser Glu Ile Leu Asp Arg Val Gly Ser
                     1               5                   10
   atg ggc cat ttc cag ttc ctg cat gta gcc ata ctg ggc ctc ccg atc       220
   Met Gly His Phe Gln Phe Leu His Val Ala Ile Leu Gly Leu Pro Ile
           15                  20                  25
   ctc aac atg gcc aac cac aac ctg ctg cag atc ttc aca gcc gcc acc       268
   Leu Asn Met Ala Asn His Asn Leu Leu Gln Ile Phe Thr Ala Ala Thr
       30                  35                  40
   cct gtc cac cac tgt cgc ccg ccc cac aat gcc tcc aca ggg cct tgg       316
   Pro Val His His Cys Arg Pro Pro His Asn Ala Ser Thr Gly Pro Trp
   45                  50                  55                  60
   gtg ctc ccc atg ggc cca aat ggg aag cct gag agg tgc ctc cgt ttt       364
   Val Leu Pro Met Gly Pro Asn Gly Lys Pro Glu Arg Cys Leu Arg Phe
                   65                  70                  75
   gta cat ccg ccc aat gcc agc ctg ccc aat gac acc cag agg gcc atg       412
   Val His Pro Pro Asn Ala Ser Leu Pro Asn Asp Thr Gln Arg Ala Met
               80                  85                  90
   gag cca tgc ctg gat ggc tgg gtc tac aac agc acc aag gac tcc att 460
   Glu Pro Cys Leu Asp Gly Trp Val Tyr Asn Ser Thr Lys Asp Ser Ile
   95 100 105
   gtg aca gag tgg gac ttg gtg tgc aac tcc aac aaa ctg aag gag atg 508
   Val Thr Glu Trp Asp Leu Val Cys Asn Ser Asn Lys Leu Lys Glu Met
   110 115 120
   gcc cag tct atc ttc atg gca ggt ata ctg att gga ggg ctc gtg ctt 556
   Ala Gln Ser Ile Phe Met Ala Gly Ile Leu Ile Gly Gly Leu Val Leu
   125 130 135 140
   gga gac ctg tct gac agg ttt ggc cgc agg ccc atc ctg acc tgc agc 604
   Gly Asp Leu Ser Asp Arg Phe Gly Arg Arg Pro Ile Leu Thr Cys Ser
   145 150 155
   tac ctg ctg ctg gca gcc agc ggc tcc ggt gca gcc ttc agc ccc acc 652
   Tyr Leu Leu Leu Ala Ala Ser Gly Ser Gly Ala Ala Phe Ser Pro Thr
   160 165 170
   ttc ccc atc tac atg gtc ttc cgc ttc ctg tgt ggc ttt ggc atc tca 700
   Phe Pro Ile Tyr Met Val Phe Arg Phe Leu Cys Gly Phe Gly Ile Ser
   175 180 185
   ggc att acc ctg agc acc gtc atc ttg aat gtg gaa tgg gtg cct acc 748
   Gly Ile Thr Leu Ser Thr Val Ile Leu Asn Val Glu Trp Val Pro Thr
   190 195 200
   cgg atg cgg gcc atc atg tcg aca gca ctc ggg tac tgc tac acc ttt 796
   Arg Met Arg Ala Ile Met Ser Thr Ala Leu Gly Tyr Cys Tyr Thr Phe
   205 210 215 220
   ggc cag ttc att ctg ccc ggc ctg gcc tac gcc atc ccc cag tgg cgt 844
   Gly Gln Phe Ile Leu Pro Gly Leu Ala Tyr Ala Ile Pro Gln Trp Arg
   225 230 235
   tgg ctg cag tta act gtg tcc att ccc ttc ttc gtc ttc ttc cta tca 892
   Trp Leu Gln Leu Thr Val Ser Ile Pro Phe Phe Val Phe Phe Leu Ser
   240 245 250
   tcc tgg tgg aca cca gag tcc ata cgc tgg ttg gtc ttg tct gga aag 940
   Ser Trp Trp Thr Pro Glu Ser Ile Arg Trp Leu Val Leu Ser Gly Lys
   255 260 265
   tcc tcg aag gcc ctg aag ata ctc cgg cgg gtg gct gtc ttc aat ggc 988
   Ser Ser Lys Ala Leu Lys Ile Leu Arg Arg Val Ala Val Phe Asn Gly
   270 275 280
   aag aag gaa gag gga gaa agg ctc agc ttg gag gag ctc aaa ctc aac 1036
   Lys Lys Glu Glu Gly Glu Arg Leu Ser Leu Glu Glu Leu Lys Leu Asn
   285 290 295 300
   ctg cag aag gag atc tcc ttg gcc aag gcc aag tac acc gca agt gac 1084
   Leu Gln Lys Glu Ile Ser Leu Ala Lys Ala Lys Tyr Thr Ala Ser Asp
   305 310 315
   ctg ttc cgg ata ccc atg ctg cgc cgc atg acc ttc tgt ctt tcc ctg 1132
   Leu Phe Arg Ile Pro Met Leu Arg Arg Met Thr Phe Cys Leu Ser Leu
   320 325 330
   gcc tgg ttt gct acc ggt ttt gcc tac tat agt ttg gct atg ggt gtg 1180
   Ala Trp Phe Ala Thr Gly Phe Ala Tyr Tyr Ser Leu Ala Met Gly Val
   335 340 345
   gaa gaa ttt gga gtc aac ctc tac atc ctc cag atc atc ttt ggt ggg 1228
   Glu Glu Phe Gly Val Asn Leu Tyr Ile Leu Gln Ile Ile Phe Gly Gly
   350 355 360
   gtc gat gtc cca gcc aag ttc atc acc atc ctc tcc tta agc tac ctg     1276
   Val Asp Val Pro Ala Lys Phe Ile Thr Ile Leu Ser Leu Ser Tyr Leu
   365                 370                 375                 380
   ggc cgg cat acc act cag gcc gct gcc ctg ctc ctg gca gga ggg gcc     1324
   Gly Arg His Thr Thr Gln Ala Ala Ala Leu Leu Leu Ala Gly Gly Ala
                   385                 390                 395
   atc ttg gct ctc acc ttt gtg ccc ttg gac ttg cag acc gtg agg aca     1372
   Ile Leu Ala Leu Thr Phe Val Pro Leu Asp Leu Gln Thr Val Arg Thr
               400                 405                 410
   gta ttg gct gtg ttt ggg aag gga tgc cta tcc agc tcc ttc agc tgc     1420
   Val Leu Ala Val Phe Gly Lys Gly Cys Leu Ser Ser Ser Phe Ser Cys
           415                 420                 425
   ctc ttc ctc tac aca agt gaa tta tac ccc aca gtc atc agg caa aca     1468
   Leu Phe Leu Tyr Thr Ser Glu Leu Tyr Pro Thr Val Ile Arg Gln Thr
       430                 435                 440
   ggt atg ggc gta agt aac ctg tgg acc cgc gtg gga agc atg gtg tcc     1516
   Gly Met Gly Val Ser Asn Leu Trp Thr Arg Val Gly Ser Met Val Ser
   445                 450                 455                 460
   ccg ctg gtg aaa atc acg ggt gag gta cag ccc ttc atc ccc aat atc     1564
   Pro Leu Val Lys Ile Thr Gly Glu Val Gln Pro Phe Ile Pro Asn Ile
                   465                 470                 475
   atc tac ggg atc acc gcc ctc ctc ggg ggc agt gct gcc ctc ttc ctg     1612
   Ile Tyr Gly Ile Thr Ala Leu Leu Gly Gly Ser Ala Ala Leu Phe Leu
               480                 485                 490
   cct gag acc ctg aat cag ccc ttg cca gag act atc gaa gac ctg gaa     1660
   Pro Glu Thr Leu Asn Gln Pro Leu Pro Glu Thr Ile Glu Asp Leu Glu
           495                 500                 505
   aac tgg tcc ctg cgg gca aag aag cca aag cag gag cca gag gtg gaa     1708
   Asn Trp Ser Leu Arg Ala Lys Lys Pro Lys Gln Glu Pro Glu Val Glu
       510                 515                 520
   aag gcc tcc cag agg atc cct cta cag cct cac gga cca ggc ctg ggc     1756
   Lys Ala Ser Gln Arg Ile Pro Leu Gln Pro His Gly Pro Gly Leu Gly
   525                 530                 535                 540
   tcc agc tga ggacaacgga accccctttc cctgccctcc agagactgat             1805
   Ser Ser
   cctagccagg caccttagga gtatagggag gccccatata ggtccatcct cctaggatga    1865
   agccttctga gagcttggtg aaggtgtctc catcaccacc accagagcct cctgcccagc    1925
   cctggccagt tcaaaggttc agccatccct gcccttgttc tccctgcaac ccaggccctg    1985
   ccattcttct gtctagccct tccccactgg ccaccttccc ccactgtccc ggtcctcttc    2045
   ccctgaggtc ccctgatatc ccctggctca gtcctaacaa gactgagtct taacaagatg    2105
   agaagtcctc cccttcttgc ctcccacact tttctttgat gggaggtttc aataaacagc    2165
   gataagaact ctaaaaaaaa aaaaaaaaaa aaaaaaaaaa aa                          2207

<210>  63
<211>  542
<212>  PRT
<213>  human

<400> 63

Met Thr Phe Ser Glu Ile Leu Asp Arg Val Gly Ser Met Gly His Phe
1               5                   10                  15

Gln Phe Leu His Val Ala Ile Leu Gly Leu Pro Ile Leu Asn Met Ala
            20                  25                  30

Asn His Asn Leu Leu Gln Ile Phe Thr Ala Ala Thr Pro Val His His
        35                  40                  45

Cys Arg Pro Pro His Asn Ala Ser Thr Gly Pro Trp Val Leu Pro Met
    50                  55                  60

Gly Pro Asn Gly Lys Pro Glu Arg Cys Leu Arg Phe Val His Pro Pro
65                  70                  75                  80

Asn Ala Ser Leu Pro Asn Asp Thr Gln Arg Ala Met Glu Pro Cys Leu
                85                  90                  95

Asp Gly Trp Val Tyr Asn Ser Thr Lys Asp Ser Ile Val Thr Glu Trp
            100                 105                 110

Asp Leu Val Cys Asn Ser Asn Lys Leu Lys Glu Met Ala Gln Ser Ile
        115                 120                 125

Phe Met Ala Gly Ile Leu Ile Gly Gly Leu Val Leu Gly Asp Leu Ser
    130                 135                 140

Asp Arg Phe Gly Arg Arg Pro Ile Leu Thr Cys Ser Tyr Leu Leu Leu
145                 150                 155                 160

Ala Ala Ser Gly Ser Gly Ala Ala Phe Ser Pro Thr Phe Pro Ile Tyr
                165                 170                 175

Met Val Phe Arg Phe Leu Cys Gly Phe Gly Ile Ser Gly Ile Thr Leu
            180                 185                 190

Ser Thr Val Ile Leu Asn Val Glu Trp Val Pro Thr Arg Met Arg Ala
        195                 200                 205

Ile Met Ser Thr Ala Leu Gly Tyr Cys Tyr Thr Phe Gly Gln Phe Ile
    210                 215                 220

Leu Pro Gly Leu Ala Tyr Ala Ile Pro Gln Trp Arg Trp Leu Gln Leu
225                 230                 235                 240

Thr Val Ser Ile Pro Phe Phe Val Phe Phe Leu Ser Ser Trp Trp Thr
                245                 250                 255

Pro Glu Ser Ile Arg Trp Leu Val Leu Ser Gly Lys Ser Ser Lys Ala
            260                 265                 270

Leu Lys Ile Leu Arg Arg Val Ala Val Phe Asn Gly Lys Lys Glu Glu
        275                 280                 285

Gly Glu Arg Leu Ser Leu Glu Glu Leu Lys Leu Asn Leu Gln Lys Glu
    290                 295                 300

Ile Ser Leu Ala Lys Ala Lys Tyr Thr Ala Ser Asp Leu Phe Arg Ile
305                 310                 315                 320

Pro Met Leu Arg Arg Met Thr Phe Cys Leu Ser Leu Ala Trp Phe Ala
                325                 330                 335

Thr Gly Phe Ala Tyr Tyr Ser Leu Ala Met Gly Val Glu Glu Phe Gly
            340                 345                 350

Val Asn Leu Tyr Ile Leu Gln Ile Ile Phe Gly Gly Val Asp Val Pro
        355                 360                 365

Ala Lys Phe Ile Thr Ile Leu Ser Leu Ser Tyr Leu Gly Arg His Thr
    370                 375                 380

Thr Gln Ala Ala Ala Leu Leu Leu Ala Gly Gly Ala Ile Leu Ala Leu
385                 390                 395                 400

Thr Phe Val Pro Leu Asp Leu Gln Thr Val Arg Thr Val Leu Ala Val
                405                 410                 415

Phe Gly Lys Gly Cys Leu Ser Ser Ser Phe Ser Cys Leu Phe Leu Tyr
            420                 425                 430

Thr Ser Glu Leu Tyr Pro Thr Val Ile Arg Gln Thr Gly Met Gly Val
        435                 440                 445

Ser Asn Leu Trp Thr Arg Val Gly Ser Met Val Ser Pro Leu Val Lys
    450                 455                 460

Ile Thr Gly Glu Val Gln Pro Phe Ile Pro Asn Ile Ile Tyr Gly Ile
465                 470                 475                 480

Thr Ala Leu Leu Gly Gly Ser Ala Ala Leu Phe Leu Pro Glu Thr Leu
                485                 490                 495

Asn Gln Pro Leu Pro Glu Thr Ile Glu Asp Leu Glu Asn Trp Ser Leu
            500                 505                 510

Arg Ala Lys Lys Pro Lys Gln Glu Pro Glu Val Glu Lys Ala Ser Gln
        515                 520                 525

Arg Ile Pro Leu Gln Pro His Gly Pro Gly Leu Gly Ser Ser
    530                 535                 540

<210> 64
<211> 1114
<212> DNA
<213> human

<400> 64
tctcatcccc ctggaaagaa atttcaaggg ataaagcacc atggatctaa cttatattcc 60

cgaagaccta tccagttgtc caaaatttgt aaataaatcc tgtcctccca ccaaccgctc 120

ttttcatgtc caggtgataa tgtattcggt tatgactgga gccatgatta tcactatttg 180

gaaacttggt tataatggtt tccatatcgc atttcaaaca gcttcactct cccacaaact 240

ttctgatcct ctccatggca accacggact ttctgctggg ttttgtcatt atgccataca 300

gcataatgcg atcagtggag agttgctggt actttgggga tggcttttgt aaattccaca 360

caagctttga catgatgctc agactgacct ccattttcca cctctgttcc attgctattg 420

accgatttta tgccgtgtgt taccctttac attacacaac caaaatgacg aactccacca 480

taaagcaact gctggcattt tgctggtcag ttcctgctct tttttctttt ggtttagttc 540

tatctgaggc cgatgtttcc ggtatgcaga gctataagat acttgttgct tgcttcaatt 600

tctgtgccct tactttcaac aaattctggg ggacaatatt gttcactaca tgtttcttta 660

cccctggctc catcatggtt ggtatttatg gcaaaatctt tatcgtttcc aaacagcatg 720

ctcgagtcat cagccatgtg cctgaaaaca caaagggggc agtgaaaaaa cacctatcca 780

agaaaaagga caggaaagca gcgaagacac tgggtatagt aatgggggtg tttctggctt 840

gctggttgcc ttgttttctt gctgttctga ttgacccata cctagactac tccactccca 900

tactaatatt ggatctttta gtgtggctcc ggtacttcaa ctctacttgc aaccctctta 960

ttcatggctt ttttaatcca tggtttcaga aagcattcaa gtacatagtg tcaggaaata 1020

tatttagctc ccattcagaa actgcaaatt tgtttcctga agcacattaa taaaaagctt 1080

tgcaaaagtg aatagaatat tgcaaatgag attg 1114

<210> 65
<211> 1778
<212> DNA
<213> human

<220>
<221> CDS
<222> (246)..(1490)

<400> 65
cggctcctct cagcggcggt ggcccaggta gaggggtccg cgctggcggc ggcggcggcg 60

ctgttccccg cgcggtccgc ggagcggggt ccgggctgcg cgacgtgggg cggcggcggc 120

actgcggccc cggcccaagc ccgaccccgg gtcccctcct cggccgcccc ccgcccggcc 180

gcccgccctc gggcctcccc ccgggccctc ggtcccctcc cccgctggcg gggcccggac 240

agaag atg gtg cag aag aaa cca gcc gaa ctt cag ggt ttc cac cgt tcg 290
Met Val Gln Lys Lys Pro Ala Glu Leu Gln Gly Phe His Arg Ser

1                 5                   10                  15

ttc aag ggg cag aac ccc ttc gag ctg gcc ttc tcc cta gac cag ccc     338
Phe Lys Gly Gln Asn Pro Phe Glu Leu Ala Phe Ser Leu Asp Gln Pro
                20                  25                  30

gac cac gga gac tct gac ttt ggc ctg cag tgc tca gcc cgc cct gac     386
Asp His Gly Asp Ser Asp Phe Gly Leu Gln Cys Ser Ala Arg Pro Asp
            35                  40                  45

atg ccc gcc agc cag ccc att gac atc ccg gac gcc aag aag agg ggc     434
Met Pro Ala Ser Gln Pro Ile Asp Ile Pro Asp Ala Lys Lys Arg Gly
        50                  55                  60

aag aag aag aag cgc ggc cgg gcc acc gac agc ttc tcg ggc agg ttt     482
Lys Lys Lys Lys Arg Gly Arg Ala Thr Asp Ser Phe Ser Gly Arg Phe
    65                  70                  75

gaa gac gtc tac cag ctg cag gaa gat gtg ctg ggg gag ggc gct cat     530
Glu Asp Val Tyr Gln Leu Gln Glu Asp Val Leu Gly Glu Gly Ala His
80                  85                  90                  95

gcc cga gtg cag acc tgc atc aac ctg atc acc agc cag gag tac gcc     578
Ala Arg Val Gln Thr Cys Ile Asn Leu Ile Thr Ser Gln Glu Tyr Ala
                100                 105                 110

gtc aag atc att gag aag cag cca ggc cac att cgg agc agg gtt ttc     626
Val Lys Ile Ile Glu Lys Gln Pro Gly His Ile Arg Ser Arg Val Phe
            115                 120                 125

agg gag gtg gag atg ctg tac cag tgc cag gga cac agg aac gtc cta     674
Arg Glu Val Glu Met Leu Tyr Gln Cys Gln Gly His Arg Asn Val Leu
        130                 135                 140

gag ctg att gag ttc ttc gag gag gag gac cgc ttc tac ctg gtg ttt     722
Glu Leu Ile Glu Phe Phe Glu Glu Glu Asp Arg Phe Tyr Leu Val Phe
    145                 150                 155

gag aag atg cgg gga ggc tcc atc ctg agc cac atc cac aag cgc cgg     770
Glu Lys Met Arg Gly Gly Ser Ile Leu Ser His Ile His Lys Arg Arg
160                 165                 170                 175

cac ttc aac gag ctg gag gcc agc gtg gtg gtg cag gac gtg gcc agc     818
His Phe Asn Glu Leu Glu Ala Ser Val Val Val Gln Asp Val Ala Ser
                180                 185                 190

gcc ttg gac ttt ctg cat aac aaa ggc atc gcc cac agg gac cta aag     866
Ala Leu Asp Phe Leu His Asn Lys Gly Ile Ala His Arg Asp Leu Lys
            195                 200                 205

ccg gaa aac atc ctc tgt gag cac ccc aac cag gtc tcc ccc gtg aag     914
Pro Glu Asn Ile Leu Cys Glu His Pro Asn Gln Val Ser Pro Val Lys
        210                 215                 220

atc tgt gac ttc gac ctg ggc agc ggc atc aaa ctc aac ggg gac tgc     962
Ile Cys Asp Phe Asp Leu Gly Ser Gly Ile Lys Leu Asn Gly Asp Cys
    225                 230                 235

tcc cct atc tcc acc ccg gag ctg ctc act ccg tgc ggc tcg gcg gag     1010
Ser Pro Ile Ser Thr Pro Glu Leu Leu Thr Pro Cys Gly Ser Ala Glu
240                 245                 250                 255

tac atg gcc ccg gag gta gtg gag gcc ttc agc gag gag gct agc atc     1058
Tyr Met Ala Pro Glu Val Val Glu Ala Phe Ser Glu Glu Ala Ser Ile
                260                 265                 270

tac gac aag cgc tgc gac ctg tgg agc ctg ggc gtc atc ttg tat atc     1106
Tyr Asp Lys Arg Cys Asp Leu Trp Ser Leu Gly Val Ile Leu Tyr Ile

            275                 280                 285

cta ctc agc ggc tac ccg ccc ttc gtg ggc cgc tgt ggc agc gac tgc 1154
Leu Leu Ser Gly Tyr Pro Pro Phe Val Gly Arg Cys Gly Ser Asp Cys
        290                 295                 300

ggc tgg gac cgc ggc gag gcc tgc cct gcc tgc cag aac atg ctg ttt 1202
Gly Trp Asp Arg Gly Glu Ala Cys Pro Ala Cys Gln Asn Met Leu Phe
    305                 310                 315

gag agc atc cag gag ggc aag tac gag ttc ccc gac aag gac tgg gcc 1250
Glu Ser Ile Gln Glu Gly Lys Tyr Glu Phe Pro Asp Lys Asp Trp Ala
320                 325                 330                 335

cac atc tcc tgc gct gcc aaa gac ctc atc tcc aag ctg ctg gtc cgt 1298
His Ile Ser Cys Ala Ala Lys Asp Leu Ile Ser Lys Leu Leu Val Arg
                340                 345                 350

gac gcc aag cag agg ctg agt gcc gcc caa gtc ctg cag cac ccc tgg 1346
Asp Ala Lys Gln Arg Leu Ser Ala Ala Gln Val Leu Gln His Pro Trp
            355                 360                 365

gtt cag ggg tgc gcc ccg gag aac acc ttg ccc act ccc atg gtc ctg 1394
Val Gln Gly Cys Ala Pro Glu Asn Thr Leu Pro Thr Pro Met Val Leu
        370                 375                 380

cag agg tgg gac agt cac ttc ctc ctc cct ccc cac ccc tgt cgc atc 1442
Gln Arg Trp Asp Ser His Phe Leu Leu Pro Pro His Pro Cys Arg Ile
    385                 390                 395

cac gtg cga cct gga gga ctg gtc aga acc gtt act gtg aat gag tga 1490
His Val Arg Pro Gly Gly Leu Val Arg Thr Val Thr Val Asn Glu
400                 405                 410

agatcctgga ggaccctggg ccccaggcca gctcccatcg ctgggggacg gtgaacggcc 1550

atgtgttaat gttacgatgt ttttaaaaga caaaaaaaaa aaaaaaacct caaaagtttt 1610

tttaaagtgg gggaaaaaca tccaagcact ttaattccaa tgtaccaggt gaactgacgg 1670

agctcagaag ttttccttta caccaactgt caatgccgga attttgtatt ctgttttgta 1730

aagatttaat aaaagtcaaa aaacttgcaa aaaaaaaaaa aaaaaaaa 1778

<210> 66
<211> 414
<212> PRT
<213> human

<400> 66

Met Val Gln Lys Lys Pro Ala Glu Leu Gln Gly Phe His Arg Ser Phe
1               5                   10                  15

Lys Gly Gln Asn Pro Phe Glu Leu Ala Phe Ser Leu Asp Gln Pro Asp
            20                  25                  30

His Gly Asp Ser Asp Phe Gly Leu Gln Cys Ser Ala Arg Pro Asp Met
        35                  40                  45

Pro Ala Ser Gln Pro Ile Asp Ile Pro Asp Ala Lys Lys Arg Gly Lys
    50                  55                  60

Lys Lys Lys Arg Gly Arg Ala Thr Asp Ser Phe Ser Gly Arg Phe Glu
65                  70                  75                  80

Asp Val Tyr Gln Leu Gln Glu Asp Val Leu Gly Glu Gly Ala His Ala
                85                  90                  95

Arg Val Gln Thr Cys Ile Asn Leu Ile Thr Ser Gln Glu Tyr Ala Val
            100                 105                 110

Lys Ile Ile Glu Lys Gln Pro Gly His Ile Arg Ser Arg Val Phe Arg
        115                 120                 125

Glu Val Glu Met Leu Tyr Gln Cys Gln Gly His Arg Asn Val Leu Glu
    130                 135                 140

Leu Ile Glu Phe Phe Glu Glu Glu Asp Arg Phe Tyr Leu Val Phe Glu
145                 150                 155                 160

Lys Met Arg Gly Gly Ser Ile Leu Ser His Ile His Lys Arg Arg His
                165                 170                 175

Phe Asn Glu Leu Glu Ala Ser Val Val Val Gln Asp Val Ala Ser Ala
            180                 185                 190

Leu Asp Phe Leu His Asn Lys Gly Ile Ala His Arg Asp Leu Lys Pro
        195                 200                 205

Glu Asn Ile Leu Cys Glu His Pro Asn Gln Val Ser Pro Val Lys Ile
    210                 215                 220

Cys Asp Phe Asp Leu Gly Ser Gly Ile Lys Leu Asn Gly Asp Cys Ser
225                 230                 235                 240

Pro Ile Ser Thr Pro Glu Leu Leu Thr Pro Cys Gly Ser Ala Glu Tyr
                245                 250                 255

Met Ala Pro Glu Val Val Glu Ala Phe Ser Glu Glu Ala Ser Ile Tyr
            260                 265                 270

Asp Lys Arg Cys Asp Leu Trp Ser Leu Gly Val Ile Leu Tyr Ile Leu
        275                 280                 285

Leu Ser Gly Tyr Pro Pro Phe Val Gly Arg Cys Gly Ser Asp Cys Gly
    290                 295                 300

Trp Asp Arg Gly Glu Ala Cys Pro Ala Cys Gln Asn Met Leu Phe Glu
305                 310                 315                 320

Ser Ile Gln Glu Gly Lys Tyr Glu Phe Pro Asp Lys Asp Trp Ala His
                325                 330                 335

Ile Ser Cys Ala Ala Lys Asp Leu Ile Ser Lys Leu Leu Val Arg Asp
            340                 345                 350

Ala Lys Gln Arg Leu Ser Ala Ala Gln Val Leu Gln His Pro Trp Val
        355                 360                 365

Gln Gly Cys Ala Pro Glu Asn Thr Leu Pro Thr Pro Met Val Leu Gln
    370                 375                 380

Arg Trp Asp Ser His Phe Leu Leu Pro Pro His Pro Cys Arg Ile His
385                 390                 395                 400

Val Arg Pro Gly Gly Leu Val Arg Thr Val Thr Val Asn Glu
                405                 410

<210> 67
<211> 3805
<212> DNA
<213> human

<220>
<221> CDS
<222> (246)..(1643)

<400> 67
   cggctcctct cagcggcggt ggcccaggta gaggggtccg cgctggcggc ggcggcggcg 60
   ctgttccccg cgcggtccgc ggagcggggt ccgggctgcg cgacgtgggg cggcggcggc 120
   actgcggccc cggcccaagc ccgaccccgg gtcccctcct cggccgcccc ccgcccggcc 180
   gcccgccctc gggcctcccc ccgggccctc ggtcccctcc cccgctggcg gggcccggac 240
   agaag atg gtg cag aag aaa cca gcc gaa ctt cag ggt ttc cac cgt tcg 290
   Met Val Gln Lys Lys Pro Ala Glu Leu Gln Gly Phe His Arg Ser
   1 5 10 15
   ttc aag ggg cag aac ccc ttc gag ctg gcc ttc tcc cta gac cag ccc 338
   Phe Lys Gly Gln Asn Pro Phe Glu Leu Ala Phe Ser Leu Asp Gln Pro
   20 25 30
   gac cac gga gac tct gac ttt ggc ctg cag tgc tca gcc cgc cct gac 386
   Asp His Gly Asp Ser Asp Phe Gly Leu Gln Cys Ser Ala Arg Pro Asp
   35 40 45
   atg ccc gcc agc cag ccc att gac atc ccg gac gcc aag aag agg ggc 434
   Met Pro Ala Ser Gln Pro Ile Asp Ile Pro Asp Ala Lys Lys Arg Gly
   50 55 60
   aag aag aag aag cgc ggc cgg gcc acc gac agc ttc tcg ggc agg ttt 482
   Lys Lys Lys Lys Arg Gly Arg Ala Thr Asp Ser Phe Ser Gly Arg Phe
   65 70 75
   gaa gac gtc tac cag ctg cag gaa gat gtg ctg ggg gag ggc gct cat 530
   Glu Asp Val Tyr Gln Leu Gln Glu Asp Val Leu Gly Glu Gly Ala His
   80 85 90 95
   gcc cga gtg cag acc tgc atc aac ctg atc acc agc cag gag tac gcc 578
   Ala Arg Val Gln Thr Cys Ile Asn Leu Ile Thr Ser Gln Glu Tyr Ala
   100 105 110
   gtc aag atc att gag aag cag cca ggc cac att cgg agc agg gtt ttc 626
   Val Lys Ile Ile Glu Lys Gln Pro Gly His Ile Arg Ser Arg Val Phe
               115                 120                 125
   agg gag gtg gag atg ctg tac cag tgc cag gga cac agg aac gtc cta      674
   Arg Glu Val Glu Met Leu Tyr Gln Cys Gln Gly His Arg Asn Val Leu
           130                 135                 140
   gag ctg att gag ttc ttc gag gag gag gac cgc ttc tac ctg gtg ttt      722
   Glu Leu Ile Glu Phe Phe Glu Glu Glu Asp Arg Phe Tyr Leu Val Phe
       145                 150                 155
   gag aag atg cgg gga ggc tcc atc ctg agc cac atc cac aag cgc cgg      770
   Glu Lys Met Arg Gly Gly Ser Ile Leu Ser His Ile His Lys Arg Arg
   160                 165                 170                 175
   cac ttc aac gag ctg gag gcc agc gtg gtg gtg cag gac gtg gcc agc      818
   His Phe Asn Glu Leu Glu Ala Ser Val Val Val Gln Asp Val Ala Ser
                   180                 185                 190
   gcc ttg gac ttt ctg cat aac aaa ggc atc gcc cac agg gac cta aag      866
   Ala Leu Asp Phe Leu His Asn Lys Gly Ile Ala His Arg Asp Leu Lys
               195                 200                 205
   ccg gaa aac atc ctc tgt gag cac ccc aac cag gtc tcc ccc gtg aag      914
   Pro Glu Asn Ile Leu Cys Glu His Pro Asn Gln Val Ser Pro Val Lys
           210                 215                 220
   atc tgt gac ttc gac ctg ggc agc ggc atc aaa ctc aac ggg gac tgc      962
   Ile Cys Asp Phe Asp Leu Gly Ser Gly Ile Lys Leu Asn Gly Asp Cys
       225                 230                 235
   tcc cct atc tcc acc ccg gag ctg ctc act ccg tgc ggc tcg gcg gag     1010
   Ser Pro Ile Ser Thr Pro Glu Leu Leu Thr Pro Cys Gly Ser Ala Glu
   240                 245                 250                 255
   tac atg gcc ccg gag gta gtg gag gcc ttc agc gag gag gct agc atc     1058
   Tyr Met Ala Pro Glu Val Val Glu Ala Phe Ser Glu Glu Ala Ser Ile
                   260                 265                 270
   tac gac aag cgc tgc gac ctg tgg agc ctg ggc gtc atc ttg tat atc     1106
   Tyr Asp Lys Arg Cys Asp Leu Trp Ser Leu Gly Val Ile Leu Tyr Ile
               275                 280                 285
   cta ctc agc ggc tac ccg ccc ttc gtg ggc cgc tgt ggc agc gac tgc     1154
   Leu Leu Ser Gly Tyr Pro Pro Phe Val Gly Arg Cys Gly Ser Asp Cys
           290                 295                 300
   ggc tgg gac cgc ggc gag gcc tgc cct gcc tgc cag aac atg ctg ttt     1202
   Gly Trp Asp Arg Gly Glu Ala Cys Pro Ala Cys Gln Asn Met Leu Phe
       305                 310                 315
   gag agc atc cag gag ggc aag tac gag ttc ccc gac aag gac tgg gcc     1250
   Glu Ser Ile Gln Glu Gly Lys Tyr Glu Phe Pro Asp Lys Asp Trp Ala
   320                 325                 330                 335
   cac atc tcc tgc gct gcc aaa gac ctc atc tcc aag ctg ctg gtc cgt     1298
   His Ile Ser Cys Ala Ala Lys Asp Leu Ile Ser Lys Leu Leu Val Arg
                   340                 345                 350
   gac gcc aag cag agg ctg agt gcc gcc caa gtc ctg cag cac ccc tgg     1346
   Asp Ala Lys Gln Arg Leu Ser Ala Ala Gln Val Leu Gln His Pro Trp
               355                 360                 365
   gtt cag ggg tgc gcc ccg gag aac acc ttg ccc act ccc atg gtc ctg     1394
   Val Gln Gly Cys Ala Pro Glu Asn Thr Leu Pro Thr Pro Met Val Leu
           370                 375                 380
   cag agg aac agc tgt gcc aaa gac ctc acg tcc ttc gcg gct gag gcc     1442
   Gln Arg Asn Ser Cys Ala Lys Asp Leu Thr Ser Phe Ala Ala Glu Ala
       385                 390                 395
   att gcc atg aac cgg cag ctg gcc cag cac gac gag gac ctg gct gag      1490
   Ile Ala Met Asn Arg Gln Leu Ala Gln His Asp Glu Asp Leu Ala Glu
   400                 405                 410                 415
   gag gag gcc gcg ggg cag ggc cag ccc gtc ctg gtc cga gct acc tca      1538
   Glu Glu Ala Ala Gly Gln Gly Gln Pro Val Leu Val Arg Ala Thr Ser
                   420                 425                 430
   cgc tgc ctg cag ctg tct cca ccc tcc cag tcc aag ctg gcg cag cgg      1586
   Arg Cys Leu Gln Leu Ser Pro Pro Ser Gln Ser Lys Leu Ala Gln Arg
               435                 440                 445
   cgg caa agg gcc agt ctg tcc tcg gcc cca gtg gtc ctg gtg gga gac      1634
   Arg Gln Arg Ala Ser Leu Ser Ser Ala Pro Val Val Leu Val Gly Asp
           450                 455                 460
   cac gcc tga ccctcccatc tcccctctgt acataggtca cccgtccccc              1683
   His Ala
       465
   aatcaaatct aaaggttttt taagctatcg ccagccggtg tccagcgggc tgcccctcct    1743
   ctgcctggat tcccaggcac taagctcagc tgaggggggt gttttataga aggtttttgc    1803
   ttttgggttt tttttttcct gtttccaccc ctccccgtta ttttttcctt tggatggtta    1863
   aaagcattgc aggcacccgg gaaggtgagc agagggtagg tgggtgggct tgtcccctcc    1923
   ccggtccccc gccctgctca cctctactat gaaggtgccc ccaggtcacc tgtgctgccc    1983
   gccatctgcc cacgtggctt gcagtgactc aggagagcag gcccacagcg tttgccatct    2043
   tgcagagctg ggagggggca caggaccctg ccctcgtgtt ccctcccagc ccgcagtatt    2103
   tcagggacag gctcttcccc tctatccctc accctgagag cacccctggt ggcttggttg    2163
   gggaagggag gggctgcctg tctctggagg tgtcaggcag gcaggtggca ggcagctcac    2223
   ccacccaccc catgggatcc cccagccctt cacccgcgcc tgccttgtcc ccatgatagt    2283
   tgacaatcgg ggcttcctgc aaggcccgtc tgtctgtcca ggactcctgg tggccagatt    2343
   cggcctccga ccttgacctt aaactgcagc tgaccccagg ggctcgccgc tgcccctccc    2403
   ctccacacca aggcctgaga cagcaggagc cccgcctggc ccgaagccgt ttccaccgca    2463
   gcaggcagag gggctggaca ggcactgtca gccaatgtgg ggggtcctga agacaccccc    2523
   ttggggcacc cgagtgcccc ttctcagggc tcagtctgac cgtagccacg tcctgcctcg    2583
   cgccgcccct cgggcctgac ctggaagctc cgtcagctcc gtccttgtcc ttagagctga    2643
   gcccagaccc cggggtctgg ccgaatcctc acccccaggg cagtgttttt ggtctgccac    2703
   cttcaggaaa acggctgcgg cctcggcctc ccttcgggca cccaggaatg cgggggtctg    2763
   ctcagtcccc ccaccctcca tgctccaacc cccgggggct gcggagcctg ctgccccctc     2823
   cccgcgggtg gggacgttct atgcaataca gggttccact ttagaagtgc gcgcggctag     2883
   ggtcaccgcc cgcccttccc ggcgcagccc ccgagctcca cagctggggc agcccctctg    2943
   gcttctaaat ccgcggtcgg gattcttcct cctgtttagt tttttagttt ttccttaaaa    3003
   aaaaacaaca catcgatgga ctttgcttcc ctgttcttga agaatacttg aatgtcgggg    3063
   ggcctggggg tgggggcctc ggagaccgtc tgcctggccc tgctgcccct cctgaatctc 3123
   gtatgatggt cacagtccgg tggccgtggg ggtgctctgc cttccctggt ccccactgcc 3183
   catatctgtg gactgcccct tccaaagacc cctggggggg gtggggcatt ccgcccaccc 3243
   ctttccccca tcacttctcg cctgtcagtg attccatgtt tcgtaacggg ggattctctg 3303
   cctttttgta tcaaagaaca agcaaatgga cccccgcccg ctgcaggcgc ccatagccat 3363
   cgggtctcta aagctgagtg gctagcagcg tttgtttgtt tgtttttttt tttttttctg 3423
   aaggtgggac agtcacttcc tcctccctcc ccacccctgt cgcatccacg tgcgacctgg 3483
   aggactggtc agaaccgtta ctgtgaatga gtgaagatcc tggaggaccc tgggccccag 3543
   gccagctccc atcgctgggg gacggtgaac ggccatgtgt taatgttacg atgtttttaa 3603
   aagacaaaaa aaaaaaaaaa acctcaaaag tttttttaaa gtgggggaaa aacatccaag 3663
   cactttaatt ccaatgtacc aggtgaactg acggagctca gaagttttcc tttacaccaa 3723
   ctgtcaatgc cggaattttg tattctgttt tgtaaagatt taataaaagt caaaaaactt 3783
   gcaaaaaaaa aaaaaaaaaa aa 3805

<210> 68
<211> 465
<212> PRT
<213> human

<400> 68

Met Val Gln Lys Lys Pro Ala Glu Leu Gln Gly Phe His Arg Ser Phe
1               5                   10                  15

Lys Gly Gln Asn Pro Phe Glu Leu Ala Phe Ser Leu Asp Gln Pro Asp
            20                  25                  30

His Gly Asp Ser Asp Phe Gly Leu Gln Cys Ser Ala Arg Pro Asp Met
        35                  40                  45

Pro Ala Ser Gln Pro Ile Asp Ile Pro Asp Ala Lys Lys Arg Gly Lys
    50                  55                  60

Lys Lys Lys Arg Gly Arg Ala Thr Asp Ser Phe Ser Gly Arg Phe Glu
65                  70                  75                  80

Asp Val Tyr Gln Leu Gln Glu Asp Val Leu Gly Glu Gly Ala His Ala
                85                  90                  95

Arg Val Gln Thr Cys Ile Asn Leu Ile Thr Ser Gln Glu Tyr Ala Val
            100                 105                 110

Lys Ile Ile Glu Lys Gln Pro Gly His Ile Arg Ser Arg Val Phe Arg
        115                 120                 125

Glu Val Glu Met Leu Tyr Gln Cys Gln Gly His Arg Asn Val Leu Glu

130                 135                 140

Leu Ile Glu Phe Phe Glu Glu Glu Asp Arg Phe Tyr Leu Val Phe Glu
145                 150                 155                 160

Lys Met Arg Gly Gly Ser Ile Leu Ser His Ile His Lys Arg Arg His
                165                 170                 175

Phe Asn Glu Leu Glu Ala Ser Val Val Val Gln Asp Val Ala Ser Ala
            180                 185                 190

Leu Asp Phe Leu His Asn Lys Gly Ile Ala His Arg Asp Leu Lys Pro
        195                 200                 205

Glu Asn Ile Leu Cys Glu His Pro Asn Gln Val Ser Pro Val Lys Ile
    210                 215                 220

Cys Asp Phe Asp Leu Gly Ser Gly Ile Lys Leu Asn Gly Asp Cys Ser
225                 230                 235                 240

Pro Ile Ser Thr Pro Glu Leu Leu Thr Pro Cys Gly Ser Ala Glu Tyr
                245                 250                 255

Met Ala Pro Glu Val Val Glu Ala Phe Ser Glu Glu Ala Ser Ile Tyr
            260                 265                 270

Asp Lys Arg Cys Asp Leu Trp Ser Leu Gly Val Ile Leu Tyr Ile Leu
        275                 280                 285

Leu Ser Gly Tyr Pro Pro Phe Val Gly Arg Cys Gly Ser Asp Cys Gly
    290                 295                 300

Trp Asp Arg Gly Glu Ala Cys Pro Ala Cys Gln Asn Met Leu Phe Glu
305                 310                 315                 320

Ser Ile Gln Glu Gly Lys Tyr Glu Phe Pro Asp Lys Asp Trp Ala His
                325                 330                 335

Ile Ser Cys Ala Ala Lys Asp Leu Ile Ser Lys Leu Leu Val Arg Asp
            340                 345                 350

Ala Lys Gln Arg Leu Ser Ala Ala Gln Val Leu Gln His Pro Trp Val
        355                 360                 365

Gln Gly Cys Ala Pro Glu Asn Thr Leu Pro Thr Pro Met Val Leu Gln
    370                 375                 380

Arg Asn Ser Cys Ala Lys Asp Leu Thr Ser Phe Ala Ala Glu Ala Ile
385                 390                 395                 400

Ala Met Asn Arg Gln Leu Ala Gln His Asp Glu Asp Leu Ala Glu Glu

405                 410                 415

Glu Ala Ala Gly Gln Gly Gln Pro Val Leu Val Arg Ala Thr Ser Arg
            420                 425                 430

Cys Leu Gln Leu Ser Pro Pro Ser Gln Ser Lys Leu Ala Gln Arg Arg
        435                 440                 445

Gln Arg Ala Ser Leu Ser Ser Ala Pro Val Val Leu Val Gly Asp His
    450                 455                 460

Ala
465

<210>  69
<211>  2012
<212>  DNA
<213>  human

<220>
<221>  CDS
<222>  (131)..(1234)

<400>  69
agggggcagg aacagcaggc ctggccagcc caaaggactc tctatccagg atgtaaatga      60

gcacactgct ggcccatgcg cctcggggct gtagagggca gcctcagagg cactgggcat     120

tcctggcacc atg gat gac gct gct gtc ctc aag cga cga ggc tac ctc        169
           Met Asp Asp Ala Ala Val Leu Lys Arg Arg Gly Tyr Leu
           1               5                   10

ctg ggg ata aat tta gga gag ggc tcc tat gca aaa gta aaa tct gct       217
Leu Gly Ile Asn Leu Gly Glu Gly Ser Tyr Ala Lys Val Lys Ser Ala
    15                  20                  25

tac tct gag cgc ctg aag ttc aat gtg gcg atc aag atc atc gac cgc       265
Tyr Ser Glu Arg Leu Lys Phe Asn Val Ala Ile Lys Ile Ile Asp Arg
30                  35                  40                  45

aag aag gcc ccc gca gac ttc ttg gag aaa ttc ctt ccc cgg gaa att       313
Lys Lys Ala Pro Ala Asp Phe Leu Glu Lys Phe Leu Pro Arg Glu Ile
                50                  55                  60

gag att ctg gcc atg tta aac cac tgc tcc atc att aag acc tac gag       361
Glu Ile Leu Ala Met Leu Asn His Cys Ser Ile Ile Lys Thr Tyr Glu
            65                  70                  75

atc ttt gag aca tca cat ggc aag gtc tac atc gtc atg gag ctc gcg       409
Ile Phe Glu Thr Ser His Gly Lys Val Tyr Ile Val Met Glu Leu Ala
        80                  85                  90

gtc cag ggc gac ctc ctc gag tta atc aaa acc cgg gga gcc ctg cat       457
Val Gln Gly Asp Leu Leu Glu Leu Ile Lys Thr Arg Gly Ala Leu His
    95                  100                 105

gag gac gaa gct cgc aag aag ttc cac cag ctt tcc ttg gcc atc aag       505
Glu Asp Glu Ala Arg Lys Lys Phe His Gln Leu Ser Leu Ala Ile Lys
110                 115                 120                 125

tac tgc cac gac ctg gac gtc gtc cac cgg gac ctc aag tgt gac aac       553
Tyr Cys His Asp Leu Asp Val Val His Arg Asp Leu Lys Cys Asp Asn

                130                 135                 140

ctt ctc ctt gac aag gac ttc aac atc aag ctg tcc gac ttc agc ttc 601
Leu Leu Leu Asp Lys Asp Phe Asn Ile Lys Leu Ser Asp Phe Ser Phe
            145                 150                 155

tcc aag cgc tgc ctg cgg gat gac agt ggt cga atg gca tta agc aag 649
Ser Lys Arg Cys Leu Arg Asp Asp Ser Gly Arg Met Ala Leu Ser Lys
        160                 165                 170

acc ttc tgt ggg tca cca gcg tat gcg gcc cca gag gtg ctg cag ggc 697
Thr Phe Cys Gly Ser Pro Ala Tyr Ala Ala Pro Glu Val Leu Gln Gly
    175                 180                 185

att ccc tac cag ccc aag gtg tac gac atc tgg agc cta ggc gtg atc 745
Ile Pro Tyr Gln Pro Lys Val Tyr Asp Ile Trp Ser Leu Gly Val Ile
190                 195                 200                 205

ctc tac atc atg gtc tgc ggc tcc atg ccc tac gac gac tcc aac atc 793
Leu Tyr Ile Met Val Cys Gly Ser Met Pro Tyr Asp Asp Ser Asn Ile
                210                 215                 220

aag aag atg ctg cgt atc cag aag gag cac cgc gtc aac ttc cca cgc 841
Lys Lys Met Leu Arg Ile Gln Lys Glu His Arg Val Asn Phe Pro Arg
            225                 230                 235

tcc aag cac ctg aca ggc gag tgc aag gac ctc atc tac cac atg ctg 889
Ser Lys His Leu Thr Gly Glu Cys Lys Asp Leu Ile Tyr His Met Leu
        240                 245                 250

cag ccc gac gtc aac cgg cgg ctc cac atc gac gag atc ctc agc cac 937
Gln Pro Asp Val Asn Arg Arg Leu His Ile Asp Glu Ile Leu Ser His
    255                 260                 265

tgc tgg atg cag ccc aag gca cgg gga tct ccc tct gtg gcc atc aac 985
Cys Trp Met Gln Pro Lys Ala Arg Gly Ser Pro Ser Val Ala Ile Asn
270                 275                 280                 285

aag gag ggg gag agt tcc cgg gga act gaa ccc ttg tgg acc ccc gaa 1033
Lys Glu Gly Glu Ser Ser Arg Gly Thr Glu Pro Leu Trp Thr Pro Glu
                290                 295                 300

cct ggc tct gac aag aag tct gcc acc aag ctg gag cct gag gga gag 1081
Pro Gly Ser Asp Lys Lys Ser Ala Thr Lys Leu Glu Pro Glu Gly Glu
            305                 310                 315

gca cag ccc cag gca cag cct gag aca aaa ccc gag ggg aca gca atg 1129
Ala Gln Pro Gln Ala Gln Pro Glu Thr Lys Pro Glu Gly Thr Ala Met
        320                 325                 330

caa atg tcc agg cag tcg gag atc ctg ggt ttc ccc agc aag ccg tcg 1177
Gln Met Ser Arg Gln Ser Glu Ile Leu Gly Phe Pro Ser Lys Pro Ser
    335                 340                 345

act atg gag aca gag gaa ggg ccc ccc caa cag cct cca gag acg cgg 1225
Thr Met Glu Thr Glu Glu Gly Pro Pro Gln Gln Pro Pro Glu Thr Arg
350                 355                 360                 365

gca cag tga gcttcttgcg gcccagggaa tgagatggag ctcacggctt 1274
Ala Gln

aaagcccaag ctctgaagaa gtcaagggtg gagccagaga aggaaggcag tcccagatga 1334

gcctctattt tcatcagctt cttctctctc cccttgaact tggtaaccca catggttctc 1394

ccgtggcccc taggtggatg aggccaaagt caaatccaag gctgagacag tcgtgcgact 1454

cctactcccc cagagcgtga cccggagcag gtgctggaca cagagcctgt ctcagcagag 1514
ggtccccact ggccgcaacg gctcagtgac agcaagagca ggaagagcag caggaaggca 1574
ccgctgtcca ccttgggcac catttatcct cctttcatcg tccccggggc agttgcgtga 1634
ccctgctggg aggccagacc aggccagact gagggtcagg ggaccaggct gggttggggg 1694
ttgggtgggg ctggacgggg acgaggcgcg gagaggaggc gggaaagagc ttcggctggg 1754
ccacgggctc gcgggcactc tcggcggcag cctcgggggc tccgactcca gctctgggca 1814
agatccataa acttttaaag aagcctcaaa acatactttt aaagcattaa gctttaaagt 1874
gctagaaatc ttgtgcccca tatgtgcttc tttcagtatt atcctagatc ctcaggagta 1934
gcatggcaac tggagaaaat gggaggaaag acaagaagaa atagaagctg atttaacacc 1994
aaaaaaaaaa aaaaaaaa 2012

<210> 70
<211> 367
<212> PRT
<213> human

<400> 70

Met Asp Asp Ala Ala Val Leu Lys Arg Arg Gly Tyr Leu Leu Gly Ile
1               5                   10                  15

Asn Leu Gly Glu Gly Ser Tyr Ala Lys Val Lys Ser Ala Tyr Ser Glu
            20                  25                  30

Arg Leu Lys Phe Asn Val Ala Ile Lys Ile Ile Asp Arg Lys Lys Ala
        35                  40                  45

Pro Ala Asp Phe Leu Glu Lys Phe Leu Pro Arg Glu Ile Glu Ile Leu
    50                  55                  60

Ala Met Leu Asn His Cys Ser Ile Ile Lys Thr Tyr Glu Ile Phe Glu
65                  70                  75                  80

Thr Ser His Gly Lys Val Tyr Ile Val Met Glu Leu Ala Val Gln Gly
                85                  90                  95

Asp Leu Leu Glu Leu Ile Lys Thr Arg Gly Ala Leu His Glu Asp Glu
            100                 105                 110

Ala Arg Lys Lys Phe His Gln Leu Ser Leu Ala Ile Lys Tyr Cys His
        115                 120                 125

Asp Leu Asp Val Val His Arg Asp Leu Lys Cys Asp Asn Leu Leu Leu
    130                 135                 140

Asp Lys Asp Phe Asn Ile Lys Leu Ser Asp Phe Ser Phe Ser Lys Arg
145                 150                 155                 160

Cys Leu Arg Asp Asp Ser Gly Arg Met Ala Leu Ser Lys Thr Phe Cys
                165                 170                 175

Gly Ser Pro Ala Tyr Ala Ala Pro Glu Val Leu Gln Gly Ile Pro Tyr
            180                 185                 190

Gln Pro Lys Val Tyr Asp Ile Trp Ser Leu Gly Val Ile Leu Tyr Ile
        195                 200                 205

Met Val Cys Gly Ser Met Pro Tyr Asp Asp Ser Asn Ile Lys Lys Met
    210                 215                 220

Leu Arg Ile Gln Lys Glu His Arg Val Asn Phe Pro Arg Ser Lys His
225                 230                 235                 240

Leu Thr Gly Glu Cys Lys Asp Leu Ile Tyr His Met Leu Gln Pro Asp
                245                 250                 255

Val Asn Arg Arg Leu His Ile Asp Glu Ile Leu Ser His Cys Trp Met
            260                 265                 270

Gln Pro Lys Ala Arg Gly Ser Pro Ser Val Ala Ile Asn Lys Glu Gly
        275                 280                 285

Glu Ser Ser Arg Gly Thr Glu Pro Leu Trp Thr Pro Glu Pro Gly Ser
    290                 295                 300

Asp Lys Lys Ser Ala Thr Lys Leu Glu Pro Glu Gly Glu Ala Gln Pro
305                 310                 315                 320

Gln Ala Gln Pro Glu Thr Lys Pro Glu Gly Thr Ala Met Gln Met Ser
                325                 330                 335

Arg Gln Ser Glu Ile Leu Gly Phe Pro Ser Lys Pro Ser Thr Met Glu
            340                 345                 350

Thr Glu Glu Gly Pro Pro Gln Gln Pro Pro Glu Thr Arg Ala Gln
        355                 360                 365

<210>  71
<211>  2982
<212>  DNA
<213>  human

<220>
<221>  CDS
<222>  (63)..(2252)

<400>  71
gccggcggtg gcgcggcgga gacccggctg gtataacaag aggattgcct gatccagcca      60
ag atg cag agc act tct aat cat ctg tgg ctt tta tct gat att tta        107
ggc caa gga gct act gca aat gtc ttt cgt gga aga cat aag aaa act 155
ggt gat tta ttt gct atc aaa gta ttt aat aac ata agc ttc ctt cgt 203
cca gtg gat gtt caa atg aga gaa ttt gaa gtg ttg aaa aaa ctc aat 251
cac aaa aat att gtc aaa tta ttt gct att gaa gag gag aca aca aca 299
aga cat aaa gta ctt att atg gaa ttt tgt cca tgt ggg agt tta tac 347
act gtt tta gaa gaa cct tct aat gcc tat gga cta cca gaa tct gaa 395
ttc tta att gtt ttg cga gat gtg gtg ggt gga atg aat cat cta cga 443
gag aat ggt ata gtg cac cgt gat atc aag cca gga aat atc atg cgt 491
gtt ata ggg gaa gat gga cag tct gtg tac aaa ctc aca gat ttt ggt 539
gca gct aga gaa tta gaa gat gat gag cag ttt gtt tct ctg tat ggc 587
aca gaa gaa tat ttg cac cct gat atg tat gag aga gca gtg cta aga 635
aaa gat cat cag aag aaa tat gga gca aca gtt gat ctt tgg agc att 683
ggg gta aca ttt tac cat gca gct act gga tca ctg cca ttt aga ccc 731
ttt gaa ggg cct cgt agg aat aaa gaa gtg atg tat aaa ata att aca 779
gga aag cct tct ggt gca ata tct gga gta cag aaa gca gaa aat gga 827
cca att gac tgg agt gga gac atg cct gtt tct tgc agt ctt tct cgg 875
ggt ctt cag gtt cta ctt acc cct gtt ctt gca aac atc ctt gaa gca 923
gat cag gaa aag tgt tgg ggt ttt gac cag ttt ttt gca gaa act agt 971
gat ata ctt cac cga atg gta att cat gtt ttt tcg cta caa caa atg 1019
aca gct cat aag att tat att cat agc tat aat act gct act ata ttt 1067
cat gaa ctg gta tat aaa caa acc aaa att att tct tca aat caa gaa 1115
ctt atc tac gaa ggg cga cgc tta gtc tta gaa cct gga agg ctg gca 1163
caa cat ttc cct aaa act act gag gaa aac cct ata ttt gta gta agc 1211
cgg gaa cct ctg aat acc ata gga tta ata tat gaa aaa att tcc ctc 1259
cct aaa gta cat cca cgt tat gat tta gac ggg gat gct agc atg gct 1307
aag gca ata aca ggg gtt gtg tgt tat gcc tgc aga att gcc agt acc 1355
tta ctg ctt tat cag gaa tta atg cga aag ggg ata cga tgg ctg att 1403
gaa tta att aaa gat gat tac aat gaa act gtt cac aaa aag aca gaa 1451
gtt gtg atc aca ttg gat ttc tgt atc aga aac att gaa aaa act gtg 1499
aaa gta tat gaa aag ttg atg aag atc aac ctg gaa gcg gca gag tta 1547
ggt gaa att tca gac ata cac acc aaa ttg ttg aga ctt tcc agt tct 1595
cag gga aca ata gaa acc agt ctt cag gat atc gac agc aga tta tct 1643
cca ggt gga tca ctg gca gac gca tgg gca cat caa gaa ggc act cat 1691
ccg aaa gac aga aat gta gaa aaa cta caa gtc ctg tta aat tgc atg 1739
aca gag att tac tat cag ttc aaa aaa gac aaa gca gaa cgt aga tta 1787
gct tat aat gaa gaa caa atc cac aaa ttt gat aag caa aaa ctg tat 1835
tac cat gcc aca aaa gct atg acg cac ttt aca gat gaa tgt gtt aaa 1883
aag tat gag gca ttt ttg aat aag tca gaa gaa tgg ata aga aag atg 1931
ctt cat ctt agg aaa cag tta tta tcg ctg act aat cag tgt ttt gat 1979
att gaa gaa gaa gta tca aaa tat caa gaa tat act aat gag tta caa 2027
gaa act ctg cct cag aaa atg ttt aca gct tcc agt gga atc aaa cat 2075
acc atg acc cca att tat cca agt tct aac aca tta gta gaa atg act 2123
ctt ggt atg aag aaa tta aag gaa gag atg gaa ggg gtg gtt aaa gaa 2171
ctt gct gaa aat aac cac att tta gaa agg ttt ggc tct tta acc atg 2219
gat ggt ggc ctt cgc aac gtt gac tgt ctt tag ctttctaata gaagtttaag 2272
aaaagtttcc gtttgcacaa gaaaataacg cttgggcatt aaatgaatgc ctttatagat 2332
agtcacttgt ttctacaatt cagtatttga tgtggtcgtg taaatatgta caatattgta 2392
aatacataaa aaatatacaa atttttggct gctgtgaaga tgtaatttta tcttttaaca 2452
tttataatta tatgaggaaa tttgacctca gtgatcacga gaagaaagcc atgaccgacc 2512
aatatgttga catactgatc ctctactctg agtggggcta aataagttat tttctctgac 2572
cgcctactgg aaatattttt aagtggaacc aaaataggca tccttacaaa tcaggaagac 2632
tgacttgaca cgtttgtaaa tggtagaacg gtggctactg tgagtgggga gcagaaccgc 2692
accactgtta tactgggata acaatttttt tgagaaggat aaagtggcat tattttattt 2752
tacaaggtgc ccagatccca gttatccttg tatccatgta atttcagatg aattattaag 2812
caaacatttt aaagtgaatt cattattaaa aactattcat ttttttcctt tggccataaa 2872
tgtgtaattg tcattaaaat tctaaggtca tttcaactgt tttaagctgt atatttcttt 2932
aattctgctt actatttcat ggaaaaaaat aaatttctca attttaatgt 2982

<210>  72
<211>  729
<212>  PRT
<213>  human

<400>  72

<210>  73
<211>  960
<212>  DNA
<213>  human

<220>
<221>  CDS
<222>  (1)..(960)

<400>  73

atg cca ttc cca aac tgc tca gcc ccc agc act gtg gtg gcc aca gct     48
Met Pro Phe Pro Asn Cys Ser Ala Pro Ser Thr Val Val Ala Thr Ala
1               5                   10                  15

gtg ggt gtc ttg ctg ggg ctg gag tgt ggg ctg ggt ctg ctg ggc aac     96
Val Gly Val Leu Leu Gly Leu Glu Cys Gly Leu Gly Leu Leu Gly Asn
            20                  25                  30

gcg gtg gcg ctg tgg acc ttc ctg ttc cgg gtc agg gtg tgg aag ccg     144
Ala Val Ala Leu Trp Thr Phe Leu Phe Arg Val Arg Val Trp Lys Pro
        35                  40                  45

tac gct gtc tac ctg ctc aac ctg gcc ctg gct gac ctg ctg ttg gct     192
Tyr Ala Val Tyr Leu Leu Asn Leu Ala Leu Ala Asp Leu Leu Leu Ala
    50                  55                  60

gcg tgc ctg cct ttc ctg gcc gcc ttc tac ctg agc ctc cag gct tgg     240
Ala Cys Leu Pro Phe Leu Ala Ala Phe Tyr Leu Ser Leu Gln Ala Trp
65                  70                  75                  80

cat ctg ggc cgt gtg ggc tgc tgg gcc ctg cac ttc ctg ctg gac ctc     288
His Leu Gly Arg Val Gly Cys Trp Ala Leu His Phe Leu Leu Asp Leu
                85                  90                  95

agc cgc agc gtg ggg atg gcc ttc ctg gcc gcc gtg gct ttg gac cgg     336
Ser Arg Ser Val Gly Met Ala Phe Leu Ala Ala Val Ala Leu Asp Arg
            100                 105                 110

tac ctc cgt gtg gtc cac cct cgg ctt aag gtc aac ctg ctg tct cct     384
Tyr Leu Arg Val Val His Pro Arg Leu Lys Val Asn Leu Leu Ser Pro
        115                 120                 125

cag gcg gcc ctg ggg gtc tcg ggc ctc gtc tgg ctc ctg atg gtc gcc     432
Gln Ala Ala Leu Gly Val Ser Gly Leu Val Trp Leu Leu Met Val Ala
    130                 135                 140

ctc acc tgc ccg ggc ttg ctc atc tct gag gcc gcc cag aac tcc acc     480
Leu Thr Cys Pro Gly Leu Leu Ile Ser Glu Ala Ala Gln Asn Ser Thr
145                 150                 155                 160

agg tgc cac agt ttc tac tcc agg gca gac ggc tcc ttc agc atc atc     528
Arg Cys His Ser Phe Tyr Ser Arg Ala Asp Gly Ser Phe Ser Ile Ile
                165                 170                 175

tgg cag gaa gca ctc tcc tgc ctt cag ttt gtc ctc ccc ttt ggc ctc     576
Trp Gln Glu Ala Leu Ser Cys Leu Gln Phe Val Leu Pro Phe Gly Leu
            180                 185                 190

atc gtg ttc tgc aat gca ggc atc atc agg gct ctc cag aaa aga ctc     624
Ile Val Phe Cys Asn Ala Gly Ile Ile Arg Ala Leu Gln Lys Arg Leu
        195                 200                 205

cgg gag cct gag aaa cag ccc aag ctt cag cgg gcc cag gca ctg gtc     672
Arg Glu Pro Glu Lys Gln Pro Lys Leu Gln Arg Ala Gln Ala Leu Val
    210                 215                 220

acc ttg gtg gtg gtg ctg ttt gct ctg tgc ttt ctg ccc tgc ttc ctg     720
Thr Leu Val Val Val Leu Phe Ala Leu Cys Phe Leu Pro Cys Phe Leu
225                 230                 235                 240

gcc aga gtc ctg atg cac atc ttc cag aat ctg ggg agc tgc agg gcc     768
Ala Arg Val Leu Met His Ile Phe Gln Asn Leu Gly Ser Cys Arg Ala
                245                 250                 255

ctt tgt gca gtg gct cat acc tcg gat gtc acg ggc agc ctc acc tac     816
Leu Cys Ala Val Ala His Thr Ser Asp Val Thr Gly Ser Leu Thr Tyr
            260                 265                 270

ctg cac agt gtg ctc aac ccc gtg gta tac tgc ttc tcc agc ccc acc 864
Leu His Ser Val Leu Asn Pro Val Val Tyr Cys Phe Ser Ser Pro Thr
        275                 280                 285

ttc agg agc tcc tat cgg agg gtc ttc cac acc ctc cga ggc aaa ggg 912
Phe Arg Ser Ser Tyr Arg Arg Val Phe His Thr Leu Arg Gly Lys Gly
    290                 295                 300

cag gca gca gag ccc cca gat ttc aac ccc aga gac tcc tat tcc tga 960
Gln Ala Ala Glu Pro Pro Asp Phe Asn Pro Arg Asp Ser Tyr Ser
305                 310                 315

<210> 74
<211> 319
<212> PRT
<213> human

<400> 74

Met Pro Phe Pro Asn Cys Ser Ala Pro Ser Thr Val Val Ala Thr Ala
1               5                   10                  15

Val Gly Val Leu Leu Gly Leu Glu Cys Gly Leu Gly Leu Leu Gly Asn
            20                  25                  30

Ala Val Ala Leu Trp Thr Phe Leu Phe Arg Val Arg Val Trp Lys Pro
        35                  40                  45

Tyr Ala Val Tyr Leu Leu Asn Leu Ala Leu Ala Asp Leu Leu Leu Ala
    50                  55                  60

Ala Cys Leu Pro Phe Leu Ala Ala Phe Tyr Leu Ser Leu Gln Ala Trp
65                  70                  75                  80

His Leu Gly Arg Val Gly Cys Trp Ala Leu His Phe Leu Leu Asp Leu
                85                  90                  95

Ser Arg Ser Val Gly Met Ala Phe Leu Ala Ala Val Ala Leu Asp Arg
            100                 105                 110

Tyr Leu Arg Val Val His Pro Arg Leu Lys Val Asn Leu Leu Ser Pro
        115                 120                 125

Gln Ala Ala Leu Gly Val Ser Gly Leu Val Trp Leu Leu Met Val Ala
    130                 135                 140

Leu Thr Cys Pro Gly Leu Leu Ile Ser Glu Ala Ala Gln Asn Ser Thr
145                 150                 155                 160

Arg Cys His Ser Phe Tyr Ser Arg Ala Asp Gly Ser Phe Ser Ile Ile
                165                 170                 175

Trp Gln Glu Ala Leu Ser Cys Leu Gln Phe Val Leu Pro Phe Gly Leu
            180                 185                 190

Ile Val Phe Cys Asn Ala Gly Ile Ile Arg Ala Leu Gln Lys Arg Leu
        195                 200                 205

Arg Glu Pro Glu Lys Gln Pro Lys Leu Gln Arg Ala Gln Ala Leu Val
    210                 215                 220

Thr Leu Val Val Val Leu Phe Ala Leu Cys Phe Leu Pro Cys Phe Leu
225                 230                 235                 240

Ala Arg Val Leu Met His Ile Phe Gln Asn Leu Gly Ser Cys Arg Ala
                245                 250                 255

Leu Cys Ala Val Ala His Thr Ser Asp Val Thr Gly Ser Leu Thr Tyr
            260                 265                 270

Leu His Ser Val Leu Asn Pro Val Val Tyr Cys Phe Ser Ser Pro Thr
        275                 280                 285

Phe Arg Ser Ser Tyr Arg Arg Val Phe His Thr Leu Arg Gly Lys Gly
    290                 295                 300

Gln Ala Ala Glu Pro Pro Asp Phe Asn Pro Arg Asp Ser Tyr Ser
305                 310                 315

<210>  75
<211>  2910
<212>  DNA
<213>  human

<220>
<221>  CDS
<222>  (113)..(1300)

<400>  75
cagcctccct ctcccacctc tgtctgcccg ctgcctcttg tctagctgct gtcaggagct      60

gactgcctcc agggctggaa tcctgtgctc cctctgtgcc cagagcccca cg atg tcg     118
                                                          Met Ser
                                                          1

gcc aac gcc aca ctg aag cca ctc tgc ccc atc ctg gag cag atg agc       166
Ala Asn Ala Thr Leu Lys Pro Leu Cys Pro Ile Leu Glu Gln Met Ser
        5                   10                  15

cgt ctc cag agc cac agc aac acc agc atc cgc tac atc gac cac gcg       214
Arg Leu Gln Ser His Ser Asn Thr Ser Ile Arg Tyr Ile Asp His Ala
    20                  25                  30

gcc gtg ctg ctg cac ggg ctg gcc tcg ctg ctg ggc ctg gtg gag aat       262
Ala Val Leu Leu His Gly Leu Ala Ser Leu Leu Gly Leu Val Glu Asn
35                  40                  45                  50

gga gtc atc ctc ttc gtg gtg ggc tgc cgc atg cgc cag acc gtg gtc       310
Gly Val Ile Leu Phe Val Val Gly Cys Arg Met Arg Gln Thr Val Val
                55                  60                  65

acc acc tgg gtg ctg cac ctg gcg ctg tcc gac ctg ttg gcc tct gct       358
Thr Thr Trp Val Leu His Leu Ala Leu Ser Asp Leu Leu Ala Ser Ala
            70                  75                  80

tcc ctg ccc ttc ttc acc tac ttc ttg gcc gtg ggc cac tcg tgg gag 406
Ser Leu Pro Phe Phe Thr Tyr Phe Leu Ala Val Gly His Ser Trp Glu
        85                  90                  95

ctg ggc acc acc ttc tgc aaa ctg cac tcc tcc atc ttc ttt ctc aac 454
Leu Gly Thr Thr Phe Cys Lys Leu His Ser Ser Ile Phe Phe Leu Asn
    100                 105                 110

atg ttc gcc agc ggc ttc ctg ctc agc gcc atc agc ctg gac cgc tgc 502
Met Phe Ala Ser Gly Phe Leu Leu Ser Ala Ile Ser Leu Asp Arg Cys
115                 120                 125                 130

ctg cag gtg gtg cgg ccg gtg tgg gcg cag aac cac cgc acc gtg gcc 550
Leu Gln Val Val Arg Pro Val Trp Ala Gln Asn His Arg Thr Val Ala
                135                 140                 145

gcg gcg cac aaa gtc tgc ctg gtg ctt tgg gca cta gcg gtg ctc aac 598
Ala Ala His Lys Val Cys Leu Val Leu Trp Ala Leu Ala Val Leu Asn
            150                 155                 160

acg gtg ccc tat ttc gtg ttc cgg gac acc atc tcg cgg ctg gac ggg 646
Thr Val Pro Tyr Phe Val Phe Arg Asp Thr Ile Ser Arg Leu Asp Gly
        165                 170                 175

cgc att atg tgc tac tac aat gtg ctg ctc ctg aac ccg ggg cct gac 694
Arg Ile Met Cys Tyr Tyr Asn Val Leu Leu Leu Asn Pro Gly Pro Asp
    180                 185                 190

cgc gat gcc acg tgc aac tcg cgg cag gtg gcc ctg gcc gtc agc aag 742
Arg Asp Ala Thr Cys Asn Ser Arg Gln Val Ala Leu Ala Val Ser Lys
195                 200                 205                 210

ttc ctg ctg gcc ttc ctg gtg ccg ctg gcg atc atc gcc tcg agc cac 790
Phe Leu Leu Ala Phe Leu Val Pro Leu Ala Ile Ile Ala Ser Ser His
                215                 220                 225

gcg gcc gtg agc ctg cgg ttg cag cac cgc ggc cgc cgg cgg cca ggc 838
Ala Ala Val Ser Leu Arg Leu Gln His Arg Gly Arg Arg Arg Pro Gly
            230                 235                 240

cgc ttc gtg cgc ctg gtg gcg gcc gtc gtg gcc gcc ttc gcg ctc tgc 886
Arg Phe Val Arg Leu Val Ala Ala Val Val Ala Ala Phe Ala Leu Cys
        245                 250                 255

tgg ggg ccc tac cac gtg ttc agc ctg ctg gag gcg cgg gcg cac gca 934
Trp Gly Pro Tyr His Val Phe Ser Leu Leu Glu Ala Arg Ala His Ala
    260                 265                 270

aac ccg ggg ctg cgg ccg ctc gtg tgg cgc ggg ctg ccc ttc gtc acc 982
Asn Pro Gly Leu Arg Pro Leu Val Trp Arg Gly Leu Pro Phe Val Thr
275                 280                 285                 290

agc ctg gcc ttc ttc aac agc gtg gcc aac ccg gtg ctc tac gtg ctc 1030
Ser Leu Ala Phe Phe Asn Ser Val Ala Asn Pro Val Leu Tyr Val Leu
                295                 300                 305

acc tgc ccc gac atg ctg cgc aag ctg cgg cgc tcg ctg cgc acg gtg 1078
Thr Cys Pro Asp Met Leu Arg Lys Leu Arg Arg Ser Leu Arg Thr Val
            310                 315                 320

ctg gag agc gtg ctg gtg gac gac agc gag ctg ggt ggc gcg gga agc 1126
Leu Glu Ser Val Leu Val Asp Asp Ser Glu Leu Gly Gly Ala Gly Ser
        325                 330                 335

agc cgc cgc cgc cgc acc tcc tcc acc gcc cgc tcg gcc tcc cct tta 1174
Ser Arg Arg Arg Arg Thr Ser Ser Thr Ala Arg Ser Ala Ser Pro Leu
    340                 345                 350

gct ctc tgc agc cgc ccg gag gaa ccg cgg ggc ccc gcg cgt ctc ctc 1222
Ala Leu Cys Ser Arg Pro Glu Glu Pro Arg Gly Pro Ala Arg Leu Leu
355                 360                 365                 370

ggc tgg ctg ctg ggc agc tgc gca gcg tcc ccg cag acg ggc ccc ctg 1270
Gly Trp Leu Leu Gly Ser Cys Ala Ala Ser Pro Gln Thr Gly Pro Leu
                375                 380                 385

aac cgg gcg ctg agc agc acc tcg agt tag aacccggccc acgtagggcg 1320
Asn Arg Ala Leu Ser Ser Thr Ser Ser
            390                 395

gcactcacac gcgaaagtat caccagggtg ccgcggttca attcgatatc cggactcctg 1380

ccgcagtgat caaagtccga ggggcgggac ccaggcacct gcattttaaa gcgccccggg 1440

agactctgaa tctttttcag aaacagtgag ttaaagcagt gcttctcaaa ccttgatgtg 1500

cctgtgaatc acctaggggt cttgttaagt gcagtctgat ccaggaggcc ggggccgggt 1560

actgagagtc tgcacttaac aagctcccag gccgagaagc cagtgcggca ggttcacagg 1620

cgaggcctgg agtaacacaa agtgaaactc ataatagact tcccactcta gggcagtgga 1680

gtcggaaggg cacacggggt gcgtctcccc ggagttcagt tttaccagat gatgggggag 1740

gggggaagga gttttatgtt aaaccatcca tgtatttttg gagaagagag aggaaaggtt 1800

tgagaagcac tgttccagcc tgccctcttc atttagccaa tgcttactgc gctagacgct 1860

tcatcccaca atcttaaggg gcagcttcta ttagccagtc tttacagctg agcacattct 1920

ggctcaggga ggttaagtga cttgcccagt ttcagggcta acgaccacag ggtctgcact 1980

ctaaccctag gcatcacatg ctcaatgact ctctggtgag cgaggacatt ctctgaccta 2040

ctcgagggac ttaagatgct accttgtgac ccagcactgc ccaaagtgct tccaaggcag 2100

aagcagcagg ggatggcgtg gtcaagcact cgggaaacct ggggctaatc aaatccaatg 2160

ggggaaatga ctaaaagtct tcggtcgtta gaagttgaat gggcacagca actctaagac 2220

tacagcacac gtcatttctt agctaagcgg accagcctcc ctgtcggcct ggtgttctgt 2280

gggatccctc tgggcactgg taatcccaag atctgtgcag ccccgcctcc aggccacatg 2340

gggctgggca gctaccattt cccttttgcg gatgggaggg gtaacttgca cctctgacct 2400

atcacttcca ctgcaccccg tctcattcct ccacctgccg tggacttggg gtcagagact 2460

gctgtgtttg agctctgcag cccagggacc gaaaagttgg tgtcaatgaa ttttgcttgg 2520

tggatgaaat gtcagtggaa gaagcagatg agaaactctt gagatcttgg tcctgtgttt 2580

tttctgccac caaaggccag ggtcactgaa ggcctggccc acagcaggtg ctgagcaaag 2640

ggaacagtga ggtgcccagc tagctgcaga gccaccctgt gttgacacct cgcccctgct 2700

ccctcccatc ccttccccct ttactcatag cacttccccc attggacacg tggtgcattt 2760

tgcttgttta ttatgttttc tctccatcag aatgaaagct cctcgagggc agggactttg 2820

gtctattgtc tgtatttgcc ggtgcctagg attgtgcctg tatgcaacag gcactcaata 2880

aatatttttg ctgtagactg gacaggcatg 2910

<210>  76
<211>  395
<212>  PRT
<213>  human

<400>  76

Met Ser Ala Asn Ala Thr Leu Lys Pro Leu Cys Pro Ile Leu Glu Gln
1               5                   10                  15

Met Ser Arg Leu Gln Ser His Ser Asn Thr Ser Ile Arg Tyr Ile Asp
            20                  25                  30

His Ala Ala Val Leu Leu His Gly Leu Ala Ser Leu Leu Gly Leu Val
        35                  40                  45

Glu Asn Gly Val Ile Leu Phe Val Val Gly Cys Arg Met Arg Gln Thr
    50                  55                  60

Val Val Thr Thr Trp Val Leu His Leu Ala Leu Ser Asp Leu Leu Ala
65                  70                  75                  80

Ser Ala Ser Leu Pro Phe Phe Thr Tyr Phe Leu Ala Val Gly His Ser
                85                  90                  95

Trp Glu Leu Gly Thr Thr Phe Cys Lys Leu His Ser Ser Ile Phe Phe
            100                 105                 110

Leu Asn Met Phe Ala Ser Gly Phe Leu Leu Ser Ala Ile Ser Leu Asp
        115                 120                 125

Arg Cys Leu Gln Val Val Arg Pro Val Trp Ala Gln Asn His Arg Thr
    130                 135                 140

Val Ala Ala Ala His Lys Val Cys Leu Val Leu Trp Ala Leu Ala Val
145                 150                 155                 160

Leu Asn Thr Val Pro Tyr Phe Val Phe Arg Asp Thr Ile Ser Arg Leu
                165                 170                 175

Asp Gly Arg Ile Met Cys Tyr Tyr Asn Val Leu Leu Leu Asn Pro Gly
            180                 185                 190

Pro Asp Arg Asp Ala Thr Cys Asn Ser Arg Gln Val Ala Leu Ala Val
        195                 200                 205

Ser Lys Phe Leu Leu Ala Phe Leu Val Pro Leu Ala Ile Ile Ala Ser
    210                 215                 220

Ser His Ala Ala Val Ser Leu Arg Leu Gln His Arg Gly Arg Arg Arg
225                 230                 235                 240

Pro Gly Arg Phe Val Arg Leu Val Ala Ala Val Val Ala Ala Phe Ala

                245                 250                 255

Leu Cys Trp Gly Pro Tyr His Val Phe Ser Leu Leu Glu Ala Arg Ala
            260                 265                 270

His Ala Asn Pro Gly Leu Arg Pro Leu Val Trp Arg Gly Leu Pro Phe
        275                 280                 285

Val Thr Ser Leu Ala Phe Phe Asn Ser Val Ala Asn Pro Val Leu Tyr
    290                 295                 300

Val Leu Thr Cys Pro Asp Met Leu Arg Lys Leu Arg Arg Ser Leu Arg
305                 310                 315                 320

Thr Val Leu Glu Ser Val Leu Val Asp Asp Ser Glu Leu Gly Gly Ala
                325                 330                 335

Gly Ser Ser Arg Arg Arg Arg Thr Ser Ser Thr Ala Arg Ser Ala Ser
            340                 345                 350

Pro Leu Ala Leu Cys Ser Arg Pro Glu Glu Pro Arg Gly Pro Ala Arg
        355                 360                 365

Leu Leu Gly Trp Leu Leu Gly Ser Cys Ala Ala Ser Pro Gln Thr Gly
    370                 375                 380

Pro Leu Asn Arg Ala Leu Ser Ser Thr Ser Ser
385                 390                 395

<210> 77
<211> 5755
<212> DNA
<213> human

<220>
<221> CDS
<222> (460)..(3111)

<400> 77
   gagtcgcgca cgcgcgcccg ggactgcctg cccctctctg tgacttgcct gtgtgtgtgc 60
   gtgtgtgtat gtgtgtgtgt gtgtgtgtgt gcgcgcgcgc gtgagtgaga gaggagagag 120
   ggagaagaga gcgcgagaga gggtgagtgt gtgtgagtgc atgggagggt gctgaatatt 180
   ccgagacact gggaccacag cggcagctcc gctgaaaact gcattcagcc agtcctccgg 240
   acttctggag cggggacagg gcgcagggca tcagcagcca ccagcaggac ctgggaaata 300
   gggattcttc tgcctccact tcaggtttta gcagcttggt gctaaattgc tgtctcaaaa 360
   tgcagaggat ctaatttgca gaggaaaaca gccaaagaag gaagaggagg aaaaggaaaa 420
   aaaaaggggt atattgtgga tgctctactt ttcttggaa atg caa aag att atg 474
   Met Gln Lys Ile Met
   1 5
   cat att tct gtc ctc ctt tct cct gtt tta tgg gga ctg att ttt ggt 522
   His Ile Ser Val Leu Leu Ser Pro Val Leu Trp Gly Leu Ile Phe Gly
   10 15 20
   gtc tct tct aac agc ata cag ata ggg ggg cta ttt cct agg ggc gcc 570
   Val Ser Ser Asn Ser Ile Gln Ile Gly Gly Leu Phe Pro Arg Gly Ala
   25 30 35
   gat caa gaa tac agt gca ttt cga gta ggg atg gtt cag ttt tcc act 618
   Asp Gln Glu Tyr Ser Ala Phe Arg Val Gly Met Val Gln Phe Ser Thr
   40 45 50
   tcg gag ttc aga ctg aca ccc cac atc gac aat ttg gag gtg gca aac 666
   Ser Glu Phe Arg Leu Thr Pro His Ile Asp Asn Leu Glu Val Ala Asn
   55 60 65
   agc ttc gca gtc act aat gct ttc tgc tcc cag ttt tcg aga gga gtc 714
   Ser Phe Ala Val Thr Asn Ala Phe Cys Ser Gln Phe Ser Arg Gly Val
   70 75 80 85
   tat gct att ttt gga ttt tat gac aag aag tct gta aat acc atc aca 762
   Tyr Ala Ile Phe Gly Phe Tyr Asp Lys Lys Ser Val Asn Thr Ile Thr
   90 95 100
   tca ttt tgc gga aca ctc cac gtc tcc ttc atc act ccc agc ttc cca 810
   Ser Phe Cys Gly Thr Leu His Val Ser Phe Ile Thr Pro Ser Phe Pro
   105 110 115
   aca gat ggc aca cat cca ttt gtc att cag atg aga ccc gac ctc aaa 858
   Thr Asp Gly Thr His Pro Phe Val Ile Gln Met Arg Pro Asp Leu Lys
   120 125 130
   gga gct ctc ctt agc ttg att gaa tac tat caa tgg gac aag ttt gca 906
   Gly Ala Leu Leu Ser Leu Ile Glu Tyr Tyr Gln Trp Asp Lys Phe Ala
   135 140 145
   tac ctc tat gac agt gac aga ggc tta tca aca ctg caa gct gtg ctg 954
   Tyr Leu Tyr Asp Ser Asp Arg Gly Leu Ser Thr Leu Gln Ala Val Leu
   150 155 160 165
   gat tct gct gct gaa aag aaa tgg caa gtg act gct atc aat gtg gga 1002
   Asp Ser Ala Ala Glu Lys Lys Trp Gln Val Thr Ala Ile Asn Val Gly
   170 175 180
   aac att aac aat gac aag aaa gat gag atg tac cga tca ctt ttt caa 1050
   Asn Ile Asn Asn Asp Lys Lys Asp Glu Met Tyr Arg Ser Leu Phe Gln
   185 190 195
   gat ctg gag tta aaa aag gaa cgg cgt gta att ctg gac tgt gaa agg 1098
   Asp Leu Glu Leu Lys Lys Glu Arg Arg Val Ile Leu Asp Cys Glu Arg
   200 205 210
   gat aaa gta aac gac att gta gac cag gtt att acc att gga aaa cat 1146
   Asp Lys Val Asn Asp Ile Val Asp Gln Val Ile Thr Ile Gly Lys His
   215 220 225
   gtt aaa ggg tac cac tac atc att gca aat ctg gga ttt act gat gga 1194
   Val Lys Gly Tyr His Tyr Ile Ile Ala Asn Leu Gly Phe Thr Asp Gly
   230 235 240 245
   gac cta tta aaa atc cag ttt gga ggt gca aat gtc tct gga ttt cag 1242
   Asp Leu Leu Lys Ile Gln Phe Gly Gly Ala Asn Val Ser Gly Phe Gln
   250 255 260
   ata gtg gac tat gat gat tcg ttg gta tct aaa ttt ata gaa aga tgg 1290
   Ile Val Asp Tyr Asp Asp Ser Leu Val Ser Lys Phe Ile Glu Arg Trp
   265 270 275
   tca aca ctg gaa gaa aaa gaa tac cct gga gct cac aca aca aca att 1338
   Ser Thr Leu Glu Glu Lys Glu Tyr Pro Gly Ala His Thr Thr Thr Ile
   280 285 290
   aag tat act tct gct ctg acc tat gat gcc gtt caa gtg atg act gaa 1386
   Lys Tyr Thr Ser Ala Leu Thr Tyr Asp Ala Val Gln Val Met Thr Glu
   295 300 305
   gcc ttc cgc aac cta agg aag caa aga att gaa atc tcc cga agg ggg 1434
   Ala Phe Arg Asn Leu Arg Lys Gln Arg Ile Glu Ile Ser Arg Arg Gly
   310 315 320 325
   aat gca gga gac tgt ctg gca aac cca gca gtg ccc tgg gga caa ggt 1482
   Asn Ala Gly Asp Cys Leu Ala Asn Pro Ala Val Pro Trp Gly Gln Gly
   330 335 340
   gta gaa ata gaa agg gcc ctc aaa cag gtt cag gtt gaa ggt ctc tca 1530
   Val Glu Ile Glu Arg Ala Leu Lys Gln Val Gln Val Glu Gly Leu Ser
   345 350 355
   gga aat ata aag ttt gac cag aat gga aaa aga ata aac tat aca att 1578
   Gly Asn Ile Lys Phe Asp Gln Asn Gly Lys Arg Ile Asn Tyr Thr Ile
   360 365 370
   aac atc atg gag ctc aaa act aat ggg ccc cgg aag att ggc tac tgg 1626
   Asn Ile Met Glu Leu Lys Thr Asn Gly Pro Arg Lys Ile Gly Tyr Trp
   375 380 385
   agt gaa gtg gac aaa atg gtt gtt acc ctt act gag ctc cct tct gga 1674
   Ser Glu Val Asp Lys Met Val Val Thr Leu Thr Glu Leu Pro Ser Gly
   390 395 400 405
   aat gac acc tct ggg ctt gag aat aag act gtt gtt gtc acc aca att 1722
   Asn Asp Thr Ser Gly Leu Glu Asn Lys Thr Val Val Val Thr Thr Ile
   410 415 420
   ttg gaa tct ccg tat gtt atg atg aag aaa aat cat gaa atg ctt gaa 1770
   Leu Glu Ser Pro Tyr Val Met Met Lys Lys Asn His Glu Met Leu Glu
   425 430 435
   ggc aat gag cgc tat gag ggc tac tgt gtt gac ctg gct gca gaa atc 1818
   Gly Asn Glu Arg Tyr Glu Gly Tyr Cys Val Asp Leu Ala Ala Glu Ile
   440 445 450
   gcc aaa cat tgt ggg ttc aag tac aag ttg aca att gtt ggt gat ggc 1866
   Ala Lys His Cys Gly Phe Lys Tyr Lys Leu Thr Ile Val Gly Asp Gly
   455 460 465
   aag tat ggg gcc agg gat gca gac acg aaa att tgg aat ggg atg gtt 1914
   Lys Tyr Gly Ala Arg Asp Ala Asp Thr Lys Ile Trp Asn Gly Met Val
   470 475 480 485
   gga gaa ctt gta tat ggg aaa gct gat att gca att gct cca tta act 1962
   Gly Glu Leu Val Tyr Gly Lys Ala Asp Ile Ala Ile Ala Pro Leu Thr
   490 495 500
   att acc ctt gtg aga gaa gag gtg att gac ttc tca aag ccc ttc atg 2010
   Ile Thr Leu Val Arg Glu Glu Val Ile Asp Phe Ser Lys Pro Phe Met
   505 510 515
   agc ctc ggg ata tct atc atg atc aag aag cct cag aag tcc aaa cca 2058
   Ser Leu Gly Ile Ser Ile Met Ile Lys Lys Pro Gln Lys Ser Lys Pro
   520 525 530
   gga gtg ttt tcc ttt ctt gat cct tta gcc tat gag atc tgg atg tgc 2106
   Gly Val Phe Ser Phe Leu Asp Pro Leu Ala Tyr Glu Ile Trp Met Cys
   535 540 545
   att gtt ttt gcc tac att ggg gtc agt gta gtt tta ttc ctg gtc agc 2154
   Ile Val Phe Ala Tyr Ile Gly Val Ser Val Val Leu Phe Leu Val Ser
   550 555 560 565
   aga ttt agc ccc tac gag tgg cac act gag gag ttt gaa gat gga aga 2202
   Arg Phe Ser Pro Tyr Glu Trp His Thr Glu Glu Phe Glu Asp Gly Arg
   570 575 580
   gaa aca caa agt agt gaa tca act aat gaa ttt ggg att ttt aat agt 2250
   Glu Thr Gln Ser Ser Glu Ser Thr Asn Glu Phe Gly Ile Phe Asn Ser
   585 590 595
   ctc tgg ttt tcc ttg ggt gcc ttt atg cgg caa gga tgc gat att tcg 2298
   Leu Trp Phe Ser Leu Gly Ala Phe Met Arg Gln Gly Cys Asp Ile Ser
   600 605 610
   cca aga tcc ctc tct ggg cgc att gtt gga ggt gtg tgg tgg ttc ttt 2346
   Pro Arg Ser Leu Ser Gly Arg Ile Val Gly Gly Val Trp Trp Phe Phe
   615 620 625
   acc ctg atc ata atc tcc tcc tac acg gct aac tta gct gcc ttc ctg 2394
   Thr Leu Ile Ile Ile Ser Ser Tyr Thr Ala Asn Leu Ala Ala Phe Leu
   630 635 640 645
   act gta gag agg atg gtg tct ccc atc gaa agt gct gag gat ctt tct 2442
   Thr Val Glu Arg Met Val Ser Pro Ile Glu Ser Ala Glu Asp Leu Ser
   650 655 660
   aag caa aca gaa att gct tat gga aca tta gac tct ggc tcc act aaa 2490
   Lys Gln Thr Glu Ile Ala Tyr Gly Thr Leu Asp Ser Gly Ser Thr Lys
   665 670 675
   gag ttt ttc agg aga tct aaa att gca gtg ttt gat aaa atg tgg acc 2538
   Glu Phe Phe Arg Arg Ser Lys Ile Ala Val Phe Asp Lys Met Trp Thr
   680 685 690
   tac atg cgg agt gcg gag ccc tct gtg ttt gtg agg act acg gcc gaa 2586
   Tyr Met Arg Ser Ala Glu Pro Ser Val Phe Val Arg Thr Thr Ala Glu
   695 700 705
   ggg gtg gct aga gtg cgg aag tcc aaa ggg aaa tat gcc tac ttg ttg 2634
   Gly Val Ala Arg Val Arg Lys Ser Lys Gly Lys Tyr Ala Tyr Leu Leu
   710 715 720 725
   gag tcc acg atg aac gag tac att gag caa agg aag cct tgc gac acc 2682
   Glu Ser Thr Met Asn Glu Tyr Ile Glu Gln Arg Lys Pro Cys Asp Thr
   730 735 740
   atg aaa gtt ggt gga aac ctg gat tcc aaa ggc tat ggc atc gca aca 2730
   Met Lys Val Gly Gly Asn Leu Asp Ser Lys Gly Tyr Gly Ile Ala Thr
   745 750 755
   cct aaa gga tcc tca tta aga acc cca gta aat ctt gca gta ttg aaa 2778
   Pro Lys Gly Ser Ser Leu Arg Thr Pro Val Asn Leu Ala Val Leu Lys
   760 765 770
   ctc agt gag caa ggc gtc tta gac aag ctg aaa aac aaa tgg tgg tac 2826
   Leu Ser Glu Gln Gly Val Leu Asp Lys Leu Lys Asn Lys Trp Trp Tyr
   775 780 785
   gat aaa ggt gaa tgt gga gcc aag gac tct gga agt aag gaa aag acc 2874
   Asp Lys Gly Glu Cys Gly Ala Lys Asp Ser Gly Ser Lys Glu Lys Thr
   790 795 800 805
   agt gcc ctc agt ctg agc aac gtt gct gga gta ttc tac atc ctt gtc 2922
   Ser Ala Leu Ser Leu Ser Asn Val Ala Gly Val Phe Tyr Ile Leu Val
   810 815 820
   ggg ggc ctt ggt ttg gca atg ctg gtg gct ttg att gag ttc tgt tac     2970
   Gly Gly Leu Gly Leu Ala Met Leu Val Ala Leu Ile Glu Phe Cys Tyr
               825                 830                 835
   aag tca agg gcc gag gcg aaa cga atg aag gtg gca aag aat gca cag     3018
   Lys Ser Arg Ala Glu Ala Lys Arg Met Lys Val Ala Lys Asn Ala Gln
           840                 845                 850
   aat att aac cca tct tcc tcg cag aat tca cag aat ttt gca act tat     3066
   Asn Ile Asn Pro Ser Ser Ser Gln Asn Ser Gln Asn Phe Ala Thr Tyr
       855                 860                 865
   aag gaa ggt tac aac gta tat ggc atc gaa agt gtt aaa att tag         3111
   Lys Glu Gly Tyr Asn Val Tyr Gly Ile Glu Ser Val Lys Ile
   870                 875                 880
   gggatgacct tgaatgatgc catgaggaac aaggcaaggc tgtcaattac aggaagtact   3171
   ggagaaaatg gacgtgttat gactccagaa tttcccaaag cagtgcatgc tgtcccttac   3231
   gtgagtcctg gcatgggaat gaatgtcagt gtgactgatc tctcgtgatt gataagaacc   3291
   ttttgagtgc cttacacaat ggttttcttg tgtgtttatt gtcaaagtgg tgagaggcat   3351
   ccagtatctt gaagactttt ctttcagcca agaattctta aatatgtgga gttcatcttg   3411
   aattgtaagg aatgattaat taaaacacaa catctttttc tactcgagtt acagacaaag   3471
   cgtggtggac atgcacagct aacatggaag tactataatt tacctgaagt ctttgtacag   3531
   acaacaaacc tgtttctgca gccactattg ttagtctctt gattcataat gacttaagca   3591
   cacttgacat caactgcatc aagatgtgac atgttttata aaaaaaggaa aaaaaacatt   3651
   taaaactaaa aaatattttt aggtattttc acaaacaaac tggcttttaa ataaatttgc   3711
   ttccatattg gttgaataag acaaaaacaa ttaaactgag tgggaagtga ataaaaaaag   3771
   gctttaggta tcgattccat atttttcaaa gccaaatatg taaatgctaa ggaaagtaaa   3831
   caaagaggag attccaatct tgtaatttaa tattgttatt aaaactttaa tgtatcctat   3891
   tctttaacat ttggtgttaa tataaaatta cttggcaatg cttgacattt gaaataaaca   3951
   tttttctatt gttttattgc aagtggtcca attaattttg cttagctaca gtttggtcat   4011
   aaatcaagtg agtttaaaga cactaccaag ttgttaggtg cccagagaaa atttctccct   4071
   tttaaaaagg ccaggtgatt tttcaaatgt aatcttgccc ccaaagtaat atctgaatat   4131
   ctttttgaca tgtctaaata tatatatata taaagaaata tttgttaaca caaaagcatt   4191
   tgatctatgt agataaatgc taatagattt aaaaagctaa tattaacaaa taccagaata   4251
   cgtgaagttc catttttaaa gtgtttgagc ttacagaaga gaaacattca ttttaaatga   4311
   agtaaaaaat gccttgaaag taattcttta gatagttgcc cattgattaa attccaaaaa   4371
   ctaaatatgt ttttagcttt aaaattataa aagctgtcat aaactttata tattatgaat   4431
   tttaaaatat gtttgagtct cctgcaatat agtttcatcc cattgacatc aattaaaaat   4491
   aaccctaata tattattttt atatttattc ctcaggtgga atggctattt taatatgccc   4551
   agtgtggata aaatgtcaca tttctgtaac ttttgactaa agagcctata tttatctagt   4611
   taatgaattt aaaggatcta tctttccctt cataaaatac ctcttatttc cattaaagcc   4671
   ccccaagttt aattaattta ggattttgaa tgattattga catccaatag ttatttttaa 4731
   tatttgtatt cttgttattt ctggaagaaa gcctttgtgt agcacttggt attttgcaaa 4791
   gtgcttttaa aacattctta cttaccgtat ttcatagaag ggaaggaaaa atgtaaggtt 4851
   taacagtaag cacttgcatt gaacatggag gcatgtggta tcatgatatt cttcactaaa 4911
   tttagctgtc cctaatcaca gatcctaagg taatataata taattttagt gcatttctcc 4971
   tcatcaggaa tgctggaggt gcattttaag ttttaataat aagtgctaga atgaccaaat 5031
   tgcagactaa ttgtttccat attgtactta aaatgagttt ttaaaagtga aaaagaaatg 5091
   actatataca atcaatgcta tttattgtac ctctgggcct actcttctaa aaattgtagc 5151
   ttatcgattt ttctctgtca agcttgaact aatgtaaata attgaaataa tgtaaagtta 5211
   tattttcatg tttttataga tacaacatga caagaataca taatgtaaga gtatttcaac 5271
   tatggataat gttgattgga taatgcacat ctcagttaca agcagtactc atagtttaat 5331
   atccatgtaa cggtgcatca atatattgct atataaatat gtctgtgtgc atataagtga 5391
   aaagtggtca aacaagagtg atgacagctg tctaaaggtt tttttattca ttttatataa 5451
   aaactgttat ggaaagacca aaatgtttat gaactattct tatgtaaatt tacaattgtc 5511
   ctttactgta cttttttgtt tacagtatag taccttattt tctgctgtgt taagtgggtg 5571
   tcaaactcca agaagacata cactttctat aacttctatt gaagatattg gaatttccaa 5631
   tttttcatgt gtactatgtc agaaaatgct ttcgatttta tttttaaatc taacatcgga 5691
   tggcttttcc ggagtgttgt aaaaacttca atcatacata aaacatgttc ttacaaaagg 5751
   caaa 5755

<210> 78
<211> 883
<212> PRT
<213> human

<400> 78

Met Gln Lys Ile Met His Ile Ser Val Leu Leu Ser Pro Val Leu Trp
1               5                   10                  15

Gly Leu Ile Phe Gly Val Ser Ser Asn Ser Ile Gln Ile Gly Gly Leu
            20                  25                  30

Phe Pro Arg Gly Ala Asp Gln Glu Tyr Ser Ala Phe Arg Val Gly Met
        35                  40                  45

Val Gln Phe Ser Thr Ser Glu Phe Arg Leu Thr Pro His Ile Asp Asn
    50                  55                  60

Leu Glu Val Ala Asn Ser Phe Ala Val Thr Asn Ala Phe Cys Ser Gln
65                  70                  75                  80

Phe Ser Arg Gly Val Tyr Ala Ile Phe Gly Phe Tyr Asp Lys Lys Ser
                85                  90                  95

Val Asn Thr Ile Thr Ser Phe Cys Gly Thr Leu His Val Ser Phe Ile
            100                 105                 110

Thr Pro Ser Phe Pro Thr Asp Gly Thr His Pro Phe Val Ile Gln Met
        115                 120                 125

Arg Pro Asp Leu Lys Gly Ala Leu Leu Ser Leu Ile Glu Tyr Tyr Gln
    130                 135                 140

Trp Asp Lys Phe Ala Tyr Leu Tyr Asp Ser Asp Arg Gly Leu Ser Thr
145                 150                 155                 160

Leu Gln Ala Val Leu Asp Ser Ala Ala Glu Lys Lys Trp Gln Val Thr
                165                 170                 175

Ala Ile Asn Val Gly Asn Ile Asn Asn Asp Lys Lys Asp Glu Met Tyr
            180                 185                 190

Arg Ser Leu Phe Gln Asp Leu Glu Leu Lys Lys Glu Arg Arg Val Ile
        195                 200                 205

Leu Asp Cys Glu Arg Asp Lys Val Asn Asp Ile Val Asp Gln Val Ile
    210                 215                 220

Thr Ile Gly Lys His Val Lys Gly Tyr His Tyr Ile Ile Ala Asn Leu
225                 230                 235                 240

Gly Phe Thr Asp Gly Asp Leu Leu Lys Ile Gln Phe Gly Gly Ala Asn
                245                 250                 255

Val Ser Gly Phe Gln Ile Val Asp Tyr Asp Asp Ser Leu Val Ser Lys
            260                 265                 270

Phe Ile Glu Arg Trp Ser Thr Leu Glu Glu Lys Glu Tyr Pro Gly Ala
        275                 280                 285

His Thr Thr Thr Ile Lys Tyr Thr Ser Ala Leu Thr Tyr Asp Ala Val
    290                 295                 300

Gln Val Met Thr Glu Ala Phe Arg Asn Leu Arg Lys Gln Arg Ile Glu
305                 310                 315                 320

Ile Ser Arg Arg Gly Asn Ala Gly Asp Cys Leu Ala Asn Pro Ala Val
                325                 330                 335

Pro Trp Gly Gln Gly Val Glu Ile Glu Arg Ala Leu Lys Gln Val Gln
            340                 345                 350

Val Glu Gly Leu Ser Gly Asn Ile Lys Phe Asp Gln Asn Gly Lys Arg
        355                 360                 365

Ile Asn Tyr Thr Ile Asn Ile Met Glu Leu Lys Thr Asn Gly Pro Arg
    370                 375                 380

Lys Ile Gly Tyr Trp Ser Glu Val Asp Lys Met Val Val Thr Leu Thr
385                 390                 395                 400

Glu Leu Pro Ser Gly Asn Asp Thr Ser Gly Leu Glu Asn Lys Thr Val
                405                 410                 415

Val Val Thr Thr Ile Leu Glu Ser Pro Tyr Val Met Met Lys Lys Asn
            420                 425                 430

His Glu Met Leu Glu Gly Asn Glu Arg Tyr Glu Gly Tyr Cys Val Asp
        435                 440                 445

Leu Ala Ala Glu Ile Ala Lys His Cys Gly Phe Lys Tyr Lys Leu Thr
    450                 455                 460

Ile Val Gly Asp Gly Lys Tyr Gly Ala Arg Asp Ala Asp Thr Lys Ile
465                 470                 475                 480

Trp Asn Gly Met Val Gly Glu Leu Val Tyr Gly Lys Ala Asp Ile Ala
                485                 490                 495

Ile Ala Pro Leu Thr Ile Thr Leu Val Arg Glu Glu Val Ile Asp Phe
            500                 505                 510

Ser Lys Pro Phe Met Ser Leu Gly Ile Ser Ile Met Ile Lys Lys Pro
        515                 520                 525

Gln Lys Ser Lys Pro Gly Val Phe Ser Phe Leu Asp Pro Leu Ala Tyr
    530                 535                 540

Glu Ile Trp Met Cys Ile Val Phe Ala Tyr Ile Gly Val Ser Val Val
545                 550                 555                 560

Leu Phe Leu Val Ser Arg Phe Ser Pro Tyr Glu Trp His Thr Glu Glu
                565                 570                 575

Phe Glu Asp Gly Arg Glu Thr Gln Ser Ser Glu Ser Thr Asn Glu Phe
            580                 585                 590

Gly Ile Phe Asn Ser Leu Trp Phe Ser Leu Gly Ala Phe Met Arg Gln
        595                 600                 605

Gly Cys Asp Ile Ser Pro Arg Ser Leu Ser Gly Arg Ile Val Gly Gly
    610                 615                 620

Val Trp Trp Phe Phe Thr Leu Ile Ile Ile Ser Ser Tyr Thr Ala Asn
625                 630                 635                 640

Leu Ala Ala Phe Leu Thr Val Glu Arg Met Val Ser Pro Ile Glu Ser
                645                 650                 655

Ala Glu Asp Leu Ser Lys Gln Thr Glu Ile Ala Tyr Gly Thr Leu Asp
            660                 665                 670

Ser Gly Ser Thr Lys Glu Phe Phe Arg Arg Ser Lys Ile Ala Val Phe
        675                 680                 685

Asp Lys Met Trp Thr Tyr Met Arg Ser Ala Glu Pro Ser Val Phe Val
    690                 695                 700

Arg Thr Thr Ala Glu Gly Val Ala Arg Val Arg Lys Ser Lys Gly Lys
705                 710                 715                 720

Tyr Ala Tyr Leu Leu Glu Ser Thr Met Asn Glu Tyr Ile Glu Gln Arg
                725                 730                 735

Lys Pro Cys Asp Thr Met Lys Val Gly Gly Asn Leu Asp Ser Lys Gly
            740                 745                 750

Tyr Gly Ile Ala Thr Pro Lys Gly Ser Ser Leu Arg Thr Pro Val Asn
        755                 760                 765

Leu Ala Val Leu Lys Leu Ser Glu Gln Gly Val Leu Asp Lys Leu Lys
    770                 775                 780

Asn Lys Trp Trp Tyr Asp Lys Gly Glu Cys Gly Ala Lys Asp Ser Gly
785                 790                 795                 800

Ser Lys Glu Lys Thr Ser Ala Leu Ser Leu Ser Asn Val Ala Gly Val
                805                 810                 815

Phe Tyr Ile Leu Val Gly Gly Leu Gly Leu Ala Met Leu Val Ala Leu
            820                 825                 830

Ile Glu Phe Cys Tyr Lys Ser Arg Ala Glu Ala Lys Arg Met Lys Val
        835                 840                 845

Ala Lys Asn Ala Gln Asn Ile Asn Pro Ser Ser Ser Gln Asn Ser Gln
    850                 855                 860

Asn Phe Ala Thr Tyr Lys Glu Gly Tyr Asn Val Tyr Gly Ile Glu Ser
865                 870                 875                 880

Val Lys Ile

<210>  79
<211>  5755

<212>  DNA
<213>  human

<220>
<221>  CDS
<222>  (460)..(3111)

<400>  79
gagtcgcgca cgcgcgcccg ggactgcctg cccctctctg tgacttgcct gtgtgtgtgc      60

gtgtgtgtat gtgtgtgtgt gtgtgtgtgt gcgcgcgcgc gtgagtgaga gaggagagag     120

ggagaagaga gcgcgagaga gggtgagtgt gtgtgagtgc atgggagggt gctgaatatt     180

ccgagacact gggaccacag cggcagctcc gctgaaaact gcattcagcc agtcctccgg     240

acttctggag cggggacagg gcgcagggca tcagcagcca ccagcaggac ctgggaaata     300

gggattcttc tgcctccact tcaggtttta gcagcttggt gctaaattgc tgtctcaaaa     360

tgcagaggat ctaatttgca gaggaaaaca gccaaagaag gaagaggagg aaaaggaaaa     420

aaaaaggggt atattgtgga tgctctactt ttcttggaa atg caa aag att atg       474
                                           Met Gln Lys Ile Met
                                           1               5

cat att tct gtc ctc ctt tct cct gtt tta tgg gga ctg att ttt ggt       522
His Ile Ser Val Leu Leu Ser Pro Val Leu Trp Gly Leu Ile Phe Gly
                10                  15                  20

gtc tct tct aac agc ata cag ata ggg ggg cta ttt cct agg ggc gcc       570
Val Ser Ser Asn Ser Ile Gln Ile Gly Gly Leu Phe Pro Arg Gly Ala
            25                  30                  35

gat caa gaa tac agt gca ttt cga gta ggg atg gtt cag ttt tcc act       618
Asp Gln Glu Tyr Ser Ala Phe Arg Val Gly Met Val Gln Phe Ser Thr
        40                  45                  50

tcg gag ttc aga ctg aca ccc cac atc gac aat ttg gag gtg gca aac       666
Ser Glu Phe Arg Leu Thr Pro His Ile Asp Asn Leu Glu Val Ala Asn
    55                  60                  65

agc ttc gca gtc act aat gct ttc tgc tcc cag ttt tcg aga gga gtc       714
Ser Phe Ala Val Thr Asn Ala Phe Cys Ser Gln Phe Ser Arg Gly Val
70                  75                  80                  85

tat gct att ttt gga ttt tat gac aag aag tct gta aat acc atc aca       762
Tyr Ala Ile Phe Gly Phe Tyr Asp Lys Lys Ser Val Asn Thr Ile Thr
                90                  95                  100

tca ttt tgc gga aca ctc cac gtc tcc ttc atc act ccc agc ttc cca       810
Ser Phe Cys Gly Thr Leu His Val Ser Phe Ile Thr Pro Ser Phe Pro
            105                 110                 115

aca gat ggc aca cat cca ttt gtc att cag atg aga ccc gac ctc aaa       858
Thr Asp Gly Thr His Pro Phe Val Ile Gln Met Arg Pro Asp Leu Lys
        120                 125                 130

gga gct ctc ctt agc ttg att gaa tac tat caa tgg gac aag ttt gca       906
Gly Ala Leu Leu Ser Leu Ile Glu Tyr Tyr Gln Trp Asp Lys Phe Ala
    135                 140                 145

tac ctc tat gac agt gac aga ggc tta tca aca ctg caa gct gtg ctg       954
Tyr Leu Tyr Asp Ser Asp Arg Gly Leu Ser Thr Leu Gln Ala Val Leu
150                 155                 160                 165

gat tct gct gct gaa aag aaa tgg caa gtg act gct atc aat gtg gga      1002

Asp Ser Ala Ala Glu Lys Lys Trp Gln Val Thr Ala Ile Asn Val Gly
                170                 175                 180

aac att aac aat gac aag aaa gat gag atg tac cga tca ctt ttt caa      1050
Asn Ile Asn Asn Asp Lys Lys Asp Glu Met Tyr Arg Ser Leu Phe Gln
            185                 190                 195

gat ctg gag tta aaa aag gaa cgg cgt gta att ctg gac tgt gaa agg      1098
Asp Leu Glu Leu Lys Lys Glu Arg Arg Val Ile Leu Asp Cys Glu Arg
        200                 205                 210

gat aaa gta aac gac att gta gac cag gtt att acc att gga aaa cat      1146
Asp Lys Val Asn Asp Ile Val Asp Gln Val Ile Thr Ile Gly Lys His
    215                 220                 225

gtt aaa ggg tac cac tac atc att gca aat ctg gga ttt act gat gga      1194
Val Lys Gly Tyr His Tyr Ile Ile Ala Asn Leu Gly Phe Thr Asp Gly
230                 235                 240                 245

gac cta tta aaa atc cag ttt gga ggt gca aat gtc tct gga ttt cag      1242
Asp Leu Leu Lys Ile Gln Phe Gly Gly Ala Asn Val Ser Gly Phe Gln
                250                 255                 260

ata gtg gac tat gat gat tcg ttg gta tct aaa ttt ata gaa aga tgg      1290
Ile Val Asp Tyr Asp Asp Ser Leu Val Ser Lys Phe Ile Glu Arg Trp
            265                 270                 275

tca aca ctg gaa gaa aaa gaa tac cct gga gct cac aca aca aca att      1338
Ser Thr Leu Glu Glu Lys Glu Tyr Pro Gly Ala His Thr Thr Thr Ile
        280                 285                 290

aag tat act tct gct ctg acc tat gat gcc gtt caa gtg atg act gaa      1386
Lys Tyr Thr Ser Ala Leu Thr Tyr Asp Ala Val Gln Val Met Thr Glu
    295                 300                 305

gcc ttc cgc aac cta agg aag caa aga att gaa atc tcc cga agg ggg      1434
Ala Phe Arg Asn Leu Arg Lys Gln Arg Ile Glu Ile Ser Arg Arg Gly
310                 315                 320                 325

aat gca gga gac tgt ctg gca aac cca gca gtg ccc tgg gga caa ggt      1482
Asn Ala Gly Asp Cys Leu Ala Asn Pro Ala Val Pro Trp Gly Gln Gly
                330                 335                 340

gta gaa ata gaa agg gcc ctc aaa cag gtt cag gtt gaa ggt ctc tca      1530
Val Glu Ile Glu Arg Ala Leu Lys Gln Val Gln Val Glu Gly Leu Ser
            345                 350                 355

gga aat ata aag ttt gac cag aat gga aaa aga ata aac tat aca att      1578
Gly Asn Ile Lys Phe Asp Gln Asn Gly Lys Arg Ile Asn Tyr Thr Ile
        360                 365                 370

aac atc atg gag ctc aaa act aat ggg ccc cgg aag att ggc tac tgg      1626
Asn Ile Met Glu Leu Lys Thr Asn Gly Pro Arg Lys Ile Gly Tyr Trp
    375                 380                 385

agt gaa gtg gac aaa atg gtt gtt acc ctt act gag ctc cct tct gga      1674
Ser Glu Val Asp Lys Met Val Val Thr Leu Thr Glu Leu Pro Ser Gly
390                 395                 400                 405

aat gac acc tct ggg ctt gag aat aag act gtt gtt gtc acc aca att      1722
Asn Asp Thr Ser Gly Leu Glu Asn Lys Thr Val Val Val Thr Thr Ile
                410                 415                 420

ttg gaa tct ccg tat gtt atg atg aag aaa aat cat gaa atg ctt gaa      1770
Leu Glu Ser Pro Tyr Val Met Met Lys Lys Asn His Glu Met Leu Glu
            425                 430                 435

ggc aat gag cgc tat gag ggc tac tgt gtt gac ctg gct gca gaa atc      1818

Gly Asn Glu Arg Tyr Glu Gly Tyr Cys Val Asp Leu Ala Ala Glu Ile
        440                     445                     450

gcc aaa cat tgt ggg ttc aag tac aag ttg aca att gtt ggt gat ggc        1866
Ala Lys His Cys Gly Phe Lys Tyr Lys Leu Thr Ile Val Gly Asp Gly
    455                     460                     465

aag tat ggg gcc agg gat gca gac acg aaa att tgg aat ggg atg gtt        1914
Lys Tyr Gly Ala Arg Asp Ala Asp Thr Lys Ile Trp Asn Gly Met Val
470                     475                     480                     485

gga gaa ctt gta tat ggg aaa gct gat att gca att gct cca tta act        1962
Gly Glu Leu Val Tyr Gly Lys Ala Asp Ile Ala Ile Ala Pro Leu Thr
                490                     495                     500

att acc ctt gtg aga gaa gag gtg att gac ttc tca aag ccc ttc atg        2010
Ile Thr Leu Val Arg Glu Glu Val Ile Asp Phe Ser Lys Pro Phe Met
            505                     510                     515

agc ctc ggg ata tct atc atg atc aag aag cct cag aag tcc aaa cca        2058
Ser Leu Gly Ile Ser Ile Met Ile Lys Lys Pro Gln Lys Ser Lys Pro
        520                     525                     530

gga gtg ttt tcc ttt ctt gat cct tta gcc tat gag atc tgg atg tgc        2106
Gly Val Phe Ser Phe Leu Asp Pro Leu Ala Tyr Glu Ile Trp Met Cys
    535                     540                     545

att gtt ttt gcc tac att ggg gtc agt gta gtt tta ttc ctg gtc agc        2154
Ile Val Phe Ala Tyr Ile Gly Val Ser Val Val Leu Phe Leu Val Ser
550                     555                     560                     565

aga ttt agc ccc tac gag tgg cac act gag gag ttt gaa gat gga aga        2202
Arg Phe Ser Pro Tyr Glu Trp His Thr Glu Glu Phe Glu Asp Gly Arg
                570                     575                     580

gaa aca caa agt agt gaa tca act aat gaa ttt ggg att ttt aat agt        2250
Glu Thr Gln Ser Ser Glu Ser Thr Asn Glu Phe Gly Ile Phe Asn Ser
            585                     590                     595

ctc tgg ttt tcc ttg ggt gcc ttt atg cgg caa gga tgc gat att tcg        2298
Leu Trp Phe Ser Leu Gly Ala Phe Met Arg Gln Gly Cys Asp Ile Ser
        600                     605                     610

cca aga tcc ctc tct ggg cgc att gtt gga ggt gtg tgg tgg ttc ttt        2346
Pro Arg Ser Leu Ser Gly Arg Ile Val Gly Gly Val Trp Trp Phe Phe
    615                     620                     625

acc ctg atc ata atc tcc tcc tac acg gct aac tta gct gcc ttc ctg        2394
Thr Leu Ile Ile Ile Ser Ser Tyr Thr Ala Asn Leu Ala Ala Phe Leu
630                     635                     640                     645

act gta gag agg atg gtg tct ccc atc gaa agt gct gag gat ctt tct        2442
Thr Val Glu Arg Met Val Ser Pro Ile Glu Ser Ala Glu Asp Leu Ser
                650                     655                     660

aag caa aca gaa att gct tat gga aca tta gac tct ggc tcc act aaa        2490
Lys Gln Thr Glu Ile Ala Tyr Gly Thr Leu Asp Ser Gly Ser Thr Lys
            665                     670                     675

gag ttt ttc agg aga tct aaa att gca gtg ttt gat aaa atg tgg acc        2538
Glu Phe Phe Arg Arg Ser Lys Ile Ala Val Phe Asp Lys Met Trp Thr
        680                     685                     690

tac atg cgg agt gcg gag ccc tct gtg ttt gtg agg act acg gcc gaa        2586
Tyr Met Arg Ser Ala Glu Pro Ser Val Phe Val Arg Thr Thr Ala Glu
    695                     700                     705

ggg gtg gct aga gtg cgg aag tcc aaa ggg aaa tat gcc tac ttg ttg        2634

Gly Val Ala Arg Val Arg Lys Ser Lys Gly Lys Tyr Ala Tyr Leu Leu
710                 715                 720                 725

gag tcc acg atg aac gag tac att gag caa agg aag cct tgc gac acc     2682
Glu Ser Thr Met Asn Glu Tyr Ile Glu Gln Arg Lys Pro Cys Asp Thr
                730                 735                 740

atg aaa gtt ggt gga aac ctg gat tcc aaa ggc tat ggc atc gca aca     2730
Met Lys Val Gly Gly Asn Leu Asp Ser Lys Gly Tyr Gly Ile Ala Thr
            745                 750                 755

cct aaa gga tcc tca tta aga aat gcg gtt aac ctc gca gta cta aaa     2778
Pro Lys Gly Ser Ser Leu Arg Asn Ala Val Asn Leu Ala Val Leu Lys
        760                 765                 770

ctg aat gaa caa ggc ctg ttg gac aaa ttg aaa aac aaa tgg tgg tac     2826
Leu Asn Glu Gln Gly Leu Leu Asp Lys Leu Lys Asn Lys Trp Trp Tyr
    775                 780                 785

gac aaa gga gag tgc ggc agc ggg gga ggt gat tcc aag gaa aag acc     2874
Asp Lys Gly Glu Cys Gly Ser Gly Gly Gly Asp Ser Lys Glu Lys Thr
790                 795                 800                 805

agt gcc ctc agt ctg agc aac gtt gct gga gta ttc tac atc ctt gtc     2922
Ser Ala Leu Ser Leu Ser Asn Val Ala Gly Val Phe Tyr Ile Leu Val
                810                 815                 820

ggg ggc ctt ggt ttg gca atg ctg gtg gct ttg att gag ttc tgt tac     2970
Gly Gly Leu Gly Leu Ala Met Leu Val Ala Leu Ile Glu Phe Cys Tyr
            825                 830                 835

aag tca agg gcc gag gcg aaa cga atg aag gtg gca aag aat gca cag     3018
Lys Ser Arg Ala Glu Ala Lys Arg Met Lys Val Ala Lys Asn Ala Gln
        840                 845                 850

aat att aac cca tct tcc tcg cag aat tca cag aat ttt gca act tat     3066
Asn Ile Asn Pro Ser Ser Ser Gln Asn Ser Gln Asn Phe Ala Thr Tyr
    855                 860                 865

aag gaa ggt tac aac gta tat ggc atc gaa agt gtt aaa att tag         3111
Lys Glu Gly Tyr Asn Val Tyr Gly Ile Glu Ser Val Lys Ile
870                 875                 880

gggatgacct tgaatgatgc catgaggaac aaggcaaggc tgtcaattac aggaagtact     3171

ggagaaaatg gacgtgttat gactccagaa tttcccaaag cagtgcatgc tgtcccttac     3231

gtgagtcctg gcatgggaat gaatgtcagt gtgactgatc tctcgtgatt gataagaacc     3291

ttttgagtgc cttacacaat ggttttcttg tgtgtttatt gtcaaagtgg tgagaggcat     3351

ccagtatctt gaagactttt ctttcagcca agaattctta aatatgtgga gttcatcttg     3411

aattgtaagg aatgattaat taaaacacaa catctttttc tactcgagtt acagacaaag     3471

cgtggtggac atgcacagct aacatggaag tactataatt tacctgaagt ctttgtacag     3531

acaacaaacc tgtttctgca gccactattg ttagtctctt gattcataat gacttaagca     3591

cacttgacat caactgcatc aagatgtgac atgttttata aaaaaaggaa aaaaaacatt     3651

taaaactaaa aaatattttt aggtattttc acaaacaaac tggcttttaa ataaatttgc     3711

ttccatattg gttgaataag acaaaaacaa ttaaactgag tgggaagtga ataaaaaaag     3771

gctttaggta tcgattccat atttttcaaa gccaaatatg taaatgctaa ggaaagtaaa     3831

caaagaggag attccaatct tgtaatttaa tattgttatt aaaactttaa tgtatcctat     3891

tctttaacat ttggtgttaa tataaaatta cttggcaatg cttgacattt gaaataaaca 3951
tttttctatt gttttattgc aagtggtcca attaattttg cttagctaca gtttggtcat 4011
aaatcaagtg agtttaaaga cactaccaag ttgttaggtg cccagagaaa atttctccct 4071
tttaaaaagg ccaggtgatt tttcaaatgt aatcttgccc ccaaagtaat atctgaatat 4131
ctttttgaca tgtctaaata tatatatata taaagaaata tttgttaaca caaaagcatt 4191
tgatctatgt agataaatgc taatagattt aaaaagctaa tattaacaaa taccagaata 4251
cgtgaagttc catttttaaa gtgtttgagc ttacagaaga gaaacattca ttttaaatga 4311
agtaaaaaat gccttgaaag taattcttta gatagttgcc cattgattaa attccaaaaa 4371
ctaaatatgt ttttagcttt aaaattataa aagctgtcat aaactttata tattatgaat 4431
tttaaaatat gtttgagtct cctgcaatat agtttcatcc cattgacatc aattaaaaat 4491
aaccctaata tattattttt atatttattc ctcaggtgga atggctattt taatatgccc 4551
agtgtggata aaatgtcaca tttctgtaac ttttgactaa agagcctata tttatctagt 4611
taatgaattt aaaggatcta tctttccctt cataaaatac ctcttatttc cattaaagcc 4671
ccccaagttt aattaattta ggattttgaa tgattattga catccaatag ttatttttaa 4731
tatttgtatt cttgttattt ctggaagaaa gcctttgtgt agcacttggt attttgcaaa 4791
gtgcttttaa aacattctta cttaccgtat ttcatagaag ggaaggaaaa atgtaaggtt 4851
taacagtaag cacttgcatt gaacatggag gcatgtggta tcatgatatt cttcactaaa 4911
tttagctgtc cctaatcaca gatcctaagg taatataata taattttagt gcatttctcc 4971
tcatcaggaa tgctggaggt gcattttaag ttttaataat aagtgctaga atgaccaaat 5031
tgcagactaa ttgtttccat attgtactta aaatgagttt ttaaaagtga aaaagaaatg 5091
actatataca atcaatgcta tttattgtac ctctgggcct actcttctaa aaattgtagc 5151
ttatcgattt ttctctgtca agcttgaact aatgtaaata attgaaataa tgtaaagtta 5211
tattttcatg tttttataga tacaacatga caagaataca taatgtaaga gtatttcaac 5271
tatggataat gttgattgga taatgcacat ctcagttaca agcagtactc atagtttaat 5331
atccatgtaa cggtgcatca atatattgct atataaatat gtctgtgtgc atataagtga 5391
aaagtggtca aacaagagtg atgacagctg tctaaaggtt tttttattca ttttatataa 5451
aaactgttat ggaaagacca aaatgtttat gaactattct tatgtaaatt tacaattgtc 5511
ctttactgta cttttttgtt tacagtatag taccttattt tctgctgtgt taagtgggtg 5571
tcaaactcca agaagacata cactttctat aacttctatt gaagatattg gaatttccaa 5631
tttttcatgt gtactatgtc agaaaatgct ttcgatttta tttttaaatc taacatcgga 5691
tggcttttcc ggagtgttgt aaaaacttca atcatacata aaacatgttc ttacaaaagg 5751
caaa 5755

<210> 80
<211> 883

<212>  PRT
<213>  human

<400>  80

Met Gln Lys Ile Met His Ile Ser Val Leu Leu Ser Pro Val Leu Trp
1               5                   10                  15

Gly Leu Ile Phe Gly Val Ser Ser Asn Ser Ile Gln Ile Gly Gly Leu
            20                  25                  30

Phe Pro Arg Gly Ala Asp Gln Glu Tyr Ser Ala Phe Arg Val Gly Met
        35                  40                  45

Val Gln Phe Ser Thr Ser Glu Phe Arg Leu Thr Pro His Ile Asp Asn
    50                  55                  60

Leu Glu Val Ala Asn Ser Phe Ala Val Thr Asn Ala Phe Cys Ser Gln
65                  70                  75                  80

Phe Ser Arg Gly Val Tyr Ala Ile Phe Gly Phe Tyr Asp Lys Lys Ser
                85                  90                  95

Val Asn Thr Ile Thr Ser Phe Cys Gly Thr Leu His Val Ser Phe Ile
            100                 105                 110

Thr Pro Ser Phe Pro Thr Asp Gly Thr His Pro Phe Val Ile Gln Met
        115                 120                 125

Arg Pro Asp Leu Lys Gly Ala Leu Leu Ser Leu Ile Glu Tyr Tyr Gln
    130                 135                 140

Trp Asp Lys Phe Ala Tyr Leu Tyr Asp Ser Asp Arg Gly Leu Ser Thr
145                 150                 155                 160

Leu Gln Ala Val Leu Asp Ser Ala Ala Glu Lys Lys Trp Gln Val Thr
                165                 170                 175

Ala Ile Asn Val Gly Asn Ile Asn Asn Asp Lys Lys Asp Glu Met Tyr
            180                 185                 190

Arg Ser Leu Phe Gln Asp Leu Glu Leu Lys Lys Glu Arg Arg Val Ile
        195                 200                 205

Leu Asp Cys Glu Arg Asp Lys Val Asn Asp Ile Val Asp Gln Val Ile
    210                 215                 220

Thr Ile Gly Lys His Val Lys Gly Tyr His Tyr Ile Ile Ala Asn Leu
225                 230                 235                 240

Gly Phe Thr Asp Gly Asp Leu Leu Lys Ile Gln Phe Gly Gly Ala Asn
                245                 250                 255

Val Ser Gly Phe Gln Ile Val Asp Tyr Asp Asp Ser Leu Val Ser Lys
            260                 265                 270

Phe Ile Glu Arg Trp Ser Thr Leu Glu Glu Lys Glu Tyr Pro Gly Ala
        275                 280                 285

His Thr Thr Thr Ile Lys Tyr Thr Ser Ala Leu Thr Tyr Asp Ala Val
    290                 295                 300

Gln Val Met Thr Glu Ala Phe Arg Asn Leu Arg Lys Gln Arg Ile Glu
305                 310                 315                 320

Ile Ser Arg Arg Gly Asn Ala Gly Asp Cys Leu Ala Asn Pro Ala Val
                325                 330                 335

Pro Trp Gly Gln Gly Val Glu Ile Glu Arg Ala Leu Lys Gln Val Gln
            340                 345                 350

Val Glu Gly Leu Ser Gly Asn Ile Lys Phe Asp Gln Asn Gly Lys Arg
        355                 360                 365

Ile Asn Tyr Thr Ile Asn Ile Met Glu Leu Lys Thr Asn Gly Pro Arg
    370                 375                 380

Lys Ile Gly Tyr Trp Ser Glu Val Asp Lys Met Val Val Thr Leu Thr
385                 390                 395                 400

Glu Leu Pro Ser Gly Asn Asp Thr Ser Gly Leu Glu Asn Lys Thr Val
                405                 410                 415

Val Val Thr Thr Ile Leu Glu Ser Pro Tyr Val Met Met Lys Lys Asn
            420                 425                 430

His Glu Met Leu Glu Gly Asn Glu Arg Tyr Glu Gly Tyr Cys Val Asp
        435                 440                 445

Leu Ala Ala Glu Ile Ala Lys His Cys Gly Phe Lys Tyr Lys Leu Thr
    450                 455                 460

Ile Val Gly Asp Gly Lys Tyr Gly Ala Arg Asp Ala Asp Thr Lys Ile
465                 470                 475                 480

Trp Asn Gly Met Val Gly Glu Leu Val Tyr Gly Lys Ala Asp Ile Ala
                485                 490                 495

Ile Ala Pro Leu Thr Ile Thr Leu Val Arg Glu Glu Val Ile Asp Phe
            500                 505                 510

Ser Lys Pro Phe Met Ser Leu Gly Ile Ser Ile Met Ile Lys Lys Pro
        515                 520                 525

Gln Lys Ser Lys Pro Gly Val Phe Ser Phe Leu Asp Pro Leu Ala Tyr
    530                 535                 540

Glu Ile Trp Met Cys Ile Val Phe Ala Tyr Ile Gly Val Ser Val Val
545                 550                 555                 560

Leu Phe Leu Val Ser Arg Phe Ser Pro Tyr Glu Trp His Thr Glu Glu
                565                 570                 575

Phe Glu Asp Gly Arg Glu Thr Gln Ser Ser Glu Ser Thr Asn Glu Phe
            580                 585                 590

Gly Ile Phe Asn Ser Leu Trp Phe Ser Leu Gly Ala Phe Met Arg Gln
        595                 600                 605

Gly Cys Asp Ile Ser Pro Arg Ser Leu Ser Gly Arg Ile Val Gly Gly
    610                 615                 620

Val Trp Trp Phe Phe Thr Leu Ile Ile Ile Ser Ser Tyr Thr Ala Asn
625                 630                 635                 640

Leu Ala Ala Phe Leu Thr Val Glu Arg Met Val Ser Pro Ile Glu Ser
                645                 650                 655

Ala Glu Asp Leu Ser Lys Gln Thr Glu Ile Ala Tyr Gly Thr Leu Asp
            660                 665                 670

Ser Gly Ser Thr Lys Glu Phe Phe Arg Arg Ser Lys Ile Ala Val Phe
        675                 680                 685

Asp Lys Met Trp Thr Tyr Met Arg Ser Ala Glu Pro Ser Val Phe Val
    690                 695                 700

Arg Thr Thr Ala Glu Gly Val Ala Arg Val Arg Lys Ser Lys Gly Lys
705                 710                 715                 720

Tyr Ala Tyr Leu Leu Glu Ser Thr Met Asn Glu Tyr Ile Glu Gln Arg
                725                 730                 735

Lys Pro Cys Asp Thr Met Lys Val Gly Gly Asn Leu Asp Ser Lys Gly
            740                 745                 750

Tyr Gly Ile Ala Thr Pro Lys Gly Ser Ser Leu Arg Asn Ala Val Asn
        755                 760                 765

Leu Ala Val Leu Lys Leu Asn Glu Gln Gly Leu Leu Asp Lys Leu Lys
    770                 775                 780

Asn Lys Trp Trp Tyr Asp Lys Gly Glu Cys Gly Ser Gly Gly Gly Asp
785                 790                 795                 800

Ser Lys Glu Lys Thr Ser Ala Leu Ser Leu Ser Asn Val Ala Gly Val
                805                 810                 815

Phe Tyr Ile Leu Val Gly Gly Leu Gly Leu Ala Met Leu Val Ala Leu
            820                 825                 830

Ile Glu Phe Cys Tyr Lys Ser Arg Ala Glu Ala Lys Arg Met Lys Val
        835                 840                 845

Ala Lys Asn Ala Gln Asn Ile Asn Pro Ser Ser Ser Gln Asn Ser Gln
    850                 855                 860

Asn Phe Ala Thr Tyr Lys Glu Gly Tyr Asn Val Tyr Gly Ile Glu Ser
865                 870                 875                 880

Val Lys Ile

<210>  81
<211>  5195
<212>  DNA
<213>  human

<220>
<221>  CDS
<222>  (294)..(2978)

<400>  81
agagatcctg ggagcgagag ggagagagag ggagcaagaa aggaagagag agcgagcgag      60

agagagcgag cgaataagag agagagtaag agggagagag aagaagagga agaagaggag     120

gcggcggcag cggaggagga ggaggactag tgtggggtgg aaaggaagag tgagcgagag     180

caagttaagg ggagggggtg taagagccag cgaattcttt ttctttttct attattattt     240

tgacgactcc tgagttgcgc ccatgctctt gtcagcttcg ttttaggcgt agc atg        296
                                                           Met
                                                           1

gcc agg cag aag aaa atg ggg caa agc gtg ctc cgg gcg gtc ttc ttt       344
Ala Arg Gln Lys Lys Met Gly Gln Ser Val Leu Arg Ala Val Phe Phe
            5                   10                  15

tta gtc ctg ggg ctt ttg ggt cat tct cac gga gga ttc ccc aac acc       392
Leu Val Leu Gly Leu Leu Gly His Ser His Gly Gly Phe Pro Asn Thr
        20                  25                  30

atc agc ata ggt gga ctt ttc atg aga aac aca gtg cag gag cac agc       440
Ile Ser Ile Gly Gly Leu Phe Met Arg Asn Thr Val Gln Glu His Ser
    35                  40                  45

gct ttc cgc ttt gcc gtg cag tta tac aac acc aac cag aac acc acc       488
Ala Phe Arg Phe Ala Val Gln Leu Tyr Asn Thr Asn Gln Asn Thr Thr
50                  55                  60                  65

gag aag ccc ttc cat ttg aat tac cac gta gat cac ttg gat tcc tcc       536
Glu Lys Pro Phe His Leu Asn Tyr His Val Asp His Leu Asp Ser Ser
                70                  75                  80

aat agt ttt tcc gtg aca aat gct ttc tgc tcc cag ttc tcg aga ggg 584
Asn Ser Phe Ser Val Thr Asn Ala Phe Cys Ser Gln Phe Ser Arg Gly
            85                  90                  95

gtg tat gcc atc ttt gga ttc tat gac cag atg tca atg aac acc ctg 632
Val Tyr Ala Ile Phe Gly Phe Tyr Asp Gln Met Ser Met Asn Thr Leu
        100                 105                 110

acc tcc ttc tgt ggg gcc ctg cac aca tcc ttt gtt acg cct agc ttc 680
Thr Ser Phe Cys Gly Ala Leu His Thr Ser Phe Val Thr Pro Ser Phe
    115                 120                 125

ccc act gac gca gat gtg cag ttt gtc atc cag atg cgc cca gcc ttg 728
Pro Thr Asp Ala Asp Val Gln Phe Val Ile Gln Met Arg Pro Ala Leu
130                 135                 140                 145

aag ggc gct att ctg agt ctt ctg ggt cat tac aag tgg gag aag ttt 776
Lys Gly Ala Ile Leu Ser Leu Leu Gly His Tyr Lys Trp Glu Lys Phe
                150                 155                 160

gtg tac ctc tat gac aca gaa cga gga ttt tcc atc ctc caa gcg att 824
Val Tyr Leu Tyr Asp Thr Glu Arg Gly Phe Ser Ile Leu Gln Ala Ile
            165                 170                 175

atg gaa gca gca gtg caa aac aac tgg caa gta aca gca agg tct gtg 872
Met Glu Ala Ala Val Gln Asn Asn Trp Gln Val Thr Ala Arg Ser Val
        180                 185                 190

gga aac ata aag gac gtc caa gaa ttc agg cgc atc att gaa gaa atg 920
Gly Asn Ile Lys Asp Val Gln Glu Phe Arg Arg Ile Ile Glu Glu Met
    195                 200                 205

gac agg agg cag gaa aag cga tac ttg att gac tgc gaa gtc gaa agg 968
Asp Arg Arg Gln Glu Lys Arg Tyr Leu Ile Asp Cys Glu Val Glu Arg
210                 215                 220                 225

att aac aca att ttg gaa cag gtt gtg atc cta ggg aaa cac tca aga 1016
Ile Asn Thr Ile Leu Glu Gln Val Val Ile Leu Gly Lys His Ser Arg
                230                 235                 240

ggt tat cac tac atg ctc gct aac ctg ggt ttt act gat att tta ctg 1064
Gly Tyr His Tyr Met Leu Ala Asn Leu Gly Phe Thr Asp Ile Leu Leu
            245                 250                 255

gaa aga gtc atg cat ggg gga gcc aac att aca ggt ttc cag att gtc 1112
Glu Arg Val Met His Gly Gly Ala Asn Ile Thr Gly Phe Gln Ile Val
        260                 265                 270

aac aat gaa aac cct atg gtt cag cag ttc ata cag cgc tgg gtg agg 1160
Asn Asn Glu Asn Pro Met Val Gln Gln Phe Ile Gln Arg Trp Val Arg
    275                 280                 285

ctg gat gaa agg gaa ttc cct gaa gcc aag aat gca cca cta aag tat 1208
Leu Asp Glu Arg Glu Phe Pro Glu Ala Lys Asn Ala Pro Leu Lys Tyr
290                 295                 300                 305

aca tct gca ttg aca cac gac gca ata ctg gtc ata gca gaa gct ttc 1256
Thr Ser Ala Leu Thr His Asp Ala Ile Leu Val Ile Ala Glu Ala Phe
                310                 315                 320

cgc tac ctg agg agg cag cga gta gat gtg tcc cgg aga gga agt gct 1304
Arg Tyr Leu Arg Arg Gln Arg Val Asp Val Ser Arg Arg Gly Ser Ala
            325                 330                 335

gga gac tgc tta gca aat cct gct gtg ccc tgg agt caa gga att gat 1352
Gly Asp Cys Leu Ala Asn Pro Ala Val Pro Trp Ser Gln Gly Ile Asp
        340                 345                 350

att gag aga gct ctg aaa atg gtg caa gta caa gga atg act gga aat 1400
Ile Glu Arg Ala Leu Lys Met Val Gln Val Gln Gly Met Thr Gly Asn
    355                 360                 365

att caa ttt gac act tat gga cgt agg aca aat tat acc atc gat gtg 1448
Ile Gln Phe Asp Thr Tyr Gly Arg Arg Thr Asn Tyr Thr Ile Asp Val
370                 375                 380                 385

tat gaa atg aaa gtc agt ggc tct cga aaa gct ggc tac tgg aat gag 1496
Tyr Glu Met Lys Val Ser Gly Ser Arg Lys Ala Gly Tyr Trp Asn Glu
                390                 395                 400

tat gaa agg ttt gtg cct ttc tca gat cag caa atc agc aat gac agt 1544
Tyr Glu Arg Phe Val Pro Phe Ser Asp Gln Gln Ile Ser Asn Asp Ser
            405                 410                 415

gca tcc tca gag aat cgg acc ata gta gtg act acc att ctg gaa tca 1592
Ala Ser Ser Glu Asn Arg Thr Ile Val Val Thr Thr Ile Leu Glu Ser
        420                 425                 430

cca tat gta atg tac aag aag aac cat gag caa ctg gaa gga aat gaa 1640
Pro Tyr Val Met Tyr Lys Lys Asn His Glu Gln Leu Glu Gly Asn Glu
    435                 440                 445

cga tat gaa ggc tat tgt gta gac cta gcc tat gaa ata gcc aaa cat 1688
Arg Tyr Glu Gly Tyr Cys Val Asp Leu Ala Tyr Glu Ile Ala Lys His
450                 455                 460                 465

gta agg atc aaa tac aaa ttg tcc atc gtt ggt gac ggg aaa tat ggt 1736
Val Arg Ile Lys Tyr Lys Leu Ser Ile Val Gly Asp Gly Lys Tyr Gly
                470                 475                 480

gca agg gat cca gag act aaa ata tgg aac ggc atg gtt ggg gaa ctt 1784
Ala Arg Asp Pro Glu Thr Lys Ile Trp Asn Gly Met Val Gly Glu Leu
            485                 490                 495

gtc tat ggg aga gct gat ata gct gtt gct cca ctc act ata aca ttg 1832
Val Tyr Gly Arg Ala Asp Ile Ala Val Ala Pro Leu Thr Ile Thr Leu
        500                 505                 510

gtc cgt gaa gaa gtc ata gat ttt tca aag cca ttc atg agc ctg ggc 1880
Val Arg Glu Glu Val Ile Asp Phe Ser Lys Pro Phe Met Ser Leu Gly
    515                 520                 525

atc tcc atc atg ata aag aag cct cag aaa tca aaa cca ggc gta ttc 1928
Ile Ser Ile Met Ile Lys Lys Pro Gln Lys Ser Lys Pro Gly Val Phe
530                 535                 540                 545

tca ttt ctg gat ccc ctg gct tat gaa atc tgg atg tgc att gtc ttt 1976
Ser Phe Leu Asp Pro Leu Ala Tyr Glu Ile Trp Met Cys Ile Val Phe
                550                 555                 560

gct tac att gga gtc agc gta gtt ctt ttc cta gtc agc agg ttc agt 2024
Ala Tyr Ile Gly Val Ser Val Val Leu Phe Leu Val Ser Arg Phe Ser
            565                 570                 575

cct tat gaa tgg cac ttg gaa gac aac aat gaa gaa cct cgt gac cca 2072
Pro Tyr Glu Trp His Leu Glu Asp Asn Asn Glu Glu Pro Arg Asp Pro
        580                 585                 590

caa agt cct cct gat cct cca aat gaa ttt gga ata ttt aac agt ctt 2120
Gln Ser Pro Pro Asp Pro Pro Asn Glu Phe Gly Ile Phe Asn Ser Leu
    595                 600                 605

tgg ttt tcc ttg ggt gcc ttt atg cag caa gga tgt gat att tct cca 2168
Trp Phe Ser Leu Gly Ala Phe Met Gln Gln Gly Cys Asp Ile Ser Pro
610                 615                 620                 625

aga tca ctc tcc ggg cgc att gtt gga ggg gtt tgg tgg ttc ttc acc 2216
Arg Ser Leu Ser Gly Arg Ile Val Gly Gly Val Trp Trp Phe Phe Thr
                630                 635                 640

ctg atc ata att tct tcc tat act gcc aat ctc gct gct ttc ctg act 2264
Leu Ile Ile Ile Ser Ser Tyr Thr Ala Asn Leu Ala Ala Phe Leu Thr
            645                 650                 655

gtg gag agg atg gtt tct ccc ata gag agt gct gaa gac tta gct aaa 2312
Val Glu Arg Met Val Ser Pro Ile Glu Ser Ala Glu Asp Leu Ala Lys
        660                 665                 670

cag act gaa att gca tat ggg acc ctg gac tcc ggt tca aca aaa gaa 2360
Gln Thr Glu Ile Ala Tyr Gly Thr Leu Asp Ser Gly Ser Thr Lys Glu
    675                 680                 685

ttt ttc aga aga tcc aaa att gct gtg tac gag aaa atg tgg tct tac 2408
Phe Phe Arg Arg Ser Lys Ile Ala Val Tyr Glu Lys Met Trp Ser Tyr
690                 695                 700                 705

atg aaa tca gcg gag cca tct gtg ttt acc aaa aca aca gca gac gga 2456
Met Lys Ser Ala Glu Pro Ser Val Phe Thr Lys Thr Thr Ala Asp Gly
                710                 715                 720

gtg gcc cga gtg cga aag tcc aag gga aag ttc gcc ttc ctg ctg gag 2504
Val Ala Arg Val Arg Lys Ser Lys Gly Lys Phe Ala Phe Leu Leu Glu
            725                 730                 735

tca acc atg aat gag tac att gag cag aga aaa cca tgt gat acg atg 2552
Ser Thr Met Asn Glu Tyr Ile Glu Gln Arg Lys Pro Cys Asp Thr Met
        740                 745                 750

aaa gtt ggt gga aat ctg gat tcc aaa ggc tat ggt gtg gca acc cct 2600
Lys Val Gly Gly Asn Leu Asp Ser Lys Gly Tyr Gly Val Ala Thr Pro
    755                 760                 765

aaa ggc tca gca tta gga acg cct gta aac ctt gca gta ttg aaa ctc 2648
Lys Gly Ser Ala Leu Gly Thr Pro Val Asn Leu Ala Val Leu Lys Leu
770                 775                 780                 785

agt gaa caa ggc atc tta gac aag ctg aaa aac aaa tgg tgg tac gat 2696
Ser Glu Gln Gly Ile Leu Asp Lys Leu Lys Asn Lys Trp Trp Tyr Asp
                790                 795                 800

aag ggg gaa tgt gga gcc aag gac tcc ggg agt aag gac aag acc agc 2744
Lys Gly Glu Cys Gly Ala Lys Asp Ser Gly Ser Lys Asp Lys Thr Ser
            805                 810                 815

gct ctg agc ctg agc aat gtg gca ggc gtt ttc tat ata ctt gtc gga 2792
Ala Leu Ser Leu Ser Asn Val Ala Gly Val Phe Tyr Ile Leu Val Gly
        820                 825                 830

ggt ctg ggg ctg gcc atg atg gtg gct ttg ata gaa ttc tgt tac aaa 2840
Gly Leu Gly Leu Ala Met Met Val Ala Leu Ile Glu Phe Cys Tyr Lys
    835                 840                 845

tca cgg gca gag tcc aaa cgc atg aaa ctc aca aag aac acc caa aac 2888
Ser Arg Ala Glu Ser Lys Arg Met Lys Leu Thr Lys Asn Thr Gln Asn
850                 855                 860                 865

ttt aag cct gct cct gcc acc aac act cag aat tat gct aca tac aga 2936
Phe Lys Pro Ala Pro Ala Thr Asn Thr Gln Asn Tyr Ala Thr Tyr Arg
                870                 875                 880

gaa ggc tac aac gtg tat gga aca gag agt gtt aag atc tag 2978
Glu Gly Tyr Asn Val Tyr Gly Thr Glu Ser Val Lys Ile
            885                 890

ggatcccttc ccactggagg catgtgatga gaggaaatca ccgaaaacgt ggctgcttca 3038
aggatcctga gccagatttc actctccttg gtgtcgggca tgacacgaat attgctgatg 3098
gtgcaatgac ctttcaatag gaaaaactga tttttttttc cttcagtgcc ttatggaaca 3158
ctctgagact cgcgacaatg caaaccatca ttgaaatctt tttgctttgc ttgaaaaaaa 3218
ataattaaaa taaaaaccaa caaaaatgga catgcaagat tccagtatgc gaaaaaaaat 3278
cttattaagt caattcaaca aaagccattc tttgatacca ctgcagagta tataaacacc 3338
atgttcttta atacacacac acacacacac acacacacac acacacattt aaattccaat 3398
tcagcaaaga ggcccatcta agctaaaaaa attaattctt cctgattaaa aagaaaaaat 3458
ctgtctccca gtgtttggga agacggactg gcatttcttc taggatctgc tgaccagatg 3518
tttttggtat ttcctgttgg tggtgatgtt ctgtgcactc tatttccttt caatgttgct 3578
gaaatgtgta tatctttaga atgtaaatgc aacacttaag aaaattcaaa cactttggaa 3638
aagggactaa acagtgattt ctctgtgttc ttgaaatggt tttgtgaaaa tgctttgata 3698
acttcccact caaagaagag atttacagag ctttcgaaat tgactttgtg tgtagcaagg 3758
gacggggcac tatcaggata cctcttggtg ctttcctaaa atggatcccg gggctttcca 3818
aggagcctgg aatttcagct cacagatctg tttttcttgc ttcagtgtgc attttaagtc 3878
aatagagctg agtatctagc attgaggtga gggaaatgct gcctatactc ccagatgtgt 3938
ttagaatatc tcagaaacaa cactgtgttt agctcggctt tctctgctaa gtatgccttt 3998
caagtgtaca ccacggagac aggaccgcgt tgcaaggcgg gacagcaggt tcagaccaca 4058
gttctcagtc tgactttact cttgctaggt ctgtcctact agctgttgcc tgctaccgcc 4118
catggctctc catcggactg catgtgtcct tttctagttt gcaaagacta aaatgcattc 4178
ccaaacctac tgctaatctg agggcctcag catcacttcc agatccttgc ttggagcagt 4238
ctctctattg actctctcag atcgctccac tgctccatgg gctatcaagt aactaactgc 4298
atacctgccg ttggcatcat cagaacagtc cgaagaaata gtctccactc actaattacc 4358
tcctatataa cgacgtatgc ttcctgtagt tcagtagttt gctctcatcg ataacgtgca 4418
ttgggaagtt tccagactgc aaaaactagg agctcgcatt catttcccaa gtgtgaccct 4478
tagatgctta gttgactcgc tgcatatttg ctcttgtctt cagaaaagaa aggaagaagt 4538
atcgttccaa cgaaatgttt ccagaaaagt gtactataaa ctttcattcc aaaaatggtg 4598
tcataagcaa acaactcact tgtcaaattt caaatggtat tgaacaaaaa aagaaagctg 4658
ttgtgttttt gttttgtttt gttttcatga aactgtgatt ttcaacttat gaatgctata 4718
atgtcccagc gcgggaagct cacgctgtgt gaacatgaag ttgtataaaa caaaccaacc 4778
aacctacaca caaatgtttt cataggcact gtataaagaa aaatgtatgt ttattaactc 4838
aaatcagttt ttcagagagg aaacgtcact gagatgaaga ggcgggtaaa ttggtttgtt 4898
attttttaaa aaaaacttgc atgtttaaaa aaaagttgat tgcttcaaat ttctgctact 4958
aacttcaagc tatgggagtt tggcagtagt cacttgagga ttttttttcc aattcttttc 5018

tttttgttgt taaagctgta cttcagtgaa cagaaaaatt gccaagcaaa ctaatggcta 5078

taaaagcgta atttgcatgt gtgggcataa actacagagc ctcattgcca tgaggtattg 5138

tacaaagttt taatacattt tgtaaataaa attgtaaaga aagaaaaaaa aaaaaaa 5195

<210> 82
<211> 894
<212> PRT
<213> human

<400> 82

Met Ala Arg Gln Lys Lys Met Gly Gln Ser Val Leu Arg Ala Val Phe
1               5                   10                  15

Phe Leu Val Leu Gly Leu Leu Gly His Ser His Gly Gly Phe Pro Asn
            20                  25                  30

Thr Ile Ser Ile Gly Gly Leu Phe Met Arg Asn Thr Val Gln Glu His
        35                  40                  45

Ser Ala Phe Arg Phe Ala Val Gln Leu Tyr Asn Thr Asn Gln Asn Thr
    50                  55                  60

Thr Glu Lys Pro Phe His Leu Asn Tyr His Val Asp His Leu Asp Ser
65                  70                  75                  80

Ser Asn Ser Phe Ser Val Thr Asn Ala Phe Cys Ser Gln Phe Ser Arg
                85                  90                  95

Gly Val Tyr Ala Ile Phe Gly Phe Tyr Asp Gln Met Ser Met Asn Thr
            100                 105                 110

Leu Thr Ser Phe Cys Gly Ala Leu His Thr Ser Phe Val Thr Pro Ser
        115                 120                 125

Phe Pro Thr Asp Ala Asp Val Gln Phe Val Ile Gln Met Arg Pro Ala
    130                 135                 140

Leu Lys Gly Ala Ile Leu Ser Leu Leu Gly His Tyr Lys Trp Glu Lys
145                 150                 155                 160

Phe Val Tyr Leu Tyr Asp Thr Glu Arg Gly Phe Ser Ile Leu Gln Ala
                165                 170                 175

Ile Met Glu Ala Ala Val Gln Asn Asn Trp Gln Val Thr Ala Arg Ser
            180                 185                 190

Val Gly Asn Ile Lys Asp Val Gln Glu Phe Arg Arg Ile Ile Glu Glu
        195                 200                 205

Met Asp Arg Arg Gln Glu Lys Arg Tyr Leu Ile Asp Cys Glu Val Glu
    210                 215                 220

Arg Ile Asn Thr Ile Leu Glu Gln Val Val Ile Leu Gly Lys His Ser
225                 230                 235                 240

Arg Gly Tyr His Tyr Met Leu Ala Asn Leu Gly Phe Thr Asp Ile Leu
                245                 250                 255

Leu Glu Arg Val Met His Gly Gly Ala Asn Ile Thr Gly Phe Gln Ile
            260                 265                 270

Val Asn Asn Glu Asn Pro Met Val Gln Gln Phe Ile Gln Arg Trp Val
        275                 280                 285

Arg Leu Asp Glu Arg Glu Phe Pro Glu Ala Lys Asn Ala Pro Leu Lys
    290                 295                 300

Tyr Thr Ser Ala Leu Thr His Asp Ala Ile Leu Val Ile Ala Glu Ala
305                 310                 315                 320

Phe Arg Tyr Leu Arg Arg Gln Arg Val Asp Val Ser Arg Arg Gly Ser
                325                 330                 335

Ala Gly Asp Cys Leu Ala Asn Pro Ala Val Pro Trp Ser Gln Gly Ile
            340                 345                 350

Asp Ile Glu Arg Ala Leu Lys Met Val Gln Val Gln Gly Met Thr Gly
        355                 360                 365

Asn Ile Gln Phe Asp Thr Tyr Gly Arg Arg Thr Asn Tyr Thr Ile Asp
    370                 375                 380

Val Tyr Glu Met Lys Val Ser Gly Ser Arg Lys Ala Gly Tyr Trp Asn
385                 390                 395                 400

Glu Tyr Glu Arg Phe Val Pro Phe Ser Asp Gln Gln Ile Ser Asn Asp
                405                 410                 415

Ser Ala Ser Ser Glu Asn Arg Thr Ile Val Val Thr Thr Ile Leu Glu
            420                 425                 430

Ser Pro Tyr Val Met Tyr Lys Lys Asn His Glu Gln Leu Glu Gly Asn
        435                 440                 445

Glu Arg Tyr Glu Gly Tyr Cys Val Asp Leu Ala Tyr Glu Ile Ala Lys
    450                 455                 460

His Val Arg Ile Lys Tyr Lys Leu Ser Ile Val Gly Asp Gly Lys Tyr
465                 470                 475                 480

Gly Ala Arg Asp Pro Glu Thr Lys Ile Trp Asn Gly Met Val Gly Glu
                485                 490                 495

Leu Val Tyr Gly Arg Ala Asp Ile Ala Val Ala Pro Leu Thr Ile Thr
            500                 505                 510

Leu Val Arg Glu Glu Val Ile Asp Phe Ser Lys Pro Phe Met Ser Leu
        515                 520                 525

Gly Ile Ser Ile Met Ile Lys Lys Pro Gln Lys Ser Lys Pro Gly Val
    530                 535                 540

Phe Ser Phe Leu Asp Pro Leu Ala Tyr Glu Ile Trp Met Cys Ile Val
545                 550                 555                 560

Phe Ala Tyr Ile Gly Val Ser Val Val Leu Phe Leu Val Ser Arg Phe
                565                 570                 575

Ser Pro Tyr Glu Trp His Leu Glu Asp Asn Asn Glu Glu Pro Arg Asp
            580                 585                 590

Pro Gln Ser Pro Pro Asp Pro Pro Asn Glu Phe Gly Ile Phe Asn Ser
        595                 600                 605

Leu Trp Phe Ser Leu Gly Ala Phe Met Gln Gln Gly Cys Asp Ile Ser
    610                 615                 620

Pro Arg Ser Leu Ser Gly Arg Ile Val Gly Gly Val Trp Trp Phe Phe
625                 630                 635                 640

Thr Leu Ile Ile Ile Ser Ser Tyr Thr Ala Asn Leu Ala Ala Phe Leu
                645                 650                 655

Thr Val Glu Arg Met Val Ser Pro Ile Glu Ser Ala Glu Asp Leu Ala
            660                 665                 670

Lys Gln Thr Glu Ile Ala Tyr Gly Thr Leu Asp Ser Gly Ser Thr Lys
        675                 680                 685

Glu Phe Phe Arg Arg Ser Lys Ile Ala Val Tyr Glu Lys Met Trp Ser
    690                 695                 700

Tyr Met Lys Ser Ala Glu Pro Ser Val Phe Thr Lys Thr Thr Ala Asp
705                 710                 715                 720

Gly Val Ala Arg Val Arg Lys Ser Lys Gly Lys Phe Ala Phe Leu Leu
                725                 730                 735

Glu Ser Thr Met Asn Glu Tyr Ile Glu Gln Arg Lys Pro Cys Asp Thr
            740                 745                 750

Met Lys Val Gly Gly Asn Leu Asp Ser Lys Gly Tyr Gly Val Ala Thr
        755                 760                 765

Pro Lys Gly Ser Ala Leu Gly Thr Pro Val Asn Leu Ala Val Leu Lys
    770                 775                 780

Leu Ser Glu Gln Gly Ile Leu Asp Lys Leu Lys Asn Lys Trp Trp Tyr
785                 790                 795                 800

Asp Lys Gly Glu Cys Gly Ala Lys Asp Ser Gly Ser Lys Asp Lys Thr
                805                 810                 815

Ser Ala Leu Ser Leu Ser Asn Val Ala Gly Val Phe Tyr Ile Leu Val
            820                 825                 830

Gly Gly Leu Gly Leu Ala Met Met Val Ala Leu Ile Glu Phe Cys Tyr
        835                 840                 845

Lys Ser Arg Ala Glu Ser Lys Arg Met Lys Leu Thr Lys Asn Thr Gln
    850                 855                 860

Asn Phe Lys Pro Ala Pro Ala Thr Asn Thr Gln Asn Tyr Ala Thr Tyr
865                 870                 875                 880

Arg Glu Gly Tyr Asn Val Tyr Gly Thr Glu Ser Val Lys Ile
                885                 890

<210>  83
<211>  1376
<212>  DNA
<213>  human

<220>
<221>  CDS
<222>  (126)..(1268)

<400>  83
gtaagaacac tctcgtgagt ctaacggtct tccggatgaa ggctatttga agtcgccata      60

acctggtcag aagtgtgcct gtcggcgggg agagaggcaa tatcaaggtt ttaaatctcg     120

gagaa atg gct ttc gtt tgc ttg gct atc gga tgc tta tat acc ttt ctg     170
      Met Ala Phe Val Cys Leu Ala Ile Gly Cys Leu Tyr Thr Phe Leu
      1               5                   10                  15

ata agc aca aca ttt ggc tgt act tca tct tca gac acc gag ata aaa       218
Ile Ser Thr Thr Phe Gly Cys Thr Ser Ser Ser Asp Thr Glu Ile Lys
                20                  25                  30

gtt aac cct cct cag gat ttt gag ata gtg gat ccc gga tac tta ggt       266
Val Asn Pro Pro Gln Asp Phe Glu Ile Val Asp Pro Gly Tyr Leu Gly
            35                  40                  45

tat ctc tat ttg caa tgg caa ccc cca ctg tct ctg gat cat ttt aag       314
Tyr Leu Tyr Leu Gln Trp Gln Pro Pro Leu Ser Leu Asp His Phe Lys
        50                  55                  60

gaa tgc aca gtg gaa tat gaa cta aaa tac cga aac att ggt agt gaa       362
Glu Cys Thr Val Glu Tyr Glu Leu Lys Tyr Arg Asn Ile Gly Ser Glu
    65                  70                  75

aca tgg aag acc atc att act aag aat cta cat tac aaa gat ggg ttt 410
Thr Trp Lys Thr Ile Ile Thr Lys Asn Leu His Tyr Lys Asp Gly Phe
80                  85                  90                  95

gat ctt aac aag ggc att gaa gcg aag ata cac acg ctt tta cca tgg 458
Asp Leu Asn Lys Gly Ile Glu Ala Lys Ile His Thr Leu Leu Pro Trp
                100                 105                 110

caa tgc aca aat gga tca gaa gtt caa agt tcc tgg gca gaa act act 506
Gln Cys Thr Asn Gly Ser Glu Val Gln Ser Ser Trp Ala Glu Thr Thr
            115                 120                 125

tat tgg ata tca cca caa gga att cca gaa act aaa gtt cag gat atg 554
Tyr Trp Ile Ser Pro Gln Gly Ile Pro Glu Thr Lys Val Gln Asp Met
        130                 135                 140

gat tgc gta tat tac aat tgg caa tat tta ctc tgt tct tgg aaa cct 602
Asp Cys Val Tyr Tyr Asn Trp Gln Tyr Leu Leu Cys Ser Trp Lys Pro
    145                 150                 155

ggc ata ggt gta ctt ctt gat acc aat tac aac ttg ttt tac tgg tat 650
Gly Ile Gly Val Leu Leu Asp Thr Asn Tyr Asn Leu Phe Tyr Trp Tyr
160                 165                 170                 175

gag ggc ttg gat cat gca tta cag tgt gtt gat tac atc aag gct gat 698
Glu Gly Leu Asp His Ala Leu Gln Cys Val Asp Tyr Ile Lys Ala Asp
                180                 185                 190

gga caa aat ata gga tgc aga ttt ccc tat ttg gag gca tca gac tat 746
Gly Gln Asn Ile Gly Cys Arg Phe Pro Tyr Leu Glu Ala Ser Asp Tyr
            195                 200                 205

aaa gat ttc tat att tgt gtt aat gga tca tca gag aac aag cct atc 794
Lys Asp Phe Tyr Ile Cys Val Asn Gly Ser Ser Glu Asn Lys Pro Ile
        210                 215                 220

aga tcc agt tat ttc act ttt cag ctt caa aat ata gtt aaa cct ttg 842
Arg Ser Ser Tyr Phe Thr Phe Gln Leu Gln Asn Ile Val Lys Pro Leu
    225                 230                 235

ccg cca gtc tat ctt act ttt act cgg gag agt tca tgt gaa att aag 890
Pro Pro Val Tyr Leu Thr Phe Thr Arg Glu Ser Ser Cys Glu Ile Lys
240                 245                 250                 255

ctg aaa tgg agc ata cct ttg gga cct att cca gca agg tgt ttt gat 938
Leu Lys Trp Ser Ile Pro Leu Gly Pro Ile Pro Ala Arg Cys Phe Asp
                260                 265                 270

tat gaa att gag atc aga gaa gat gat act acc ttg gtg act gct aca 986
Tyr Glu Ile Glu Ile Arg Glu Asp Asp Thr Thr Leu Val Thr Ala Thr
            275                 280                 285

gtt gaa aat gaa aca tac acc ttg aaa aca aca aat gaa acc cga caa 1034
Val Glu Asn Glu Thr Tyr Thr Leu Lys Thr Thr Asn Glu Thr Arg Gln
        290                 295                 300

tta tgc ttt gta gta aga agc aaa gtg aat att tat tgc tca gat gac 1082
Leu Cys Phe Val Val Arg Ser Lys Val Asn Ile Tyr Cys Ser Asp Asp
    305                 310                 315

gga att tgg agt gag tgg agt gat aaa caa tgc tgg gaa ggt gaa gac 1130
Gly Ile Trp Ser Glu Trp Ser Asp Lys Gln Cys Trp Glu Gly Glu Asp
320                 325                 330                 335

cta tcg aag aaa act ttg cta cgt ttc tgg cta cca ttt ggt ttc atc 1178
Leu Ser Lys Lys Thr Leu Leu Arg Phe Trp Leu Pro Phe Gly Phe Ile
                340                 345                 350

tta ata tta gtt ata ttt gta acc ggt ctg ctt ttg cgt aag cca aac     1226
Leu Ile Leu Val Ile Phe Val Thr Gly Leu Leu Leu Arg Lys Pro Asn
        355                 360                 365

acc tac cca aaa atg att cca gaa ttt ttc tgt gat aca tga             1268
Thr Tyr Pro Lys Met Ile Pro Glu Phe Phe Cys Asp Thr
        370                 375                 380

agactttcca tatcaagaga catggtattg actcaacagt ttccagtcat ggccaaatgt   1328

tcaatatgag tctcaataaa ctgaattttt cttgcgaatg ttgaaaaa                1376

<210>  84
<211>  380
<212>  PRT
<213>  human

<400>  84

Met Ala Phe Val Cys Leu Ala Ile Gly Cys Leu Tyr Thr Phe Leu Ile
1               5                   10                  15

Ser Thr Thr Phe Gly Cys Thr Ser Ser Ser Asp Thr Glu Ile Lys Val
            20                  25                  30

Asn Pro Pro Gln Asp Phe Glu Ile Val Asp Pro Gly Tyr Leu Gly Tyr
        35                  40                  45

Leu Tyr Leu Gln Trp Gln Pro Pro Leu Ser Leu Asp His Phe Lys Glu
    50                  55                  60

Cys Thr Val Glu Tyr Glu Leu Lys Tyr Arg Asn Ile Gly Ser Glu Thr
65                  70                  75                  80

Trp Lys Thr Ile Ile Thr Lys Asn Leu His Tyr Lys Asp Gly Phe Asp
                85                  90                  95

Leu Asn Lys Gly Ile Glu Ala Lys Ile His Thr Leu Leu Pro Trp Gln
            100                 105                 110

Cys Thr Asn Gly Ser Glu Val Gln Ser Ser Trp Ala Glu Thr Thr Tyr
        115                 120                 125

Trp Ile Ser Pro Gln Gly Ile Pro Glu Thr Lys Val Gln Asp Met Asp
    130                 135                 140

Cys Val Tyr Tyr Asn Trp Gln Tyr Leu Leu Cys Ser Trp Lys Pro Gly
145                 150                 155                 160

Ile Gly Val Leu Leu Asp Thr Asn Tyr Asn Leu Phe Tyr Trp Tyr Glu
                165                 170                 175

Gly Leu Asp His Ala Leu Gln Cys Val Asp Tyr Ile Lys Ala Asp Gly
            180                 185                 190

Gln Asn Ile Gly Cys Arg Phe Pro Tyr Leu Glu Ala Ser Asp Tyr Lys
        195                 200                 205

Asp Phe Tyr Ile Cys Val Asn Gly Ser Ser Glu Asn Lys Pro Ile Arg
    210                 215                 220

Ser Ser Tyr Phe Thr Phe Gln Leu Gln Asn Ile Val Lys Pro Leu Pro
225                 230                 235                 240

Pro Val Tyr Leu Thr Phe Thr Arg Glu Ser Ser Cys Glu Ile Lys Leu
                245                 250                 255

Lys Trp Ser Ile Pro Leu Gly Pro Ile Pro Ala Arg Cys Phe Asp Tyr
            260                 265                 270

Glu Ile Glu Ile Arg Glu Asp Asp Thr Thr Leu Val Thr Ala Thr Val
        275                 280                 285

Glu Asn Glu Thr Tyr Thr Leu Lys Thr Thr Asn Glu Thr Arg Gln Leu
    290                 295                 300

Cys Phe Val Val Arg Ser Lys Val Asn Ile Tyr Cys Ser Asp Asp Gly
305                 310                 315                 320

Ile Trp Ser Glu Trp Ser Asp Lys Gln Cys Trp Glu Gly Glu Asp Leu
                325                 330                 335

Ser Lys Lys Thr Leu Leu Arg Phe Trp Leu Pro Phe Gly Phe Ile Leu
            340                 345                 350

Ile Leu Val Ile Phe Val Thr Gly Leu Leu Leu Arg Lys Pro Asn Thr
        355                 360                 365

Tyr Pro Lys Met Ile Pro Glu Phe Phe Cys Asp Thr
    370                 375                 380

<210>  85
<211>  2668
<212>  DNA
<213>  human

<220>
<221>  CDS
<222>  (485)..(2284)

<400>  85
ctctctggat aggaagaaat atagtagaac cctttgaaaa tggatatttt cacatatttt      60

cgttcagata caaaagctgg cagttactga aataaggact tgaagttcct tcctcttttt     120

tttatgtctt aagagcagga aataaagaga cagctgaagg tgtagccttg accaactgaa     180

agggaaatct tcatcctctg aaaaaacata tgtgattctc aaaaaacgca tctggaaaat     240

tgataaagaa gcgattctgt agattctccc agcgctgttg ggctctcaat tccttctgtg 300

aaggacaaca tatggtgatg gggaaatcag aagctttgag accctctaca cctggatatg 360

aatccccctt ctaatactta ccagaaatga aggggatact cagggcagag ttctgaatct 420

caaaacactc tactctggca aaggaatgaa gttattggag tgatgacagg aacacgggag 480

aaca atg ctc tgt ttg ggc tgg ata ttt ctt tgg ctt gtt gca gga gag 529
Met Leu Cys Leu Gly Trp Ile Phe Leu Trp Leu Val Ala Gly Glu
1               5                   10                  15

cga att aaa gga ttt aat att tca ggt tgt tcc aca aaa aaa ctc ctt 577
Arg Ile Lys Gly Phe Asn Ile Ser Gly Cys Ser Thr Lys Lys Leu Leu
                20                  25                  30

tgg aca tat tct aca agg agt gaa gag gaa ttt gtc tta ttt tgt gat 625
Trp Thr Tyr Ser Thr Arg Ser Glu Glu Glu Phe Val Leu Phe Cys Asp
            35                  40                  45

tta cca gag cca cag aaa tca cat ttc tgc cac aga aat cga ctc tca 673
Leu Pro Glu Pro Gln Lys Ser His Phe Cys His Arg Asn Arg Leu Ser
        50                  55                  60

cca aaa caa gtc cct gag cac ctg ccc ttc atg ggt agt aac gac cta 721
Pro Lys Gln Val Pro Glu His Leu Pro Phe Met Gly Ser Asn Asp Leu
    65                  70                  75

tct gat gtc caa tgg tac caa caa cct tcg aat gga gat cca tta gag 769
Ser Asp Val Gln Trp Tyr Gln Gln Pro Ser Asn Gly Asp Pro Leu Glu
80                  85                  90                  95

gac att agg aaa agc tat cct cac atc att cag gac aaa tgt acc ctt 817
Asp Ile Arg Lys Ser Tyr Pro His Ile Ile Gln Asp Lys Cys Thr Leu
                100                 105                 110

cac ttt ttg acc cca ggg gtg aat aat tct ggg tca tat att tgt aga 865
His Phe Leu Thr Pro Gly Val Asn Asn Ser Gly Ser Tyr Ile Cys Arg
            115                 120                 125

ccc aag atg att aag agc ccc tat gat gta gcc tgt tgt gtc aag atg 913
Pro Lys Met Ile Lys Ser Pro Tyr Asp Val Ala Cys Cys Val Lys Met
        130                 135                 140

att tta gaa gtt aag ccc cag aca aat gca tcc tgt gag tat tcc gca 961
Ile Leu Glu Val Lys Pro Gln Thr Asn Ala Ser Cys Glu Tyr Ser Ala
    145                 150                 155

tca cat aag caa gac cta ctt ctt ggg agc act ggc tct att tct tgc 1009
Ser His Lys Gln Asp Leu Leu Leu Gly Ser Thr Gly Ser Ile Ser Cys
160                 165                 170                 175

ccc agt ctc agc tgc caa agt gat gca caa agt cca gcg gta acc tgg 1057
Pro Ser Leu Ser Cys Gln Ser Asp Ala Gln Ser Pro Ala Val Thr Trp
                180                 185                 190

tac aag aat gga aaa ctc ctc tct gtg gaa agg agc aac cga atc gta 1105
Tyr Lys Asn Gly Lys Leu Leu Ser Val Glu Arg Ser Asn Arg Ile Val
            195                 200                 205

gtg gat gaa gtt tat gac tat cac cag ggc aca tat gta tgt gat tac 1153
Val Asp Glu Val Tyr Asp Tyr His Gln Gly Thr Tyr Val Cys Asp Tyr
        210                 215                 220

act cag tcg gat act gtg agt tcg tgg aca gtc aga gct gtt gtt caa 1201
Thr Gln Ser Asp Thr Val Ser Ser Trp Thr Val Arg Ala Val Val Gln
    225                 230                 235

gtg aga acc att gtg gga gac act aaa ctc aaa cca gat att ctg gat 1249
Val Arg Thr Ile Val Gly Asp Thr Lys Leu Lys Pro Asp Ile Leu Asp
240                 245                 250                 255

cct gtc gag gac aca ctg gaa gta gaa ctt gga aag cct tta act att 1297
Pro Val Glu Asp Thr Leu Glu Val Glu Leu Gly Lys Pro Leu Thr Ile
                260                 265                 270

agc tgc aaa gca cga ttt ggc ttt gaa agg gtc ttt aac cct gtc ata 1345
Ser Cys Lys Ala Arg Phe Gly Phe Glu Arg Val Phe Asn Pro Val Ile
            275                 280                 285

aaa tgg tac atc aaa gat tct gac cta gag tgg gaa gtc tca gta cct 1393
Lys Trp Tyr Ile Lys Asp Ser Asp Leu Glu Trp Glu Val Ser Val Pro
        290                 295                 300

gag gcg aaa agt att aaa tcc act tta aag gat gaa atc att gag cgt 1441
Glu Ala Lys Ser Ile Lys Ser Thr Leu Lys Asp Glu Ile Ile Glu Arg
    305                 310                 315

aat atc atc ttg gaa aaa gtc act cag cgt gat ctt cgc agg aag ttt 1489
Asn Ile Ile Leu Glu Lys Val Thr Gln Arg Asp Leu Arg Arg Lys Phe
320                 325                 330                 335

gtt tgc ttt gtc cag aac tcc att gga aac aca acc cag tcc gtc caa 1537
Val Cys Phe Val Gln Asn Ser Ile Gly Asn Thr Thr Gln Ser Val Gln
                340                 345                 350

ctg aaa gaa aag aga gga gtg gtg ctc ctg tac atc ctg ctt ggc acc 1585
Leu Lys Glu Lys Arg Gly Val Val Leu Leu Tyr Ile Leu Leu Gly Thr
            355                 360                 365

atc ggg acc ctg gtg gcc gtg ctg gcg gcg agt gcc ctc ctc tac agg 1633
Ile Gly Thr Leu Val Ala Val Leu Ala Ala Ser Ala Leu Leu Tyr Arg
        370                 375                 380

cac tgg att gaa ata gtg ctg ctg tac cgg acc tac cag agc aag gat 1681
His Trp Ile Glu Ile Val Leu Leu Tyr Arg Thr Tyr Gln Ser Lys Asp
    385                 390                 395

cag acg ctt ggg gat aaa aag gat ttt gat gct ttc gta tcc tat gca 1729
Gln Thr Leu Gly Asp Lys Lys Asp Phe Asp Ala Phe Val Ser Tyr Ala
400                 405                 410                 415

aaa tgg agc tct ttt cca agt gag gcc act tca tct ctg agt gaa gaa 1777
Lys Trp Ser Ser Phe Pro Ser Glu Ala Thr Ser Ser Leu Ser Glu Glu
                420                 425                 430

cac ttg gcc ctg agc cta ttt cct gat gtt tta gaa aac aaa tat gga 1825
His Leu Ala Leu Ser Leu Phe Pro Asp Val Leu Glu Asn Lys Tyr Gly
            435                 440                 445

tat agc ctg tgt ttg ctt gaa aga gat gtg gct cca gga gga gtg tat 1873
Tyr Ser Leu Cys Leu Leu Glu Arg Asp Val Ala Pro Gly Gly Val Tyr
        450                 455                 460

gca gaa gac att gtg agc att att aag aga agc aga aga gga ata ttt 1921
Ala Glu Asp Ile Val Ser Ile Ile Lys Arg Ser Arg Arg Gly Ile Phe
    465                 470                 475

atc ttg agc ccc aac tat gtc aat gga ccc agt atc ttt gaa cta caa 1969
Ile Leu Ser Pro Asn Tyr Val Asn Gly Pro Ser Ile Phe Glu Leu Gln
480                 485                 490                 495

gca gca gtg aat ctt gcc ttg gat gat caa aca ctg aaa ctc att tta 2017
Ala Ala Val Asn Leu Ala Leu Asp Asp Gln Thr Leu Lys Leu Ile Leu
                500                 505                 510

att aag ttc tgt tac ttc caa gag cca gag tct cta cct cat ctc gtg 2065
Ile Lys Phe Cys Tyr Phe Gln Glu Pro Glu Ser Leu Pro His Leu Val
            515                 520                 525

aaa aaa gct ctc agg gtt ttg ccc aca gtt act tgg aga ggc tta aaa 2113
Lys Lys Ala Leu Arg Val Leu Pro Thr Val Thr Trp Arg Gly Leu Lys
        530                 535                 540

tca gtt cct ccc aat tct agg ttc tgg gcc aaa atg cgc tac cac atg 2161
Ser Val Pro Pro Asn Ser Arg Phe Trp Ala Lys Met Arg Tyr His Met
    545                 550                 555

cct gtg aaa aac tct cag gga ttc acg tgg aac cag ctc aga att acc 2209
Pro Val Lys Asn Ser Gln Gly Phe Thr Trp Asn Gln Leu Arg Ile Thr
560                 565                 570                 575

tct agg att ttt cag tgg aaa gga ctc agt aga aca gaa acc act ggg 2257
Ser Arg Ile Phe Gln Trp Lys Gly Leu Ser Arg Thr Glu Thr Thr Gly
                580                 585                 590

agg agc tcc cag cct aag gaa tgg tga aatgagccct ggagccccct 2304
Arg Ser Ser Gln Pro Lys Glu Trp
            595

ccagtccagt ccctgggata gagatgttgc tggacagaac tcacagctct gtgtgtgtgt 2364

gttcaggctg ataggaaatt caaagagtct cctgccagca ccaagcaagc ttgatggaca 2424

atggagtggg attgagactg tggtttagag cctttgattt cctggactgg actgacggcg 2484

agtgaattct ctagaccttg ggtactttca gtacacaaca cccctaagat ttcccagtgg 2544

tccgagcaga atcagaaaat acagctactt ctgccttatg gctagggaac tgtcatgtct 2604

accatgtatt gtacatatga ctttatgtat acttgcaatc aaataaatat tattttatta 2664

gaaa 2668

<210> 86
<211> 599
<212> PRT
<213> human

<400> 86

Met Leu Cys Leu Gly Trp Ile Phe Leu Trp Leu Val Ala Gly Glu Arg
1               5                   10                  15

Ile Lys Gly Phe Asn Ile Ser Gly Cys Ser Thr Lys Lys Leu Leu Trp
            20                  25                  30

Thr Tyr Ser Thr Arg Ser Glu Glu Glu Phe Val Leu Phe Cys Asp Leu
        35                  40                  45

Pro Glu Pro Gln Lys Ser His Phe Cys His Arg Asn Arg Leu Ser Pro
    50                  55                  60

Lys Gln Val Pro Glu His Leu Pro Phe Met Gly Ser Asn Asp Leu Ser
65                  70                  75                  80

Asp Val Gln Trp Tyr Gln Gln Pro Ser Asn Gly Asp Pro Leu Glu Asp
                85                  90                  95

Ile Arg Lys Ser Tyr Pro His Ile Ile Gln Asp Lys Cys Thr Leu His
            100                 105                 110

Phe Leu Thr Pro Gly Val Asn Asn Ser Gly Ser Tyr Ile Cys Arg Pro
        115                 120                 125

Lys Met Ile Lys Ser Pro Tyr Asp Val Ala Cys Cys Val Lys Met Ile
    130                 135                 140

Leu Glu Val Lys Pro Gln Thr Asn Ala Ser Cys Glu Tyr Ser Ala Ser
145                 150                 155                 160

His Lys Gln Asp Leu Leu Leu Gly Ser Thr Gly Ser Ile Ser Cys Pro
                165                 170                 175

Ser Leu Ser Cys Gln Ser Asp Ala Gln Ser Pro Ala Val Thr Trp Tyr
            180                 185                 190

Lys Asn Gly Lys Leu Leu Ser Val Glu Arg Ser Asn Arg Ile Val Val
        195                 200                 205

Asp Glu Val Tyr Asp Tyr His Gln Gly Thr Tyr Val Cys Asp Tyr Thr
    210                 215                 220

Gln Ser Asp Thr Val Ser Ser Trp Thr Val Arg Ala Val Val Gln Val
225                 230                 235                 240

Arg Thr Ile Val Gly Asp Thr Lys Leu Lys Pro Asp Ile Leu Asp Pro
                245                 250                 255

Val Glu Asp Thr Leu Glu Val Glu Leu Gly Lys Pro Leu Thr Ile Ser
            260                 265                 270

Cys Lys Ala Arg Phe Gly Phe Glu Arg Val Phe Asn Pro Val Ile Lys
        275                 280                 285

Trp Tyr Ile Lys Asp Ser Asp Leu Glu Trp Glu Val Ser Val Pro Glu
    290                 295                 300

Ala Lys Ser Ile Lys Ser Thr Leu Lys Asp Glu Ile Ile Glu Arg Asn
305                 310                 315                 320

Ile Ile Leu Glu Lys Val Thr Gln Arg Asp Leu Arg Arg Lys Phe Val
                325                 330                 335

Cys Phe Val Gln Asn Ser Ile Gly Asn Thr Thr Gln Ser Val Gln Leu
            340                 345                 350

Lys Glu Lys Arg Gly Val Val Leu Leu Tyr Ile Leu Leu Gly Thr Ile
        355                 360                 365

Gly Thr Leu Val Ala Val Leu Ala Ala Ser Ala Leu Leu Tyr Arg His
    370                 375                 380

Trp Ile Glu Ile Val Leu Leu Tyr Arg Thr Tyr Gln Ser Lys Asp Gln
385                 390                 395                 400

Thr Leu Gly Asp Lys Lys Asp Phe Asp Ala Phe Val Ser Tyr Ala Lys
                405                 410                 415

Trp Ser Ser Phe Pro Ser Glu Ala Thr Ser Ser Leu Ser Glu Glu His
            420                 425                 430

Leu Ala Leu Ser Leu Phe Pro Asp Val Leu Glu Asn Lys Tyr Gly Tyr
        435                 440                 445

Ser Leu Cys Leu Leu Glu Arg Asp Val Ala Pro Gly Gly Val Tyr Ala
    450                 455                 460

Glu Asp Ile Val Ser Ile Ile Lys Arg Ser Arg Arg Gly Ile Phe Ile
465                 470                 475                 480

Leu Ser Pro Asn Tyr Val Asn Gly Pro Ser Ile Phe Glu Leu Gln Ala
                485                 490                 495

Ala Val Asn Leu Ala Leu Asp Asp Gln Thr Leu Lys Leu Ile Leu Ile
            500                 505                 510

Lys Phe Cys Tyr Phe Gln Glu Pro Glu Ser Leu Pro His Leu Val Lys
        515                 520                 525

Lys Ala Leu Arg Val Leu Pro Thr Val Thr Trp Arg Gly Leu Lys Ser
    530                 535                 540

Val Pro Pro Asn Ser Arg Phe Trp Ala Lys Met Arg Tyr His Met Pro
545                 550                 555                 560

Val Lys Asn Ser Gln Gly Phe Thr Trp Asn Gln Leu Arg Ile Thr Ser
                565                 570                 575

Arg Ile Phe Gln Trp Lys Gly Leu Ser Arg Thr Glu Thr Thr Gly Arg
            580                 585                 590

Ser Ser Gln Pro Lys Glu Trp
        595

<210>  87
<211>  3978
<212>  DNA
<213>  human

<220>
<221>  CDS
<222>  (164)..(1348)

<400>  87
ggcggcggcg gcggcggcgg ccccgggcgc tgagcgggtg cccggcgcgg agagcggcga      60
gcgcagccat gccccaggcc gcctccgggg cagcagcagc ggcggccggg gccgaggcgc     120
gggccggggg cgccgggggg ccggcggcgg cccgggcggg acg atg aag cgg cag       175
aac gtg cgc acg ctg gcg ctc atc gtg tgc acc ttc acc tac ctg ctg       223
gtg ggc gcc gcg gtc ttc gac gcg ctg gag tcg gag ccc gag ctg atc       271
gag cgg cag cgg ctg gag ctg cgg cag cag gag ctg cgg gcg cgc tac       319
aac ctc agc cag ggc ggc tac gag gag ctg gag cgc gtc gtg ctg cgc       367
ctc aag ccg cac aag gcc ggc gtg cag tgg cgc ttc gcc ggc tcc ttc       415
tac ttc gcc atc acc gtc atc acc acc atc ggc tac ggg cac gcg gca       463
ccc agc acg gat ggc ggc aag gtg ttc tgc atg ttc tac gcg ctg ctg       511
ggc atc ccg ctc acg ctc gtc atg ttc cag agc ctg ggc gag cgc atc       559
aac acc ttg gtg agg tac ctg ctg cac cgc gcc aag aag ggg ctg ggc       607
atg cgg cgc gcc gac gtg tcc atg gcc aac atg gtg ctc atc ggc ttc       655
ttc tcg tgc atc agc acg ctg tgc atc ggc gcc gcc gcc ttc tcc cac       703
tac gag cac tgg acc ttc ttc cag gcc tac tac tac tgc ttc atc acc       751
ctc acc acc atc ggc ttc ggc gac tac gtg gcg ctg cag aag gac cag       799
gcc ctg cag acg cag ccg cag tac gtg gcc ttc agc ttc gtc tac atc       847
ctt acg ggc ctc acg gtc atc ggc gcc ttc ctc aac ctc gtg gtg ctg 895
cgc ttc atg acc atg aac gcc gag gac gag aag cgc gac gcc gag cac 943
cgc gcg ctg ctc acg cgc aac ggg cag gcg ggc ggc ggc gga ggg ggt 991
ggc agc gcg cac act acg gac acc gcc tca tcc acg gcg gca gcg ggc 1039
ggc ggc ggc ttc cgc aac gtc tac gcg gag gtg ctg cac ttc cag tcc 1087
atg tgc tcg tgc ctg tgg tac aag agc cgc gag aag ctg cag tac tcc 1135
atc ccc atg atc atc ccg cgg gac ctc tcc acg tcc gac acg tgc gtg 1183
gag cag agc cac tcg tcg ccg gga ggg ggc ggc cgc tac agc gac acg 1231
ccc tcg cga cgc tgc ctg tgc agc ggg gcg cca cgc tcc gcc atc agc 1279
tcg gtg tcc acg ggt ctg cac agc ctg tcc acc ttc cgc ggc ctc atg 1327
aag cgc agg agc tcc gtg tga ctgccccgag gggcctggag cacctggggg 1378
cgcgggcggg ggacccctgc tgggaggcca ggagactgcc cctgctgcct tctgcccagt 1438
gggaccccgc acaacatccc tcaccactct cccccagcac ccccatctcc gactgtgcct 1498
gcttgcacca gccggcagga ggccgggctc tgaggacccc tggggccccc atcggagccc 1558
tgcaaattcc gagaaatgtg aaacttggtg gggtcaggga ggaaaggcag aagctgggag 1618
cctcccttcc ctttgaaaat ctaagaagct cccagtcctc agagaccctg ctggtaccca 1678
gacccccacc ttcggagggg acttcatgtt ccgtgtacgt ttgcatctct atttatacct 1738
ctgtcctgct aggtctccca ccttcccttg gttccaaaag ccagggtgtc tatgtccaag 1798
tcacccctac tcagccccac tccccttcct catccccagc tgtgtctccc aacctccctt 1858
cgtgttgttt tgcatggctt tgcagttatg gagaaagtgg aaacccagca gtccctaaag 1918
ctggtcccca gaaagcagga cagaaagaag gagggacagg caggcagcag gaggggcgag 1978
ctgggaggca ggaggcagcg gcctgtcagt ctgcagaatg gtcgcactgg aggttcaagc 2038
taactggcct ccagccacat tctcatagca ggtaggactt cagccttcca gacactgccc 2098
ttagaatctg gaacagaaga cttcagactc accataattg ctgataatta cccactctta 2158
aatttgtcga gtgattttta gcctctgaaa actctatgct ggccactgat tcctttgagt 2218
ctcacaaaac cctacttagg tcatcagggc aggagttctc actcccattt tacagatgag 2278
aatactgagg cctggacagg tgaagtgacc agagagcaaa aggcaaaggg gtgggggctg 2338
ggtgcagtgg ctcacacctg tattcccaac acttttggag gctgaggttg gaggattgct 2398
tgagcccagg aatttgagac cagcctaggt gacatagtga gaccccatct ctacaaaaaa 2458
taaaaaatta accaggtgtg gtggcacgtg cctgggagtc ccagcgactt gggaggctga 2518
ggtgggagga ttgtttgagc ctgggaggtc gaggctgtag tgagccctga ttgcaccact 2578
gtactccagc ctgggtgaca gggcaagacc ctgtctcaaa aaaaaaaaaa aaaatggcaa 2638
agggagacaa gagcccagcc tgcttgttgc tagccaaagt gttctttcct tccagcttgg 2698
cctgctctta aaagcaaagc tcctgcagtg tacatcctgg cattgtgtgg ctacctgggt 2758
tttaaaccag aatcagaagt cccggatcag agggcactgc tgaggttcag cctcttctct 2818
tcttggccag gaggcagcag ctctgaatgg gcccctgagg ctgcacaggg gcctttgtca 2878
ctggggcgca tgcttacaaa cagtgcagtt cttgggaccg aggtaagcag ggctgggtct 2938
catggcagaa aggccaggat ctggggctct aggaatttgg gaattgggca gagtggccaa 2998
gaaagctggc aggcatatcc tatgggacat cacacctggc accattgtca ttgttggtgc 3058
ctgtgtccca agtagctagt gataagctga ggctgcagca agaaacaccc ttcccaggtg 3118
ggggagtttg gaccagaggt gccctctgcc caccacacct gcaacccaga agcccagatg 3178
gaacgcagct gacgaaggtg atgcttgagg ctcacttttg gggccccaca gctggagccg 3238
gtataatgac tgggacaaca tcaaggggtg gatgaggggc ctctcctccc gcaacactgc 3298
cttcccatgc tgttcccctg ccagctcctt aacactgccg accaaggcca gccctggcat 3358
tcagggaaat tggagggcag cacccgtagg gtggccagcc tcaggcccca ccccagctgt 3418
gtcctctagt ctctggggac ccctgggggg aagaagtcta ccctgcttgt gagtcccgtc 3478
tcagtgtgga ggaactggct gcacgtggga cctgaaggtg ccctctgtgt ttatgttggg 3538
ggtggggggg cagtgctggc tgcctctgtc ctgtgtgtga ccctgccctc gaagggtcct 3598
gtcctgtcag tcccgaggga gccacaacca aagctgcgga gagaaggtgg ggaagggtgc 3658
agaatggccg tggggcacag cgtggcagac tgttcagtct ctgctgggtc tttcctaggg 3718
acctggaagg ccagtgttgc ttccccctca ctccctttca ctgcaggcag cctctctgct 3778
tccccaatgc cttatgcctg ggcacactgc cacagaatat gcaatatgtg tgggtgacca 3838
tgccctcacg accacacccc caccccgggc agcccccgga ctccaaaggt cgtggctgcc 3898
acagcctccc tcagctcttc ctgcctatct gtcttcacac tgagaatggc gcccaataaa 3958
tgctatccac ggagaccagg 3978

<210> 88
<211> 394

<212>  PRT
<213>  human

<400>  88

<210> 89
<211> 4564
<212> DNA
<213> human

<220>
<221> CDS
<222> (153)..(3551)

<400> 89
agtggcggcg gcggcgggtc cggaggtggc ggcaggtggc cccgcgcgcg gcggccggcc 60

cggccggggg cgggcgggaa ggtggcgcct cgggcggggg ccggtccctg caccaggtga 120

cctgttccgg cccggatccg ggcggcctcg cc atg cag cgg cgc ggc gcg ggg 173
Met Gln Arg Arg Gly Ala Gly
1               5

ctc ggg tgg ccg cgg cag cag cag cag caa ccc ccg ccg ctc gcg gtc 221
Leu Gly Trp Pro Arg Gln Gln Gln Gln Gln Pro Pro Pro Leu Ala Val
        10                  15                  20

ggc ccc cgg gcc gca gcc atg gtc ccg agt ggc ggc gtc ccc cag ggc 269
Gly Pro Arg Ala Ala Ala Met Val Pro Ser Gly Gly Val Pro Gln Gly
    25                  30                  35

ctc ggc ggc cgc tct gcc tgc gcg ctg ctc ctg ctc tgc tac ctg aat 317
Leu Gly Gly Arg Ser Ala Cys Ala Leu Leu Leu Leu Cys Tyr Leu Asn
40                  45                  50                  55

gtt gta cca tct ttg ggt agg cag act tcc ctg acg aca tca gtg ata 365
Val Val Pro Ser Leu Gly Arg Gln Thr Ser Leu Thr Thr Ser Val Ile
                60                  65                  70

ccc aaa gct gag cag agc gtg gct tac aaa gac ttt att tat ttt act 413
Pro Lys Ala Glu Gln Ser Val Ala Tyr Lys Asp Phe Ile Tyr Phe Thr
            75                  80                  85

gtc ttt gaa gga aac gtt cgc aac gtt tct gaa gtc tcg gtt gag tat 461
Val Phe Glu Gly Asn Val Arg Asn Val Ser Glu Val Ser Val Glu Tyr
        90                  95                  100

tta tgc tct cag cct tgt gtt gtc aat ttg gaa gca gtt gtt tca tct 509
Leu Cys Ser Gln Pro Cys Val Val Asn Leu Glu Ala Val Val Ser Ser
    105                 110                 115

gag ttc aga agt agc att ccc gtg tac aaa aaa agg tgg aag aat gag 557
Glu Phe Arg Ser Ser Ile Pro Val Tyr Lys Lys Arg Trp Lys Asn Glu
120                 125                 130                 135

aaa cat ctt cac acc agc agg aca caa ata gta cat gtg aaa ttt cca 605
Lys His Leu His Thr Ser Arg Thr Gln Ile Val His Val Lys Phe Pro
                140                 145                 150

agc att atg gtt tac aga gat gat tat ttc atc aga cat tcc atc tct 653
Ser Ile Met Val Tyr Arg Asp Asp Tyr Phe Ile Arg His Ser Ile Ser
            155                 160                 165

gta tct gca gtg ata gta cgc gcc tgg att act cac aaa tac agt ggc 701
Val Ser Ala Val Ile Val Arg Ala Trp Ile Thr His Lys Tyr Ser Gly
        170                 175                 180

aga gac tgg aat gtt aaa tgg gag gaa aac ttg ctc cat gct gta gca 749
Arg Asp Trp Asn Val Lys Trp Glu Glu Asn Leu Leu His Ala Val Ala
    185                 190                 195

aag aat tat acc ctc ctg cag acc atc ccg cct ttt gaa cgc cct ttc 797
Lys Asn Tyr Thr Leu Leu Gln Thr Ile Pro Pro Phe Glu Arg Pro Phe
200                 205                 210                 215

aaa gat cat caa gtg tgc ctt gag tgg aac atg ggt tat att tgg aac 845
Lys Asp His Gln Val Cys Leu Glu Trp Asn Met Gly Tyr Ile Trp Asn
                220                 225                 230

ctt cgg gca aac agg att cca cag tgt cct ctg gaa aat gat gtg gtt 893
Leu Arg Ala Asn Arg Ile Pro Gln Cys Pro Leu Glu Asn Asp Val Val
            235                 240                 245

gcc ctg ctt ggc ttt cct tat gcc tcc agt gga gaa aac aca ggc att 941
Ala Leu Leu Gly Phe Pro Tyr Ala Ser Ser Gly Glu Asn Thr Gly Ile
        250                 255                 260

gtc aag aag ttc ccg agg ttt cgg aac cga gag ctg gag gcc act cga 989
Val Lys Lys Phe Pro Arg Phe Arg Asn Arg Glu Leu Glu Ala Thr Arg
    265                 270                 275

cgc cag agg atg gat tac cca gtg ttt act gtt tca ttg tgg ctt tat 1037
Arg Gln Arg Met Asp Tyr Pro Val Phe Thr Val Ser Leu Trp Leu Tyr
280                 285                 290                 295

tta ctc cat tat tgc aag gcc aac ctc tgt ggg att ctg tac ttt gtt 1085
Leu Leu His Tyr Cys Lys Ala Asn Leu Cys Gly Ile Leu Tyr Phe Val
                300                 305                 310

gac tct aat gag atg tac ggc aca cct tct gta ttt ctt acg gaa gag 1133
Asp Ser Asn Glu Met Tyr Gly Thr Pro Ser Val Phe Leu Thr Glu Glu
            315                 320                 325

ggc tat ttg cat att cag atg cat ctt gtc aaa ggg gaa gac ctt gct 1181
Gly Tyr Leu His Ile Gln Met His Leu Val Lys Gly Glu Asp Leu Ala
        330                 335                 340

gta aaa act aaa ttc atc ata cct ttg aag gag tgg ttt cga ctg gat 1229
Val Lys Thr Lys Phe Ile Ile Pro Leu Lys Glu Trp Phe Arg Leu Asp
    345                 350                 355

atc tct ttt aac gga ggc cag ata gta gta acc act agc att gga cag 1277
Ile Ser Phe Asn Gly Gly Gln Ile Val Val Thr Thr Ser Ile Gly Gln
360                 365                 370                 375

gat ttg aaa agc tac cac aat cag acc att agc ttc cgg gag gat ttc 1325
Asp Leu Lys Ser Tyr His Asn Gln Thr Ile Ser Phe Arg Glu Asp Phe
                380                 385                 390

cat tat aat gac aca gct ggg tac ttc att att gga ggg agc agg tat 1373
His Tyr Asn Asp Thr Ala Gly Tyr Phe Ile Ile Gly Gly Ser Arg Tyr
            395                 400                 405

gtg gct ggc att gaa ggg ttt ttt gga ccc ctg aag tac tat cgc ctt 1421
Val Ala Gly Ile Glu Gly Phe Phe Gly Pro Leu Lys Tyr Tyr Arg Leu
        410                 415                 420

cgc agt ctg cac ccc gcc cag att ttt aat ccc ctc ctt gag aag caa 1469
Arg Ser Leu His Pro Ala Gln Ile Phe Asn Pro Leu Leu Glu Lys Gln
    425                 430                 435

ctt gct gaa caa atc aag tta tat tat gaa agg tgt gct gag gtt caa 1517
Leu Ala Glu Gln Ile Lys Leu Tyr Tyr Glu Arg Cys Ala Glu Val Gln
440                 445                 450                 455

gaa ata gta tct gtg tat gca tct gca gca aag cac ggg ggc gag aga 1565
Glu Ile Val Ser Val Tyr Ala Ser Ala Ala Lys His Gly Gly Glu Arg
                460                 465                 470

caa gaa gca tgc cac ctc cac aac tcc tac ctg gac ctc cag cgc agg 1613
Gln Glu Ala Cys His Leu His Asn Ser Tyr Leu Asp Leu Gln Arg Arg
            475                 480                 485

tat ggg aga ccc tcg atg tgc aga gcc ttc ccc tgg gag aag gag ctg 1661
Tyr Gly Arg Pro Ser Met Cys Arg Ala Phe Pro Trp Glu Lys Glu Leu
        490                 495                 500

aaa gac aaa cac ccc agc ttg ttc cag gca ttg ctg gag atg gat ctg 1709
Lys Asp Lys His Pro Ser Leu Phe Gln Ala Leu Leu Glu Met Asp Leu
    505                 510                 515

ctg acc gtg cca agg aac caa aat gaa tct gta tca gaa atc ggt ggg 1757
Leu Thr Val Pro Arg Asn Gln Asn Glu Ser Val Ser Glu Ile Gly Gly
520                 525                 530                 535

aag ata ttt gag aag gct gta aag aga ctc tct agc att gat ggt ctt 1805
Lys Ile Phe Glu Lys Ala Val Lys Arg Leu Ser Ser Ile Asp Gly Leu
                540                 545                 550

cac caa att agc tct atc gtc ccc ttt ctg acg gat tcc agc tgc tgt 1853
His Gln Ile Ser Ser Ile Val Pro Phe Leu Thr Asp Ser Ser Cys Cys
            555                 560                 565

gga tac cat aaa gca tcc tac tac ctt gca gtc ttt tat gag act gga 1901
Gly Tyr His Lys Ala Ser Tyr Tyr Leu Ala Val Phe Tyr Glu Thr Gly
        570                 575                 580

tta aat gtt cct cgg gat cag ctg cag ggc atg ttg tat agt ttg gtt 1949
Leu Asn Val Pro Arg Asp Gln Leu Gln Gly Met Leu Tyr Ser Leu Val
    585                 590                 595

gga ggc cag ggg agt gag agg ctg tct tca atg aat ctt ggg tat aaa 1997
Gly Gly Gln Gly Ser Glu Arg Leu Ser Ser Met Asn Leu Gly Tyr Lys
600                 605                 610                 615

cac tac cag ggt att gac aac tac ccc ctg gac tgg gaa ctg tcg tat 2045
His Tyr Gln Gly Ile Asp Asn Tyr Pro Leu Asp Trp Glu Leu Ser Tyr
                620                 625                 630

gcc tac tac agc aac att gcc acc aag aca ccc ctt gac cag cac aca 2093
Ala Tyr Tyr Ser Asn Ile Ala Thr Lys Thr Pro Leu Asp Gln His Thr
            635                 640                 645

ctg caa gga gat cag gca tat gtt gaa aca att aga cta aaa gat gat 2141
Leu Gln Gly Asp Gln Ala Tyr Val Glu Thr Ile Arg Leu Lys Asp Asp
        650                 655                 660

gaa ata ctc aag gta caa acc aaa gaa gat gga gat gtc ttt atg tgg 2189
Glu Ile Leu Lys Val Gln Thr Lys Glu Asp Gly Asp Val Phe Met Trp
    665                 670                 675

ttg aag cat gaa gct acc cga ggc aat gca gca gct cag caa cga ttg 2237
Leu Lys His Glu Ala Thr Arg Gly Asn Ala Ala Ala Gln Gln Arg Leu
680                 685                 690                 695

gcc cag atg ctg ttc tgg ggg cag caa ggt gtg gcc aag aat ccc gaa 2285
Ala Gln Met Leu Phe Trp Gly Gln Gln Gly Val Ala Lys Asn Pro Glu
                700                 705                 710

gca gca att gag tgg tac gcc aag ggc gcc ctg gag acg gag gat cct 2333
Ala Ala Ile Glu Trp Tyr Ala Lys Gly Ala Leu Glu Thr Glu Asp Pro
            715                 720                 725

gcg tta atc tat gac tat gcc att gtg cta ttc aag ggt caa gga gta 2381
Ala Leu Ile Tyr Asp Tyr Ala Ile Val Leu Phe Lys Gly Gln Gly Val
        730                 735                 740

aaa aag aac aga cgg ctt gcc tta gag ctg atg aag aaa gca gct tcc 2429
Lys Lys Asn Arg Arg Leu Ala Leu Glu Leu Met Lys Lys Ala Ala Ser
    745                 750                 755

aag gga ttg cat cag gca gtc aat ggc ctg gga tgg tat tac cac aaa 2477
Lys Gly Leu His Gln Ala Val Asn Gly Leu Gly Trp Tyr Tyr His Lys
760                 765                 770                 775

ttc aag aaa aat tac gcc aaa gca gca aag tac tgg tta aaa gca gaa 2525
Phe Lys Lys Asn Tyr Ala Lys Ala Ala Lys Tyr Trp Leu Lys Ala Glu
                780                 785                 790

gaa atg ggg aac cca gat gcg tca tac aat ctt gga gtc ctg cat ttg 2573
Glu Met Gly Asn Pro Asp Ala Ser Tyr Asn Leu Gly Val Leu His Leu
            795                 800                 805

gat ggc atc ttc cct gga gtt cct gga agg aat caa act tta gct ggt 2621
Asp Gly Ile Phe Pro Gly Val Pro Gly Arg Asn Gln Thr Leu Ala Gly
        810                 815                 820

gaa tat ttc cat aag gct gcg caa ggt gga cac atg gaa ggg acc ttg 2669
Glu Tyr Phe His Lys Ala Ala Gln Gly Gly His Met Glu Gly Thr Leu
    825                 830                 835

tgg tgt tct ctc tac tat atc aca ggc aac ctg gag aca ttc cct aga 2717
Trp Cys Ser Leu Tyr Tyr Ile Thr Gly Asn Leu Glu Thr Phe Pro Arg
840                 845                 850                 855

gat cct gag aaa gct gtt gta tgg gca aaa cat gta gct gag aaa aat 2765
Asp Pro Glu Lys Ala Val Val Trp Ala Lys His Val Ala Glu Lys Asn
                860                 865                 870

ggc tac ttg ggc cat gtc atc cgc aaa ggc ctc aat gcc tac ctg gaa 2813
Gly Tyr Leu Gly His Val Ile Arg Lys Gly Leu Asn Ala Tyr Leu Glu
            875                 880                 885

ggt tca tgg cat gaa gct ttg ctg tat tat gtt tta gca gca gaa act 2861
Gly Ser Trp His Glu Ala Leu Leu Tyr Tyr Val Leu Ala Ala Glu Thr
        890                 895                 900

gga att gaa gtg tca cag aca aat tta gca cac atc tgt gag gag agg 2909
Gly Ile Glu Val Ser Gln Thr Asn Leu Ala His Ile Cys Glu Glu Arg
    905                 910                 915

cca gac ctg gcc agg aga tac ttg ggt gtt aac tgt gtt tgg aga tac 2957
Pro Asp Leu Ala Arg Arg Tyr Leu Gly Val Asn Cys Val Trp Arg Tyr
920                 925                 930                 935

tat aat ttc tct gtt ttt caa atc gat gct cct tcc ttt gca tat ttg 3005
Tyr Asn Phe Ser Val Phe Gln Ile Asp Ala Pro Ser Phe Ala Tyr Leu
                940                 945                 950

aag atg gga gac ctt tac tac tat ggc cac caa aac cag tca caa gac 3053
Lys Met Gly Asp Leu Tyr Tyr Tyr Gly His Gln Asn Gln Ser Gln Asp
            955                 960                 965

ctg gag ttg tct gtg cag atg tac gcc caa gcc gcc ctg gat gga gac 3101
Leu Glu Leu Ser Val Gln Met Tyr Ala Gln Ala Ala Leu Asp Gly Asp
        970                 975                 980

tcc cag gga ttt ttt aac ctg gcc ctg cta atc gag gaa ggt acg ata 3149
Ser Gln Gly Phe Phe Asn Leu Ala Leu Leu Ile Glu Glu Gly Thr Ile
    985                 990                 995

atc cca cac cat atc ttg gat ttc ttg gaa att gac tca act ctc 3194
Ile Pro His His Ile Leu Asp Phe Leu Glu Ile Asp Ser Thr Leu
1000                1005                1010

cat tct aat aac atc tcc att ctc cag gaa ctg tac gaa agg tgc 3239
His Ser Asn Asn Ile Ser Ile Leu Gln Glu Leu Tyr Glu Arg Cys
1015                1020                1025

tgg agc cac agt aac gag gag tcc ttc agc ccc tgc tcc ttg gcc 3284
Trp Ser His Ser Asn Glu Glu Ser Phe Ser Pro Cys Ser Leu Ala
1030                1035                1040

tgg ctt tac ctg cac ttg cgg ctt ctc tgg ggt gct atc ctg cac 3329
Trp Leu Tyr Leu His Leu Arg Leu Leu Trp Gly Ala Ile Leu His
1045                1050                1055

tca gcc ctg atc tac ttt ctg gga acc ttt ctg cta tcc ata ttg 3374
Ser Ala Leu Ile Tyr Phe Leu Gly Thr Phe Leu Leu Ser Ile Leu
1060                1065                1070

atc gcc tgg act gtg cag tat ttc cag tct gtc tca gca agc gat 3419
Ile Ala Trp Thr Val Gln Tyr Phe Gln Ser Val Ser Ala Ser Asp
1075                1080                1085

ccc cct cca aga cca tcc cag gcc tcc cca gac act gcc acg tcc 3464
Pro Pro Pro Arg Pro Ser Gln Ala Ser Pro Asp Thr Ala Thr Ser
1090                1095                1100

act gca agt cca gct gtg act cca gct gca gat gcc tct gac caa 3509
Thr Ala Ser Pro Ala Val Thr Pro Ala Ala Asp Ala Ser Asp Gln
1105                1110                1115

gac cag ccc aca gta act aat aac ccg gag cca cgt ggg tga 3551
Asp Gln Pro Thr Val Thr Asn Asn Pro Glu Pro Arg Gly
1120                1125                1130

actgtgcact ccagttctct ccagatgaga gagaatcttt tcaacagctg gtattgggaa 3611

gctggggcca gggcatgatc ctgataaaca ccttaaatgt cttgtcaact ggatgcaaat 3671

tttgcaattg gtgtcatttt ttttaaagtc aaattacaag gaagtaccca gatcaggcag 3731

tggtaatacc aaaggtcatc aaacacatac aaggaacatc ttgatcatag ggcatgtggg 3791

gaagtttact gggccatcac agacttttgt tctagtgatt gtatgtatta ggagtcatag 3851

catgccctac ggcagatctg gattcttata cactaagatg tgtcttaaga atcacagtgc 3911

gtgcttcatc cctttattga agaacagaaa attatgacta ctctacaagg tggataatat 3971

tttggtacct gtgcttgcca cagccctgtt cctcaaagct gaattgatag atttctcttt 4031

gacttccaag acctagcagt tataaggcac cttgaaataa attgtttgtg cctggaaatg 4091

cagggagggc aatagctttg taaattggtt tacatttttc tccttgaatt tttctagggt 4151

cctagtgctt ccgaatcatt taatggcatt gtcggatatc ttttacattt caattgcaat 4211

ccatgaaatt acatttagaa gattcttagt acttaactgt agtcttctcc atgaattaca 4271

cgttagaata gactggcagc aacggaatat gcagcaagta agcctctagc ttatagtttc 4331

atccctaccc ctcatgcctg cgtgagtctg tacagggata tgtgtgtgtg tgtgtgtgtg 4391

tgtgtgttag agaggaagag gaagagcaga atgtctgtat actacatgct gctaaggtag 4451

tgaataaatc agtaatgcaa tattgtgggt ccaaactact ctttgcacta ctttatttac 4511

agtagtaaat aaaattattt ttatacaatt gactaccaaa aaaaaaaaaa aaa 4564

<210> 90
<211> 1132
<212> PRT
<213> human

<400> 90

Met Gln Arg Arg Gly Ala Gly Leu Gly Trp Pro Arg Gln Gln Gln Gln
1               5                   10                  15

Gln Pro Pro Pro Leu Ala Val Gly Pro Arg Ala Ala Ala Met Val Pro
            20                  25                  30

Ser Gly Gly Val Pro Gln Gly Leu Gly Gly Arg Ser Ala Cys Ala Leu
        35                  40                  45

Leu Leu Leu Cys Tyr Leu Asn Val Val Pro Ser Leu Gly Arg Gln Thr
    50                  55                  60

Ser Leu Thr Thr Ser Val Ile Pro Lys Ala Glu Gln Ser Val Ala Tyr
65                  70                  75                  80

Lys Asp Phe Ile Tyr Phe Thr Val Phe Glu Gly Asn Val Arg Asn Val
                85                  90                  95

Ser Glu Val Ser Val Glu Tyr Leu Cys Ser Gln Pro Cys Val Val Asn
            100                 105                 110

Leu Glu Ala Val Val Ser Ser Glu Phe Arg Ser Ser Ile Pro Val Tyr
        115                 120                 125

Lys Lys Arg Trp Lys Asn Glu Lys His Leu His Thr Ser Arg Thr Gln
    130                 135                 140

Ile Val His Val Lys Phe Pro Ser Ile Met Val Tyr Arg Asp Asp Tyr
145                 150                 155                 160

Phe Ile Arg His Ser Ile Ser Val Ser Ala Val Ile Val Arg Ala Trp
                165                 170                 175

Ile Thr His Lys Tyr Ser Gly Arg Asp Trp Asn Val Lys Trp Glu Glu
            180                 185                 190

Asn Leu Leu His Ala Val Ala Lys Asn Tyr Thr Leu Leu Gln Thr Ile
        195                 200                 205

Pro Pro Phe Glu Arg Pro Phe Lys Asp His Gln Val Cys Leu Glu Trp
    210                 215                 220

Asn Met Gly Tyr Ile Trp Asn Leu Arg Ala Asn Arg Ile Pro Gln Cys
225                 230                 235                 240

Pro Leu Glu Asn Asp Val Val Ala Leu Leu Gly Phe Pro Tyr Ala Ser
                245                 250                 255

Ser Gly Glu Asn Thr Gly Ile Val Lys Lys Phe Pro Arg Phe Arg Asn
            260                 265                 270

Arg Glu Leu Glu Ala Thr Arg Arg Gln Arg Met Asp Tyr Pro Val Phe
        275                 280                 285

Thr Val Ser Leu Trp Leu Tyr Leu Leu His Tyr Cys Lys Ala Asn Leu
    290                 295                 300

Cys Gly Ile Leu Tyr Phe Val Asp Ser Asn Glu Met Tyr Gly Thr Pro
305                 310                 315                 320

Ser Val Phe Leu Thr Glu Glu Gly Tyr Leu His Ile Gln Met His Leu
                325                 330                 335

Val Lys Gly Glu Asp Leu Ala Val Lys Thr Lys Phe Ile Ile Pro Leu
            340                 345                 350

Lys Glu Trp Phe Arg Leu Asp Ile Ser Phe Asn Gly Gly Gln Ile Val
        355                 360                 365

Val Thr Thr Ser Ile Gly Gln Asp Leu Lys Ser Tyr His Asn Gln Thr
    370                 375                 380

Ile Ser Phe Arg Glu Asp Phe His Tyr Asn Asp Thr Ala Gly Tyr Phe
385                 390                 395                 400

Ile Ile Gly Gly Ser Arg Tyr Val Ala Gly Ile Glu Gly Phe Phe Gly
                405                 410                 415

Pro Leu Lys Tyr Tyr Arg Leu Arg Ser Leu His Pro Ala Gln Ile Phe
            420                 425                 430

Asn Pro Leu Leu Glu Lys Gln Leu Ala Glu Gln Ile Lys Leu Tyr Tyr
        435                 440                 445

Glu Arg Cys Ala Glu Val Gln Glu Ile Val Ser Val Tyr Ala Ser Ala
    450                 455                 460

Ala Lys His Gly Gly Glu Arg Gln Glu Ala Cys His Leu His Asn Ser
465                 470                 475                 480

Tyr Leu Asp Leu Gln Arg Arg Tyr Gly Arg Pro Ser Met Cys Arg Ala
                485                 490                 495

Phe Pro Trp Glu Lys Glu Leu Lys Asp Lys His Pro Ser Leu Phe Gln
            500                 505                 510

Ala Leu Leu Glu Met Asp Leu Leu Thr Val Pro Arg Asn Gln Asn Glu
        515                 520                 525

Ser Val Ser Glu Ile Gly Gly Lys Ile Phe Glu Lys Ala Val Lys Arg
    530                 535                 540

Leu Ser Ser Ile Asp Gly Leu His Gln Ile Ser Ser Ile Val Pro Phe
545                 550                 555                 560

Leu Thr Asp Ser Ser Cys Cys Gly Tyr His Lys Ala Ser Tyr Tyr Leu
                565                 570                 575

Ala Val Phe Tyr Glu Thr Gly Leu Asn Val Pro Arg Asp Gln Leu Gln
            580                 585                 590

Gly Met Leu Tyr Ser Leu Val Gly Gly Gln Gly Ser Glu Arg Leu Ser
        595                 600                 605

Ser Met Asn Leu Gly Tyr Lys His Tyr Gln Gly Ile Asp Asn Tyr Pro
    610                 615                 620

Leu Asp Trp Glu Leu Ser Tyr Ala Tyr Tyr Ser Asn Ile Ala Thr Lys
625                 630                 635                 640

Thr Pro Leu Asp Gln His Thr Leu Gln Gly Asp Gln Ala Tyr Val Glu
                645                 650                 655

Thr Ile Arg Leu Lys Asp Asp Glu Ile Leu Lys Val Gln Thr Lys Glu
            660                 665                 670

Asp Gly Asp Val Phe Met Trp Leu Lys His Glu Ala Thr Arg Gly Asn
        675                 680                 685

Ala Ala Ala Gln Gln Arg Leu Ala Gln Met Leu Phe Trp Gly Gln Gln
    690                 695                 700

Gly Val Ala Lys Asn Pro Glu Ala Ala Ile Glu Trp Tyr Ala Lys Gly
705                 710                 715                 720

Ala Leu Glu Thr Glu Asp Pro Ala Leu Ile Tyr Asp Tyr Ala Ile Val
                725                 730                 735

Leu Phe Lys Gly Gln Gly Val Lys Lys Asn Arg Arg Leu Ala Leu Glu
            740                 745                 750

Leu Met Lys Lys Ala Ala Ser Lys Gly Leu His Gln Ala Val Asn Gly
        755                 760                 765

Leu Gly Trp Tyr Tyr His Lys Phe Lys Lys Asn Tyr Ala Lys Ala Ala
    770                 775                 780

Lys Tyr Trp Leu Lys Ala Glu Glu Met Gly Asn Pro Asp Ala Ser Tyr
785                 790                 795                 800

Asn Leu Gly Val Leu His Leu Asp Gly Ile Phe Pro Gly Val Pro Gly
                805                 810                 815

Arg Asn Gln Thr Leu Ala Gly Glu Tyr Phe His Lys Ala Ala Gln Gly
            820                 825                 830

Gly His Met Glu Gly Thr Leu Trp Cys Ser Leu Tyr Tyr Ile Thr Gly
        835                 840                 845

Asn Leu Glu Thr Phe Pro Arg Asp Pro Glu Lys Ala Val Val Trp Ala
    850                 855                 860

Lys His Val Ala Glu Lys Asn Gly Tyr Leu Gly His Val Ile Arg Lys
865                 870                 875                 880

Gly Leu Asn Ala Tyr Leu Glu Gly Ser Trp His Glu Ala Leu Leu Tyr
                885                 890                 895

Tyr Val Leu Ala Ala Glu Thr Gly Ile Glu Val Ser Gln Thr Asn Leu
            900                 905                 910

Ala His Ile Cys Glu Glu Arg Pro Asp Leu Ala Arg Arg Tyr Leu Gly
        915                 920                 925

Val Asn Cys Val Trp Arg Tyr Tyr Asn Phe Ser Val Phe Gln Ile Asp
    930                 935                 940

Ala Pro Ser Phe Ala Tyr Leu Lys Met Gly Asp Leu Tyr Tyr Tyr Gly
945                 950                 955                 960

His Gln Asn Gln Ser Gln Asp Leu Glu Leu Ser Val Gln Met Tyr Ala
                965                 970                 975

Gln Ala Ala Leu Asp Gly Asp Ser Gln Gly Phe Phe Asn Leu Ala Leu
            980                 985                 990

Leu Ile Glu Glu Gly Thr Ile Ile  Pro His His Ile Leu  Asp Phe Leu
        995                 1000                 1005

Glu Ile  Asp Ser Thr Leu His  Ser Asn Asn Ile Ser  Ile Leu Gln
    1010                 1015                 1020

Glu Leu  Tyr Glu Arg Cys Trp  Ser His Ser Asn Glu  Glu Ser Phe
    1025                 1030                 1035

Ser Pro  Cys Ser Leu Ala Trp  Leu Tyr Leu His Leu  Arg Leu Leu
    1040                 1045                 1050

Trp Gly  Ala Ile Leu His Ser  Ala Leu Ile Tyr Phe  Leu Gly Thr
    1055                 1060                 1065

Phe Leu  Leu Ser Ile Leu Ile  Ala Trp Thr Val Gln  Tyr Phe Gln
    1070                 1075                 1080

Ser Val  Ser Ala Ser Asp Pro  Pro Pro Arg Pro Ser  Gln Ala Ser
    1085                 1090                 1095

Pro Asp  Thr Ala Thr Ser Thr  Ala Ser Pro Ala Val  Thr Pro Ala
    1100                 1105                 1110

Ala Asp  Ala Ser Asp Gln Asp  Gln Pro Thr Val Thr  Asn Asn Pro
    1115                 1120                 1125

Glu Pro  Arg Gly
    1130

<210>  91
<211>  1691
<212>  DNA
<213>  human

<220>
<221>  CDS
<222>  (19)..(1251)

<400>  91
gtctgcaccg gcagcacc atg tcg ctc atg gtc gtc agc atg gcg tgt gtt      51
                    Met Ser Leu Met Val Val Ser Met Ala Cys Val
                    1               5                   10

ggg ttc ttc ttg ctg gag ggg ccc tgg cca cat gtg ggt ggt cag gac      99
Gly Phe Phe Leu Leu Glu Gly Pro Trp Pro His Val Gly Gly Gln Asp
            15                  20                  25

aag ccc ttc ctc tct gcc tgg ccc ggc act gtg gtg tct gaa gga caa      147
Lys Pro Phe Leu Ser Ala Trp Pro Gly Thr Val Val Ser Glu Gly Gln
        30                  35                  40

cat gtg act ctt cag tgt cgc tct cgt ctt ggg ttt aac gaa ttc agt      195
His Val Thr Leu Gln Cys Arg Ser Arg Leu Gly Phe Asn Glu Phe Ser
    45                  50                  55

ctg tcc aaa gaa gac ggg atg cct gtc cct gag ctc tac aac aga ata      243
Leu Ser Lys Glu Asp Gly Met Pro Val Pro Glu Leu Tyr Asn Arg Ile
60                  65                  70                  75

ttc cgg aac agc ttt ctc atg ggc cct gtg acc cca gca cat gca ggg      291
Phe Arg Asn Ser Phe Leu Met Gly Pro Val Thr Pro Ala His Ala Gly
                80                  85                  90

acc tac aga tgt tgc agt tca cac cca cac tcc ccc act ggg tgg tcg      339
Thr Tyr Arg Cys Cys Ser Ser His Pro His Ser Pro Thr Gly Trp Ser
            95                  100                 105

gca ccc agc aac cct gtg gtg atc atg gtc aca gga gtc cac aga aaa      387
Ala Pro Ser Asn Pro Val Val Ile Met Val Thr Gly Val His Arg Lys
        110                 115                 120

cct tcc ctc ctg gcc cac cca ggt ccc ctg gtg aaa tcg gga gag acg      435
Pro Ser Leu Leu Ala His Pro Gly Pro Leu Val Lys Ser Gly Glu Thr
    125                 130                 135

gtc atc ctg caa tgt tgg tca gat gtc agg ttt gag cgc ttc ctt ctg      483
Val Ile Leu Gln Cys Trp Ser Asp Val Arg Phe Glu Arg Phe Leu Leu
140                 145                 150                 155

cac aga gag ggg atc act gag gac ccc ttg cgc ctc gtt gga cag ctc      531
His Arg Glu Gly Ile Thr Glu Asp Pro Leu Arg Leu Val Gly Gln Leu
                160                 165                 170

cac gat gcg ggt tcc cag gtc aac tat tcc atg ggt ccc atg aca cct      579
His Asp Ala Gly Ser Gln Val Asn Tyr Ser Met Gly Pro Met Thr Pro
            175                 180                 185

gcc ctt gca ggg acc tac aga tgc ttt ggt tct gtc act cac tta ccc      627
Ala Leu Ala Gly Thr Tyr Arg Cys Phe Gly Ser Val Thr His Leu Pro
        190                 195                 200

tat gag ttg tcg gct ccc agt gac cct ctg gac atc gtg gtc gta ggt      675
Tyr Glu Leu Ser Ala Pro Ser Asp Pro Leu Asp Ile Val Val Val Gly
    205                 210                 215

cta tat ggg aaa cct tct ctc tca gcc cag ccg ggc ccc acg gtt cag      723
Leu Tyr Gly Lys Pro Ser Leu Ser Ala Gln Pro Gly Pro Thr Val Gln
220                 225                 230                 235

gca gga gag aat gtg acc ttg tcc tgc agc tcc cgg agc ttg ttt gac      771
Ala Gly Glu Asn Val Thr Leu Ser Cys Ser Ser Arg Ser Leu Phe Asp
                240                 245                 250

att tac cat cta tcc agg gag gca gag gcc ggt gaa ctt agg ctc act      819
Ile Tyr His Leu Ser Arg Glu Ala Glu Ala Gly Glu Leu Arg Leu Thr
            255                 260                 265

gcg gtg ctg agg gtc aat gga aca ttc cag gcc aac ttc cct ctg ggc 867
Ala Val Leu Arg Val Asn Gly Thr Phe Gln Ala Asn Phe Pro Leu Gly
        270                 275                 280

cct gtg acc cac gga ggg aac tac aga tgc ttc ggc tct ttc cgt gcc 915
Pro Val Thr His Gly Gly Asn Tyr Arg Cys Phe Gly Ser Phe Arg Ala
    285                 290                 295

ctg ccc cac gcg tgg tca gac ccg agt gac cca ctg ccc gtt tct gtc 963
Leu Pro His Ala Trp Ser Asp Pro Ser Asp Pro Leu Pro Val Ser Val
300                 305                 310                 315

aca ggt aac tcc aga cac ctg cac gtt ctg att ggg acc tca gtg gtc 1011
Thr Gly Asn Ser Arg His Leu His Val Leu Ile Gly Thr Ser Val Val
                320                 325                 330

atc atc ccc ttt gct atc ctc ctc ttc ttt ctc ctt cat cgc tgg tgt 1059
Ile Ile Pro Phe Ala Ile Leu Leu Phe Phe Leu Leu His Arg Trp Cys
            335                 340                 345

gcc aac aaa aag aat gct gtt gta atg gac caa gag cct gca ggg aac 1107
Ala Asn Lys Lys Asn Ala Val Val Met Asp Gln Glu Pro Ala Gly Asn
        350                 355                 360

aga aca gtg aac agg gag gac tct gat gaa caa gac cct cag gag gtg 1155
Arg Thr Val Asn Arg Glu Asp Ser Asp Glu Gln Asp Pro Gln Glu Val
    365                 370                 375

aca tac gca cag ttg aat cac tgc gtt ttc aca cag aga aaa atc act 1203
Thr Tyr Ala Gln Leu Asn His Cys Val Phe Thr Gln Arg Lys Ile Thr
380                 385                 390                 395

cgc cct tct cag agg ccc aag aca ccc cca aca gat acc agc gtg taa 1251
Arg Pro Ser Gln Arg Pro Lys Thr Pro Pro Thr Asp Thr Ser Val
                400                 405                 410

cacggaactt ccaaatgctg agcgcagatc caaagttgtc ttctgtccac tagcaccaca 1311

gtcaggcctt gatgggatct tctagggaga caatagccct gtctcaaaac cgggttgcca 1371

gctcccatgt accagcagct ggactctgaa ggcgtgagtc tgcatcttag ggcatcgctc 1431

ttcctcacac cacgaatctg aacatgcctc tctcttgctt acaaatgtct aaggtcccca 1491

ctgcctgctg gagagaaaac acacttgctt agcccacaat tctccatttc acttgacccc 1551

tgcccacctc tccaacctaa ctggcttact tcctagtcta cttgaggctg cgatcacact 1611

gaggaactca caattccaaa catataagag gctccctctt aacacggcac ttagatacgt 1671

gctattccac ctttcctcag 1691

<210> 92
<211> 410
<212> PRT
<213> human

<400> 92

Met Ser Leu Met Val Val Ser Met Ala Cys Val Gly Phe Phe Leu Leu
1               5                   10                  15

Glu Gly Pro Trp Pro His Val Gly Gly Gln Asp Lys Pro Phe Leu Ser
            20                  25                  30

Ala Trp Pro Gly Thr Val Val Ser Glu Gly Gln His Val Thr Leu Gln
        35                  40                  45

Cys Arg Ser Arg Leu Gly Phe Asn Glu Phe Ser Leu Ser Lys Glu Asp
    50                  55                  60

Gly Met Pro Val Pro Glu Leu Tyr Asn Arg Ile Phe Arg Asn Ser Phe
65                  70                  75                  80

Leu Met Gly Pro Val Thr Pro Ala His Ala Gly Thr Tyr Arg Cys Cys
                85                  90                  95

Ser Ser His Pro His Ser Pro Thr Gly Trp Ser Ala Pro Ser Asn Pro
            100                 105                 110

Val Val Ile Met Val Thr Gly Val His Arg Lys Pro Ser Leu Leu Ala
        115                 120                 125

His Pro Gly Pro Leu Val Lys Ser Gly Glu Thr Val Ile Leu Gln Cys
    130                 135                 140

Trp Ser Asp Val Arg Phe Glu Arg Phe Leu Leu His Arg Glu Gly Ile
145                 150                 155                 160

Thr Glu Asp Pro Leu Arg Leu Val Gly Gln Leu His Asp Ala Gly Ser
                165                 170                 175

Gln Val Asn Tyr Ser Met Gly Pro Met Thr Pro Ala Leu Ala Gly Thr
            180                 185                 190

Tyr Arg Cys Phe Gly Ser Val Thr His Leu Pro Tyr Glu Leu Ser Ala
        195                 200                 205

Pro Ser Asp Pro Leu Asp Ile Val Val Val Gly Leu Tyr Gly Lys Pro
    210                 215                 220

Ser Leu Ser Ala Gln Pro Gly Pro Thr Val Gln Ala Gly Glu Asn Val
225                 230                 235                 240

Thr Leu Ser Cys Ser Ser Arg Ser Leu Phe Asp Ile Tyr His Leu Ser
                245                 250                 255

Arg Glu Ala Glu Ala Gly Glu Leu Arg Leu Thr Ala Val Leu Arg Val
            260                 265                 270

Asn Gly Thr Phe Gln Ala Asn Phe Pro Leu Gly Pro Val Thr His Gly
        275                 280                 285

Gly Asn Tyr Arg Cys Phe Gly Ser Phe Arg Ala Leu Pro His Ala Trp
    290                 295                 300

Ser Asp Pro Ser Asp Pro Leu Pro Val Ser Val Thr Gly Asn Ser Arg
305                 310                 315                 320

His Leu His Val Leu Ile Gly Thr Ser Val Val Ile Ile Pro Phe Ala
                325                 330                 335

Ile Leu Leu Phe Phe Leu Leu His Arg Trp Cys Ala Asn Lys Lys Asn
            340                 345                 350

Ala Val Val Met Asp Gln Glu Pro Ala Gly Asn Arg Thr Val Asn Arg
        355                 360                 365

Glu Asp Ser Asp Glu Gln Asp Pro Gln Glu Val Thr Tyr Ala Gln Leu
    370                 375                 380

Asn His Cys Val Phe Thr Gln Arg Lys Ile Thr Arg Pro Ser Gln Arg
385                 390                 395                 400

Pro Lys Thr Pro Pro Thr Asp Thr Ser Val
                405                 410

<210>  93
<211>  1910
<212>  DNA
<213>  human

<220>
<221>  CDS
<222>  (183)..(1652)

<400>  93
ctcactgcca cacgcagctc aacctgagct acacagccag atgcgagatg cttctctgct      60

gatctgagtc tgcctgcagc atggaccttg gtcttccctg aagcatctcc agggctggag     120

ggacgactgc catgcaccga gggctcatcc atccgcagag cagggcagtg ggaggagacg     180

ct atg acc ccc atc gtc aca gtc ctg atc tgt ctc agg ctg agt ctg        227
   Met Thr Pro Ile Val Thr Val Leu Ile Cys Leu Arg Leu Ser Leu
   1               5                   10                  15

ggc ccc cgg acc cac gtg cag gca ggg acc ctc ccc aag ccc aca ctc       275
Gly Pro Arg Thr His Val Gln Ala Gly Thr Leu Pro Lys Pro Thr Leu
                20                  25                  30

tgg gct gag cca ggc tct gtg atc acc cag ggg agt ccc gtg acc ctc       323
Trp Ala Glu Pro Gly Ser Val Ile Thr Gln Gly Ser Pro Val Thr Leu
            35                  40                  45

tgg tgt cag ggg atc ctg gag acc cag gag tac cgt ctg tat aga gaa       371
Trp Cys Gln Gly Ile Leu Glu Thr Gln Glu Tyr Arg Leu Tyr Arg Glu
        50                  55                  60

aag aaa aca gca ccc tgg att aca cgg atc cca cag gag att gtg aag       419
Lys Lys Thr Ala Pro Trp Ile Thr Arg Ile Pro Gln Glu Ile Val Lys
    65                  70                  75

aag ggc cag ttc ccc atc cca tcc atc acc tgg gaa cac aca ggg cgg       467
Lys Gly Gln Phe Pro Ile Pro Ser Ile Thr Trp Glu His Thr Gly Arg

80                    85                    90                    95

tat cgc tgt ttc tac ggt agc cac act gca ggc tgg tca gag ccc agt          515
Tyr Arg Cys Phe Tyr Gly Ser His Thr Ala Gly Trp Ser Glu Pro Ser
                100                 105                 110

gac ccc ctg gag ctg gtg gtg aca gga gcc tac atc aaa ccc acc ctc          563
Asp Pro Leu Glu Leu Val Val Thr Gly Ala Tyr Ile Lys Pro Thr Leu
            115                 120                 125

tca gct cta ccc agc cct gtg gtg acc tca gga ggg aac gtg acc ctc          611
Ser Ala Leu Pro Ser Pro Val Val Thr Ser Gly Gly Asn Val Thr Leu
        130                 135                 140

cat tgt gtc tca cag gtg gca ttt ggc agc ttc att ctg tgt aag gaa          659
His Cys Val Ser Gln Val Ala Phe Gly Ser Phe Ile Leu Cys Lys Glu
    145                 150                 155

gga gaa gat gaa cac cca caa tgc ctg aac tca cag ccc cgt acc cat          707
Gly Glu Asp Glu His Pro Gln Cys Leu Asn Ser Gln Pro Arg Thr His
160                 165                 170                 175

ggg tgg tcc cgg gcc atc ttc tct gtg ggc ccc gtg agc ccg agt cgc          755
Gly Trp Ser Arg Ala Ile Phe Ser Val Gly Pro Val Ser Pro Ser Arg
                180                 185                 190

agg tgg tcg tac agg tgc tat gct tat gac tcg aac tct ccc cat gtg          803
Arg Trp Ser Tyr Arg Cys Tyr Ala Tyr Asp Ser Asn Ser Pro His Val
            195                 200                 205

tgg tct cta ccc agt gat ctc ctg gag ctc ctg gtc cta ggt gtt tct          851
Trp Ser Leu Pro Ser Asp Leu Leu Glu Leu Leu Val Leu Gly Val Ser
        210                 215                 220

aag aag cca tca ctc tca gtg cag cca ggt cct ata gtg gcc cct ggg          899
Lys Lys Pro Ser Leu Ser Val Gln Pro Gly Pro Ile Val Ala Pro Gly
    225                 230                 235

gag agc ctg acc ctc cag tgt gtt tct gat gtc agc tac gac aga ttt          947
Glu Ser Leu Thr Leu Gln Cys Val Ser Asp Val Ser Tyr Asp Arg Phe
240                 245                 250                 255

gtt ctg tat aag gag gga gaa cgt gac ttc ctc cag ctc cct ggc cca          995
Val Leu Tyr Lys Glu Gly Glu Arg Asp Phe Leu Gln Leu Pro Gly Pro
                260                 265                 270

cag ccc cag gct ggg ctc tcc cag gcc aac ttc acc ctg ggc cct gtg         1043
Gln Pro Gln Ala Gly Leu Ser Gln Ala Asn Phe Thr Leu Gly Pro Val
            275                 280                 285

agc cgc tcc tac ggg ggc cag tac aga tgc tcc ggt gca tac aac ctc         1091
Ser Arg Ser Tyr Gly Gly Gln Tyr Arg Cys Ser Gly Ala Tyr Asn Leu
        290                 295                 300

tcc tcc gag tgg tcg gcc ccc agc gac ccc ctg gac atc ctg atc gca         1139
Ser Ser Glu Trp Ser Ala Pro Ser Asp Pro Leu Asp Ile Leu Ile Ala
    305                 310                 315

gga cag ttc cgt ggc aga ccc ttc atc tcg gtg cat ccg ggc ccc acg         1187
Gly Gln Phe Arg Gly Arg Pro Phe Ile Ser Val His Pro Gly Pro Thr
320                 325                 330                 335

gtg gcc tca gga gag aac gtg acc ctg ctg tgt cag tca tgg ggg ccg         1235
Val Ala Ser Gly Glu Asn Val Thr Leu Leu Cys Gln Ser Trp Gly Pro
                340                 345                 350

ttc cac act ttc ctt ctg acc aag gcg gga gca gct gat gcc ccc ctc         1283
Phe His Thr Phe Leu Leu Thr Lys Ala Gly Ala Ala Asp Ala Pro Leu

                355                 360                 365

cgt ctc aga tca ata cac gaa tat cct aag tac cag gct gaa ttc cct      1331
Arg Leu Arg Ser Ile His Glu Tyr Pro Lys Tyr Gln Ala Glu Phe Pro
        370                 375                 380

atg agt cct gtg acc tca gcc cac tcg ggg acc tac agg tgc tac ggc      1379
Met Ser Pro Val Thr Ser Ala His Ser Gly Thr Tyr Arg Cys Tyr Gly
    385                 390                 395

tca ctc agc tcc aac ccc tac ctg ctg tct cac ccc agt gac tcc ctg      1427
Ser Leu Ser Ser Asn Pro Tyr Leu Leu Ser His Pro Ser Asp Ser Leu
400                 405                 410                 415

gag ctc atg gtc tca gga gca gct gag acc ctc agc cca cca caa aac      1475
Glu Leu Met Val Ser Gly Ala Ala Glu Thr Leu Ser Pro Pro Gln Asn
                420                 425                 430

aag tcc gat tcc aag gct gga gca gct aac acc ctc agc cca tca caa      1523
Lys Ser Asp Ser Lys Ala Gly Ala Ala Asn Thr Leu Ser Pro Ser Gln
            435                 440                 445

aac aag act gcc tca cac ccc cag gat tac aca gtg gag aat ctc atc      1571
Asn Lys Thr Ala Ser His Pro Gln Asp Tyr Thr Val Glu Asn Leu Ile
        450                 455                 460

cgc atg ggc ata gct ggc ttg gtc ctg gtg gtc ctc ggg att ctg cta      1619
Arg Met Gly Ile Ala Gly Leu Val Leu Val Val Leu Gly Ile Leu Leu
    465                 470                 475

ttt gag gct cag cac agc cag aga agc ctc tga gatgcagccg ggaggtgaac    1672
Phe Glu Ala Gln His Ser Gln Arg Ser Leu
480                 485

agcagagaga agaatgtacc cttcagagtg gtggagcctt gggaacagat ctgatgatgc    1732

caggaggttc cgggagacaa tttagggctg atgttatctg gactgtctgc caatcatttt    1792

tagagggagg aatcagtgtt ggattgcaga gacattttct ggagtgatcc atgaaggacc    1852

attaacatgt gatacctttc ctctctatta atgttgactt cccttggttg gatcctct      1910

<210>  94
<211>  489
<212>  PRT
<213>  human

<400>  94

Met Thr Pro Ile Val Thr Val Leu Ile Cys Leu Arg Leu Ser Leu Gly
1               5                   10                  15

Pro Arg Thr His Val Gln Ala Gly Thr Leu Pro Lys Pro Thr Leu Trp
            20                  25                  30

Ala Glu Pro Gly Ser Val Ile Thr Gln Gly Ser Pro Val Thr Leu Trp
        35                  40                  45

Cys Gln Gly Ile Leu Glu Thr Gln Glu Tyr Arg Leu Tyr Arg Glu Lys
    50                  55                  60

Lys Thr Ala Pro Trp Ile Thr Arg Ile Pro Gln Glu Ile Val Lys Lys
65                  70                  75                  80

Gly Gln Phe Pro Ile Pro Ser Ile Thr Trp Glu His Thr Gly Arg Tyr
                85                  90                  95

Arg Cys Phe Tyr Gly Ser His Thr Ala Gly Trp Ser Glu Pro Ser Asp
            100                 105                 110

Pro Leu Glu Leu Val Val Thr Gly Ala Tyr Ile Lys Pro Thr Leu Ser
        115                 120                 125

Ala Leu Pro Ser Pro Val Val Thr Ser Gly Gly Asn Val Thr Leu His
    130                 135                 140

Cys Val Ser Gln Val Ala Phe Gly Ser Phe Ile Leu Cys Lys Glu Gly
145                 150                 155                 160

Glu Asp Glu His Pro Gln Cys Leu Asn Ser Gln Pro Arg Thr His Gly
                165                 170                 175

Trp Ser Arg Ala Ile Phe Ser Val Gly Pro Val Ser Pro Ser Arg Arg
            180                 185                 190

Trp Ser Tyr Arg Cys Tyr Ala Tyr Asp Ser Asn Ser Pro His Val Trp
        195                 200                 205

Ser Leu Pro Ser Asp Leu Leu Glu Leu Leu Val Leu Gly Val Ser Lys
    210                 215                 220

Lys Pro Ser Leu Ser Val Gln Pro Gly Pro Ile Val Ala Pro Gly Glu
225                 230                 235                 240

Ser Leu Thr Leu Gln Cys Val Ser Asp Val Ser Tyr Asp Arg Phe Val
                245                 250                 255

Leu Tyr Lys Glu Gly Glu Arg Asp Phe Leu Gln Leu Pro Gly Pro Gln
            260                 265                 270

Pro Gln Ala Gly Leu Ser Gln Ala Asn Phe Thr Leu Gly Pro Val Ser
        275                 280                 285

Arg Ser Tyr Gly Gly Gln Tyr Arg Cys Ser Gly Ala Tyr Asn Leu Ser
    290                 295                 300

Ser Glu Trp Ser Ala Pro Ser Asp Pro Leu Asp Ile Leu Ile Ala Gly
305                 310                 315                 320

Gln Phe Arg Gly Arg Pro Phe Ile Ser Val His Pro Gly Pro Thr Val
                325                 330                 335

Ala Ser Gly Glu Asn Val Thr Leu Leu Cys Gln Ser Trp Gly Pro Phe
            340                 345                 350

His Thr Phe Leu Leu Thr Lys Ala Gly Ala Ala Asp Ala Pro Leu Arg
        355                 360                 365

Leu Arg Ser Ile His Glu Tyr Pro Lys Tyr Gln Ala Glu Phe Pro Met
    370                 375                 380

Ser Pro Val Thr Ser Ala His Ser Gly Thr Tyr Arg Cys Tyr Gly Ser
385                 390                 395                 400

Leu Ser Ser Asn Pro Tyr Leu Leu Ser His Pro Ser Asp Ser Leu Glu
                405                 410                 415

Leu Met Val Ser Gly Ala Ala Glu Thr Leu Ser Pro Pro Gln Asn Lys
            420                 425                 430

Ser Asp Ser Lys Ala Gly Ala Ala Asn Thr Leu Ser Pro Ser Gln Asn
        435                 440                 445

Lys Thr Ala Ser His Pro Gln Asp Tyr Thr Val Glu Asn Leu Ile Arg
    450                 455                 460

Met Gly Ile Ala Gly Leu Val Leu Val Val Leu Gly Ile Leu Leu Phe
465                 470                 475                 480

Glu Ala Gln His Ser Gln Arg Ser Leu
                485

<210>  95
<211>  6671
<212>  DNA
<213>  human

<220>
<221>  CDS
<222>  (792)..(3587)

<400>  95
   cagagatcgc gagcgaggca ccagcctgca gccggccccc agcacatcct cagccgcaca      60
   gacactcggc gaggtggagg tgagggcggg cgccagcgaa ctcggagagg ggctcgctca     120
   ctcccaggcg atcccagccg ccaccgccgc cgcaccagca gcagcaacag cagcagcagc     180
   ttccttcctc agactcccct cgagaggctg gccaagcggg tgtagccgtt gggggaggct     240
   cccgccgggg gaacccggcg aggacaagag cagggcggcc gccttccact cgggctgtcc     300
   ggcggcggct gcctccgccc gtgtgtccgt caagggtgcc gcgggatgtg tgtcagttta     360
   cgcctctgag atcacacagc tgcctggggg ccgtgtgatg cccaaggcaa gtcttggttt     420
   taattattat tattatcatt attgttacgc ttggctttcg ggaaatactc gtgatatttg     480
   taggataaag gaaatgacac tttgaggaac tggagagaac atatatgcgt tttgttttta     540
   agaggaaaac cgtgttctct tcccggcttg ttccctcttt gctgatttca ggagctactc     600
   tcctcctggt gaggtggaaa ttccagcaag aatagaggtg aagacaagcc accaggactc 660
   aggagggaaa cgctgaccat tagaaacctc tgcataagac gttgtaagga ggaaaataaa 720
   agagagaaaa acacaaagat ttaaacaaga aacctacgaa cccagctctg gaaagagcca 780
   ccttctccaa a atg gat atg ttt cct ctc acc tgg gtt ttc tta gcc ctc 830
   Met Asp Met Phe Pro Leu Thr Trp Val Phe Leu Ala Leu
   1 5 10
   tac ttt tca aga cac caa gtg aga ggc caa cca gac cca ccg tgc gga 878
   Tyr Phe Ser Arg His Gln Val Arg Gly Gln Pro Asp Pro Pro Cys Gly
   15 20 25
   ggt cgt ttg aat tcc aaa gat gct ggc tat atc acc tct ccc ggt tac 926
   Gly Arg Leu Asn Ser Lys Asp Ala Gly Tyr Ile Thr Ser Pro Gly Tyr
   30 35 40 45
   ccc cag gac tac ccc tcc cac cag aac tgc gag tgg att gtt tac gcc 974
   Pro Gln Asp Tyr Pro Ser His Gln Asn Cys Glu Trp Ile Val Tyr Ala
   50 55 60
   ccc gaa ccc aac cag aag att gtc ctc aac ttc aac cct cac ttt gaa 1022
   Pro Glu Pro Asn Gln Lys Ile Val Leu Asn Phe Asn Pro His Phe Glu
   65 70 75
   atc gag aag cac gac tgc aag tat gac ttt atc gag att cgg gat ggg 1070
   Ile Glu Lys His Asp Cys Lys Tyr Asp Phe Ile Glu Ile Arg Asp Gly
   80 85 90
   gac agt gaa tcc gca gac ctc ctg ggc aaa cac tgt ggg aac atc gcc 1118
   Asp Ser Glu Ser Ala Asp Leu Leu Gly Lys His Cys Gly Asn Ile Ala
   95 100 105
   ccg ccc acc atc atc tcc tcg ggc tcc atg ctc tac atc aag ttc acc 1166
   Pro Pro Thr Ile Ile Ser Ser Gly Ser Met Leu Tyr Ile Lys Phe Thr
   110 115 120 125
   tcc gac tac gcc cgg cag ggg gca ggc ttc tct ctg cgc tac gag atc 1214
   Ser Asp Tyr Ala Arg Gln Gly Ala Gly Phe Ser Leu Arg Tyr Glu Ile
   130 135 140
   ttc aag aca ggc tct gaa gat tgc tca aaa aac ttc aca agc ccc aac 1262
   Phe Lys Thr Gly Ser Glu Asp Cys Ser Lys Asn Phe Thr Ser Pro Asn
   145 150 155
   ggg acc atc gaa tct cct ggg ttt cct gag aag tat cca cac aac ttg 1310
   Gly Thr Ile Glu Ser Pro Gly Phe Pro Glu Lys Tyr Pro His Asn Leu
   160 165 170
   gac tgc acc ttt acc atc ctg gcc aaa ccc aag atg gag atc atc ctg 1358
   Asp Cys Thr Phe Thr Ile Leu Ala Lys Pro Lys Met Glu Ile Ile Leu
   175 180 185
   cag ttc ctg atc ttt gac ctg gag cat gac cct ttg cag gtg gga gag 1406
   Gln Phe Leu Ile Phe Asp Leu Glu His Asp Pro Leu Gln Val Gly Glu
   190 195 200 205
   ggg gac tgc aag tac gat tgg ctg gac atc tgg gat ggc att cca cat 1454
   Gly Asp Cys Lys Tyr Asp Trp Leu Asp Ile Trp Asp Gly Ile Pro His
   210 215 220
   gtt ggc ccc ctg att ggc aag tac tgt ggg acc aaa aca ccc tct gaa 1502
   Val Gly Pro Leu Ile Gly Lys Tyr Cys Gly Thr Lys Thr Pro Ser Glu
   225 230 235
   ctt cgt tca tcg acg ggg atc ctc tcc ctg acc ttt cac acg gac atg 1550
   Leu Arg Ser Ser Thr Gly Ile Leu Ser Leu Thr Phe His Thr Asp Met
           240                 245                 250
   gcg gtg gcc aag gat ggc ttc tct gcg cgt tac tac ctg gtc cac caa      1598
   Ala Val Ala Lys Asp Gly Phe Ser Ala Arg Tyr Tyr Leu Val His Gln
       255                 260                 265
   gag cca cta gag aac ttt cag tgc aat gtt cct ctg ggc atg gag tct      1646
   Glu Pro Leu Glu Asn Phe Gln Cys Asn Val Pro Leu Gly Met Glu Ser
   270                 275                 280                 285
   ggc cgg att gct aat gaa cag atc agt gcc tca tct acc tac tct gat      1694
   Gly Arg Ile Ala Asn Glu Gln Ile Ser Ala Ser Ser Thr Tyr Ser Asp
                   290                 295                 300
   ggg agg tgg acc cct caa caa agc cgg ctc cat ggt gat gac aat ggc      1742
   Gly Arg Trp Thr Pro Gln Gln Ser Arg Leu His Gly Asp Asp Asn Gly
               305                 310                 315
   tgg acc ccc aac ttg gat tcc aac aag gag tat ctc cag gtg gac ctg      1790
   Trp Thr Pro Asn Leu Asp Ser Asn Lys Glu Tyr Leu Gln Val Asp Leu
           320                 325                 330
   cgc ttt tta acc atg ctc acg gcc atc gca aca cag gga gcg att tcc      1838
   Arg Phe Leu Thr Met Leu Thr Ala Ile Ala Thr Gln Gly Ala Ile Ser
       335                 340                 345
   agg gaa aca cag aat ggc tac tat gtc aaa tcc tac aag ctg gaa gtc      1886
   Arg Glu Thr Gln Asn Gly Tyr Tyr Val Lys Ser Tyr Lys Leu Glu Val
   350                 355                 360                 365
   agc act aat gga gag gac tgg atg gtg tac cgg cat ggc aaa aac cac      1934
   Ser Thr Asn Gly Glu Asp Trp Met Val Tyr Arg His Gly Lys Asn His
                   370                 375                 380
   aag gta ttt caa gcc aac aac gat gca act gag gtg gtt ctg aac aag      1982
   Lys Val Phe Gln Ala Asn Asn Asp Ala Thr Glu Val Val Leu Asn Lys
               385                 390                 395
   ctc cac gct cca ctg ctg aca agg ttt gtt aga atc cgc cct cag acc      2030
   Leu His Ala Pro Leu Leu Thr Arg Phe Val Arg Ile Arg Pro Gln Thr
           400                 405                 410
   tgg cac tca ggt atc gcc ctc cgg ctg gag ctc ttc ggc tgc cgg gtc      2078
   Trp His Ser Gly Ile Ala Leu Arg Leu Glu Leu Phe Gly Cys Arg Val
       415                 420                 425
   aca gat gct ccc tgc tcc aac atg ctg ggg atg ctc tca ggc ctc att      2126
   Thr Asp Ala Pro Cys Ser Asn Met Leu Gly Met Leu Ser Gly Leu Ile
   430                 435                 440                 445
   gca gac tcc cag atc tcc gcc tct tcc acc cag gaa tac ctc tgg agc      2174
   Ala Asp Ser Gln Ile Ser Ala Ser Ser Thr Gln Glu Tyr Leu Trp Ser
                   450                 455                 460
   ccc agt gca gcc cgc ctg gtc agc agc cgc tcg ggc tgg ttc cct cga      2222
   Pro Ser Ala Ala Arg Leu Val Ser Ser Arg Ser Gly Trp Phe Pro Arg
               465                 470                 475
   atc cct cag gcc cag ccc ggt gag gag tgg ctt cag gta gat ctg gga      2270
   Ile Pro Gln Ala Gln Pro Gly Glu Glu Trp Leu Gln Val Asp Leu Gly
           480                 485                 490
   aca ccc aag aca gtg aaa ggt gtc atc atc cag gga gcc cgc gga gga      2318
   Thr Pro Lys Thr Val Lys Gly Val Ile Ile Gln Gly Ala Arg Gly Gly
       495                 500                 505
   gac agt atc act gct gtg gaa gcc aga gca ttt gtg cgc aag ttc aaa      2366
   Asp Ser Ile Thr Ala Val Glu Ala Arg Ala Phe Val Arg Lys Phe Lys
   510                 515                 520                 525
   gtc tcc tac agc cta aac ggc aag gac tgg gaa tac att cag gac ccc     2414
   Val Ser Tyr Ser Leu Asn Gly Lys Asp Trp Glu Tyr Ile Gln Asp Pro
                   530                 535                 540
   agg acc cag cag cca aag ctg ttc gaa ggg aac atg cac tat gac acc     2462
   Arg Thr Gln Gln Pro Lys Leu Phe Glu Gly Asn Met His Tyr Asp Thr
               545                 550                 555
   cct gac atc cga agg ttt gac ccc att ccg gca cag tat gtg cgg gta     2510
   Pro Asp Ile Arg Arg Phe Asp Pro Ile Pro Ala Gln Tyr Val Arg Val
           560                 565                 570
   tac ccg gag agg tgg tcg ccg gcg ggg att ggg atg cgg ctg gag gtg     2558
   Tyr Pro Glu Arg Trp Ser Pro Ala Gly Ile Gly Met Arg Leu Glu Val
       575                 580                 585
   ctg ggc tgt gac tgg aca gac tcc aag ccc acg gta gag acg ctg gga     2606
   Leu Gly Cys Asp Trp Thr Asp Ser Lys Pro Thr Val Glu Thr Leu Gly
   590                 595                 600                 605
   ccc act gtg aag agc gaa gag aca acc acc ccc tac ccc acc gaa gag     2654
   Pro Thr Val Lys Ser Glu Glu Thr Thr Thr Pro Tyr Pro Thr Glu Glu
                   610                 615                 620
   gag gcc aca gag tgt ggg gag aac tgc agc ttt gag gat gac aaa gat     2702
   Glu Ala Thr Glu Cys Gly Glu Asn Cys Ser Phe Glu Asp Asp Lys Asp
               625                 630                 635
   ttg cag ctc cct tcg gga ttc aat tgc aac ttc gat ttc ctc gag gag     2750
   Leu Gln Leu Pro Ser Gly Phe Asn Cys Asn Phe Asp Phe Leu Glu Glu
           640                 645                 650
   ccc tgt ggt tgg atg tat gac cat gcc aag tgg ctc cgg acc acc tgg     2798
   Pro Cys Gly Trp Met Tyr Asp His Ala Lys Trp Leu Arg Thr Thr Trp
       655                 660                 665
   gcc agc agc tcc agc cca aac gac cgg acg ttt cca gat gac agg aat     2846
   Ala Ser Ser Ser Ser Pro Asn Asp Arg Thr Phe Pro Asp Asp Arg Asn
   670                 675                 680                 685
   ttc ttg cgg ctg cag agt gac agc cag aga gag ggc cag tat gcc cgg     2894
   Phe Leu Arg Leu Gln Ser Asp Ser Gln Arg Glu Gly Gln Tyr Ala Arg
                   690                 695                 700
   ctc atc agc ccc cct gtc cac ctg ccc cga agc ccg gtg tgc atg gag     2942
   Leu Ile Ser Pro Pro Val His Leu Pro Arg Ser Pro Val Cys Met Glu
               705                 710                 715
   ttc cag tac cag gcc acg ggc ggc cgc ggg gtg gcg ctg cag gtg gtg     2990
   Phe Gln Tyr Gln Ala Thr Gly Gly Arg Gly Val Ala Leu Gln Val Val
           720                 725                 730
   cgg gaa gcc agc cag gag agc aag ttg ctg tgg gtc atc cgt gag gac     3038
   Arg Glu Ala Ser Gln Glu Ser Lys Leu Leu Trp Val Ile Arg Glu Asp
       735                 740                 745
   cag ggc ggc gag tgg aag cac ggg cgg atc atc ctg ccc agc tac gac     3086
   Gln Gly Gly Glu Trp Lys His Gly Arg Ile Ile Leu Pro Ser Tyr Asp
   750                 755                 760                 765
   atg gag tac cag att gtg ttc gag gga gtg ata ggg aaa gga cgt tcc     3134
   Met Glu Tyr Gln Ile Val Phe Glu Gly Val Ile Gly Lys Gly Arg Ser
                   770                 775                 780
   gga gag att gcc att gat gac att cgg ata agc act gat gtc cca ctg     3182
   Gly Glu Ile Ala Ile Asp Asp Ile Arg Ile Ser Thr Asp Val Pro Leu
               785                 790                 795
   gag aac tgc atg gaa ccc atc tcg gct ttt gca ggt gag aat ttt aaa      3230
   Glu Asn Cys Met Glu Pro Ile Ser Ala Phe Ala Gly Glu Asn Phe Lys
           800                 805                 810
   gtg gac atc cca gaa ata cat gag aga gaa gga tat gaa gat gaa att      3278
   Val Asp Ile Pro Glu Ile His Glu Arg Glu Gly Tyr Glu Asp Glu Ile
       815                 820                 825
   gat gat gaa tac gag gtg gac tgg agc aat tct tct tct gca acc tca      3326
   Asp Asp Glu Tyr Glu Val Asp Trp Ser Asn Ser Ser Ser Ala Thr Ser
   830                 835                 840                 845
   ggg tct ggc gcc ccc tcg acc gac aaa gaa aag agc tgg ctg tac acc      3374
   Gly Ser Gly Ala Pro Ser Thr Asp Lys Glu Lys Ser Trp Leu Tyr Thr
                   850                 855                 860
   ctg gat ccc atc ctc atc acc atc atc gcc atg agc tca ctg ggc gtc      3422
   Leu Asp Pro Ile Leu Ile Thr Ile Ile Ala Met Ser Ser Leu Gly Val
               865                 870                 875
   ctc ctg ggg gcc acc tgt gca ggc ctc ctg ctc tac tgc acc tgt tcc      3470
   Leu Leu Gly Ala Thr Cys Ala Gly Leu Leu Leu Tyr Cys Thr Cys Ser
           880                 885                 890
   tac tcg ggc ctg agc tcc cga agc tgc acc aca ctg gag aac tac aac      3518
   Tyr Ser Gly Leu Ser Ser Arg Ser Cys Thr Thr Leu Glu Asn Tyr Asn
       895                 900                 905
   ttc gag ctc tac gat ggc ctt aag cac aag gtc aag atg aac cac caa      3566
   Phe Glu Leu Tyr Asp Gly Leu Lys His Lys Val Lys Met Asn His Gln
   910                 915                 920                 925
   aag tgc tgc tcc gag gca tga cggattgcac ctgaatccta tctgacgttt      3617
   Lys Cys Cys Ser Glu Ala
                   930
   cattccagca agaggggctg gggaagatta catttttttt tcctttggaa actgaatgcc      3677
   ataatctcga tcaaaccgat ccagaatacc gaaggtatgg acaggacaga aaagcgagtc      3737
   gcaggaggaa gggagatgca gccgcacagg ggatgattac cctcctagga ccgcggtggc      3797
   taagtcattg caggaacggg gctgtgttct ctgctgggac aaaacaggag ctcatctctt      3857
   tggggtcaca gttctatttt gtttgtgagt ttgtattatt attattatta ttattattat      3917
   tatattttat ttctttggtc tgtgagcaac tcaaagaggc agaagaggag aatgactttt      3977
   ccagaataga agtggagcag tgatcattat tctccgcttt ctctttctaa tcaacacttg      4037
   aaaagcaaag tgtcttttca gcctttccat ctttacaaat aaaactcaaa aaagccgtcc      4097
   agcttatccc atcctctgat tgtcttctga cttaagggat ttactgtggt gtaggttctg      4157
   ccagccaacc ctacaagctg ccatttccag tcctagcatt taagtaggat gttgttgcct      4217
   ttaacttttc ttatccaggg gaaaattgcc attttagggt cagcatgaac agctctttct      4277
   tgtatgcgat ttaaaacaaa ctggaaagga aacttcacac gtcaaaatcc atagaagcgc      4337
   ctggacgagg cttaaagtgc tttgtgagtg aataggagcc attcgctaat tctagaccca      4397
   cagtgtctgg tggtggggct tcccttgtgg ggcttctggt ggtggttttg cctttctttt      4457
   tccctcctcc atgttcttct aaaacatata catatataca tacacacata cacatattct      4517
   tcaggtctct aagcccctgg aagcagcatt gtgtgatatt ctcagaggca ggggaaaata 4577
   gagggaaaaa tagagactat tggtatgttc tccccatcag cgagttattg taactggtca 4637
   ccactggacg ggaaggagaa cagaggagag ggaaagagaa gcccaacctc tgtgatcata 4697
   tgagggccaa ggctgagcag tgtagacaga gaccctttga aatgcatttg tctctcaaat 4757
   agactagtaa acaccgactt ctcctttggg ttacaaacac catttcaacc tttcgggaga 4817
   gtcagagcta ggatgtacaa gaactgattc taaccagaag tccgcaagta ctgtggacaa 4877
   gaatgcttaa ccatgctgct tcagccttga gagacctagg ttcttacaca tatgcacaca 4937
   cgcatacaca catgcacgca cacacacata cacacatgca cgcacgcacg catgcacacc 4997
   aatttatgtt tttattaagt gccttgaaaa aatgaagaaa aatgtatttt ccctttatgt 5057
   aaaaattagt gaatatctta tgaattaagg cattcctctt tccctaaccc cgatggctcc 5117
   attcccaagt accccaactc actgctgatc ctattaaagg aatgagtcct gctacccgag 5177
   tggtagtcat agccctagat gactctcaac tactcttcaa agggaggcat caggaataga 5237
   atgaaactgt gtgaaggata agattgttcg catcaagatc caaatcttga tttcatatta 5297
   acgcctaagg attgcctgtg tgctggaaat atatttgaaa ctcaaccagt atgcccagcc 5357
   tattgcatat cattgtcaga ccatttttgc tgctgtggtc acccacgatt tcatttgtct 5417
   tatacccagg tgaaagggga agggtgaatg ggactggctg gttcctttaa atgttaactt 5477
   atggaaatgc tagttcaaat ggtaatgtca cagtgttttg tatgcagaga gcaagagttc 5537
   aaccaacagc tgtttattca tgtgtgtgtg tctttgctgc tttgagttct ctgtatctac 5597
   tgtgtatgtg aatggtcatg tgggactcag tggtggtgtt gtgactttga cctagggtcc 5657
   gagtgtcaca gctgatcttg gcactcggca ctcattggca cagtggtagt tagaggtgaa 5717
   aagtagagct gtcaagccca agggcttagc tttagggctc ctcctgagtt cggcccacag 5777
   tagaagcaag attttaacta gccccttttc ctcttcaccc tcccatgatg cgcagtgttc 5837
   agaaagctgg taagtcctag ggatttccag aagtagcctg cagaagaagg taagtttgaa 5897
   agccactcca ggggtcctga tgctgtcatg ctcagtgagc cattttacag ttctccaaag 5957
   tctagccctg tttcggacct gcacttcacc tctaagttat gtacaactca acctgcatcc 6017
   ctctaaaagt cctatatcca tattcaccat tggctaattt gaggccctga gtgggccttg 6077
   aatgctaaaa agaagcaggg tacgcagggc tacatgtaga taccacacca aggctggagg 6137
   ctggtctgtc ataagacaga aagaaagacg ctgggcccaa ttttgacttg gccaggggac 6197
   accttggtgt gtttgttatc tttatctgtg ggtaggctag ctgacccatc tccttgagtc 6257
   attccctttg ggaaacccca ctgccagtat tgatctcctt tttgccttgt actgaatgac 6317
   acattacctc cacactctcc cggactaggt ggtcaacagg gccacagggt tgctttctgt 6377
   ctttggtggg gcaggggagt tgacagggat gagggtccaa ggaataagca tgaatgacaa 6437
   gaaaacaagg gaaagagtta acctgtcaca tagcaggtta actttttcag ggtttgcagt 6497
   tagaggtatt cgaccattca ctggctgagc cagatcacgg gaacttgaga gcttttactg 6557
   tgattcttca atgtaaaaaa taaacaacaa tgtcaaactg tgtttatatg atttgtataa 6617
   agccttttta agattactat ttaaataaac attataccag agataaaaaa aaaa 6671

<210> 96
<211> 931
<212> PRT
<213> human

<400> 96

Met Asp Met Phe Pro Leu Thr Trp Val Phe Leu Ala Leu Tyr Phe Ser
1               5                   10                  15

Arg His Gln Val Arg Gly Gln Pro Asp Pro Pro Cys Gly Gly Arg Leu
            20                  25                  30

Asn Ser Lys Asp Ala Gly Tyr Ile Thr Ser Pro Gly Tyr Pro Gln Asp
        35                  40                  45

Tyr Pro Ser His Gln Asn Cys Glu Trp Ile Val Tyr Ala Pro Glu Pro
    50                  55                  60

Asn Gln Lys Ile Val Leu Asn Phe Asn Pro His Phe Glu Ile Glu Lys
65                  70                  75                  80

His Asp Cys Lys Tyr Asp Phe Ile Glu Ile Arg Asp Gly Asp Ser Glu
                85                  90                  95

Ser Ala Asp Leu Leu Gly Lys His Cys Gly Asn Ile Ala Pro Pro Thr
            100                 105                 110

Ile Ile Ser Ser Gly Ser Met Leu Tyr Ile Lys Phe Thr Ser Asp Tyr
        115                 120                 125

Ala Arg Gln Gly Ala Gly Phe Ser Leu Arg Tyr Glu Ile Phe Lys Thr
    130                 135                 140

Gly Ser Glu Asp Cys Ser Lys Asn Phe Thr Ser Pro Asn Gly Thr Ile
145                 150                 155                 160

Glu Ser Pro Gly Phe Pro Glu Lys Tyr Pro His Asn Leu Asp Cys Thr
                165                 170                 175

Phe Thr Ile Leu Ala Lys Pro Lys Met Glu Ile Ile Leu Gln Phe Leu
            180                 185                 190

Ile Phe Asp Leu Glu His Asp Pro Leu Gln Val Gly Glu Gly Asp Cys
        195                 200                 205

Lys Tyr Asp Trp Leu Asp Ile Trp Asp Gly Ile Pro His Val Gly Pro
    210                 215                 220

Leu Ile Gly Lys Tyr Cys Gly Thr Lys Thr Pro Ser Glu Leu Arg Ser
225                 230                 235                 240

Ser Thr Gly Ile Leu Ser Leu Thr Phe His Thr Asp Met Ala Val Ala
                245                 250                 255

Lys Asp Gly Phe Ser Ala Arg Tyr Tyr Leu Val His Gln Glu Pro Leu
            260                 265                 270

Glu Asn Phe Gln Cys Asn Val Pro Leu Gly Met Glu Ser Gly Arg Ile
        275                 280                 285

Ala Asn Glu Gln Ile Ser Ala Ser Ser Thr Tyr Ser Asp Gly Arg Trp
    290                 295                 300

Thr Pro Gln Gln Ser Arg Leu His Gly Asp Asp Asn Gly Trp Thr Pro
305                 310                 315                 320

Asn Leu Asp Ser Asn Lys Glu Tyr Leu Gln Val Asp Leu Arg Phe Leu
                325                 330                 335

Thr Met Leu Thr Ala Ile Ala Thr Gln Gly Ala Ile Ser Arg Glu Thr
            340                 345                 350

Gln Asn Gly Tyr Tyr Val Lys Ser Tyr Lys Leu Glu Val Ser Thr Asn
        355                 360                 365

Gly Glu Asp Trp Met Val Tyr Arg His Gly Lys Asn His Lys Val Phe
    370                 375                 380

Gln Ala Asn Asn Asp Ala Thr Glu Val Val Leu Asn Lys Leu His Ala
385                 390                 395                 400

Pro Leu Leu Thr Arg Phe Val Arg Ile Arg Pro Gln Thr Trp His Ser
                405                 410                 415

Gly Ile Ala Leu Arg Leu Glu Leu Phe Gly Cys Arg Val Thr Asp Ala
            420                 425                 430

Pro Cys Ser Asn Met Leu Gly Met Leu Ser Gly Leu Ile Ala Asp Ser
        435                 440                 445

Gln Ile Ser Ala Ser Ser Thr Gln Glu Tyr Leu Trp Ser Pro Ser Ala
    450                 455                 460

Ala Arg Leu Val Ser Ser Arg Ser Gly Trp Phe Pro Arg Ile Pro Gln
465                 470                 475                 480

Ala Gln Pro Gly Glu Glu Trp Leu Gln Val Asp Leu Gly Thr Pro Lys
                485                 490                 495

Thr Val Lys Gly Val Ile Ile Gln Gly Ala Arg Gly Gly Asp Ser Ile
            500                 505                 510

Thr Ala Val Glu Ala Arg Ala Phe Val Arg Lys Phe Lys Val Ser Tyr
        515                 520                 525

Ser Leu Asn Gly Lys Asp Trp Glu Tyr Ile Gln Asp Pro Arg Thr Gln
    530                 535                 540

Gln Pro Lys Leu Phe Glu Gly Asn Met His Tyr Asp Thr Pro Asp Ile
545                 550                 555                 560

Arg Arg Phe Asp Pro Ile Pro Ala Gln Tyr Val Arg Val Tyr Pro Glu
                565                 570                 575

Arg Trp Ser Pro Ala Gly Ile Gly Met Arg Leu Glu Val Leu Gly Cys
            580                 585                 590

Asp Trp Thr Asp Ser Lys Pro Thr Val Glu Thr Leu Gly Pro Thr Val
        595                 600                 605

Lys Ser Glu Glu Thr Thr Thr Pro Tyr Pro Thr Glu Glu Glu Ala Thr
    610                 615                 620

Glu Cys Gly Glu Asn Cys Ser Phe Glu Asp Asp Lys Asp Leu Gln Leu
625                 630                 635                 640

Pro Ser Gly Phe Asn Cys Asn Phe Asp Phe Leu Glu Glu Pro Cys Gly
                645                 650                 655

Trp Met Tyr Asp His Ala Lys Trp Leu Arg Thr Thr Trp Ala Ser Ser
            660                 665                 670

Ser Ser Pro Asn Asp Arg Thr Phe Pro Asp Asp Arg Asn Phe Leu Arg
        675                 680                 685

Leu Gln Ser Asp Ser Gln Arg Glu Gly Gln Tyr Ala Arg Leu Ile Ser
    690                 695                 700

Pro Pro Val His Leu Pro Arg Ser Pro Val Cys Met Glu Phe Gln Tyr
705                 710                 715                 720

Gln Ala Thr Gly Gly Arg Gly Val Ala Leu Gln Val Val Arg Glu Ala
                725                 730                 735

Ser Gln Glu Ser Lys Leu Leu Trp Val Ile Arg Glu Asp Gln Gly Gly
            740                 745                 750

Glu Trp Lys His Gly Arg Ile Ile Leu Pro Ser Tyr Asp Met Glu Tyr
        755                 760                 765

Gln Ile Val Phe Glu Gly Val Ile Gly Lys Gly Arg Ser Gly Glu Ile
    770                 775                 780

Ala Ile Asp Asp Ile Arg Ile Ser Thr Asp Val Pro Leu Glu Asn Cys
785                 790                 795                 800

Met Glu Pro Ile Ser Ala Phe Ala Gly Glu Asn Phe Lys Val Asp Ile
                805                 810                 815

Pro Glu Ile His Glu Arg Glu Gly Tyr Glu Asp Glu Ile Asp Asp Glu
            820                 825                 830

Tyr Glu Val Asp Trp Ser Asn Ser Ser Ser Ala Thr Ser Gly Ser Gly
        835                 840                 845

Ala Pro Ser Thr Asp Lys Glu Lys Ser Trp Leu Tyr Thr Leu Asp Pro
    850                 855                 860

Ile Leu Ile Thr Ile Ile Ala Met Ser Ser Leu Gly Val Leu Leu Gly
865                 870                 875                 880

Ala Thr Cys Ala Gly Leu Leu Leu Tyr Cys Thr Cys Ser Tyr Ser Gly
                885                 890                 895

Leu Ser Ser Arg Ser Cys Thr Thr Leu Glu Asn Tyr Asn Phe Glu Leu
            900                 905                 910

Tyr Asp Gly Leu Lys His Lys Val Lys Met Asn His Gln Lys Cys Cys
        915                 920                 925

Ser Glu Ala
    930

<210>  97
<211>  6656
<212>  DNA
<213>  human

<220>
<221>  CDS
<222>  (792)..(3572)

<400>  97
cagagatcgc gagcgaggca ccagcctgca gccggccccc agcacatcct cagccgcaca      60

gacactcggc gaggtggagg tgagggcggg cgccagcgaa ctcggagagg ggctcgctca     120

ctcccaggcg atcccagccg ccaccgccgc cgcaccagca gcagcaacag cagcagcagc     180

ttccttcctc agactcccct cgagaggctg gccaagcggg tgtagccgtt gggggaggct     240

cccgccgggg gaacccggcg aggacaagag cagggcggcc gccttccact cgggctgtcc     300

ggcggcggct gcctccgccc gtgtgtccgt caagggtgcc gcgggatgtg tgtcagttta     360

cgcctctgag atcacacagc tgcctggggg ccgtgtgatg cccaaggcaa gtcttggttt 420

taattattat tattatcatt attgttacgc ttggctttcg ggaaatactc gtgatatttg 480

taggataaag gaaatgacac tttgaggaac tggagagaac atatatgcgt tttgttttta 540

agaggaaaac cgtgttctct tcccggcttg ttccctcttt gctgatttca ggagctactc 600

tcctcctggt gaggtggaaa ttccagcaag aatagaggtg aagacaagcc accaggactc 660

aggagggaaa cgctgaccat tagaaacctc tgcataagac gttgtaagga ggaaaataaa 720

agagagaaaa acacaaagat ttaaacaaga aacctacgaa cccagctctg gaaagagcca 780

ccttctccaa a atg gat atg ttt cct ctc acc tgg gtt ttc tta gcc ctc 830
Met Asp Met Phe Pro Leu Thr Trp Val Phe Leu Ala Leu
1               5                   10

tac ttt tca aga cac caa gtg aga ggc caa cca gac cca ccg tgc gga 878
Tyr Phe Ser Arg His Gln Val Arg Gly Gln Pro Asp Pro Pro Cys Gly
    15                  20                  25

ggt cgt ttg aat tcc aaa gat gct ggc tat atc acc tct ccc ggt tac 926
Gly Arg Leu Asn Ser Lys Asp Ala Gly Tyr Ile Thr Ser Pro Gly Tyr
30                  35                  40                  45

ccc cag gac tac ccc tcc cac cag aac tgc gag tgg att gtt tac gcc 974
Pro Gln Asp Tyr Pro Ser His Gln Asn Cys Glu Trp Ile Val Tyr Ala
                50                  55                  60

ccc gaa ccc aac cag aag att gtc ctc aac ttc aac cct cac ttt gaa 1022
Pro Glu Pro Asn Gln Lys Ile Val Leu Asn Phe Asn Pro His Phe Glu
            65                  70                  75

atc gag aag cac gac tgc aag tat gac ttt atc gag att cgg gat ggg 1070
Ile Glu Lys His Asp Cys Lys Tyr Asp Phe Ile Glu Ile Arg Asp Gly
        80                  85                  90

gac agt gaa tcc gca gac ctc ctg ggc aaa cac tgt ggg aac atc gcc 1118
Asp Ser Glu Ser Ala Asp Leu Leu Gly Lys His Cys Gly Asn Ile Ala
    95                  100                 105

ccg ccc acc atc atc tcc tcg ggc tcc atg ctc tac atc aag ttc acc 1166
Pro Pro Thr Ile Ile Ser Ser Gly Ser Met Leu Tyr Ile Lys Phe Thr
110                 115                 120                 125

tcc gac tac gcc cgg cag ggg gca ggc ttc tct ctg cgc tac gag atc 1214
Ser Asp Tyr Ala Arg Gln Gly Ala Gly Phe Ser Leu Arg Tyr Glu Ile
                130                 135                 140

ttc aag aca ggc tct gaa gat tgc tca aaa aac ttc aca agc ccc aac 1262
Phe Lys Thr Gly Ser Glu Asp Cys Ser Lys Asn Phe Thr Ser Pro Asn
            145                 150                 155

ggg acc atc gaa tct cct ggg ttt cct gag aag tat cca cac aac ttg 1310
Gly Thr Ile Glu Ser Pro Gly Phe Pro Glu Lys Tyr Pro His Asn Leu
        160                 165                 170

gac tgc acc ttt acc atc ctg gcc aaa ccc aag atg gag atc atc ctg 1358
Asp Cys Thr Phe Thr Ile Leu Ala Lys Pro Lys Met Glu Ile Ile Leu
    175                 180                 185

cag ttc ctg atc ttt gac ctg gag cat gac cct ttg cag gtg gga gag 1406
Gln Phe Leu Ile Phe Asp Leu Glu His Asp Pro Leu Gln Val Gly Glu
190                 195                 200                 205

ggg gac tgc aag tac gat tgg ctg gac atc tgg gat ggc att cca cat 1454
Gly Asp Cys Lys Tyr Asp Trp Leu Asp Ile Trp Asp Gly Ile Pro His

                210                 215                 220

gtt ggc ccc ctg att ggc aag tac tgt ggg acc aaa aca ccc tct gaa 1502
Val Gly Pro Leu Ile Gly Lys Tyr Cys Gly Thr Lys Thr Pro Ser Glu
            225                 230                 235

ctt cgt tca tcg acg ggg atc ctc tcc ctg acc ttt cac acg gac atg 1550
Leu Arg Ser Ser Thr Gly Ile Leu Ser Leu Thr Phe His Thr Asp Met
        240                 245                 250

gcg gtg gcc aag gat ggc ttc tct gcg cgt tac tac ctg gtc cac caa 1598
Ala Val Ala Lys Asp Gly Phe Ser Ala Arg Tyr Tyr Leu Val His Gln
    255                 260                 265

gag cca cta gag aac ttt cag tgc aat gtt cct ctg ggc atg gag tct 1646
Glu Pro Leu Glu Asn Phe Gln Cys Asn Val Pro Leu Gly Met Glu Ser
270                 275                 280                 285

ggc cgg att gct aat gaa cag atc agt gcc tca tct acc tac tct gat 1694
Gly Arg Ile Ala Asn Glu Gln Ile Ser Ala Ser Ser Thr Tyr Ser Asp
                290                 295                 300

ggg agg tgg acc cct caa caa agc cgg ctc cat ggt gat gac aat ggc 1742
Gly Arg Trp Thr Pro Gln Gln Ser Arg Leu His Gly Asp Asp Asn Gly
            305                 310                 315

tgg acc ccc aac ttg gat tcc aac aag gag tat ctc cag gtg gac ctg 1790
Trp Thr Pro Asn Leu Asp Ser Asn Lys Glu Tyr Leu Gln Val Asp Leu
        320                 325                 330

cgc ttt tta acc atg ctc acg gcc atc gca aca cag gga gcg att tcc 1838
Arg Phe Leu Thr Met Leu Thr Ala Ile Ala Thr Gln Gly Ala Ile Ser
    335                 340                 345

agg gaa aca cag aat ggc tac tat gtc aaa tcc tac aag ctg gaa gtc 1886
Arg Glu Thr Gln Asn Gly Tyr Tyr Val Lys Ser Tyr Lys Leu Glu Val
350                 355                 360                 365

agc act aat gga gag gac tgg atg gtg tac cgg cat ggc aaa aac cac 1934
Ser Thr Asn Gly Glu Asp Trp Met Val Tyr Arg His Gly Lys Asn His
                370                 375                 380

aag gta ttt caa gcc aac aac gat gca act gag gtg gtt ctg aac aag 1982
Lys Val Phe Gln Ala Asn Asn Asp Ala Thr Glu Val Val Leu Asn Lys
            385                 390                 395

ctc cac gct cca ctg ctg aca agg ttt gtt aga atc cgc cct cag acc 2030
Leu His Ala Pro Leu Leu Thr Arg Phe Val Arg Ile Arg Pro Gln Thr
        400                 405                 410

tgg cac tca ggt atc gcc ctc cgg ctg gag ctc ttc ggc tgc cgg gtc 2078
Trp His Ser Gly Ile Ala Leu Arg Leu Glu Leu Phe Gly Cys Arg Val
    415                 420                 425

aca gat gct ccc tgc tcc aac atg ctg ggg atg ctc tca ggc ctc att 2126
Thr Asp Ala Pro Cys Ser Asn Met Leu Gly Met Leu Ser Gly Leu Ile
430                 435                 440                 445

gca gac tcc cag atc tcc gcc tct tcc acc cag gaa tac ctc tgg agc 2174
Ala Asp Ser Gln Ile Ser Ala Ser Ser Thr Gln Glu Tyr Leu Trp Ser
                450                 455                 460

ccc agt gca gcc cgc ctg gtc agc agc cgc tcg ggc tgg ttc cct cga 2222
Pro Ser Ala Ala Arg Leu Val Ser Ser Arg Ser Gly Trp Phe Pro Arg
            465                 470                 475

atc cct cag gcc cag ccc ggt gag gag tgg ctt cag gta gat ctg gga 2270
Ile Pro Gln Ala Gln Pro Gly Glu Glu Trp Leu Gln Val Asp Leu Gly

480                 485                 490

aca ccc aag aca gtg aaa ggt gtc atc atc cag gga gcc cgc gga gga      2318
Thr Pro Lys Thr Val Lys Gly Val Ile Ile Gln Gly Ala Arg Gly Gly
    495                 500                 505

gac agt atc act gct gtg gaa gcc aga gca ttt gtg cgc aag ttc aaa      2366
Asp Ser Ile Thr Ala Val Glu Ala Arg Ala Phe Val Arg Lys Phe Lys
510                 515                 520                 525

gtc tcc tac agc cta aac ggc aag gac tgg gaa tac att cag gac ccc      2414
Val Ser Tyr Ser Leu Asn Gly Lys Asp Trp Glu Tyr Ile Gln Asp Pro
                530                 535                 540

agg acc cag cag cca aag ctg ttc gaa ggg aac atg cac tat gac acc      2462
Arg Thr Gln Gln Pro Lys Leu Phe Glu Gly Asn Met His Tyr Asp Thr
            545                 550                 555

cct gac atc cga agg ttt gac ccc att ccg gca cag tat gtg cgg gta      2510
Pro Asp Ile Arg Arg Phe Asp Pro Ile Pro Ala Gln Tyr Val Arg Val
        560                 565                 570

tac ccg gag agg tgg tcg ccg gcg ggg att ggg atg cgg ctg gag gtg      2558
Tyr Pro Glu Arg Trp Ser Pro Ala Gly Ile Gly Met Arg Leu Glu Val
    575                 580                 585

ctg ggc tgt gac tgg aca gac tcc aag ccc acg gta gag acg ctg gga      2606
Leu Gly Cys Asp Trp Thr Asp Ser Lys Pro Thr Val Glu Thr Leu Gly
590                 595                 600                 605

ccc act gtg aag agc gaa gag aca acc acc ccc tac ccc acc gaa gag      2654
Pro Thr Val Lys Ser Glu Glu Thr Thr Thr Pro Tyr Pro Thr Glu Glu
                610                 615                 620

gag gcc aca gag tgt ggg gag aac tgc agc ttt gag gat gac aaa gat      2702
Glu Ala Thr Glu Cys Gly Glu Asn Cys Ser Phe Glu Asp Asp Lys Asp
            625                 630                 635

ttg cag ctc cct tcg gga ttc aat tgc aac ttc gat ttc ctc gag gag      2750
Leu Gln Leu Pro Ser Gly Phe Asn Cys Asn Phe Asp Phe Leu Glu Glu
        640                 645                 650

ccc tgt ggt tgg atg tat gac cat gcc aag tgg ctc cgg acc acc tgg      2798
Pro Cys Gly Trp Met Tyr Asp His Ala Lys Trp Leu Arg Thr Thr Trp
    655                 660                 665

gcc agc agc tcc agc cca aac gac cgg acg ttt cca gat gac agg aat      2846
Ala Ser Ser Ser Ser Pro Asn Asp Arg Thr Phe Pro Asp Asp Arg Asn
670                 675                 680                 685

ttc ttg cgg ctg cag agt gac agc cag aga gag ggc cag tat gcc cgg      2894
Phe Leu Arg Leu Gln Ser Asp Ser Gln Arg Glu Gly Gln Tyr Ala Arg
                690                 695                 700

ctc atc agc ccc cct gtc cac ctg ccc cga agc ccg gtg tgc atg gag      2942
Leu Ile Ser Pro Pro Val His Leu Pro Arg Ser Pro Val Cys Met Glu
            705                 710                 715

ttc cag tac cag gcc acg ggc ggc cgc ggg gtg gcg ctg cag gtg gtg      2990
Phe Gln Tyr Gln Ala Thr Gly Gly Arg Gly Val Ala Leu Gln Val Val
        720                 725                 730

cgg gaa gcc agc cag gag agc aag ttg ctg tgg gtc atc cgt gag gac      3038
Arg Glu Ala Ser Gln Glu Ser Lys Leu Leu Trp Val Ile Arg Glu Asp
    735                 740                 745

cag ggc ggc gag tgg aag cac ggg cgg atc atc ctg ccc agc tac gac      3086
Gln Gly Gly Glu Trp Lys His Gly Arg Ile Ile Leu Pro Ser Tyr Asp

750                 755                 760                 765

atg gag tac cag att gtg ttc gag gga gtg ata ggg aaa gga cgt tcc     3134
Met Glu Tyr Gln Ile Val Phe Glu Gly Val Ile Gly Lys Gly Arg Ser
                770                 775                 780

gga gag att gcc att gat gac att cgg ata agc act gat gtc cca ctg     3182
Gly Glu Ile Ala Ile Asp Asp Ile Arg Ile Ser Thr Asp Val Pro Leu
            785                 790                 795

gag aac tgc atg gaa ccc atc tcg gct ttt gca gtg gac atc cca gaa     3230
Glu Asn Cys Met Glu Pro Ile Ser Ala Phe Ala Val Asp Ile Pro Glu
        800                 805                 810

ata cat gag aga gaa gga tat gaa gat gaa att gat gat gaa tac gag     3278
Ile His Glu Arg Glu Gly Tyr Glu Asp Glu Ile Asp Asp Glu Tyr Glu
    815                 820                 825

gtg gac tgg agc aat tct tct tct gca acc tca ggg tct ggc gcc ccc     3326
Val Asp Trp Ser Asn Ser Ser Ser Ala Thr Ser Gly Ser Gly Ala Pro
830                 835                 840                 845

tcg acc gac aaa gaa aag agc tgg ctg tac acc ctg gat ccc atc ctc     3374
Ser Thr Asp Lys Glu Lys Ser Trp Leu Tyr Thr Leu Asp Pro Ile Leu
                850                 855                 860

atc acc atc atc gcc atg agc tca ctg ggc gtc ctc ctg ggg gcc acc     3422
Ile Thr Ile Ile Ala Met Ser Ser Leu Gly Val Leu Leu Gly Ala Thr
            865                 870                 875

tgt gca ggc ctc ctg ctc tac tgc acc tgt tcc tac tcg ggc ctg agc     3470
Cys Ala Gly Leu Leu Leu Tyr Cys Thr Cys Ser Tyr Ser Gly Leu Ser
        880                 885                 890

tcc cga agc tgc acc aca ctg gag aac tac aac ttc gag ctc tac gat     3518
Ser Arg Ser Cys Thr Thr Leu Glu Asn Tyr Asn Phe Glu Leu Tyr Asp
    895                 900                 905

ggc ctt aag cac aag gtc aag atg aac cac caa aag tgc tgc tcc gag     3566
Gly Leu Lys His Lys Val Lys Met Asn His Gln Lys Cys Cys Ser Glu
910                 915                 920                 925

gca tga cggattgcac ctgaatccta tctgacgttt cattccagca agaggggctg     3622
Ala

gggaagatta catttttttt tcctttggaa actgaatgcc ataatctcga tcaaaccgat     3682

ccagaatacc gaaggtatgg acaggacaga aaagcgagtc gcaggaggaa gggagatgca     3742

gccgcacagg ggatgattac cctcctagga ccgcggtggc taagtcattg caggaacggg     3802

gctgtgttct ctgctgggac aaaacaggag ctcatctctt tggggtcaca gttctatttt     3862

gtttgtgagt ttgtattatt attattatta ttattattat tatattttat ttctttggtc     3922

tgtgagcaac tcaaagaggc agaagaggag aatgactttt ccagaataga agtggagcag     3982

tgatcattat tctccgcttt ctctttctaa tcaacacttg aaaagcaaag tgtcttttca     4042

gcctttccat ctttacaaat aaaactcaaa aaagccgtcc agcttatccc atcctctgat     4102

tgtcttctga cttaagggat ttactgtggt gtaggttctg ccagccaacc ctacaagctg     4162

ccatttccag tcctagcatt taagtaggat gttgttgcct ttaacttttc ttatccaggg     4222

gaaaattgcc attttagggt cagcatgaac agctctttct tgtatgcgat ttaaaacaaa     4282

ctggaaagga aacttcacac gtcaaaatcc atagaagcgc ctggacgagg cttaaagtgc 4342
tttgtgagtg aataggagcc attcgctaat tctagaccca cagtgtctgg tggtggggct 4402
tcccttgtgg ggcttctggt ggtggttttg ccttttcttt tccctcctcc atgttcttct 4462
aaaacatata catatataca tacacacata cacatattct tcaggtctct aagcccctgg 4522
aagcagcatt gtgtgatatt ctcagaggca ggggaaaata gagggaaaaa tagagactat 4582
tggtatgttc tccccatcag cgagttattg taactggtca ccactggacg ggaaggagaa 4642
cagaggagag ggaaagagaa gcccaacctc tgtgatcata tgagggccaa ggctgagcag 4702
tgtagacaga gaccctttga aatgcatttg tctctcaaat agactagtaa acaccgactt 4762
ctcctttggg ttacaaacac catttcaacc tttcgggaga gtcagagcta ggatgtacaa 4822
gaactgattc taaccagaag tccgcaagta ctgtggacaa gaatgcttaa ccatgctgct 4882
tcagccttga gagacctagg ttcttacaca tatgcacaca cgcatacaca catgcacgca 4942
cacacacata cacacatgca cgcacgcacg catgcacacc aatttatgtt tttattaagt 5002
gccttgaaaa aatgaagaaa aatgtatttt ccctttatgt aaaaattagt gaatatctta 5062
tgaattaagg cattcctctt tccctaaccc cgatggctcc attcccaagt accccaactc 5122
actgctgatc ctattaaagg aatgagtcct gctacccgag tggtagtcat agccctagat 5182
gactctcaac tactcttcaa agggaggcat caggaataga atgaaactgt gtgaaggata 5242
agattgttcg catcaagatc caaatcttga tttcatatta acgcctaagg attgcctgtg 5302
tgctggaaat atatttgaaa ctcaaccagt atgcccagcc tattgcatat cattgtcaga 5362
ccatttttgc tgctgtggtc acccacgatt tcatttgtct tatacccagg tgaaagggga 5422
agggtgaatg ggactggctg gttcctttaa atgttaactt atggaaatgc tagttcaaat 5482
ggtaatgtca cagtgttttg tatgcagaga gcaagagttc aaccaacagc tgtttattca 5542
tgtgtgtgtg tctttgctgc tttgagttct ctgtatctac tgtgtatgtg aatggtcatg 5602
tgggactcag tggtggtgtt gtgactttga cctagggtcc gagtgtcaca gctgatcttg 5662
gcactcggca ctcattggca cagtggtagt tagaggtgaa aagtagagct gtcaagccca 5722
agggcttagc tttagggctc ctcctgagtt cggcccacag tagaagcaag attttaacta 5782
gccccttttc ctcttcaccc tcccatgatg cgcagtgttc agaaagctgg taagtcctag 5842
ggatttccag aagtagcctg cagaagaagg taagtttgaa agccactcca ggggtcctga 5902
tgctgtcatg ctcagtgagc cattttacag ttctccaaag tctagccctg tttcggacct 5962
gcacttcacc tctaagttat gtacaactca acctgcatcc ctctaaaagt cctatatcca 6022
tattcaccat tggctaattt gaggccctga gtgggccttg aatgctaaaa agaagcaggg 6082
tacgcagggc tacatgtaga taccacacca aggctggagg ctggtctgtc ataagacaga 6142
aagaaagacg ctgggcccaa ttttgacttg gccaggggac accttggtgt gtttgttatc 6202
tttatctgtg ggtaggctag ctgacccatc tccttgagtc attccctttg ggaaacccca 6262
ctgccagtat tgatctcctt tttgccttgt actgaatgac acattacctc cacactctcc 6322

cggactaggt ggtcaacagg gccacagggt tgctttctgt ctttggtggg gcaggggagt 6382
tgacagggat gagggtccaa ggaataagca tgaatgacaa gaaaacaagg gaaagagtta 6442
acctgtcaca tagcaggtta actttttcag ggtttgcagt tagaggtatt cgaccattca 6502
ctggctgagc cagatcacgg gaacttgaga gcttttactg tgattcttca atgtaaaaaa 6562
taaacaacaa tgtcaaactg tgtttatatg atttgtataa agccttttta agattactat 6622
ttaaataaac attataccag agataaaaaa aaaa 6656

<210> 98
<211> 926
<212> PRT
<213> human

<400> 98

Met Asp Met Phe Pro Leu Thr Trp Val Phe Leu Ala Leu Tyr Phe Ser
1               5                   10                  15

Arg His Gln Val Arg Gly Gln Pro Asp Pro Pro Cys Gly Gly Arg Leu
            20                  25                  30

Asn Ser Lys Asp Ala Gly Tyr Ile Thr Ser Pro Gly Tyr Pro Gln Asp
        35                  40                  45

Tyr Pro Ser His Gln Asn Cys Glu Trp Ile Val Tyr Ala Pro Glu Pro
    50                  55                  60

Asn Gln Lys Ile Val Leu Asn Phe Asn Pro His Phe Glu Ile Glu Lys
65                  70                  75                  80

His Asp Cys Lys Tyr Asp Phe Ile Glu Ile Arg Asp Gly Asp Ser Glu
                85                  90                  95

Ser Ala Asp Leu Leu Gly Lys His Cys Gly Asn Ile Ala Pro Pro Thr
            100                 105                 110

Ile Ile Ser Ser Gly Ser Met Leu Tyr Ile Lys Phe Thr Ser Asp Tyr
        115                 120                 125

Ala Arg Gln Gly Ala Gly Phe Ser Leu Arg Tyr Glu Ile Phe Lys Thr
    130                 135                 140

Gly Ser Glu Asp Cys Ser Lys Asn Phe Thr Ser Pro Asn Gly Thr Ile
145                 150                 155                 160

Glu Ser Pro Gly Phe Pro Glu Lys Tyr Pro His Asn Leu Asp Cys Thr
                165                 170                 175

Phe Thr Ile Leu Ala Lys Pro Lys Met Glu Ile Ile Leu Gln Phe Leu
            180                 185                 190

Ile Phe Asp Leu Glu His Asp Pro Leu Gln Val Gly Glu Gly Asp Cys
        195                 200                 205

Lys Tyr Asp Trp Leu Asp Ile Trp Asp Gly Ile Pro His Val Gly Pro
    210                 215                 220

Leu Ile Gly Lys Tyr Cys Gly Thr Lys Thr Pro Ser Glu Leu Arg Ser
225                 230                 235                 240

Ser Thr Gly Ile Leu Ser Leu Thr Phe His Thr Asp Met Ala Val Ala
                245                 250                 255

Lys Asp Gly Phe Ser Ala Arg Tyr Tyr Leu Val His Gln Glu Pro Leu
            260                 265                 270

Glu Asn Phe Gln Cys Asn Val Pro Leu Gly Met Glu Ser Gly Arg Ile
        275                 280                 285

Ala Asn Glu Gln Ile Ser Ala Ser Ser Thr Tyr Ser Asp Gly Arg Trp
    290                 295                 300

Thr Pro Gln Gln Ser Arg Leu His Gly Asp Asp Asn Gly Trp Thr Pro
305                 310                 315                 320

Asn Leu Asp Ser Asn Lys Glu Tyr Leu Gln Val Asp Leu Arg Phe Leu
                325                 330                 335

Thr Met Leu Thr Ala Ile Ala Thr Gln Gly Ala Ile Ser Arg Glu Thr
            340                 345                 350

Gln Asn Gly Tyr Tyr Val Lys Ser Tyr Lys Leu Glu Val Ser Thr Asn
        355                 360                 365

Gly Glu Asp Trp Met Val Tyr Arg His Gly Lys Asn His Lys Val Phe
    370                 375                 380

Gln Ala Asn Asn Asp Ala Thr Glu Val Val Leu Asn Lys Leu His Ala
385                 390                 395                 400

Pro Leu Leu Thr Arg Phe Val Arg Ile Arg Pro Gln Thr Trp His Ser
                405                 410                 415

Gly Ile Ala Leu Arg Leu Glu Leu Phe Gly Cys Arg Val Thr Asp Ala
            420                 425                 430

Pro Cys Ser Asn Met Leu Gly Met Leu Ser Gly Leu Ile Ala Asp Ser
        435                 440                 445

Gln Ile Ser Ala Ser Ser Thr Gln Glu Tyr Leu Trp Ser Pro Ser Ala
    450                 455                 460

Ala Arg Leu Val Ser Ser Arg Ser Gly Trp Phe Pro Arg Ile Pro Gln
465                 470                 475                 480

Ala Gln Pro Gly Glu Glu Trp Leu Gln Val Asp Leu Gly Thr Pro Lys
                485                 490                 495

Thr Val Lys Gly Val Ile Ile Gln Gly Ala Arg Gly Gly Asp Ser Ile
            500                 505                 510

Thr Ala Val Glu Ala Arg Ala Phe Val Arg Lys Phe Lys Val Ser Tyr
        515                 520                 525

Ser Leu Asn Gly Lys Asp Trp Glu Tyr Ile Gln Asp Pro Arg Thr Gln
    530                 535                 540

Gln Pro Lys Leu Phe Glu Gly Asn Met His Tyr Asp Thr Pro Asp Ile
545                 550                 555                 560

Arg Arg Phe Asp Pro Ile Pro Ala Gln Tyr Val Arg Val Tyr Pro Glu
                565                 570                 575

Arg Trp Ser Pro Ala Gly Ile Gly Met Arg Leu Glu Val Leu Gly Cys
            580                 585                 590

Asp Trp Thr Asp Ser Lys Pro Thr Val Glu Thr Leu Gly Pro Thr Val
        595                 600                 605

Lys Ser Glu Glu Thr Thr Thr Pro Tyr Pro Thr Glu Glu Glu Ala Thr
    610                 615                 620

Glu Cys Gly Glu Asn Cys Ser Phe Glu Asp Asp Lys Asp Leu Gln Leu
625                 630                 635                 640

Pro Ser Gly Phe Asn Cys Asn Phe Asp Phe Leu Glu Glu Pro Cys Gly
                645                 650                 655

Trp Met Tyr Asp His Ala Lys Trp Leu Arg Thr Thr Trp Ala Ser Ser
            660                 665                 670

Ser Ser Pro Asn Asp Arg Thr Phe Pro Asp Asp Arg Asn Phe Leu Arg
        675                 680                 685

Leu Gln Ser Asp Ser Gln Arg Glu Gly Gln Tyr Ala Arg Leu Ile Ser
    690                 695                 700

Pro Pro Val His Leu Pro Arg Ser Pro Val Cys Met Glu Phe Gln Tyr
705                 710                 715                 720

Gln Ala Thr Gly Gly Arg Gly Val Ala Leu Gln Val Val Arg Glu Ala
                725                 730                 735

Ser Gln Glu Ser Lys Leu Leu Trp Val Ile Arg Glu Asp Gln Gly Gly
            740                 745                 750

Glu Trp Lys His Gly Arg Ile Ile Leu Pro Ser Tyr Asp Met Glu Tyr
        755                 760                 765

Gln Ile Val Phe Glu Gly Val Ile Gly Lys Gly Arg Ser Gly Glu Ile
    770                 775                 780

Ala Ile Asp Asp Ile Arg Ile Ser Thr Asp Val Pro Leu Glu Asn Cys
785                 790                 795                 800

Met Glu Pro Ile Ser Ala Phe Ala Val Asp Ile Pro Glu Ile His Glu
                805                 810                 815

Arg Glu Gly Tyr Glu Asp Glu Ile Asp Asp Glu Tyr Glu Val Asp Trp
            820                 825                 830

Ser Asn Ser Ser Ser Ala Thr Ser Gly Ser Gly Ala Pro Ser Thr Asp
        835                 840                 845

Lys Glu Lys Ser Trp Leu Tyr Thr Leu Asp Pro Ile Leu Ile Thr Ile
    850                 855                 860

Ile Ala Met Ser Ser Leu Gly Val Leu Leu Gly Ala Thr Cys Ala Gly
865                 870                 875                 880

Leu Leu Leu Tyr Cys Thr Cys Ser Tyr Ser Gly Leu Ser Ser Arg Ser
                885                 890                 895

Cys Thr Thr Leu Glu Asn Tyr Asn Phe Glu Leu Tyr Asp Gly Leu Lys
            900                 905                 910

His Lys Val Lys Met Asn His Gln Lys Cys Cys Ser Glu Ala
        915                 920                 925

<210>  99
<211>  6605
<212>  DNA
<213>  human

<220>
<221>  CDS
<222>  (792)..(3521)

<400>  99
cagagatcgc gagcgaggca ccagcctgca gccggccccc agcacatcct cagccgcaca      60

gacactcggc gaggtggagg tgagggcggg cgccagcgaa ctcggagagg ggctcgctca     120

ctcccaggcg atcccagccg ccaccgccgc cgcaccagca gcagcaacag cagcagcagc     180

ttccttcctc agactcccct cgagaggctg gccaagcggg tgtagccgtt gggggaggct     240

cccgccgggg gaacccggcg aggacaagag cagggcggcc gccttccact cgggctgtcc     300

ggcggcggct gcctccgccc gtgtgtccgt caagggtgcc gcgggatgtg tgtcagttta 360

cgcctctgag atcacacagc tgcctggggg ccgtgtgatg cccaaggcaa gtcttggttt 420

taattattat tattatcatt attgttacgc ttggctttcg ggaaatactc gtgatatttg 480

taggataaag gaaatgacac tttgaggaac tggagagaac atatatgcgt tttgttttta 540

agaggaaaac cgtgttctct tcccggcttg ttccctcttt gctgatttca ggagctactc 600

tcctcctggt gaggtggaaa ttccagcaag aatagaggtg aagacaagcc accaggactc 660

aggagggaaa cgctgaccat tagaaacctc tgcataagac gttgtaagga ggaaaataaa 720

agagagaaaa acacaaagat ttaaacaaga aacctacgaa cccagctctg gaaagagcca 780

ccttctccaa a atg gat atg ttt cct ctc acc tgg gtt ttc tta gcc ctc 830
Met Asp Met Phe Pro Leu Thr Trp Val Phe Leu Ala Leu
1               5                   10

tac ttt tca aga cac caa gtg aga ggc caa cca gac cca ccg tgc gga 878
Tyr Phe Ser Arg His Gln Val Arg Gly Gln Pro Asp Pro Pro Cys Gly
    15                  20                  25

ggt cgt ttg aat tcc aaa gat gct ggc tat atc acc tct ccc ggt tac 926
Gly Arg Leu Asn Ser Lys Asp Ala Gly Tyr Ile Thr Ser Pro Gly Tyr
30                  35                  40                  45

ccc cag gac tac ccc tcc cac cag aac tgc gag tgg att gtt tac gcc 974
Pro Gln Asp Tyr Pro Ser His Gln Asn Cys Glu Trp Ile Val Tyr Ala
                50                  55                  60

ccc gaa ccc aac cag aag att gtc ctc aac ttc aac cct cac ttt gaa 1022
Pro Glu Pro Asn Gln Lys Ile Val Leu Asn Phe Asn Pro His Phe Glu
            65                  70                  75

atc gag aag cac gac tgc aag tat gac ttt atc gag att cgg gat ggg 1070
Ile Glu Lys His Asp Cys Lys Tyr Asp Phe Ile Glu Ile Arg Asp Gly
        80                  85                  90

gac agt gaa tcc gca gac ctc ctg ggc aaa cac tgt ggg aac atc gcc 1118
Asp Ser Glu Ser Ala Asp Leu Leu Gly Lys His Cys Gly Asn Ile Ala
    95                  100                 105

ccg ccc acc atc atc tcc tcg ggc tcc atg ctc tac atc aag ttc acc 1166
Pro Pro Thr Ile Ile Ser Ser Gly Ser Met Leu Tyr Ile Lys Phe Thr
110                 115                 120                 125

tcc gac tac gcc cgg cag ggg gca ggc ttc tct ctg cgc tac gag atc 1214
Ser Asp Tyr Ala Arg Gln Gly Ala Gly Phe Ser Leu Arg Tyr Glu Ile
                130                 135                 140

ttc aag aca ggc tct gaa gat tgc tca aaa aac ttc aca agc ccc aac 1262
Phe Lys Thr Gly Ser Glu Asp Cys Ser Lys Asn Phe Thr Ser Pro Asn
            145                 150                 155

ggg acc atc gaa tct cct ggg ttt cct gag aag tat cca cac aac ttg 1310
Gly Thr Ile Glu Ser Pro Gly Phe Pro Glu Lys Tyr Pro His Asn Leu
        160                 165                 170

gac tgc acc ttt acc atc ctg gcc aaa ccc aag atg gag atc atc ctg 1358
Asp Cys Thr Phe Thr Ile Leu Ala Lys Pro Lys Met Glu Ile Ile Leu
    175                 180                 185

cag ttc ctg atc ttt gac ctg gag cat gac cct ttg cag gtg gga gag 1406
Gln Phe Leu Ile Phe Asp Leu Glu His Asp Pro Leu Gln Val Gly Glu
190                 195                 200                 205

ggg gac tgc aag tac gat tgg ctg gac atc tgg gat ggc att cca cat 1454
Gly Asp Cys Lys Tyr Asp Trp Leu Asp Ile Trp Asp Gly Ile Pro His
                210                 215                 220

gtt ggc ccc ctg att ggc aag tac tgt ggg acc aaa aca ccc tct gaa 1502
Val Gly Pro Leu Ile Gly Lys Tyr Cys Gly Thr Lys Thr Pro Ser Glu
            225                 230                 235

ctt cgt tca tcg acg ggg atc ctc tcc ctg acc ttt cac acg gac atg 1550
Leu Arg Ser Ser Thr Gly Ile Leu Ser Leu Thr Phe His Thr Asp Met
        240                 245                 250

gcg gtg gcc aag gat ggc ttc tct gcg cgt tac tac ctg gtc cac caa 1598
Ala Val Ala Lys Asp Gly Phe Ser Ala Arg Tyr Tyr Leu Val His Gln
    255                 260                 265

gag cca cta gag aac ttt cag tgc aat gtt cct ctg ggc atg gag tct 1646
Glu Pro Leu Glu Asn Phe Gln Cys Asn Val Pro Leu Gly Met Glu Ser
270                 275                 280                 285

ggc cgg att gct aat gaa cag atc agt gcc tca tct acc tac tct gat 1694
Gly Arg Ile Ala Asn Glu Gln Ile Ser Ala Ser Ser Thr Tyr Ser Asp
                290                 295                 300

ggg agg tgg acc cct caa caa agc cgg ctc cat ggt gat gac aat ggc 1742
Gly Arg Trp Thr Pro Gln Gln Ser Arg Leu His Gly Asp Asp Asn Gly
            305                 310                 315

tgg acc ccc aac ttg gat tcc aac aag gag tat ctc cag gtg gac ctg 1790
Trp Thr Pro Asn Leu Asp Ser Asn Lys Glu Tyr Leu Gln Val Asp Leu
        320                 325                 330

cgc ttt tta acc atg ctc acg gcc atc gca aca cag gga gcg att tcc 1838
Arg Phe Leu Thr Met Leu Thr Ala Ile Ala Thr Gln Gly Ala Ile Ser
    335                 340                 345

agg gaa aca cag aat ggc tac tat gtc aaa tcc tac aag ctg gaa gtc 1886
Arg Glu Thr Gln Asn Gly Tyr Tyr Val Lys Ser Tyr Lys Leu Glu Val
350                 355                 360                 365

agc act aat gga gag gac tgg atg gtg tac cgg cat ggc aaa aac cac 1934
Ser Thr Asn Gly Glu Asp Trp Met Val Tyr Arg His Gly Lys Asn His
                370                 375                 380

aag gta ttt caa gcc aac aac gat gca act gag gtg gtt ctg aac aag 1982
Lys Val Phe Gln Ala Asn Asn Asp Ala Thr Glu Val Val Leu Asn Lys
            385                 390                 395

ctc cac gct cca ctg ctg aca agg ttt gtt aga atc cgc cct cag acc 2030
Leu His Ala Pro Leu Leu Thr Arg Phe Val Arg Ile Arg Pro Gln Thr
        400                 405                 410

tgg cac tca ggt atc gcc ctc cgg ctg gag ctc ttc ggc tgc cgg gtc 2078
Trp His Ser Gly Ile Ala Leu Arg Leu Glu Leu Phe Gly Cys Arg Val
    415                 420                 425

aca gat gct ccc tgc tcc aac atg ctg ggg atg ctc tca ggc ctc att 2126
Thr Asp Ala Pro Cys Ser Asn Met Leu Gly Met Leu Ser Gly Leu Ile
430                 435                 440                 445

gca gac tcc cag atc tcc gcc tct tcc acc cag gaa tac ctc tgg agc 2174
Ala Asp Ser Gln Ile Ser Ala Ser Ser Thr Gln Glu Tyr Leu Trp Ser
                450                 455                 460

ccc agt gca gcc cgc ctg gtc agc agc cgc tcg ggc tgg ttc cct cga 2222
Pro Ser Ala Ala Arg Leu Val Ser Ser Arg Ser Gly Trp Phe Pro Arg
            465                 470                 475

atc cct cag gcc cag ccc ggt gag gag tgg ctt cag gta gat ctg gga 2270
Ile Pro Gln Ala Gln Pro Gly Glu Glu Trp Leu Gln Val Asp Leu Gly
        480                 485                 490

aca ccc aag aca gtg aaa ggt gtc atc atc cag gga gcc cgc gga gga 2318
Thr Pro Lys Thr Val Lys Gly Val Ile Ile Gln Gly Ala Arg Gly Gly
    495                 500                 505

gac agt atc act gct gtg gaa gcc aga gca ttt gtg cgc aag ttc aaa 2366
Asp Ser Ile Thr Ala Val Glu Ala Arg Ala Phe Val Arg Lys Phe Lys
510                 515                 520                 525

gtc tcc tac agc cta aac ggc aag gac tgg gaa tac att cag gac ccc 2414
Val Ser Tyr Ser Leu Asn Gly Lys Asp Trp Glu Tyr Ile Gln Asp Pro
                530                 535                 540

agg acc cag cag cca aag ctg ttc gaa ggg aac atg cac tat gac acc 2462
Arg Thr Gln Gln Pro Lys Leu Phe Glu Gly Asn Met His Tyr Asp Thr
            545                 550                 555

cct gac atc cga agg ttt gac ccc att ccg gca cag tat gtg cgg gta 2510
Pro Asp Ile Arg Arg Phe Asp Pro Ile Pro Ala Gln Tyr Val Arg Val
        560                 565                 570

tac ccg gag agg tgg tcg ccg gcg ggg att ggg atg cgg ctg gag gtg 2558
Tyr Pro Glu Arg Trp Ser Pro Ala Gly Ile Gly Met Arg Leu Glu Val
    575                 580                 585

ctg ggc tgt gac tgg aca gac tcc aag ccc acg gta gag acg ctg gga 2606
Leu Gly Cys Asp Trp Thr Asp Ser Lys Pro Thr Val Glu Thr Leu Gly
590                 595                 600                 605

ccc act gtg aag agc gaa gag aca acc acc ccc tac ccc acc gaa gag 2654
Pro Thr Val Lys Ser Glu Glu Thr Thr Thr Pro Tyr Pro Thr Glu Glu
                610                 615                 620

gag gcc aca gag tgt ggg gag aac tgc agc ttt gag gat gac aaa gat 2702
Glu Ala Thr Glu Cys Gly Glu Asn Cys Ser Phe Glu Asp Asp Lys Asp
            625                 630                 635

ttg cag ctc cct tcg gga ttc aat tgc aac ttc gat ttc ctc gag gag 2750
Leu Gln Leu Pro Ser Gly Phe Asn Cys Asn Phe Asp Phe Leu Glu Glu
        640                 645                 650

ccc tgt ggt tgg atg tat gac cat gcc aag tgg ctc cgg acc acc tgg 2798
Pro Cys Gly Trp Met Tyr Asp His Ala Lys Trp Leu Arg Thr Thr Trp
    655                 660                 665

gcc agc agc tcc agc cca aac gac cgg acg ttt cca gat gac agg aat 2846
Ala Ser Ser Ser Ser Pro Asn Asp Arg Thr Phe Pro Asp Asp Arg Asn
670                 675                 680                 685

ttc ttg cgg ctg cag agt gac agc cag aga gag ggc cag tat gcc cgg 2894
Phe Leu Arg Leu Gln Ser Asp Ser Gln Arg Glu Gly Gln Tyr Ala Arg
                690                 695                 700

ctc atc agc ccc cct gtc cac ctg ccc cga agc ccg gtg tgc atg gag 2942
Leu Ile Ser Pro Pro Val His Leu Pro Arg Ser Pro Val Cys Met Glu
            705                 710                 715

ttc cag tac cag gcc acg ggc ggc cgc ggg gtg gcg ctg cag gtg gtg 2990
Phe Gln Tyr Gln Ala Thr Gly Gly Arg Gly Val Ala Leu Gln Val Val
        720                 725                 730

cgg gaa gcc agc cag gag agc aag ttg ctg tgg gtc atc cgt gag gac 3038
Arg Glu Ala Ser Gln Glu Ser Lys Leu Leu Trp Val Ile Arg Glu Asp
    735                 740                 745

cag ggc ggc gag tgg aag cac ggg cgg atc atc ctg ccc agc tac gac 3086
Gln Gly Gly Glu Trp Lys His Gly Arg Ile Ile Leu Pro Ser Tyr Asp
750                 755                 760                 765

atg gag tac cag att gtg ttc gag gga gtg ata ggg aaa gga cgt tcc 3134
Met Glu Tyr Gln Ile Val Phe Glu Gly Val Ile Gly Lys Gly Arg Ser
                770                 775                 780

gga gag att gcc att gat gac att cgg ata agc act gat gtc cca ctg 3182
Gly Glu Ile Ala Ile Asp Asp Ile Arg Ile Ser Thr Asp Val Pro Leu
            785                 790                 795

gag aac tgc atg gaa ccc atc tcg gct ttt gca gat gaa tac gag gtg 3230
Glu Asn Cys Met Glu Pro Ile Ser Ala Phe Ala Asp Glu Tyr Glu Val
        800                 805                 810

gac tgg agc aat tct tct tct gca acc tca ggg tct ggc gcc ccc tcg 3278
Asp Trp Ser Asn Ser Ser Ser Ala Thr Ser Gly Ser Gly Ala Pro Ser
    815                 820                 825

acc gac aaa gaa aag agc tgg ctg tac acc ctg gat ccc atc ctc atc 3326
Thr Asp Lys Glu Lys Ser Trp Leu Tyr Thr Leu Asp Pro Ile Leu Ile
830                 835                 840                 845

acc atc atc gcc atg agc tca ctg ggc gtc ctc ctg ggg gcc acc tgt 3374
Thr Ile Ile Ala Met Ser Ser Leu Gly Val Leu Leu Gly Ala Thr Cys
                850                 855                 860

gca ggc ctc ctg ctc tac tgc acc tgt tcc tac tcg ggc ctg agc tcc 3422
Ala Gly Leu Leu Leu Tyr Cys Thr Cys Ser Tyr Ser Gly Leu Ser Ser
            865                 870                 875

cga agc tgc acc aca ctg gag aac tac aac ttc gag ctc tac gat ggc 3470
Arg Ser Cys Thr Thr Leu Glu Asn Tyr Asn Phe Glu Leu Tyr Asp Gly
        880                 885                 890

ctt aag cac aag gtc aag atg aac cac caa aag tgc tgc tcc gag gca 3518
Leu Lys His Lys Val Lys Met Asn His Gln Lys Cys Cys Ser Glu Ala
    895                 900                 905

tga cggattgcac ctgaatccta tctgacgttt cattccagca agaggggctg 3571

gggaagatta catttttttt tcctttggaa actgaatgcc ataatctcga tcaaaccgat 3631

ccagaatacc gaaggtatgg acaggacaga aaagcgagtc gcaggaggaa gggagatgca 3691

gccgcacagg ggatgattac cctcctagga ccgcggtggc taagtcattg caggaacggg 3751

gctgtgttct ctgctgggac aaaacaggag ctcatctctt tggggtcaca gttctatttt 3811

gtttgtgagt ttgtattatt attattatta ttattattat tatattttat ttctttggtc 3871

tgtgagcaac tcaaagaggc agaagaggag aatgactttt ccagaataga agtggagcag 3931

tgatcattat tctccgcttt ctctttctaa tcaacacttg aaaagcaaag tgtcttttca 3991

gcctttccat ctttacaaat aaaactcaaa aaagccgtcc agcttatccc atcctctgat 4051

tgtcttctga cttaagggat ttactgtggt gtaggttctg ccagccaacc ctacaagctg 4111

ccatttccag tcctagcatt taagtaggat gttgttgcct ttaacttttc ttatccaggg 4171

gaaaattgcc attttagggt cagcatgaac agctctttct tgtatgcgat ttaaaacaaa 4231

ctggaaagga aacttcacac gtcaaaatcc atagaagcgc ctggacgagg cttaaagtgc 4291

tttgtgagtg aataggagcc attcgctaat tctagaccca cagtgtctgg tggtggggct 4351

tcccttgtgg ggcttctggt ggtggttttg ccttttcttt tccctcctcc atgttcttct 4411
aaaacatata catatataca tacacacata cacatattct tcaggtctct aagcccctgg 4471
aagcagcatt gtgtgatatt ctcagaggca ggggaaaata gagggaaaaa tagagactat 4531
tggtatgttc tccccatcag cgagttattg taactggtca ccactggacg ggaaggagaa 4591
cagaggagag ggaaagagaa gcccaacctc tgtgatcata tgagggccaa ggctgagcag 4651
tgtagacaga gaccctttga aatgcatttg tctctcaaat agactagtaa acaccgactt 4711
ctcctttggg ttacaaacac catttcaacc tttcgggaga gtcagagcta ggatgtacaa 4771
gaactgattc taaccagaag tccgcaagta ctgtggacaa gaatgcttaa ccatgctgct 4831
tcagccttga gagacctagg ttcttacaca tatgcacaca cgcatacaca catgcacgca 4891
cacacacata cacacatgca cgcacgcacg catgcacacc aatttatgtt tttattaagt 4951
gccttgaaaa aatgaagaaa aatgtatttt ccctttatgt aaaaattagt gaatatctta 5011
tgaattaagg cattcctctt tccctaaccc cgatggctcc attcccaagt accccaactc 5071
actgctgatc ctattaaagg aatgagtcct gctacccgag tggtagtcat agccctagat 5131
gactctcaac tactcttcaa agggaggcat caggaataga atgaaactgt gtgaaggata 5191
agattgttcg catcaagatc caaatcttga tttcatatta acgcctaagg attgcctgtg 5251
tgctggaaat atatttgaaa ctcaaccagt atgcccagcc tattgcatat cattgtcaga 5311
ccatttttgc tgctgtggtc acccacgatt tcatttgtct tatacccagg tgaaagggga 5371
agggtgaatg ggactggctg gttcctttaa atgttaactt atggaaatgc tagttcaaat 5431
ggtaatgtca cagtgttttg tatgcagaga gcaagagttc aaccaacagc tgtttattca 5491
tgtgtgtgtg tctttgctgc tttgagttct ctgtatctac tgtgtatgtg aatggtcatg 5551
tgggactcag tggtggtgtt gtgactttga cctagggtcc gagtgtcaca gctgatcttg 5611
gcactcggca ctcattggca cagtggtagt tagaggtgaa aagtagagct gtcaagccca 5671
agggcttagc tttagggctc ctcctgagtt cggcccacag tagaagcaag attttaacta 5731
gccccttttc ctcttcaccc tcccatgatg cgcagtgttc agaaagctgg taagtcctag 5791
ggatttccag aagtagcctg cagaagaagg taagtttgaa agccactcca ggggtcctga 5851
tgctgtcatg ctcagtgagc cattttacag ttctccaaag tctagccctg tttcggacct 5911
gcacttcacc tctaagttat gtacaactca acctgcatcc ctctaaaagt cctatatcca 5971
tattcaccat tggctaattt gaggccctga gtgggccttg aatgctaaaa agaagcaggg 6031
tacgcagggc tacatgtaga taccacacca aggctggagg ctggtctgtc ataagacaga 6091
aagaaagacg ctgggcccaa ttttgacttg gccaggggac accttggtgt gtttgttatc 6151
tttatctgtg ggtaggctag ctgacccatc tccttgagtc attccctttg ggaaacccca 6211
ctgccagtat tgatctcctt tttgccttgt actgaatgac acattacctc cacactctcc 6271
cggactaggt ggtcaacagg gccacagggt tgctttctgt ctttggtggg gcaggggagt 6331
tgacagggat gagggtccaa ggaataagca tgaatgacaa gaaaacaagg gaaagagtta 6391

acctgtcaca tagcaggtta actttttcag ggtttgcagt tagaggtatt cgaccattca 6451

ctggctgagc cagatcacgg gaacttgaga gcttttactg tgattcttca atgtaaaaaa 6511

taaacaacaa tgtcaaactg tgtttatatg atttgtataa agccttttta agattactat 6571

ttaaataaac attataccag agataaaaaa aaaa 6605

<210> 100
<211> 909
<212> PRT
<213> human

<400> 100

Met Asp Met Phe Pro Leu Thr Trp Val Phe Leu Ala Leu Tyr Phe Ser
1               5                   10                  15

Arg His Gln Val Arg Gly Gln Pro Asp Pro Pro Cys Gly Gly Arg Leu
            20                  25                  30

Asn Ser Lys Asp Ala Gly Tyr Ile Thr Ser Pro Gly Tyr Pro Gln Asp
        35                  40                  45

Tyr Pro Ser His Gln Asn Cys Glu Trp Ile Val Tyr Ala Pro Glu Pro
    50                  55                  60

Asn Gln Lys Ile Val Leu Asn Phe Asn Pro His Phe Glu Ile Glu Lys
65                  70                  75                  80

His Asp Cys Lys Tyr Asp Phe Ile Glu Ile Arg Asp Gly Asp Ser Glu
                85                  90                  95

Ser Ala Asp Leu Leu Gly Lys His Cys Gly Asn Ile Ala Pro Pro Thr
            100                 105                 110

Ile Ile Ser Ser Gly Ser Met Leu Tyr Ile Lys Phe Thr Ser Asp Tyr
        115                 120                 125

Ala Arg Gln Gly Ala Gly Phe Ser Leu Arg Tyr Glu Ile Phe Lys Thr
    130                 135                 140

Gly Ser Glu Asp Cys Ser Lys Asn Phe Thr Ser Pro Asn Gly Thr Ile
145                 150                 155                 160

Glu Ser Pro Gly Phe Pro Glu Lys Tyr Pro His Asn Leu Asp Cys Thr
                165                 170                 175

Phe Thr Ile Leu Ala Lys Pro Lys Met Glu Ile Ile Leu Gln Phe Leu
            180                 185                 190

Ile Phe Asp Leu Glu His Asp Pro Leu Gln Val Gly Glu Gly Asp Cys
        195                 200                 205

Lys Tyr Asp Trp Leu Asp Ile Trp Asp Gly Ile Pro His Val Gly Pro
    210                 215                 220

Leu Ile Gly Lys Tyr Cys Gly Thr Lys Thr Pro Ser Glu Leu Arg Ser
225                 230                 235                 240

Ser Thr Gly Ile Leu Ser Leu Thr Phe His Thr Asp Met Ala Val Ala
                245                 250                 255

Lys Asp Gly Phe Ser Ala Arg Tyr Tyr Leu Val His Gln Glu Pro Leu
            260                 265                 270

Glu Asn Phe Gln Cys Asn Val Pro Leu Gly Met Glu Ser Gly Arg Ile
        275                 280                 285

Ala Asn Glu Gln Ile Ser Ala Ser Ser Thr Tyr Ser Asp Gly Arg Trp
    290                 295                 300

Thr Pro Gln Gln Ser Arg Leu His Gly Asp Asp Asn Gly Trp Thr Pro
305                 310                 315                 320

Asn Leu Asp Ser Asn Lys Glu Tyr Leu Gln Val Asp Leu Arg Phe Leu
                325                 330                 335

Thr Met Leu Thr Ala Ile Ala Thr Gln Gly Ala Ile Ser Arg Glu Thr
            340                 345                 350

Gln Asn Gly Tyr Tyr Val Lys Ser Tyr Lys Leu Glu Val Ser Thr Asn
        355                 360                 365

Gly Glu Asp Trp Met Val Tyr Arg His Gly Lys Asn His Lys Val Phe
    370                 375                 380

Gln Ala Asn Asn Asp Ala Thr Glu Val Val Leu Asn Lys Leu His Ala
385                 390                 395                 400

Pro Leu Leu Thr Arg Phe Val Arg Ile Arg Pro Gln Thr Trp His Ser
                405                 410                 415

Gly Ile Ala Leu Arg Leu Glu Leu Phe Gly Cys Arg Val Thr Asp Ala
            420                 425                 430

Pro Cys Ser Asn Met Leu Gly Met Leu Ser Gly Leu Ile Ala Asp Ser
        435                 440                 445

Gln Ile Ser Ala Ser Ser Thr Gln Glu Tyr Leu Trp Ser Pro Ser Ala
    450                 455                 460

Ala Arg Leu Val Ser Ser Arg Ser Gly Trp Phe Pro Arg Ile Pro Gln
465                 470                 475                 480

Ala Gln Pro Gly Glu Glu Trp Leu Gln Val Asp Leu Gly Thr Pro Lys
                485                 490                 495

Thr Val Lys Gly Val Ile Ile Gln Gly Ala Arg Gly Gly Asp Ser Ile
            500                 505                 510

Thr Ala Val Glu Ala Arg Ala Phe Val Arg Lys Phe Lys Val Ser Tyr
        515                 520                 525

Ser Leu Asn Gly Lys Asp Trp Glu Tyr Ile Gln Asp Pro Arg Thr Gln
    530                 535                 540

Gln Pro Lys Leu Phe Glu Gly Asn Met His Tyr Asp Thr Pro Asp Ile
545                 550                 555                 560

Arg Arg Phe Asp Pro Ile Pro Ala Gln Tyr Val Arg Val Tyr Pro Glu
                565                 570                 575

Arg Trp Ser Pro Ala Gly Ile Gly Met Arg Leu Glu Val Leu Gly Cys
            580                 585                 590

Asp Trp Thr Asp Ser Lys Pro Thr Val Glu Thr Leu Gly Pro Thr Val
        595                 600                 605

Lys Ser Glu Glu Thr Thr Thr Pro Tyr Pro Thr Glu Glu Glu Ala Thr
    610                 615                 620

Glu Cys Gly Glu Asn Cys Ser Phe Glu Asp Asp Lys Asp Leu Gln Leu
625                 630                 635                 640

Pro Ser Gly Phe Asn Cys Asn Phe Asp Phe Leu Glu Glu Pro Cys Gly
                645                 650                 655

Trp Met Tyr Asp His Ala Lys Trp Leu Arg Thr Thr Trp Ala Ser Ser
            660                 665                 670

Ser Ser Pro Asn Asp Arg Thr Phe Pro Asp Asp Arg Asn Phe Leu Arg
        675                 680                 685

Leu Gln Ser Asp Ser Gln Arg Glu Gly Gln Tyr Ala Arg Leu Ile Ser
    690                 695                 700

Pro Pro Val His Leu Pro Arg Ser Pro Val Cys Met Glu Phe Gln Tyr
705                 710                 715                 720

Gln Ala Thr Gly Gly Arg Gly Val Ala Leu Gln Val Val Arg Glu Ala
                725                 730                 735

Ser Gln Glu Ser Lys Leu Leu Trp Val Ile Arg Glu Asp Gln Gly Gly
            740                 745                 750

Glu Trp Lys His Gly Arg Ile Ile Leu Pro Ser Tyr Asp Met Glu Tyr
        755                 760                 765

Gln Ile Val Phe Glu Gly Val Ile Gly Lys Gly Arg Ser Gly Glu Ile
    770                 775                 780

Ala Ile Asp Asp Ile Arg Ile Ser Thr Asp Val Pro Leu Glu Asn Cys
785                 790                 795                 800

Met Glu Pro Ile Ser Ala Phe Ala Asp Glu Tyr Glu Val Asp Trp Ser
                805                 810                 815

Asn Ser Ser Ser Ala Thr Ser Gly Ser Gly Ala Pro Ser Thr Asp Lys
            820                 825                 830

Glu Lys Ser Trp Leu Tyr Thr Leu Asp Pro Ile Leu Ile Thr Ile Ile
        835                 840                 845

Ala Met Ser Ser Leu Gly Val Leu Leu Gly Ala Thr Cys Ala Gly Leu
    850                 855                 860

Leu Leu Tyr Cys Thr Cys Ser Tyr Ser Gly Leu Ser Ser Arg Ser Cys
865                 870                 875                 880

Thr Thr Leu Glu Asn Tyr Asn Phe Glu Leu Tyr Asp Gly Leu Lys His
                885                 890                 895

Lys Val Lys Met Asn His Gln Lys Cys Cys Ser Glu Ala
            900                 905

<210>  101
<211>  4163
<212>  DNA
<213>  human

<220>
<221>  CDS
<222>  (792)..(3512)

<400>  101
   cagagatcgc gagcgaggca ccagcctgca gccggccccc agcacatcct cagccgcaca      60
   gacactcggc gaggtggagg tgagggcggg cgccagcgaa ctcggagagg ggctcgctca     120
   ctcccaggcg atcccagccg ccaccgccgc cgcaccagca gcagcaacag cagcagcagc     180
   ttccttcctc agactcccct cgagaggctg gccaagcggg tgtagccgtt gggggaggct     240
   cccgccgggg gaacccggcg aggacaagag cagggcggcc gccttccact cgggctgtcc     300
   ggcggcggct gcctccgccc gtgtgtccgt caagggtgcc gcgggatgtg tgtcagttta     360
   cgcctctgag atcacacagc tgcctggggg ccgtgtgatg cccaaggcaa gtcttggttt     420
   taattattat tattatcatt attgttacgc ttggctttcg ggaaatactc gtgatatttg     480
   taggataaag gaaatgacac tttgaggaac tggagagaac atatatgcgt tttgttttta 540
   agaggaaaac cgtgttctct tcccggcttg ttccctcttt gctgatttca ggagctactc 600
   tcctcctggt gaggtggaaa ttccagcaag aatagaggtg aagacaagcc accaggactc 660
   aggagggaaa cgctgaccat tagaaacctc tgcataagac gttgtaagga ggaaaataaa 720
   agagagaaaa acacaaagat ttaaacaaga aacctacgaa cccagctctg gaaagagcca 780
   ccttctccaa a atg gat atg ttt cct ctc acc tgg gtt ttc tta gcc ctc 830
   Met Asp Met Phe Pro Leu Thr Trp Val Phe Leu Ala Leu
   1 5 10
   tac ttt tca aga cac caa gtg aga ggc caa cca gac cca ccg tgc gga 878
   Tyr Phe Ser Arg His Gln Val Arg Gly Gln Pro Asp Pro Pro Cys Gly
   15 20 25
   ggt cgt ttg aat tcc aaa gat gct ggc tat atc acc tct ccc ggt tac 926
   Gly Arg Leu Asn Ser Lys Asp Ala Gly Tyr Ile Thr Ser Pro Gly Tyr
   30 35 40 45
   ccc cag gac tac ccc tcc cac cag aac tgc gag tgg att gtt tac gcc 974
   Pro Gln Asp Tyr Pro Ser His Gln Asn Cys Glu Trp Ile Val Tyr Ala
   50 55 60
   ccc gaa ccc aac cag aag att gtc ctc aac ttc aac cct cac ttt gaa 1022
   Pro Glu Pro Asn Gln Lys Ile Val Leu Asn Phe Asn Pro His Phe Glu
   65 70 75
   atc gag aag cac gac tgc aag tat gac ttt atc gag att cgg gat ggg 1070
   Ile Glu Lys His Asp Cys Lys Tyr Asp Phe Ile Glu Ile Arg Asp Gly
   80 85 90
   gac agt gaa tcc gca gac ctc ctg ggc aaa cac tgt ggg aac atc gcc 1118
   Asp Ser Glu Ser Ala Asp Leu Leu Gly Lys His Cys Gly Asn Ile Ala
   95 100 105
   ccg ccc acc atc atc tcc tcg ggc tcc atg ctc tac atc aag ttc acc 1166
   Pro Pro Thr Ile Ile Ser Ser Gly Ser Met Leu Tyr Ile Lys Phe Thr
   110 115 120 125
   tcc gac tac gcc cgg cag ggg gca ggc ttc tct ctg cgc tac gag atc 1214
   Ser Asp Tyr Ala Arg Gln Gly Ala Gly Phe Ser Leu Arg Tyr Glu Ile
   130 135 140
   ttc aag aca ggc tct gaa gat tgc tca aaa aac ttc aca agc ccc aac 1262
   Phe Lys Thr Gly Ser Glu Asp Cys Ser Lys Asn Phe Thr Ser Pro Asn
   145 150 155
   ggg acc atc gaa tct cct ggg ttt cct gag aag tat cca cac aac ttg 1310
   Gly Thr Ile Glu Ser Pro Gly Phe Pro Glu Lys Tyr Pro His Asn Leu
   160 165 170
   gac tgc acc ttt acc atc ctg gcc aaa ccc aag atg gag atc atc ctg 1358
   Asp Cys Thr Phe Thr Ile Leu Ala Lys Pro Lys Met Glu Ile Ile Leu
   175 180 185
   cag ttc ctg atc ttt gac ctg gag cat gac cct ttg cag gtg gga gag 1406
   Gln Phe Leu Ile Phe Asp Leu Glu His Asp Pro Leu Gln Val Gly Glu
   190 195 200 205
   ggg gac tgc aag tac gat tgg ctg gac atc tgg gat ggc att cca cat 1454
   Gly Asp Cys Lys Tyr Asp Trp Leu Asp Ile Trp Asp Gly Ile Pro His
   210 215 220
   gtt ggc ccc ctg att ggc aag tac tgt ggg acc aaa aca ccc tct gaa 1502
   Val Gly Pro Leu Ile Gly Lys Tyr Cys Gly Thr Lys Thr Pro Ser Glu
   225 230 235
   ctt cgt tca tcg acg ggg atc ctc tcc ctg acc ttt cac acg gac atg 1550
   Leu Arg Ser Ser Thr Gly Ile Leu Ser Leu Thr Phe His Thr Asp Met
   240 245 250
   gcg gtg gcc aag gat ggc ttc tct gcg cgt tac tac ctg gtc cac caa 1598
   Ala Val Ala Lys Asp Gly Phe Ser Ala Arg Tyr Tyr Leu Val His Gln
   255 260 265
   gag cca cta gag aac ttt cag tgc aat gtt cct ctg ggc atg gag tct 1646
   Glu Pro Leu Glu Asn Phe Gln Cys Asn Val Pro Leu Gly Met Glu Ser
   270 275 280 285
   ggc cgg att gct aat gaa cag atc agt gcc tca tct acc tac tct gat 1694
   Gly Arg Ile Ala Asn Glu Gln Ile Ser Ala Ser Ser Thr Tyr Ser Asp
   290 295 300
   ggg agg tgg acc cct caa caa agc cgg ctc cat ggt gat gac aat ggc 1742
   Gly Arg Trp Thr Pro Gln Gln Ser Arg Leu His Gly Asp Asp Asn Gly
   305 310 315
   tgg acc ccc aac ttg gat tcc aac aag gag tat ctc cag gtg gac ctg 1790
   Trp Thr Pro Asn Leu Asp Ser Asn Lys Glu Tyr Leu Gln Val Asp Leu
   320 325 330
   cgc ttt tta acc atg ctc acg gcc atc gca aca cag gga gcg att tcc 1838
   Arg Phe Leu Thr Met Leu Thr Ala Ile Ala Thr Gln Gly Ala Ile Ser
   335 340 345
   agg gaa aca cag aat ggc tac tat gtc aaa tcc tac aag ctg gaa gtc 1886
   Arg Glu Thr Gln Asn Gly Tyr Tyr Val Lys Ser Tyr Lys Leu Glu Val
   350 355 360 365
   agc act aat gga gag gac tgg atg gtg tac cgg cat ggc aaa aac cac 1934
   Ser Thr Asn Gly Glu Asp Trp Met Val Tyr Arg His Gly Lys Asn His
   370 375 380
   aag gta ttt caa gcc aac aac gat gca act gag gtg gtt ctg aac aag 1982
   Lys Val Phe Gln Ala Asn Asn Asp Ala Thr Glu Val Val Leu Asn Lys
   385 390 395
   ctc cac gct cca ctg ctg aca agg ttt gtt aga atc cgc cct cag acc 2030
   Leu His Ala Pro Leu Leu Thr Arg Phe Val Arg Ile Arg Pro Gln Thr
   400 405 410
   tgg cac tca ggt atc gcc ctc cgg ctg gag ctc ttc ggc tgc cgg gtc 2078
   Trp His Ser Gly Ile Ala Leu Arg Leu Glu Leu Phe Gly Cys Arg Val
   415 420 425
   aca gat gct ccc tgc tcc aac atg ctg ggg atg ctc tca ggc ctc att 2126
   Thr Asp Ala Pro Cys Ser Asn Met Leu Gly Met Leu Ser Gly Leu Ile
   430 435 440 445
   gca gac tcc cag atc tcc gcc tct tcc acc cag gaa tac ctc tgg agc 2174
   Ala Asp Ser Gln Ile Ser Ala Ser Ser Thr Gln Glu Tyr Leu Trp Ser
   450 455 460
   ccc agt gca gcc cgc ctg gtc agc agc cgc tcg ggc tgg ttc cct cga 2222
   Pro Ser Ala Ala Arg Leu Val Ser Ser Arg Ser Gly Trp Phe Pro Arg
   465 470 475
   atc cct cag gcc cag ccc ggt gag gag tgg ctt cag gta gat ctg gga 2270
   Ile Pro Gln Ala Gln Pro Gly Glu Glu Trp Leu Gln Val Asp Leu Gly
   480 485 490
   aca ccc aag aca gtg aaa ggt gtc atc atc cag gga gcc cgc gga gga 2318
   Thr Pro Lys Thr Val Lys Gly Val Ile Ile Gln Gly Ala Arg Gly Gly
   495                 500                 505
   gac agt atc act gct gtg gaa gcc aga gca ttt gtg cgc aag ttc aaa      2366
   Asp Ser Ile Thr Ala Val Glu Ala Arg Ala Phe Val Arg Lys Phe Lys
   510                 515                 520                 525
   gtc tcc tac agc cta aac ggc aag gac tgg gaa tac att cag gac ccc      2414
   Val Ser Tyr Ser Leu Asn Gly Lys Asp Trp Glu Tyr Ile Gln Asp Pro
                   530                 535                 540
   agg acc cag cag cca aag ctg ttc gaa ggg aac atg cac tat gac acc      2462
   Arg Thr Gln Gln Pro Lys Leu Phe Glu Gly Asn Met His Tyr Asp Thr
               545                 550                 555
   cct gac atc cga agg ttt gac ccc att ccg gca cag tat gtg cgg gta      2510
   Pro Asp Ile Arg Arg Phe Asp Pro Ile Pro Ala Gln Tyr Val Arg Val
           560                 565                 570
   tac ccg gag agg tgg tcg ccg gcg ggg att ggg atg cgg ctg gag gtg      2558
   Tyr Pro Glu Arg Trp Ser Pro Ala Gly Ile Gly Met Arg Leu Glu Val
       575                 580                 585
   ctg ggc tgt gac tgg aca gac tcc aag ccc acg gta gag acg ctg gga      2606
   Leu Gly Cys Asp Trp Thr Asp Ser Lys Pro Thr Val Glu Thr Leu Gly
   590                 595                 600                 605
   ccc act gtg aag agc gaa gag aca acc acc ccc tac ccc acc gaa gag      2654
   Pro Thr Val Lys Ser Glu Glu Thr Thr Thr Pro Tyr Pro Thr Glu Glu
                   610                 615                 620
   gag gcc aca gag tgt ggg gag aac tgc agc ttt gag gat gac aaa gat      2702
   Glu Ala Thr Glu Cys Gly Glu Asn Cys Ser Phe Glu Asp Asp Lys Asp
               625                 630                 635
   ttg cag ctc cct tcg gga ttc aat tgc aac ttc gat ttc ctc gag gag      2750
   Leu Gln Leu Pro Ser Gly Phe Asn Cys Asn Phe Asp Phe Leu Glu Glu
           640                 645                 650
   ccc tgt ggt tgg atg tat gac cat gcc aag tgg ctc cgg acc acc tgg      2798
   Pro Cys Gly Trp Met Tyr Asp His Ala Lys Trp Leu Arg Thr Thr Trp
       655                 660                 665
   gcc agc agc tcc agc cca aac gac cgg acg ttt cca gat gac agg aat      2846
   Ala Ser Ser Ser Ser Pro Asn Asp Arg Thr Phe Pro Asp Asp Arg Asn
   670                 675                 680                 685
   ttc ttg cgg ctg cag agt gac agc cag aga gag ggc cag tat gcc cgg      2894
   Phe Leu Arg Leu Gln Ser Asp Ser Gln Arg Glu Gly Gln Tyr Ala Arg
                   690                 695                 700
   ctc atc agc ccc cct gtc cac ctg ccc cga agc ccg gtg tgc atg gag      2942
   Leu Ile Ser Pro Pro Val His Leu Pro Arg Ser Pro Val Cys Met Glu
               705                 710                 715
   ttc cag tac cag gcc acg ggc ggc cgc ggg gtg gcg ctg cag gtg gtg      2990
   Phe Gln Tyr Gln Ala Thr Gly Gly Arg Gly Val Ala Leu Gln Val Val
           720                 725                 730
   cgg gaa gcc agc cag gag agc aag ttg ctg tgg gtc atc cgt gag gac      3038
   Arg Glu Ala Ser Gln Glu Ser Lys Leu Leu Trp Val Ile Arg Glu Asp
       735                 740                 745
   cag ggc ggc gag tgg aag cac ggg cgg atc atc ctg ccc agc tac gac      3086
   Gln Gly Gly Glu Trp Lys His Gly Arg Ile Ile Leu Pro Ser Tyr Asp
   750                 755                 760                 765
   atg gag tac cag att gtg ttc gag gga gtg ata ggg aaa gga cgt tcc      3134
   Met Glu Tyr Gln Ile Val Phe Glu Gly Val Ile Gly Lys Gly Arg Ser
   770 775 780
   gga gag att gcc att gat gac att cgg ata agc act gat gtc cca ctg 3182
   Gly Glu Ile Ala Ile Asp Asp Ile Arg Ile Ser Thr Asp Val Pro Leu
   785 790 795
   gag aac tgc atg gaa ccc atc tcg gct ttt gca ggt gag aat ttt aaa 3230
   Glu Asn Cys Met Glu Pro Ile Ser Ala Phe Ala Gly Glu Asn Phe Lys
   800 805 810
   ggg ggc acc ctc ctg cca ggg acc gag ccc aca gtg gac acg gtg ccc 3278
   Gly Gly Thr Leu Leu Pro Gly Thr Glu Pro Thr Val Asp Thr Val Pro
   815 820 825
   atg cag ccc atc cca gcc tac tgg tat tac gta atg gcc gcc ggg ggc 3326
   Met Gln Pro Ile Pro Ala Tyr Trp Tyr Tyr Val Met Ala Ala Gly Gly
   830 835 840 845
   gcc gtg ctg gtg ctg gtc tcc gtc gcg ctg gcc ctg gtg ctc cac tac 3374
   Ala Val Leu Val Leu Val Ser Val Ala Leu Ala Leu Val Leu His Tyr
   850 855 860
   cac cgg ttc cgc tat gcg gcc aag aag acc gat cac tcc atc acc tac 3422
   His Arg Phe Arg Tyr Ala Ala Lys Lys Thr Asp His Ser Ile Thr Tyr
   865 870 875
   aaa acc tcc cac tac acc aac ggg gcc cct ctg gcg gtg gag ccc acc 3470
   Lys Thr Ser His Tyr Thr Asn Gly Ala Pro Leu Ala Val Glu Pro Thr
   880 885 890
   cta acc att aag cta gag caa gac cgt ggc tcg cac tgc tga 3512
   Leu Thr Ile Lys Leu Glu Gln Asp Arg Gly Ser His Cys
   895 900 905
   gggccgaagc aagaacagca cccaaaacaa acgagaaaga ctgcaaacat gttgcctcga 3572
   ttttgcactt ttttctcctc gcctagtttc tgtgtgaact ctcagacatc tctttcccgg 3632
   atccccaacc ctgagcactc ttatcaatcc caaccatcct ccttgggttc attttggttt 3692
   ctggtttttc tttttccttt ttgttgattc caaaccaaca aacccaactc taatgctgca 3752
   tcttggacta tccgaagaga tccaccccca agcactccac aactcaaggc tcagctggtt 3812
   ttgttccaga gactggttcg cttgtttttt ccccttgcct tatcccatac ctcctctcag 3872
   tgggcagtct gccaggagac gtgaggggaa gcctggatct gtgtgtatgt acatagtaga 3932
   catgtgtgtg tgtgaatagc tctctgtgtg tgggtgtgtg agagagcggc tggttcattg 3992
   tgtgtgtgtt tgggcgaggg gtgagtgttc agagagggcc cctttaactc ttatgttact 4052
   tctcctgggg tacattttac aagaaaataa tatactgtac aagttttgtt tacttggaga 4112
   agagattgaa gctttttgtt gccttatcta aaaaaaaaaa aaaaaaaaaa a 4163

<210> 102
<211> 906
<212> PRT
<213> human

<400> 102

Met Asp Met Phe Pro Leu Thr Trp Val Phe Leu Ala Leu Tyr Phe Ser
1               5                   10                  15

Arg His Gln Val Arg Gly Gln Pro Asp Pro Pro Cys Gly Gly Arg Leu
            20                  25                  30

Asn Ser Lys Asp Ala Gly Tyr Ile Thr Ser Pro Gly Tyr Pro Gln Asp
        35                  40                  45

Tyr Pro Ser His Gln Asn Cys Glu Trp Ile Val Tyr Ala Pro Glu Pro
    50                  55                  60

Asn Gln Lys Ile Val Leu Asn Phe Asn Pro His Phe Glu Ile Glu Lys
65                  70                  75                  80

His Asp Cys Lys Tyr Asp Phe Ile Glu Ile Arg Asp Gly Asp Ser Glu
                85                  90                  95

Ser Ala Asp Leu Leu Gly Lys His Cys Gly Asn Ile Ala Pro Pro Thr
            100                 105                 110

Ile Ile Ser Ser Gly Ser Met Leu Tyr Ile Lys Phe Thr Ser Asp Tyr
        115                 120                 125

Ala Arg Gln Gly Ala Gly Phe Ser Leu Arg Tyr Glu Ile Phe Lys Thr
    130                 135                 140

Gly Ser Glu Asp Cys Ser Lys Asn Phe Thr Ser Pro Asn Gly Thr Ile
145                 150                 155                 160

Glu Ser Pro Gly Phe Pro Glu Lys Tyr Pro His Asn Leu Asp Cys Thr
                165                 170                 175

Phe Thr Ile Leu Ala Lys Pro Lys Met Glu Ile Ile Leu Gln Phe Leu
            180                 185                 190

Ile Phe Asp Leu Glu His Asp Pro Leu Gln Val Gly Glu Gly Asp Cys
        195                 200                 205

Lys Tyr Asp Trp Leu Asp Ile Trp Asp Gly Ile Pro His Val Gly Pro
    210                 215                 220

Leu Ile Gly Lys Tyr Cys Gly Thr Lys Thr Pro Ser Glu Leu Arg Ser
225                 230                 235                 240

Ser Thr Gly Ile Leu Ser Leu Thr Phe His Thr Asp Met Ala Val Ala
                245                 250                 255

Lys Asp Gly Phe Ser Ala Arg Tyr Tyr Leu Val His Gln Glu Pro Leu
            260                 265                 270

Glu Asn Phe Gln Cys Asn Val Pro Leu Gly Met Glu Ser Gly Arg Ile
        275                 280                 285

Ala Asn Glu Gln Ile Ser Ala Ser Ser Thr Tyr Ser Asp Gly Arg Trp
    290                 295                 300

Thr Pro Gln Gln Ser Arg Leu His Gly Asp Asp Asn Gly Trp Thr Pro
305                 310                 315                 320

Asn Leu Asp Ser Asn Lys Glu Tyr Leu Gln Val Asp Leu Arg Phe Leu
                325                 330                 335

Thr Met Leu Thr Ala Ile Ala Thr Gln Gly Ala Ile Ser Arg Glu Thr
            340                 345                 350

Gln Asn Gly Tyr Tyr Val Lys Ser Tyr Lys Leu Glu Val Ser Thr Asn
        355                 360                 365

Gly Glu Asp Trp Met Val Tyr Arg His Gly Lys Asn His Lys Val Phe
    370                 375                 380

Gln Ala Asn Asn Asp Ala Thr Glu Val Val Leu Asn Lys Leu His Ala
385                 390                 395                 400

Pro Leu Leu Thr Arg Phe Val Arg Ile Arg Pro Gln Thr Trp His Ser
                405                 410                 415

Gly Ile Ala Leu Arg Leu Glu Leu Phe Gly Cys Arg Val Thr Asp Ala
            420                 425                 430

Pro Cys Ser Asn Met Leu Gly Met Leu Ser Gly Leu Ile Ala Asp Ser
        435                 440                 445

Gln Ile Ser Ala Ser Ser Thr Gln Glu Tyr Leu Trp Ser Pro Ser Ala
    450                 455                 460

Ala Arg Leu Val Ser Ser Arg Ser Gly Trp Phe Pro Arg Ile Pro Gln
465                 470                 475                 480

Ala Gln Pro Gly Glu Glu Trp Leu Gln Val Asp Leu Gly Thr Pro Lys
                485                 490                 495

Thr Val Lys Gly Val Ile Ile Gln Gly Ala Arg Gly Gly Asp Ser Ile
            500                 505                 510

Thr Ala Val Glu Ala Arg Ala Phe Val Arg Lys Phe Lys Val Ser Tyr
        515                 520                 525

Ser Leu Asn Gly Lys Asp Trp Glu Tyr Ile Gln Asp Pro Arg Thr Gln
    530                 535                 540

Gln Pro Lys Leu Phe Glu Gly Asn Met His Tyr Asp Thr Pro Asp Ile
545                 550                 555                 560

Arg Arg Phe Asp Pro Ile Pro Ala Gln Tyr Val Arg Val Tyr Pro Glu
                565                 570                 575

Arg Trp Ser Pro Ala Gly Ile Gly Met Arg Leu Glu Val Leu Gly Cys
            580                 585                 590

Asp Trp Thr Asp Ser Lys Pro Thr Val Glu Thr Leu Gly Pro Thr Val
        595                 600                 605

Lys Ser Glu Glu Thr Thr Thr Pro Tyr Pro Thr Glu Glu Glu Ala Thr
    610                 615                 620

Glu Cys Gly Glu Asn Cys Ser Phe Glu Asp Asp Lys Asp Leu Gln Leu
625                 630                 635                 640

Pro Ser Gly Phe Asn Cys Asn Phe Asp Phe Leu Glu Glu Pro Cys Gly
                645                 650                 655

Trp Met Tyr Asp His Ala Lys Trp Leu Arg Thr Thr Trp Ala Ser Ser
            660                 665                 670

Ser Ser Pro Asn Asp Arg Thr Phe Pro Asp Asp Arg Asn Phe Leu Arg
        675                 680                 685

Leu Gln Ser Asp Ser Gln Arg Glu Gly Gln Tyr Ala Arg Leu Ile Ser
    690                 695                 700

Pro Pro Val His Leu Pro Arg Ser Pro Val Cys Met Glu Phe Gln Tyr
705                 710                 715                 720

Gln Ala Thr Gly Gly Arg Gly Val Ala Leu Gln Val Val Arg Glu Ala
                725                 730                 735

Ser Gln Glu Ser Lys Leu Leu Trp Val Ile Arg Glu Asp Gln Gly Gly
            740                 745                 750

Glu Trp Lys His Gly Arg Ile Ile Leu Pro Ser Tyr Asp Met Glu Tyr
        755                 760                 765

Gln Ile Val Phe Glu Gly Val Ile Gly Lys Gly Arg Ser Gly Glu Ile
    770                 775                 780

Ala Ile Asp Asp Ile Arg Ile Ser Thr Asp Val Pro Leu Glu Asn Cys
785                 790                 795                 800

Met Glu Pro Ile Ser Ala Phe Ala Gly Glu Asn Phe Lys Gly Gly Thr
                805                 810                 815

Leu Leu Pro Gly Thr Glu Pro Thr Val Asp Thr Val Pro Met Gln Pro
            820                 825                 830

Ile Pro Ala Tyr Trp Tyr Tyr Val Met Ala Ala Gly Gly Ala Val Leu
        835                 840                 845

Val Leu Val Ser Val Ala Leu Ala Leu Val Leu His Tyr His Arg Phe
    850                 855                 860

Arg Tyr Ala Ala Lys Lys Thr Asp His Ser Ile Thr Tyr Lys Thr Ser
865                 870                 875                 880

His Tyr Thr Asn Gly Ala Pro Leu Ala Val Glu Pro Thr Leu Thr Ile
                885                 890                 895

Lys Leu Glu Gln Asp Arg Gly Ser His Cys
            900                 905

<210>  103
<211>  4148
<212>  DNA
<213>  human

<220>
<221>  CDS
<222>  (792)..(3497)

<400>  103
cagagatcgc gagcgaggca ccagcctgca gccggccccc agcacatcct cagccgcaca      60

gacactcggc gaggtggagg tgagggcggg cgccagcgaa ctcggagagg ggctcgctca     120

ctcccaggcg atcccagccg ccaccgccgc cgcaccagca gcagcaacag cagcagcagc     180

ttccttcctc agactcccct cgagaggctg gccaagcggg tgtagccgtt gggggaggct     240

cccgccgggg gaacccggcg aggacaagag cagggcggcc gccttccact cgggctgtcc     300

ggcggcggct gcctccgccc gtgtgtccgt caagggtgcc gcgggatgtg tgtcagttta     360

cgcctctgag atcacacagc tgcctggggg ccgtgtgatg cccaaggcaa gtcttggttt     420

taattattat tattatcatt attgttacgc ttggctttcg ggaaatactc gtgatatttg     480

taggataaag gaaatgacac tttgaggaac tggagagaac atatatgcgt ttgtttttta     540

agaggaaaac cgtgttctct tcccggcttg ttccctcttt gctgatttca ggagctactc     600

tcctcctggt gaggtggaaa ttccagcaag aatagaggtg aagacaagcc accaggactc     660

aggagggaaa cgctgaccat tagaaacctc tgcataagac gttgtaagga ggaaaataaa     720

agagagaaaa acacaaagat ttaaacaaga aacctacgaa cccagctctg gaaagagcca     780

ccttctccaa a atg gat atg ttt cct ctc acc tgg gtt ttc tta gcc ctc      830
             Met Asp Met Phe Pro Leu Thr Trp Val Phe Leu Ala Leu
             1               5                   10

tac ttt tca aga cac caa gtg aga ggc caa cca gac cca ccg tgc gga      878
Tyr Phe Ser Arg His Gln Val Arg Gly Gln Pro Asp Pro Pro Cys Gly
    15                  20                  25

ggt cgt ttg aat tcc aaa gat gct ggc tat atc acc tct ccc ggt tac      926
Gly Arg Leu Asn Ser Lys Asp Ala Gly Tyr Ile Thr Ser Pro Gly Tyr
30                  35                  40                  45

ccc cag gac tac ccc tcc cac cag aac tgc gag tgg att gtt tac gcc 974
Pro Gln Asp Tyr Pro Ser His Gln Asn Cys Glu Trp Ile Val Tyr Ala
                50                  55                  60

ccc gaa ccc aac cag aag att gtc ctc aac ttc aac cct cac ttt gaa 1022
Pro Glu Pro Asn Gln Lys Ile Val Leu Asn Phe Asn Pro His Phe Glu
            65                  70                  75

atc gag aag cac gac tgc aag tat gac ttt atc gag att cgg gat ggg 1070
Ile Glu Lys His Asp Cys Lys Tyr Asp Phe Ile Glu Ile Arg Asp Gly
        80                  85                  90

gac agt gaa tcc gca gac ctc ctg ggc aaa cac tgt ggg aac atc gcc 1118
Asp Ser Glu Ser Ala Asp Leu Leu Gly Lys His Cys Gly Asn Ile Ala
    95                  100                 105

ccg ccc acc atc atc tcc tcg ggc tcc atg ctc tac atc aag ttc acc 1166
Pro Pro Thr Ile Ile Ser Ser Gly Ser Met Leu Tyr Ile Lys Phe Thr
110                 115                 120                 125

tcc gac tac gcc cgg cag ggg gca ggc ttc tct ctg cgc tac gag atc 1214
Ser Asp Tyr Ala Arg Gln Gly Ala Gly Phe Ser Leu Arg Tyr Glu Ile
                130                 135                 140

ttc aag aca ggc tct gaa gat tgc tca aaa aac ttc aca agc ccc aac 1262
Phe Lys Thr Gly Ser Glu Asp Cys Ser Lys Asn Phe Thr Ser Pro Asn
            145                 150                 155

ggg acc atc gaa tct cct ggg ttt cct gag aag tat cca cac aac ttg 1310
Gly Thr Ile Glu Ser Pro Gly Phe Pro Glu Lys Tyr Pro His Asn Leu
        160                 165                 170

gac tgc acc ttt acc atc ctg gcc aaa ccc aag atg gag atc atc ctg 1358
Asp Cys Thr Phe Thr Ile Leu Ala Lys Pro Lys Met Glu Ile Ile Leu
    175                 180                 185

cag ttc ctg atc ttt gac ctg gag cat gac cct ttg cag gtg gga gag 1406
Gln Phe Leu Ile Phe Asp Leu Glu His Asp Pro Leu Gln Val Gly Glu
190                 195                 200                 205

ggg gac tgc aag tac gat tgg ctg gac atc tgg gat ggc att cca cat 1454
Gly Asp Cys Lys Tyr Asp Trp Leu Asp Ile Trp Asp Gly Ile Pro His
                210                 215                 220

gtt ggc ccc ctg att ggc aag tac tgt ggg acc aaa aca ccc tct gaa 1502
Val Gly Pro Leu Ile Gly Lys Tyr Cys Gly Thr Lys Thr Pro Ser Glu
            225                 230                 235

ctt cgt tca tcg acg ggg atc ctc tcc ctg acc ttt cac acg gac atg 1550
Leu Arg Ser Ser Thr Gly Ile Leu Ser Leu Thr Phe His Thr Asp Met
        240                 245                 250

gcg gtg gcc aag gat ggc ttc tct gcg cgt tac tac ctg gtc cac caa 1598
Ala Val Ala Lys Asp Gly Phe Ser Ala Arg Tyr Tyr Leu Val His Gln
    255                 260                 265

gag cca cta gag aac ttt cag tgc aat gtt cct ctg ggc atg gag tct 1646
Glu Pro Leu Glu Asn Phe Gln Cys Asn Val Pro Leu Gly Met Glu Ser
270                 275                 280                 285

ggc cgg att gct aat gaa cag atc agt gcc tca tct acc tac tct gat 1694
Gly Arg Ile Ala Asn Glu Gln Ile Ser Ala Ser Ser Thr Tyr Ser Asp
                290                 295                 300

ggg agg tgg acc cct caa caa agc cgg ctc cat ggt gat gac aat ggc 1742
Gly Arg Trp Thr Pro Gln Gln Ser Arg Leu His Gly Asp Asp Asn Gly
            305                 310                 315

tgg acc ccc aac ttg gat tcc aac aag gag tat ctc cag gtg gac ctg 1790
Trp Thr Pro Asn Leu Asp Ser Asn Lys Glu Tyr Leu Gln Val Asp Leu
        320                 325                 330

cgc ttt tta acc atg ctc acg gcc atc gca aca cag gga gcg att tcc 1838
Arg Phe Leu Thr Met Leu Thr Ala Ile Ala Thr Gln Gly Ala Ile Ser
    335                 340                 345

agg gaa aca cag aat ggc tac tat gtc aaa tcc tac aag ctg gaa gtc 1886
Arg Glu Thr Gln Asn Gly Tyr Tyr Val Lys Ser Tyr Lys Leu Glu Val
350                 355                 360                 365

agc act aat gga gag gac tgg atg gtg tac cgg cat ggc aaa aac cac 1934
Ser Thr Asn Gly Glu Asp Trp Met Val Tyr Arg His Gly Lys Asn His
                370                 375                 380

aag gta ttt caa gcc aac aac gat gca act gag gtg gtt ctg aac aag 1982
Lys Val Phe Gln Ala Asn Asn Asp Ala Thr Glu Val Val Leu Asn Lys
            385                 390                 395

ctc cac gct cca ctg ctg aca agg ttt gtt aga atc cgc cct cag acc 2030
Leu His Ala Pro Leu Leu Thr Arg Phe Val Arg Ile Arg Pro Gln Thr
        400                 405                 410

tgg cac tca ggt atc gcc ctc cgg ctg gag ctc ttc ggc tgc cgg gtc 2078
Trp His Ser Gly Ile Ala Leu Arg Leu Glu Leu Phe Gly Cys Arg Val
    415                 420                 425

aca gat gct ccc tgc tcc aac atg ctg ggg atg ctc tca ggc ctc att 2126
Thr Asp Ala Pro Cys Ser Asn Met Leu Gly Met Leu Ser Gly Leu Ile
430                 435                 440                 445

gca gac tcc cag atc tcc gcc tct tcc acc cag gaa tac ctc tgg agc 2174
Ala Asp Ser Gln Ile Ser Ala Ser Ser Thr Gln Glu Tyr Leu Trp Ser
                450                 455                 460

ccc agt gca gcc cgc ctg gtc agc agc cgc tcg ggc tgg ttc cct cga 2222
Pro Ser Ala Ala Arg Leu Val Ser Ser Arg Ser Gly Trp Phe Pro Arg
            465                 470                 475

atc cct cag gcc cag ccc ggt gag gag tgg ctt cag gta gat ctg gga 2270
Ile Pro Gln Ala Gln Pro Gly Glu Glu Trp Leu Gln Val Asp Leu Gly
        480                 485                 490

aca ccc aag aca gtg aaa ggt gtc atc atc cag gga gcc cgc gga gga 2318
Thr Pro Lys Thr Val Lys Gly Val Ile Ile Gln Gly Ala Arg Gly Gly
    495                 500                 505

gac agt atc act gct gtg gaa gcc aga gca ttt gtg cgc aag ttc aaa 2366
Asp Ser Ile Thr Ala Val Glu Ala Arg Ala Phe Val Arg Lys Phe Lys
510                 515                 520                 525

gtc tcc tac agc cta aac ggc aag gac tgg gaa tac att cag gac ccc 2414
Val Ser Tyr Ser Leu Asn Gly Lys Asp Trp Glu Tyr Ile Gln Asp Pro
                530                 535                 540

agg acc cag cag cca aag ctg ttc gaa ggg aac atg cac tat gac acc 2462
Arg Thr Gln Gln Pro Lys Leu Phe Glu Gly Asn Met His Tyr Asp Thr
            545                 550                 555

cct gac atc cga agg ttt gac ccc att ccg gca cag tat gtg cgg gta 2510
Pro Asp Ile Arg Arg Phe Asp Pro Ile Pro Ala Gln Tyr Val Arg Val
        560                 565                 570

tac ccg gag agg tgg tcg ccg gcg ggg att ggg atg cgg ctg gag gtg 2558
Tyr Pro Glu Arg Trp Ser Pro Ala Gly Ile Gly Met Arg Leu Glu Val
    575                 580                 585

ctg ggc tgt gac tgg aca gac tcc aag ccc acg gta gag acg ctg gga 2606
Leu Gly Cys Asp Trp Thr Asp Ser Lys Pro Thr Val Glu Thr Leu Gly
590                 595                 600                 605

ccc act gtg aag agc gaa gag aca acc acc ccc tac ccc acc gaa gag 2654
Pro Thr Val Lys Ser Glu Glu Thr Thr Thr Pro Tyr Pro Thr Glu Glu
                610                 615                 620

gag gcc aca gag tgt ggg gag aac tgc agc ttt gag gat gac aaa gat 2702
Glu Ala Thr Glu Cys Gly Glu Asn Cys Ser Phe Glu Asp Asp Lys Asp
            625                 630                 635

ttg cag ctc cct tcg gga ttc aat tgc aac ttc gat ttc ctc gag gag 2750
Leu Gln Leu Pro Ser Gly Phe Asn Cys Asn Phe Asp Phe Leu Glu Glu
        640                 645                 650

ccc tgt ggt tgg atg tat gac cat gcc aag tgg ctc cgg acc acc tgg 2798
Pro Cys Gly Trp Met Tyr Asp His Ala Lys Trp Leu Arg Thr Thr Trp
    655                 660                 665

gcc agc agc tcc agc cca aac gac cgg acg ttt cca gat gac agg aat 2846
Ala Ser Ser Ser Ser Pro Asn Asp Arg Thr Phe Pro Asp Asp Arg Asn
670                 675                 680                 685

ttc ttg cgg ctg cag agt gac agc cag aga gag ggc cag tat gcc cgg 2894
Phe Leu Arg Leu Gln Ser Asp Ser Gln Arg Glu Gly Gln Tyr Ala Arg
                690                 695                 700

ctc atc agc ccc cct gtc cac ctg ccc cga agc ccg gtg tgc atg gag 2942
Leu Ile Ser Pro Pro Val His Leu Pro Arg Ser Pro Val Cys Met Glu
            705                 710                 715

ttc cag tac cag gcc acg ggc ggc cgc ggg gtg gcg ctg cag gtg gtg 2990
Phe Gln Tyr Gln Ala Thr Gly Gly Arg Gly Val Ala Leu Gln Val Val
        720                 725                 730

cgg gaa gcc agc cag gag agc aag ttg ctg tgg gtc atc cgt gag gac 3038
Arg Glu Ala Ser Gln Glu Ser Lys Leu Leu Trp Val Ile Arg Glu Asp
    735                 740                 745

cag ggc ggc gag tgg aag cac ggg cgg atc atc ctg ccc agc tac gac 3086
Gln Gly Gly Glu Trp Lys His Gly Arg Ile Ile Leu Pro Ser Tyr Asp
750                 755                 760                 765

atg gag tac cag att gtg ttc gag gga gtg ata ggg aaa gga cgt tcc 3134
Met Glu Tyr Gln Ile Val Phe Glu Gly Val Ile Gly Lys Gly Arg Ser
                770                 775                 780

gga gag att gcc att gat gac att cgg ata agc act gat gtc cca ctg 3182
Gly Glu Ile Ala Ile Asp Asp Ile Arg Ile Ser Thr Asp Val Pro Leu
            785                 790                 795

gag aac tgc atg gaa ccc atc tcg gct ttt gca ggg ggc acc ctc ctg 3230
Glu Asn Cys Met Glu Pro Ile Ser Ala Phe Ala Gly Gly Thr Leu Leu
        800                 805                 810

cca ggg acc gag ccc aca gtg gac acg gtg ccc atg cag ccc atc cca 3278
Pro Gly Thr Glu Pro Thr Val Asp Thr Val Pro Met Gln Pro Ile Pro
    815                 820                 825

gcc tac tgg tat tac gta atg gcc gcc ggg ggc gcc gtg ctg gtg ctg 3326
Ala Tyr Trp Tyr Tyr Val Met Ala Ala Gly Gly Ala Val Leu Val Leu
830                 835                 840                 845

gtc tcc gtc gcg ctg gcc ctg gtg ctc cac tac cac cgg ttc cgc tat 3374
Val Ser Val Ala Leu Ala Leu Val Leu His Tyr His Arg Phe Arg Tyr
                850                 855                 860

gcg gcc aag aag acc gat cac tcc atc acc tac aaa acc tcc cac tac     3422
Ala Ala Lys Lys Thr Asp His Ser Ile Thr Tyr Lys Thr Ser His Tyr
            865                 870                 875

acc aac ggg gcc cct ctg gcg gtg gag ccc acc cta acc att aag cta     3470
Thr Asn Gly Ala Pro Leu Ala Val Glu Pro Thr Leu Thr Ile Lys Leu
        880                 885                 890

gag caa gac cgt ggc tcg cac tgc tga gggccgaagc aagaacagca           3517
Glu Gln Asp Arg Gly Ser His Cys
    895                 900

cccaaaacaa acgagaaaga ctgcaaacat gttgcctcga ttttgcactt ttttctcctc    3577

gcctagtttc tgtgtgaact ctcagacatc tctttcccgg atccccaacc ctgagcactc   3637

ttatcaatcc caaccatcct ccttgggttc attttggttt ctggtttttc tttttccttt   3697

ttgttgattc caaaccaaca aacccaactc taatgctgca tcttggacta tccgaagaga   3757

tccaccccca agcactccac aactcaaggc tcagctggtt ttgttccaga gactggttcg   3817

cttgtttttt ccccttgcct tatcccatac ctcctctcag tgggcagtct gccaggagac   3877

gtgaggggaa gcctggatct gtgtgtatgt acatagtaga catgtgtgtg tgtgaatagc   3937

tctctgtgtg tgggtgtgtg agagagcggc tggttcattg tgtgtgtgtt tgggcgaggg   3997

gtgagtgttc agagagggcc cctttaactc ttatgttact tctcctgggg tacattttac   4057

aagaaaataa tatactgtac aagttttgtt tacttggaga agagattgaa gctttttgtt   4117

gccttatcta aaaaaaaaaa aaaaaaaaaa a                                  4148

<210>  104
<211>  901
<212>  PRT
<213>  human

<400>  104

Met Asp Met Phe Pro Leu Thr Trp Val Phe Leu Ala Leu Tyr Phe Ser
1               5                   10                  15

Arg His Gln Val Arg Gly Gln Pro Asp Pro Pro Cys Gly Gly Arg Leu
            20                  25                  30

Asn Ser Lys Asp Ala Gly Tyr Ile Thr Ser Pro Gly Tyr Pro Gln Asp
        35                  40                  45

Tyr Pro Ser His Gln Asn Cys Glu Trp Ile Val Tyr Ala Pro Glu Pro
    50                  55                  60

Asn Gln Lys Ile Val Leu Asn Phe Asn Pro His Phe Glu Ile Glu Lys
65                  70                  75                  80

His Asp Cys Lys Tyr Asp Phe Ile Glu Ile Arg Asp Gly Asp Ser Glu
                85                  90                  95

Ser Ala Asp Leu Leu Gly Lys His Cys Gly Asn Ile Ala Pro Pro Thr

            100                 105                 110

Ile Ile Ser Ser Gly Ser Met Leu Tyr Ile Lys Phe Thr Ser Asp Tyr
        115                 120                 125

Ala Arg Gln Gly Ala Gly Phe Ser Leu Arg Tyr Glu Ile Phe Lys Thr
    130                 135                 140

Gly Ser Glu Asp Cys Ser Lys Asn Phe Thr Ser Pro Asn Gly Thr Ile
145                 150                 155                 160

Glu Ser Pro Gly Phe Pro Glu Lys Tyr Pro His Asn Leu Asp Cys Thr
                165                 170                 175

Phe Thr Ile Leu Ala Lys Pro Lys Met Glu Ile Ile Leu Gln Phe Leu
            180                 185                 190

Ile Phe Asp Leu Glu His Asp Pro Leu Gln Val Gly Glu Gly Asp Cys
        195                 200                 205

Lys Tyr Asp Trp Leu Asp Ile Trp Asp Gly Ile Pro His Val Gly Pro
    210                 215                 220

Leu Ile Gly Lys Tyr Cys Gly Thr Lys Thr Pro Ser Glu Leu Arg Ser
225                 230                 235                 240

Ser Thr Gly Ile Leu Ser Leu Thr Phe His Thr Asp Met Ala Val Ala
                245                 250                 255

Lys Asp Gly Phe Ser Ala Arg Tyr Tyr Leu Val His Gln Glu Pro Leu
            260                 265                 270

Glu Asn Phe Gln Cys Asn Val Pro Leu Gly Met Glu Ser Gly Arg Ile
        275                 280                 285

Ala Asn Glu Gln Ile Ser Ala Ser Ser Thr Tyr Ser Asp Gly Arg Trp
    290                 295                 300

Thr Pro Gln Gln Ser Arg Leu His Gly Asp Asp Asn Gly Trp Thr Pro
305                 310                 315                 320

Asn Leu Asp Ser Asn Lys Glu Tyr Leu Gln Val Asp Leu Arg Phe Leu
                325                 330                 335

Thr Met Leu Thr Ala Ile Ala Thr Gln Gly Ala Ile Ser Arg Glu Thr
            340                 345                 350

Gln Asn Gly Tyr Tyr Val Lys Ser Tyr Lys Leu Glu Val Ser Thr Asn
        355                 360                 365

Gly Glu Asp Trp Met Val Tyr Arg His Gly Lys Asn His Lys Val Phe

370                 375                 380

Gln Ala Asn Asn Asp Ala Thr Glu Val Val Leu Asn Lys Leu His Ala
385                 390                 395                 400

Pro Leu Leu Thr Arg Phe Val Arg Ile Arg Pro Gln Thr Trp His Ser
                405                 410                 415

Gly Ile Ala Leu Arg Leu Glu Leu Phe Gly Cys Arg Val Thr Asp Ala
            420                 425                 430

Pro Cys Ser Asn Met Leu Gly Met Leu Ser Gly Leu Ile Ala Asp Ser
        435                 440                 445

Gln Ile Ser Ala Ser Ser Thr Gln Glu Tyr Leu Trp Ser Pro Ser Ala
    450                 455                 460

Ala Arg Leu Val Ser Ser Arg Ser Gly Trp Phe Pro Arg Ile Pro Gln
465                 470                 475                 480

Ala Gln Pro Gly Glu Glu Trp Leu Gln Val Asp Leu Gly Thr Pro Lys
                485                 490                 495

Thr Val Lys Gly Val Ile Ile Gln Gly Ala Arg Gly Gly Asp Ser Ile
            500                 505                 510

Thr Ala Val Glu Ala Arg Ala Phe Val Arg Lys Phe Lys Val Ser Tyr
        515                 520                 525

Ser Leu Asn Gly Lys Asp Trp Glu Tyr Ile Gln Asp Pro Arg Thr Gln
    530                 535                 540

Gln Pro Lys Leu Phe Glu Gly Asn Met His Tyr Asp Thr Pro Asp Ile
545                 550                 555                 560

Arg Arg Phe Asp Pro Ile Pro Ala Gln Tyr Val Arg Val Tyr Pro Glu
                565                 570                 575

Arg Trp Ser Pro Ala Gly Ile Gly Met Arg Leu Glu Val Leu Gly Cys
            580                 585                 590

Asp Trp Thr Asp Ser Lys Pro Thr Val Glu Thr Leu Gly Pro Thr Val
        595                 600                 605

Lys Ser Glu Glu Thr Thr Thr Pro Tyr Pro Thr Glu Glu Glu Ala Thr
    610                 615                 620

Glu Cys Gly Glu Asn Cys Ser Phe Glu Asp Asp Lys Asp Leu Gln Leu
625                 630                 635                 640

Pro Ser Gly Phe Asn Cys Asn Phe Asp Phe Leu Glu Glu Pro Cys Gly

                645                 650                 655

Trp Met Tyr Asp His Ala Lys Trp Leu Arg Thr Thr Trp Ala Ser Ser
            660                 665                 670

Ser Ser Pro Asn Asp Arg Thr Phe Pro Asp Asp Arg Asn Phe Leu Arg
        675                 680                 685

Leu Gln Ser Asp Ser Gln Arg Glu Gly Gln Tyr Ala Arg Leu Ile Ser
    690                 695                 700

Pro Pro Val His Leu Pro Arg Ser Pro Val Cys Met Glu Phe Gln Tyr
705                 710                 715                 720

Gln Ala Thr Gly Gly Arg Gly Val Ala Leu Gln Val Val Arg Glu Ala
                725                 730                 735

Ser Gln Glu Ser Lys Leu Leu Trp Val Ile Arg Glu Asp Gln Gly Gly
            740                 745                 750

Glu Trp Lys His Gly Arg Ile Ile Leu Pro Ser Tyr Asp Met Glu Tyr
        755                 760                 765

Gln Ile Val Phe Glu Gly Val Ile Gly Lys Gly Arg Ser Gly Glu Ile
    770                 775                 780

Ala Ile Asp Asp Ile Arg Ile Ser Thr Asp Val Pro Leu Glu Asn Cys
785                 790                 795                 800

Met Glu Pro Ile Ser Ala Phe Ala Gly Gly Thr Leu Leu Pro Gly Thr
                805                 810                 815

Glu Pro Thr Val Asp Thr Val Pro Met Gln Pro Ile Pro Ala Tyr Trp
            820                 825                 830

Tyr Tyr Val Met Ala Ala Gly Gly Ala Val Leu Val Leu Val Ser Val
        835                 840                 845

Ala Leu Ala Leu Val Leu His Tyr His Arg Phe Arg Tyr Ala Ala Lys
    850                 855                 860

Lys Thr Asp His Ser Ile Thr Tyr Lys Thr Ser His Tyr Thr Asn Gly
865                 870                 875                 880

Ala Pro Leu Ala Val Glu Pro Thr Leu Thr Ile Lys Leu Glu Gln Asp
                885                 890                 895

Arg Gly Ser His Cys
            900

<210> 105

<211>  2601
<212>  DNA
<213>  human

<220>
<221>  CDS
<222>  (792)..(2459)

<400>  105
cagagatcgc gagcgaggca ccagcctgca gccggccccc agcacatcct cagccgcaca      60

gacactcggc gaggtggagg tgagggcggg cgccagcgaa ctcggagagg ggctcgctca     120

ctcccaggcg atcccagccg ccaccgccgc cgcaccagca gcagcaacag cagcagcagc     180

ttccttcctc agactcccct cgagaggctg gccaagcggg tgtagccgtt gggggaggct     240

cccgccgggg gaacccggcg aggacaagag cagggcggcc gccttccact cgggctgtcc     300

ggcggcggct gcctccgccc gtgtgtccgt caagggtgcc gcgggatgtg tgtcagttta     360

cgcctctgag atcacacagc tgcctggggg ccgtgtgatg cccaaggcaa gtcttggttt     420

taattattat tattatcatt attgttacgc ttggctttcg ggaaatactc gtgatatttg     480

taggataaag gaaatgacac tttgaggaac tggagagaac atatatgcgt tttgttttta     540

agaggaaaac cgtgttctct tcccggcttg ttccctcttt gctgatttca ggagctactc     600

tcctcctggt gaggtggaaa ttccagcaag aatagaggtg aagacaagcc accaggactc     660

aggagggaaa cgctgaccat tagaaacctc tgcataagac gttgtaagga ggaaaataaa     720

agagagaaaa acacaaagat ttaaacaaga aacctacgaa cccagctctg gaaagagcca     780

ccttctccaa a atg gat atg ttt cct ctc acc tgg gtt ttc tta gcc ctc      830
             Met Asp Met Phe Pro Leu Thr Trp Val Phe Leu Ala Leu
             1               5                   10

tac ttt tca aga cac caa gtg aga ggc caa cca gac cca ccg tgc gga      878
Tyr Phe Ser Arg His Gln Val Arg Gly Gln Pro Asp Pro Pro Cys Gly
    15                  20                  25

ggt cgt ttg aat tcc aaa gat gct ggc tat atc acc tct ccc ggt tac      926
Gly Arg Leu Asn Ser Lys Asp Ala Gly Tyr Ile Thr Ser Pro Gly Tyr
30                  35                  40                  45

ccc cag gac tac ccc tcc cac cag aac tgc gag tgg att gtt tac gcc      974
Pro Gln Asp Tyr Pro Ser His Gln Asn Cys Glu Trp Ile Val Tyr Ala
                50                  55                  60

ccc gaa ccc aac cag aag att gtc ctc aac ttc aac cct cac ttt gaa     1022
Pro Glu Pro Asn Gln Lys Ile Val Leu Asn Phe Asn Pro His Phe Glu
            65                  70                  75

atc gag aag cac gac tgc aag tat gac ttt atc gag att cgg gat ggg     1070
Ile Glu Lys His Asp Cys Lys Tyr Asp Phe Ile Glu Ile Arg Asp Gly
        80                  85                  90

gac agt gaa tcc gca gac ctc ctg ggc aaa cac tgt ggg aac atc gcc     1118
Asp Ser Glu Ser Ala Asp Leu Leu Gly Lys His Cys Gly Asn Ile Ala
    95                  100                 105

ccg ccc acc atc atc tcc tcg ggc tcc atg ctc tac atc aag ttc acc     1166
Pro Pro Thr Ile Ile Ser Ser Gly Ser Met Leu Tyr Ile Lys Phe Thr
110                 115                 120                 125

tcc gac tac gcc cgg cag ggg gca ggc ttc tct ctg cgc tac gag atc 1214
Ser Asp Tyr Ala Arg Gln Gly Ala Gly Phe Ser Leu Arg Tyr Glu Ile
                130                 135                 140

ttc aag aca ggc tct gaa gat tgc tca aaa aac ttc aca agc ccc aac 1262
Phe Lys Thr Gly Ser Glu Asp Cys Ser Lys Asn Phe Thr Ser Pro Asn
            145                 150                 155

ggg acc atc gaa tct cct ggg ttt cct gag aag tat cca cac aac ttg 1310
Gly Thr Ile Glu Ser Pro Gly Phe Pro Glu Lys Tyr Pro His Asn Leu
        160                 165                 170

gac tgc acc ttt acc atc ctg gcc aaa ccc aag atg gag atc atc ctg 1358
Asp Cys Thr Phe Thr Ile Leu Ala Lys Pro Lys Met Glu Ile Ile Leu
    175                 180                 185

cag ttc ctg atc ttt gac ctg gag cat gac cct ttg cag gtg gga gag 1406
Gln Phe Leu Ile Phe Asp Leu Glu His Asp Pro Leu Gln Val Gly Glu
190                 195                 200                 205

ggg gac tgc aag tac gat tgg ctg gac atc tgg gat ggc att cca cat 1454
Gly Asp Cys Lys Tyr Asp Trp Leu Asp Ile Trp Asp Gly Ile Pro His
                210                 215                 220

gtt ggc ccc ctg att ggc aag tac tgt ggg acc aaa aca ccc tct gaa 1502
Val Gly Pro Leu Ile Gly Lys Tyr Cys Gly Thr Lys Thr Pro Ser Glu
            225                 230                 235

ctt cgt tca tcg acg ggg atc ctc tcc ctg acc ttt cac acg gac atg 1550
Leu Arg Ser Ser Thr Gly Ile Leu Ser Leu Thr Phe His Thr Asp Met
        240                 245                 250

gcg gtg gcc aag gat ggc ttc tct gcg cgt tac tac ctg gtc cac caa 1598
Ala Val Ala Lys Asp Gly Phe Ser Ala Arg Tyr Tyr Leu Val His Gln
    255                 260                 265

gag cca cta gag aac ttt cag tgc aat gtt cct ctg ggc atg gag tct 1646
Glu Pro Leu Glu Asn Phe Gln Cys Asn Val Pro Leu Gly Met Glu Ser
270                 275                 280                 285

ggc cgg att gct aat gaa cag atc agt gcc tca tct acc tac tct gat 1694
Gly Arg Ile Ala Asn Glu Gln Ile Ser Ala Ser Ser Thr Tyr Ser Asp
                290                 295                 300

ggg agg tgg acc cct caa caa agc cgg ctc cat ggt gat gac aat ggc 1742
Gly Arg Trp Thr Pro Gln Gln Ser Arg Leu His Gly Asp Asp Asn Gly
            305                 310                 315

tgg acc ccc aac ttg gat tcc aac aag gag tat ctc cag gtg gac ctg 1790
Trp Thr Pro Asn Leu Asp Ser Asn Lys Glu Tyr Leu Gln Val Asp Leu
        320                 325                 330

cgc ttt tta acc atg ctc acg gcc atc gca aca cag gga gcg att tcc 1838
Arg Phe Leu Thr Met Leu Thr Ala Ile Ala Thr Gln Gly Ala Ile Ser
    335                 340                 345

agg gaa aca cag aat ggc tac tat gtc aaa tcc tac aag ctg gaa gtc 1886
Arg Glu Thr Gln Asn Gly Tyr Tyr Val Lys Ser Tyr Lys Leu Glu Val
350                 355                 360                 365

agc act aat gga gag gac tgg atg gtg tac cgg cat ggc aaa aac cac 1934
Ser Thr Asn Gly Glu Asp Trp Met Val Tyr Arg His Gly Lys Asn His
                370                 375                 380

aag gta ttt caa gcc aac aac gat gca act gag gtg gtt ctg aac aag 1982
Lys Val Phe Gln Ala Asn Asn Asp Ala Thr Glu Val Val Leu Asn Lys
            385                 390                 395

ctc cac gct cca ctg ctg aca agg ttt gtt aga atc cgc cct cag acc     2030
Leu His Ala Pro Leu Leu Thr Arg Phe Val Arg Ile Arg Pro Gln Thr
        400                 405                 410

tgg cac tca ggt atc gcc ctc cgg ctg gag ctc ttc ggc tgc cgg gtc     2078
Trp His Ser Gly Ile Ala Leu Arg Leu Glu Leu Phe Gly Cys Arg Val
    415                 420                 425

aca gat gct ccc tgc tcc aac atg ctg ggg atg ctc tca ggc ctc att     2126
Thr Asp Ala Pro Cys Ser Asn Met Leu Gly Met Leu Ser Gly Leu Ile
430                 435                 440                 445

gca gac tcc cag atc tcc gcc tct tcc acc cag gaa tac ctc tgg agc     2174
Ala Asp Ser Gln Ile Ser Ala Ser Ser Thr Gln Glu Tyr Leu Trp Ser
                450                 455                 460

ccc agt gca gcc cgc ctg gtc agc agc cgc tcg ggc tgg ttc cct cga     2222
Pro Ser Ala Ala Arg Leu Val Ser Ser Arg Ser Gly Trp Phe Pro Arg
            465                 470                 475

atc cct cag gcc cag ccc ggt gag gag tgg ctt cag gta gat ctg gga     2270
Ile Pro Gln Ala Gln Pro Gly Glu Glu Trp Leu Gln Val Asp Leu Gly
        480                 485                 490

aca ccc aag aca gtg aaa ggt gtc atc atc cag gga gcc cgc gga gga     2318
Thr Pro Lys Thr Val Lys Gly Val Ile Ile Gln Gly Ala Arg Gly Gly
    495                 500                 505

gac agt atc act gct gtg gaa gcc aga gca ttt gtg cgc aag ttc aaa     2366
Asp Ser Ile Thr Ala Val Glu Ala Arg Ala Phe Val Arg Lys Phe Lys
510                 515                 520                 525

gtc tcc tac agc cta aac ggc aag gac tgg gaa tac att cag gac ccc     2414
Val Ser Tyr Ser Leu Asn Gly Lys Asp Trp Glu Tyr Ile Gln Asp Pro
                530                 535                 540

agg acc cag cag cca aag gta ggc tgt tct tgg agg cct ctg taa         2459
Arg Thr Gln Gln Pro Lys Val Gly Cys Ser Trp Arg Pro Leu
            545                 550                 555

cgttaccctc aacagggagg ctaagtgtgg tacagggagt tgagactgat gatgtcccat   2519

ctaaacagtc gtcatccaac tcctgaaatc caataaaaca aatatcgttt gagagattaa   2579

aaaaaaaaaa aaaaaaaaaa aa                                            2601

<210>  106
<211>  555
<212>  PRT
<213>  human

<400>  106

Met Asp Met Phe Pro Leu Thr Trp Val Phe Leu Ala Leu Tyr Phe Ser
1               5                   10                  15

Arg His Gln Val Arg Gly Gln Pro Asp Pro Pro Cys Gly Gly Arg Leu
            20                  25                  30

Asn Ser Lys Asp Ala Gly Tyr Ile Thr Ser Pro Gly Tyr Pro Gln Asp
        35                  40                  45

Tyr Pro Ser His Gln Asn Cys Glu Trp Ile Val Tyr Ala Pro Glu Pro
    50                  55                  60

Asn Gln Lys Ile Val Leu Asn Phe Asn Pro His Phe Glu Ile Glu Lys
65                  70                  75                  80

His Asp Cys Lys Tyr Asp Phe Ile Glu Ile Arg Asp Gly Asp Ser Glu
                85                  90                  95

Ser Ala Asp Leu Leu Gly Lys His Cys Gly Asn Ile Ala Pro Pro Thr
            100                 105                 110

Ile Ile Ser Ser Gly Ser Met Leu Tyr Ile Lys Phe Thr Ser Asp Tyr
        115                 120                 125

Ala Arg Gln Gly Ala Gly Phe Ser Leu Arg Tyr Glu Ile Phe Lys Thr
    130                 135                 140

Gly Ser Glu Asp Cys Ser Lys Asn Phe Thr Ser Pro Asn Gly Thr Ile
145                 150                 155                 160

Glu Ser Pro Gly Phe Pro Glu Lys Tyr Pro His Asn Leu Asp Cys Thr
                165                 170                 175

Phe Thr Ile Leu Ala Lys Pro Lys Met Glu Ile Ile Leu Gln Phe Leu
            180                 185                 190

Ile Phe Asp Leu Glu His Asp Pro Leu Gln Val Gly Glu Gly Asp Cys
        195                 200                 205

Lys Tyr Asp Trp Leu Asp Ile Trp Asp Gly Ile Pro His Val Gly Pro
    210                 215                 220

Leu Ile Gly Lys Tyr Cys Gly Thr Lys Thr Pro Ser Glu Leu Arg Ser
225                 230                 235                 240

Ser Thr Gly Ile Leu Ser Leu Thr Phe His Thr Asp Met Ala Val Ala
                245                 250                 255

Lys Asp Gly Phe Ser Ala Arg Tyr Tyr Leu Val His Gln Glu Pro Leu
            260                 265                 270

Glu Asn Phe Gln Cys Asn Val Pro Leu Gly Met Glu Ser Gly Arg Ile
        275                 280                 285

Ala Asn Glu Gln Ile Ser Ala Ser Ser Thr Tyr Ser Asp Gly Arg Trp
    290                 295                 300

Thr Pro Gln Gln Ser Arg Leu His Gly Asp Asp Asn Gly Trp Thr Pro
305                 310                 315                 320

Asn Leu Asp Ser Asn Lys Glu Tyr Leu Gln Val Asp Leu Arg Phe Leu
                325                 330                 335

Thr Met Leu Thr Ala Ile Ala Thr Gln Gly Ala Ile Ser Arg Glu Thr
            340                 345                 350

Gln Asn Gly Tyr Tyr Val Lys Ser Tyr Lys Leu Glu Val Ser Thr Asn
        355                 360                 365

Gly Glu Asp Trp Met Val Tyr Arg His Gly Lys Asn His Lys Val Phe
    370                 375                 380

Gln Ala Asn Asn Asp Ala Thr Glu Val Val Leu Asn Lys Leu His Ala
385                 390                 395                 400

Pro Leu Leu Thr Arg Phe Val Arg Ile Arg Pro Gln Thr Trp His Ser
                405                 410                 415

Gly Ile Ala Leu Arg Leu Glu Leu Phe Gly Cys Arg Val Thr Asp Ala
            420                 425                 430

Pro Cys Ser Asn Met Leu Gly Met Leu Ser Gly Leu Ile Ala Asp Ser
        435                 440                 445

Gln Ile Ser Ala Ser Ser Thr Gln Glu Tyr Leu Trp Ser Pro Ser Ala
    450                 455                 460

Ala Arg Leu Val Ser Ser Arg Ser Gly Trp Phe Pro Arg Ile Pro Gln
465                 470                 475                 480

Ala Gln Pro Gly Glu Glu Trp Leu Gln Val Asp Leu Gly Thr Pro Lys
                485                 490                 495

Thr Val Lys Gly Val Ile Ile Gln Gly Ala Arg Gly Gly Asp Ser Ile
            500                 505                 510

Thr Ala Val Glu Ala Arg Ala Phe Val Arg Lys Phe Lys Val Ser Tyr
        515                 520                 525

Ser Leu Asn Gly Lys Asp Trp Glu Tyr Ile Gln Asp Pro Arg Thr Gln
    530                 535                 540

Gln Pro Lys Val Gly Cys Ser Trp Arg Pro Leu
545                 550                 555

<210>  107
<211>  933
<212>  DNA
<213>  human

<220>
<221>  CDS
<222>  (1)..(933)

<400> 107
atg gaa cca cag aac acc aca cag gta tca atg ttt gtc ctc tta ggg 48
Met Glu Pro Gln Asn Thr Thr Gln Val Ser Met Phe Val Leu Leu Gly
1               5                   10                  15

ttt tca cag acc caa gag ctc cag aaa ttc ctg ttc ctt ctg ttc ctg 96
Phe Ser Gln Thr Gln Glu Leu Gln Lys Phe Leu Phe Leu Leu Phe Leu
            20                  25                  30

tta gtc tat gtt acc acc att gtg gga aac ctc ctt atc atg gtc aca 144
Leu Val Tyr Val Thr Thr Ile Val Gly Asn Leu Leu Ile Met Val Thr
        35                  40                  45

gtg act ttt gac tgc cgg ctc cac aca ccc atg tat ttt ctg ctc cga 192
Val Thr Phe Asp Cys Arg Leu His Thr Pro Met Tyr Phe Leu Leu Arg
    50                  55                  60

aat cta gct ctc ata gac ctc tgc tat tcc aca gtc acc tct cca aag 240
Asn Leu Ala Leu Ile Asp Leu Cys Tyr Ser Thr Val Thr Ser Pro Lys
65                  70                  75                  80

atg ctg gtg gac ttc ctc cat gag acc aag acg atc tcc tac cag ggc 288
Met Leu Val Asp Phe Leu His Glu Thr Lys Thr Ile Ser Tyr Gln Gly
                85                  90                  95

tgc atg gcc cag atc ttc ttc ttc cac ctt ttg gga ggt ggg act gtc 336
Cys Met Ala Gln Ile Phe Phe Phe His Leu Leu Gly Gly Gly Thr Val
            100                 105                 110

ttt ttt ctc tca gtc atg gcc tat gac cgc tac ata gcc atc tcc cag 384
Phe Phe Leu Ser Val Met Ala Tyr Asp Arg Tyr Ile Ala Ile Ser Gln
        115                 120                 125

ccc ctc cgg tat gtc acc atc atg aac act caa ttg tgt gtg ggc ctg 432
Pro Leu Arg Tyr Val Thr Ile Met Asn Thr Gln Leu Cys Val Gly Leu
    130                 135                 140

gta gta gcc gcc tgg gtg ggg ggc ttt gtc cac tcc att gtc caa ctg 480
Val Val Ala Ala Trp Val Gly Gly Phe Val His Ser Ile Val Gln Leu
145                 150                 155                 160

gct ctg ata ctt cca ctg ccc ttc tgt ggc ccc aat atc cta gat aac 528
Ala Leu Ile Leu Pro Leu Pro Phe Cys Gly Pro Asn Ile Leu Asp Asn
                165                 170                 175

ttc tac tgt gat gtt ccc caa gta ctg aga ctt gcc tgc act gat acc 576
Phe Tyr Cys Asp Val Pro Gln Val Leu Arg Leu Ala Cys Thr Asp Thr
            180                 185                 190

tcc ctc ctg gag ttc ctc atg atc tcc aac agt ggg ctg cta gtt atc 624
Ser Leu Leu Glu Phe Leu Met Ile Ser Asn Ser Gly Leu Leu Val Ile
        195                 200                 205

atc tgg ttc ctc ctc ctt ctg atc tct tat act gtc atc ctg gtg atg 672
Ile Trp Phe Leu Leu Leu Leu Ile Ser Tyr Thr Val Ile Leu Val Met
    210                 215                 220

ctg agg tcc cac tcg gga aag gca agg agg aag gca gct tcc acc tgc 720
Leu Arg Ser His Ser Gly Lys Ala Arg Arg Lys Ala Ala Ser Thr Cys
225                 230                 235                 240

acc acc cac atc atc gtg gtg tcc atg atc ttc att ccc tgt atc tat 768
Thr Thr His Ile Ile Val Val Ser Met Ile Phe Ile Pro Cys Ile Tyr
                245                 250                 255

atc tat acc tgg ccc ttc acc cca ttc ctc atg gac aag gct gtg tcc 816
Ile Tyr Thr Trp Pro Phe Thr Pro Phe Leu Met Asp Lys Ala Val Ser
            260                 265                 270

atc agc tac aca gtc atg acc ccc atg ctc aac ccc atg atc tac acc 864
Ile Ser Tyr Thr Val Met Thr Pro Met Leu Asn Pro Met Ile Tyr Thr
        275                 280                 285

ctg aga aac cag gac atg aaa gca gcc atg agg aga tta ggc aag tgc 912
Leu Arg Asn Gln Asp Met Lys Ala Ala Met Arg Arg Leu Gly Lys Cys
    290                 295                 300

cta gta att tgc agg gag taa 933
Leu Val Ile Cys Arg Glu
305                 310

<210> 108
<211> 310
<212> PRT
<213> human

<400> 108

Met Glu Pro Gln Asn Thr Thr Gln Val Ser Met Phe Val Leu Leu Gly
1               5                   10                  15

Phe Ser Gln Thr Gln Glu Leu Gln Lys Phe Leu Phe Leu Leu Phe Leu
            20                  25                  30

Leu Val Tyr Val Thr Thr Ile Val Gly Asn Leu Leu Ile Met Val Thr
        35                  40                  45

Val Thr Phe Asp Cys Arg Leu His Thr Pro Met Tyr Phe Leu Leu Arg
    50                  55                  60

Asn Leu Ala Leu Ile Asp Leu Cys Tyr Ser Thr Val Thr Ser Pro Lys
65                  70                  75                  80

Met Leu Val Asp Phe Leu His Glu Thr Lys Thr Ile Ser Tyr Gln Gly
                85                  90                  95

Cys Met Ala Gln Ile Phe Phe Phe His Leu Leu Gly Gly Gly Thr Val
            100                 105                 110

Phe Phe Leu Ser Val Met Ala Tyr Asp Arg Tyr Ile Ala Ile Ser Gln
        115                 120                 125

Pro Leu Arg Tyr Val Thr Ile Met Asn Thr Gln Leu Cys Val Gly Leu
    130                 135                 140

Val Val Ala Ala Trp Val Gly Gly Phe Val His Ser Ile Val Gln Leu
145                 150                 155                 160

Ala Leu Ile Leu Pro Leu Pro Phe Cys Gly Pro Asn Ile Leu Asp Asn
                165                 170                 175

Phe Tyr Cys Asp Val Pro Gln Val Leu Arg Leu Ala Cys Thr Asp Thr
            180                 185                 190

Ser Leu Leu Glu Phe Leu Met Ile Ser Asn Ser Gly Leu Leu Val Ile
        195                 200                 205

Ile Trp Phe Leu Leu Leu Leu Ile Ser Tyr Thr Val Ile Leu Val Met
    210                 215                 220

Leu Arg Ser His Ser Gly Lys Ala Arg Arg Lys Ala Ala Ser Thr Cys
225                 230                 235                 240

Thr Thr His Ile Ile Val Val Ser Met Ile Phe Ile Pro Cys Ile Tyr
                245                 250                 255

Ile Tyr Thr Trp Pro Phe Thr Pro Phe Leu Met Asp Lys Ala Val Ser
            260                 265                 270

Ile Ser Tyr Thr Val Met Thr Pro Met Leu Asn Pro Met Ile Tyr Thr
        275                 280                 285

Leu Arg Asn Gln Asp Met Lys Ala Ala Met Arg Arg Leu Gly Lys Cys
    290                 295                 300

Leu Val Ile Cys Arg Glu
305                 310

<210>  109
<211>  10759
<212>  DNA
<213>  human

<220>
<221>  CDS
<222>  (712)..(2010)

<400>  109
attctccgcg gcgcggcggc gagtgctggc tgcggtaccc tcccgctcgc ttgtccgtct      60

gcgcgcccgc gatgtgacca gccgactggg ggcgggctgg cggctctgcc tgcggcagcg     120

catcggtttt tctcctggca ggtccctaat tgagccgtga ttcccttgga gccagagaca     180

cccaccaggg gctcggagcg ccgcgctccg gggctggagg cagctgcagc gctcgggtcc     240

gcgcggccgg gtgcagtagc ctcagtccca gccgccgcct cgctgagtcg tgtgccctgg     300

cgaatgcccg gcgcccggca cagctgagcc aaggcgactg gtgcagagcg gcggcgcctg     360

gtcctgcact tgccgctgct gccgggctgt gccggggcgg cggcgctgcg cgccggagtt     420

tgcagaggct gcctccgagc gccgtccgcc aggaagactc cctgcctcct gagtcccaaa     480

aacacgagat ttttctccta tttcagcagt tggccacaac ttcattgctg ggaaaaggcc     540

aagaaagaag agagaactct caccacaaca tttcaaaggg aatacattgc cagaacttgg     600

aatactctac tggacaaaca tttcctccaa ggacacagct ctctgcctcc atgtcaccac     660

ctttgaagga ctgactgatt ccctcggctg gtgccagtgc ctgctcctgc c atg ggg      717
                                                         Met Gly

                                                         1

ccc gcg ggg agc ctg ctg ggc agc gga cag atg cag atc acc ctg tgg 765
Pro Ala Gly Ser Leu Leu Gly Ser Gly Gln Met Gln Ile Thr Leu Trp
        5                   10                  15

gga agt ctg gct gct gtc gcc att ttc ttc gtc atc acc ttc ctc atc 813
Gly Ser Leu Ala Ala Val Ala Ile Phe Phe Val Ile Thr Phe Leu Ile
    20                  25                  30

ttc ctg tgc tct agt tgt gac agg gaa aag aag ccg cga cag cat agt 861
Phe Leu Cys Ser Ser Cys Asp Arg Glu Lys Lys Pro Arg Gln His Ser
35                  40                  45                  50

ggg gac cat gag aac ctg atg aac gtg cct tca gac aag gag atg ttc 909
Gly Asp His Glu Asn Leu Met Asn Val Pro Ser Asp Lys Glu Met Phe
                55                  60                  65

agc cgt tca gtt act agc ctg gca aca gat gct cct gcc agc agt gag 957
Ser Arg Ser Val Thr Ser Leu Ala Thr Asp Ala Pro Ala Ser Ser Glu
            70                  75                  80

cag aat ggg gca ctc acc aat ggg gac att ctt tca gag gac agt act 1005
Gln Asn Gly Ala Leu Thr Asn Gly Asp Ile Leu Ser Glu Asp Ser Thr
        85                  90                  95

ctg acc tgc atg cag cat tac gag gaa gtc cag aca tcg gcc tcg gat 1053
Leu Thr Cys Met Gln His Tyr Glu Glu Val Gln Thr Ser Ala Ser Asp
    100                 105                 110

ctg ctg gat tcc cag gac agc aca ggg aaa cca aaa tgt cat cag agt 1101
Leu Leu Asp Ser Gln Asp Ser Thr Gly Lys Pro Lys Cys His Gln Ser
115                 120                 125                 130

cgg gag ctg ccc aga atc cct ccc gag agc gca gtg gat acc atg ctc 1149
Arg Glu Leu Pro Arg Ile Pro Pro Glu Ser Ala Val Asp Thr Met Leu
                135                 140                 145

acg gcg aga agt gtg gac ggg gac cag ggg ctg ggg atg gaa ggg ccc 1197
Thr Ala Arg Ser Val Asp Gly Asp Gln Gly Leu Gly Met Glu Gly Pro
            150                 155                 160

tat gaa gtg ctc aag gac agc tcc tcc caa gaa aac atg gtg gag gac 1245
Tyr Glu Val Leu Lys Asp Ser Ser Ser Gln Glu Asn Met Val Glu Asp
        165                 170                 175

tgc ttg tat gaa act gtg aaa gag atc aag gag gtg gct gca gct gca 1293
Cys Leu Tyr Glu Thr Val Lys Glu Ile Lys Glu Val Ala Ala Ala Ala
    180                 185                 190

cac ctg gag aaa ggc cac agt ggc aag gca aaa tct act tct gcc tcg 1341
His Leu Glu Lys Gly His Ser Gly Lys Ala Lys Ser Thr Ser Ala Ser
195                 200                 205                 210

aaa gag ctc cca ggg ccc cag act gaa ggc aaa gct gag ttt gct gaa 1389
Lys Glu Leu Pro Gly Pro Gln Thr Glu Gly Lys Ala Glu Phe Ala Glu
                215                 220                 225

tat gcc tcg gtg gac aga aac aaa aaa tgt cgt caa agt gtt aat gta 1437
Tyr Ala Ser Val Asp Arg Asn Lys Lys Cys Arg Gln Ser Val Asn Val
            230                 235                 240

gag agt atc ctt gga aat tca tgt gat cca gaa gag gag gcc cca cca 1485
Glu Ser Ile Leu Gly Asn Ser Cys Asp Pro Glu Glu Glu Ala Pro Pro
        245                 250                 255

cct gtc cct gtt aag ctt ctg gac gag aat gaa aac ctt cag gag aag 1533
Pro Val Pro Val Lys Leu Leu Asp Glu Asn Glu Asn Leu Gln Glu Lys

260                 265                 270

gaa ggg gga gag gcg gaa gag agt gcc aca gac acg acc agt gaa act     1581
Glu Gly Gly Glu Ala Glu Glu Ser Ala Thr Asp Thr Thr Ser Glu Thr
275                 280                 285                 290

aac aag aga ttt agc tca ttg tca tac aag tct cgg gaa gaa gac ccc     1629
Asn Lys Arg Phe Ser Ser Leu Ser Tyr Lys Ser Arg Glu Glu Asp Pro
                295                 300                 305

act ctc aca gaa gaa gag atc tca gct atg tac tca tca gta aat aaa     1677
Thr Leu Thr Glu Glu Glu Ile Ser Ala Met Tyr Ser Ser Val Asn Lys
            310                 315                 320

cct gga cag tta gtg aat aaa tcg ggg cag tcg ctt aca gtt ccg gag     1725
Pro Gly Gln Leu Val Asn Lys Ser Gly Gln Ser Leu Thr Val Pro Glu
        325                 330                 335

tcc acc tac acc tcc att caa ggg gac cca cag agg tca ccc tcc tcc     1773
Ser Thr Tyr Thr Ser Ile Gln Gly Asp Pro Gln Arg Ser Pro Ser Ser
    340                 345                 350

tgt aat gat ctc tat gct act gtt aaa gac ttc gaa aaa act cca aac     1821
Cys Asn Asp Leu Tyr Ala Thr Val Lys Asp Phe Glu Lys Thr Pro Asn
355                 360                 365                 370

agc aca ctt cca cca gca ggg agg ccc agc gag gag cca gag cct gat     1869
Ser Thr Leu Pro Pro Ala Gly Arg Pro Ser Glu Glu Pro Glu Pro Asp
                375                 380                 385

tat gaa gcg ata cag act ctc aac aga gag gaa gaa aag gcc acc ctg     1917
Tyr Glu Ala Ile Gln Thr Leu Asn Arg Glu Glu Glu Lys Ala Thr Leu
            390                 395                 400

ggg acc aat ggc cac cac ggt ctc gtc cca aag gag aac gac tac gag     1965
Gly Thr Asn Gly His His Gly Leu Val Pro Lys Glu Asn Asp Tyr Glu
        405                 410                 415

agc ata agt gac ttg cag caa ggc aga gat att acc agg ctc tag         2010
Ser Ile Ser Asp Leu Gln Gln Gly Arg Asp Ile Thr Arg Leu
    420                 425                 430

caacccagaa gacaaccctg ggtagcctgt gatcagtgtc tggagacgtt tcttctgtgg     2070

aagagaagaa gtgacacaaa cctatacttc atatgctgct ttagtcacct gaagatggtt     2130

ggagaggccc tgtcgactgt tctcccagtt gttcagtttc tgagacagag aggtacggac     2190

taggctgcac ctgagtgtgc ccctgcctgc cagatggaca ggtacaccca agcacatctc     2250

cctgctgcac cctcaccacc cacaaaagat cccagctgtc agtggtctca tctcattagt     2310

gaggaaagcc aagctgtatg gaaaagctgc actcaccaag gaccacaatg cccccggcct     2370

aaagtactgc cattcagaaa agcaggtttt tcttcctctc tttccttttc tctgtctgct     2430

actgacttta aggctttttc ccccttgaaa tgtccagatt cctgtggttc atcccaagga     2490

aattttcaca caaagcttgg cctttgccct caatataggt gttttaggat ggtgacaaac     2550

catggctgct gctttctgcc cagctcgcca gtcctcccca aagagttgcg catcagcacc     2610

tggggatctg gaccctgcgg gtgaagggat ggggagggac gtccctggag tctcttctgt     2670

ctttgttcct tcttattttg gcattcgata tcagcagcct ctccccaaag tacttgaagt     2730

cagttttaga tgctttattt tatttttcta gtcaaaaacg tgtttccccc agtgtttgaa     2790

aactcgtccg aatcttttca gtattttcca tgagtattgt ggtacttcta gacttgttta 2850
agcccagaac tcattccttc aaaacagaga gccttaatct ttatgttggg acacagacca 2910
catatttgga cggcagccat gcatccatcg ctgaagggct gtggacatga atgtgtattt 2970
cccatggtct ccgctgccca caccaacagt gtggcatctc ataagttaac tgctacccta 3030
aggtaatcta agattaaaat gtaaacattt atttttgtta tgtaagttta taagatgttt 3090
tatgttcaat gcctaatttc tcaaaagtgc cagaaaaaaa tgtatattag ctattttgat 3150
tttatgtaca atgatttata ctctcctttt gaaaagatac cataaagcac ataagctaga 3210
tcactacaag gagctgttat cttttttcta atcaagtgtt taaaacactg atggttttta 3270
aagactcacc tttttaaatg gtacttggag ctcctgattc aaattaccta gaccccctag 3330
agaaataaat ggaatataca taaataatca ttttcagtgg tttatggtgg gcaatattgc 3390
aatatttgaa atggtaaaaa tggaaagaag aacaaaatat gatgagaggt ggctgtgaat 3450
tataaacctc ataaaagtgt cataattcca ttaaggttta attatatttt ttcagaaaac 3510
agtgatgaat tctgtagtcc agtgcttgcc aatgcaaatt gcctattgga atcttcttcc 3570
tatattttac aaacatcagt ggctgaaata gctcagagta agagctcagc ctggtttgaa 3630
tttaatcatc tctttagatc ttataaggcc agcattagga aacttgttca cttttcattt 3690
tcaaaggagc ctagttgaag tgctattatg agtgtgggct atggaaagac agcttttcct 3750
acactgataa agaaaaaaaa tgaggaaatt atttcatccc cttgtgacat ctgtgacttt 3810
ttggatttaa taatcttgct gtttttcctc tttatgacaa agaatataat tgggaggatg 3870
aagtgtctta aaaattgtag agaccagctc actggaatgt ttttccatcc ctgtattcat 3930
ggcttgactt tgtgactgct ctacactgca tgtctgacat tgcagagtga gctatgttga 3990
ggtaaactgg ttggttgtca ttattttgca atcagcctgg tctctcccat gaagatgtcg 4050
tgtgcataag cacaatcatc actgattaga agatcacagc agaataccct tggattagag 4110
agaagttcgt accttgcatt tctctgaatt ctagtctctc ataagcactg ctttgctgga 4170
tgattttcac tgctttgtgt taatgacttt gagcgatctc tcacatgatg gggttcttta 4230
gtacatggta acagccatgt catcttacac acctagcatt gtgaatgctg tagtgacatc 4290
ctttataggc accttacagc tcaaaacttt tgtttcattt catgccttac ttatcaaaaa 4350
ggcaggaaag taggtatgat ctctaaagta aaaaaaaaaa aaaaaaaaaa actttttata 4410
gaaagctcat aaataatcat gtcattttgc aattttgtta ccaaaatttc ccccaagagt 4470
tttcaaatat tagttctgca atgtggctat gaaatatgca ctgaaatata ccttttaatt 4530
tgagaaccag tggttagaat aagctgtgat ataaagtatt ttcagtgtac ttttaaagga 4590
actataaggc cctccagcat aaacgctaaa agaatagatg gtagcacagg ccatgagggc 4650
tgggggagag aagcagagtg aaccttagaa agatggctca gctatttgga gcactggata 4710
ttttactgaa gttatttact gaggcaccat cactgttttg actgtacagt atagtttttc 4770
ataaatttca tcacatttac tttgttcaga atctgggctt gaatctttga gttggacaaa 4830

agcctatggt ttcttttaaa aagtttcatc ttgagctaat gctacagttt aaataaaatg 4890
tatgaaaggt agttatcaaa aacaatttgt atatttaaaa ttctattttg acatccattt 4950
tttacagcga attatagcag gttatgtgaa atttaaaaat aaattttagg aaattttttt 5010
atcttgtaag taagaattat aaatctatct cagttaaaag taggatcttt gtttttattc 5070
agatattttg aaagtttaga agcattttgt atgctacttt ggtatttctt aagtatagtc 5130
agcaggaaag ggtaacctgt tttatgcaaa atttttttaa tttgtcaaat gtttttagtg 5190
tgtctacttt ctgaaatagt ctcaatatcc tgtctgtatc ctaggcagat aactacacaa 5250
gaacaaaacg ctatatagaa aagaaaatat ttgaggaaat cttaaattcc ataagaaagt 5310
aattcttgca agaatgttgg ctacatattt ttttttctgt cctgaataaa ttacatcatt 5370
aggtctgttg aagcttttga agctaccact attctttctg ggataaaggc taattactga 5430
tttatctgcc ctcaaaatcc cagagttaat tctaattgag tactaaatta acagtaagta 5490
gttaacacag agaactaaaa tttaaccgca ttatatcact gtatatattt ctcatgattt 5550
ttgctaatta gagcatttac ttaaccagta ataaactaca cacacacaca cacacacaca 5610
cacgtatacg tgtgtgtgtg tatatacttg gctctgacac aactctggtg cttaattagg 5670
atatgttcta ttatgtattt tgaatgttta tcctgctagt gtgatgaact tttgttggaa 5730
aagcagaatt agaaggaaca gtttttcaat cagtttcatt tggtaattac aggaaaacct 5790
tgagttgctc taatttaatt tttccaatta atacatttag actttaaatt gcatccgtaa 5850
ttctcttggc tgaaaaacag tggtatttta agacagtttt gttatacatg tgacttactt 5910
ttgactcagc ctgagaggag gaaatgtaaa gagataagag atttggccca aagcagcaag 5970
agactagtaa ccttgagcca ggacgaagcc aagacaacgt gactcccaag tagcaggagc 6030
agaggtcggt cacctgtggc attctgtttc attccctttt aaaattatga gactttatag 6090
gcagtgtaaa acatcagcag gcagcgatgt gataggatgt gcgaaaaagc tggtctgata 6150
ccatggctat aattacagag ctttagttca attaggattt tgtaaatgag caaatgacct 6210
ttttttccag tgccccttgt aatagttaat atgagtccat gcaatcttgt gatgccattc 6270
tcctgaaggt gttgatttct ggtacccagc cagcttagct ttggctgctg gaagcacagt 6330
aggtactgat gattacttcc tggaaatctt gacaggctta ttgtactgtg taatggggaa 6390
aagttaaagt ataatgtttg gtgttaataa atgactgatg tgaaaatagg aacatgtgtc 6450
tttaatatca aatatggctt tatttgcagt gaaatctaaa ttccttattc tacagtagta 6510
ttttcttgcc ctgtgttgta tattttccta aatacatttt tcctgacagt ggctttaggc 6570
cagttgaaca cacttagact gaaagtgttt aacttaaaga gatcagtcca gaaaccatga 6630
taaattagaa tctttatctg gaattaccaa attaaaattt aaaatcatgg tccagaagag 6690
aacaaacatt catggttttt tttatcctac tgctcatttt taggtctgtg tttacatcta 6750
gtctctacta tttgtgaagt atgtcattat ttttttcaag ttcttctttg acttaactga 6810
aaaataatat tccaaattct taatgtaaca tgaaaagaga aatacaattt ttgcttaaaa 6870

gacatttttt aaaaagcgtt caattcagta atcatttctg ttaatacagg aagttttttt 6930
ggcttgccag ttatatactg tgggtatttt ttaaaatgtg ctacccttgg gttgtctcat 6990
atcaccttgc gtaatcatgt attacaaagg ttgataattg tcatttctca gatgtctgta 7050
tgttacattg gcagcagtaa aatgttttaa tgttgttacc ttttaaataa ttgtgttgta 7110
ttaacatgcc tcatattttc tggggaaact tgaaatatat ctaaacaaaa aaggtctgtt 7170
ataaatagga attggcattt cattgttaat gttttttcct cataaaaata gttacaccaa 7230
aagtgacata ttgtctatta catgggccca atcagtatta aagtactccc cttactcaaa 7290
aatattaaaa aataattcat gacacttagt agcattttca ttgattgttt caaaagatct 7350
tcataatggt caaattgtcc aattcacaaa agagtgagaa agttgttttc agtactggga 7410
atttttaaac cttagtttta agaccaaaaa tatctcttat tagcttgaac attttgtgat 7470
tacttttccc tccccggatc tagtcctttt aaggagtaag tctaaagaat gaggccatga 7530
agtcactatt tcctcccacc tcagtttgtc tcctggtact tagctggcct atcgctttgt 7590
gtggcaatac ccctgggtcg cttccccact cagccttctg tatctgctgt tcacaggaca 7650
tgccattatg tctttccagt acggatcaca gtgctgccat atcacagacc ctgccagccg 7710
gcacagaacc atgcctcgtc acagctctgc ccaactcaca ggctgccaag atgaggccac 7770
aggtccacct agggcggcag atgctggtgc tatttgtcac gacttttgcc aaaacatgta 7830
cactgttgct cacatctttc gtaagaatgg ttgacactaa ggggccatgg aaaagcactt 7890
ttaaaaaatt atgtggtgga atcaaactca cagaggcaga aatgttagtg ccgtgctcta 7950
accaagtaag cttagtgttc cccacagtga tctgtatctg gcctgagtct cctcaagcag 8010
caacccccac tgtccagcat gtggaaggta gatccttatg acaacaccag acccatagtt 8070
accaggaaag aaaaaggaag ctgagtgttg tgaaggggaa gaaatccttt ttataagaag 8130
taatcatttt taggccgggt gcagtggctc atgcctgtaa tcccagcact ttgggaggct 8190
gagacaggtg gatcacttga ggtcaggagt ttgagacctg cctggccaac atgatgaaac 8250
cctgtctcta ctaaaaatgc aaaaaaaatt agccaggcat ggtggcatgt gcctgtagtc 8310
ccaactactc aggaggctga ggcaggagaa tcacttgaac ctgggaggca gaggttgcaa 8370
tgagctgaga tcgcaacact gcactccagc ctgggcgaca gagcaagact ccatctcaaa 8430
aacaaaaaca aaaaaaaaac ctttttaaag taaattgagt gtaaaatgtt ttcaccatga 8490
agccattgca ccctctcccc caggaaacag tgcctaagga tgatgtcaag ttacagccag 8550
tttgctctga tcccccagga cctaagaaaa ctgtgagtgc ttcagcatgc aaaggtagag 8610
ctaccagata aggagaataa cttggccatt tatagcaaac tgcctgactt tggagtgaga 8670
aaccaagcac cttctaggtc ctaggatagg tgaattggag gtgcagctgc tgaagttccc 8730
attttctaca tacttcagga gccagggtgt ttcatttggt tttgtaaaac tttgctgtaa 8790
gtcatcacag ttaattcttt gaatggacat tttagagtgt aacttttttt caaaattagc 8850
agaacagata tctctgctca ttctctttat acatttagtt ttttaaagtt cccctagtat 8910

acctgttagg cactctaaat aggacacagt taaagctaaa agaaggacca gttgctccct 8970
agagcctaca gttcagaact atcttgttgt catgtcctgg tttggtttgg tttgttttta 9030
actcaacaag ttggaaacca tgagtttaaa gcaaactatt tccttttctt aagcttaaag 9090
agaatttttt ttttaatctt tagctcctca tttaggtagt gaaggcttcc ctgaaagaca 9150
gcagtgccct tctctggaag aagaggctgg gaccaaaagc ctgagtctat gggttgagga 9210
ttttgagcat tcctttgaca agcttctatg tatttttaat ataagactca taaatgaacc 9270
tgatgtggtg ttcatgaccc atcccttaag catgcttgct ttttttaact ttactttttg 9330
agtatagtac ttgtcttgtt tttcctggag ccatgctagg atttaattcc tatgtgccac 9390
tacccgccca tgtcctggaa gatggccatg cctgcagcaa tgctcatgtc ttgaggagaa 9450
gcaatgtgaa aagcatcagg aaagctgaac cttacgttca agtcagcaaa catttgtgag 9510
cattctagta tgcttgcgac cttgacactt tttaaaaatt atctttttat ttgcttcgag 9570
tatcaaccgt atagttgact ctagtggggc gaagggagca gagatgagag caaaattcag 9630
acttgctgcc cctgtgtgtc tcagttctca tgtctctctg agaaatgtgt tggcccaatt 9690
ccctatctgc cactctgagt ctcttgagta aatgtgtgta aatgagaaaa attatctcaa 9750
agatttaact tattatttag atattttctt ctttttaaac tcaggaataa acctggtttg 9810
tagataatat aatatggcta ctagagctat aattgagtag gtgactagaa gcattttttc 9870
aaatatattt cttgtaaaga catctacttt gttttaacta ggaaaagaaa ctcctcatat 9930
taaaataaac atttccataa gcactttcta gaagagtgta gcatccatct accatccatc 9990
ccagaatgtt tgtatcttgc tcatagtatt tcaaaaattt ttatgggtga aaaaaatgca 10050
cttttttatt tattggaaaa gtgtagagtt attgaattaa attctcaagt gggacaattt 10110
agtgtaaagg gaaacatggg ttttggagac aagagcacag ggctgtggtc cctgttccag 10170
cattgacttt actatgtgac ctcgagcaaa tgatttaact tctctgtgcc tcagtttctc 10230
catatacaaa atggggataa tgatactaac cattgcctac ctcatagaga tgttatgggg 10290
acaaacgaaa taatagagat aagcacgaat gctttcaact cttcggaaga aaggtgctat 10350
ataaattgaa gttgtgtttt taatgatcca attattaatt atgtttaggg tttaaataac 10410
aatactgtta gtcatgttaa gaataagttt tgttttgatg catatttcca cttctcttct 10470
gtggccagat cttgatcatt caaccagtaa tggtacctga ggaactgaaa tgggtatttg 10530
ttttcgtatg tttctgccag tagtatttca attgaatatc cagaaaagac tggagtttaa 10590
cttgtaatat cttatagttt acatgtaata aaacttattc aagctatata taaaagtatg 10650
attacatgta aatagcaggt atgttgtaat taaaggcact tatcaaattg tctttgttct 10710
tttttaattg taataaaagt cagttttaca taaaaaaaaa aaaaaaaaa 10759

<210> 110
<211> 432
<212> PRT
<213> human

<400> 110

Met Gly Pro Ala Gly Ser Leu Leu Gly Ser Gly Gln Met Gln Ile Thr
1               5                   10                  15

Leu Trp Gly Ser Leu Ala Ala Val Ala Ile Phe Phe Val Ile Thr Phe
            20                  25                  30

Leu Ile Phe Leu Cys Ser Ser Cys Asp Arg Glu Lys Lys Pro Arg Gln
        35                  40                  45

His Ser Gly Asp His Glu Asn Leu Met Asn Val Pro Ser Asp Lys Glu
    50                  55                  60

Met Phe Ser Arg Ser Val Thr Ser Leu Ala Thr Asp Ala Pro Ala Ser
65                  70                  75                  80

Ser Glu Gln Asn Gly Ala Leu Thr Asn Gly Asp Ile Leu Ser Glu Asp
                85                  90                  95

Ser Thr Leu Thr Cys Met Gln His Tyr Glu Glu Val Gln Thr Ser Ala
            100                 105                 110

Ser Asp Leu Leu Asp Ser Gln Asp Ser Thr Gly Lys Pro Lys Cys His
        115                 120                 125

Gln Ser Arg Glu Leu Pro Arg Ile Pro Pro Glu Ser Ala Val Asp Thr
    130                 135                 140

Met Leu Thr Ala Arg Ser Val Asp Gly Asp Gln Gly Leu Gly Met Glu
145                 150                 155                 160

Gly Pro Tyr Glu Val Leu Lys Asp Ser Ser Ser Gln Glu Asn Met Val
                165                 170                 175

Glu Asp Cys Leu Tyr Glu Thr Val Lys Glu Ile Lys Glu Val Ala Ala
            180                 185                 190

Ala Ala His Leu Glu Lys Gly His Ser Gly Lys Ala Lys Ser Thr Ser
        195                 200                 205

Ala Ser Lys Glu Leu Pro Gly Pro Gln Thr Glu Gly Lys Ala Glu Phe
    210                 215                 220

Ala Glu Tyr Ala Ser Val Asp Arg Asn Lys Lys Cys Arg Gln Ser Val
225                 230                 235                 240

Asn Val Glu Ser Ile Leu Gly Asn Ser Cys Asp Pro Glu Glu Glu Ala
                245                 250                 255

Pro Pro Pro Val Pro Val Lys Leu Leu Asp Glu Asn Glu Asn Leu Gln
            260                 265                 270

Glu Lys Glu Gly Gly Glu Ala Glu Glu Ser Ala Thr Asp Thr Thr Ser
        275                 280                 285

Glu Thr Asn Lys Arg Phe Ser Ser Leu Ser Tyr Lys Ser Arg Glu Glu
    290                 295                 300

Asp Pro Thr Leu Thr Glu Glu Glu Ile Ser Ala Met Tyr Ser Ser Val
305                 310                 315                 320

Asn Lys Pro Gly Gln Leu Val Asn Lys Ser Gly Gln Ser Leu Thr Val
                325                 330                 335

Pro Glu Ser Thr Tyr Thr Ser Ile Gln Gly Asp Pro Gln Arg Ser Pro
            340                 345                 350

Ser Ser Cys Asn Asp Leu Tyr Ala Thr Val Lys Asp Phe Glu Lys Thr
        355                 360                 365

Pro Asn Ser Thr Leu Pro Pro Ala Gly Arg Pro Ser Glu Glu Pro Glu
    370                 375                 380

Pro Asp Tyr Glu Ala Ile Gln Thr Leu Asn Arg Glu Glu Glu Lys Ala
385                 390                 395                 400

Thr Leu Gly Thr Asn Gly His His Gly Leu Val Pro Lys Glu Asn Asp
                405                 410                 415

Tyr Glu Ser Ile Ser Asp Leu Gln Gln Gly Arg Asp Ile Thr Arg Leu
            420                 425                 430

<210>  111
<211>  4599
<212>  DNA
<213>  human

<220>
<221>  CDS
<222>  (1)..(2793)

<400>  111
atg gga ggg agc tgc gcg cag agg cgc cgg gcc ggc ccg cgg cag gtg      48
Met Gly Gly Ser Cys Ala Gln Arg Arg Arg Ala Gly Pro Arg Gln Val
1               5                   10                  15

cta ttt cct ttg ctg ctg cct ttg ttc tac ccc acc ctg agt gag ccg      96
Leu Phe Pro Leu Leu Leu Pro Leu Phe Tyr Pro Thr Leu Ser Glu Pro
            20                  25                  30

atc cgc tac tcg att ccg gag gag ctg gcc aag ggc tcg gtg gtg ggg      144
Ile Arg Tyr Ser Ile Pro Glu Glu Leu Ala Lys Gly Ser Val Val Gly
        35                  40                  45

aac ctc gct aag gat cta ggg ctc agt gtc ctg gat gtg tcg gct cgc      192
Asn Leu Ala Lys Asp Leu Gly Leu Ser Val Leu Asp Val Ser Ala Arg
    50                  55                  60

aag ctg cga gtg agc gcg gag aag ctg cac ttc agc gta gac gcg gag 240
Lys Leu Arg Val Ser Ala Glu Lys Leu His Phe Ser Val Asp Ala Glu
65                  70                  75                  80

agc ggg gac tta ctt gtg aag aac cga ata gac cgt gag caa ata tgc 288
Ser Gly Asp Leu Leu Val Lys Asn Arg Ile Asp Arg Glu Gln Ile Cys
                85                  90                  95

aaa gag aga aga aga tgt gag ttg caa ttg gaa gct gtg gtg gaa aat 336
Lys Glu Arg Arg Arg Cys Glu Leu Gln Leu Glu Ala Val Val Glu Asn
            100                 105                 110

cct tta aat att ttt cat gtc att gtg gtg att gag gat gtt aat gac 384
Pro Leu Asn Ile Phe His Val Ile Val Val Ile Glu Asp Val Asn Asp
        115                 120                 125

cac gcc cct caa ttt gat aaa aag gaa ata cat tta gaa att ttc gaa 432
His Ala Pro Gln Phe Asp Lys Lys Glu Ile His Leu Glu Ile Phe Glu
    130                 135                 140

tct gca tcc gct ggt aca cga cta tcg ctt gac cct gcc acg gat cct 480
Ser Ala Ser Ala Gly Thr Arg Leu Ser Leu Asp Pro Ala Thr Asp Pro
145                 150                 155                 160

gat ata aac ata aac tca att aaa gat tat aag ata aac tct aat cct 528
Asp Ile Asn Ile Asn Ser Ile Lys Asp Tyr Lys Ile Asn Ser Asn Pro
                165                 170                 175

tat ttt tca tta atg gtt aga gtt aat tcc gat ggt ggc aaa tac cca 576
Tyr Phe Ser Leu Met Val Arg Val Asn Ser Asp Gly Gly Lys Tyr Pro
            180                 185                 190

gag tta tct ctg gag aaa ctc cta gac cgg gaa gaa cag aga tct cat 624
Glu Leu Ser Leu Glu Lys Leu Leu Asp Arg Glu Glu Gln Arg Ser His
        195                 200                 205

agc ttg ata ttg act gcc ttg gac gga ggg gac cca cca aga agt gcc 672
Ser Leu Ile Leu Thr Ala Leu Asp Gly Gly Asp Pro Pro Arg Ser Ala
    210                 215                 220

acc gct cac ata gaa att tct gtc aag gat acc aat gat aac ccc ccg 720
Thr Ala His Ile Glu Ile Ser Val Lys Asp Thr Asn Asp Asn Pro Pro
225                 230                 235                 240

gtt ttc agc aga gac gaa tat aga att agt ctt agt gaa aat ctg ccc 768
Val Phe Ser Arg Asp Glu Tyr Arg Ile Ser Leu Ser Glu Asn Leu Pro
                245                 250                 255

cct ggg tcc cct gtg ttg caa gtg aca gcc act gac cag gat gag ggg 816
Pro Gly Ser Pro Val Leu Gln Val Thr Ala Thr Asp Gln Asp Glu Gly
            260                 265                 270

gtc aat gct gag ata aac tac tac ttc cga agc act gcc cag agc aca 864
Val Asn Ala Glu Ile Asn Tyr Tyr Phe Arg Ser Thr Ala Gln Ser Thr
        275                 280                 285

aaa cat atg ttc tca ttg gat gag aaa aca ggt atg att aag aat aac 912
Lys His Met Phe Ser Leu Asp Glu Lys Thr Gly Met Ile Lys Asn Asn
    290                 295                 300

cag tca ttt gat ttt gaa gat gta gaa agg tac acc atg gaa gtg gaa 960
Gln Ser Phe Asp Phe Glu Asp Val Glu Arg Tyr Thr Met Glu Val Glu
305                 310                 315                 320

gcg aag gac gga ggt ggt ctc tct acc cag tgt aaa gta atc ata gaa 1008
Ala Lys Asp Gly Gly Gly Leu Ser Thr Gln Cys Lys Val Ile Ile Glu
                325                 330                 335

atc ctt gat gaa aac gac aac agc cca gaa ata atc atc act tct ctc 1056
Ile Leu Asp Glu Asn Asp Asn Ser Pro Glu Ile Ile Ile Thr Ser Leu
            340                 345                 350

tct gat cag att ttg gag aat tca cct cca gga atg gtt gtt gcc ctc 1104
Ser Asp Gln Ile Leu Glu Asn Ser Pro Pro Gly Met Val Val Ala Leu
        355                 360                 365

ttc aaa aca cgg gat ctg gat ttc gga gga aat gga gaa gtc agg tgt 1152
Phe Lys Thr Arg Asp Leu Asp Phe Gly Gly Asn Gly Glu Val Arg Cys
    370                 375                 380

aat ata gaa aca gac att cca ttc aag att tat tct tct tcc aat aac 1200
Asn Ile Glu Thr Asp Ile Pro Phe Lys Ile Tyr Ser Ser Ser Asn Asn
385                 390                 395                 400

tac tac aaa ctg gtg aca gat gga gcc ctg gac cga gag cag aca cca 1248
Tyr Tyr Lys Leu Val Thr Asp Gly Ala Leu Asp Arg Glu Gln Thr Pro
                405                 410                 415

gaa tac aat gtc acc atc gta gcc act gac agg ggc aag ccg cct ctt 1296
Glu Tyr Asn Val Thr Ile Val Ala Thr Asp Arg Gly Lys Pro Pro Leu
            420                 425                 430

tct tcc agt aga agc atc acc ttg tat gtc gct gac atc aac gac aac 1344
Ser Ser Ser Arg Ser Ile Thr Leu Tyr Val Ala Asp Ile Asn Asp Asn
        435                 440                 445

gcc cca gtt ttc gac cag acg tcc tac gtg gtc cac gtg gcc gag aac 1392
Ala Pro Val Phe Asp Gln Thr Ser Tyr Val Val His Val Ala Glu Asn
    450                 455                 460

aac ccg cca gga gcc tcc att gcg caa gtg agc gcc tct gac ccg gat 1440
Asn Pro Pro Gly Ala Ser Ile Ala Gln Val Ser Ala Ser Asp Pro Asp
465                 470                 475                 480

ttg ggg ctc aat ggc cac atc tcc tac tct ata gtg gcg agt gac cta 1488
Leu Gly Leu Asn Gly His Ile Ser Tyr Ser Ile Val Ala Ser Asp Leu
                485                 490                 495

gag ccc ctg gcg gtg tcg tca tac gtg tca gtg agc gcg cag agc ggg 1536
Glu Pro Leu Ala Val Ser Ser Tyr Val Ser Val Ser Ala Gln Ser Gly
            500                 505                 510

gtg gtg ttc gcg cag cgc gcc ttt gat cac gag cag ctg cgc gcc ttc 1584
Val Val Phe Ala Gln Arg Ala Phe Asp His Glu Gln Leu Arg Ala Phe
        515                 520                 525

gcg ctc acg ctg cag gcc cgc gac cac ggc tcg ccc acg ctc agc gcc 1632
Ala Leu Thr Leu Gln Ala Arg Asp His Gly Ser Pro Thr Leu Ser Ala
    530                 535                 540

aac gtg agc ctg cgc gtg ttg gtg gga gac cgc aat gac aac gca ccg 1680
Asn Val Ser Leu Arg Val Leu Val Gly Asp Arg Asn Asp Asn Ala Pro
545                 550                 555                 560

cgg gtg ctg tac cca gct ctg ggt cct gac ggc tcc gcg ttc ttc gat 1728
Arg Val Leu Tyr Pro Ala Leu Gly Pro Asp Gly Ser Ala Phe Phe Asp
                565                 570                 575

atg gta cct cgc tct gca gag ccc ggc tac cta gtg act aag gtg gta 1776
Met Val Pro Arg Ser Ala Glu Pro Gly Tyr Leu Val Thr Lys Val Val
            580                 585                 590

gcg gtg gac gcc gac tcg gga cac aac gcc tgg ctg tcc tac cac gtg 1824
Ala Val Asp Ala Asp Ser Gly His Asn Ala Trp Leu Ser Tyr His Val
        595                 600                 605

ctg cag gcc agt gag ccc gga ctc ttc agc ctg ggg ctg cgc act ggg 1872
Leu Gln Ala Ser Glu Pro Gly Leu Phe Ser Leu Gly Leu Arg Thr Gly
    610                 615                 620

gag gtg cgc acg gct cga gcc tta ggc gac agg gac gca gcc cgc cag 1920
Glu Val Arg Thr Ala Arg Ala Leu Gly Asp Arg Asp Ala Ala Arg Gln
625                 630                 635                 640

cgc ctg ctg gtc gct gtg cgt gac ggt gga cag ccg cca ctc tct gcc 1968
Arg Leu Leu Val Ala Val Arg Asp Gly Gly Gln Pro Pro Leu Ser Ala
                645                 650                 655

acc gcc acg ctt cat ctg gtc ttc gca gac aac ttg caa gag ata ctg 2016
Thr Ala Thr Leu His Leu Val Phe Ala Asp Asn Leu Gln Glu Ile Leu
            660                 665                 670

cca gac ctc agc gac cgc cct gta ctc tct gac ccc cag gct gaa ctg 2064
Pro Asp Leu Ser Asp Arg Pro Val Leu Ser Asp Pro Gln Ala Glu Leu
        675                 680                 685

cag ttt tac ctg gtg gtg gcc ttg gcc tta atc tca gtg ctc ttc ctc 2112
Gln Phe Tyr Leu Val Val Ala Leu Ala Leu Ile Ser Val Leu Phe Leu
    690                 695                 700

ctc gcc gtg att ctg gcc att gcc ttg cgc ctg cga cgc tct ctc agc 2160
Leu Ala Val Ile Leu Ala Ile Ala Leu Arg Leu Arg Arg Ser Leu Ser
705                 710                 715                 720

cct gct act tgg gac tgc ttc cat cct ggt ctc tgt gtc aag tct gga 2208
Pro Ala Thr Trp Asp Cys Phe His Pro Gly Leu Cys Val Lys Ser Gly
                725                 730                 735

cct gta gtt ccc ccc aac tac agt gag ggg act ttg cct tat tct tat 2256
Pro Val Val Pro Pro Asn Tyr Ser Glu Gly Thr Leu Pro Tyr Ser Tyr
            740                 745                 750

aat ctg tgc att gca cat acg ggt aca aaa gag ttt aat ttc cta aaa 2304
Asn Leu Cys Ile Ala His Thr Gly Thr Lys Glu Phe Asn Phe Leu Lys
        755                 760                 765

tgc agt gtg ccc cta cat tcc aat gaa gac atg gtt tgc agt gtt tct 2352
Cys Ser Val Pro Leu His Ser Asn Glu Asp Met Val Cys Ser Val Ser
    770                 775                 780

cct gga gcc tta att cca cct cat ggt ggg gag gat ttg act tca cat 2400
Pro Gly Ala Leu Ile Pro Pro His Gly Gly Glu Asp Leu Thr Ser His
785                 790                 795                 800

cct gag act ctg act tcg caa gcc ccg ccc aac acg gac tgg cgt ttc 2448
Pro Glu Thr Leu Thr Ser Gln Ala Pro Pro Asn Thr Asp Trp Arg Phe
                805                 810                 815

tct cag gcc cag aga ccc ggc acc agc ggc tcc caa aat ggc gat gac 2496
Ser Gln Ala Gln Arg Pro Gly Thr Ser Gly Ser Gln Asn Gly Asp Asp
            820                 825                 830

acc ggc acc tgg ccc aac aac cag ttt gac aca gag atg ctg caa gcc 2544
Thr Gly Thr Trp Pro Asn Asn Gln Phe Asp Thr Glu Met Leu Gln Ala
        835                 840                 845

atg atc ttg gcg tcc gcc agt gaa gct gct gat ggg agc tcc acc ctg 2592
Met Ile Leu Ala Ser Ala Ser Glu Ala Ala Asp Gly Ser Ser Thr Leu
    850                 855                 860

gga ggg ggt gcc ggc acc atg gga ttg agc gcc cgc tac gga ccc cag 2640
Gly Gly Gly Ala Gly Thr Met Gly Leu Ser Ala Arg Tyr Gly Pro Gln
865                 870                 875                 880

ttc acc ctg cag cac gtg ccc gac tac cgc cag aat gtc tac atc cca     2688
Phe Thr Leu Gln His Val Pro Asp Tyr Arg Gln Asn Val Tyr Ile Pro
                885                 890                 895

ggc agc aat gcc aca ctg acc aac gca gct ggc aag cgg gat ggc aag     2736
Gly Ser Asn Ala Thr Leu Thr Asn Ala Ala Gly Lys Arg Asp Gly Lys
            900                 905                 910

gcc cca gca ggt ggc aat ggc aac aag aag aag tcg ggc aag aag gag     2784
Ala Pro Ala Gly Gly Asn Gly Asn Lys Lys Lys Ser Gly Lys Lys Glu
        915                 920                 925

aag aag taa catggaggcc aggccaagag ccacagggcg gcctctcccc             2833
Lys Lys
    930

aaccagccca gcttctcctt acctgcaccc aggcctcaga gtttcagggc taacccccag   2893

aatactggta ggggccaagg ccatgctccc cttgggaaac agaaacaagt gcccagtcag   2953

cacctacccc ttccccccca gggggttgaa tatgcaaaag cagttccgct gggaaccccc   3013

atccaatcaa ctgctgtacc catgggggta gtggggttac tgtagacacc aagaaccatt   3073

tgccacaccc cgtttagtta cagctgaact cctccatctt ccaaatcaat caggcccatc   3133

catcccatgc ctccctcctc cccaccccac tccaacagtt cctctttccc gagtaaggtg   3193

gttggggtgt tgaagtacca agtaacctac aagcctccta gttctgaaaa gttggaaggg   3253

catcatgacc tcttggcctc tcctttgatt ctcaatcttc ccccaaagca tggtttggtg   3313

ccagcccctt cacctccttc cagagcccaa gatcaatgct caagttttgg aggacatgat   3373

caccatcccc atggtactga tgcttgctgg atttagggag ggcattttgc taccaagcct   3433

cttcccaacg ccctggggac cagtcttctg ttttgttttt cattgtttga cgtttccact   3493

gcatgccttg acttccccca cctcctcctc aaacaagaga ctccactgca tgttccaaga   3553

cagtatgggg tggtaagata aggaagggaa gtgtgtggat gtggatggtg ggggcatgga    3613

caaagcttga cacatcaagt tatcaaggcc ttggaggagg ctctgtatgt cctcagggga   3673

ctgacaacat cctccagatt ccagccataa accaataact aggctggacc cttcccacta   3733

cataataggg ctcagcccag gcagccagct ttgggctgag ctaacaggac caatggatta   3793

aactggcatt tcagtccaag gaagctcgaa gcaggtttag gaccaggtcc ccttgagagg   3853

tcagaggggc ctctgtgggt gctgggtact ccagaggtgc cactggtgga agggtcagcg   3913

gagccccagc aggaagggtg ggccagccag gccattctta gtccctgggt tggggaggca   3973

gggagctagg gcagggacca aatgaacaga aagtctcagc ccaggatggg gcttcttcaa   4033

cagggcccct gccctcctga agcctcagtc cttcaccttg ccaggtgccg tttctcttcc   4093

gtgaaggcca ctgcccaggt ccccagtgcg ccccctagtg gccatagcct ggttaaagtt   4153

ccccagtgcc tccttgtgca tagaccttct tctcccaccc ccttctgccc ctgggtcccc   4213

ggccatccag cggggctgcc agagaacccc agacctgccc ttacagtagt gtagcgcccc   4273

ctccctcttt cggctggtgt agaatagcca gtagtgtagt gcggtgtgct tttacgtgat   4333

ggcgggtggg cagcgggcgg cgggctccgc gcagccgtct gtccttgatc tgcccgcggc   4393

ggcccgtgtt gtgttttgtg ctgtgtccac gcgctaaggc gaccccctcc cccgtactga 4453

cttctcctat aagcgcttct cttcgcatag tcacgtagct cccaccccac cctcttcctg 4513

tgtctcacgc aagttttata ctctaatatt tatatggctt tttttcttcg acaaaaaaat 4573

aataaaacgt ttcttctgaa aagctg 4599

<210> 112
<211> 930
<212> PRT
<213> human

<400> 112

Met Gly Gly Ser Cys Ala Gln Arg Arg Arg Ala Gly Pro Arg Gln Val
1               5                   10                  15

Leu Phe Pro Leu Leu Leu Pro Leu Phe Tyr Pro Thr Leu Ser Glu Pro
            20                  25                  30

Ile Arg Tyr Ser Ile Pro Glu Glu Leu Ala Lys Gly Ser Val Val Gly
        35                  40                  45

Asn Leu Ala Lys Asp Leu Gly Leu Ser Val Leu Asp Val Ser Ala Arg
    50                  55                  60

Lys Leu Arg Val Ser Ala Glu Lys Leu His Phe Ser Val Asp Ala Glu
65                  70                  75                  80

Ser Gly Asp Leu Leu Val Lys Asn Arg Ile Asp Arg Glu Gln Ile Cys
                85                  90                  95

Lys Glu Arg Arg Arg Cys Glu Leu Gln Leu Glu Ala Val Val Glu Asn
            100                 105                 110

Pro Leu Asn Ile Phe His Val Ile Val Val Ile Glu Asp Val Asn Asp
        115                 120                 125

His Ala Pro Gln Phe Asp Lys Lys Glu Ile His Leu Glu Ile Phe Glu
    130                 135                 140

Ser Ala Ser Ala Gly Thr Arg Leu Ser Leu Asp Pro Ala Thr Asp Pro
145                 150                 155                 160

Asp Ile Asn Ile Asn Ser Ile Lys Asp Tyr Lys Ile Asn Ser Asn Pro
                165                 170                 175

Tyr Phe Ser Leu Met Val Arg Val Asn Ser Asp Gly Gly Lys Tyr Pro
            180                 185                 190

Glu Leu Ser Leu Glu Lys Leu Leu Asp Arg Glu Glu Gln Arg Ser His
        195                 200                 205

Ser Leu Ile Leu Thr Ala Leu Asp Gly Gly Asp Pro Pro Arg Ser Ala
    210                 215                 220

Thr Ala His Ile Glu Ile Ser Val Lys Asp Thr Asn Asp Asn Pro Pro
225                 230                 235                 240

Val Phe Ser Arg Asp Glu Tyr Arg Ile Ser Leu Ser Glu Asn Leu Pro
                245                 250                 255

Pro Gly Ser Pro Val Leu Gln Val Thr Ala Thr Asp Gln Asp Glu Gly
            260                 265                 270

Val Asn Ala Glu Ile Asn Tyr Tyr Phe Arg Ser Thr Ala Gln Ser Thr
        275                 280                 285

Lys His Met Phe Ser Leu Asp Glu Lys Thr Gly Met Ile Lys Asn Asn
    290                 295                 300

Gln Ser Phe Asp Phe Glu Asp Val Glu Arg Tyr Thr Met Glu Val Glu
305                 310                 315                 320

Ala Lys Asp Gly Gly Gly Leu Ser Thr Gln Cys Lys Val Ile Ile Glu
                325                 330                 335

Ile Leu Asp Glu Asn Asp Asn Ser Pro Glu Ile Ile Ile Thr Ser Leu
            340                 345                 350

Ser Asp Gln Ile Leu Glu Asn Ser Pro Pro Gly Met Val Val Ala Leu
        355                 360                 365

Phe Lys Thr Arg Asp Leu Asp Phe Gly Gly Asn Gly Glu Val Arg Cys
    370                 375                 380

Asn Ile Glu Thr Asp Ile Pro Phe Lys Ile Tyr Ser Ser Ser Asn Asn
385                 390                 395                 400

Tyr Tyr Lys Leu Val Thr Asp Gly Ala Leu Asp Arg Glu Gln Thr Pro
                405                 410                 415

Glu Tyr Asn Val Thr Ile Val Ala Thr Asp Arg Gly Lys Pro Pro Leu
            420                 425                 430

Ser Ser Ser Arg Ser Ile Thr Leu Tyr Val Ala Asp Ile Asn Asp Asn
        435                 440                 445

Ala Pro Val Phe Asp Gln Thr Ser Tyr Val Val His Val Ala Glu Asn
    450                 455                 460

Asn Pro Pro Gly Ala Ser Ile Ala Gln Val Ser Ala Ser Asp Pro Asp
465                 470                 475                 480

Leu Gly Leu Asn Gly His Ile Ser Tyr Ser Ile Val Ala Ser Asp Leu
                485                 490                 495

Glu Pro Leu Ala Val Ser Ser Tyr Val Ser Val Ser Ala Gln Ser Gly
            500                 505                 510

Val Val Phe Ala Gln Arg Ala Phe Asp His Glu Gln Leu Arg Ala Phe
        515                 520                 525

Ala Leu Thr Leu Gln Ala Arg Asp His Gly Ser Pro Thr Leu Ser Ala
    530                 535                 540

Asn Val Ser Leu Arg Val Leu Val Gly Asp Arg Asn Asp Asn Ala Pro
545                 550                 555                 560

Arg Val Leu Tyr Pro Ala Leu Gly Pro Asp Gly Ser Ala Phe Phe Asp
                565                 570                 575

Met Val Pro Arg Ser Ala Glu Pro Gly Tyr Leu Val Thr Lys Val Val
            580                 585                 590

Ala Val Asp Ala Asp Ser Gly His Asn Ala Trp Leu Ser Tyr His Val
        595                 600                 605

Leu Gln Ala Ser Glu Pro Gly Leu Phe Ser Leu Gly Leu Arg Thr Gly
    610                 615                 620

Glu Val Arg Thr Ala Arg Ala Leu Gly Asp Arg Asp Ala Ala Arg Gln
625                 630                 635                 640

Arg Leu Leu Val Ala Val Arg Asp Gly Gly Gln Pro Pro Leu Ser Ala
                645                 650                 655

Thr Ala Thr Leu His Leu Val Phe Ala Asp Asn Leu Gln Glu Ile Leu
            660                 665                 670

Pro Asp Leu Ser Asp Arg Pro Val Leu Ser Asp Pro Gln Ala Glu Leu
        675                 680                 685

Gln Phe Tyr Leu Val Val Ala Leu Ala Leu Ile Ser Val Leu Phe Leu
    690                 695                 700

Leu Ala Val Ile Leu Ala Ile Ala Leu Arg Leu Arg Arg Ser Leu Ser
705                 710                 715                 720

Pro Ala Thr Trp Asp Cys Phe His Pro Gly Leu Cys Val Lys Ser Gly
                725                 730                 735

Pro Val Val Pro Pro Asn Tyr Ser Glu Gly Thr Leu Pro Tyr Ser Tyr
            740                 745                 750

Asn Leu Cys Ile Ala His Thr Gly Thr Lys Glu Phe Asn Phe Leu Lys
        755                 760                 765

Cys Ser Val Pro Leu His Ser Asn Glu Asp Met Val Cys Ser Val Ser
    770                 775                 780

Pro Gly Ala Leu Ile Pro Pro His Gly Gly Glu Asp Leu Thr Ser His
785                 790                 795                 800

Pro Glu Thr Leu Thr Ser Gln Ala Pro Pro Asn Thr Asp Trp Arg Phe
                805                 810                 815

Ser Gln Ala Gln Arg Pro Gly Thr Ser Gly Ser Gln Asn Gly Asp Asp
            820                 825                 830

Thr Gly Thr Trp Pro Asn Asn Gln Phe Asp Thr Glu Met Leu Gln Ala
        835                 840                 845

Met Ile Leu Ala Ser Ala Ser Glu Ala Ala Asp Gly Ser Ser Thr Leu
    850                 855                 860

Gly Gly Gly Ala Gly Thr Met Gly Leu Ser Ala Arg Tyr Gly Pro Gln
865                 870                 875                 880

Phe Thr Leu Gln His Val Pro Asp Tyr Arg Gln Asn Val Tyr Ile Pro
                885                 890                 895

Gly Ser Asn Ala Thr Leu Thr Asn Ala Ala Gly Lys Arg Asp Gly Lys
            900                 905                 910

Ala Pro Ala Gly Gly Asn Gly Asn Lys Lys Lys Ser Gly Lys Lys Glu
        915                 920                 925

Lys Lys
    930

<210>  113
<211>  2463
<212>  DNA
<213>  human

<220>
<221>  CDS
<222>  (1)..(2463)

<400>  113
atg gga ggg agc tgc gcg cag agg cgc cgg gcc ggc ccg cgg cag gtg        48
Met Gly Gly Ser Cys Ala Gln Arg Arg Arg Ala Gly Pro Arg Gln Val
1               5                   10                  15

cta ttt cct ttg ctg ctg cct ttg ttc tac ccc acc ctg agt gag ccg        96
Leu Phe Pro Leu Leu Leu Pro Leu Phe Tyr Pro Thr Leu Ser Glu Pro
            20                  25                  30

atc cgc tac tcg att ccg gag gag ctg gcc aag ggc tcg gtg gtg ggg 144
Ile Arg Tyr Ser Ile Pro Glu Glu Leu Ala Lys Gly Ser Val Val Gly
        35                  40                  45

aac ctc gct aag gat cta ggg ctc agt gtc ctg gat gtg tcg gct cgc 192
Asn Leu Ala Lys Asp Leu Gly Leu Ser Val Leu Asp Val Ser Ala Arg
    50                  55                  60

aag ctg cga gtg agc gcg gag aag ctg cac ttc agc gta gac gcg gag 240
Lys Leu Arg Val Ser Ala Glu Lys Leu His Phe Ser Val Asp Ala Glu
65                  70                  75                  80

agc ggg gac tta ctt gtg aag aac cga ata gac cgt gag caa ata tgc 288
Ser Gly Asp Leu Leu Val Lys Asn Arg Ile Asp Arg Glu Gln Ile Cys
                85                  90                  95

aaa gag aga aga aga tgt gag ttg caa ttg gaa gct gtg gtg gaa aat 336
Lys Glu Arg Arg Arg Cys Glu Leu Gln Leu Glu Ala Val Val Glu Asn
            100                 105                 110

cct tta aat att ttt cat gtc att gtg gtg att gag gat gtt aat gac 384
Pro Leu Asn Ile Phe His Val Ile Val Val Ile Glu Asp Val Asn Asp
        115                 120                 125

cac gcc cct caa ttt gat aaa aag gaa ata cat tta gaa att ttc gaa 432
His Ala Pro Gln Phe Asp Lys Lys Glu Ile His Leu Glu Ile Phe Glu
    130                 135                 140

tct gca tcc gct ggt aca cga cta tcg ctt gac cct gcc acg gat cct 480
Ser Ala Ser Ala Gly Thr Arg Leu Ser Leu Asp Pro Ala Thr Asp Pro
145                 150                 155                 160

gat ata aac ata aac tca att aaa gat tat aag ata aac tct aat cct 528
Asp Ile Asn Ile Asn Ser Ile Lys Asp Tyr Lys Ile Asn Ser Asn Pro
                165                 170                 175

tat ttt tca tta atg gtt aga gtt aat tcc gat ggt ggc aaa tac cca 576
Tyr Phe Ser Leu Met Val Arg Val Asn Ser Asp Gly Gly Lys Tyr Pro
            180                 185                 190

gag tta tct ctg gag aaa ctc cta gac cgg gaa gaa cag aga tct cat 624
Glu Leu Ser Leu Glu Lys Leu Leu Asp Arg Glu Glu Gln Arg Ser His
        195                 200                 205

agc ttg ata ttg act gcc ttg gac gga ggg gac cca cca aga agt gcc 672
Ser Leu Ile Leu Thr Ala Leu Asp Gly Gly Asp Pro Pro Arg Ser Ala
    210                 215                 220

acc gct cac ata gaa att tct gtc aag gat acc aat gat aac ccc ccg 720
Thr Ala His Ile Glu Ile Ser Val Lys Asp Thr Asn Asp Asn Pro Pro
225                 230                 235                 240

gtt ttc agc aga gac gaa tat aga att agt ctt agt gaa aat ctg ccc 768
Val Phe Ser Arg Asp Glu Tyr Arg Ile Ser Leu Ser Glu Asn Leu Pro
                245                 250                 255

cct ggg tcc cct gtg ttg caa gtg aca gcc act gac cag gat gag ggg 816
Pro Gly Ser Pro Val Leu Gln Val Thr Ala Thr Asp Gln Asp Glu Gly
            260                 265                 270

gtc aat gct gag ata aac tac tac ttc cga agc act gcc cag agc aca 864
Val Asn Ala Glu Ile Asn Tyr Tyr Phe Arg Ser Thr Ala Gln Ser Thr
        275                 280                 285

aaa cat atg ttc tca ttg gat gag aaa aca ggt atg att aag aat aac 912
Lys His Met Phe Ser Leu Asp Glu Lys Thr Gly Met Ile Lys Asn Asn
    290                 295                 300

cag tca ttt gat ttt gaa gat gta gaa agg tac acc atg gaa gtg gaa 960
Gln Ser Phe Asp Phe Glu Asp Val Glu Arg Tyr Thr Met Glu Val Glu
305                 310                 315                 320

gcg aag gac gga ggt ggt ctc tct acc cag tgt aaa gta atc ata gaa 1008
Ala Lys Asp Gly Gly Gly Leu Ser Thr Gln Cys Lys Val Ile Ile Glu
                325                 330                 335

atc ctt gat gaa aac gac aac agc cca gaa ata atc atc act tct ctc 1056
Ile Leu Asp Glu Asn Asp Asn Ser Pro Glu Ile Ile Ile Thr Ser Leu
            340                 345                 350

tct gat cag att ttg gag aat tca cct cca gga atg gtt gtt gcc ctc 1104
Ser Asp Gln Ile Leu Glu Asn Ser Pro Pro Gly Met Val Val Ala Leu
        355                 360                 365

ttc aaa aca cgg gat ctg gat ttc gga gga aat gga gaa gtc agg tgt 1152
Phe Lys Thr Arg Asp Leu Asp Phe Gly Gly Asn Gly Glu Val Arg Cys
    370                 375                 380

aat ata gaa aca gac att cca ttc aag att tat tct tct tcc aat aac 1200
Asn Ile Glu Thr Asp Ile Pro Phe Lys Ile Tyr Ser Ser Ser Asn Asn
385                 390                 395                 400

tac tac aaa ctg gtg aca gat gga gcc ctg gac cga gag cag aca cca 1248
Tyr Tyr Lys Leu Val Thr Asp Gly Ala Leu Asp Arg Glu Gln Thr Pro
                405                 410                 415

gaa tac aat gtc acc atc gta gcc act gac agg ggc aag ccg cct ctt 1296
Glu Tyr Asn Val Thr Ile Val Ala Thr Asp Arg Gly Lys Pro Pro Leu
            420                 425                 430

tct tcc agt aga agc atc acc ttg tat gtc gct gac atc aac gac aac 1344
Ser Ser Ser Arg Ser Ile Thr Leu Tyr Val Ala Asp Ile Asn Asp Asn
        435                 440                 445

gcc cca gtt ttc gac cag acg tcc tac gtg gtc cac gtg gcc gag aac 1392
Ala Pro Val Phe Asp Gln Thr Ser Tyr Val Val His Val Ala Glu Asn
    450                 455                 460

aac ccg cca gga gcc tcc att gcg caa gtg agc gcc tct gac ccg gat 1440
Asn Pro Pro Gly Ala Ser Ile Ala Gln Val Ser Ala Ser Asp Pro Asp
465                 470                 475                 480

ttg ggg ctc aat ggc cac atc tcc tac tct ata gtg gcg agt gac cta 1488
Leu Gly Leu Asn Gly His Ile Ser Tyr Ser Ile Val Ala Ser Asp Leu
                485                 490                 495

gag ccc ctg gcg gtg tcg tca tac gtg tca gtg agc gcg cag agc ggg 1536
Glu Pro Leu Ala Val Ser Ser Tyr Val Ser Val Ser Ala Gln Ser Gly
            500                 505                 510

gtg gtg ttc gcg cag cgc gcc ttt gat cac gag cag ctg cgc gcc ttc 1584
Val Val Phe Ala Gln Arg Ala Phe Asp His Glu Gln Leu Arg Ala Phe
        515                 520                 525

gcg ctc acg ctg cag gcc cgc gac cac ggc tcg ccc acg ctc agc gcc 1632
Ala Leu Thr Leu Gln Ala Arg Asp His Gly Ser Pro Thr Leu Ser Ala
    530                 535                 540

aac gtg agc ctg cgc gtg ttg gtg gga gac cgc aat gac aac gca ccg 1680
Asn Val Ser Leu Arg Val Leu Val Gly Asp Arg Asn Asp Asn Ala Pro
545                 550                 555                 560

cgg gtg ctg tac cca gct ctg ggt cct gac ggc tcc gcg ttc ttc gat 1728
Arg Val Leu Tyr Pro Ala Leu Gly Pro Asp Gly Ser Ala Phe Phe Asp
                565                 570                 575

atg gta cct cgc tct gca gag ccc ggc tac cta gtg act aag gtg gta     1776
Met Val Pro Arg Ser Ala Glu Pro Gly Tyr Leu Val Thr Lys Val Val
            580                 585                 590

gcg gtg gac gcc gac tcg gga cac aac gcc tgg ctg tcc tac cac gtg     1824
Ala Val Asp Ala Asp Ser Gly His Asn Ala Trp Leu Ser Tyr His Val
        595                 600                 605

ctg cag gcc agt gag ccc gga ctc ttc agc ctg ggg ctg cgc act ggg     1872
Leu Gln Ala Ser Glu Pro Gly Leu Phe Ser Leu Gly Leu Arg Thr Gly
    610                 615                 620

gag gtg cgc acg gct cga gcc tta ggc gac agg gac gca gcc cgc cag     1920
Glu Val Arg Thr Ala Arg Ala Leu Gly Asp Arg Asp Ala Ala Arg Gln
625                 630                 635                 640

cgc ctg ctg gtc gct gtg cgt gac ggt gga cag ccg cca ctc tct gcc     1968
Arg Leu Leu Val Ala Val Arg Asp Gly Gly Gln Pro Pro Leu Ser Ala
                645                 650                 655

acc gcc acg ctt cat ctg gtc ttc gca gac aac ttg caa gag ata ctg     2016
Thr Ala Thr Leu His Leu Val Phe Ala Asp Asn Leu Gln Glu Ile Leu
            660                 665                 670

cca gac ctc agc gac cgc cct gta ctc tct gac ccc cag gct gaa ctg     2064
Pro Asp Leu Ser Asp Arg Pro Val Leu Ser Asp Pro Gln Ala Glu Leu
        675                 680                 685

cag ttt tac ctg gtg gtg gcc ttg gcc tta atc tca gtg ctc ttc ctc     2112
Gln Phe Tyr Leu Val Val Ala Leu Ala Leu Ile Ser Val Leu Phe Leu
    690                 695                 700

ctc gcc gtg att ctg gcc att gcc ttg cgc ctg cga cgc tct ctc agc     2160
Leu Ala Val Ile Leu Ala Ile Ala Leu Arg Leu Arg Arg Ser Leu Ser
705                 710                 715                 720

cct gct act tgg gac tgc ttc cat cct ggt ctc tgt gtc aag tct gga     2208
Pro Ala Thr Trp Asp Cys Phe His Pro Gly Leu Cys Val Lys Ser Gly
                725                 730                 735

cct gta gtt ccc ccc aac tac agt gag ggg act ttg cct tat tct tat     2256
Pro Val Val Pro Pro Asn Tyr Ser Glu Gly Thr Leu Pro Tyr Ser Tyr
            740                 745                 750

aat ctg tgc att gca cat acg ggt aca aaa gag ttt aat ttc cta aaa     2304
Asn Leu Cys Ile Ala His Thr Gly Thr Lys Glu Phe Asn Phe Leu Lys
        755                 760                 765

tgc agt gtg ccc cta cat tcc aat gaa gac atg gtt tgc agt gtt tct     2352
Cys Ser Val Pro Leu His Ser Asn Glu Asp Met Val Cys Ser Val Ser
    770                 775                 780

cct gga gcc tta att cca cct cat ggt ggg gag gat ttg act tca cat     2400
Pro Gly Ala Leu Ile Pro Pro His Gly Gly Glu Asp Leu Thr Ser His
785                 790                 795                 800

cct gag act ctg act tcg gtg agt ttc tct ttt ttg tgt gtg att tat     2448
Pro Glu Thr Leu Thr Ser Val Ser Phe Ser Phe Leu Cys Val Ile Tyr
                805                 810                 815

cta ata gtc tac tag                                                 2463
Leu Ile Val Tyr
            820

<210>  114
<211>  820
<212>  PRT

<213>  human

<400>  114

Met Gly Gly Ser Cys Ala Gln Arg Arg Arg Ala Gly Pro Arg Gln Val
1               5                   10                  15

Leu Phe Pro Leu Leu Leu Pro Leu Phe Tyr Pro Thr Leu Ser Glu Pro
            20                  25                  30

Ile Arg Tyr Ser Ile Pro Glu Glu Leu Ala Lys Gly Ser Val Val Gly
        35                  40                  45

Asn Leu Ala Lys Asp Leu Gly Leu Ser Val Leu Asp Val Ser Ala Arg
    50                  55                  60

Lys Leu Arg Val Ser Ala Glu Lys Leu His Phe Ser Val Asp Ala Glu
65                  70                  75                  80

Ser Gly Asp Leu Leu Val Lys Asn Arg Ile Asp Arg Glu Gln Ile Cys
                85                  90                  95

Lys Glu Arg Arg Arg Cys Glu Leu Gln Leu Glu Ala Val Val Glu Asn
            100                 105                 110

Pro Leu Asn Ile Phe His Val Ile Val Val Ile Glu Asp Val Asn Asp
        115                 120                 125

His Ala Pro Gln Phe Asp Lys Lys Glu Ile His Leu Glu Ile Phe Glu
    130                 135                 140

Ser Ala Ser Ala Gly Thr Arg Leu Ser Leu Asp Pro Ala Thr Asp Pro
145                 150                 155                 160

Asp Ile Asn Ile Asn Ser Ile Lys Asp Tyr Lys Ile Asn Ser Asn Pro
                165                 170                 175

Tyr Phe Ser Leu Met Val Arg Val Asn Ser Asp Gly Gly Lys Tyr Pro
            180                 185                 190

Glu Leu Ser Leu Glu Lys Leu Leu Asp Arg Glu Glu Gln Arg Ser His
        195                 200                 205

Ser Leu Ile Leu Thr Ala Leu Asp Gly Gly Asp Pro Pro Arg Ser Ala
    210                 215                 220

Thr Ala His Ile Glu Ile Ser Val Lys Asp Thr Asn Asp Asn Pro Pro
225                 230                 235                 240

Val Phe Ser Arg Asp Glu Tyr Arg Ile Ser Leu Ser Glu Asn Leu Pro
                245                 250                 255

Pro Gly Ser Pro Val Leu Gln Val Thr Ala Thr Asp Gln Asp Glu Gly
            260                 265                 270

Val Asn Ala Glu Ile Asn Tyr Tyr Phe Arg Ser Thr Ala Gln Ser Thr
        275                 280                 285

Lys His Met Phe Ser Leu Asp Glu Lys Thr Gly Met Ile Lys Asn Asn
    290                 295                 300

Gln Ser Phe Asp Phe Glu Asp Val Glu Arg Tyr Thr Met Glu Val Glu
305                 310                 315                 320

Ala Lys Asp Gly Gly Gly Leu Ser Thr Gln Cys Lys Val Ile Ile Glu
                325                 330                 335

Ile Leu Asp Glu Asn Asp Asn Ser Pro Glu Ile Ile Ile Thr Ser Leu
            340                 345                 350

Ser Asp Gln Ile Leu Glu Asn Ser Pro Pro Gly Met Val Val Ala Leu
        355                 360                 365

Phe Lys Thr Arg Asp Leu Asp Phe Gly Gly Asn Gly Glu Val Arg Cys
    370                 375                 380

Asn Ile Glu Thr Asp Ile Pro Phe Lys Ile Tyr Ser Ser Ser Asn Asn
385                 390                 395                 400

Tyr Tyr Lys Leu Val Thr Asp Gly Ala Leu Asp Arg Glu Gln Thr Pro
                405                 410                 415

Glu Tyr Asn Val Thr Ile Val Ala Thr Asp Arg Gly Lys Pro Pro Leu
            420                 425                 430

Ser Ser Ser Arg Ser Ile Thr Leu Tyr Val Ala Asp Ile Asn Asp Asn
        435                 440                 445

Ala Pro Val Phe Asp Gln Thr Ser Tyr Val Val His Val Ala Glu Asn
    450                 455                 460

Asn Pro Pro Gly Ala Ser Ile Ala Gln Val Ser Ala Ser Asp Pro Asp
465                 470                 475                 480

Leu Gly Leu Asn Gly His Ile Ser Tyr Ser Ile Val Ala Ser Asp Leu
                485                 490                 495

Glu Pro Leu Ala Val Ser Ser Tyr Val Ser Val Ser Ala Gln Ser Gly
            500                 505                 510

Val Val Phe Ala Gln Arg Ala Phe Asp His Glu Gln Leu Arg Ala Phe
        515                 520                 525

Ala Leu Thr Leu Gln Ala Arg Asp His Gly Ser Pro Thr Leu Ser Ala
    530                 535                 540

Asn Val Ser Leu Arg Val Leu Val Gly Asp Arg Asn Asp Asn Ala Pro
545                 550                 555                 560

Arg Val Leu Tyr Pro Ala Leu Gly Pro Asp Gly Ser Ala Phe Phe Asp
                565                 570                 575

Met Val Pro Arg Ser Ala Glu Pro Gly Tyr Leu Val Thr Lys Val Val
            580                 585                 590

Ala Val Asp Ala Asp Ser Gly His Asn Ala Trp Leu Ser Tyr His Val
        595                 600                 605

Leu Gln Ala Ser Glu Pro Gly Leu Phe Ser Leu Gly Leu Arg Thr Gly
    610                 615                 620

Glu Val Arg Thr Ala Arg Ala Leu Gly Asp Arg Asp Ala Ala Arg Gln
625                 630                 635                 640

Arg Leu Leu Val Ala Val Arg Asp Gly Gly Gln Pro Pro Leu Ser Ala
                645                 650                 655

Thr Ala Thr Leu His Leu Val Phe Ala Asp Asn Leu Gln Glu Ile Leu
            660                 665                 670

Pro Asp Leu Ser Asp Arg Pro Val Leu Ser Asp Pro Gln Ala Glu Leu
        675                 680                 685

Gln Phe Tyr Leu Val Val Ala Leu Ala Leu Ile Ser Val Leu Phe Leu
    690                 695                 700

Leu Ala Val Ile Leu Ala Ile Ala Leu Arg Leu Arg Arg Ser Leu Ser
705                 710                 715                 720

Pro Ala Thr Trp Asp Cys Phe His Pro Gly Leu Cys Val Lys Ser Gly
                725                 730                 735

Pro Val Val Pro Pro Asn Tyr Ser Glu Gly Thr Leu Pro Tyr Ser Tyr
            740                 745                 750

Asn Leu Cys Ile Ala His Thr Gly Thr Lys Glu Phe Asn Phe Leu Lys
        755                 760                 765

Cys Ser Val Pro Leu His Ser Asn Glu Asp Met Val Cys Ser Val Ser
    770                 775                 780

Pro Gly Ala Leu Ile Pro Pro His Gly Gly Glu Asp Leu Thr Ser His
785                 790                 795                 800

Pro Glu Thr Leu Thr Ser Val Ser Phe Ser Phe Leu Cys Val Ile Tyr
                805                 810                 815

Leu Ile Val Tyr
            820

<210>  115
<211>  3427
<212>  DNA
<213>  human

<220>
<221>  CDS
<222>  (1)..(2976)

<400>  115
atg agt gca gtg ggc ttg gta ctc ctg gtg ctt gct ctg agg ctt agg      48
Met Ser Ala Val Gly Leu Val Leu Leu Val Leu Ala Leu Arg Leu Arg
1               5                   10                  15

gcc acc act gtt aag cca gag gaa ggc agc ttt tgc tct aac agc cag      96
Ala Thr Thr Val Lys Pro Glu Glu Gly Ser Phe Cys Ser Asn Ser Gln
            20                  25                  30

gtg gcc ttc aga gat gct tgc tat gaa ttt gtg cca ctc gga cgc acc     144
Val Ala Phe Arg Asp Ala Cys Tyr Glu Phe Val Pro Leu Gly Arg Thr
        35                  40                  45

ttc cgt gat gcc cag agc tgg tgc gag ggg caa gga ggc cat ttg gtc     192
Phe Arg Asp Ala Gln Ser Trp Cys Glu Gly Gln Gly Gly His Leu Val
    50                  55                  60

ttc att cag gac gaa ggc acc cag tgg ttt ctg cag aag cac atc tcc     240
Phe Ile Gln Asp Glu Gly Thr Gln Trp Phe Leu Gln Lys His Ile Ser
65                  70                  75                  80

cag gac agg gaa tgg tgg att gga ctc acg tgg aac ttg gca cgg aat     288
Gln Asp Arg Glu Trp Trp Ile Gly Leu Thr Trp Asn Leu Ala Arg Asn
                85                  90                  95

gga acc acg gag ggg ccg ggg act tgg ttg gac acc tcc aat gtg acc     336
Gly Thr Thr Glu Gly Pro Gly Thr Trp Leu Asp Thr Ser Asn Val Thr
            100                 105                 110

tac agc aac tgg cat gga ggg cag gcc gcc gct gcc cct gac acc tgc     384
Tyr Ser Asn Trp His Gly Gly Gln Ala Ala Ala Ala Pro Asp Thr Cys
        115                 120                 125

ggc cac atc ggg aga ggc cct tcc tct gag tgg gtg acc tcg gac tgc     432
Gly His Ile Gly Arg Gly Pro Ser Ser Glu Trp Val Thr Ser Asp Cys
    130                 135                 140

gcc cag acc ttc gcc ttc atg tgc gag ttc cgg gtt ggc cag agc ctg     480
Ala Gln Thr Phe Ala Phe Met Cys Glu Phe Arg Val Gly Gln Ser Leu
145                 150                 155                 160

gcc tgc gag gga ctc aat gcc acc gtg cac tgt ggc ttg ggg cag gtc     528
Ala Cys Glu Gly Leu Asn Ala Thr Val His Cys Gly Leu Gly Gln Val
                165                 170                 175

atc cag gtc cag gat gcc gtc tac ggg cgc cag aat ccc cat ttc tgc     576
Ile Gln Val Gln Asp Ala Val Tyr Gly Arg Gln Asn Pro His Phe Cys
            180                 185                 190

acc cag gat gcc ggg cgt cct tca gat ctg gag cag gga tgc agc tgg     624

Thr Gln Asp Ala Gly Arg Pro Ser Asp Leu Glu Gln Gly Cys Ser Trp
        195                 200                 205

gcc aac gtc aag gag gag gtg gca ggc cag tgc cag gag cta cag tca     672
Ala Asn Val Lys Glu Glu Val Ala Gly Gln Cys Gln Glu Leu Gln Ser
    210                 215                 220

tgc cag gtg gca gca gat gag acc tac ttt gga aac ctg tgt ccg act     720
Cys Gln Val Ala Ala Asp Glu Thr Tyr Phe Gly Asn Leu Cys Pro Thr
225                 230                 235                 240

cag ggc agt tac ctg tgg gtg cag tac cag tgc cgg gaa gcc ctg cag     768
Gln Gly Ser Tyr Leu Trp Val Gln Tyr Gln Cys Arg Glu Ala Leu Gln
                245                 250                 255

ctg atg gtg tcc agt gag agt ttc atc ttt gac aat gtc acc atc tcc     816
Leu Met Val Ser Ser Glu Ser Phe Ile Phe Asp Asn Val Thr Ile Ser
            260                 265                 270

ctg acg tgg ctc ctc tca ccc tac ata gga aac ctg tcc tgt ata att     864
Leu Thr Trp Leu Leu Ser Pro Tyr Ile Gly Asn Leu Ser Cys Ile Ile
        275                 280                 285

agt aca gga gac agc cac act ttt gat ccc tac aac ccg ccg agt gtg     912
Ser Thr Gly Asp Ser His Thr Phe Asp Pro Tyr Asn Pro Pro Ser Val
    290                 295                 300

tcc agc aat gtg acc cac cag ttc aca tct cct ggg gaa ttc acc gtg     960
Ser Ser Asn Val Thr His Gln Phe Thr Ser Pro Gly Glu Phe Thr Val
305                 310                 315                 320

ttt gct gaa tgc aca acc agt gag tgg cat gtg aca gct cag agg cag     1008
Phe Ala Glu Cys Thr Thr Ser Glu Trp His Val Thr Ala Gln Arg Gln
                325                 330                 335

gtg acc gtg cga gac aag atg gag acg ctc agt gtg act gca tgc tcc     1056
Val Thr Val Arg Asp Lys Met Glu Thr Leu Ser Val Thr Ala Cys Ser
            340                 345                 350

ggc ctg tcc cag tca gga gcc ggc cct ctc tgc cag gct gtc ttt ggg     1104
Gly Leu Ser Gln Ser Gly Ala Gly Pro Leu Cys Gln Ala Val Phe Gly
        355                 360                 365

gat cct ctg tgg att cag gtg gag ctt gat gga gga aca gga gtg act     1152
Asp Pro Leu Trp Ile Gln Val Glu Leu Asp Gly Gly Thr Gly Val Thr
    370                 375                 380

tac act gtg ctt ttg ggt gac ata acc ctg gcc gag tcc acc acc caa     1200
Tyr Thr Val Leu Leu Gly Asp Ile Thr Leu Ala Glu Ser Thr Thr Gln
385                 390                 395                 400

aag ggc tcg cta ccg tac aat ctg atc ctg gac aga gaa acc cag aaa     1248
Lys Gly Ser Leu Pro Tyr Asn Leu Ile Leu Asp Arg Glu Thr Gln Lys
                405                 410                 415

ctg atg ggc cct ggg agg cac cgc ctg gag atc caa gcc acc ggc aac     1296
Leu Met Gly Pro Gly Arg His Arg Leu Glu Ile Gln Ala Thr Gly Asn
            420                 425                 430

acc acc acc tct aca atc tcc aga aac att aca gtc cac ttg gtg gag     1344
Thr Thr Thr Ser Thr Ile Ser Arg Asn Ile Thr Val His Leu Val Glu
        435                 440                 445

ctg ctg tca ggg ctg cag gcc tcc tgg gct tct gac cat ttg gag ctt     1392
Leu Leu Ser Gly Leu Gln Ala Ser Trp Ala Ser Asp His Leu Glu Leu
    450                 455                 460

gga cag gac cta ttg atc acc atc tca ttg gct cag ggc acc ccg gaa     1440

Gly Gln Asp Leu Leu Ile Thr Ile Ser Leu Ala Gln Gly Thr Pro Glu
465                 470                 475                 480

gag ctg acc ttt gag gtg gct gga ctc aat gca acc ttc tcc cac gag      1488
Glu Leu Thr Phe Glu Val Ala Gly Leu Asn Ala Thr Phe Ser His Glu
                485                 490                 495

caa gtg agc ttt gga gag cca ttt ggg att tgc cgc ctg gct gtt ccg      1536
Gln Val Ser Phe Gly Glu Pro Phe Gly Ile Cys Arg Leu Ala Val Pro
            500                 505                 510

gtt gaa ggc acc ttt ctg gtc acc atg ctg gtg agg aat gcc ttc tcc      1584
Val Glu Gly Thr Phe Leu Val Thr Met Leu Val Arg Asn Ala Phe Ser
        515                 520                 525

aac ctg agt ttg gaa atc ggg aac atc act atc aca gcc cct tcc ggt      1632
Asn Leu Ser Leu Glu Ile Gly Asn Ile Thr Ile Thr Ala Pro Ser Gly
    530                 535                 540

ctc caa gaa cca tct ggg atg aat gct gaa gga aag agt aaa gat aaa      1680
Leu Gln Glu Pro Ser Gly Met Asn Ala Glu Gly Lys Ser Lys Asp Lys
545                 550                 555                 560

ggc gat atg gag gtg tac atc caa cct ggt cca tat gtg gat cct ttc      1728
Gly Asp Met Glu Val Tyr Ile Gln Pro Gly Pro Tyr Val Asp Pro Phe
                565                 570                 575

acg aca gtg acc ctg ggc tgg cca gac aat gac aag gag tta cgc ttc      1776
Thr Thr Val Thr Leu Gly Trp Pro Asp Asn Asp Lys Glu Leu Arg Phe
            580                 585                 590

caa tgg tca tgt ggg tct tgc tgg gct ctg tgg agc agc tgt gtt gag      1824
Gln Trp Ser Cys Gly Ser Cys Trp Ala Leu Trp Ser Ser Cys Val Glu
        595                 600                 605

agg cag ctg ctt cgc aca gac cag agg gag ctg gtg gtt cca gca tcc      1872
Arg Gln Leu Leu Arg Thr Asp Gln Arg Glu Leu Val Val Pro Ala Ser
    610                 615                 620

tgc ctg ccg ccg cct gac tct gct gtc acc ctg cgc ctg gct gtt ctg      1920
Cys Leu Pro Pro Pro Asp Ser Ala Val Thr Leu Arg Leu Ala Val Leu
625                 630                 635                 640

aga ggc caa gag ctg gag aac agg gca gag cag tgc ctc tac gtg tct      1968
Arg Gly Gln Glu Leu Glu Asn Arg Ala Glu Gln Cys Leu Tyr Val Ser
                645                 650                 655

gcg ccc tgg gaa ctc agg cct cga gtc agc tgt gag agg aac tgc agg      2016
Ala Pro Trp Glu Leu Arg Pro Arg Val Ser Cys Glu Arg Asn Cys Arg
            660                 665                 670

cca gtt aat gcc agc aaa gac att ctg ctc agg gtc acc atg ggg gag      2064
Pro Val Asn Ala Ser Lys Asp Ile Leu Leu Arg Val Thr Met Gly Glu
        675                 680                 685

gac tct cca gtg gct atg ttc agc tgg tat ttg gac aac acc cca aca      2112
Asp Ser Pro Val Ala Met Phe Ser Trp Tyr Leu Asp Asn Thr Pro Thr
    690                 695                 700

gag cag gct gag ccc ctc ctg gat gcc tgc aga ctc aga gga ttt tgg      2160
Glu Gln Ala Glu Pro Leu Leu Asp Ala Cys Arg Leu Arg Gly Phe Trp
705                 710                 715                 720

cca agg tcc tta acc ctc ctc cag agc aac acc tcc acg ttg ctg ttg      2208
Pro Arg Ser Leu Thr Leu Leu Gln Ser Asn Thr Ser Thr Leu Leu Leu
                725                 730                 735

aac agc tcg ttt ctg cag tcc cgg gga gag gtc atc cga atc aga gcc      2256

Asn Ser Ser Phe Leu Gln Ser Arg Gly Glu Val Ile Arg Ile Arg Ala
            740                 745                 750

aca gca ctg acc agg cat gcc tat ggg gag gac acc tat gtg atc agc     2304
Thr Ala Leu Thr Arg His Ala Tyr Gly Glu Asp Thr Tyr Val Ile Ser
        755                 760                 765

act gtg cct ccc cgt gag gtg cct gcc tgc act att gcc cca gag gag     2352
Thr Val Pro Pro Arg Glu Val Pro Ala Cys Thr Ile Ala Pro Glu Glu
    770                 775                 780

ggc acc gtt ctg acg agc ttt gcc atc ttc tgc aac gcc tcc aca gcc     2400
Gly Thr Val Leu Thr Ser Phe Ala Ile Phe Cys Asn Ala Ser Thr Ala
785                 790                 795                 800

ctg gga ccc ctg gag ttc tgc ttc tgt ctg gaa tca ggt tcc tgc cta     2448
Leu Gly Pro Leu Glu Phe Cys Phe Cys Leu Glu Ser Gly Ser Cys Leu
                805                 810                 815

cac tgt ggc cct gaa cct gcc ctc cca tca gtg tat ctg cca ctt gga     2496
His Cys Gly Pro Glu Pro Ala Leu Pro Ser Val Tyr Leu Pro Leu Gly
            820                 825                 830

gag gag aac aat gac ttt gtg ctg aca gta gtt att tct gcc acc aat     2544
Glu Glu Asn Asn Asp Phe Val Leu Thr Val Val Ile Ser Ala Thr Asn
        835                 840                 845

cgt gca ggg gac acg cag cag acc cag gcc atg gct aag gtg gca ctc     2592
Arg Ala Gly Asp Thr Gln Gln Thr Gln Ala Met Ala Lys Val Ala Leu
    850                 855                 860

gga gac aca tgt gtt gag gat gta gca ttc cag gct gcc gtg tca gag     2640
Gly Asp Thr Cys Val Glu Asp Val Ala Phe Gln Ala Ala Val Ser Glu
865                 870                 875                 880

aaa atc ccc aca gct ctg caa ggc gag ggt ggc ccc gag cag ctc ctc     2688
Lys Ile Pro Thr Ala Leu Gln Gly Glu Gly Gly Pro Glu Gln Leu Leu
                885                 890                 895

cag ctg gcc aag gct gtg tcc tcc atg ctg aac caa gag cat gaa agc     2736
Gln Leu Ala Lys Ala Val Ser Ser Met Leu Asn Gln Glu His Glu Ser
            900                 905                 910

cag ggc tca gga cag tca ctg agc ata gac gtc aga cag aag gtc aga     2784
Gln Gly Ser Gly Gln Ser Leu Ser Ile Asp Val Arg Gln Lys Val Arg
        915                 920                 925

gag cat gtg ctg gga tca ctg tct gca gtc acc acc ggc ttg gag gac     2832
Glu His Val Leu Gly Ser Leu Ser Ala Val Thr Thr Gly Leu Glu Asp
    930                 935                 940

gtg cag agg gtg cag gag ctg gcc gag gtg ctg aga gag gtg acc tgc     2880
Val Gln Arg Val Gln Glu Leu Ala Glu Val Leu Arg Glu Val Thr Cys
945                 950                 955                 960

cgg agt aag gaa ctc aca ccc tcg gcc cag ggg tcc tgc atg ggc gat     2928
Arg Ser Lys Glu Leu Thr Pro Ser Ala Gln Gly Ser Cys Met Gly Asp
                965                 970                 975

tca tgg gaa ggt gcc cct cct gct gcc cat gta tct cac gct agg tga     2976
Ser Trp Glu Gly Ala Pro Pro Ala Ala His Val Ser His Ala Arg
            980                 985                 990

gagggcctgt ttgcccagac tctcactcct gcatctgctg gtgagcaagt tgagggagta     3036

actgaatctc attaatattt gggtggccaa atgtgagtcc agacactgct actgactgcc     3096

catgttctca acttcagtac atgccagcct gacatctggc tgccagctcc tgtgcttcct     3156

tatccttctg ggggtcttct ctgaccctca gagcctactg tacctgccca tatggctgtg 3216
aattcatgca gtgttcagtg actaatgaag ctggcttata aacaccccag ctacctcacc 3276
cctcttgatg gataatccca agcaggagtg aatcccaggt aatgggcttg atcacactcc 3336
tgtactggct tccacccttc cctgtctcac accccctact ccccacaagt gtttcttggg 3396
atcatgtcca aataaatgac ttgctctcaa a 3427

<210> 116
<211> 991
<212> PRT
<213> human

<400> 116

Met Ser Ala Val Gly Leu Val Leu Leu Val Leu Ala Leu Arg Leu Arg
1               5                   10                  15

Ala Thr Thr Val Lys Pro Glu Glu Gly Ser Phe Cys Ser Asn Ser Gln
            20                  25                  30

Val Ala Phe Arg Asp Ala Cys Tyr Glu Phe Val Pro Leu Gly Arg Thr
        35                  40                  45

Phe Arg Asp Ala Gln Ser Trp Cys Glu Gly Gln Gly Gly His Leu Val
    50                  55                  60

Phe Ile Gln Asp Glu Gly Thr Gln Trp Phe Leu Gln Lys His Ile Ser
65                  70                  75                  80

Gln Asp Arg Glu Trp Trp Ile Gly Leu Thr Trp Asn Leu Ala Arg Asn
                85                  90                  95

Gly Thr Thr Glu Gly Pro Gly Thr Trp Leu Asp Thr Ser Asn Val Thr
            100                 105                 110

Tyr Ser Asn Trp His Gly Gly Gln Ala Ala Ala Ala Pro Asp Thr Cys
        115                 120                 125

Gly His Ile Gly Arg Gly Pro Ser Ser Glu Trp Val Thr Ser Asp Cys
    130                 135                 140

Ala Gln Thr Phe Ala Phe Met Cys Glu Phe Arg Val Gly Gln Ser Leu
145                 150                 155                 160

Ala Cys Glu Gly Leu Asn Ala Thr Val His Cys Gly Leu Gly Gln Val
                165                 170                 175

Ile Gln Val Gln Asp Ala Val Tyr Gly Arg Gln Asn Pro His Phe Cys
            180                 185                 190

Thr Gln Asp Ala Gly Arg Pro Ser Asp Leu Glu Gln Gly Cys Ser Trp

        195                 200                 205

Ala Asn Val Lys Glu Glu Val Ala Gly Gln Cys Gln Glu Leu Gln Ser
    210                 215                 220

Cys Gln Val Ala Ala Asp Glu Thr Tyr Phe Gly Asn Leu Cys Pro Thr
225                 230                 235                 240

Gln Gly Ser Tyr Leu Trp Val Gln Tyr Gln Cys Arg Glu Ala Leu Gln
                245                 250                 255

Leu Met Val Ser Ser Glu Ser Phe Ile Phe Asp Asn Val Thr Ile Ser
            260                 265                 270

Leu Thr Trp Leu Leu Ser Pro Tyr Ile Gly Asn Leu Ser Cys Ile Ile
        275                 280                 285

Ser Thr Gly Asp Ser His Thr Phe Asp Pro Tyr Asn Pro Pro Ser Val
    290                 295                 300

Ser Ser Asn Val Thr His Gln Phe Thr Ser Pro Gly Glu Phe Thr Val
305                 310                 315                 320

Phe Ala Glu Cys Thr Thr Ser Glu Trp His Val Thr Ala Gln Arg Gln
                325                 330                 335

Val Thr Val Arg Asp Lys Met Glu Thr Leu Ser Val Thr Ala Cys Ser
            340                 345                 350

Gly Leu Ser Gln Ser Gly Ala Gly Pro Leu Cys Gln Ala Val Phe Gly
        355                 360                 365

Asp Pro Leu Trp Ile Gln Val Glu Leu Asp Gly Gly Thr Gly Val Thr
    370                 375                 380

Tyr Thr Val Leu Leu Gly Asp Ile Thr Leu Ala Glu Ser Thr Thr Gln
385                 390                 395                 400

Lys Gly Ser Leu Pro Tyr Asn Leu Ile Leu Asp Arg Glu Thr Gln Lys
                405                 410                 415

Leu Met Gly Pro Gly Arg His Arg Leu Glu Ile Gln Ala Thr Gly Asn
            420                 425                 430

Thr Thr Thr Ser Thr Ile Ser Arg Asn Ile Thr Val His Leu Val Glu
        435                 440                 445

Leu Leu Ser Gly Leu Gln Ala Ser Trp Ala Ser Asp His Leu Glu Leu
    450                 455                 460

Gly Gln Asp Leu Leu Ile Thr Ile Ser Leu Ala Gln Gly Thr Pro Glu

465                 470                 475                 480

Glu Leu Thr Phe Glu Val Ala Gly Leu Asn Ala Thr Phe Ser His Glu
                485                 490                 495

Gln Val Ser Phe Gly Glu Pro Phe Gly Ile Cys Arg Leu Ala Val Pro
            500                 505                 510

Val Glu Gly Thr Phe Leu Val Thr Met Leu Val Arg Asn Ala Phe Ser
        515                 520                 525

Asn Leu Ser Leu Glu Ile Gly Asn Ile Thr Ile Thr Ala Pro Ser Gly
    530                 535                 540

Leu Gln Glu Pro Ser Gly Met Asn Ala Glu Gly Lys Ser Lys Asp Lys
545                 550                 555                 560

Gly Asp Met Glu Val Tyr Ile Gln Pro Gly Pro Tyr Val Asp Pro Phe
                565                 570                 575

Thr Thr Val Thr Leu Gly Trp Pro Asp Asn Asp Lys Glu Leu Arg Phe
            580                 585                 590

Gln Trp Ser Cys Gly Ser Cys Trp Ala Leu Trp Ser Ser Cys Val Glu
        595                 600                 605

Arg Gln Leu Leu Arg Thr Asp Gln Arg Glu Leu Val Val Pro Ala Ser
    610                 615                 620

Cys Leu Pro Pro Pro Asp Ser Ala Val Thr Leu Arg Leu Ala Val Leu
625                 630                 635                 640

Arg Gly Gln Glu Leu Glu Asn Arg Ala Glu Gln Cys Leu Tyr Val Ser
                645                 650                 655

Ala Pro Trp Glu Leu Arg Pro Arg Val Ser Cys Glu Arg Asn Cys Arg
            660                 665                 670

Pro Val Asn Ala Ser Lys Asp Ile Leu Leu Arg Val Thr Met Gly Glu
        675                 680                 685

Asp Ser Pro Val Ala Met Phe Ser Trp Tyr Leu Asp Asn Thr Pro Thr
    690                 695                 700

Glu Gln Ala Glu Pro Leu Leu Asp Ala Cys Arg Leu Arg Gly Phe Trp
705                 710                 715                 720

Pro Arg Ser Leu Thr Leu Leu Gln Ser Asn Thr Ser Thr Leu Leu Leu
                725                 730                 735

Asn Ser Ser Phe Leu Gln Ser Arg Gly Glu Val Ile Arg Ile Arg Ala

            740                 745                 750

Thr Ala Leu Thr Arg His Ala Tyr Gly Glu Asp Thr Tyr Val Ile Ser
        755                 760                 765

Thr Val Pro Pro Arg Glu Val Pro Ala Cys Thr Ile Ala Pro Glu Glu
    770                 775                 780

Gly Thr Val Leu Thr Ser Phe Ala Ile Phe Cys Asn Ala Ser Thr Ala
785                 790                 795                 800

Leu Gly Pro Leu Glu Phe Cys Phe Cys Leu Glu Ser Gly Ser Cys Leu
                805                 810                 815

His Cys Gly Pro Glu Pro Ala Leu Pro Ser Val Tyr Leu Pro Leu Gly
            820                 825                 830

Glu Glu Asn Asn Asp Phe Val Leu Thr Val Val Ile Ser Ala Thr Asn
        835                 840                 845

Arg Ala Gly Asp Thr Gln Gln Thr Gln Ala Met Ala Lys Val Ala Leu
    850                 855                 860

Gly Asp Thr Cys Val Glu Asp Val Ala Phe Gln Ala Ala Val Ser Glu
865                 870                 875                 880

Lys Ile Pro Thr Ala Leu Gln Gly Glu Gly Gly Pro Glu Gln Leu Leu
                885                 890                 895

Gln Leu Ala Lys Ala Val Ser Ser Met Leu Asn Gln Glu His Glu Ser
            900                 905                 910

Gln Gly Ser Gly Gln Ser Leu Ser Ile Asp Val Arg Gln Lys Val Arg
        915                 920                 925

Glu His Val Leu Gly Ser Leu Ser Ala Val Thr Thr Gly Leu Glu Asp
    930                 935                 940

Val Gln Arg Val Gln Glu Leu Ala Glu Val Leu Arg Glu Val Thr Cys
945                 950                 955                 960

Arg Ser Lys Glu Leu Thr Pro Ser Ala Gln Gly Ser Cys Met Gly Asp
                965                 970                 975

Ser Trp Glu Gly Ala Pro Pro Ala Ala His Val Ser His Ala Arg
            980                 985                 990

<210> 117
<211> 7624
<212> DNA
<213> human

<220>
<221>  CDS
<222>  (1)..(7380)

<400>  117
atg agt gca gtg ggc ttg gta ctc ctg gtg ctt gct ctg agg ctt agg      48
Met Ser Ala Val Gly Leu Val Leu Leu Val Leu Ala Leu Arg Leu Arg
1               5                   10                  15

gcc acc act gtt aag cca gag gaa ggc agc ttt tgc tct aac agc cag      96
Ala Thr Thr Val Lys Pro Glu Glu Gly Ser Phe Cys Ser Asn Ser Gln
            20                  25                  30

gtg gcc ttc aga gat gct tgc tat gaa ttt gtg cca ctc gga cgc acc      144
Val Ala Phe Arg Asp Ala Cys Tyr Glu Phe Val Pro Leu Gly Arg Thr
        35                  40                  45

ttc cgt gat gcc cag agc tgg tgc gag ggg caa gga ggc cat ttg gtc      192
Phe Arg Asp Ala Gln Ser Trp Cys Glu Gly Gln Gly Gly His Leu Val
    50                  55                  60

ttc att cag gac gaa ggc acc cag tgg ttt ctg cag aag cac atc tcc      240
Phe Ile Gln Asp Glu Gly Thr Gln Trp Phe Leu Gln Lys His Ile Ser
65                  70                  75                  80

cag gac agg gaa tgg tgg att gga ctc acg tgg aac ttg gca cgg aat      288
Gln Asp Arg Glu Trp Trp Ile Gly Leu Thr Trp Asn Leu Ala Arg Asn
                85                  90                  95

gga acc acg gag ggg ccg ggg act tgg ttg gac acc tcc aat gtg acc      336
Gly Thr Thr Glu Gly Pro Gly Thr Trp Leu Asp Thr Ser Asn Val Thr
            100                 105                 110

tac agc aac tgg cat gga ggg cag gcc gcc gct gcc cct gac acc tgc      384
Tyr Ser Asn Trp His Gly Gly Gln Ala Ala Ala Ala Pro Asp Thr Cys
        115                 120                 125

ggc cac atc ggg aga ggc cct tcc tct gag tgg gtg acc tcg gac tgc      432
Gly His Ile Gly Arg Gly Pro Ser Ser Glu Trp Val Thr Ser Asp Cys
    130                 135                 140

gcc cag acc ttc gcc ttc atg tgc gag ttc cgg gtt ggc cag agc ctg      480
Ala Gln Thr Phe Ala Phe Met Cys Glu Phe Arg Val Gly Gln Ser Leu
145                 150                 155                 160

gcc tgc gag gga ctc aat gcc acc gtg cac tgt ggc ttg ggg cag gtc      528
Ala Cys Glu Gly Leu Asn Ala Thr Val His Cys Gly Leu Gly Gln Val
                165                 170                 175

atc cag gtc cag gat gcc gtc tac ggg cgc cag aat ccc cat ttc tgc      576
Ile Gln Val Gln Asp Ala Val Tyr Gly Arg Gln Asn Pro His Phe Cys
            180                 185                 190

acc cag gat gcc ggg cgt cct tca gat ctg gag cag gga tgc agc tgg      624
Thr Gln Asp Ala Gly Arg Pro Ser Asp Leu Glu Gln Gly Cys Ser Trp
        195                 200                 205

gcc aac gtc aag gag gag gtg gca ggc cag tgc cag gag cta cag tca      672
Ala Asn Val Lys Glu Glu Val Ala Gly Gln Cys Gln Glu Leu Gln Ser
    210                 215                 220

tgc cag gtg gca gca gat gag acc tac ttt gga aac ctg tgt ccg act      720
Cys Gln Val Ala Ala Asp Glu Thr Tyr Phe Gly Asn Leu Cys Pro Thr
225                 230                 235                 240

cag ggc agt tac ctg tgg gtg cag tac cag tgc cgg gaa gcc ctg cag      768
Gln Gly Ser Tyr Leu Trp Val Gln Tyr Gln Cys Arg Glu Ala Leu Gln

245                 250                 255

ctg atg gtg tcc agt gag agt ttc atc ttt gac aat gtc acc atc tcc     816
Leu Met Val Ser Ser Glu Ser Phe Ile Phe Asp Asn Val Thr Ile Ser
        260                 265                 270

ctg acg tgg ctc ctc tca ccc tac ata gga aac ctg tcc tgt ata att     864
Leu Thr Trp Leu Leu Ser Pro Tyr Ile Gly Asn Leu Ser Cys Ile Ile
    275                 280                 285

agt aca gga gac agc cac act ttt gat ccc tac aac ccg ccg agt gtg     912
Ser Thr Gly Asp Ser His Thr Phe Asp Pro Tyr Asn Pro Pro Ser Val
    290                 295                 300

tcc agc aat gtg acc cac cag ttc aca tct cct ggg gaa ttc acc gtg     960
Ser Ser Asn Val Thr His Gln Phe Thr Ser Pro Gly Glu Phe Thr Val
305                 310                 315                 320

ttt gct gaa tgc aca acc agt gag tgg cat gtg aca gct cag agg cag     1008
Phe Ala Glu Cys Thr Thr Ser Glu Trp His Val Thr Ala Gln Arg Gln
                325                 330                 335

gtg acc gtg cga gac aag atg gag acg ctc agt gtg act gca tgc tcc     1056
Val Thr Val Arg Asp Lys Met Glu Thr Leu Ser Val Thr Ala Cys Ser
        340                 345                 350

ggc ctg tcc cag tca gga gcc ggc cct ctc tgc cag gct gtc ttt ggg     1104
Gly Leu Ser Gln Ser Gly Ala Gly Pro Leu Cys Gln Ala Val Phe Gly
        355                 360                 365

gat cct ctg tgg att cag gtg gag ctt gat gga gga aca gga gtg act     1152
Asp Pro Leu Trp Ile Gln Val Glu Leu Asp Gly Gly Thr Gly Val Thr
    370                 375                 380

tac act gtg ctt ttg ggt gac ata acc ctg gcc gag tcc acc acc caa     1200
Tyr Thr Val Leu Leu Gly Asp Ile Thr Leu Ala Glu Ser Thr Thr Gln
385                 390                 395                 400

aag ggc tcg cta ccg tac aat ctg atc ctg gac aga gaa acc cag aaa     1248
Lys Gly Ser Leu Pro Tyr Asn Leu Ile Leu Asp Arg Glu Thr Gln Lys
                405                 410                 415

ctg atg ggc cct ggg agg cac cgc ctg gag atc caa gcc acc ggc aac     1296
Leu Met Gly Pro Gly Arg His Arg Leu Glu Ile Gln Ala Thr Gly Asn
        420                 425                 430

acc acc acc tct aca atc tcc aga aac att aca gtc cac ttg gtg gag     1344
Thr Thr Thr Ser Thr Ile Ser Arg Asn Ile Thr Val His Leu Val Glu
        435                 440                 445

ctg ctg tca ggg ctg cag gcc tcc tgg gct tct gac cat ttg gag ctt     1392
Leu Leu Ser Gly Leu Gln Ala Ser Trp Ala Ser Asp His Leu Glu Leu
    450                 455                 460

gga cag gac cta ttg atc acc atc tca ttg gct cag ggc acc ccg gaa     1440
Gly Gln Asp Leu Leu Ile Thr Ile Ser Leu Ala Gln Gly Thr Pro Glu
465                 470                 475                 480

gag ctg acc ttt gag gtg gct gga ctc aat gca acc ttc tcc cac gag     1488
Glu Leu Thr Phe Glu Val Ala Gly Leu Asn Ala Thr Phe Ser His Glu
                485                 490                 495

caa gtg agc ttt gga gag cca ttt ggg att tgc cgc ctg gct gtt ccg     1536
Gln Val Ser Phe Gly Glu Pro Phe Gly Ile Cys Arg Leu Ala Val Pro
        500                 505                 510

gtt gaa ggc acc ttt ctg gtc acc atg ctg gtg agg aat gcc ttc tcc     1584
Val Glu Gly Thr Phe Leu Val Thr Met Leu Val Arg Asn Ala Phe Ser

        515                 520                 525

aac ctg agt ttg gaa atc ggg aac atc act atc aca gcc cct tcc ggt 1632
Asn Leu Ser Leu Glu Ile Gly Asn Ile Thr Ile Thr Ala Pro Ser Gly
    530                 535                 540

ctc caa gaa cca tct ggg atg aat gct gaa gga aag agt aaa gat aaa 1680
Leu Gln Glu Pro Ser Gly Met Asn Ala Glu Gly Lys Ser Lys Asp Lys
545                 550                 555                 560

ggc gat atg gag gtg tac atc caa cct ggt cca tat gtg gat cct ttc 1728
Gly Asp Met Glu Val Tyr Ile Gln Pro Gly Pro Tyr Val Asp Pro Phe
                565                 570                 575

acg aca gtg acc ctg ggc tgg cca gac aat gac aag gag tta cgc ttc 1776
Thr Thr Val Thr Leu Gly Trp Pro Asp Asn Asp Lys Glu Leu Arg Phe
            580                 585                 590

caa tgg tca tgt ggg tct tgc tgg gct ctg tgg agc agc tgt gtt gag 1824
Gln Trp Ser Cys Gly Ser Cys Trp Ala Leu Trp Ser Ser Cys Val Glu
        595                 600                 605

agg cag ctg ctt cgc aca gac cag agg gag ctg gtg gtt cca gca tcc 1872
Arg Gln Leu Leu Arg Thr Asp Gln Arg Glu Leu Val Val Pro Ala Ser
    610                 615                 620

tgc ctg ccg ccg cct gac tct gct gtc acc ctg cgc ctg gct gtt ctg 1920
Cys Leu Pro Pro Pro Asp Ser Ala Val Thr Leu Arg Leu Ala Val Leu
625                 630                 635                 640

aga ggc caa gag ctg gag aac agg gca gag cag tgc ctc tac gtg tct 1968
Arg Gly Gln Glu Leu Glu Asn Arg Ala Glu Gln Cys Leu Tyr Val Ser
                645                 650                 655

gcg ccc tgg gaa ctc agg cct cga gtc agc tgt gag agg aac tgc agg 2016
Ala Pro Trp Glu Leu Arg Pro Arg Val Ser Cys Glu Arg Asn Cys Arg
            660                 665                 670

cca gtt aat gcc agc aaa gac att ctg ctc agg gtc acc atg ggg gag 2064
Pro Val Asn Ala Ser Lys Asp Ile Leu Leu Arg Val Thr Met Gly Glu
        675                 680                 685

gac tct cca gtg gct atg ttc agc tgg tat ttg gac aac acc cca aca 2112
Asp Ser Pro Val Ala Met Phe Ser Trp Tyr Leu Asp Asn Thr Pro Thr
    690                 695                 700

gag cag gct gag ccc ctc ctg gat gcc tgc aga ctc aga gga ttt tgg 2160
Glu Gln Ala Glu Pro Leu Leu Asp Ala Cys Arg Leu Arg Gly Phe Trp
705                 710                 715                 720

cca agg tcc tta acc ctc ctc cag agc aac acc tcc acg ttg ctg ttg 2208
Pro Arg Ser Leu Thr Leu Leu Gln Ser Asn Thr Ser Thr Leu Leu Leu
                725                 730                 735

aac agc tcg ttt ctg cag tcc cgg gga gag gtc atc cga atc aga gcc 2256
Asn Ser Ser Phe Leu Gln Ser Arg Gly Glu Val Ile Arg Ile Arg Ala
            740                 745                 750

aca gca ctg acc agg cat gcc tat ggg gag gac acc tat gtg atc agc 2304
Thr Ala Leu Thr Arg His Ala Tyr Gly Glu Asp Thr Tyr Val Ile Ser
        755                 760                 765

act gtg cct ccc cgt gag gtg cct gcc tgc act att gcc cca gag gag 2352
Thr Val Pro Pro Arg Glu Val Pro Ala Cys Thr Ile Ala Pro Glu Glu
    770                 775                 780

ggc acc gtt ctg acg agc ttt gcc atc ttc tgc aac gcc tcc aca gcc 2400
Gly Thr Val Leu Thr Ser Phe Ala Ile Phe Cys Asn Ala Ser Thr Ala

785                    790                    795                    800

ctg gga ccc ctg gag ttc tgc ttc tgt ctg gaa tca ggt tcc tgc cta     2448
Leu Gly Pro Leu Glu Phe Cys Phe Cys Leu Glu Ser Gly Ser Cys Leu
                805                    810                    815

cac tgt ggc cct gaa cct gcc ctc cca tca gtg tat ctg cca ctt gga     2496
His Cys Gly Pro Glu Pro Ala Leu Pro Ser Val Tyr Leu Pro Leu Gly
            820                    825                    830

gag gag aac aat gac ttt gtg ctg aca gta gtt att tct gcc acc aat     2544
Glu Glu Asn Asn Asp Phe Val Leu Thr Val Val Ile Ser Ala Thr Asn
        835                    840                    845

cgt gca ggg gac acg cag cag acc cag gcc atg gct aag gtg gca ctc     2592
Arg Ala Gly Asp Thr Gln Gln Thr Gln Ala Met Ala Lys Val Ala Leu
    850                    855                    860

gga gac aca tgt gtt gag gat gta gca ttc cag gct gcc gtg tca gag     2640
Gly Asp Thr Cys Val Glu Asp Val Ala Phe Gln Ala Ala Val Ser Glu
865                    870                    875                    880

aaa atc ccc aca gct ctg caa ggc gag ggt ggc ccc gag cag ctc ctc     2688
Lys Ile Pro Thr Ala Leu Gln Gly Glu Gly Gly Pro Glu Gln Leu Leu
                885                    890                    895

cag ctg gcc aag gct gtg tcc tcc atg ctg aac caa gag cat gaa agc     2736
Gln Leu Ala Lys Ala Val Ser Ser Met Leu Asn Gln Glu His Glu Ser
            900                    905                    910

cag ggc tca gga cag tca ctg agc ata gac gtc aga cag aag gtc aga     2784
Gln Gly Ser Gly Gln Ser Leu Ser Ile Asp Val Arg Gln Lys Val Arg
        915                    920                    925

gag cat gtg ctg gga tca ctg tct gca gtc acc acc ggc ttg gag gac     2832
Glu His Val Leu Gly Ser Leu Ser Ala Val Thr Thr Gly Leu Glu Asp
    930                    935                    940

gtg cag agg gtg cag gag ctg gcc gag gtg ctg aga gag gtg acc tgc     2880
Val Gln Arg Val Gln Glu Leu Ala Glu Val Leu Arg Glu Val Thr Cys
945                    950                    955                    960

cgg agt aag gaa ctc aca ccc tcg gcc cag tgg gag gcc agc ttg gct     2928
Arg Ser Lys Glu Leu Thr Pro Ser Ala Gln Trp Glu Ala Ser Leu Ala
                965                    970                    975

cta cag cat gcc agt gag gcc ctg ttg aca gtg agt gcc aag gcc cgc     2976
Leu Gln His Ala Ser Glu Ala Leu Leu Thr Val Ser Ala Lys Ala Arg
            980                    985                    990

cct gag gac cag agg cgc cag gca  gcc acc agg gac ctg  ttt cag gct     3024
Pro Glu Asp Gln Arg Arg Gln Ala  Ala Thr Arg Asp Leu  Phe Gln Ala
        995                    1000                    1005

gtg ggc  agt gtg ctg gaa gct  tcc ctg agc aac aga  cca gaa gag     3069
Val Gly  Ser Val Leu Glu Ala  Ser Leu Ser Asn Arg  Pro Glu Glu
    1010                    1015                    1020

cct gcg  gag gcc agc agc agc  cag att gcc aca gtg  ctg cgg ctg     3114
Pro Ala  Glu Ala Ser Ser Ser  Gln Ile Ala Thr Val  Leu Arg Leu
    1025                    1030                    1035

ctt cga  gtc atg gag cat gtg  cag acc acc ctc ctg  ctg gga aaa     3159
Leu Arg  Val Met Glu His Val  Gln Thr Thr Leu Leu  Leu Gly Lys
    1040                    1045                    1050

ctg cca  ggg ggc ctt cca gcc  atg ctg gcc acc ccc  tcc atc tct     3204
Leu Pro  Gly Gly Leu Pro Ala  Met Leu Ala Thr Pro  Ser Ile Ser

1055                    1060                    1065

gtg tac aca aac aga ata caa ccc tgg agt tgg caa ggc tcc tcc        3249
Val Tyr Thr Asn Arg Ile Gln Pro Trp Ser Trp Gln Gly Ser Ser
    1070                    1075                    1080

ctg cgc cct gat gcc gca gac tct gca acc ttc atg ctg ccc gct        3294
Leu Arg Pro Asp Ala Ala Asp Ser Ala Thr Phe Met Leu Pro Ala
    1085                    1090                    1095

gcc tcc tcc ctc agc tct ctg gag ggc ggc cag gag ccc gtg gat        3339
Ala Ser Ser Leu Ser Ser Leu Glu Gly Gly Gln Glu Pro Val Asp
    1100                    1105                    1110

ata aag atc atg agt ttc cca aag agc ccc ttt cca gcc cga agc        3384
Ile Lys Ile Met Ser Phe Pro Lys Ser Pro Phe Pro Ala Arg Ser
    1115                    1120                    1125

cac ttt gat gtc agc ggg act gtc ggt ggc ctc cgt gtg acc agc        3429
His Phe Asp Val Ser Gly Thr Val Gly Gly Leu Arg Val Thr Ser
    1130                    1135                    1140

cct agt ggt caa ctc ata cct gtg aag aat ctg tcg gag aat atc        3474
Pro Ser Gly Gln Leu Ile Pro Val Lys Asn Leu Ser Glu Asn Ile
    1145                    1150                    1155

gag atc ctg ctg ccc cgg cat tca caa aga cac agc cag ccg acc        3519
Glu Ile Leu Leu Pro Arg His Ser Gln Arg His Ser Gln Pro Thr
    1160                    1165                    1170

gtg ttg aac ctg acc agt cct gaa gct ttg tgg gtg aac gtg act        3564
Val Leu Asn Leu Thr Ser Pro Glu Ala Leu Trp Val Asn Val Thr
    1175                    1180                    1185

tca ggg gag gca acc ttg ggg atc cag ctg cac tgg aga cca gac        3609
Ser Gly Glu Ala Thr Leu Gly Ile Gln Leu His Trp Arg Pro Asp
    1190                    1195                    1200

att gca ctc acg ctt agc ctg ggc tat ggc tac cac ccc aac aag        3654
Ile Ala Leu Thr Leu Ser Leu Gly Tyr Gly Tyr His Pro Asn Lys
    1205                    1210                    1215

agc agc tac gat gcc caa act cac ctc gta cca atg gtg gct cca        3699
Ser Ser Tyr Asp Ala Gln Thr His Leu Val Pro Met Val Ala Pro
    1220                    1225                    1230

gat gag ctg ccc acg tgg atc ctg agc cca cag gac ctg cgt ttt        3744
Asp Glu Leu Pro Thr Trp Ile Leu Ser Pro Gln Asp Leu Arg Phe
    1235                    1240                    1245

gga gaa ggg gtc tac tat ttg act gtg gtc cct gag tct gac ctg        3789
Gly Glu Gly Val Tyr Tyr Leu Thr Val Val Pro Glu Ser Asp Leu
    1250                    1255                    1260

gag cca gcc ccc ggc agg gac ctc acg gtt ggc atc acc acc ttc        3834
Glu Pro Ala Pro Gly Arg Asp Leu Thr Val Gly Ile Thr Thr Phe
    1265                    1270                    1275

ctg tct cac tgt gtg ttc tgg gat gag gtc cag gag act tgg gac        3879
Leu Ser His Cys Val Phe Trp Asp Glu Val Gln Glu Thr Trp Asp
    1280                    1285                    1290

gac tca gga tgc cag gtg ggg cct cgg acc agc ccc tac cag aca        3924
Asp Ser Gly Cys Gln Val Gly Pro Arg Thr Ser Pro Tyr Gln Thr
    1295                    1300                    1305

cac tgc ctc tgc aac cac ctc act ttc ttc gga agc acg ttc ctg        3969
His Cys Leu Cys Asn His Leu Thr Phe Phe Gly Ser Thr Phe Leu

1310                    1315                    1320

gtg atg tcc aat gcc atc aac atc cac cag act gct gag ctc ttt      4014
Val Met Ser Asn Ala Ile Asn Ile His Gln Thr Ala Glu Leu Phe
    1325                1330                1335

gcc acc ttt gag gac aac cct gtg gtc gtg acc acc gtg ggc tgc      4059
Ala Thr Phe Glu Asp Asn Pro Val Val Val Thr Thr Val Gly Cys
    1340                1345                1350

ctg tgt gtg gtc tac gtg ctg gtg gtg atc tgg gcg agg agg aag      4104
Leu Cys Val Val Tyr Val Leu Val Val Ile Trp Ala Arg Arg Lys
    1355                1360                1365

gac gct cag gat cag gcc aag gtg aag gtc aca gtg ctg gaa gac      4149
Asp Ala Gln Asp Gln Ala Lys Val Lys Val Thr Val Leu Glu Asp
    1370                1375                1380

aat gat ccc ttt gct cag tac cac tac ctg gtg aca gtc tac aca      4194
Asn Asp Pro Phe Ala Gln Tyr His Tyr Leu Val Thr Val Tyr Thr
    1385                1390                1395

gga cac cga cga ggg gca gcc acg tcc tca aag gtg act gtc acc      4239
Gly His Arg Arg Gly Ala Ala Thr Ser Ser Lys Val Thr Val Thr
    1400                1405                1410

ctg tat ggc ctg gat gga gag aga gag ccc cac cac ctg gct gat      4284
Leu Tyr Gly Leu Asp Gly Glu Arg Glu Pro His His Leu Ala Asp
    1415                1420                1425

ccc gac act ccg gtt ttt gag cga gga gca gtg gat gcc ttc ctc      4329
Pro Asp Thr Pro Val Phe Glu Arg Gly Ala Val Asp Ala Phe Leu
    1430                1435                1440

ctc tcc acc ctg ttc ccc ctg gga gaa ctg cgg agc ctc cgg ctg      4374
Leu Ser Thr Leu Phe Pro Leu Gly Glu Leu Arg Ser Leu Arg Leu
    1445                1450                1455

tgg cat gac aac tca ggg gac cgg cca tcg tgg tat gtg agc cgg      4419
Trp His Asp Asn Ser Gly Asp Arg Pro Ser Trp Tyr Val Ser Arg
    1460                1465                1470

gtg ctg gtc tat gac ctg gtg atg gac cgg aag tgg tat ttc ctg      4464
Val Leu Val Tyr Asp Leu Val Met Asp Arg Lys Trp Tyr Phe Leu
    1475                1480                1485

tgc aac tcc tgg cta tcc atc aat gtt gga gat tgc gtc ctc gat      4509
Cys Asn Ser Trp Leu Ser Ile Asn Val Gly Asp Cys Val Leu Asp
    1490                1495                1500

aag gtg ttt cct gtg gcc acg gag cag gac aga aaa caa ttc agc      4554
Lys Val Phe Pro Val Ala Thr Glu Gln Asp Arg Lys Gln Phe Ser
    1505                1510                1515

cac ctg ttt ttc atg aag act tcc gcg ggc ttc cag gat gga cac      4599
His Leu Phe Phe Met Lys Thr Ser Ala Gly Phe Gln Asp Gly His
    1520                1525                1530

atc tgg tat tcg atc ttc agc cgc tgc gct cgc agc agc ttc acc      4644
Ile Trp Tyr Ser Ile Phe Ser Arg Cys Ala Arg Ser Ser Phe Thr
    1535                1540                1545

cgc gtc cag agg gtg tcc tgc tgc ttc tcc ctg ctg ctg tgc acc      4689
Arg Val Gln Arg Val Ser Cys Cys Phe Ser Leu Leu Leu Cys Thr
    1550                1555                1560

atg ctg acc agc atc atg ttc tgg ggg gtc ccc aag gac cca gct      4734
Met Leu Thr Ser Ile Met Phe Trp Gly Val Pro Lys Asp Pro Ala

1565                    1570                    1575

gag caa aag atg gac ttg ggt aaa att gaa ttc acc tgg cag gag          4779
Glu Gln Lys Met Asp Leu Gly Lys Ile Glu Phe Thr Trp Gln Glu
    1580                    1585                    1590

gtg atg att ggc ctg gag agc tcc atc ctc atg ttc ccc atc aac          4824
Val Met Ile Gly Leu Glu Ser Ser Ile Leu Met Phe Pro Ile Asn
    1595                    1600                    1605

ctc ctg att gtt cag atc ttt cag aac acc cgt ccc cgg gtc gcg          4869
Leu Leu Ile Val Gln Ile Phe Gln Asn Thr Arg Pro Arg Val Ala
    1610                    1615                    1620

aag gag cag aac act gga aaa tgg gac cgg ggg tcc ccc aac ctg          4914
Lys Glu Gln Asn Thr Gly Lys Trp Asp Arg Gly Ser Pro Asn Leu
    1625                    1630                    1635

act ccc tcc cca cag ccc atg gag gac ggc ctt ctg aca cct gag          4959
Thr Pro Ser Pro Gln Pro Met Glu Asp Gly Leu Leu Thr Pro Glu
    1640                    1645                    1650

gca gtg acc aag gat gtg tca aga atc gtc agc tcc ctc ttc aaa          5004
Ala Val Thr Lys Asp Val Ser Arg Ile Val Ser Ser Leu Phe Lys
    1655                    1660                    1665

gct ctc aag gtg cca tcc ccc gcc ttg ggc tgg gac tca gtg aac          5049
Ala Leu Lys Val Pro Ser Pro Ala Leu Gly Trp Asp Ser Val Asn
    1670                    1675                    1680

ttg atg gac atc aac agt ctc ctc gcc ttg gtg gaa gat gtc att          5094
Leu Met Asp Ile Asn Ser Leu Leu Ala Leu Val Glu Asp Val Ile
    1685                    1690                    1695

tat cca cag aac aca tca ggg cag gtg ttc tgg gag gaa gcc aaa          5139
Tyr Pro Gln Asn Thr Ser Gly Gln Val Phe Trp Glu Glu Ala Lys
    1700                    1705                    1710

aag aga gag gac cct gta aca ctc act ttg ggg tca tca gaa atg          5184
Lys Arg Glu Asp Pro Val Thr Leu Thr Leu Gly Ser Ser Glu Met
    1715                    1720                    1725

aaa gag aaa tca cag tgt ccc aag ccc aag gcg gca cgg agt ggc          5229
Lys Glu Lys Ser Gln Cys Pro Lys Pro Lys Ala Ala Arg Ser Gly
    1730                    1735                    1740

ccc tgg aag gac agc gcc tac agg cag tgt ctg tac ctt cag ctg          5274
Pro Trp Lys Asp Ser Ala Tyr Arg Gln Cys Leu Tyr Leu Gln Leu
    1745                    1750                    1755

gaa cac gtg gag caa gag ctg cgg ctg gtg ggg ccc cga ggc ttc          5319
Glu His Val Glu Gln Glu Leu Arg Leu Val Gly Pro Arg Gly Phe
    1760                    1765                    1770

tcc cag ccc cac agc cat gcc cag gcc ctc agg cag ctg cag acc          5364
Ser Gln Pro His Ser His Ala Gln Ala Leu Arg Gln Leu Gln Thr
    1775                    1780                    1785

ctg aag ggc ggc ctg ggg gta cag ccg ggc acc tgg gcc cct gca          5409
Leu Lys Gly Gly Leu Gly Val Gln Pro Gly Thr Trp Ala Pro Ala
    1790                    1795                    1800

cat gcc agc gct ctt cag gtg agc aaa ccc cct caa ggc ctg ccc          5454
His Ala Ser Ala Leu Gln Val Ser Lys Pro Pro Gln Gly Leu Pro
    1805                    1810                    1815

tgg tgg tgc atc ctg gtg ggc tgg ctc ctg gta gcg gcc acc agt          5499
Trp Trp Cys Ile Leu Val Gly Trp Leu Leu Val Ala Ala Thr Ser

1820                    1825                    1830

ggc gtg gcg gcc ttc ttc acc atg ctc tac ggc ctg cac tac ggg          5544
Gly Val Ala Ala Phe Phe Thr Met Leu Tyr Gly Leu His Tyr Gly
    1835                    1840                    1845

agg gcc agc tcc ctc agg tgg ctc atc tcc atg gct gtc tcc ttc          5589
Arg Ala Ser Ser Leu Arg Trp Leu Ile Ser Met Ala Val Ser Phe
    1850                    1855                    1860

gtg gag agc atg ttc gtc acc cag ccc ctg aag gtg ctg gga ttc          5634
Val Glu Ser Met Phe Val Thr Gln Pro Leu Lys Val Leu Gly Phe
    1865                    1870                    1875

gct gct ttc ttt gca ctg gtc ttg aag aga gtg gac gat gag gag          5679
Ala Ala Phe Phe Ala Leu Val Leu Lys Arg Val Asp Asp Glu Glu
    1880                    1885                    1890

gat act gtg gcc ccg ctg cca gga cat ctg ttg ggc cca gac ccc          5724
Asp Thr Val Ala Pro Leu Pro Gly His Leu Leu Gly Pro Asp Pro
    1895                    1900                    1905

tat gcc ttg ttc cga gca cga aga aac agc agc agg gat gtc tac          5769
Tyr Ala Leu Phe Arg Ala Arg Arg Asn Ser Ser Arg Asp Val Tyr
    1910                    1915                    1920

cag cca cct ctc acc gct gcc att gag aag atg aaa acc acc cac          5814
Gln Pro Pro Leu Thr Ala Ala Ile Glu Lys Met Lys Thr Thr His
    1925                    1930                    1935

ctc aag gaa cag aaa gca ttt gcc ctc atc aga gaa atc ctg gca          5859
Leu Lys Glu Gln Lys Ala Phe Ala Leu Ile Arg Glu Ile Leu Ala
    1940                    1945                    1950

tac ttg ggc ttc ctg tgg atg cta ctg ctc gtg gcc tac ggg cag          5904
Tyr Leu Gly Phe Leu Trp Met Leu Leu Leu Val Ala Tyr Gly Gln
    1955                    1960                    1965

agg gac ccc agc gcc tac cac ctc aac aga cac ctc cag cac agc          5949
Arg Asp Pro Ser Ala Tyr His Leu Asn Arg His Leu Gln His Ser
    1970                    1975                    1980

ttc acc agg ggc ttt tca ggt gtg ctc ggc ttc cga gag ttc ttc          5994
Phe Thr Arg Gly Phe Ser Gly Val Leu Gly Phe Arg Glu Phe Phe
    1985                    1990                    1995

aag tgg gcc aac acc acc ctc gtg agt aac ctg tat ggt cac ccc          6039
Lys Trp Ala Asn Thr Thr Leu Val Ser Asn Leu Tyr Gly His Pro
    2000                    2005                    2010

cca ggc ttt atc act gat ggg aac tcc aag ctg gtt ggc agt gcc          6084
Pro Gly Phe Ile Thr Asp Gly Asn Ser Lys Leu Val Gly Ser Ala
    2015                    2020                    2025

cag att cgt caa gtg agg gtc cag gaa agc tct tgc cct ctt gcc          6129
Gln Ile Arg Gln Val Arg Val Gln Glu Ser Ser Cys Pro Leu Ala
    2030                    2035                    2040

cag cag ccg cag gca tat ctc aac gga tgc cgt gca cca tat tcc          6174
Gln Gln Pro Gln Ala Tyr Leu Asn Gly Cys Arg Ala Pro Tyr Ser
    2045                    2050                    2055

ctg gat gct gaa gac atg gca gac tat ggg gaa ggc tgg aat gcc          6219
Leu Asp Ala Glu Asp Met Ala Asp Tyr Gly Glu Gly Trp Asn Ala
    2060                    2065                    2070

acc acc ctc agt gaa tgg cag tac cag agc cag gac caa cgt caa          6264
Thr Thr Leu Ser Glu Trp Gln Tyr Gln Ser Gln Asp Gln Arg Gln

2075                2080                2085

ggg tat ccc atc tgg ggc aaa ctc act gtg tac cgg gga gga ggc      6309
Gly Tyr Pro Ile Trp Gly Lys Leu Thr Val Tyr Arg Gly Gly Gly
    2090                2095                2100

tac gtg gtc ccc ttg ggg act gat cgc caa agc acg tca aga att      6354
Tyr Val Val Pro Leu Gly Thr Asp Arg Gln Ser Thr Ser Arg Ile
    2105                2110                2115

ctc cgc tat ctc ttt gac aac acc tgg ctg gac gcc ctg acc aga      6399
Leu Arg Tyr Leu Phe Asp Asn Thr Trp Leu Asp Ala Leu Thr Arg
    2120                2125                2130

gct gtg ttt gtg gag tcc act gtc tac aac gcc aac gtc aac ctg      6444
Ala Val Phe Val Glu Ser Thr Val Tyr Asn Ala Asn Val Asn Leu
    2135                2140                2145

ttc tgc att gtc acg ctg acg cta gag acc agc gct ctg ggc acc      6489
Phe Cys Ile Val Thr Leu Thr Leu Glu Thr Ser Ala Leu Gly Thr
    2150                2155                2160

ttt ttt acg cac gcg gcc ctg cag agc ctc cgc ctg tac ccc ttc      6534
Phe Phe Thr His Ala Ala Leu Gln Ser Leu Arg Leu Tyr Pro Phe
    2165                2170                2175

acc gac ggc tgg cac ccc ttc gtg gta gcg gca gag ctc atc tac      6579
Thr Asp Gly Trp His Pro Phe Val Val Ala Ala Glu Leu Ile Tyr
    2180                2185                2190

ttc ctc ttc ctc ctc tac tac atg gtg gtg cag ggc aag cgc atg      6624
Phe Leu Phe Leu Leu Tyr Tyr Met Val Val Gln Gly Lys Arg Met
    2195                2200                2205

agt aaa gag acg tgg ggc tat ttc tgc agc aag tgg aac ctt ctg      6669
Ser Lys Glu Thr Trp Gly Tyr Phe Cys Ser Lys Trp Asn Leu Leu
    2210                2215                2220

gag ctg gcc atc atc ctg gcc agc tgg agc gcc ctg gcg gtg ttt      6714
Glu Leu Ala Ile Ile Leu Ala Ser Trp Ser Ala Leu Ala Val Phe
    2225                2230                2235

gtg aag agg gct gtc ctg gcc gaa agg gac ctc cag cgc tgc cgg      6759
Val Lys Arg Ala Val Leu Ala Glu Arg Asp Leu Gln Arg Cys Arg
    2240                2245                2250

aac cac agg gag gaa ggc atc agc ttc agt gag aca gca gca gcc      6804
Asn His Arg Glu Glu Gly Ile Ser Phe Ser Glu Thr Ala Ala Ala
    2255                2260                2265

gat gcc gcc ctt ggc tac atc att gcc ttc ctg gta ctc ctg tcc      6849
Asp Ala Ala Leu Gly Tyr Ile Ile Ala Phe Leu Val Leu Leu Ser
    2270                2275                2280

aca gtg aag ctt tgg cat ctg ctc agg ttg aat ccc aaa atg aac      6894
Thr Val Lys Leu Trp His Leu Leu Arg Leu Asn Pro Lys Met Asn
    2285                2290                2295

atg atc acg gca gcc cta cgc cgt gcc tgg ggc gac att tca ggc      6939
Met Ile Thr Ala Ala Leu Arg Arg Ala Trp Gly Asp Ile Ser Gly
    2300                2305                2310

ttt atg att gtc atc ctt acc atg ctc ctg gct tac tcc atc gcg      6984
Phe Met Ile Val Ile Leu Thr Met Leu Leu Ala Tyr Ser Ile Ala
    2315                2320                2325

tca aac ttg ata ttt ggt tgg aaa ctc cgt tcc tac aaa acc ctc      7029
Ser Asn Leu Ile Phe Gly Trp Lys Leu Arg Ser Tyr Lys Thr Leu

2330                2335                2340

tttgat gcg gcg gag acg atg gtc agc ctt cag ctg gga atc ttc      7074
Phe Asp Ala Ala Glu Thr Met Val Ser Leu Gln Leu Gly Ile Phe
    2345                2350                2355

aac tac gag gag gtc ctg gac tat agc cca gtg ctt ggc tcc ttc      7119
Asn Tyr Glu Glu Val Leu Asp Tyr Ser Pro Val Leu Gly Ser Phe
    2360                2365                2370

ctc att gga tcc tgc att gtt ttt atg aca ttt gtg gtg ctg aac      7164
Leu Ile Gly Ser Cys Ile Val Phe Met Thr Phe Val Val Leu Asn
    2375                2380                2385

ctg ttt atc tct gtc atc ctg gtg gcc ttc agt gag gag caa aaa      7209
Leu Phe Ile Ser Val Ile Leu Val Ala Phe Ser Glu Glu Gln Lys
    2390                2395                2400

tac tat cag ctg tcg gag gaa ggg gag atc gta gat ttg ctg ctg      7254
Tyr Tyr Gln Leu Ser Glu Glu Gly Glu Ile Val Asp Leu Leu Leu
    2405                2410                2415

atg aaa ata ctc agt ttc ctg ggc att aag tct aag aga gag gag      7299
Met Lys Ile Leu Ser Phe Leu Gly Ile Lys Ser Lys Arg Glu Glu
    2420                2425                2430

cct gga agc agc agg gag cag cct ggg tct ctg tcc cag act cgc      7344
Pro Gly Ser Ser Arg Glu Gln Pro Gly Ser Leu Ser Gln Thr Arg
    2435                2440                2445

cac tct cga cca gca caa gct ttg ccc aag gac taa gctgttcgtc      7390
His Ser Arg Pro Ala Gln Ala Leu Pro Lys Asp
    2450                2455

cacacgccac catctaccag tggggacgcc cagggcctcg ggcgtacgct taccagcgac      7450

tctatagtct ctctaggtca tagctttcat gtccattgaa gaattaccaa gcccagcaag      7510

taaaaaaaat tacaataaca gagatatatg taaaattgcc aatactatga gagcttcttt      7570

gttcaaatgt atttcctctt attataaata aaacaaatat tacatggagt ttgg      7624

<210>  118
<211>  2459
<212>  PRT
<213>  human

<400>  118

Met Ser Ala Val Gly Leu Val Leu Leu Val Leu Ala Leu Arg Leu Arg
1               5                   10                  15

Ala Thr Thr Val Lys Pro Glu Glu Gly Ser Phe Cys Ser Asn Ser Gln
            20                  25                  30

Val Ala Phe Arg Asp Ala Cys Tyr Glu Phe Val Pro Leu Gly Arg Thr
        35                  40                  45

Phe Arg Asp Ala Gln Ser Trp Cys Glu Gly Gln Gly Gly His Leu Val
    50                  55                  60

Phe Ile Gln Asp Glu Gly Thr Gln Trp Phe Leu Gln Lys His Ile Ser
65                  70                  75                  80

Gln Asp Arg Glu Trp Trp Ile Gly Leu Thr Trp Asn Leu Ala Arg Asn
                85                  90                  95

Gly Thr Thr Glu Gly Pro Gly Thr Trp Leu Asp Thr Ser Asn Val Thr
            100                 105                 110

Tyr Ser Asn Trp His Gly Gly Gln Ala Ala Ala Ala Pro Asp Thr Cys
        115                 120                 125

Gly His Ile Gly Arg Gly Pro Ser Ser Glu Trp Val Thr Ser Asp Cys
    130                 135                 140

Ala Gln Thr Phe Ala Phe Met Cys Glu Phe Arg Val Gly Gln Ser Leu
145                 150                 155                 160

Ala Cys Glu Gly Leu Asn Ala Thr Val His Cys Gly Leu Gly Gln Val
                165                 170                 175

Ile Gln Val Gln Asp Ala Val Tyr Gly Arg Gln Asn Pro His Phe Cys
            180                 185                 190

Thr Gln Asp Ala Gly Arg Pro Ser Asp Leu Glu Gln Gly Cys Ser Trp
        195                 200                 205

Ala Asn Val Lys Glu Glu Val Ala Gly Gln Cys Gln Glu Leu Gln Ser
    210                 215                 220

Cys Gln Val Ala Ala Asp Glu Thr Tyr Phe Gly Asn Leu Cys Pro Thr
225                 230                 235                 240

Gln Gly Ser Tyr Leu Trp Val Gln Tyr Gln Cys Arg Glu Ala Leu Gln
                245                 250                 255

Leu Met Val Ser Ser Glu Ser Phe Ile Phe Asp Asn Val Thr Ile Ser
            260                 265                 270

Leu Thr Trp Leu Leu Ser Pro Tyr Ile Gly Asn Leu Ser Cys Ile Ile
        275                 280                 285

Ser Thr Gly Asp Ser His Thr Phe Asp Pro Tyr Asn Pro Pro Ser Val
    290                 295                 300

Ser Ser Asn Val Thr His Gln Phe Thr Ser Pro Gly Glu Phe Thr Val
305                 310                 315                 320

Phe Ala Glu Cys Thr Thr Ser Glu Trp His Val Thr Ala Gln Arg Gln
                325                 330                 335

Val Thr Val Arg Asp Lys Met Glu Thr Leu Ser Val Thr Ala Cys Ser
            340                 345                 350

Gly Leu Ser Gln Ser Gly Ala Gly Pro Leu Cys Gln Ala Val Phe Gly
        355                 360                 365

Asp Pro Leu Trp Ile Gln Val Glu Leu Asp Gly Gly Thr Gly Val Thr
    370                 375                 380

Tyr Thr Val Leu Leu Gly Asp Ile Thr Leu Ala Glu Ser Thr Thr Gln
385                 390                 395                 400

Lys Gly Ser Leu Pro Tyr Asn Leu Ile Leu Asp Arg Glu Thr Gln Lys
                405                 410                 415

Leu Met Gly Pro Gly Arg His Arg Leu Glu Ile Gln Ala Thr Gly Asn
            420                 425                 430

Thr Thr Thr Ser Thr Ile Ser Arg Asn Ile Thr Val His Leu Val Glu
        435                 440                 445

Leu Leu Ser Gly Leu Gln Ala Ser Trp Ala Ser Asp His Leu Glu Leu
    450                 455                 460

Gly Gln Asp Leu Leu Ile Thr Ile Ser Leu Ala Gln Gly Thr Pro Glu
465                 470                 475                 480

Glu Leu Thr Phe Glu Val Ala Gly Leu Asn Ala Thr Phe Ser His Glu
                485                 490                 495

Gln Val Ser Phe Gly Glu Pro Phe Gly Ile Cys Arg Leu Ala Val Pro
            500                 505                 510

Val Glu Gly Thr Phe Leu Val Thr Met Leu Val Arg Asn Ala Phe Ser
        515                 520                 525

Asn Leu Ser Leu Glu Ile Gly Asn Ile Thr Ile Thr Ala Pro Ser Gly
    530                 535                 540

Leu Gln Glu Pro Ser Gly Met Asn Ala Glu Gly Lys Ser Lys Asp Lys
545                 550                 555                 560

Gly Asp Met Glu Val Tyr Ile Gln Pro Gly Pro Tyr Val Asp Pro Phe
                565                 570                 575

Thr Thr Val Thr Leu Gly Trp Pro Asp Asn Asp Lys Glu Leu Arg Phe
            580                 585                 590

Gln Trp Ser Cys Gly Ser Cys Trp Ala Leu Trp Ser Ser Cys Val Glu
        595                 600                 605

Arg Gln Leu Leu Arg Thr Asp Gln Arg Glu Leu Val Val Pro Ala Ser
    610                 615                 620

Cys Leu Pro Pro Pro Asp Ser Ala Val Thr Leu Arg Leu Ala Val Leu
625                 630                 635                 640

Arg Gly Gln Glu Leu Glu Asn Arg Ala Glu Gln Cys Leu Tyr Val Ser
                645                 650                 655

Ala Pro Trp Glu Leu Arg Pro Arg Val Ser Cys Glu Arg Asn Cys Arg
            660                 665                 670

Pro Val Asn Ala Ser Lys Asp Ile Leu Leu Arg Val Thr Met Gly Glu
        675                 680                 685

Asp Ser Pro Val Ala Met Phe Ser Trp Tyr Leu Asp Asn Thr Pro Thr
    690                 695                 700

Glu Gln Ala Glu Pro Leu Leu Asp Ala Cys Arg Leu Arg Gly Phe Trp
705                 710                 715                 720

Pro Arg Ser Leu Thr Leu Leu Gln Ser Asn Thr Ser Thr Leu Leu Leu
                725                 730                 735

Asn Ser Ser Phe Leu Gln Ser Arg Gly Glu Val Ile Arg Ile Arg Ala
            740                 745                 750

Thr Ala Leu Thr Arg His Ala Tyr Gly Glu Asp Thr Tyr Val Ile Ser
        755                 760                 765

Thr Val Pro Pro Arg Glu Val Pro Ala Cys Thr Ile Ala Pro Glu Glu
    770                 775                 780

Gly Thr Val Leu Thr Ser Phe Ala Ile Phe Cys Asn Ala Ser Thr Ala
785                 790                 795                 800

Leu Gly Pro Leu Glu Phe Cys Phe Cys Leu Glu Ser Gly Ser Cys Leu
                805                 810                 815

His Cys Gly Pro Glu Pro Ala Leu Pro Ser Val Tyr Leu Pro Leu Gly
            820                 825                 830

Glu Glu Asn Asn Asp Phe Val Leu Thr Val Val Ile Ser Ala Thr Asn
        835                 840                 845

Arg Ala Gly Asp Thr Gln Gln Thr Gln Ala Met Ala Lys Val Ala Leu
    850                 855                 860

Gly Asp Thr Cys Val Glu Asp Val Ala Phe Gln Ala Ala Val Ser Glu
865                 870                 875                 880

Lys Ile Pro Thr Ala Leu Gln Gly Glu Gly Gly Pro Glu Gln Leu Leu
                885                 890                 895

Gln Leu Ala Lys Ala Val Ser Ser Met Leu Asn Gln Glu His Glu Ser
            900                 905                 910

Gln Gly Ser Gly Gln Ser Leu Ser Ile Asp Val Arg Gln Lys Val Arg
        915                 920                 925

Glu His Val Leu Gly Ser Leu Ser Ala Val Thr Thr Gly Leu Glu Asp
    930                 935                 940

Val Gln Arg Val Gln Glu Leu Ala Glu Val Leu Arg Glu Val Thr Cys
945                 950                 955                 960

Arg Ser Lys Glu Leu Thr Pro Ser Ala Gln Trp Glu Ala Ser Leu Ala
                965                 970                 975

Leu Gln His Ala Ser Glu Ala Leu Leu Thr Val Ser Ala Lys Ala Arg
            980                 985                 990

Pro Glu Asp Gln Arg Arg Gln Ala  Ala Thr Arg Asp Leu  Phe Gln Ala
        995                 1000                 1005

Val Gly  Ser Val Leu Glu Ala  Ser Leu Ser Asn Arg  Pro Glu Glu
    1010                 1015                 1020

Pro Ala  Glu Ala Ser Ser Ser  Gln Ile Ala Thr Val  Leu Arg Leu
    1025                 1030                 1035

Leu Arg  Val Met Glu His Val  Gln Thr Thr Leu Leu  Leu Gly Lys
    1040                 1045                 1050

Leu Pro  Gly Gly Leu Pro Ala  Met Leu Ala Thr Pro  Ser Ile Ser
    1055                 1060                 1065

Val Tyr  Thr Asn Arg Ile Gln  Pro Trp Ser Trp Gln  Gly Ser Ser
    1070                 1075                 1080

Leu Arg  Pro Asp Ala Ala Asp  Ser Ala Thr Phe Met  Leu Pro Ala
    1085                 1090                 1095

Ala Ser  Ser Leu Ser Ser Leu  Glu Gly Gly Gln Glu  Pro Val Asp
    1100                 1105                 1110

Ile Lys  Ile Met Ser Phe Pro  Lys Ser Pro Phe Pro  Ala Arg Ser
    1115                 1120                 1125

His Phe  Asp Val Ser Gly Thr  Val Gly Gly Leu Arg  Val Thr Ser
    1130                 1135                 1140

Pro Ser  Gly Gln Leu Ile Pro  Val Lys Asn Leu Ser  Glu Asn Ile
    1145                 1150                 1155

Glu Ile Leu Leu Pro Arg His Ser Gln Arg His Ser Gln Pro Thr
    1160                1165                1170

Val Leu Asn Leu Thr Ser Pro Glu Ala Leu Trp Val Asn Val Thr
    1175                1180                1185

Ser Gly Glu Ala Thr Leu Gly Ile Gln Leu His Trp Arg Pro Asp
    1190                1195                1200

Ile Ala Leu Thr Leu Ser Leu Gly Tyr Gly Tyr His Pro Asn Lys
    1205                1210                1215

Ser Ser Tyr Asp Ala Gln Thr His Leu Val Pro Met Val Ala Pro
    1220                1225                1230

Asp Glu Leu Pro Thr Trp Ile Leu Ser Pro Gln Asp Leu Arg Phe
    1235                1240                1245

Gly Glu Gly Val Tyr Tyr Leu Thr Val Val Pro Glu Ser Asp Leu
    1250                1255                1260

Glu Pro Ala Pro Gly Arg Asp Leu Thr Val Gly Ile Thr Thr Phe
    1265                1270                1275

Leu Ser His Cys Val Phe Trp Asp Glu Val Gln Glu Thr Trp Asp
    1280                1285                1290

Asp Ser Gly Cys Gln Val Gly Pro Arg Thr Ser Pro Tyr Gln Thr
    1295                1300                1305

His Cys Leu Cys Asn His Leu Thr Phe Phe Gly Ser Thr Phe Leu
    1310                1315                1320

Val Met Ser Asn Ala Ile Asn Ile His Gln Thr Ala Glu Leu Phe
    1325                1330                1335

Ala Thr Phe Glu Asp Asn Pro Val Val Val Thr Thr Val Gly Cys
    1340                1345                1350

Leu Cys Val Val Tyr Val Leu Val Val Ile Trp Ala Arg Arg Lys
    1355                1360                1365

Asp Ala Gln Asp Gln Ala Lys Val Lys Val Thr Val Leu Glu Asp
    1370                1375                1380

Asn Asp Pro Phe Ala Gln Tyr His Tyr Leu Val Thr Val Tyr Thr
    1385                1390                1395

Gly His Arg Arg Gly Ala Ala Thr Ser Ser Lys Val Thr Val Thr
    1400                1405                1410

Leu Tyr Gly Leu Asp Gly Glu Arg Glu Pro His His Leu Ala Asp
    1415                1420                1425

Pro Asp Thr Pro Val Phe Glu Arg Gly Ala Val Asp Ala Phe Leu
    1430                1435                1440

Leu Ser Thr Leu Phe Pro Leu Gly Glu Leu Arg Ser Leu Arg Leu
    1445                1450                1455

Trp His Asp Asn Ser Gly Asp Arg Pro Ser Trp Tyr Val Ser Arg
    1460                1465                1470

Val Leu Val Tyr Asp Leu Val Met Asp Arg Lys Trp Tyr Phe Leu
    1475                1480                1485

Cys Asn Ser Trp Leu Ser Ile Asn Val Gly Asp Cys Val Leu Asp
    1490                1495                1500

Lys Val Phe Pro Val Ala Thr Glu Gln Asp Arg Lys Gln Phe Ser
    1505                1510                1515

His Leu Phe Phe Met Lys Thr Ser Ala Gly Phe Gln Asp Gly His
    1520                1525                1530

Ile Trp Tyr Ser Ile Phe Ser Arg Cys Ala Arg Ser Ser Phe Thr
    1535                1540                1545

Arg Val Gln Arg Val Ser Cys Cys Phe Ser Leu Leu Leu Cys Thr
    1550                1555                1560

Met Leu Thr Ser Ile Met Phe Trp Gly Val Pro Lys Asp Pro Ala
    1565                1570                1575

Glu Gln Lys Met Asp Leu Gly Lys Ile Glu Phe Thr Trp Gln Glu
    1580                1585                1590

Val Met Ile Gly Leu Glu Ser Ser Ile Leu Met Phe Pro Ile Asn
    1595                1600                1605

Leu Leu Ile Val Gln Ile Phe Gln Asn Thr Arg Pro Arg Val Ala
    1610                1615                1620

Lys Glu Gln Asn Thr Gly Lys Trp Asp Arg Gly Ser Pro Asn Leu
    1625                1630                1635

Thr Pro Ser Pro Gln Pro Met Glu Asp Gly Leu Leu Thr Pro Glu
    1640                1645                1650

Ala Val Thr Lys Asp Val Ser Arg Ile Val Ser Ser Leu Phe Lys
    1655                1660                1665

Ala Leu Lys Val Pro Ser Pro Ala Leu Gly Trp Asp Ser Val Asn
    1670                1675                1680

Leu Met Asp Ile Asn Ser Leu Leu Ala Leu Val Glu Asp Val Ile
    1685                1690                1695

Tyr Pro Gln Asn Thr Ser Gly Gln Val Phe Trp Glu Glu Ala Lys
    1700                1705                1710

Lys Arg Glu Asp Pro Val Thr Leu Thr Leu Gly Ser Ser Glu Met
    1715                1720                1725

Lys Glu Lys Ser Gln Cys Pro Lys Pro Lys Ala Ala Arg Ser Gly
    1730                1735                1740

Pro Trp Lys Asp Ser Ala Tyr Arg Gln Cys Leu Tyr Leu Gln Leu
    1745                1750                1755

Glu His Val Glu Gln Glu Leu Arg Leu Val Gly Pro Arg Gly Phe
    1760                1765                1770

Ser Gln Pro His Ser His Ala Gln Ala Leu Arg Gln Leu Gln Thr
    1775                1780                1785

Leu Lys Gly Gly Leu Gly Val Gln Pro Gly Thr Trp Ala Pro Ala
    1790                1795                1800

His Ala Ser Ala Leu Gln Val Ser Lys Pro Pro Gln Gly Leu Pro
    1805                1810                1815

Trp Trp Cys Ile Leu Val Gly Trp Leu Leu Val Ala Ala Thr Ser
    1820                1825                1830

Gly Val Ala Ala Phe Phe Thr Met Leu Tyr Gly Leu His Tyr Gly
    1835                1840                1845

Arg Ala Ser Ser Leu Arg Trp Leu Ile Ser Met Ala Val Ser Phe
    1850                1855                1860

Val Glu Ser Met Phe Val Thr Gln Pro Leu Lys Val Leu Gly Phe
    1865                1870                1875

Ala Ala Phe Phe Ala Leu Val Leu Lys Arg Val Asp Asp Glu Glu
    1880                1885                1890

Asp Thr Val Ala Pro Leu Pro Gly His Leu Leu Gly Pro Asp Pro
    1895                1900                1905

Tyr Ala Leu Phe Arg Ala Arg Arg Asn Ser Ser Arg Asp Val Tyr
    1910                1915                1920

Gln Pro Pro Leu Thr Ala Ala Ile Glu Lys Met Lys Thr Thr His
    1925                1930                1935

Leu Lys Glu Gln Lys Ala Phe Ala Leu Ile Arg Glu Ile Leu Ala
    1940                1945                1950

Tyr Leu Gly Phe Leu Trp Met Leu Leu Leu Val Ala Tyr Gly Gln
    1955                1960                1965

Arg Asp Pro Ser Ala Tyr His Leu Asn Arg His Leu Gln His Ser
    1970                1975                1980

Phe Thr Arg Gly Phe Ser Gly Val Leu Gly Phe Arg Glu Phe Phe
    1985                1990                1995

Lys Trp Ala Asn Thr Thr Leu Val Ser Asn Leu Tyr Gly His Pro
    2000                2005                2010

Pro Gly Phe Ile Thr Asp Gly Asn Ser Lys Leu Val Gly Ser Ala
    2015                2020                2025

Gln Ile Arg Gln Val Arg Val Gln Glu Ser Ser Cys Pro Leu Ala
    2030                2035                2040

Gln Gln Pro Gln Ala Tyr Leu Asn Gly Cys Arg Ala Pro Tyr Ser
    2045                2050                2055

Leu Asp Ala Glu Asp Met Ala Asp Tyr Gly Glu Gly Trp Asn Ala
    2060                2065                2070

Thr Thr Leu Ser Glu Trp Gln Tyr Gln Ser Gln Asp Gln Arg Gln
    2075                2080                2085

Gly Tyr Pro Ile Trp Gly Lys Leu Thr Val Tyr Arg Gly Gly Gly
    2090                2095                2100

Tyr Val Val Pro Leu Gly Thr Asp Arg Gln Ser Thr Ser Arg Ile
    2105                2110                2115

Leu Arg Tyr Leu Phe Asp Asn Thr Trp Leu Asp Ala Leu Thr Arg
    2120                2125                2130

Ala Val Phe Val Glu Ser Thr Val Tyr Asn Ala Asn Val Asn Leu
    2135                2140                2145

Phe Cys Ile Val Thr Leu Thr Leu Glu Thr Ser Ala Leu Gly Thr
    2150                2155                2160

Phe Phe Thr His Ala Ala Leu Gln Ser Leu Arg Leu Tyr Pro Phe
    2165                2170                2175

Thr Asp Gly Trp His Pro Phe Val Val Ala Ala Glu Leu Ile Tyr
    2180                2185                2190

Phe Leu Phe Leu Leu Tyr Tyr Met Val Val Gln Gly Lys Arg Met
    2195                2200                2205

Ser Lys Glu Thr Trp Gly Tyr Phe Cys Ser Lys Trp Asn Leu Leu
    2210                2215                2220

Glu Leu Ala Ile Ile Leu Ala Ser Trp Ser Ala Leu Ala Val Phe
    2225                2230                2235

Val Lys Arg Ala Val Leu Ala Glu Arg Asp Leu Gln Arg Cys Arg
    2240                2245                2250

Asn His Arg Glu Glu Gly Ile Ser Phe Ser Glu Thr Ala Ala Ala
    2255                2260                2265

Asp Ala Ala Leu Gly Tyr Ile Ile Ala Phe Leu Val Leu Leu Ser
    2270                2275                2280

Thr Val Lys Leu Trp His Leu Leu Arg Leu Asn Pro Lys Met Asn
    2285                2290                2295

Met Ile Thr Ala Ala Leu Arg Arg Ala Trp Gly Asp Ile Ser Gly
    2300                2305                2310

Phe Met Ile Val Ile Leu Thr Met Leu Leu Ala Tyr Ser Ile Ala
    2315                2320                2325

Ser Asn Leu Ile Phe Gly Trp Lys Leu Arg Ser Tyr Lys Thr Leu
    2330                2335                2340

Phe Asp Ala Ala Glu Thr Met Val Ser Leu Gln Leu Gly Ile Phe
    2345                2350                2355

Asn Tyr Glu Glu Val Leu Asp Tyr Ser Pro Val Leu Gly Ser Phe
    2360                2365                2370

Leu Ile Gly Ser Cys Ile Val Phe Met Thr Phe Val Val Leu Asn
    2375                2380                2385

Leu Phe Ile Ser Val Ile Leu Val Ala Phe Ser Glu Glu Gln Lys
    2390                2395                2400

Tyr Tyr Gln Leu Ser Glu Glu Gly Glu Ile Val Asp Leu Leu Leu
    2405                2410                2415

Met Lys Ile Leu Ser Phe Leu Gly Ile Lys Ser Lys Arg Glu Glu
    2420                2425                2430

Pro Gly Ser Ser Arg Glu Gln Pro Gly Ser Leu Ser Gln Thr Arg
    2435                2440                2445

His Ser Arg Pro Ala Gln Ala Leu Pro Lys Asp
    2450                2455

<210>  119
<211>  3737
<212>  DNA
<213>  human

<220>
<221>  CDS
<222>  (81)..(3080)

<400>  119
ggcgtccgcg cacacctccc cgcgccgccg ccgccaccgc ccgcactccg ccgcctctgc      60

ccgcaaccgc tgagccatcc atg ggg gtc gcg ggc cgc aac cgt ccc ggg gcg      113
                      Met Gly Val Ala Gly Arg Asn Arg Pro Gly Ala
                      1               5                   10

gcc tgg gcg gtg ctg ctg ctg ctg ctg ctg ctg ccg cca ctg ctg ctg      161
Ala Trp Ala Val Leu Leu Leu Leu Leu Leu Leu Pro Pro Leu Leu Leu
            15                  20                  25

ctg gcg ggg gcc gtc ccg ccg ggt cgg ggc cgt gcc gcg ggg ccg cag      209
Leu Ala Gly Ala Val Pro Pro Gly Arg Gly Arg Ala Ala Gly Pro Gln
        30                  35                  40

gag gat gta gat gag tgt gcc caa ggg cta gat gac tgc cat gcc gac      257
Glu Asp Val Asp Glu Cys Ala Gln Gly Leu Asp Asp Cys His Ala Asp
    45                  50                  55

gcc ctg tgt cag aac aca ccc acc tcc tac aag tgc tcc tgc aag cct      305
Ala Leu Cys Gln Asn Thr Pro Thr Ser Tyr Lys Cys Ser Cys Lys Pro
60                  65                  70                  75

ggc tac caa ggg gaa ggc agg cag tgt gag gac atc gat gaa tgt gga      353
Gly Tyr Gln Gly Glu Gly Arg Gln Cys Glu Asp Ile Asp Glu Cys Gly
                80                  85                  90

aat gag ctc aat gga ggc tgt gtc cat gac tgt ttg aat att cca ggc      401
Asn Glu Leu Asn Gly Gly Cys Val His Asp Cys Leu Asn Ile Pro Gly
            95                  100                 105

aat tat cgt tgc act tgt ttt gat ggc ttc atg ttg gct cat gac ggt      449
Asn Tyr Arg Cys Thr Cys Phe Asp Gly Phe Met Leu Ala His Asp Gly
        110                 115                 120

cat aat tgt ctt gat gtg gac gag tgc ctg gag aac aat ggc ggc tgc      497
His Asn Cys Leu Asp Val Asp Glu Cys Leu Glu Asn Asn Gly Gly Cys
    125                 130                 135

cag cat acc tgt gtc aac gtc atg ggg agc tat gag tgc tgc tgc aag      545
Gln His Thr Cys Val Asn Val Met Gly Ser Tyr Glu Cys Cys Cys Lys
140                 145                 150                 155

gag ggg ttt ttc ctg agt gac aat cag cac acc tgc att cac cgc tcg      593
Glu Gly Phe Phe Leu Ser Asp Asn Gln His Thr Cys Ile His Arg Ser
                160                 165                 170

gaa gag ggc ctg agc tgc atg aat aag gat cac ggc tgt agt cac atc      641

Glu Glu Gly Leu Ser Cys Met Asn Lys Asp His Gly Cys Ser His Ile
            175                 180                 185

tgc aag gag gcc cca agg ggc agc gtc gcc tgt gag tgc agg cct ggt 689
Cys Lys Glu Ala Pro Arg Gly Ser Val Ala Cys Glu Cys Arg Pro Gly
        190                 195                 200

ttt gag ctg gcc aag aac cag aga gac tgc atc ttg acc tgt aac cat 737
Phe Glu Leu Ala Lys Asn Gln Arg Asp Cys Ile Leu Thr Cys Asn His
    205                 210                 215

ggg aac ggt ggg tgc cag cac tcc tgt gac gat aca gcc gat ggc cca 785
Gly Asn Gly Gly Cys Gln His Ser Cys Asp Asp Thr Ala Asp Gly Pro
220                 225                 230                 235

gag tgc agc tgc cat cca cag tac aag atg cac aca gat ggg agg agc 833
Glu Cys Ser Cys His Pro Gln Tyr Lys Met His Thr Asp Gly Arg Ser
                240                 245                 250

tgc ctt gag cga gag gac act gtc ctg gag gtg aca gag agc aac acc 881
Cys Leu Glu Arg Glu Asp Thr Val Leu Glu Val Thr Glu Ser Asn Thr
            255                 260                 265

aca tca gtg gtg gat ggg gat aaa cgg gtg aaa cgg cgg ctg ctc atg 929
Thr Ser Val Val Asp Gly Asp Lys Arg Val Lys Arg Arg Leu Leu Met
        270                 275                 280

gaa acg tgt gct gtc aac aat gga ggc tgt gac cgc acc tgt aag gat 977
Glu Thr Cys Ala Val Asn Asn Gly Gly Cys Asp Arg Thr Cys Lys Asp
    285                 290                 295

act tcg aca ggt gtc cac tgc agt tgt cct gtt gga ttc act ctc cag 1025
Thr Ser Thr Gly Val His Cys Ser Cys Pro Val Gly Phe Thr Leu Gln
300                 305                 310                 315

ttg gat ggg aag aca tgt aaa gat att gat gag tgc cag acc cgc aat 1073
Leu Asp Gly Lys Thr Cys Lys Asp Ile Asp Glu Cys Gln Thr Arg Asn
                320                 325                 330

gga ggt tgt gat cat ttc tgc aaa aac atc gtg ggc agt ttt gac tgc 1121
Gly Gly Cys Asp His Phe Cys Lys Asn Ile Val Gly Ser Phe Asp Cys
            335                 340                 345

ggc tgc aag aaa gga ttt aaa tta tta aca gat gag aag tct tgc caa 1169
Gly Cys Lys Lys Gly Phe Lys Leu Leu Thr Asp Glu Lys Ser Cys Gln
        350                 355                 360

gat gtg gat gag tgc tct ttg gat agg acc tgt gac cac agc tgc atc 1217
Asp Val Asp Glu Cys Ser Leu Asp Arg Thr Cys Asp His Ser Cys Ile
    365                 370                 375

aac cac cct ggc aca ttt gct tgt gct tgc aac cga ggg tac acc ctg 1265
Asn His Pro Gly Thr Phe Ala Cys Ala Cys Asn Arg Gly Tyr Thr Leu
380                 385                 390                 395

tat ggc ttc acc cac tgt gga gac acc aat gag tgc agc atc aac aac 1313
Tyr Gly Phe Thr His Cys Gly Asp Thr Asn Glu Cys Ser Ile Asn Asn
                400                 405                 410

gga ggc tgt cag cag gtc tgt gtg aac aca gtg ggc agc tat gaa tgc 1361
Gly Gly Cys Gln Gln Val Cys Val Asn Thr Val Gly Ser Tyr Glu Cys
            415                 420                 425

cag tgc cac cct ggg tac aag ctc cac tgg aat aaa aaa gac tgt gtg 1409
Gln Cys His Pro Gly Tyr Lys Leu His Trp Asn Lys Lys Asp Cys Val
        430                 435                 440

gaa gtg aag ggg ctc ctg ccc aca agt gtg tca ccc cgt gtg tcc ctg 1457

Glu Val Lys Gly Leu Leu Pro Thr Ser Val Ser Pro Arg Val Ser Leu
    445                 450                 455

cac tgc ggt aag agt ggt gga gga gac ggg tgc ttc ctc aga tgt cac      1505
His Cys Gly Lys Ser Gly Gly Gly Asp Gly Cys Phe Leu Arg Cys His
460                 465                 470                 475

tct ggc att cac ctc tct tca gat gtc acc acc atc agg aca agt gta      1553
Ser Gly Ile His Leu Ser Ser Asp Val Thr Thr Ile Arg Thr Ser Val
                480                 485                 490

acc ttt aag cta aat gaa ggc aag tgt agt ttg aaa aat gct gag ctg      1601
Thr Phe Lys Leu Asn Glu Gly Lys Cys Ser Leu Lys Asn Ala Glu Leu
            495                 500                 505

ttt ccc gag ggt ctg cga cca gca cta cca gag aag cac agc tca gta      1649
Phe Pro Glu Gly Leu Arg Pro Ala Leu Pro Glu Lys His Ser Ser Val
        510                 515                 520

aaa gag agc ttc cgc tac gta aac ctt aca tgc agc tct ggc aag caa      1697
Lys Glu Ser Phe Arg Tyr Val Asn Leu Thr Cys Ser Ser Gly Lys Gln
    525                 530                 535

gtc cca gga gcc cct ggc cga cca agc acc cct aag gaa atg ttt atc      1745
Val Pro Gly Ala Pro Gly Arg Pro Ser Thr Pro Lys Glu Met Phe Ile
540                 545                 550                 555

act gtt gag ttt gag ctt gaa act aac caa aag gag gtg aca gct tct      1793
Thr Val Glu Phe Glu Leu Glu Thr Asn Gln Lys Glu Val Thr Ala Ser
                560                 565                 570

tgt gac ctg agc tgc atc gta aag cga acc gag aag cgg ctc cgt aaa      1841
Cys Asp Leu Ser Cys Ile Val Lys Arg Thr Glu Lys Arg Leu Arg Lys
            575                 580                 585

gcc atc cgc acg ctc aga aag gcc gtc cac agg gag cag ttt cac ctc      1889
Ala Ile Arg Thr Leu Arg Lys Ala Val His Arg Glu Gln Phe His Leu
        590                 595                 600

cag ctc tca ggc atg aac ctc gac gtg gct aaa aag cct ccc aga aca      1937
Gln Leu Ser Gly Met Asn Leu Asp Val Ala Lys Lys Pro Pro Arg Thr
    605                 610                 615

tct gaa cgc cag gca gag tcc tgt gga gtg ggc cag ggt cat gca gaa      1985
Ser Glu Arg Gln Ala Glu Ser Cys Gly Val Gly Gln Gly His Ala Glu
620                 625                 630                 635

aac caa tgt gtc agt tgc agg gct ggg acc tat tat gat gga gca cga      2033
Asn Gln Cys Val Ser Cys Arg Ala Gly Thr Tyr Tyr Asp Gly Ala Arg
                640                 645                 650

gaa cgc tgc att tta tgt cca aat gga acc ttc caa aat gag gaa gga      2081
Glu Arg Cys Ile Leu Cys Pro Asn Gly Thr Phe Gln Asn Glu Glu Gly
            655                 660                 665

caa atg act tgt gaa cca tgc cca aga cca gga aat tct ggg gcc ctg      2129
Gln Met Thr Cys Glu Pro Cys Pro Arg Pro Gly Asn Ser Gly Ala Leu
        670                 675                 680

aag acc cca gaa gct tgg aat atg tct gaa tgt gga ggt ctg tgt caa      2177
Lys Thr Pro Glu Ala Trp Asn Met Ser Glu Cys Gly Gly Leu Cys Gln
    685                 690                 695

cct ggt gaa tat tct gca gat ggc ttt gca cct tgc cag ctc tgt gcc      2225
Pro Gly Glu Tyr Ser Ala Asp Gly Phe Ala Pro Cys Gln Leu Cys Ala
700                 705                 710                 715

ctg ggc acg ttc cag cct gaa gct ggt cga act tcc tgc ttc ccc tgt      2273

Leu Gly Thr Phe Gln Pro Glu Ala Gly Arg Thr Ser Cys Phe Pro Cys
                720                 725                 730

gga gga ggc ctt gcc acc aaa cat cag gga gct act tcc ttt cag gac 2321
Gly Gly Gly Leu Ala Thr Lys His Gln Gly Ala Thr Ser Phe Gln Asp
            735                 740                 745

tgt gaa acc aga gtt caa tgt tca cct gga cat ttc tac aac acc acc 2369
Cys Glu Thr Arg Val Gln Cys Ser Pro Gly His Phe Tyr Asn Thr Thr
        750                 755                 760

act cac cga tgt att cgt tgc cca gtg gga aca tac cag cct gaa ttt 2417
Thr His Arg Cys Ile Arg Cys Pro Val Gly Thr Tyr Gln Pro Glu Phe
    765                 770                 775

gga aaa aat aat tgt gtt tct tgc cca gga aat act acg act gac ttt 2465
Gly Lys Asn Asn Cys Val Ser Cys Pro Gly Asn Thr Thr Thr Asp Phe
780                 785                 790                 795

gat ggc tcc aca aac ata acc cag tgt aaa aac aga aga tgt gga ggg 2513
Asp Gly Ser Thr Asn Ile Thr Gln Cys Lys Asn Arg Arg Cys Gly Gly
                800                 805                 810

gag ctg gga gat ttc act ggg tac att gaa tcc cca aac tac cca ggc 2561
Glu Leu Gly Asp Phe Thr Gly Tyr Ile Glu Ser Pro Asn Tyr Pro Gly
            815                 820                 825

aat tac cca gcc aac acc gag tgt acg tgg acc atc aac cca ccc ccc 2609
Asn Tyr Pro Ala Asn Thr Glu Cys Thr Trp Thr Ile Asn Pro Pro Pro
        830                 835                 840

aag cgc cgc atc ctg atc gtg gtc cct gag atc ttc ctg ccc ata gag 2657
Lys Arg Arg Ile Leu Ile Val Val Pro Glu Ile Phe Leu Pro Ile Glu
    845                 850                 855

gac gac tgt ggg gac tat ctg gtg atg cgg aaa acc tct tca tcc aat 2705
Asp Asp Cys Gly Asp Tyr Leu Val Met Arg Lys Thr Ser Ser Ser Asn
860                 865                 870                 875

tct gtg aca aca tat gaa acc tgc cag acc tac gaa cgc ccc atc gcc 2753
Ser Val Thr Thr Tyr Glu Thr Cys Gln Thr Tyr Glu Arg Pro Ile Ala
                880                 885                 890

ttc acc tcc agg tca aag aag ctg tgg att cag ttc aag tcc aat gaa 2801
Phe Thr Ser Arg Ser Lys Lys Leu Trp Ile Gln Phe Lys Ser Asn Glu
            895                 900                 905

ggg aac agc gct aga ggg ttc cag gtc cca tac gtg aca tat gat gag 2849
Gly Asn Ser Ala Arg Gly Phe Gln Val Pro Tyr Val Thr Tyr Asp Glu
        910                 915                 920

gac tac cag gaa ctc att gaa gac ata gtt cga gat ggc agg ctc tat 2897
Asp Tyr Gln Glu Leu Ile Glu Asp Ile Val Arg Asp Gly Arg Leu Tyr
    925                 930                 935

gca tct gag aac cat cag gaa ata ctt aag gat aag aaa ctt atc aag 2945
Ala Ser Glu Asn His Gln Glu Ile Leu Lys Asp Lys Lys Leu Ile Lys
940                 945                 950                 955

gct ctg ttt gat gtc ctg gcc cat ccc cag aac tat ttc aag tac aca 2993
Ala Leu Phe Asp Val Leu Ala His Pro Gln Asn Tyr Phe Lys Tyr Thr
                960                 965                 970

gcc cag gag tcc cga gag atg ttt cca aga tcg ttc atc cga ttg cta 3041
Ala Gln Glu Ser Arg Glu Met Phe Pro Arg Ser Phe Ile Arg Leu Leu
            975                 980                 985

cgt tcc aaa gtg tcc agg ttt ttg aga cct tac aaa tga ctcagcccac 3090

Arg Ser Lys Val Ser Arg Phe Leu Arg Pro Tyr Lys
        990                 995

gtgccactca atacaaatgt tctgctatag ggttggtggg acagagctgt cttccttctg    3150

catgtcagca cagtcgggta ttgctgcctc ccgtatcagt gactcattag agttcaattt    3210

ttatagataa tacagatatt ttggtaaatt gaacttggtt tttctttccc agcatcgtgg    3270

atgtagactg agaatggctt tgagtggcat cagcttctca ctgctgtggg cggatgtctt    3330

ggatagatca cgggctggct gagctggact ttggtcagcc taggtgagac tcacctgtcc    3390

ttctggggtc ttactcctcc tcaaggagtc tgtagtggaa aggaggccac agaataagct    3450

gcttattctg aaacttcagc ttcctctagc ccggccctct ctaagggagc cctctgcact    3510

cgtgtgcagg ctctgaccag gcagaacagg caagagggga gggaaggaga cccctgcagg    3570

ctccctccac ccaccttgag acctgggagg actcagtttc tccacagcct tctccagcct    3630

gtgtgataca agtttgatcc caggaacttg agttctaagc agtgctcgtg aaaaaaaaaa    3690

gcagaaagaa ttagaaataa ataaaaacta agcacttctg gagacat                  3737

<210>  120
<211>  999
<212>  PRT
<213>  human

<400>  120

Met Gly Val Ala Gly Arg Asn Arg Pro Gly Ala Ala Trp Ala Val Leu
1               5                   10                  15

Leu Leu Leu Leu Leu Leu Pro Pro Leu Leu Leu Leu Ala Gly Ala Val
            20                  25                  30

Pro Pro Gly Arg Gly Arg Ala Ala Gly Pro Gln Glu Asp Val Asp Glu
        35                  40                  45

Cys Ala Gln Gly Leu Asp Asp Cys His Ala Asp Ala Leu Cys Gln Asn
    50                  55                  60

Thr Pro Thr Ser Tyr Lys Cys Ser Cys Lys Pro Gly Tyr Gln Gly Glu
65                  70                  75                  80

Gly Arg Gln Cys Glu Asp Ile Asp Glu Cys Gly Asn Glu Leu Asn Gly
                85                  90                  95

Gly Cys Val His Asp Cys Leu Asn Ile Pro Gly Asn Tyr Arg Cys Thr
            100                 105                 110

Cys Phe Asp Gly Phe Met Leu Ala His Asp Gly His Asn Cys Leu Asp
        115                 120                 125

Val Asp Glu Cys Leu Glu Asn Asn Gly Gly Cys Gln His Thr Cys Val
    130                 135                 140

Asn Val Met Gly Ser Tyr Glu Cys Cys Cys Lys Glu Gly Phe Phe Leu
145                 150                 155                 160

Ser Asp Asn Gln His Thr Cys Ile His Arg Ser Glu Glu Gly Leu Ser
                165                 170                 175

Cys Met Asn Lys Asp His Gly Cys Ser His Ile Cys Lys Glu Ala Pro
            180                 185                 190

Arg Gly Ser Val Ala Cys Glu Cys Arg Pro Gly Phe Glu Leu Ala Lys
        195                 200                 205

Asn Gln Arg Asp Cys Ile Leu Thr Cys Asn His Gly Asn Gly Gly Cys
    210                 215                 220

Gln His Ser Cys Asp Asp Thr Ala Asp Gly Pro Glu Cys Ser Cys His
225                 230                 235                 240

Pro Gln Tyr Lys Met His Thr Asp Gly Arg Ser Cys Leu Glu Arg Glu
                245                 250                 255

Asp Thr Val Leu Glu Val Thr Glu Ser Asn Thr Thr Ser Val Val Asp
            260                 265                 270

Gly Asp Lys Arg Val Lys Arg Arg Leu Leu Met Glu Thr Cys Ala Val
        275                 280                 285

Asn Asn Gly Gly Cys Asp Arg Thr Cys Lys Asp Thr Ser Thr Gly Val
    290                 295                 300

His Cys Ser Cys Pro Val Gly Phe Thr Leu Gln Leu Asp Gly Lys Thr
305                 310                 315                 320

Cys Lys Asp Ile Asp Glu Cys Gln Thr Arg Asn Gly Gly Cys Asp His
                325                 330                 335

Phe Cys Lys Asn Ile Val Gly Ser Phe Asp Cys Gly Cys Lys Lys Gly
            340                 345                 350

Phe Lys Leu Leu Thr Asp Glu Lys Ser Cys Gln Asp Val Asp Glu Cys
        355                 360                 365

Ser Leu Asp Arg Thr Cys Asp His Ser Cys Ile Asn His Pro Gly Thr
    370                 375                 380

Phe Ala Cys Ala Cys Asn Arg Gly Tyr Thr Leu Tyr Gly Phe Thr His
385                 390                 395                 400

Cys Gly Asp Thr Asn Glu Cys Ser Ile Asn Asn Gly Gly Cys Gln Gln
                405                 410                 415

Val Cys Val Asn Thr Val Gly Ser Tyr Glu Cys Gln Cys His Pro Gly
            420                 425                 430

Tyr Lys Leu His Trp Asn Lys Lys Asp Cys Val Glu Val Lys Gly Leu
        435                 440                 445

Leu Pro Thr Ser Val Ser Pro Arg Val Ser Leu His Cys Gly Lys Ser
    450                 455                 460

Gly Gly Gly Asp Gly Cys Phe Leu Arg Cys His Ser Gly Ile His Leu
465                 470                 475                 480

Ser Ser Asp Val Thr Thr Ile Arg Thr Ser Val Thr Phe Lys Leu Asn
                485                 490                 495

Glu Gly Lys Cys Ser Leu Lys Asn Ala Glu Leu Phe Pro Glu Gly Leu
            500                 505                 510

Arg Pro Ala Leu Pro Glu Lys His Ser Ser Val Lys Glu Ser Phe Arg
        515                 520                 525

Tyr Val Asn Leu Thr Cys Ser Ser Gly Lys Gln Val Pro Gly Ala Pro
    530                 535                 540

Gly Arg Pro Ser Thr Pro Lys Glu Met Phe Ile Thr Val Glu Phe Glu
545                 550                 555                 560

Leu Glu Thr Asn Gln Lys Glu Val Thr Ala Ser Cys Asp Leu Ser Cys
                565                 570                 575

Ile Val Lys Arg Thr Glu Lys Arg Leu Arg Lys Ala Ile Arg Thr Leu
            580                 585                 590

Arg Lys Ala Val His Arg Glu Gln Phe His Leu Gln Leu Ser Gly Met
        595                 600                 605

Asn Leu Asp Val Ala Lys Lys Pro Pro Arg Thr Ser Glu Arg Gln Ala
    610                 615                 620

Glu Ser Cys Gly Val Gly Gln Gly His Ala Glu Asn Gln Cys Val Ser
625                 630                 635                 640

Cys Arg Ala Gly Thr Tyr Tyr Asp Gly Ala Arg Glu Arg Cys Ile Leu
                645                 650                 655

Cys Pro Asn Gly Thr Phe Gln Asn Glu Glu Gly Gln Met Thr Cys Glu
            660                 665                 670

Pro Cys Pro Arg Pro Gly Asn Ser Gly Ala Leu Lys Thr Pro Glu Ala
        675                 680                 685

Trp Asn Met Ser Glu Cys Gly Gly Leu Cys Gln Pro Gly Glu Tyr Ser
    690                 695                 700

Ala Asp Gly Phe Ala Pro Cys Gln Leu Cys Ala Leu Gly Thr Phe Gln
705                 710                 715                 720

Pro Glu Ala Gly Arg Thr Ser Cys Phe Pro Cys Gly Gly Gly Leu Ala
                725                 730                 735

Thr Lys His Gln Gly Ala Thr Ser Phe Gln Asp Cys Glu Thr Arg Val
            740                 745                 750

Gln Cys Ser Pro Gly His Phe Tyr Asn Thr Thr Thr His Arg Cys Ile
        755                 760                 765

Arg Cys Pro Val Gly Thr Tyr Gln Pro Glu Phe Gly Lys Asn Asn Cys
    770                 775                 780

Val Ser Cys Pro Gly Asn Thr Thr Thr Asp Phe Asp Gly Ser Thr Asn
785                 790                 795                 800

Ile Thr Gln Cys Lys Asn Arg Arg Cys Gly Gly Glu Leu Gly Asp Phe
                805                 810                 815

Thr Gly Tyr Ile Glu Ser Pro Asn Tyr Pro Gly Asn Tyr Pro Ala Asn
            820                 825                 830

Thr Glu Cys Thr Trp Thr Ile Asn Pro Pro Pro Lys Arg Arg Ile Leu
        835                 840                 845

Ile Val Val Pro Glu Ile Phe Leu Pro Ile Glu Asp Asp Cys Gly Asp
    850                 855                 860

Tyr Leu Val Met Arg Lys Thr Ser Ser Ser Asn Ser Val Thr Thr Tyr
865                 870                 875                 880

Glu Thr Cys Gln Thr Tyr Glu Arg Pro Ile Ala Phe Thr Ser Arg Ser
                885                 890                 895

Lys Lys Leu Trp Ile Gln Phe Lys Ser Asn Glu Gly Asn Ser Ala Arg
            900                 905                 910

Gly Phe Gln Val Pro Tyr Val Thr Tyr Asp Glu Asp Tyr Gln Glu Leu
        915                 920                 925

Ile Glu Asp Ile Val Arg Asp Gly Arg Leu Tyr Ala Ser Glu Asn His
    930                 935                 940

Gln Glu Ile Leu Lys Asp Lys Lys Leu Ile Lys Ala Leu Phe Asp Val
945                 950                 955                 960

Leu Ala His Pro Gln Asn Tyr Phe Lys Tyr Thr Ala Gln Glu Ser Arg
                965                 970                 975

Glu Met Phe Pro Arg Ser Phe Ile Arg Leu Leu Arg Ser Lys Val Ser
            980                 985                 990

Arg Phe Leu Arg Pro Tyr Lys
        995

<210>  121
<211>  2469
<212>  DNA
<213>  human

<220>
<221>  CDS
<222>  (100)..(1296)

<400>  121
gaactaaggg aggcagggct tgtcttctct cctcagagca gagtctgcga tcttcccagc      60

acagacgttt ggacagagca ggctcctaag gtctccaga atg ccc gtg cca gcc        114
                                           Met Pro Val Pro Ala
                                           1               5

tcc tgg ccc cac ctt cct agt cct ttc ctg ctg atg acg cta ctg ctg       162
Ser Trp Pro His Leu Pro Ser Pro Phe Leu Leu Met Thr Leu Leu Leu
                10                  15                  20

ggg aga ctc aca gga gtg gca ggt gag gac gag cta cag gtg att cag       210
Gly Arg Leu Thr Gly Val Ala Gly Glu Asp Glu Leu Gln Val Ile Gln
            25                  30                  35

cct gaa aag tcc gta tca gtt gca gct gga gag tcg gcc act ctg cgc       258
Pro Glu Lys Ser Val Ser Val Ala Ala Gly Glu Ser Ala Thr Leu Arg
        40                  45                  50

tgt gct atg acg tcc ctg atc cct gtg ggg ccc atc atg tgg ttt aga       306
Cys Ala Met Thr Ser Leu Ile Pro Val Gly Pro Ile Met Trp Phe Arg
    55                  60                  65

gga gct gga gca ggc cgg gaa tta atc tac aat cag aaa gaa ggc cac       354
Gly Ala Gly Ala Gly Arg Glu Leu Ile Tyr Asn Gln Lys Glu Gly His
70                  75                  80                  85

ttc cca cgg gta aca act gtt tca gaa ctc aca aag aga aac aac ctg       402
Phe Pro Arg Val Thr Thr Val Ser Glu Leu Thr Lys Arg Asn Asn Leu
                90                  95                  100

gac ttt tcc atc agc atc agt aac atc acc cca gca gac gcc ggc acc       450
Asp Phe Ser Ile Ser Ile Ser Asn Ile Thr Pro Ala Asp Ala Gly Thr
            105                 110                 115

tac tac tgt gtg aag ttc cgg aaa ggg agc cct gac gac gtg gag ttt       498
Tyr Tyr Cys Val Lys Phe Arg Lys Gly Ser Pro Asp Asp Val Glu Phe
        120                 125                 130

aag tct gga gca ggc act gag ctg tct gtg cgc gcc aaa ccc tct gcc       546
Lys Ser Gly Ala Gly Thr Glu Leu Ser Val Arg Ala Lys Pro Ser Ala
    135                 140                 145

ccc gtg gta tcg ggc cct gcg gtg agg gcc aca cct gag cac aca gtg       594
Pro Val Val Ser Gly Pro Ala Val Arg Ala Thr Pro Glu His Thr Val

150                 155                 160                 165

agc ttc acc tgc gag tcc cat ggc ttc tct ccc aga gac atc acc ctg      642
Ser Phe Thr Cys Glu Ser His Gly Phe Ser Pro Arg Asp Ile Thr Leu
                170                 175                 180

aaa tgg ttc aaa aat ggg aat gag ctc tca gac ttc cag acc aac gtg      690
Lys Trp Phe Lys Asn Gly Asn Glu Leu Ser Asp Phe Gln Thr Asn Val
            185                 190                 195

gac ccc gca gga gac agt gtg tcc tac agc atc cac agc aca gcc agg      738
Asp Pro Ala Gly Asp Ser Val Ser Tyr Ser Ile His Ser Thr Ala Arg
        200                 205                 210

gtg gtg ctg acc cgt ggg gac gtt cac tct caa gtc atc tgc gag ata      786
Val Val Leu Thr Arg Gly Asp Val His Ser Gln Val Ile Cys Glu Ile
    215                 220                 225

gcc cac atc acc ttg cag ggg gac cct ctt cgt ggg act gcc aac ttg      834
Ala His Ile Thr Leu Gln Gly Asp Pro Leu Arg Gly Thr Ala Asn Leu
230                 235                 240                 245

tct gag gcc atc cga gtt cca ccc acc ttg gag gtt act caa cag ccc      882
Ser Glu Ala Ile Arg Val Pro Pro Thr Leu Glu Val Thr Gln Gln Pro
                250                 255                 260

atg agg gca gag aac cag gca aac gtc acc tgc cag gtg agc aat ttc      930
Met Arg Ala Glu Asn Gln Ala Asn Val Thr Cys Gln Val Ser Asn Phe
            265                 270                 275

tac ccc cgg gga cta cag ctg acc tgg ttg gag aat gga aat gtg tcc      978
Tyr Pro Arg Gly Leu Gln Leu Thr Trp Leu Glu Asn Gly Asn Val Ser
        280                 285                 290

cgg aca gaa aca gct tcg acc ctc ata gag aac aag gat ggc acc tac      1026
Arg Thr Glu Thr Ala Ser Thr Leu Ile Glu Asn Lys Asp Gly Thr Tyr
    295                 300                 305

aac tgg atg agc tgg ctc ctg gtg aac acc tgt gcc cac agg gac gat      1074
Asn Trp Met Ser Trp Leu Leu Val Asn Thr Cys Ala His Arg Asp Asp
310                 315                 320                 325

gtg gtg ctc acc tgt cag gtg gag cat gat ggg cag caa gca gtc agc      1122
Val Val Leu Thr Cys Gln Val Glu His Asp Gly Gln Gln Ala Val Ser
                330                 335                 340

aaa agc tat gcc ctg gag atc tca gcg cac cag aag gag cac ggc tca      1170
Lys Ser Tyr Ala Leu Glu Ile Ser Ala His Gln Lys Glu His Gly Ser
            345                 350                 355

gat atc acc cat gaa gca gcg ctg gct cct act gct cca ctc ctc gta      1218
Asp Ile Thr His Glu Ala Ala Leu Ala Pro Thr Ala Pro Leu Leu Val
        360                 365                 370

gct ctc ctc ctg ggc ccc aag ctg cta ctg gtg gtt ggt gtc tct gcc      1266
Ala Leu Leu Leu Gly Pro Lys Leu Leu Leu Val Val Gly Val Ser Ala
    375                 380                 385

atc tac atc tgc tgg aaa cag aag gcc tga ctgaccctca gtctctgctg      1316
Ile Tyr Ile Cys Trp Lys Gln Lys Ala
390                 395

cctcctcctt tcttgagaag ctcagcctga gagaaggagc tggcgagaac cttccccaca      1376

ctcagctcca aacgcctcct ctcccaggtc atctgcctgc ccacacgctc ctgttccacc      1436

ttcacaagac catgatgccc caaagcagtg tctctattca cggtcctgag caggggccat      1496

gggattgggc tctgggcact gactcatggc acctccctag aaggtgagaa acactccaaa 1556
tctaaacaca ccaggacttc tcccatccgt cgccttggga ctggccataa accacagact 1616
ctctccaggc tctcaagagt tatcctgtct tctggattcc tgcctacccc aactccccca 1676
gccttgttga ggttctctac tgcctcctga atacacatga acccctatac caattttaag 1736
aaaaaaatga ttctctttcc tctttgtcca agcatcctat ccctcaaacc caaaaagaaa 1796
gaagctctcc cttctctctc tgtgatggag acagtatttc ttctagtatc ctgcagcctt 1856
cccagtcctg ctgcttgtgg tagaaatgct gccacagccc aacattgagg agccctcgat 1916
gactgccctt tacaactcat attcagttct gcctccaaaa tgcatgtgtc cacttacatg 1976
agatggtaaa tgtttaacaa tggactttct gaaagggaaa aaccaaaagc tgttttgcag 2036
tgcttgccaa tttctctagt gtaataactc ccaacctgac caatttcaca ctgccaacag 2096
ttaaacaacc agattgcaag attcctgaaa tttaacaatt ggttttcagg gcccagtcca 2156
agcctgctgc tggaaacctc agagttaaat ccctattctc cacacctctc acctccacca 2216
cccctccctg tcccagccag catcatctct ttggggacca ctcctctggc tttcattttt 2276
cagccacagt gattctttgg aaaagtcaaa tcatatcact tctctgcttc ttccccaaca 2336
cagctgcatg gctcccgctc tccctccttc aagtctctgc tcaatgtcac ttcattaaag 2396
gcaccttcta taaactaaaa aaaaaaaaaa aaaaaaaaaa aaaaaaaaaa aaaaaaaaaa 2456
aaaaaaaaaa aaa 2469

<210> 122
<211> 398
<212> PRT
<213> human

<400> 122

Met Pro Val Pro Ala Ser Trp Pro His Leu Pro Ser Pro Phe Leu Leu
1               5                   10                  15

Met Thr Leu Leu Leu Gly Arg Leu Thr Gly Val Ala Gly Glu Asp Glu
            20                  25                  30

Leu Gln Val Ile Gln Pro Glu Lys Ser Val Ser Val Ala Ala Gly Glu
        35                  40                  45

Ser Ala Thr Leu Arg Cys Ala Met Thr Ser Leu Ile Pro Val Gly Pro
    50                  55                  60

Ile Met Trp Phe Arg Gly Ala Gly Ala Gly Arg Glu Leu Ile Tyr Asn
65                  70                  75                  80

Gln Lys Glu Gly His Phe Pro Arg Val Thr Thr Val Ser Glu Leu Thr
                85                  90                  95

Lys Arg Asn Asn Leu Asp Phe Ser Ile Ser Ile Ser Asn Ile Thr Pro
            100                 105                 110

Ala Asp Ala Gly Thr Tyr Tyr Cys Val Lys Phe Arg Lys Gly Ser Pro
        115                 120                 125

Asp Asp Val Glu Phe Lys Ser Gly Ala Gly Thr Glu Leu Ser Val Arg
    130                 135                 140

Ala Lys Pro Ser Ala Pro Val Val Ser Gly Pro Ala Val Arg Ala Thr
145                 150                 155                 160

Pro Glu His Thr Val Ser Phe Thr Cys Glu Ser His Gly Phe Ser Pro
                165                 170                 175

Arg Asp Ile Thr Leu Lys Trp Phe Lys Asn Gly Asn Glu Leu Ser Asp
            180                 185                 190

Phe Gln Thr Asn Val Asp Pro Ala Gly Asp Ser Val Ser Tyr Ser Ile
        195                 200                 205

His Ser Thr Ala Arg Val Val Leu Thr Arg Gly Asp Val His Ser Gln
    210                 215                 220

Val Ile Cys Glu Ile Ala His Ile Thr Leu Gln Gly Asp Pro Leu Arg
225                 230                 235                 240

Gly Thr Ala Asn Leu Ser Glu Ala Ile Arg Val Pro Pro Thr Leu Glu
                245                 250                 255

Val Thr Gln Gln Pro Met Arg Ala Glu Asn Gln Ala Asn Val Thr Cys
            260                 265                 270

Gln Val Ser Asn Phe Tyr Pro Arg Gly Leu Gln Leu Thr Trp Leu Glu
        275                 280                 285

Asn Gly Asn Val Ser Arg Thr Glu Thr Ala Ser Thr Leu Ile Glu Asn
    290                 295                 300

Lys Asp Gly Thr Tyr Asn Trp Met Ser Trp Leu Leu Val Asn Thr Cys
305                 310                 315                 320

Ala His Arg Asp Asp Val Val Leu Thr Cys Gln Val Glu His Asp Gly
                325                 330                 335

Gln Gln Ala Val Ser Lys Ser Tyr Ala Leu Glu Ile Ser Ala His Gln
            340                 345                 350

Lys Glu His Gly Ser Asp Ile Thr His Glu Ala Ala Leu Ala Pro Thr
        355                 360                 365

Ala Pro Leu Leu Val Ala Leu Leu Leu Gly Pro Lys Leu Leu Leu Val
    370                 375                 380

Val Gly Val Ser Ala Ile Tyr Ile Cys Trp Lys Gln Lys Ala
385                 390                 395

<210>  123
<211>  1818
<212>  DNA
<213>  human

<220>
<221>  CDS
<222>  (100)..(645)

<400>  123
gaactaaggg aggcagggct tgtcttctct cctcagagca gagtctgcga tcttcccagc      60

acagacgttt ggacagagca ggctcctaag gtctccaga atg ccc gtg cca gcc        114
                                           Met Pro Val Pro Ala
                                           1               5

tcc tgg ccc cac ctt cct agt cct ttc ctg ctg atg acg cta ctg ctg       162
Ser Trp Pro His Leu Pro Ser Pro Phe Leu Leu Met Thr Leu Leu Leu
                10                  15                  20

ggg aga ctc aca gga gtg gca ggt gag gac gag cta cag gtg att cag       210
Gly Arg Leu Thr Gly Val Ala Gly Glu Asp Glu Leu Gln Val Ile Gln
                25                  30                  35

cct gaa aag tcc gta tca gtt gca gct gga gag tcg gcc act ctg cgc       258
Pro Glu Lys Ser Val Ser Val Ala Ala Gly Glu Ser Ala Thr Leu Arg
        40                  45                  50

tgt gct atg acg tcc ctg atc cct gtg ggg ccc atc atg tgg ttt aga       306
Cys Ala Met Thr Ser Leu Ile Pro Val Gly Pro Ile Met Trp Phe Arg
    55                  60                  65

gga gct gga gca ggc cgg gaa tta atc tac aat cag aaa gaa ggc cac       354
Gly Ala Gly Ala Gly Arg Glu Leu Ile Tyr Asn Gln Lys Glu Gly His
70                  75                  80                  85

ttc cca cgg gta aca act gtt tca gaa ctc aca aag aga aac aac ctg       402
Phe Pro Arg Val Thr Thr Val Ser Glu Leu Thr Lys Arg Asn Asn Leu
                90                  95                  100

gac ttt tcc atc agc atc agt aac atc acc cca gca gac gcc ggc acc       450
Asp Phe Ser Ile Ser Ile Ser Asn Ile Thr Pro Ala Asp Ala Gly Thr
            105                 110                 115

tac tac tgt gtg aag ttc cgg aaa ggg agc cct gac gac gtg gag ttt       498
Tyr Tyr Cys Val Lys Phe Arg Lys Gly Ser Pro Asp Asp Val Glu Phe
        120                 125                 130

aag tct gga gca ggc act gag ctg tct gtg cgc gaa gca gcg ctg gct       546
Lys Ser Gly Ala Gly Thr Glu Leu Ser Val Arg Glu Ala Ala Leu Ala
    135                 140                 145

cct act gct cca ctc ctc gta gct ctc ctc ctg ggc ccc aag ctg cta       594
Pro Thr Ala Pro Leu Leu Val Ala Leu Leu Leu Gly Pro Lys Leu Leu
150                 155                 160                 165

ctg gtg gtt ggt gtc tct gcc atc tac atc tgc tgg aaa cag aag gcc       642
Leu Val Val Gly Val Ser Ala Ile Tyr Ile Cys Trp Lys Gln Lys Ala
                170                 175                 180

tga ctgaccctca gtctctgctg cctcctcctt tcttgagaag ctcagcctga            695

gagaaggagc tggcgagaac cttccccaca ctcagctcca aacgcctcct ctcccaggtc 755
atctgcctgc ccacacgctc ctgttccacc ttcacaagac catgatgccc caaagcagtg 815
tctctattca cggtcctgag caggggccat gggattgggc tctgggcact gactcatggc 875
acctccctag aaggtgagaa acactccaaa tctaaacaca ccaggacttc tcccatccgt 935
cgccttggga ctggccataa accacagact ctctccaggc tctcaagagt tatcctgtct 995
tctggattcc tgcctacccc aactccccca gccttgttga ggttctctac tgcctcctga 1055
atacacatga acccctatac caattttaag aaaaaaatga ttctctttcc tctttgtcca 1115
agcatcctat ccctcaaacc caaaaagaaa gaagctctcc cttctctctc tgtgatggag 1175
acagtatttc ttctagtatc ctgcagcctt cccagtcctg ctgcttgtgg tagaaatgct 1235
gccacagccc aacattgagg agccctcgat gactgccctt tacaactcat attcagttct 1295
gcctccaaaa tgcatgtgtc cacttacatg agatggtaaa tgtttaacaa tggactttct 1355
gaaagggaaa aaccaaaagc tgttttgcag tgcttgccaa tttctctagt gtaataactc 1415
ccaacctgac caatttcaca ctgccaacag ttaaacaacc agattgcaag attcctgaaa 1475
tttaacaatt ggttttcagg gcccagtcca agcctgctgc tggaaacctc agagttaaat 1535
ccctattctc cacacctctc acctccacca cccctccctg tcccagccag catcatctct 1595
ttggggacca ctcctctggc tttcattttt cagccacagt gattctttgg aaaagtcaaa 1655
tcatatcact tctctgcttc ttccccaaca cagctgcatg gctcccgctc tccctccttc 1715
aagtctctgc tcaatgtcac ttcattaaag gcaccttcta taaactaaaa aaaaaaaaaa 1775
aaaaaaaaaa aaaaaaaaaa aaaaaaaaaa aaaaaaaaaa aaa 1818

<210> 124
<211> 181
<212> PRT
<213> human

<400> 124

Met Pro Val Pro Ala Ser Trp Pro His Leu Pro Ser Pro Phe Leu Leu
1               5                   10                  15

Met Thr Leu Leu Leu Gly Arg Leu Thr Gly Val Ala Gly Glu Asp Glu
            20                  25                  30

Leu Gln Val Ile Gln Pro Glu Lys Ser Val Ser Val Ala Ala Gly Glu
        35                  40                  45

Ser Ala Thr Leu Arg Cys Ala Met Thr Ser Leu Ile Pro Val Gly Pro
    50                  55                  60

Ile Met Trp Phe Arg Gly Ala Gly Ala Gly Arg Glu Leu Ile Tyr Asn
65                  70                  75                  80

Gln Lys Glu Gly His Phe Pro Arg Val Thr Thr Val Ser Glu Leu Thr

85                  90                  95

Lys Arg Asn Asn Leu Asp Phe Ser Ile Ser Ile Ser Asn Ile Thr Pro
            100                 105                 110

Ala Asp Ala Gly Thr Tyr Tyr Cys Val Lys Phe Arg Lys Gly Ser Pro
        115                 120                 125

Asp Asp Val Glu Phe Lys Ser Gly Ala Gly Thr Glu Leu Ser Val Arg
    130                 135                 140

Glu Ala Ala Leu Ala Pro Thr Ala Pro Leu Leu Val Ala Leu Leu Leu
145                 150                 155                 160

Gly Pro Lys Leu Leu Leu Val Val Gly Val Ser Ala Ile Tyr Ile Cys
                165                 170                 175

Trp Lys Gln Lys Ala
            180

<210>  125
<211>  2241
<212>  DNA
<213>  human

<220>
<221>  CDS
<222>  (100)..(1296)

<400>  125
gaactaaggg aggcagggct tgtcttctct cctcagagca gagtctgcga tcttcccagc      60

acagacgttt ggacagagca ggctcctaag gtctccaga atg ccc gtg cca gcc        114
                                           Met Pro Val Pro Ala
                                           1               5

tcc tgg ccc cac ctt cct agt cct ttc ctg ctg atg acg cta ctg ctg        162
Ser Trp Pro His Leu Pro Ser Pro Phe Leu Leu Met Thr Leu Leu Leu
                10                  15                  20

ggg aga ctc aca ggg gta gct ggc gag gaa gag ctg cag gtg att cag        210
Gly Arg Leu Thr Gly Val Ala Gly Glu Glu Glu Leu Gln Val Ile Gln
            25                  30                  35

cct gac aag tcc ata tca gtt gca gct gga gag tcg gcc act ctg cac        258
Pro Asp Lys Ser Ile Ser Val Ala Ala Gly Glu Ser Ala Thr Leu His
        40                  45                  50

tgc act gtg act tcc ctg atc cct gtg ggg ccc atc cag tgg ttc aga        306
Cys Thr Val Thr Ser Leu Ile Pro Val Gly Pro Ile Gln Trp Phe Arg
    55                  60                  65

gga gct gga cca ggc cgg gaa tta atc tac aat cag aaa gaa ggc cac        354
Gly Ala Gly Pro Gly Arg Glu Leu Ile Tyr Asn Gln Lys Glu Gly His
70                  75                  80                  85

ttc cca cgg gta aca act gtt tca gac ctc aca aag aga aac aac atg        402
Phe Pro Arg Val Thr Thr Val Ser Asp Leu Thr Lys Arg Asn Asn Met
                90                  95                  100

gac ttt tcc atc cgc atc agt aac atc acc cca gca gat gcc ggc acc 450
Asp Phe Ser Ile Arg Ile Ser Asn Ile Thr Pro Ala Asp Ala Gly Thr
            105                 110                 115

tac tac tgt gtg aag ttc cgg aaa ggg agc ccc gac cac gtg gag ttt 498
Tyr Tyr Cys Val Lys Phe Arg Lys Gly Ser Pro Asp His Val Glu Phe
        120                 125                 130

aag tct gga gca ggc acc gag ctg tct gtg cgt gcc aaa ccc tct gcc 546
Lys Ser Gly Ala Gly Thr Glu Leu Ser Val Arg Ala Lys Pro Ser Ala
    135                 140                 145

ccc gtg gta tcg ggc cct gcg gcg agg gcc aca cct cag cac aca gtg 594
Pro Val Val Ser Gly Pro Ala Ala Arg Ala Thr Pro Gln His Thr Val
150                 155                 160                 165

agc ttc acc tgc gag tcc cac ggc ttc tca ccc aga gac atc acc ctg 642
Ser Phe Thr Cys Glu Ser His Gly Phe Ser Pro Arg Asp Ile Thr Leu
                170                 175                 180

aaa tgg ttc aaa aat ggg aat gag ctc tca gac ttc cag acc aac gtg 690
Lys Trp Phe Lys Asn Gly Asn Glu Leu Ser Asp Phe Gln Thr Asn Val
            185                 190                 195

gac ccc gca gga gac agt gtg tcc tac agc atc cac agc aca gcc aag 738
Asp Pro Ala Gly Asp Ser Val Ser Tyr Ser Ile His Ser Thr Ala Lys
        200                 205                 210

gtg gtg ctg acc cgc gag gac gtt cac tct caa gtc atc tgc gag gtg 786
Val Val Leu Thr Arg Glu Asp Val His Ser Gln Val Ile Cys Glu Val
    215                 220                 225

gcc cac gtc acc ttg cag ggg gac cct ctt cgt ggg act gcc aac ttg 834
Ala His Val Thr Leu Gln Gly Asp Pro Leu Arg Gly Thr Ala Asn Leu
230                 235                 240                 245

tct gag acc atc cga gtt cca ccc acc ttg gag gtt act caa cag ccc 882
Ser Glu Thr Ile Arg Val Pro Pro Thr Leu Glu Val Thr Gln Gln Pro
                250                 255                 260

gtg agg gca gag aac cag gtg aat gtc acc tgc cag gtg agg aag ttc 930
Val Arg Ala Glu Asn Gln Val Asn Val Thr Cys Gln Val Arg Lys Phe
            265                 270                 275

tac ccc cag aga cta cag ctg acc tgg ttg gag aat gga aac gtg tcc 978
Tyr Pro Gln Arg Leu Gln Leu Thr Trp Leu Glu Asn Gly Asn Val Ser
        280                 285                 290

agg aca gaa acg gcc tca acc ctt aca gaa aac aag gat ggt acc tac 1026
Arg Thr Glu Thr Ala Ser Thr Leu Thr Glu Asn Lys Asp Gly Thr Tyr
    295                 300                 305

aac tgg atg agc tgg ctc ctg gtg aat gta tct gcc cac agg gat gat 1074
Asn Trp Met Ser Trp Leu Leu Val Asn Val Ser Ala His Arg Asp Asp
310                 315                 320                 325

gtg aag ctc acc tgc cag gtg gag cat gac ggg cag cca gcg gtc agc 1122
Val Lys Leu Thr Cys Gln Val Glu His Asp Gly Gln Pro Ala Val Ser
                330                 335                 340

aaa agc cat gac ctg aag gtc tca gcc cac ccg aag gag cag ggc tca 1170
Lys Ser His Asp Leu Lys Val Ser Ala His Pro Lys Glu Gln Gly Ser
            345                 350                 355

aat act gct cct ggc cca gca ctg gct tct gct gct cca ctt ctc ata 1218
Asn Thr Ala Pro Gly Pro Ala Leu Ala Ser Ala Ala Pro Leu Leu Ile
        360                 365                 370

gct ttc ctc ctg ggc ccc aag gtg ctg ctg gtg gtt ggt gtc tct gtc     1266
Ala Phe Leu Leu Gly Pro Lys Val Leu Leu Val Val Gly Val Ser Val
    375                 380                 385

atc tat gtc tac tgg aag cag aag gcc tga ctgcatccca gaaggagacc     1316
Ile Tyr Val Tyr Trp Lys Gln Lys Ala
390                 395

agcaccagga atacaacagc ctcccaggaa atgtttctta aatgatgagc agatgattaa     1376

atatggacat gttacccaca gctccttccc tcctcccctg ccacatggaa cccccagtct     1436

ctcctgccta cttccttcct tgaatggctc tgactgagag aaggggctgg tgagaagctt     1496

ccccagactg agctccaaac acctcccctc ccaggacatc ttcctgccca caggctcctg     1556

ttgctccttt gcagagtcct tgattctcca gagcagtctt cattcatggt cctgagcaga     1616

ggccatggga tggggctctg gggagtgact catgacacct ccctaggatg tgggaaacac     1676

gccaaatcta aacacatcag gacacctctc ctccatcgtc ttggactctc cgtaagccac     1736

aaactgcagt ccagattctc aagagttatt ctgccttctg gattcctgcc tacccaatta     1796

cccagccttg ttgagattct ctattgcctc ctgaatacaa attaacactc atcctggttt     1856

taaggagaaa aatgattatc cttcctcttt gtttatacgt atgatataat ttggatattt     1916

tttccctaca aatctcaatg tgaaatttca tcccctatgt tggaggtggg gtcaggtggg     1976

aggtgtttgg gtcatgggga tggattcatc atgaatgact tggtgttatc ttggtctatt     2036

agcacccaca agatctgatt gtttaaaagt gcctagcagc tccctcttct ctttttctct     2096

tcctctctcc ccatgttatg ccaactccac ttcactgtcc actgtgagca gaagcactct     2156

gaggccctca gcagcagatg caagtaccat gcttcttgta cagtctgcag aaccaaaagc     2216

caaataaacc tctctttata aatta                                       2241

<210>  126
<211>  398
<212>  PRT
<213>  human

<400>  126

Met Pro Val Pro Ala Ser Trp Pro His Leu Pro Ser Pro Phe Leu Leu
1               5                   10                  15

Met Thr Leu Leu Leu Gly Arg Leu Thr Gly Val Ala Gly Glu Glu Glu
            20                  25                  30

Leu Gln Val Ile Gln Pro Asp Lys Ser Ile Ser Val Ala Ala Gly Glu
        35                  40                  45

Ser Ala Thr Leu His Cys Thr Val Thr Ser Leu Ile Pro Val Gly Pro
    50                  55                  60

Ile Gln Trp Phe Arg Gly Ala Gly Pro Gly Arg Glu Leu Ile Tyr Asn
65                  70                  75                  80

Gln Lys Glu Gly His Phe Pro Arg Val Thr Thr Val Ser Asp Leu Thr
                85                  90                  95

Lys Arg Asn Asn Met Asp Phe Ser Ile Arg Ile Ser Asn Ile Thr Pro
            100                 105                 110

Ala Asp Ala Gly Thr Tyr Tyr Cys Val Lys Phe Arg Lys Gly Ser Pro
        115                 120                 125

Asp His Val Glu Phe Lys Ser Gly Ala Gly Thr Glu Leu Ser Val Arg
    130                 135                 140

Ala Lys Pro Ser Ala Pro Val Val Ser Gly Pro Ala Ala Arg Ala Thr
145                 150                 155                 160

Pro Gln His Thr Val Ser Phe Thr Cys Glu Ser His Gly Phe Ser Pro
                165                 170                 175

Arg Asp Ile Thr Leu Lys Trp Phe Lys Asn Gly Asn Glu Leu Ser Asp
            180                 185                 190

Phe Gln Thr Asn Val Asp Pro Ala Gly Asp Ser Val Ser Tyr Ser Ile
        195                 200                 205

His Ser Thr Ala Lys Val Val Leu Thr Arg Glu Asp Val His Ser Gln
    210                 215                 220

Val Ile Cys Glu Val Ala His Val Thr Leu Gln Gly Asp Pro Leu Arg
225                 230                 235                 240

Gly Thr Ala Asn Leu Ser Glu Thr Ile Arg Val Pro Pro Thr Leu Glu
                245                 250                 255

Val Thr Gln Gln Pro Val Arg Ala Glu Asn Gln Val Asn Val Thr Cys
            260                 265                 270

Gln Val Arg Lys Phe Tyr Pro Gln Arg Leu Gln Leu Thr Trp Leu Glu
        275                 280                 285

Asn Gly Asn Val Ser Arg Thr Glu Thr Ala Ser Thr Leu Thr Glu Asn
    290                 295                 300

Lys Asp Gly Thr Tyr Asn Trp Met Ser Trp Leu Leu Val Asn Val Ser
305                 310                 315                 320

Ala His Arg Asp Asp Val Lys Leu Thr Cys Gln Val Glu His Asp Gly
                325                 330                 335

Gln Pro Ala Val Ser Lys Ser His Asp Leu Lys Val Ser Ala His Pro
            340                 345                 350

Lys Glu Gln Gly Ser Asn Thr Ala Pro Gly Pro Ala Leu Ala Ser Ala
        355                 360                 365

Ala Pro Leu Leu Ile Ala Phe Leu Leu Gly Pro Lys Val Leu Leu Val
    370                 375                 380

Val Gly Val Ser Val Ile Tyr Val Tyr Trp Lys Gln Lys Ala
385                 390                 395

<210> 127
<211> 4199
<212> DNA
<213> human

<220>
<221> CDS
<222> (460)..(2985)

<400> 127
agaaaaggag gaaggagaac attgctgtag cttggatcta caacctaaga aagcaagagt 60

gatcaatctc agctctgtta aacatcttgt ttacttactg cattcagcag cttgcaaatg 120

gttaactata tgcaaaaaag tcagcatagc tgtgaagtat gccgtgaatt ttaattgagg 180

gaaaaaggga caattgcttc aggatgctct agtatgcact ctgcttgaaa tattttcaat 240

gaaatgctca gtattctatc tttgaccaga ggttttaact ttatgaagct atgggacttg 300

acaaaaagtg atatttgaga agaaagtacg cagtggttgg tgttttcttt tttttaataa 360

aggaattgaa ttactttgaa cacctcttcc agctgtgcat tacagataac gtcaggaaga 420

gtctctgctt tacagaatcg gatttcatca catgacaac atg aag ctg tgg att 474
Met Lys Leu Trp Ile
1               5

cat ctc ttt tat tca tct ctc ctt gcc tgt ata tct tta cac tcc caa 522
His Leu Phe Tyr Ser Ser Leu Leu Ala Cys Ile Ser Leu His Ser Gln
                10                  15                  20

act cca gtg ctc tca tcc aga ggc tct tgt gat tct ctt tgc aat tgt 570
Thr Pro Val Leu Ser Ser Arg Gly Ser Cys Asp Ser Leu Cys Asn Cys
            25                  30                  35

gag gaa aaa gat ggc aca atg cta ata aat tgt gaa gca aaa ggt atc 618
Glu Glu Lys Asp Gly Thr Met Leu Ile Asn Cys Glu Ala Lys Gly Ile
        40                  45                  50

aag atg gta tct gaa ata agt gtg cca cca tca cga cct ttc caa cta 666
Lys Met Val Ser Glu Ile Ser Val Pro Pro Ser Arg Pro Phe Gln Leu
    55                  60                  65

agc tta tta aat aac ggc ttg acg atg ctt cac aca aat gac ttt tct 714
Ser Leu Leu Asn Asn Gly Leu Thr Met Leu His Thr Asn Asp Phe Ser
70                  75                  80                  85

ggg ctt acc aat gct att tca ata cac ctt gga ttt aac aat att gca 762
Gly Leu Thr Asn Ala Ile Ser Ile His Leu Gly Phe Asn Asn Ile Ala
                90                  95                  100

gat att gag ata ggt gca ttt aat ggc ctt ggc ctc ctg aaa caa ctt 810
Asp Ile Glu Ile Gly Ala Phe Asn Gly Leu Gly Leu Leu Lys Gln Leu
            105                 110                 115

cat atc aat cac aat tct tta gaa att ctt aaa gag gat act ttc cat 858
His Ile Asn His Asn Ser Leu Glu Ile Leu Lys Glu Asp Thr Phe His
        120                 125                 130

gga ctg gaa aac ctg gaa ttc ctg caa gca gat aac aat ttt atc aca 906
Gly Leu Glu Asn Leu Glu Phe Leu Gln Ala Asp Asn Asn Phe Ile Thr
    135                 140                 145

gtg att gaa cca agt gcc ttt agc aag ctc aac aga ctc aaa gtg tta 954
Val Ile Glu Pro Ser Ala Phe Ser Lys Leu Asn Arg Leu Lys Val Leu
150                 155                 160                 165

att tta aat gac aat gct att gag agt ctt cct cca aac atc ttc cga 1002
Ile Leu Asn Asp Asn Ala Ile Glu Ser Leu Pro Pro Asn Ile Phe Arg
                170                 175                 180

ttt gtt cct tta acc cat cta gat ctt cgt gga aat caa tta caa aca 1050
Phe Val Pro Leu Thr His Leu Asp Leu Arg Gly Asn Gln Leu Gln Thr
            185                 190                 195

ttg cct tat gtt ggt ttt ctc gaa cac att ggc cga ata ttg gat ctt 1098
Leu Pro Tyr Val Gly Phe Leu Glu His Ile Gly Arg Ile Leu Asp Leu
        200                 205                 210

cag ttg gag gac aac aaa tgg gcc tgc aat tgt gac tta ttg cag tta 1146
Gln Leu Glu Asp Asn Lys Trp Ala Cys Asn Cys Asp Leu Leu Gln Leu
    215                 220                 225

aaa act tgg ttg gag aac atg cct cca cag tct ata att ggt gat gtt 1194
Lys Thr Trp Leu Glu Asn Met Pro Pro Gln Ser Ile Ile Gly Asp Val
230                 235                 240                 245

gtc tgc aac agc cct cca ttt ttt aaa gga agt ata ctc agt aga cta 1242
Val Cys Asn Ser Pro Pro Phe Phe Lys Gly Ser Ile Leu Ser Arg Leu
                250                 255                 260

aag aag gaa tct att tgc cct act cca cca gtg tat gaa gaa cat gag 1290
Lys Lys Glu Ser Ile Cys Pro Thr Pro Pro Val Tyr Glu Glu His Glu
            265                 270                 275

gat cct tca gga tca tta cat ctg gca gca aca tct tca ata aat gat 1338
Asp Pro Ser Gly Ser Leu His Leu Ala Ala Thr Ser Ser Ile Asn Asp
        280                 285                 290

agt cgc atg tca act aag acc acg tcc att cta aaa cta ccc acc aaa 1386
Ser Arg Met Ser Thr Lys Thr Thr Ser Ile Leu Lys Leu Pro Thr Lys
    295                 300                 305

gca cca ggt ttg ata cct tat att aca aag cca tcc act caa ctt cca 1434
Ala Pro Gly Leu Ile Pro Tyr Ile Thr Lys Pro Ser Thr Gln Leu Pro
310                 315                 320                 325

gga cct tac tgc cct att cct tgt aac tgc aaa gtc cta tcc cca tca 1482
Gly Pro Tyr Cys Pro Ile Pro Cys Asn Cys Lys Val Leu Ser Pro Ser
                330                 335                 340

gga ctt cta ata cat tgt cag gag cgc aac att gaa agc tta tca gat 1530
Gly Leu Leu Ile His Cys Gln Glu Arg Asn Ile Glu Ser Leu Ser Asp
            345                 350                 355

ctg aga cct cct ccg caa aat cct aga aag ctc att cta gcg gga aat 1578
Leu Arg Pro Pro Pro Gln Asn Pro Arg Lys Leu Ile Leu Ala Gly Asn
        360                 365                 370

att att cac agt tta atg aag tct gat cta gtg gaa tat ttc act ttg 1626
Ile Ile His Ser Leu Met Lys Ser Asp Leu Val Glu Tyr Phe Thr Leu
    375                 380                 385

gaa atg ctt cac ttg gga aac aat cgt att gaa gtt ctt gaa gaa gga 1674
Glu Met Leu His Leu Gly Asn Asn Arg Ile Glu Val Leu Glu Glu Gly
390                 395                 400                 405

tcg ttt atg aac cta acg aga tta caa aaa ctc tat cta aat ggt aac 1722
Ser Phe Met Asn Leu Thr Arg Leu Gln Lys Leu Tyr Leu Asn Gly Asn
                410                 415                 420

cac ctg acc aaa tta agt aaa ggc atg ttc ctt ggt ctc cat aat ctt 1770
His Leu Thr Lys Leu Ser Lys Gly Met Phe Leu Gly Leu His Asn Leu
            425                 430                 435

gaa tac tta tat ctt gaa tac aat gcc att aag gaa ata ctg cca gga 1818
Glu Tyr Leu Tyr Leu Glu Tyr Asn Ala Ile Lys Glu Ile Leu Pro Gly
        440                 445                 450

acc ttt aat cca atg cct aaa ctt aaa gtc ctg tat tta aat aac aac 1866
Thr Phe Asn Pro Met Pro Lys Leu Lys Val Leu Tyr Leu Asn Asn Asn
    455                 460                 465

ctc ctc caa gtt tta cca cca cat att ttt tca ggg gtt cct cta act 1914
Leu Leu Gln Val Leu Pro Pro His Ile Phe Ser Gly Val Pro Leu Thr
470                 475                 480                 485

aag gta aat ctt aaa aca aac cag ttt acc cat cta cct gta agt aat 1962
Lys Val Asn Leu Lys Thr Asn Gln Phe Thr His Leu Pro Val Ser Asn
                490                 495                 500

att ttg gat gat ctt gat ttg cta acc cag att gac ctt gag gat aac 2010
Ile Leu Asp Asp Leu Asp Leu Leu Thr Gln Ile Asp Leu Glu Asp Asn
            505                 510                 515

ccc tgg gac tgc tcc tgt gac ctg gtt gga ctg cag caa tgg ata caa 2058
Pro Trp Asp Cys Ser Cys Asp Leu Val Gly Leu Gln Gln Trp Ile Gln
        520                 525                 530

aag tta agc aag aac aca gtg aca gat gac atc ctc tgc act tcc ccc 2106
Lys Leu Ser Lys Asn Thr Val Thr Asp Asp Ile Leu Cys Thr Ser Pro
    535                 540                 545

ggg cat ctc gac aaa aag gaa ttg aaa gcc cta aat agt gaa att ctc 2154
Gly His Leu Asp Lys Lys Glu Leu Lys Ala Leu Asn Ser Glu Ile Leu
550                 555                 560                 565

tgt cca ggt tta gta aat aac cca tcc atg cca aca cag act agt tac 2202
Cys Pro Gly Leu Val Asn Asn Pro Ser Met Pro Thr Gln Thr Ser Tyr
                570                 575                 580

ctt atg gtc acc act cct gca aca aca aca aat acg gct gat act att 2250
Leu Met Val Thr Thr Pro Ala Thr Thr Thr Asn Thr Ala Asp Thr Ile
            585                 590                 595

tta cga tct ctt acg gac gct gtg cca ctg tct gtt cta ata ttg gga 2298
Leu Arg Ser Leu Thr Asp Ala Val Pro Leu Ser Val Leu Ile Leu Gly
        600                 605                 610

ctt ctg att atg ttc atc act att gtt ttc tgt gct gca ggg ata gtg 2346
Leu Leu Ile Met Phe Ile Thr Ile Val Phe Cys Ala Ala Gly Ile Val
    615                 620                 625

gtt ctt gtt ctt cac cgc agg aga aga tac aaa aag aaa caa gta gat 2394
Val Leu Val Leu His Arg Arg Arg Arg Tyr Lys Lys Lys Gln Val Asp
630                 635                 640                 645

gag caa atg aga gac aac agt cct gtg cat ctt cag tac agc atg tat 2442
Glu Gln Met Arg Asp Asn Ser Pro Val His Leu Gln Tyr Ser Met Tyr
                650                 655                 660

ggc cat aaa acc act cat cac act act gaa aga ccc tct gcc tca ctc 2490
Gly His Lys Thr Thr His His Thr Thr Glu Arg Pro Ser Ala Ser Leu
            665                 670                 675

tat gaa cag cac atg gtg agc ccc atg gtt cat gtc tat aga agt cca 2538
Tyr Glu Gln His Met Val Ser Pro Met Val His Val Tyr Arg Ser Pro
        680                 685                 690

tcc ttt ggt cca aag cat ctg gaa gag gaa gaa gag agg aat gag aaa 2586
Ser Phe Gly Pro Lys His Leu Glu Glu Glu Glu Glu Arg Asn Glu Lys
    695                 700                 705

gaa gga agt gat gca aaa cat ctc caa aga agt ctt ttg gaa cag gaa 2634
Glu Gly Ser Asp Ala Lys His Leu Gln Arg Ser Leu Leu Glu Gln Glu
710                 715                 720                 725

aat cat tca cca ctc aca ggg tca aat atg aaa tac aaa acc acg aac 2682
Asn His Ser Pro Leu Thr Gly Ser Asn Met Lys Tyr Lys Thr Thr Asn
                730                 735                 740

caa tca aca gaa ttt tta tcc ttc caa gat gcc agc tca ttg tac aga 2730
Gln Ser Thr Glu Phe Leu Ser Phe Gln Asp Ala Ser Ser Leu Tyr Arg
            745                 750                 755

aac att tta gaa aaa gaa agg gaa ctt cag caa ctg gga atc aca gaa 2778
Asn Ile Leu Glu Lys Glu Arg Glu Leu Gln Gln Leu Gly Ile Thr Glu
        760                 765                 770

tac cta agg aaa aac att gct cag ctc cag cct gat atg gag gca cat 2826
Tyr Leu Arg Lys Asn Ile Ala Gln Leu Gln Pro Asp Met Glu Ala His
    775                 780                 785

tat cct gga gcc cac gaa gag ctg aag tta atg gaa aca tta atg tac 2874
Tyr Pro Gly Ala His Glu Glu Leu Lys Leu Met Glu Thr Leu Met Tyr
790                 795                 800                 805

tca cgt cca agg aag gta tta gtg gaa cag aca aaa aat gag tat ttt 2922
Ser Arg Pro Arg Lys Val Leu Val Glu Gln Thr Lys Asn Glu Tyr Phe
                810                 815                 820

gaa ctt aaa gct aat tta cat gct gaa cct gac tat tta gaa gtc ctg 2970
Glu Leu Lys Ala Asn Leu His Ala Glu Pro Asp Tyr Leu Glu Val Leu
            825                 830                 835

gag cag caa aca tag atggagagtt tgagggcttt cgcagaaatg ctgtgattct 3025
Glu Gln Gln Thr
        840

gttttaagtc cataccttgt aaataagtgc cttacgtgag tgtgtcatca atcagaacct 3085

aagcacagca gtaaactatg gggaaaaaaa aagaagaaga aaaagaaact cagggatcac 3145

tgggagaagc catggcatta tcttcaggca atttagtctg tcccaaataa aataaatcct 3205

tgcatgtaaa tcattcaagg attatagtaa tatttcatat actgaaaagt gtctcatagg 3265

agtcctcttg cacatctaaa aaggctgaac atttaagtat cccgaatttt cttgaattgc 3325

tttccctata gattaattac aattggattt catcatttaa aaaccatact tgtatatgta 3385

gttataatat gtaaggaata cattgtttat aaccagtatg tacttcaaaa atgtgtattg 3445

tcaaacatac ctaactttct tgcaataaat gcaaaagaaa ctggaacttg acaattataa 3505

atagtaatag tgaagaaaaa atagaaaggt tgcaattata taggccatgg gtggctcaaa 3565

actttgaaca tttgagctta aacaaatgcc actctcatgc attctaaatt aaaaagttaa 3625

aatgattaat agttcaggtg gaagaaataa gcatactttt tgggttttct acacattttg 3685

tgtagacaat tttaatgtca gtgctgctgt gaactaaagt atgtcattta tgctcaaagt 3745

ttaattcttc ttcttgggat attttaaaaa tgctactgag attctgctgt aaatatgact 3805

agagaatata ttgggtttgc tttatttcat aggcttaatt ctttgtaaat ctgaatgacc 3865

ataatagaaa tacatttctt gtggcaagta attcacagtt gtaaagtaaa taggaaaaat 3925

tattttattt ttattgatgt acattgatag atgccataaa tcagtagcaa aaggcacttc 3985

taaaggtaag tggtttaagt tgcctcaaga gagggacaat gtagctttat tttacaagaa 4045

ggcatagtta gatttctatg aaatatttat tctgtacagt tttatatagt tttggttcac 4105

aaaagtaatt attcttgggt gcctttcaag aaaattaaaa atactactca ctacaataaa 4165

actaaaatga aaactcaaaa aaaaaaaaaa aaaa 4199

<210> 128
<211> 841
<212> PRT
<213> human

<400> 128

Met Lys Leu Trp Ile His Leu Phe Tyr Ser Ser Leu Leu Ala Cys Ile
1               5                   10                  15

Ser Leu His Ser Gln Thr Pro Val Leu Ser Ser Arg Gly Ser Cys Asp
            20                  25                  30

Ser Leu Cys Asn Cys Glu Glu Lys Asp Gly Thr Met Leu Ile Asn Cys
        35                  40                  45

Glu Ala Lys Gly Ile Lys Met Val Ser Glu Ile Ser Val Pro Pro Ser
    50                  55                  60

Arg Pro Phe Gln Leu Ser Leu Leu Asn Asn Gly Leu Thr Met Leu His
65                  70                  75                  80

Thr Asn Asp Phe Ser Gly Leu Thr Asn Ala Ile Ser Ile His Leu Gly
                85                  90                  95

Phe Asn Asn Ile Ala Asp Ile Glu Ile Gly Ala Phe Asn Gly Leu Gly
            100                 105                 110

Leu Leu Lys Gln Leu His Ile Asn His Asn Ser Leu Glu Ile Leu Lys
        115                 120                 125

Glu Asp Thr Phe His Gly Leu Glu Asn Leu Glu Phe Leu Gln Ala Asp
    130                 135                 140

Asn Asn Phe Ile Thr Val Ile Glu Pro Ser Ala Phe Ser Lys Leu Asn
145                 150                 155                 160

Arg Leu Lys Val Leu Ile Leu Asn Asp Asn Ala Ile Glu Ser Leu Pro
                165                 170                 175

Pro Asn Ile Phe Arg Phe Val Pro Leu Thr His Leu Asp Leu Arg Gly
            180                 185                 190

Asn Gln Leu Gln Thr Leu Pro Tyr Val Gly Phe Leu Glu His Ile Gly
        195                 200                 205

Arg Ile Leu Asp Leu Gln Leu Glu Asp Asn Lys Trp Ala Cys Asn Cys
    210                 215                 220

Asp Leu Leu Gln Leu Lys Thr Trp Leu Glu Asn Met Pro Pro Gln Ser
225                 230                 235                 240

Ile Ile Gly Asp Val Val Cys Asn Ser Pro Pro Phe Phe Lys Gly Ser
                245                 250                 255

Ile Leu Ser Arg Leu Lys Lys Glu Ser Ile Cys Pro Thr Pro Pro Val
            260                 265                 270

Tyr Glu Glu His Glu Asp Pro Ser Gly Ser Leu His Leu Ala Ala Thr
        275                 280                 285

Ser Ser Ile Asn Asp Ser Arg Met Ser Thr Lys Thr Thr Ser Ile Leu
    290                 295                 300

Lys Leu Pro Thr Lys Ala Pro Gly Leu Ile Pro Tyr Ile Thr Lys Pro
305                 310                 315                 320

Ser Thr Gln Leu Pro Gly Pro Tyr Cys Pro Ile Pro Cys Asn Cys Lys
                325                 330                 335

Val Leu Ser Pro Ser Gly Leu Leu Ile His Cys Gln Glu Arg Asn Ile
            340                 345                 350

Glu Ser Leu Ser Asp Leu Arg Pro Pro Pro Gln Asn Pro Arg Lys Leu
        355                 360                 365

Ile Leu Ala Gly Asn Ile Ile His Ser Leu Met Lys Ser Asp Leu Val
    370                 375                 380

Glu Tyr Phe Thr Leu Glu Met Leu His Leu Gly Asn Asn Arg Ile Glu
385                 390                 395                 400

Val Leu Glu Glu Gly Ser Phe Met Asn Leu Thr Arg Leu Gln Lys Leu
                405                 410                 415

Tyr Leu Asn Gly Asn His Leu Thr Lys Leu Ser Lys Gly Met Phe Leu
            420                 425                 430

Gly Leu His Asn Leu Glu Tyr Leu Tyr Leu Glu Tyr Asn Ala Ile Lys
        435                 440                 445

Glu Ile Leu Pro Gly Thr Phe Asn Pro Met Pro Lys Leu Lys Val Leu
    450                 455                 460

Tyr Leu Asn Asn Asn Leu Leu Gln Val Leu Pro Pro His Ile Phe Ser
465                 470                 475                 480

Gly Val Pro Leu Thr Lys Val Asn Leu Lys Thr Asn Gln Phe Thr His
                485                 490                 495

Leu Pro Val Ser Asn Ile Leu Asp Asp Leu Asp Leu Leu Thr Gln Ile
            500                 505                 510

Asp Leu Glu Asp Asn Pro Trp Asp Cys Ser Cys Asp Leu Val Gly Leu
        515                 520                 525

Gln Gln Trp Ile Gln Lys Leu Ser Lys Asn Thr Val Thr Asp Asp Ile
    530                 535                 540

Leu Cys Thr Ser Pro Gly His Leu Asp Lys Lys Glu Leu Lys Ala Leu
545                 550                 555                 560

Asn Ser Glu Ile Leu Cys Pro Gly Leu Val Asn Asn Pro Ser Met Pro
                565                 570                 575

Thr Gln Thr Ser Tyr Leu Met Val Thr Thr Pro Ala Thr Thr Thr Asn
            580                 585                 590

Thr Ala Asp Thr Ile Leu Arg Ser Leu Thr Asp Ala Val Pro Leu Ser
        595                 600                 605

Val Leu Ile Leu Gly Leu Leu Ile Met Phe Ile Thr Ile Val Phe Cys
    610                 615                 620

Ala Ala Gly Ile Val Val Leu Val Leu His Arg Arg Arg Arg Tyr Lys
625                 630                 635                 640

Lys Lys Gln Val Asp Glu Gln Met Arg Asp Asn Ser Pro Val His Leu
                645                 650                 655

Gln Tyr Ser Met Tyr Gly His Lys Thr Thr His His Thr Thr Glu Arg
            660                 665                 670

Pro Ser Ala Ser Leu Tyr Glu Gln His Met Val Ser Pro Met Val His
        675                 680                 685

Val Tyr Arg Ser Pro Ser Phe Gly Pro Lys His Leu Glu Glu Glu Glu
    690                 695                 700

Glu Arg Asn Glu Lys Glu Gly Ser Asp Ala Lys His Leu Gln Arg Ser
705                 710                 715                 720

Leu Leu Glu Gln Glu Asn His Ser Pro Leu Thr Gly Ser Asn Met Lys
                725                 730                 735

Tyr Lys Thr Thr Asn Gln Ser Thr Glu Phe Leu Ser Phe Gln Asp Ala
            740                 745                 750

Ser Ser Leu Tyr Arg Asn Ile Leu Glu Lys Glu Arg Glu Leu Gln Gln
        755                 760                 765

Leu Gly Ile Thr Glu Tyr Leu Arg Lys Asn Ile Ala Gln Leu Gln Pro
    770                 775                 780

Asp Met Glu Ala His Tyr Pro Gly Ala His Glu Glu Leu Lys Leu Met
785                 790                 795                 800

Glu Thr Leu Met Tyr Ser Arg Pro Arg Lys Val Leu Val Glu Gln Thr
                805                 810                 815

Lys Asn Glu Tyr Phe Glu Leu Lys Ala Asn Leu His Ala Glu Pro Asp
            820                 825                 830

Tyr Leu Glu Val Leu Glu Gln Gln Thr
        835                 840

<210>  129
<211>  1452
<212>  DNA
<213>  human

<220>
<221>  CDS
<222>  (118)..(1431)

<400>  129
   cgacttcacg cagggtccgt ggggtccccg cggcgcgcag cggctgaagg aggccccagg      60
   gccttggcga ccgcagcggc ggctttagcg tcagtgacta ggcagcaggg ggtcagg        117
   atg cgg cga agc tcc cgc ccg ggc tcg gcc tcg tcc tcg cgc aag cac        165
   Met Arg Arg Ser Ser Arg Pro Gly Ser Ala Ser Ser Ser Arg Lys His
   1               5                   10                  15
   acg ccc aac ttt ttc agc gag aac agc tca atg agc atc acc tcg gag        213
   Thr Pro Asn Phe Phe Ser Glu Asn Ser Ser Met Ser Ile Thr Ser Glu
               20                  25                  30
   gac agc aaa ggg ctc cgg tca gcg gag ccc ggg cct ggg gag ccc gag        261
   Asp Ser Lys Gly Leu Arg Ser Ala Glu Pro Gly Pro Gly Glu Pro Glu
           35                  40                  45
   ggc aga aga gcc cgg ggc ccg agc tgc ggt gag ccc gcc ttg agc gcg        309
   Gly Arg Arg Ala Arg Gly Pro Ser Cys Gly Glu Pro Ala Leu Ser Ala
       50                  55                  60
   gga gtg ccc gga gga acc aca tgg gca gga agc tct cag cag aag cca     357
   Gly Val Pro Gly Gly Thr Thr Trp Ala Gly Ser Ser Gln Gln Lys Pro
   65                  70                  75                  80
   gcg cct cgg agc cac aac tgg cag aca gcc tgt ggc gcg gca acc gtg     405
   Ala Pro Arg Ser His Asn Trp Gln Thr Ala Cys Gly Ala Ala Thr Val
                   85                  90                  95
   agg ggc ggg gcc tcg gaa ccg act ggg tct cca gta gtc tct gag gag     453
   Arg Gly Gly Ala Ser Glu Pro Thr Gly Ser Pro Val Val Ser Glu Glu
               100                 105                 110
   ccg ctc gac ctt ctc ccg acc ctg gat ctg agg cag gag atg cct ccc     501
   Pro Leu Asp Leu Leu Pro Thr Leu Asp Leu Arg Gln Glu Met Pro Pro
           115                 120                 125
   ccg cgg gtg ttc aag agc ttt ctg agc ctg ctc ttc cag ggg ctg agc     549
   Pro Arg Val Phe Lys Ser Phe Leu Ser Leu Leu Phe Gln Gly Leu Ser
       130                 135                 140
   gtg ttg tta tcc ctg gca gga gac gtg ctg gtc agc atg tac agg gag     597
   Val Leu Leu Ser Leu Ala Gly Asp Val Leu Val Ser Met Tyr Arg Glu
   145                 150                 155                 160
   gtc tgt tcc atc cgc ttc ctg ttc acg gct gtg tcg ctg ctg agc ctc     645
   Val Cys Ser Ile Arg Phe Leu Phe Thr Ala Val Ser Leu Leu Ser Leu
                   165                 170                 175
   ttt ctg tca gca ttc tgg ctg ggg ctt ctg tac ctg gtc tct cct ttg     693
   Phe Leu Ser Ala Phe Trp Leu Gly Leu Leu Tyr Leu Val Ser Pro Leu
               180                 185                 190
   gag aat gaa cct aag gag atg ctg act cta agt gag tac cac gag cgc     741
   Glu Asn Glu Pro Lys Glu Met Leu Thr Leu Ser Glu Tyr His Glu Arg
           195                 200                 205
   gtg cgc tcc cag ggg cag cag ctg cag cag ctc cag gcc gag ctg gat     789
   Val Arg Ser Gln Gly Gln Gln Leu Gln Gln Leu Gln Ala Glu Leu Asp
       210                 215                 220
   aaa ctc cac aag gag gtg tcc act gtt cgg gca gcc aac agc gag aga     837
   Lys Leu His Lys Glu Val Ser Thr Val Arg Ala Ala Asn Ser Glu Arg
   225                 230                 235                 240
   gtg gcc aag ctc gtg ttc cag agg ctg aat gag gat ttt gtg cgg aag     885
   Val Ala Lys Leu Val Phe Gln Arg Leu Asn Glu Asp Phe Val Arg Lys
                   245                 250                 255
   ccc gac tat gct ttg agc tct gtg gga gcc tcc atc gac ctg cag aag     933
   Pro Asp Tyr Ala Leu Ser Ser Val Gly Ala Ser Ile Asp Leu Gln Lys
               260                 265                 270
   aca tcc cac gat tac gca gac agg aac act gcc tac ttc tgg aat cgc     981
   Thr Ser His Asp Tyr Ala Asp Arg Asn Thr Ala Tyr Phe Trp Asn Arg
           275                 280                 285
   ttc agc ttc tgg aac tac gca cgg ccg ccc acg gtt atc ctg gag ccc     1029
   Phe Ser Phe Trp Asn Tyr Ala Arg Pro Pro Thr Val Ile Leu Glu Pro
       290                 295                 300
   cac gtg ttc cct ggg aat tgc tgg gct ttt gaa ggc gac caa ggc cag     1077
   His Val Phe Pro Gly Asn Cys Trp Ala Phe Glu Gly Asp Gln Gly Gln
   305                 310                 315                 320
   gtg gtg atc caa ctg ccg ggc cga gtg cag ctg agc gac atc act ctg     1125
   Val Val Ile Gln Leu Pro Gly Arg Val Gln Leu Ser Asp Ile Thr Leu
                   325                 330                 335
   cag cat cca ccg ccc agc gtg gag cac acc gga gga gcc aac agc gcc 1173
   Gln His Pro Pro Pro Ser Val Glu His Thr Gly Gly Ala Asn Ser Ala
   340 345 350
   ccc cgc gat ttc gcg gtc ttt ggc ctc cag gtt tat gat gaa act gaa 1221
   Pro Arg Asp Phe Ala Val Phe Gly Leu Gln Val Tyr Asp Glu Thr Glu
   355 360 365
   gtt tcc ttg ggg aaa ttc acc ttc gat gtt gag aaa tcg gag att cag 1269
   Val Ser Leu Gly Lys Phe Thr Phe Asp Val Glu Lys Ser Glu Ile Gln
   370 375 380
   act ttc cac ctg cag aat gac ccc cca gct gcc ttt ccc aag gtg aag 1317
   Thr Phe His Leu Gln Asn Asp Pro Pro Ala Ala Phe Pro Lys Val Lys
   385 390 395 400
   atc cag att cta agc aac tgg ggc cac ccc cgt ttc acg tgc ttg tat 1365
   Ile Gln Ile Leu Ser Asn Trp Gly His Pro Arg Phe Thr Cys Leu Tyr
   405 410 415
   cga gtc cgt gcc cac ggt gtg cga acc tca gag ggg gca gag ggc agt 1413
   Arg Val Arg Ala His Gly Val Arg Thr Ser Glu Gly Ala Glu Gly Ser
   420 425 430
   gca cag ggg ccc cat taa acatgctgat ttttggagta g                      1452
   Ala Gln Gly Pro His
           435

<210> 130
<211> 437
<212> PRT
<213> human

<400> 130

Met Arg Arg Ser Ser Arg Pro Gly Ser Ala Ser Ser Ser Arg Lys His
1               5                   10                  15

Thr Pro Asn Phe Phe Ser Glu Asn Ser Ser Met Ser Ile Thr Ser Glu
            20                  25                  30

Asp Ser Lys Gly Leu Arg Ser Ala Glu Pro Gly Pro Gly Glu Pro Glu
        35                  40                  45

Gly Arg Arg Ala Arg Gly Pro Ser Cys Gly Glu Pro Ala Leu Ser Ala
    50                  55                  60

Gly Val Pro Gly Gly Thr Thr Trp Ala Gly Ser Ser Gln Gln Lys Pro
65                  70                  75                  80

Ala Pro Arg Ser His Asn Trp Gln Thr Ala Cys Gly Ala Ala Thr Val
                85                  90                  95

Arg Gly Gly Ala Ser Glu Pro Thr Gly Ser Pro Val Val Ser Glu Glu
            100                 105                 110

Pro Leu Asp Leu Leu Pro Thr Leu Asp Leu Arg Gln Glu Met Pro Pro
        115                 120                 125

Pro Arg Val Phe Lys Ser Phe Leu Ser Leu Leu Phe Gln Gly Leu Ser
    130                 135                 140

Val Leu Leu Ser Leu Ala Gly Asp Val Leu Val Ser Met Tyr Arg Glu
145                 150                 155                 160

Val Cys Ser Ile Arg Phe Leu Phe Thr Ala Val Ser Leu Leu Ser Leu
                165                 170                 175

Phe Leu Ser Ala Phe Trp Leu Gly Leu Leu Tyr Leu Val Ser Pro Leu
            180                 185                 190

Glu Asn Glu Pro Lys Glu Met Leu Thr Leu Ser Glu Tyr His Glu Arg
        195                 200                 205

Val Arg Ser Gln Gly Gln Gln Leu Gln Gln Leu Gln Ala Glu Leu Asp
    210                 215                 220

Lys Leu His Lys Glu Val Ser Thr Val Arg Ala Ala Asn Ser Glu Arg
225                 230                 235                 240

Val Ala Lys Leu Val Phe Gln Arg Leu Asn Glu Asp Phe Val Arg Lys
                245                 250                 255

Pro Asp Tyr Ala Leu Ser Ser Val Gly Ala Ser Ile Asp Leu Gln Lys
            260                 265                 270

Thr Ser His Asp Tyr Ala Asp Arg Asn Thr Ala Tyr Phe Trp Asn Arg
        275                 280                 285

Phe Ser Phe Trp Asn Tyr Ala Arg Pro Pro Thr Val Ile Leu Glu Pro
    290                 295                 300

His Val Phe Pro Gly Asn Cys Trp Ala Phe Glu Gly Asp Gln Gly Gln
305                 310                 315                 320

Val Val Ile Gln Leu Pro Gly Arg Val Gln Leu Ser Asp Ile Thr Leu
                325                 330                 335

Gln His Pro Pro Pro Ser Val Glu His Thr Gly Gly Ala Asn Ser Ala
            340                 345                 350

Pro Arg Asp Phe Ala Val Phe Gly Leu Gln Val Tyr Asp Glu Thr Glu
        355                 360                 365

Val Ser Leu Gly Lys Phe Thr Phe Asp Val Glu Lys Ser Glu Ile Gln
    370                 375                 380

Thr Phe His Leu Gln Asn Asp Pro Pro Ala Ala Phe Pro Lys Val Lys
385                 390                 395                 400

Ile Gln Ile Leu Ser Asn Trp Gly His Pro Arg Phe Thr Cys Leu Tyr
                405                 410                 415

Arg Val Arg Ala His Gly Val Arg Thr Ser Glu Gly Ala Glu Gly Ser
            420                 425                 430

Ala Gln Gly Pro His
        435

<210>  131
<211>  24
<212>  DNA
<213>  artificial

<220>
<223>  primer

<400>  131
cgcuuauguu aauaguaaud tudt                                              24

<210>  132
<211>  23
<212>  DNA
<213>  artificial

<220>
<223>  primer

<400>  132
auuacuauua acauaagcgd adt                                               23

<210>  133
<211>  21
<212>  DNA
<213>  artificial

<220>
<223>  primer

<400>  133
atcgcttatg ttaatagtaa t                                                 21

<210>  134
<211>  23
<212>  DNA
<213>  artificial

<220>
<223>  primer

<400>  134
gggccauuua gauuaggaad tdt                                               23

<210>  135
<211>  23
<212>  DNA
<213>  artificial

<220>
<223>  primer

<400>  135
uuccuaaucu aaauggcccd tdt                                              23

<210>  136
<211>  21
<212>  DNA
<213>  artificial

<220>
<223>  primer

<400>  136
aagggccatt tagattagga a                                                21